(19) Europäisches Patentamt
European Patent Office
Office européen des brevets

(11) Publication number: **0 178 101**
**B1**

(12) # EUROPEAN PATENT SPECIFICATION

(45) Date of publication of patent specification: **16.05.90**

(21) Application number: **85306946.6**

(22) Date of filing: **27.09.85**

(51) Int. Cl.$^5$: **C 07 D 251/16,**
C 07 D 239/42,
C 07 D 403/12,
C 07 D 405/12,
C 07 D 409/12,
C 07 D 249/14,
C 07 D 491/048,
C 07 D 491/052,
C 07 C 311/16, A 01 N 47/36

(54) **Herbicidal ortho-heterocyclic sulfonamides.**

(30) Priority: **28.09.84 US 655530**
**09.07.85 US 752112**
**15.11.84 US 671852**
**25.07.85 US 757739**
**13.11.84 US 671072**
**22.07.85 US 756538**
**15.11.84 US 671851**
**11.07.85 US 753118**

(43) Date of publication of application:
**16.04.86 Bulletin 86/16**

(45) Publication of the grant of the patent:
**16.05.90 Bulletin 90/20**

(84) Designated Contracting States:
**BE CH DE FR GB IT LI LU NL SE**

(56) References cited:
**EP-A- 13 480** **EP-A- 103 543**
**EP-A- 30 142** **EP-A- 116 518**
**EP-A- 44 209** **EP-A-0 023 140**
**EP-A- 85 476** **EP-A-0 030 433**
**EP-A- 95 925**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Thompson, Mark Ewell**
**34 Austin Circle**
**Wilmington Delaware 19810 (US)**

(74) Representative: **Hildyard, Edward Martin et al**
**Frank B. Dehn & Co. European Patent Attorneys**
**Imperial House 15-19 Kingsway**
**London WC2B 6UZ (GB)**

(56) References cited:
**EP-A-0 039 239** **EP-A-0 084 224**
**EP-A-0 041 404** **EP-A-0 101 670**
**EP-A-0 045 196** **EP-A-0 107 979**
**EP-A-0 057 546** **GB-A-2 112 783**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 178 101 B1

## Description

European Patent Application (EP—A) No. 83,975, published July 20, 1983, discloses herbicidal benzenesulfonamides of formula

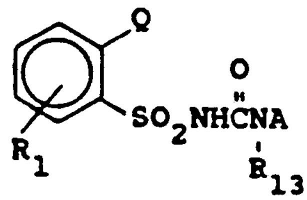

wherein

Q is selected from various five or six-membered aromatic or partially unsaturated heterocyclic rings containing 2 or 3 heteroatoms selected from O, S or NR.

European Patent Application (EP—A) No. 85,476, published August 10, 1983, discloses herbicidal benzenesulfonamides of formulae

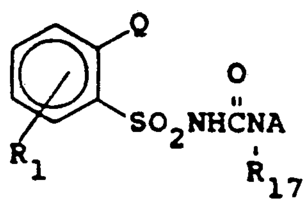

and

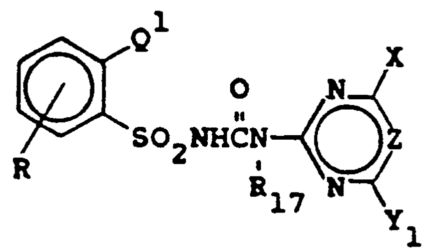

wherein

Q is selected from various 5-membered aromatic heterocycles, and their dihydro and tetrahydro analogs, which contain one heteroatom selected from O, S or NR, or Q is a saturated or partially unsaturated 6-membered ring containing one heteroatom selected from O or S; and

$Q^1$ is a 6-membered aromatic heterocycle containing one to three N atoms.

South African Patent Application 838,416, published May, 1984, discloses herbicidal benzenesulfonamides of formula

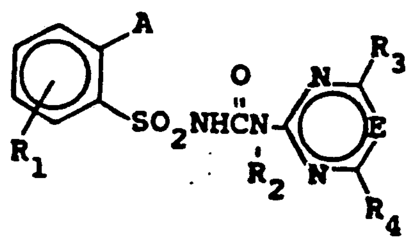

wherein

A is an unsaturated or only partially saturated 5- or 6-membered heterocyclic ring system which is bonded through a carbon atom and contains 1, 2 or 3 heteroatoms.

U.S. 4,370,480, issued January 25, 1983, discloses herbicidal benzenesulfonamides of formula

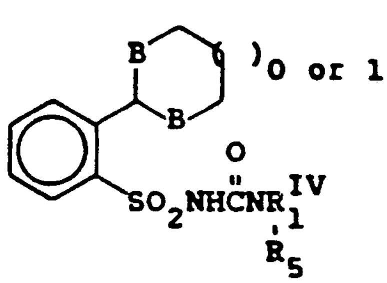

wherein

B is O or $S(O)_G$; and

G is O or 2.

EP—A—116,518, published 22 August 1984, Swiss priority 4 February 1983, discloses herbicidal sulfonylureas of the formula

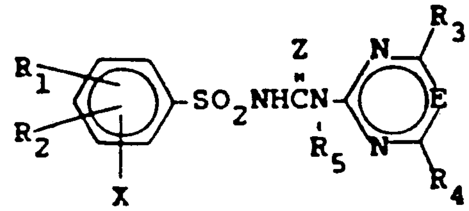

where, in part,

X is $-NR_6R_7$, $-N(SO_2R_9)_2$ or 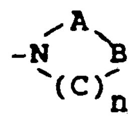 ;

A is $-CO-$, $-SO_2-$, $-CONR_{23}-$ or $-CO_2-$;
B is $C_1$—$C_4$ alkylene or $C_2$—$C_4$ alkenylene;
C is $-CO-$, $CR_{21}R_{22}$ or $-SO_2-$; and
n is 0 or 1.

Herbicidal thiophene sulfonamides are disclosed in European Patent Application (EP—A) Nos. 30,142, published June 10, 1981, and 97,122, Ciba-Geigy, published December 28, 1983, and in U.S. Patents 4,127,405, 4,169,719, 4,398,939 and 4,441,910.

EP—A—95,925, published December 7, 1983, discloses herbicidal pyrazolesulfonamides in which the group adjacent to the sulfonamide moiety may be selected from H, $C_1$—$C_3$ alkyl, F, Cl, Br, $NO_2$, $OR_{16}$, $CO_2R_{23}$, $S(O)_nR_{24}$ or $SO_2NR_{19}R_{20}$.

EP—A—87,780, published September 7, 1983, filed by Nissan Chemical Industries claims pyrazole sulfonamides of general formula

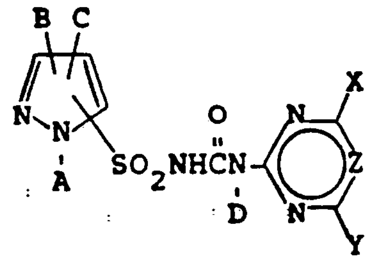

wherein
A is H, $C_1$—$C_8$ alkyl or optionally substituted phenyl;
B and C are independently H, halogen, $NO_2$, $C_2$—$C_8$ alkyl, $CO_2R$, etc.; and
D is H or $C_1$—$C_8$ alkyl.

EP—A—96,003, Ciba-Geigy, published November 28, 1983, discloses herbicidal compounds of general formula

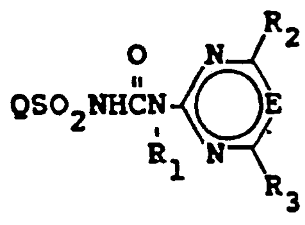

wherein
$R_1$ is H or $C_1$—$C_3$ alkyl; and
Q is an unsubstituted or substituted 5-membered heterocycle radical which is bound by way of a carbon atom and which contains 2 or 3 identical or different heteroatoms. Herbicidal pyrrole sulfonamides are disclosed in EP—A—39,239.

Unexamined Japanese Patent Application 9,013,778, priority July 15, 1982, filed by Nihon Noyaku describes herbicidal pyrazolesulfonamides with halo, alkyl and alkoxycarbonyl substituents.

Herbicidal pyridinesulfonamides are disclosed in European Patent Application (EP—A) Nos. 13,480, published July 23, 1980; 35,893, published September 16, 1981; 97,122, Ciba-Geigy, published December 28, 1983; and 103,543, Ciba-Geigy, published March 21, 1984; and in U.S. Patent 4,435,206.

European Patent Application EP—A No. 44,209, published January 20, 1982, discloses herbicidal benzenesulfonamides of formula

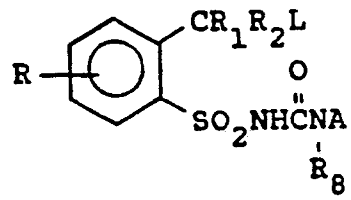

wherein

L is $CO_2R_{10}$, $CONR_3R_4$, CN, Cl, Br, $NR_3R_4$, $N^+R_3R_4R_4'$, $N(R_4)C(O)R_5$, $N(R_4)C(O)NHR_6$, $N(R_4)C(O)OR_7$, $S(O)_nR_7$, $OR_9$, $SO_2NR_3R_4$, OH, $OC(O)R_{11}$, $OC(O)NHR_{12}$ or $OC(O)OR_{13}$;

$R_1$ is H, Cl or $C_1$—$C_4$ alkyl; and

$R_2$ is H or $CH_3$.

U.S. 4,435,205, issued March 6, 1984, discloses herbicidal benzenesulfonamides of formula

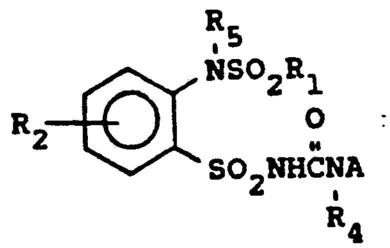

wherein

$R_1$ is $C_1$—$C_4$ alkyl, $C_1$—$C_4$ alkyl substituted with 1—3 atoms of F, Cl or Br,

$CH_2CH_2OCH_3$, $CH_2CH_2CH_2OCH_3$ or 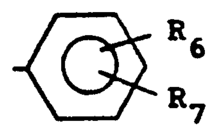 ;

and

$R_5$ is $C_1$—$C_4$ alkyl.

U.S. 4,368,069, issued January 11, 1983, discloses herbicidal benzenesulfonamides of formula

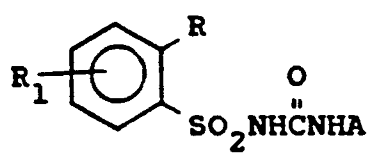

wherein

R is $—(CR_5R_6)_n—R_2$;

n is 0 or 1: and

$R_2$ may be $C_5$—$C_6$ cycloalkenyl.

U.S. 4,225,337, issued September 30, 1980, discloses herbicidal benzenesulfonamides of formula

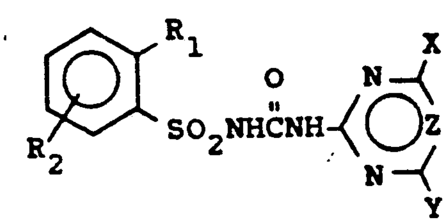

wherein

$R_1$ may be H;

$R_2$ is NCO, $NHC(O)OR_3$, $NHC(O)SR_3$, $NHC(O)R_3$, $NHC(O)NR_4R_5$ or $NR_6R_7$; and

$R_6$ and $R_7$ may be taken together as $—(CH_2)_n—$ where n is 4 or 5 or as $—CH_2CH_2OCH_2CH_2—$.

Additionally the disclosure in the following U.S. Patents relate to the matter disclosed in the above applications: U.S. 4,348,219; U.S. 4,348,220; U.S. 4,397,679; and U.S. 4,332,611.

Summary of the Invention

This invention pertains to novel compounds of Formula I, agriculturally suitable compositions containing them and their method-of-use as general and/or selective preemergent and/or postemergent herbicides or plant growth regulants.

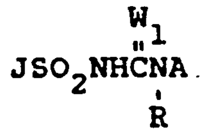

I

wherein

J is

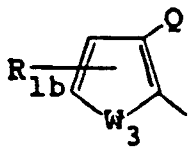 ,

J-5

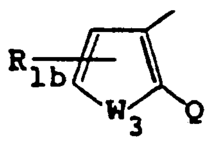 ,

J-6

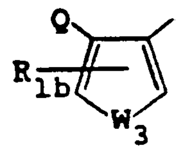 ,

J-7

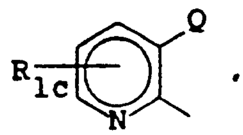 ,

J-8

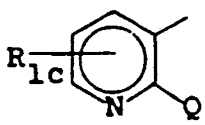 ,

J-9

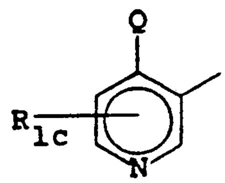 or

J-10

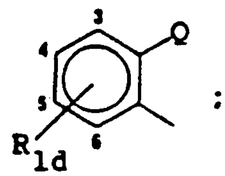 ;

J-11

$W_1$ is O or S;

R is H or $CH_3$;

$W_3$ is O, S or NR''';

$R_{1b}$, $R_{1c}$ and $R_{1d}$ are independently H, $C_1$—$C_3$ alkyl, $C_1$—$C_3$ haloalkyl, $C_1$—$C_3$ haloalkoxy, halogen, nitro, $C_1$—$C_3$ alkoxy, $SO_2NR^{I}R^{II}$, $C_1$—$C_3$ alkylthio, $C_1$—$C_3$ alkylsulfinyl, $C_1$—$C_3$ haloalkylthio, $C_1$—$C_3$ alkylsulfonyl, $CO_2R^{III}$, amino, $C_1$—$C_3$ alkylamino, di($C_1$—$C_3$ alkyl)amino, $CH_2CN$, $CH_2OCH_3$ or $CH_2SCH_3$;

$R^{I}$ is H, $C_1$—$C_4$ alkyl, $C_2$—$C_3$ cyanoalkyl, methoxy or ethoxy;

$R^{II}$ is H, $C_1$—$C_4$ alkyl or $C_3$—$C_4$ alkenyl; or

$R^{I}$ and $R^{II}$ may be taken together as $-(CH_2)_3-$, $-(CH_2)_4-$, $-(CH_2)_5-$ or $-CH_2CH_2OCH_2CH_2-$;

$R^{III}$ is $C_1$—$C_4$ alkyl, $C_3$—$C_4$ alkenyl, $C_3$—$C_4$ alkynyl, $C_2$—$C_4$ haloalkyl, $C_2$—$C_3$ cyanoalkyl, $C_5$—$C_6$ cycloalkyl, $C_4$—$C_7$ cycloalkylalkyl or $C_2$—$C_4$ alkoxyalkyl;

Q is

Q is 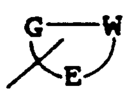 ,

$Q_1$

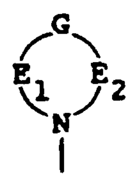 or

$Q_2$

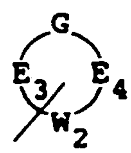 ;

$Q_3$

G is C=O or $SO_2$;

W is O, S, $CHR_2$ or $NR_3$;

$W_2$ is O, S, $SO_2$, $CHR_2$ or $NR_3$;

$R_2$ is H, $C_1$—$C_2$ alkyl, Cl, F or Br;

$R_3$ is H, $C_1$—$C_4$ alkyl, $C_1$—$C_4$ haloalkyl, $C_2$—$C_4$ alkoxyalkyl, $C_2$—$C_4$ cyanoalkyl, $C_3$—$C_4$ alkenyl or $C_3$—$C_4$ alkynyl;

E is $C_3$—$C_4$ alkylene, $C_3$—$C_4$ alkenylene or $C_4$ alkenyldienyl;

$E_1$ and $E_3$ are independently $C_1$—$C_2$ alkylene or $C_2$ alkenylene;

$E_2$ and $E_4$ are independently $C_2$—$C_3$ alkylene or $C_2$—$C_3$ alkenylene; and

E, $E_1$, $E_2$, $E_3$ and $E_4$ may optionally be substituted by 1—4 groups selected from $C_1$—$C_4$ alkyl, $C_1$—$C_4$ alkoxy, $C_1$—$C_4$ alkenyl, OH, halogen and $C_1$—$C_4$ haloalkoxy; further, when W is O, $CHR_2$ or $NR_3$, one of the carbon atoms of E may be in the form of a carbonyl group, and when $W_2$ is O, $CHR_2$ or $NR_3$, one of the

carbon atoms of $E_3$ or $E_4$ may be in the form of a carbonyl group, provided that said carbonyl groups are not bonded directly to G;

A is 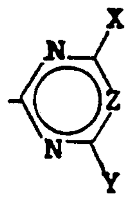 , 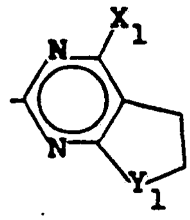 , 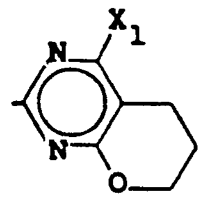 , 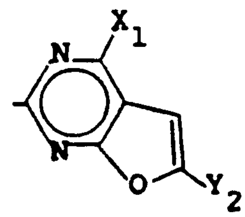 ,

A-1 A-2 A-3 A-4

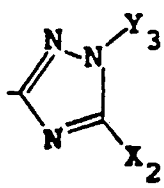 or 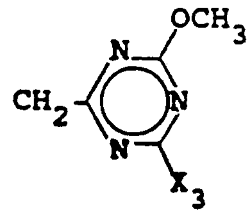 ;

A-5 A-6

X is H, $C_1$—$C_4$ alkyl, $C_1$—$C_4$ alkoxy, $C_1$—$C_4$ haloalkoxy, $C_1$—$C_4$ haloalkyl, $C_1$—$C_4$ haloalkylthio, $C_1$—$C_4$ alkylthio, halogen, $C_2$—$C_5$ alkoxyalkyl, $C_2$—$C_5$ alkoxyalkoxy, amino, $C_1$—$C_3$ alkylamino or di($C_1$—$C_3$ alkyl)amino;

Y is H, $C_1$—$C_4$ alkyl, $C_1$—$C_4$ alkoxy, $C_1$—$C_4$ haloalkoxy, $C_1$—$C_4$ haloalkylthio, $C_1$—$C_4$ alkylthio, $C_2$—$C_5$ alkoxyalkyl, $C_2$—$C_5$ alkoxyalkoxy, amino, $C_1$—$C_3$ alkylamino, di($C_1$—$C_3$ alkyl)amino, $C_3$—$C_4$ alkenyloxy, $C_3$—$C_4$ alkynyloxy, $C_2$—$C_5$ alkylthioalkyl, $C_1$—$C_4$ haloalkyl, $C_3$—$C_5$ cycloalkyl, $C_2$—$C_4$ alkynyl,

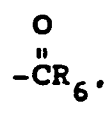 , 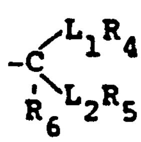 , 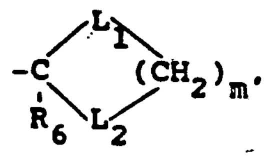 , 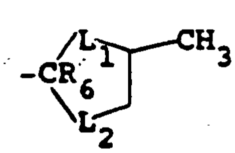 ,

$N(OCH_3)CH_3$, $C_2$—$C_5$ alkylsulfinylalkyl or $C_2$—$C_5$ alkylsulfonylalkyl;

m is 2 or 3;

$L_1$ and $L_2$ are independently O or S;

$R_4$ and $R_5$ are independently $C_1$—$C_2$ alkyl;

$R_6$ is H or $CH_3$;

Z is CH or N;

$Y_1$ is O or $CH_2$;

$X_1$ is $CH_3$, $OCH_3$, $OC_2H_5$ or $OCF_2H$;

$Y_2$ is H or $CH_3$;

$X_2$ is $CH_3$, $OCH_3$ or $SCH_3$;

$Y_3$ is $CH_3$, $CH_2CH_3$ or $CH_2CF_3$; and

$X_3$ is $CH_3$ or $OCH_3$;

and their agriculturally suitable salts;

provided that

a) when G is $SO_2$, then W is O, $CHR_2$ or $NR_3$;

b) when $E_1$ or $E_3$ is $C_2$ alkylene or $C_2$ alkenylene; then $E_2$ or $E_4$ is $C_2$ alkylene or $C_2$ alkenylene;

c) when X is Cl, F, Br or I, then Z is CH and Y is $OCH_3$, $OC_2H_5$, $N(OCH_3)CH_3$, $NHCH_3$, $N(CH_3)_2$ or $OCF_2H$;

d) when X or Y is $OCF_2H$, then Z is CH;

e) when the total number of carbon atoms of X and Y is greater than four, then the number of carbons of $R_{1b}$, $R_{1c}$ or $R_{1d}$ is less than or equal to two and the number of carbons of Q is less than or equal to eight; and

f) when $W_1$ is S, then R is H, A is A—1, and Y is

$CH_3$, $OCH_3$, $OC_2H_5$, $CH_2OCH_3$, $C_2H_5$, $CF_3$, $SCH_3$, $OCH_2CH{=}CH_2$,

$OCH_2C{\equiv}CH$, $OCH_2CH_2OCH_3$, $CH(OCH_3)_2$ or 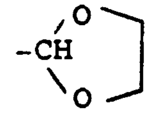.

In the above definitions, the term "alkyl" used either alone or in compound words such as "alkylthio" or "haloalkyl", denotes straight chain or branched alkyl, e.g., methyl, ethyl, *n*-propyl, isopropyl or the different butyl isomers.

Alkoxy denotes methoxy, ethoxy, *n*-propoxy, isopropoxy and the different butoxy isomers.

Alkenyl denotes straight chain or branched alkenes, e.g., vinyl, 1-propenyl, 2-propenyl, 3-propenyl and the different butenyl isomers; $C_1$ alkenyl denotes an exocyclic double bond.

Alkynyl denotes straight chain or branched alkynes, e.g., ethynyl, 1-propynyl, 2-propynyl and the different butynyl isomers.

The term "halogen", either alone or in compound words such as "haloalkyl", denotes fluorine, chlorine, bromine or iodine.

In terms such as $C_2$—$C_3$ alkylthioalkyl, the specified number of carbon atoms is meant to define the *total* number of carbon atoms in that substituent group. For example, $C_2$—$C_3$ alkylthioalkyl would designate $CH_2SCH_3$, $CH_2SC_2H_5$, $CH_2CH_2SCH_2$, and $C_2$—$C_5$ alkoxyalkoxy would represent $OCH_2OCH_2$ through $O(CH_2)_4OCH_3$ or $OCH_2O(CH_2)_3CH_3$ and the various structural isomers embraced therein.

$C_4$—$C_7$ cycloalkylalkyl means cyclopropylmethyl through cyclopropylbutyl or cyclohexylmethyl.

Alkylsulfonyl denotes methylsulfonyl, ethylsulfonyl and the different propylsulfonyl isomers.

Alkylthio, alkylsulfinyl, alkylamino, etc. are defined in analogous manner.

Alkylene denotes methylene (—$CH_2$—), ethylene (—$CH_2CH_2$—), propylene or butylene; alkenylene denotes —CH=CH—, —$CH{=}CHCH_2$—, —$CH{=}CHCH_2CH_2$— or —$CH_2CH{=}CHCH_2$—; and alkenyldienyl denotes —CH=CH—CH=CH—.

Preferred for reasons of increased ease of synthesis and/or greater herbicidal efficacy are:

1) Compounds of Formula I where

$W_1$ is O; and

R is H.

2) Compounds of Preferred 1 where

$R_{1b}$ is H, Cl, Br, $NO_2$, $CH_3$, $OCH_3$ or $CF_3$;

$R_{1c}$ is H, Cl, $CH_3$, $OCH_3$ or $N(CH_3)_2$;

$R_{1d}$ is H, $CH_3$, $OCH_3$, Cl, Br, F, $NO_2$, $CF_3$ or $OCF_2H$, and is not in the 4-position;

$W_3$ is S;

X is $CH_3$, $OCH_3$, $OCH_2CH_3$, Cl, F, Br, I, $OCF_2H$, $CH_2F$, $OCH_2CH_2F$, $OCH_2CHF_2$, $OCH_2CF_3$, $CF_3$, $CH_2Cl$ or $CH_2Br$;

Y is

H, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH{=}CH_2$, $OCH_2C{\equiv}CH$, $CH_2OCH_2CH_3$, $OCH_2CH_2OCH_3$,

$CH_2SCH_3$, 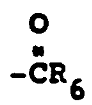, 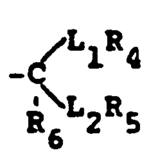, 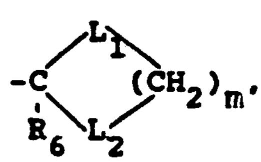,

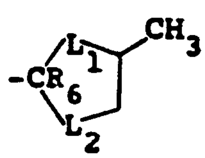, $OCF_2H$, $SCF_2H$, cyclopropyl, $C{\equiv}CH$ or $C{\equiv}CCH_3$.

3) Compounds of Preferred 2 where Q is $Q_1$.

4) Compounds of Preferred 2 where Q is $Q_2$.

5) Compounds of Preferred 2 where Q is $Q_3$.

6) Compounds of Preferred 2 where Q is

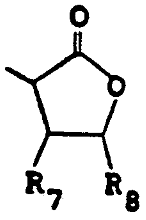 ,
Q-1

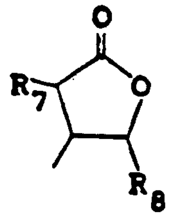 ,
Q-2

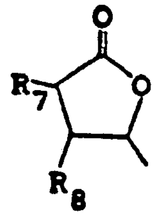 ,
Q-3

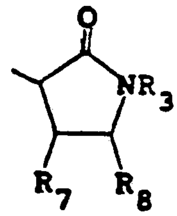 ,
Q-4

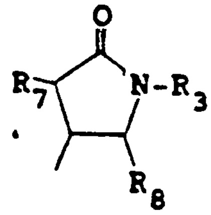 ,
Q-5

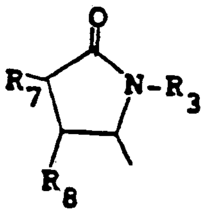 ,
Q-6

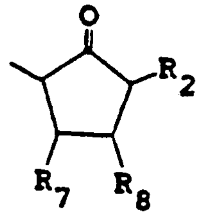 ,
Q-8

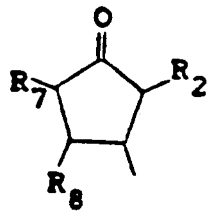 ,
Q-9

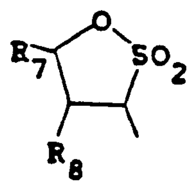 ,
Q-10

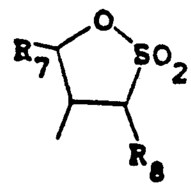 ,
Q-11

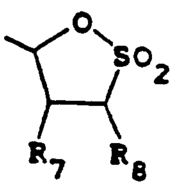 ,
Q-12

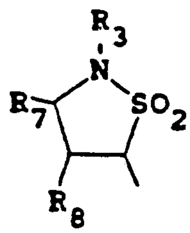 ,
Q-13

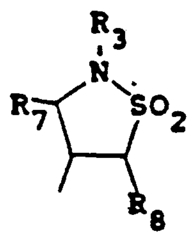 ,
Q-14

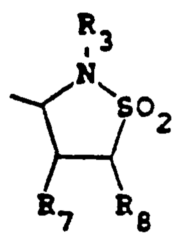 ,
Q-15

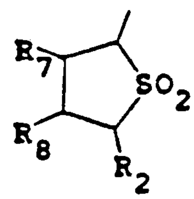 ,
Q-17

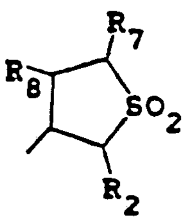 ,
Q-18

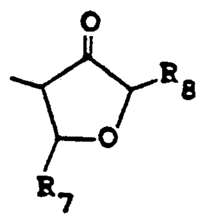 ,
Q-19

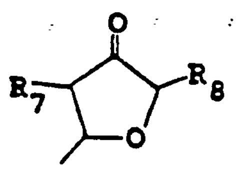 ,
Q-20

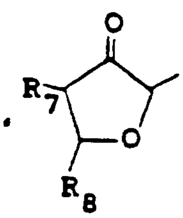 ,
Q-21

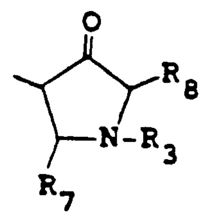 ,
Q-22

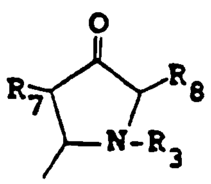 ,
Q-23

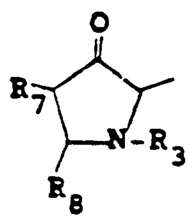 ,
Q-24

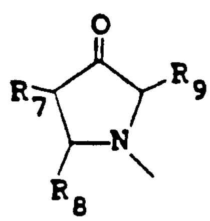 ,
Q-25

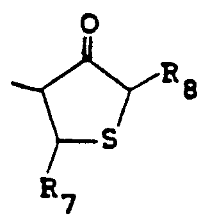 ,
Q-26

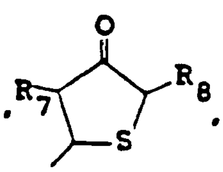 ,
Q-27

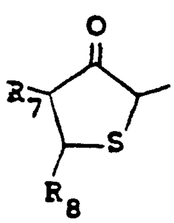 ,
Q-28

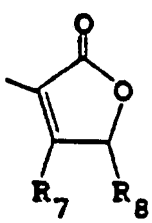 ,
Q-29

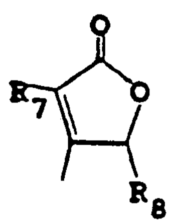 ,
Q-30

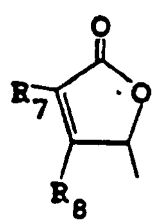 ,
Q-31

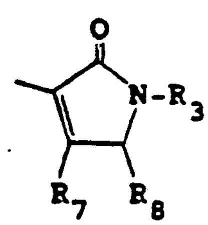 ,
Q-32

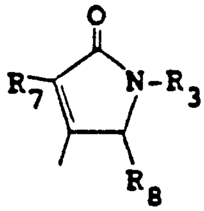 ,

Q-33

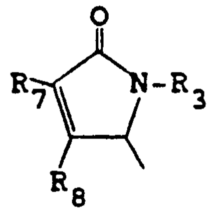 ,

Q-34

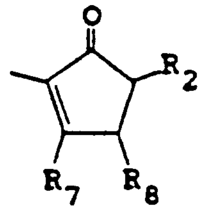 ,

Q-36

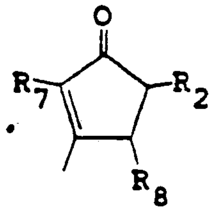 ,

Q-37

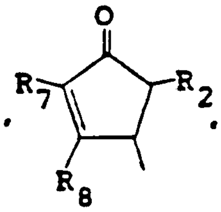 ,

Q-38

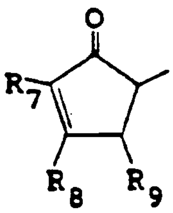 ,

Q-39

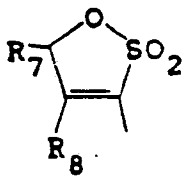 ,

Q-40

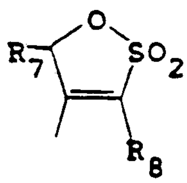 ,

Q-41

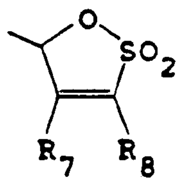 ,

Q-42

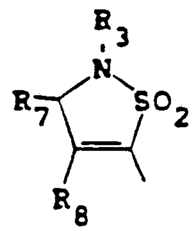 ,

Q-43

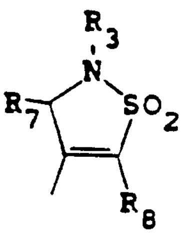 ,

Q-44

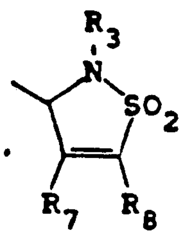 ,

Q-45

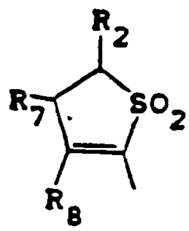 ,

Q-47

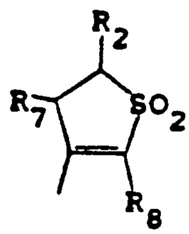 ,

Q-48

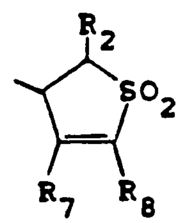 ,

Q-49

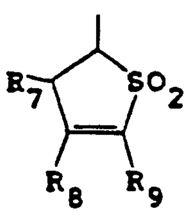 ,

Q-50

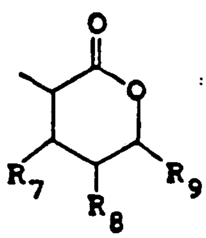 ,

Q-51

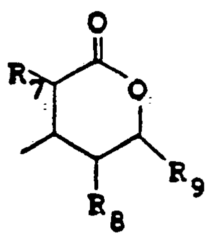 ,

Q-52

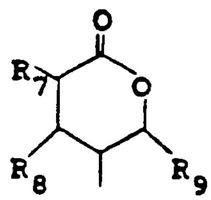 ,

Q-53

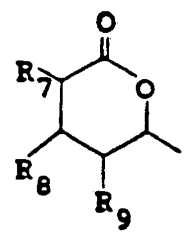 ,

Q-54

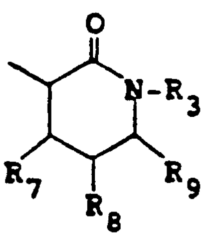 ,

Q-55

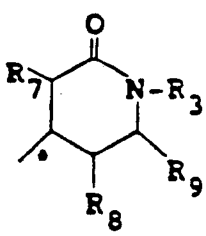 ,

Q-56

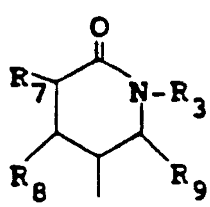 ,

Q-57

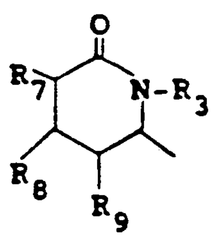 ,

Q-58

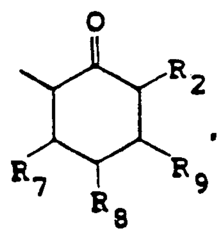 ,

Q-60

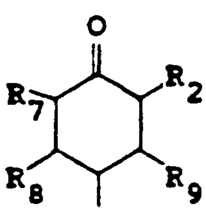 ,

Q-61

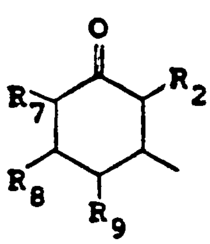 ,

Q-62

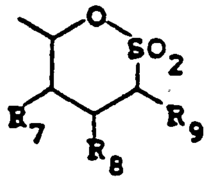 ,

Q-63

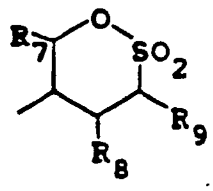 ,

Q-64

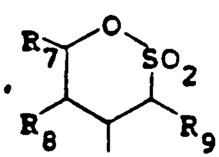 ,

Q-65

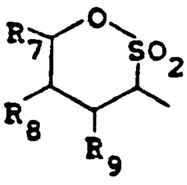 ,

Q-66

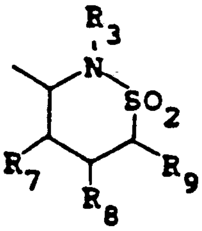 ,

Q-67

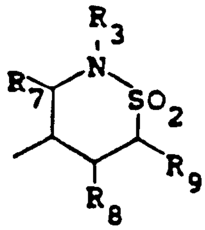 ,

Q-68

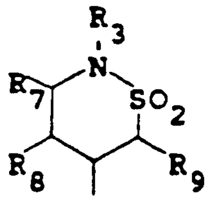 ,

Q-69

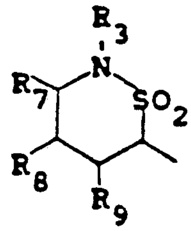 ,

Q-70

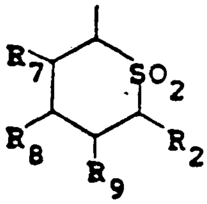 ,

Q-72

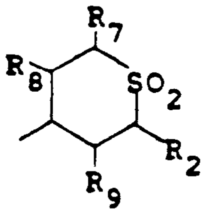 ,

Q-73

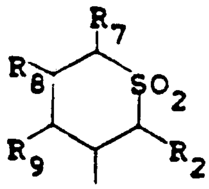 ,

Q-74

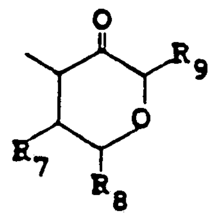 ,

Q-75

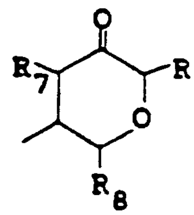 ,

Q-76

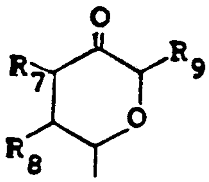 ,

Q-77

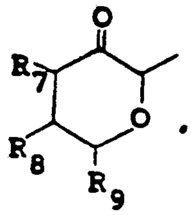 ,

Q-78

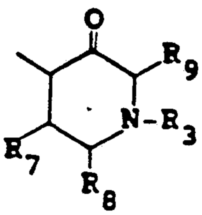 ,

Q-79

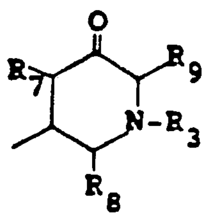 ,

Q-80

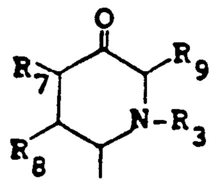 ,

Q-81

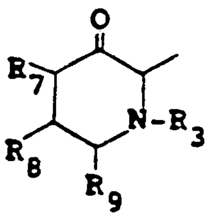 ,

Q-82

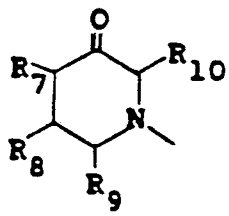 ,

Q-83

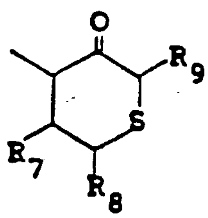 ,

Q-84

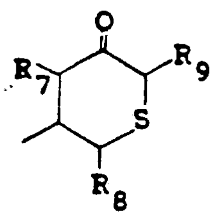 ,

Q-85

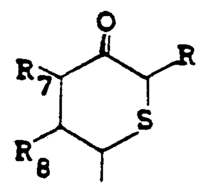 ,

Q-86

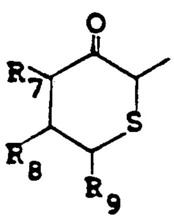 ,

Q-87

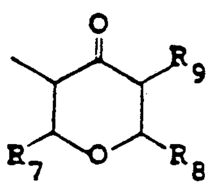 ,

Q-88

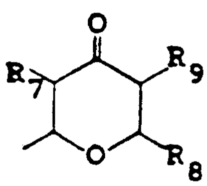 ,

Q-89

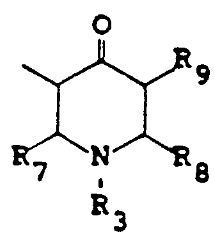 ,

Q-90

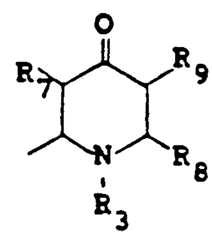 ,

Q-91

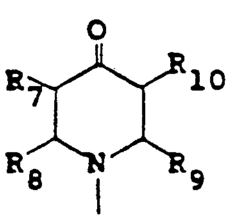 ,

Q-92

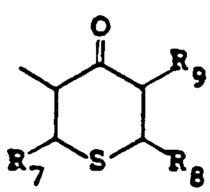 ,

Q-93

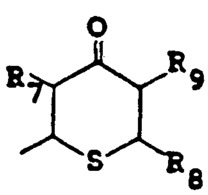 ,

Q-94

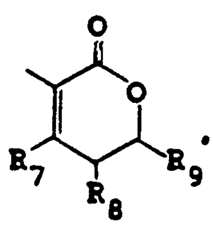 ,

Q-95

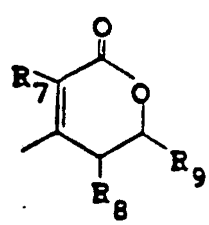 ,

Q-96

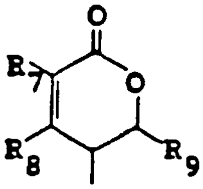

Q-97,

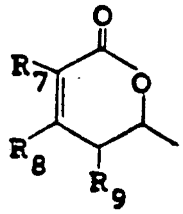

Q-98,

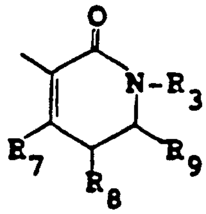

Q-99,

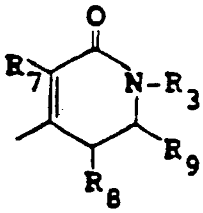

Q-100,

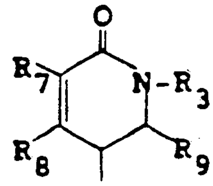

Q-101,

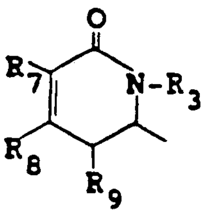

Q-102,

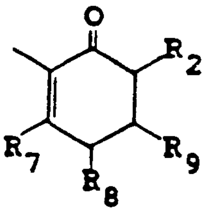

Q-104,

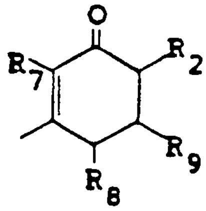

Q-105,

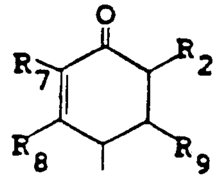

Q-106,

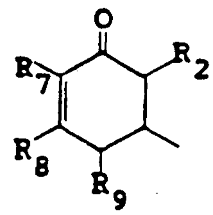

Q-107,

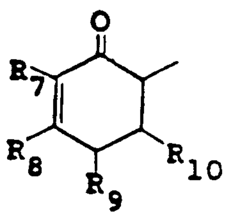

Q-108,

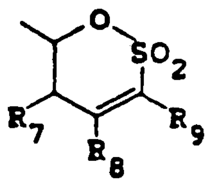

Q-109,

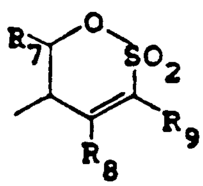

Q-110,

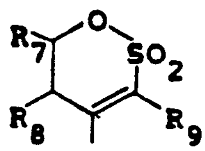

Q-111,

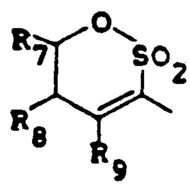

Q-112,

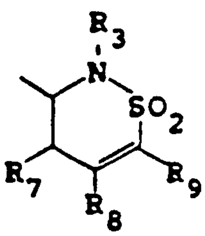

Q-113,

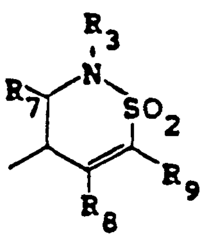

Q-114,

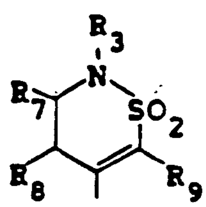

Q-115,

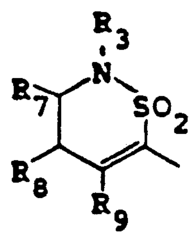

Q-116,

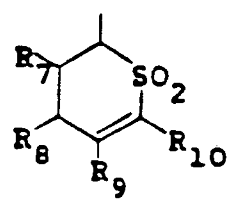

Q-118,

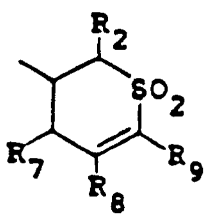

Q-119,

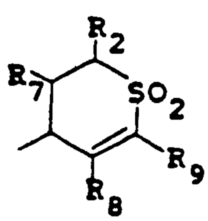

Q-120,

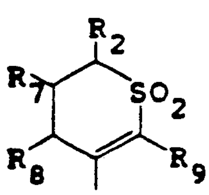

Q-121,

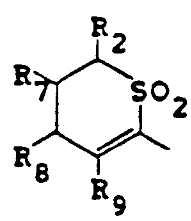

Q-122,

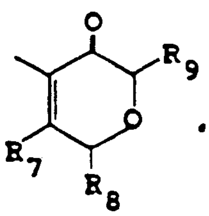

Q-123,

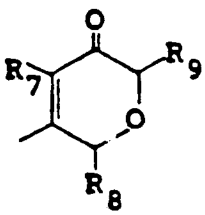

Q-124,

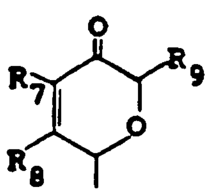

Q-125,

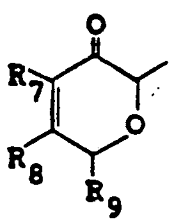

Q-126,

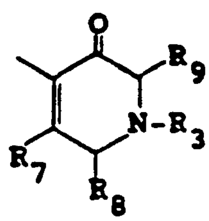

Q-127,

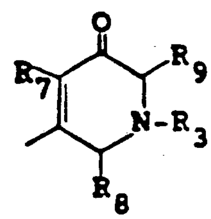

Q-128,

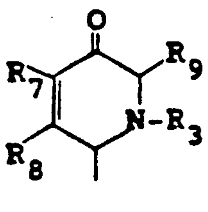,
Q-129

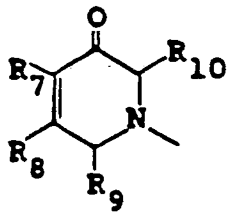,
Q-130

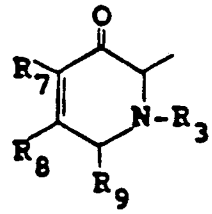,
Q-131

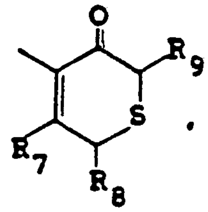,
Q-132

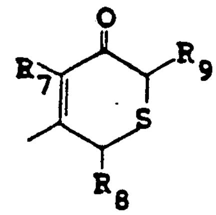,
Q-133

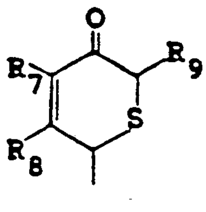,
Q-134

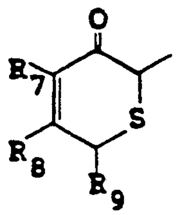,
Q-135

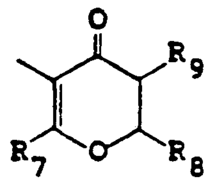,
Q-136

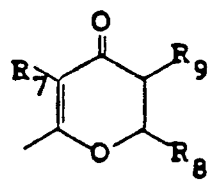,
Q-137

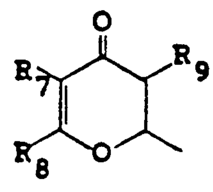,
Q-138

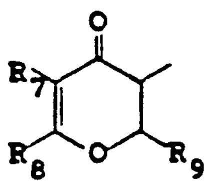,
Q-139

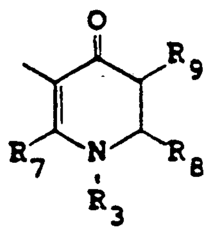,
Q-140

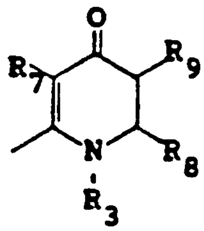,
Q-141

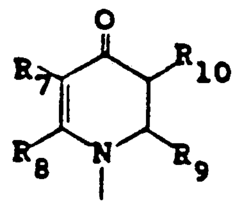,
Q-142

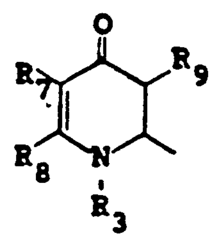,
Q-143

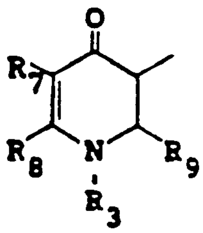,
Q-144

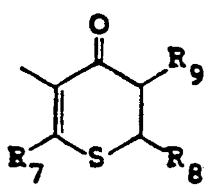,
Q-145

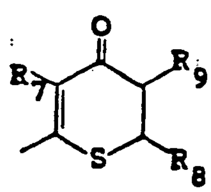,
Q-146

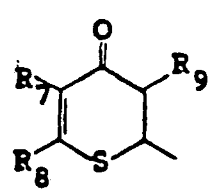,
Q-147

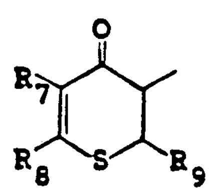,
Q-148

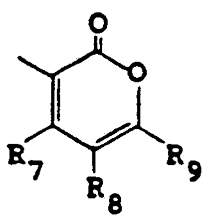,
Q-149

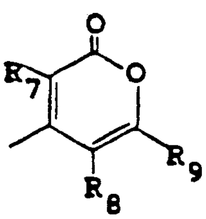,
Q-150

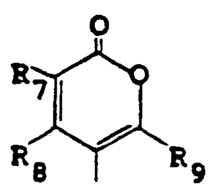,
Q-151

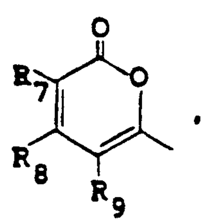,
Q-152

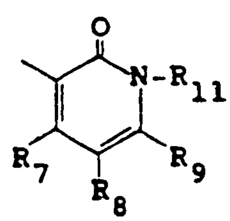,
Q-153

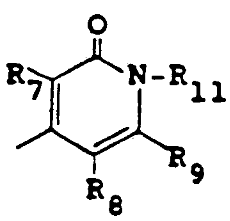,
Q-154

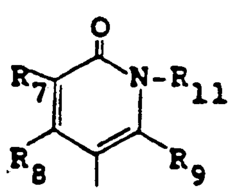,
Q-155

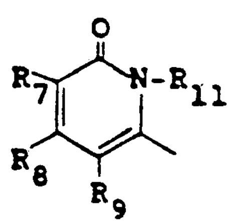,
Q-156

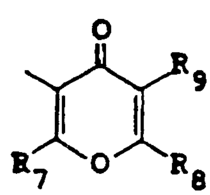,
Q-158

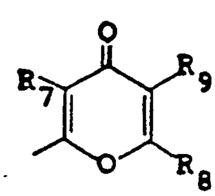,
Q-159

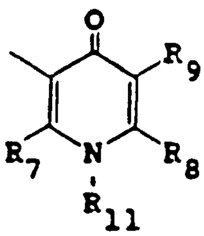 ,
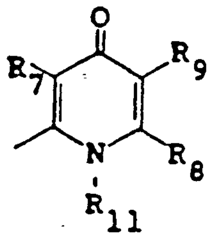 ,
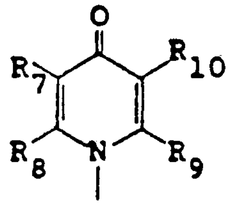 ,
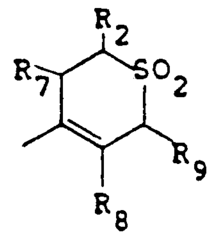 ,
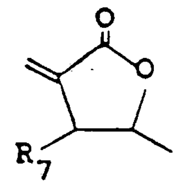 ,

Q-160 Q-161 Q-162 Q-163 Q-164

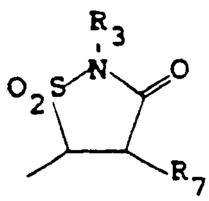 ,
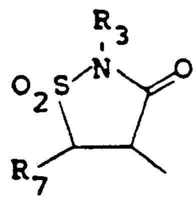 or
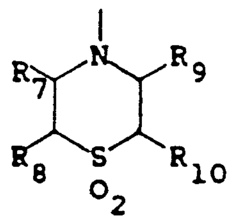 ;

Q-165 Q-166 Q-167

$R_7$, $R_8$, $R_9$ and $R_{10}$ are independently H or $CH_3$; and
$R_{11}$ is H, $CH_3$ or $CH_2CH_3$;
7) Compounds of Preferred 6 where J is J—5, J—6, J—8, J—9 or J—11;
$R_{1b}$ is H, $CH_3$ or Cl;
$R_{1c}$ is H:
$R_{1d}$ is H, $CH_3$, $OCH_3$ or Cl; and
Y is $CH_3$, $OCH_3$, $C_2H_5$, $OC_2H_5$, $CH_2OCH_3$, $NHCH_3$, $CH(OCH_3)_2$ or cyclopropyl.
8) Compounds of Preferred 7 where
$R_2$ is H or $CH_3$; and
$R_3$ is H, $CH_3$ or $C_2H_5$.
9) Compounds of Preferred 8 where
A is A—1; and
X is $CH_3$, $OCH_3$, Cl or $OCF_2H$.
10) Compounds of Preferred 9 where Q is Q—1.
11) Compounds of Preferred 9 where Q is Q—2.
12) Compounds of Preferred 9 where Q is Q—3.
13) Compounds of Preferred 9 where Q is Q—4.
14) Compounds of Preferred 9 where Q is Q—5.
15) Compounds of Preferred 9 where Q is Q—6.
16) Compounds of Preferred 9 where Q is Q—8.
17) Compounds of Preferred 9 where Q is Q—9.
18) Compounds of Preferred 9 where Q is Q—10.
19) Compounds of Preferred 9 where Q is Q—11.
20) Compounds of Preferred 9 where Q is Q—12.
21) Compounds of Preferred 9 where Q is Q—13.
22) Compounds of Preferred 9 where Q is Q—14.
23) Compounds of Preferred 9 where Q is Q—15.
24) Compounds of Preferred 9 where Q is Q—17.
25) Compounds of Preferred 9 where Q is Q—18.
26) Compounds of Preferred 9 where Q is Q—19.
27) Compounds of Preferred 9 where Q is Q—20.
28) Compounds of Preferred 9 where Q is Q—21.
29) Compounds of Preferred 9 where Q is Q—22.
30) Compounds of Preferred 9 where Q is Q—23.
31) Compounds of Preferred 9 where Q is Q—24.
32) Compounds of Preferred 9 where Q is Q—25.
33) Compounds of Preferred 9 where Q is Q—26.
34) Compounds of Preferred 9 where Q is Q—27.
35) Compounds of Preferred 9 where Q is Q—28.
36) Compounds of Preferred 9 where Q is Q—29.
37) Compounds of Preferred 9 where Q is Q—30.
38) Compounds of Preferred 9 where Q is Q—31.
39) Compounds of Preferred 9 where Q is Q—32.

40) Compounds of Preferred 9 where Q is Q—33.
41) Compounds of Preferred 9 where Q is Q—34.
42) Compounds of Preferred 9 where Q is Q—36.
43) Compounds of Preferred 9 where Q is Q—37.
44) Compounds of Preferred 9 where Q is Q—38.
45) Compounds of Preferred 9 where Q is Q—39.
46) Compounds of Preferred 9 where Q is Q—40.
47) Compounds of Preferred 9 where Q is Q—41.
48) Compounds of Preferred 9 where Q is Q—42.
49) Compounds of Preferred 9 where Q is Q—43.
50) Compounds of Preferred 9 where Q is Q—44.
51) Compounds of Preferred 9 where Q is Q—45.
52) Compounds of Preferred 9 where Q is Q—47.
53) Compounds of Preferred 9 where Q is Q—48.
54) Compounds of Preferred 9 where Q is Q—49.
55) Compounds of Preferred 9 where Q is Q—50.
56) Compounds of Preferred 9 where Q is Q—51.
57) Compounds of Preferred 9 where Q is Q—52.
58) Compounds of Preferred 9 where Q is Q—53.
59) Compounds of Preferred 9 where Q is Q—54.
60) Compounds of Preferred 9 where Q is Q—55.
61) Compounds of Preferred 9 where Q is Q—56.
62) Compounds of Preferred 9 where Q is Q—57.
63) Compounds of Preferred 9 where Q is Q—58.
64) Compounds of Preferred 9 where Q is Q—60.
65) Compounds of Preferred 9 where Q is Q—61.
66) Compounds of Preferred 9 where Q is Q—62.
67) Compounds of Preferred 9 where Q is Q—63.
68) Compounds of Preferred 9 where Q is Q—64.
69) Compounds of Preferred 9 where Q is Q—65.
70) Compounds of Preferred 9 where Q is Q—66.
71) Compounds of Preferred 9 where Q is Q—67.
72) Compounds of Preferred 9 where Q is Q—68.
73) Compounds of Preferred 9 where Q is Q—69.
74) Compounds of Preferred 9 where Q is Q—70.
75) Compounds of Preferred 9 where Q is Q—72.
76) Compounds of Preferred 9 where Q is Q—73.
77) Compounds of Preferred 9 where Q is Q—74.
78) Compounds of Preferred 9 where Q is Q—75.
79) Compounds of Preferred 9 where Q is Q—76.
80) Compounds of Preferred 9 where Q is Q—77.
81) Compounds of Preferred 9 where Q is Q—78.
82) Compounds of Preferred 9 where Q is Q—79.
83) Compounds of Preferred 9 where Q is Q—80.
84) Compounds of Preferred 9 where Q is Q—81.
85) Compounds of Preferred 9 where Q is Q—82.
86) Compounds of Preferred 9 where Q is Q—83.
87) Compounds of Preferred 9 where Q is Q—84.
88) Compounds of Preferred 9 where Q is Q—85.
89) Compounds of Preferred 9 where Q is Q—86.
90) Compounds of Preferred 9 where Q is Q—87.
91) Compounds of Preferred 9 where Q is Q—88.
92) Compounds of Preferred 9 where Q is Q—89.
93) Compounds of Preferred 9 where Q is Q—90.
94) Compounds of Preferred 9 where Q is Q—91.
95) Compounds of Preferred 9 where Q is Q—92.
96) Compounds of Preferred 9 where Q is Q—93.
97) Compounds of Preferred 9 where Q is Q—94.
98) Compounds of Preferred 9 where Q is Q—95.
99) Compounds of Preferred 9 where Q is Q—96.
100) Compounds of Preferred 9 where Q is Q—97.
101) Compounds of Preferred 9 where Q is Q—98.
102) Compounds of Preferred 9 where Q is Q—99.
103) Compounds of Preferred 9 where Q is Q—100.
104) Compounds of Preferred 9 where Q is Q—101.

105) Compounds of Preferred 9 where Q is Q—102.
106) Compounds of Preferred 9 where Q is Q—104.
107) Compounds of Preferred 9 where Q is Q—105.
108) Compounds of Preferred 9 where Q is Q—106.
109) Compounds of Preferred 9 where Q is Q—107.
110) Compounds of Preferred 9 where Q is Q—108.
111) Compounds of Preferred 9 where Q is Q—109.
112) Compounds of Preferred 9 where Q is Q—110.
113) Compounds of Preferred 9 where Q is Q—111.
114) Compounds of Preferred 9 where Q is Q—112.
115) Compounds of Preferred 9 where Q is Q—113.
116) Compounds of Preferred 9 where Q is Q—114.
117) Compounds of Preferred 9 where Q is Q—115.
118) Compounds of Preferred 9 where Q is Q—116.
119) Compounds of Preferred 9 where Q is Q—118.
120) Compounds of Preferred 9 where Q is Q—119.
121) Compounds of Preferred 9 where Q is Q—120.
122) Compounds of Preferred 9 where Q is Q—121.
123) Compounds of Preferred 9 where Q is Q—122.
124) Compounds of Preferred 9 where Q is Q—123.
125) Compounds of Preferred 9 where Q is Q—124.
126) Compounds of Preferred 9 where Q is Q—125.
127) Compounds of Preferred 9 where Q is Q—126.
128) Compounds of Preferred 9 where Q is Q—127.
129) Compounds of Preferred 9 where Q is Q—128.
130) Compounds of Preferred 9 where Q is Q—129.
131) Compounds of Preferred 9 where Q is Q—130.
132) Compounds of Preferred 9 where Q is Q—131.
133) Compounds of Preferred 9 where Q is Q—132.
134) Compounds of Preferred 9 where Q is Q—133.
135) Compounds of Preferred 9 where Q is Q—134.
136) Compounds of Preferred 9 where Q is Q—135.
137) Compounds of Preferred 9 where Q is Q—136.
138) Compounds of Preferred 9 where Q is Q—137.
139) Compounds of Preferred 9 where Q is Q—138.
140) Compounds of Preferred 9 where Q is Q—139.
141) Compounds of Preferred 9 where Q is Q—140.
142) Compounds of Preferred 9 where Q is Q—141.
143) Compounds of Preferred 9 where Q is Q—142.
144) Compounds of Preferred 9 where Q is Q—143.
145) Compounds of Preferred 9 where Q is Q—144.
146) Compounds of Preferred 9 where Q is Q—145.
147) Compounds of Preferred 9 where Q is Q—146.
148) Compounds of Preferred 9 where Q is Q—147.
149) Compounds of Preferred 9 where Q is Q—148.
150) Compounds of Preferred 9 where Q is Q—149.
151) Compounds of Preferred 9 where Q is Q—150.
152) Compounds of Preferred 9 where Q is Q—151.
153) Compounds of Preferred 9 where Q is Q—152.
154) Compounds of Preferred 9 where Q is Q—153.
155) Compounds of Preferred 9 where Q is Q—154.
156) Compounds of Preferred 9 where Q is Q—155.
157) Compounds of Preferred 9 where Q is Q—156.
158) Compounds of Preferred 9 where Q is Q—158.
159) Compounds of Preferred 9 where Q is Q—159.
160) Compounds of Preferred 9 where Q is Q—160.
161) Compounds of Preferred 9 where Q is Q—161.
162) Compounds of Preferred 9 where Q is Q—162.
163) Compounds of Preferred 9 where Q is Q—163.
164) Compounds of Preferred 9 where Q is Q—164.
165) Compounds of Preferred 9 where Q is Q—165.
166) Compounds of Preferred 9 where Q is Q—166.
167) Compounds of Preferred 9 where Q is Q—167.

Specifically preferred for reasons of greatest ease of synthesis and/or greatest herbicidal efficacy are:

N-[(4,6-dimethoxypyrimidin-2-yl)aminocarbonyl]-2-tetrahydro-2-oxo-3-furanylbenzenesulfonamide, m.p. 163.5—166.5°C;

N-[(4-methoxy-6-methyltriazin-2-yl)aminocarbonyl]-2-tetrahydro-2-oxo-3-furanylbenzenesulfonamide, m.p. 186.5—188.5°C;

N-[(4,6-dimethoxypyrimidin-2-yl)aminocarbonyl]-2-(tetrahydro-2-oxofuran-3-yl)-3-thiophenesulfonamide;

N-[(4-methoxy-6-methyltriazin-2-yl)aminocarbonyl]-2-(tetrahydro-2-oxofuran-3-yl)-3-thiophenesulfonamide;

N-[(4,6-dimethylpyrimidin-2-yl)aminocarbonyl]-2-(3-oxo-1-cyclohexen-1-yl)-3-thiophenesulfonamide, m.p. 190°—192°C;

N-[(4,6-dimethoxypyrimidin-2-yl)aminocarbonyl]-2-(tetrahydro-2-oxofuran-3-yl)-3-pyridinesulfonamide;

N-[(4-methoxy-6-methyltriazin-2-yl)aminocarbonyl]-2-(tetrahydro-2-oxofuran-3-yl)-3-pyridinesulfonamide; and

N-[(4,6-dimethoxypyrimidin-2-yl)aminocarbonyl]-2-(4-thiomorpholinyl)-3-pyridinesulfonamide, S,S-dioxide, m.p. 204°—205°C.

## Detailed Description of the Invention

### Synthesis

The following equations are illustrated for the synthesis of compounds of Formula I when J is J—11 but can also be used to synthesis J—5 through J—10 in analogous manners by one skilled in the art, taking into account the chemical compatibility with the reaction conditions of Equations 1—81.

Compounds of Formula I can be synthesized by one or more of the methods shown below in Equations 1, 2 and 3. Reagents and reaction conditions are given by way of illustration only.

Equation 1 depicts the reaction of sulfonyl isocyanates and isothiocyanates II with the appropriate heterocyclic amines of Formula III to give the desired sulfonylureas I.

Equation 1

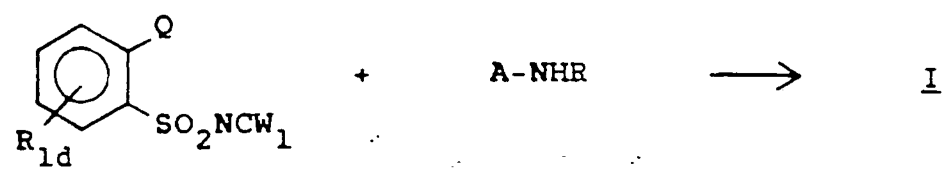

II    III

wherein

$R_{1d}$, Q, R, $W_1$ and A are as previously defined.

The reaction of Equation 1 is best carried out in an inert aprotic solvent such as methylene chloride, tetrahydrofuran or acetonitrile at a temperature between 0° and 82°C. A catalytic amount of 1,4-diazabicyclo[2.2.2]octane (DABCO) may be used to accelerate the reaction. In the cases in which the products are insoluble in the reaction solvent, they may be isolated by simple filtration. When the products are soluble, they can be isolated by evaporation of the solvent and trituration of the residue with solvents such as 1-chlorobutane, diethyl ether or ethyl acetate, and filtration.

Compounds of Formula Ia where $W_1$ is O can also be prepared as shown below in Equation 2 by treating sulfonamides of Formula IV with the methyl ester of a pyrimidine or triazine carbamic acid of Formula V in the presence of an equimolar quantity of trimethyl aluminum.

Equation 2

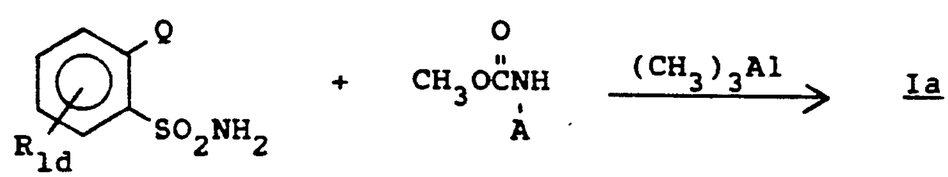


IV    V

wherein

$R_{1d}$, Q, H and A are as previously defined and $W_1$ is O.

The reaction of Equation 2 is best carried out at temperatures between 25° and 83°C in a solvent such as

methylene chloride or 1,2-dichloroethane for 12 to 96 hours under an inert atmosphere, as taught in European Patent Application (EP—A) No. 83,975 (published July 20, 1983). The products of Formula Ia are conveniently isolated by acidifying the reaction solution with dilute aqueous hydrochloric acid, and extraction with a suitable solvent such as methylene chloride or ethyl acetate. If necessary, purification can be achieved by recrystallization or column chromatography. The methyl carbamates V can be synthesized by treatment of the corresponding heterocyclic amines of Formula III with dimethyl carbonate or methyl chloroformate in the presence of a base such as sodium hydride or pyridine.

Alternatively, compounds of Formula Ia can be prepared as shown below in Equation 3 by the reaction of sulfonamides IV with the phenyl ester of the appropriate carbamic acid, VI, in the presence of an equimolar quantity of a tertiary amine base such as 1,8-diazabicyclo[5.4.0]undec-7-ene (DBU).

Equation 3

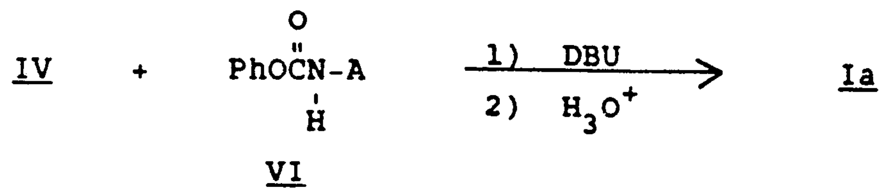

IV VI Ia

wherein

$R_{1d}$, Q, H and A are as previously defined and $W_1$ is O.

The reaction shown in Equation 3 is best carried out at about 25°C in a suitable solvent such as dioxane or acetonitrile for 1—2 hours under an inert atmosphere as described in European Patent Application No. 70,804 (published January 26, 1983). The desired products of Formula Ia can be conveniently isolated by acidifying the reaction solution with dilute aqueous hydrochloric acid. In certain cases, the products are insoluble and may be filtered. Alternatively, the aqueous layer can be extracted with a solvent such as methylene chloride or ethyl acetate. Drying and evaporation of the solvent then affords the desired products. The phenyl carbamates VI can be synthesized by treatment of the corresponding heterocyclic amines of Formula III with diphenyl carbonate or phenyl chloroformate in the presence of a base such as sodium hydride, pyridine, or potassium carbonate with a catalytic amount of 4-dimethylaminopyridine. The mixture is stirred at temperatures between 25° and 65°C in a suitable solvent such as tetrahydrofuran for 12—36 hours.

A judicious choice of the appropriate methods for preparing compounds of Formulas I and Ia must take into account the nature of the substituents Q and $R_{1d}$, and their chemical compatibility with the reaction conditions of Equations 1—3.

Sulfonyl isocyanates of Formula IIa can be prepared as shown in Equation 4 by the reaction of sulfonamides of general structure IV with phosgene in the presence of *n*-butyl isocyanate and a catalytic amount of 1,4-diazabicyclo[2.2.2]octane (DABCO).

Equation 4

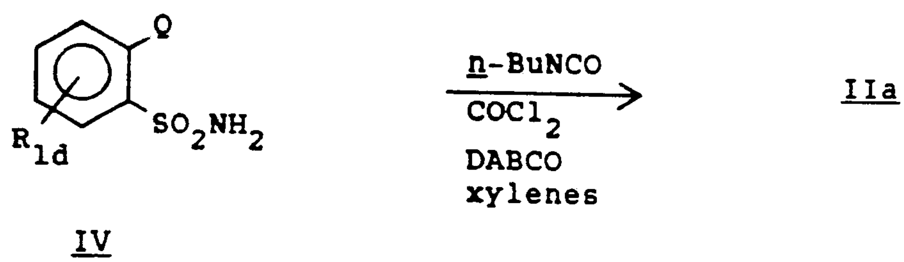

IV IIa

wherein

Q and $R_{1d}$ are as previously defined and $W_1$ is O.

The reaction shown in Equation 4 is best carried out according to the procedure described in United States Patent 4,238,621.

Alternatively, sulfonyl isocyanates IIa can be prepared via phosgenation of the preformed *n*-butylureas of Formula VII as represented in Equation 5.

Equation 5

IV

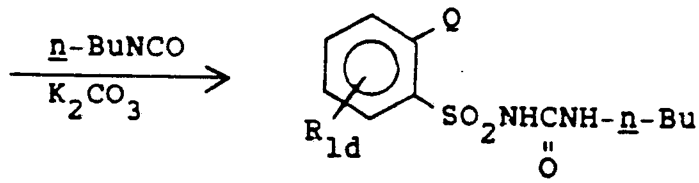


VII

VII

$COCl_2$
DABCO
xylenes

IIa

wherein

Q and $R_{1d}$ are as previously defined and $W_1$ is O.

The compounds of Formula VII are conveniently prepared by stirring a mixture of the appropriate sulfonamide IV, anhydrous potassium carbonate, and *n*-butyl isocyanate in a suitable solvent such as acetone or methyl ethyl ketone at 25° to 80°C until all of the isocyanate has reacted. The products are isolated by quenching in dilute aqueous hydrochloric acid and recrystallizing the insoluble solid. The *n*-butylureas VII are then treated with phosgene and a catalytic amount of DABCO in refluxing xylenes or chlorobenzene in a manner analogous to that described in the reference cited for Equation 4.

Another, somewhat milder, method for the preparation of sulfonyl isocyanates IIa is shown in Equation 6. Treatment of sulfonamides of Formula IV with thionyl chloride gives intermediate N-sulfinylsulfonamides VIII, which afford sulfonyl isocyanates II upon exposure to phosgene in the presence of a catalytic amount of pyridine.

Equation 6

IV

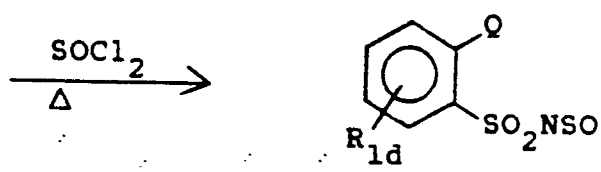


VIII

VIII

$COCl_2$
cat. pyridine
toluene
Δ

IIa

wherein

Q and $R_{1d}$ are as previously defined and $W_1$ is O.

The reaction of Equation 6 can best be performed according to the procedure of H. Ulrich B. Tucker and A. Sayigh, *J. Org. Chem., 34,* 3200 (1969).

Sulfonyl isothiocyanates of Formula II, where $W_1$ is S, can be prepared by treatment of sulfonamides of Formula IV with carbon disulfide and potassium hydroxide followed by reaction of the dipotassium salt with phosgene according to K. Hartke, *Arch. Pharm.,* 229, 174 (1966).

A judicious choice of the appropriate method for preparing compounds of Formula IIa must take into account the nature of the substituents Q and $R_{1d}$, and their chemical compatibility with the reaction conditions of Equations 4—6.

The requisite sulfonamides of Formula IV can be synthesized by one or more of the methods shown below in Equations 7, 8 and 9.

Equation 7 depicts the reaction of sulfonyl chlorides of Formula IX with ammonia to give sulfonamides of Formula IVa.

Equation 7

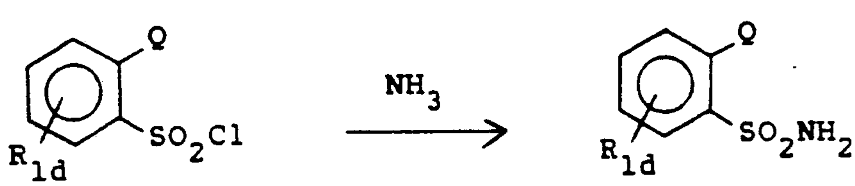


IX

IVa

wherein

Q and $R_{1d}$ are as previously defined.

The amination of Equation 7 is conveniently effected by adding at least two molar equivalents of either anhydrous ammonia or concentrated ammonium hydroxide to a solution of the sulfonyl chloride IX in a suitable solvent such as diethyl ether, tetrahydrofuran, or methylene chloride at temperatures between −30° and 25°C. The desired sulfonamides of Formula IVa are isolated either by filtration, in which case the by-product ammonium chloride is removed by washing with water, or extraction into a suitable organic solvent such as methylene chloride or ethyl acetate. Drying and evaporation of the solvent then affords the products IVa, which are usually sufficiently pure to be carried directly on to the next step.

Sulfonamides of Formula IVb can be prepared as shown in Equation 8 by treatment of the corresponding N-*t*-butylsulfonamides X with an appropriate acid such as trifluoroacetic (TFA), polyphosphoric (PPA), or p-toluenesulfonic acid (p-TSA).

Equation 8

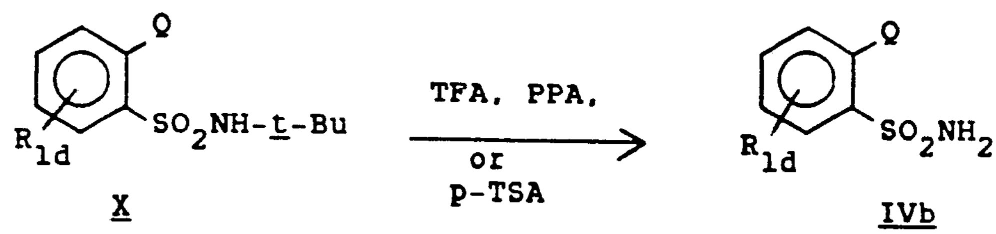


X IVb

wherein

Q and $R_{1d}$ are as previously defined.

The reaction of Equation 8 is conveniently carried out by stirring a solution of the compound of Formula X in excess trifluoroacetic acid (approximately 0.3 M) at about 25°C for 1—24 hours. The desired sulfonamides of Formula IVb are then isolated by removal of the volatiles *in vacuo* and crystallization from a suitable solvent such as diethyl ether, 1-chlorobutane, or ethyl acetate. Alternatively, the N-*t*-butylsulfonamides of Formula X can be treated with a catalytic amount of p-toluenesulfonic acid monohydrate in a solvent such as toluene or xylenes at reflux temperature for 1—6 hours. The desired products are then isolated in a manner analogous to the one described above. For use of polyphosphoric acid in the deprotection of N-*t*-butylsulfonamides, see J. G. Lombardino, *J. Org. Chem., 36,* 1843 (1971); for use of trifluoroacetic acid, see J. D. Catt and W. L. Matier, *J. Org. Chem., 39,* 566 (1974).

Alternatively, sulfonamides of Formula IVc, where Q is Q—1, can be synthesized via the two-step procedure represented below in Equation 9(a) starting from the 1-2-benzothiazin 1,1-dioxides of Formula XI. In a similar fashion, sulfonamides of Formula IVd, where Q is Q—51, can be prepared via the two-step procedure shown in Equation 9(b) starting from the same compounds XI.

Equation 9

(a)

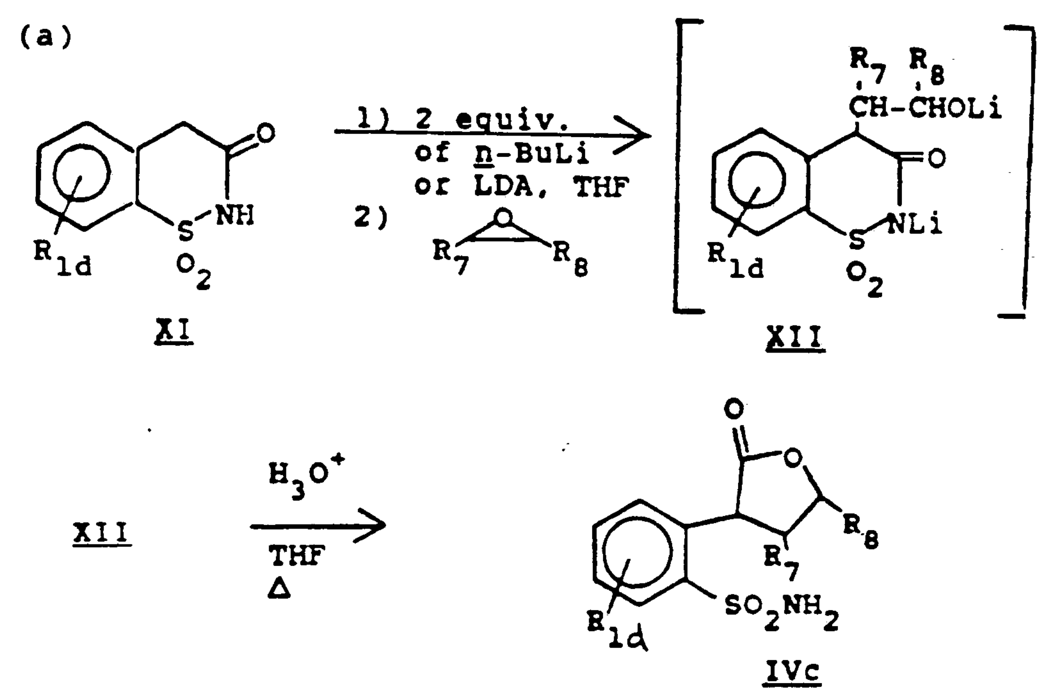


XI XII IVc

(b)

XI 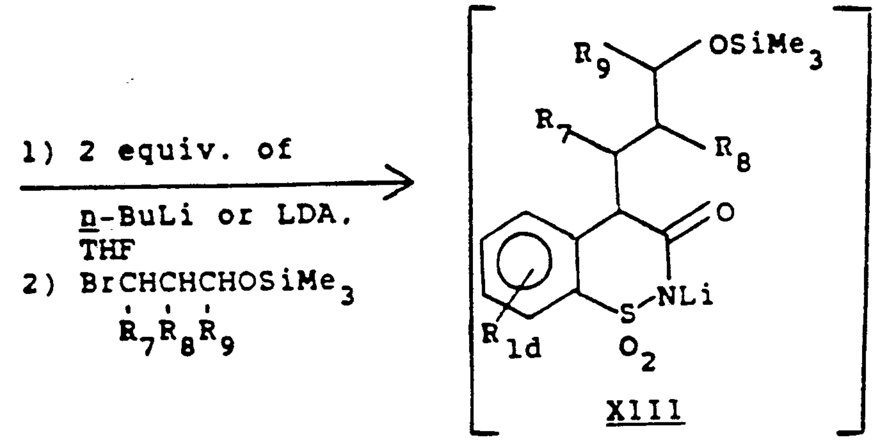


XIII 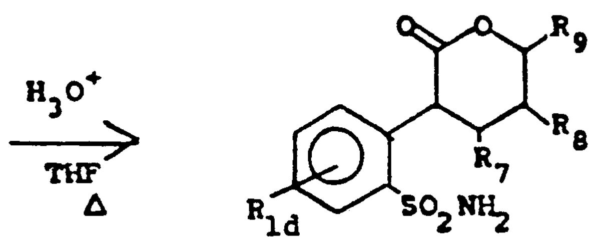


IVd

wherein

$R_{1d}$, $R_7$, $R_8$, and $R_9$ are as previously defined.

Equation 9(a)

The transformation shown above in Equation 9(a) can be conveniently carried out by adding a suitable base such as *n*-butyllithium (*n*-BuLi) or lithium diisopropylamide (LDA) to a solution of the compound of general structure XI in a solvent such as tetrahydrofuran at −78°C under an inert atmosphere. To ensure complete dianion formation, the reaction mixture is typically allowed to warm to about −30°C over a period of 0.5 to 1 hour, and is then recooled and treated with the appropriate epoxide. After being stirred overnight at −78°C to 25°C, the reaction solution is acidified with dilute aqueous hydrochloric acid and the water layer extracted with a suitable solvent such as diethyl ether or methylene chloride. Drying and evaporation of the organic extracts affords a crude residue which is immediately dissolved in an organic solvent such as tetrahydrofuran, and heated at reflux temperature in the presence of a mineral acid such as hydrochloric acid for 1 to 4 hours. The desired products of Formula IVc are isolated by extraction into an organic solvent such as diethyl ether or methylene chloride. Drying and evaporation of the organic extracts affords the crude sulfonamides IVc, which are typically purified by silica gel chromatography; elution is achieved with an appropriate solvent system such as 40—80% ethyl acetate-hexanes containing 1% methanol.

Equation 9(b)

The transformation depicted in Equation 9(b) is carried out in a manner analogous to the one described for Equation 9(a), except that a protected bromoalkanol is employed in the reaction with the dianions of 1,2-benzothiazin 1,1-dioxides XI to give intermediates of Formula XIII. These compounds are then treated with aqueous acid as described above to afford the desired sulfonamides of Formula IVd, which can be purified by column chromatography if necessary.

The 1,2-benzothiazin 1,1-dioxides XI can be prepared as shown below in Equation 10 by treatment of sulfonamides of Formula XIV with aqueous sodium hydroxide.

Equation 10

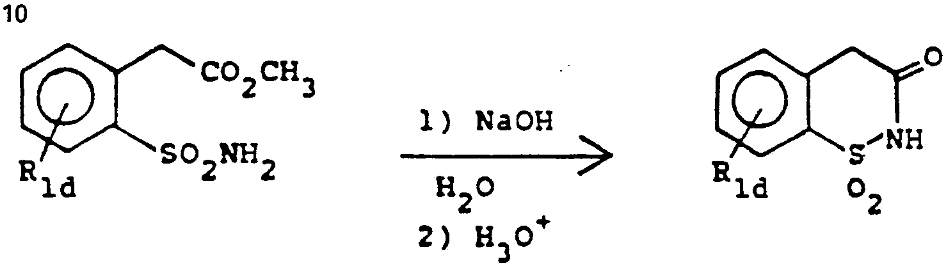


XIV XI

wherein

$R_{1d}$ is as previously defined.

The ring-closure reaction shown in Equation 10 can be effected by stirring a solution of the compound of Formula XIV in excess 10% aqueous sodium hydroxide at about 25°C for 1—12 hours. The products of Formula XI are then isolated by acidifying the cooled (0°—10°C) reaction mixture with concentrated hydrochloric acid, and filtration. These compounds are generally sufficiently pure to be carried directly on to the next step.

The requisite sulfonamides of Formula XIV are known in the art and can be synthesized by methods taught in European Patent Application No. 44,209 (published January 20, 1982).

Sulfonyl chlorides of Formula IX can be prepared by one or more of the methods shown below in Equations 11, 12 and 13.

Equation 11 depicts the diazotization of appropriately substituted aniline derivations of Formula XV and subsequent coupling with sulfur dioxide in the presence of either cupric or cuprous chloride to give the desired products of Formula IX.

Equation 11

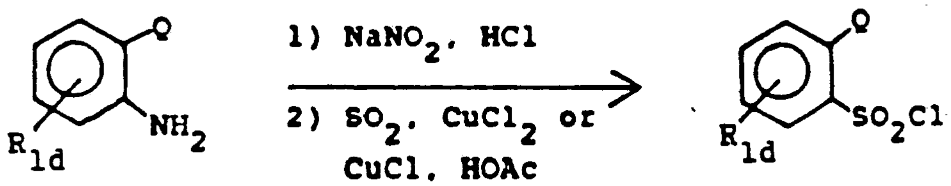


XV　　IX

wherein

Q and $R_{1d}$ are as previously defined.

The reaction of Equation 11 is accomplished by treating a solution of the substituted aniline XV in concentrated hydrochloric acid with a solution of sodium nitrite in water at −5° to 5°C. After being stirred for 10—30 minutes at about 0°C, the solution is added to a mixture of excess sulfur dioxide and a catalytic amount of cupric chloride or cuprous chloride in glacial acetic acid at about 10°C. The temperature is maintained at about 10°C for 1/4—1 hour, then raised to 25°C and stirred for 2—24 hours. This solution is then poured into a large excess of ice-water. The desired sulfonyl chlorides IX can be isolated by filtration, or by extraction into a solvent such as diethyl ether or methylene chloride, followed by drying and evaporation of the solvent.

Sulfonyl chlorides of Formula IX can also be prepared as shown below in Equation 12 by metal-halogen exchange of appropriately substituted aryl bromides XVIa, where D is Br, and trapping with sulfuryl chloride.

Equation 12

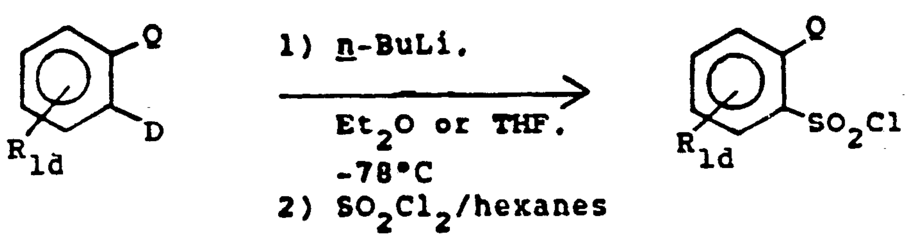


XVIa　　IX

wherein

Q and $R_{1d}$ are as previously defined, and D is Br.

The lithiation shown in Equation 12 can be carried out according to the procedure of S. H. Bhattacharya, *et al., J. Chem. Soc (C).* 1265 (1968).

Alternatively, compounds of Formula IX can be prepared via oxidative chlorination of the appropriate thioethers of Formula XVIb, where D is $SR_{12}$, as represented in Equation 13.

Equation 13

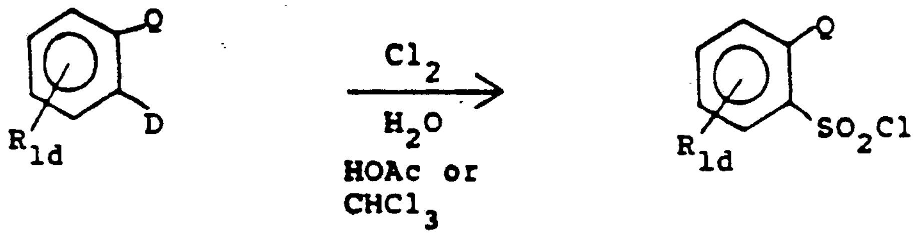


XVIb IX

wherein

Q and $R_{1d}$ are as previously defined, D is $SR_{12}$ and $R_{12}$ is $C_2$—$C_4$ alkyl or benzyl.

The reaction of Equation 13 can be accomplished by treating a solution of the thioether XVIb in a suitable solvent such as chloroform or methylene chloride; in some cases, it is advantageous to use acetic acid as solvent. The reaction is carried out in the presence of at least 2.5 equivalents of water and at least 3 molar equivalents of chlorine at 0°—30°C for 1 to 5 hours. The products can be isolated by removal of the solvent *in vacuo* and are generally sufficiently pure to be carried directly on to the next step.

The requisite aniline derivatives of Formula XV can be prepared in a straightforward manner by reduction of the corresponding nitro compounds of Formula XVIc, where D is $NO_2$, as shown in Equation 13a.

Equation 13a

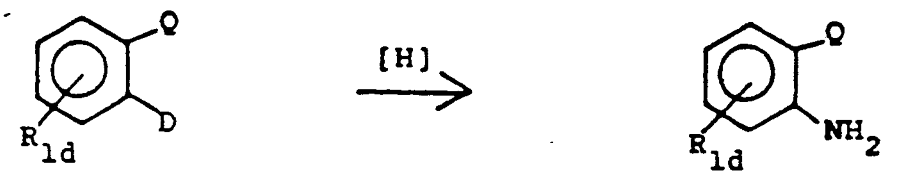


XVIc XV

wherein

Q and $R_{1d}$ are as previously defined, and D is $NO_2$.

A wide variety of methods exists for effecting the reduction of aromatic nitro groups to the corresponding aniline derivatives. One of the more common procedures involves treating the nitro compounds of Formula XVIc with a slight excess of stannous chloride dihydrate in concentrated hydrochloric acid at temperatures between 25°C and 80°C. Alternatively reduction can be accomplished with iron powder in glacial acetic acid as described by Hazlet and Dornfeld, *J. AM. Chem Soc., 66,* 1781 (1944), and by West, *J. Chem. Soc., 127,* 494 (1925). For a general review, see Groggins in "Unit Processes in Organic Synthesis", McGraw-Hill Book Co., New York, 1947, pp 73—128.

A judicious choice of the appropriate method for preparing compounds of Formula IX must take into account the nature of the substituents Q and $R_{1d}$, and their chemical compatibility with the reaction conditions of Equations 11—13a.

Sulfonamides of Formulas XVIIa and XVIIb can be prepared as shown in Equations 14(a) and 14(b) by hydrogenolysis of the benzyl ethers XVIIIa and XVIIIb, followed by lactonization under acidic conditions.

Equation 14

(a)

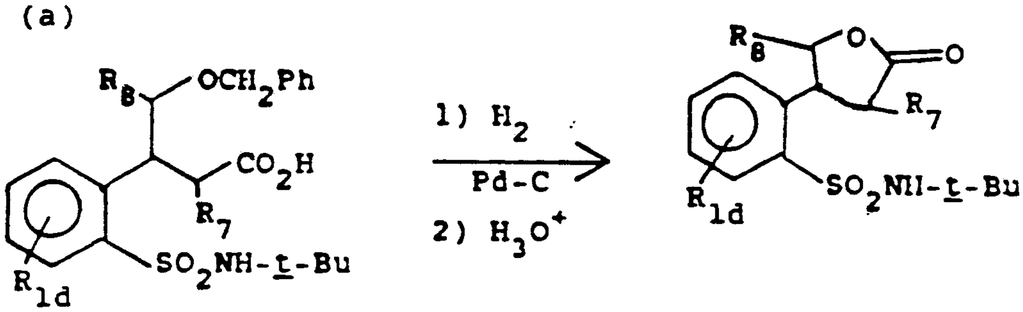


XVIIIa XVIIa

(b)

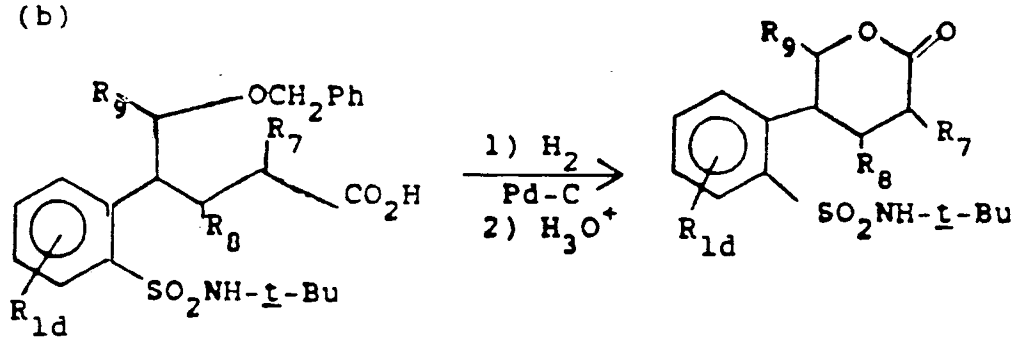


XVIIIb XVIIb

wherein

$R_{1d}$, $R_7$, $R_8$ and $R_9$ are as previously defined.

The hydrogenolysis of benzyl ethers to generate alcohols as shown in the first step of Equations 14(a) and 14(b) is well precedented in the literature and can be accomplished by subjecting compounds of Formulas XVIIIa and XVIIIb to a hydrogen atmosphere in the presence of a suitable catalyst such as palladium-on-carbon. For relevant references, see C. H. Heathcock and R. Ratcliffe, *J. Am. Chem. Soc., 93,* 1746 (1971), and A. M. Felix, *et al., J. Org., Chem., 43,* 4194 (1978). The second step represented above in Equations 14(a) and 14(b) involves the formation of 5- or 6-membered ring lactones from the corresponding 5- or 6-hydroxy carboxylic acids, an extremely facile cyclization which often occurs spontaneously. This lactonization process can be aided by heating the hydroxy acids in the presence of a suitable acid such as hydrochloric or sulfuric acid. For a discussion of this reaction and useful references, see J. March, "Advanced Organic Chemistry", 2nd Ed., McGraw-Hill Book Co., New York, 1977, pp. 363—365.

Alternatively, sulfonamides of Formula XVIIa can be synthesized via iodolactonization of the appropriate unsaturated carboxylic acids of Formula XIX, followed by reductive cleavage of the carbon-iodine bond as shown in Equation 15.

Equation 15

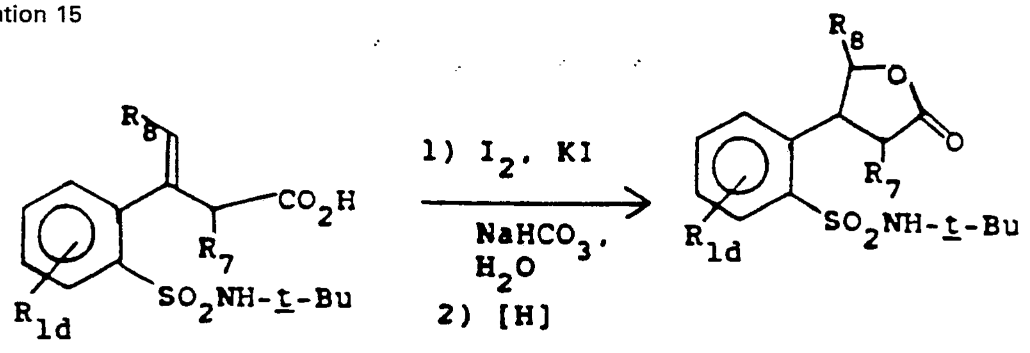


XIX XVIIa

wherein

$R_{1d}$, $R_7$ and $R_8$ are as previously defined.

The first step of the reaction of Equation 15 can be accomplished according to the procedure of J. Klein, *J. Am. Chem. Soc., 81,* 3611 (1959). The intermediate iodolactones obtained from this reaction are then treated with suitable reducing agent such as hydrogen over Raney nickel catalyst (see reference cited above) or zinc in acetic acid as described by C. Heathcock, *et al., J. Am. Chem. Soc., 92,* 1326 (1970).

Sulfonamides of Formula XVIIc can be conveniently prepared as shown below in Equation 16, by a two-step procedure analogous to that shown in Equations 14(a) and 14(b) except that the starting carboxylic acids are those of Formula XVIIIc.

Equation 16

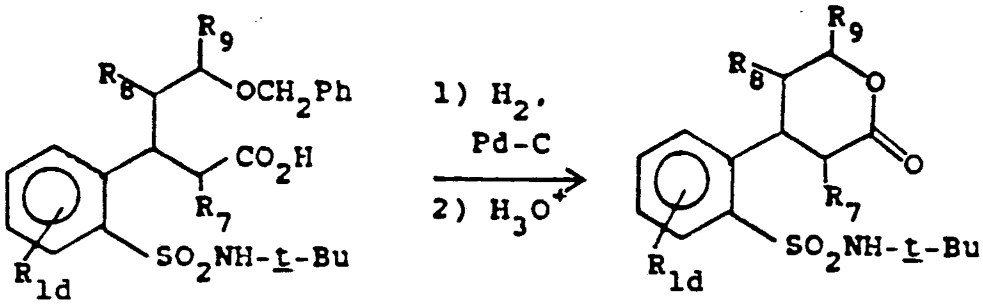


XVIIIc XVIIc

wherein $R_{1d}$, $R_7$, $R_8$ and $R_9$ are as previously defined.

The transformation shown above in Equation 16 can be effected in a manner identical to that described for Equations 14(a) and 14(b).

Sulfonamides of Formulas XVIId and XVIIe can be synthesized by the three-step sequence of reactions outlined below in Equations 17(a) and 17(b) which involves: (1) addition of the dianions of suitable N-*t*-butyl benzenesulfonamides of Formula XX to the appropriate β- or γ-formyl esters to give hydroxy esters XXIa and XXIb, (2) saponification of the esters XXIa and XXIb to afford the corresponding γ- or δ-hydroxy carboxylic acids, and (3) acid-induced lactonization.

Equation 17

(a)

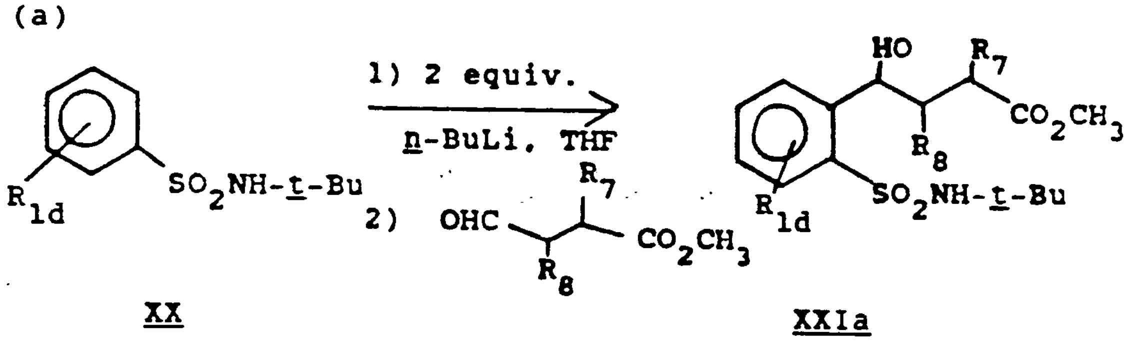


XX XXIa

XXIa

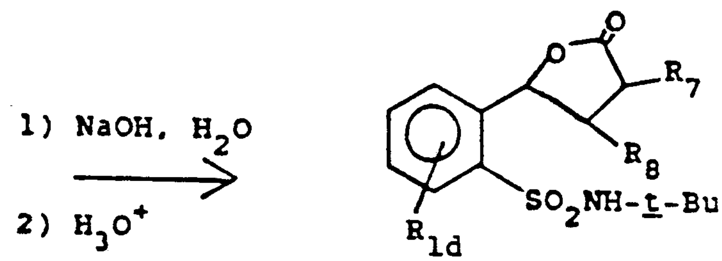


XVIId

(b)

XX

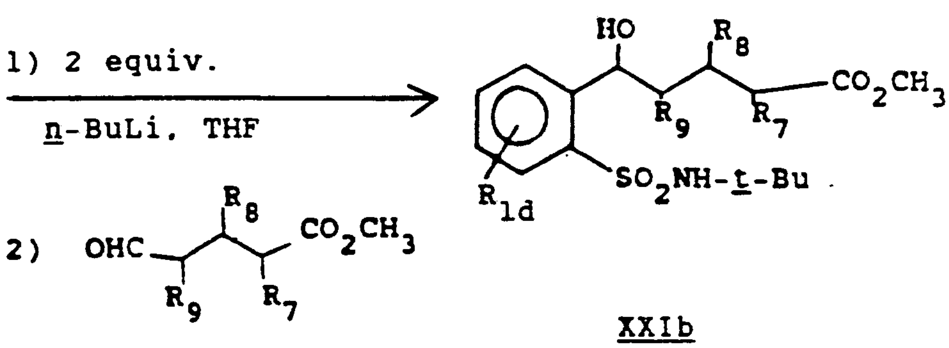


XXIb

XXIb

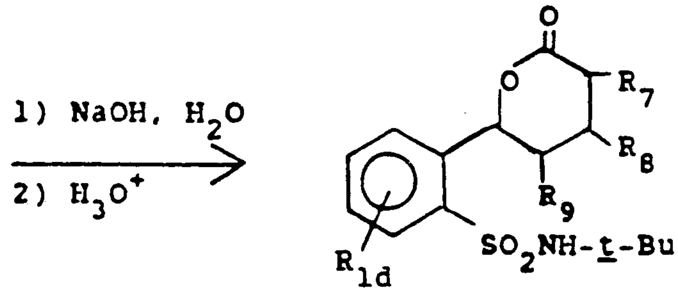


XVIIe

wherein

$R_{1d}$, $R_7$, $R_8$ and $R_9$ are as previously defined.

The first step of Equations 17(a) and 17(b) can be carried out by treating the appropriate N-*t*-butylbenzenesulfonamide XX with *n*-butyllithium in a solvent such as tetrahydrofuran at 0°—25°C according to the procedure of J. G. Lombardino, *J. Org. Chem., 36.* 1843 (1971). Addition of suitably substituted β- or γ-formyl esters to these dianions affords the hydroxy esters of Formulas XXIa and XXIb. Saponification of these hydroxy esters can be most easily accomplished by treatment with excess aqueous sodium hydroxide solution at about 25°C for 1—6 hours. The desired products are obtained by acidifying with concentrated hydrochloric acid (ice-water cooling) and either filtration or extraction into a suitable organic solvent such as methylene chloride, diethyl ether, or ethyl acetate. These γ- and δ-hydroxy carboxylic acids may then spontaneously cyclize to give the desired products of Formulas XVIId and XVIIe; if not, lactonization may be achieved in the manner identical to that described for Equation 14 above.

Alternatively, sulfonamides of Formula XVIId can be prepared by iodolactonization of the appropriate unsaturated carboxylic acids of Formula XXII, followed by reductive cleavage of the carbon-iodine bond as shown in in Equation 18.

Equation 18

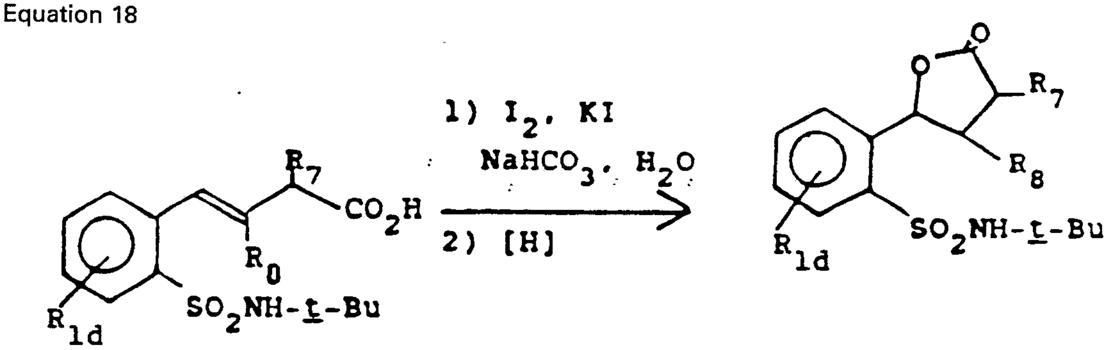


XXII

XVIId

wherein

$R_{1d}$, $R_7$ and $R_8$ are as previously defined.

The transformation shown in Equation 18 above can be achieved in a manner identical to that described for Equation 15.

Sulfonamides of Formula XVIIe can also be synthesized by a Baeyer-Villiger reaction on the appropriately substituted cyclopentanones XXIII as depicted in Equation 19.

Equation 19

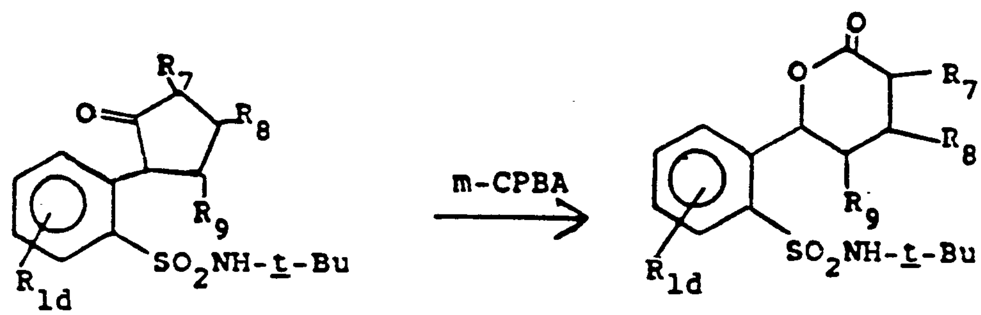


XXIII

XVIIe

wherein

$R_{1d}$, $R_7$, $R_8$ and $R_9$ are as previously defined.

The oxidation shown in Equation 19 can be carried out by treating a solution of the ketone XXIII in a suitable solvent such as chloroform or methylene chloride with *m*-chloroperoxybenzoic acid (m-CPBA) according to the methods described by S. L. Friess, *J. AM. Chem. Soc., 71,* 2571 (1949), and S. L. Friess and P.E Frankenburg, *ibid., 74,* 2679 (1952). For a review of the Baeyer Villiger reaction, refer to C. H. Hassall, *Org. Reactions, 9,* 73 (1957).

It should be recognized that removal of the *tert*-butyl protecting group from compounds of Formulas XVIIa—XVIIe by one of the methods described above in Equation 8 will furnish the primary sulfonamides of Formula IVb, where Q is Q—53, Q—52, Q—3 and Q—54, which can then be converted to compounds of Formula 1 with the corresponding Q substituents.

Compounds of Formula XXIV(a-g) can be prepared by treatment of the corresponding lactones IVc, XVIIa, XVIId, IVd, XVIIc, XVIIb and XVIIe, respectively, with ammonia or the appropriate primary amine, $R_3NH_2$, as shown below in Equation 20.

Equation 20

(a)

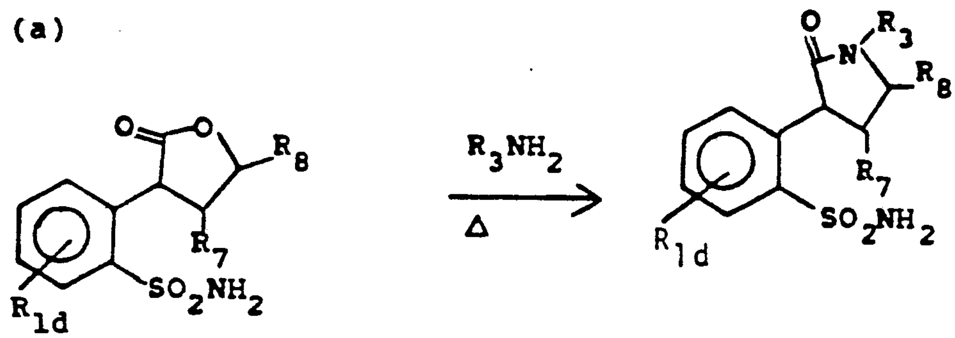


IVc XXIVa

(b)

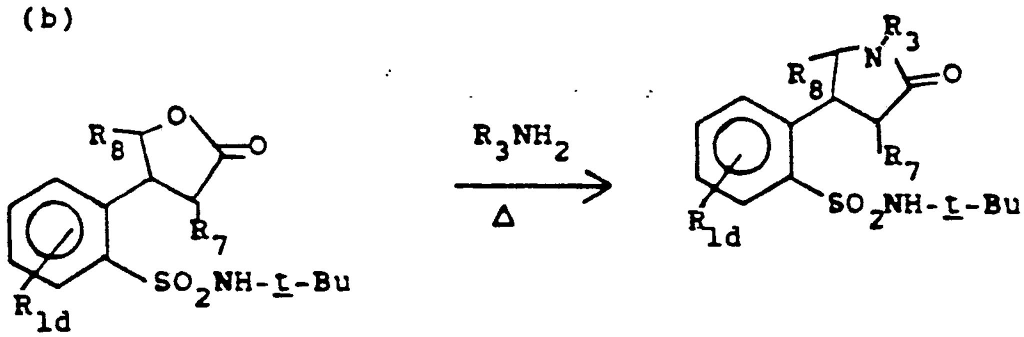


XVIIa XXIVb

(c)

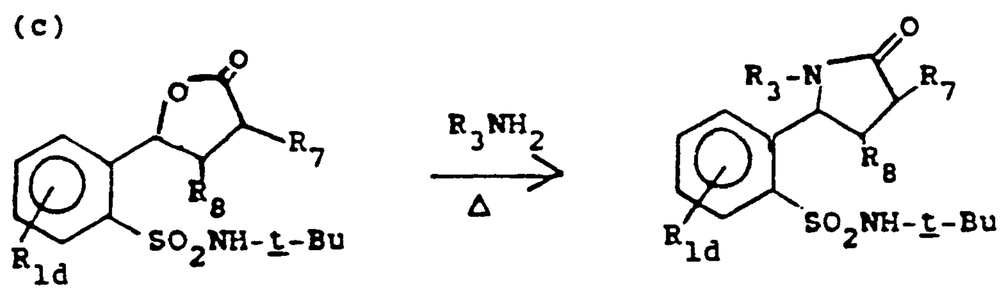


XVIId XXIVc

(d)

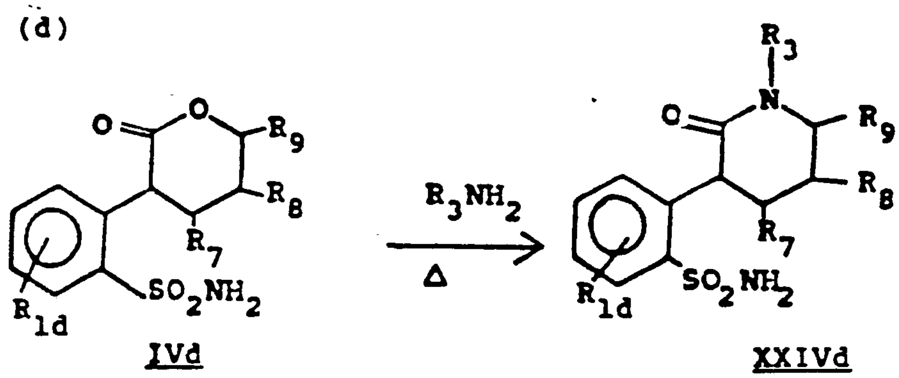


IVd XXIVd

(e)

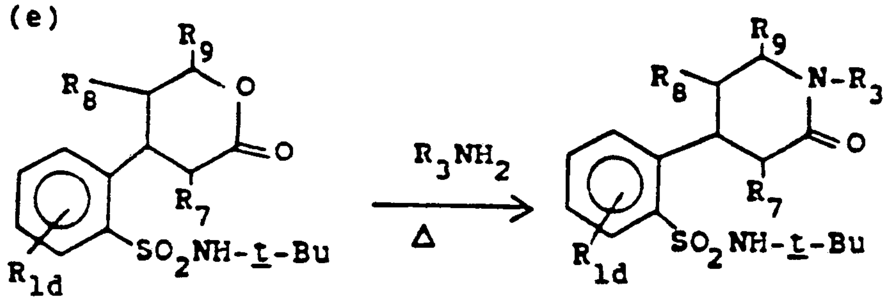


XVIIc XXIVe

(f)

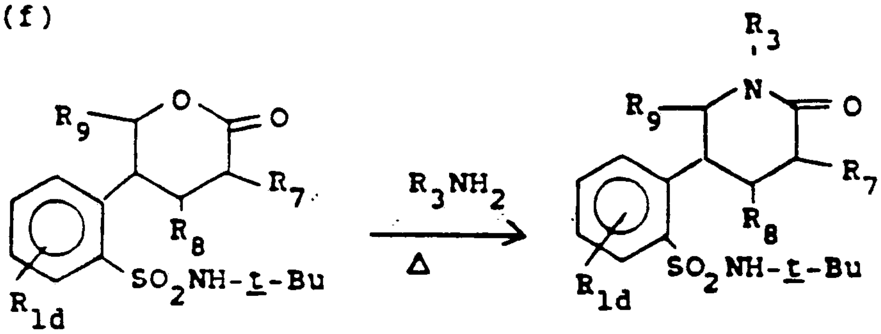


XVIIb XXIVf

(g)

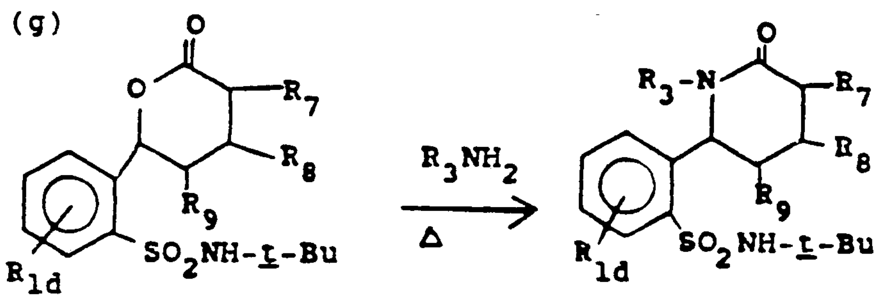


XVIIe XXIVg

wherein

$R_{1d}$, $R_3$, $R_7$, $R_8$ and $R_9$ are as previously defined.

The conversion of lactones to lactams as shown in Equations 20(a)—20(g) is a well-known process and can be effectively carried out according to the procedures of Scott and Kearse, *J. Org. Chem.*, *5* 598 (1940), and Jones, *et al.*, *J. Am. Chem. Soc.*, *48*, 181 (1926); *49*, 2528 (1927).

Subsequent treatment of N-*t*-butylsulfonamides of Formulas XXIVb, c and e-g according to one of the methods described in Equation 8 will furnish the primary sulfonamides of Formula IVb where Q is Q—5, Q—6, Q—56, Q—57 and Q—58, which can then be converted to compounds of Formula I with the corresponding substituents.

Ketones of Formulas XXIIIa and XXIIIb can be prepared via the two-step sequence of reactions shown below in Equation 22, starting from the appropriate olefins of Formulas XXVIa and XXVIb.

Equation 22

(a)

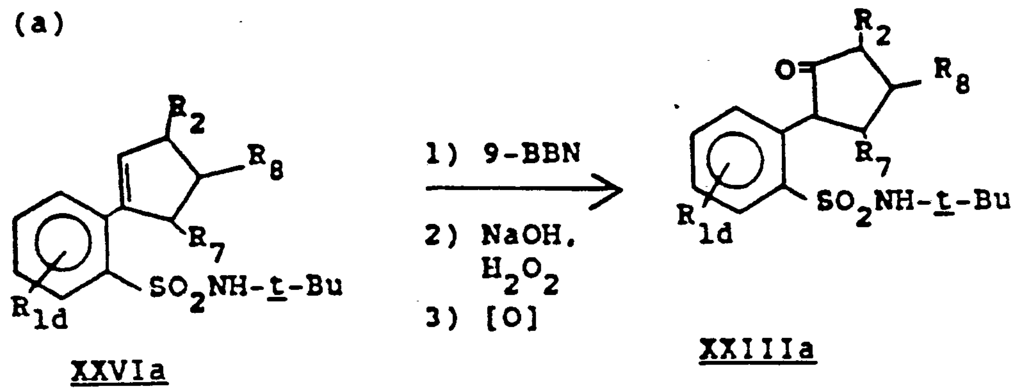


XXVIa XXIIIa

(b)

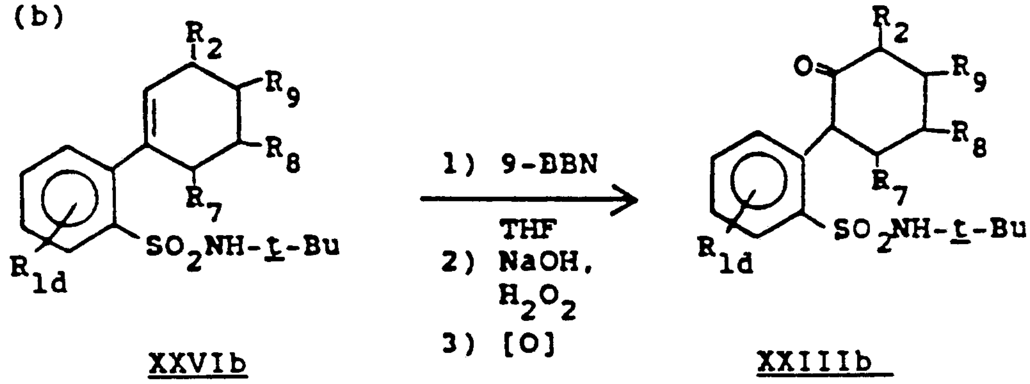


XXVIb XXIIIb

wherein

$R_{1d}$, $R_2$, $R_7$, $R_8$ and $R_9$ are as previously defined.

The first step shown in Equations 22(a) and 22(b) above involves treating olefins of Formulas XXVIa and XXVIb with 9-borabicyclo[3.3.1]nonane (9—BBN) in a suitable solvent such as tetrahydrofuran, followed by an oxidative workup with basic hydrogen peroxide to generate intermediate secondary alcohols. For details of this procedure, see E. F. Knights and H. C. Brown, *J. Am. Chem. Soc., 90,* 5280, 5281 (1968). The oxidation of these intermediate secondary alcohols to afford the desired products of Formulas XXIIIa and XXIIIb can be accomplished by any one of numerous methods; e.g., with chromium trioxide in aqueous sulfuric acid (E. R. H. Jones, *et al., J. Chem. Soc.,* 2548 (1953)), chromium trioxide-pyridine (G. I. Poos, G. E. Arth, R. E. Beyler and L. H. Sareff, *J. Am. Chem. Soc., 75,* 422 (1953)), or pyridinium chlorochromate (E. J. Corey and T. L. Suggs, *Tetrahedron Lett.,* 2647 (1975)).

The requisite olefins of Formulas XXVIa and XXVIb can be synthesized by the two-step sequence of reactions represented in Equation 23, starting from the appropriate N-*t*-butylbenzenesulfonamides of Formula XX.

Equation 23

(a)

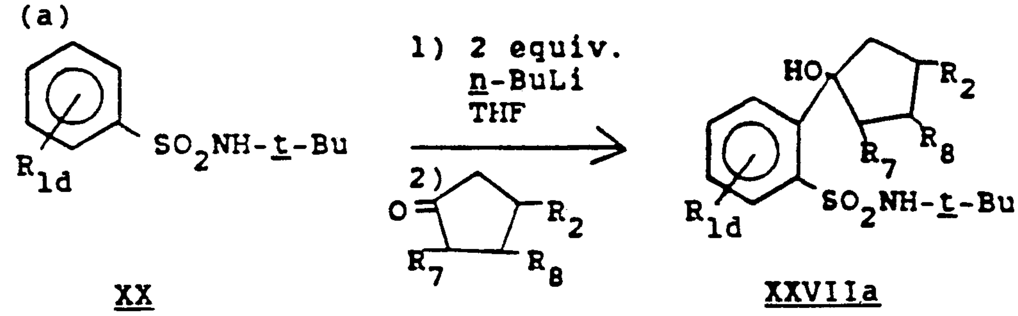


XX XXVIIa

XXVIIa
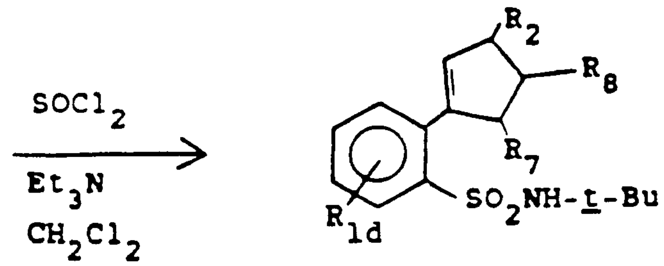


XXVIa

(b)

XX
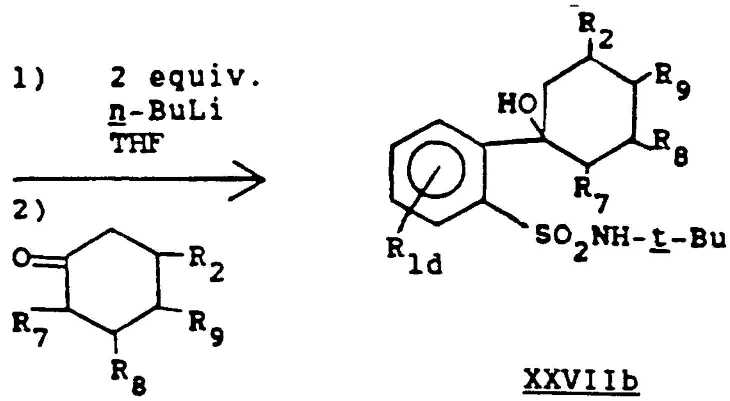


XXVIIb

XXVIIb
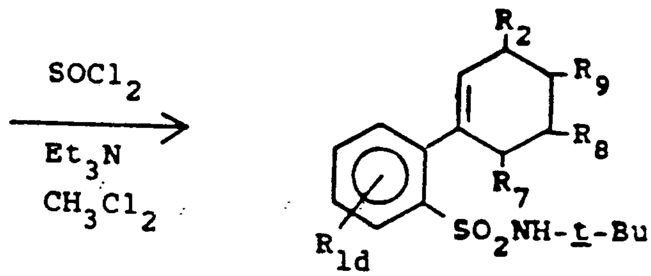


XXVIb

wherein

$R_{1d}$, $R_2$, $R_7$, $R_8$ and $R_9$ are as previously defined.

The first step of Equations 23(a) and 23(b) can be carried out as described for Equation 17. Addition of the appropriate cycloalkanone derivatives to the dianions of sulfonamides XX, and subsequent aqueous acid workup gives intermediate alcohols of Formulas XXVIIa and XXVIIb. The second step depicted in Equations 23(a) and 23(b) involves treatment of the alcohols XXVIIa and XXVIIb with thionyl chloride in the presence of a suitable acid scavenger such as triethylamine at 0°—25°C to give the olefins of Formulas XXVIa and XXVIb, respectively. For a detailed description of this standard dehydration method, refer to Linstead and Meade, *J. Chem. Soc.*, 942 (1934), or Cook and Lawrence, *J. Chem. Soc.*, 1637 (1935).

Ketones of Formulas XXIIIc and XXIIId can be conveniently synthesized by a process somewhat related to that of Equation 23, except that α,β-unsaturated cycloalkenones are employed in the reaction with dianions of N-*t*-butylbenzenesulfonamides XX instead of cycloalkanones. The result is a 1,4-addition to give the desired products of Formulas XXIIIc and XXIIId as shown below in Equation 24.

Equation 24

(a)

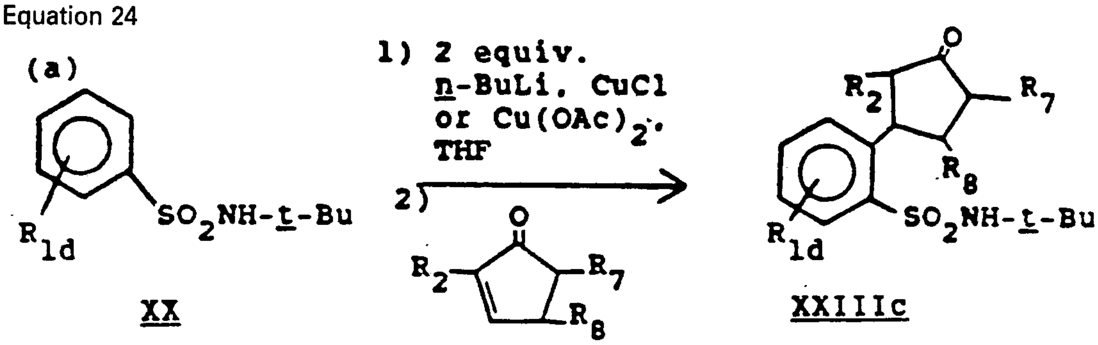


XX

XXIIIc

(b)

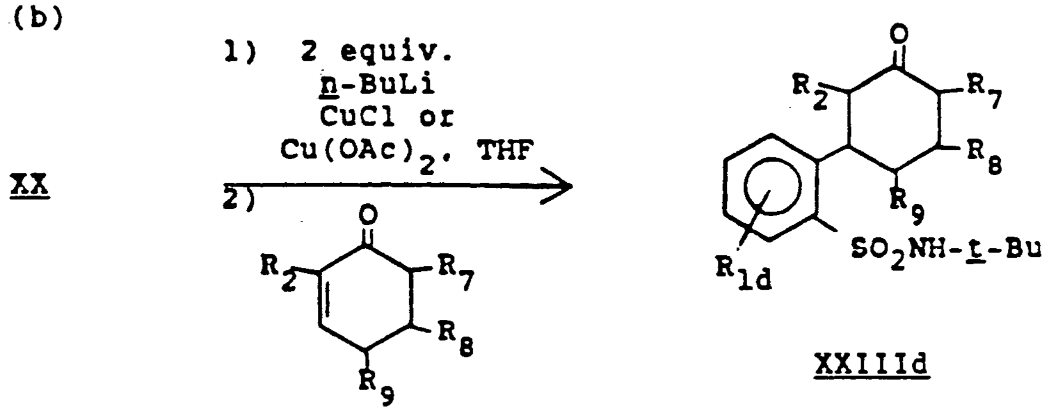


XX

XXIIId

wherein

$R_{1d}$, $R_2$, $R_7$, $R_8$ and $R_9$ are as previously defined.

The first step shown in Equations 24(a) and 24(b) involves the formation of N-*t*-butylbenzenesulfonamide dianions as described for Equation 17. However, in the case of the reactions shown above in Equation 24, a suitable copper ion catalyst such as cuprous chloride or cupric acetate is added to form aryl copper reagents, which then undergo a conjugate addition to substituted cycloalkenones to generate the desired products of Formulas XXIIIc and XXIIId after aqueous workup. Such a transformation is well precedented in the literature; for relevant examples see Gorlier, Harmon, Levisalles and Wagnon, *Chem. Comm.*, 88 (1973); Posner, *Org. Reactions, 19,* 1 (1972); or House, *Acc. Chem. Res., 9,* 59 (1976).

Ketones of Formula XXIIIe, where $R_8$ is H, can be synthesized in a straightforward manner via the three-step sequence of reactions shown in Equation 25 involving: (a) selective reduction of esters of Formula XXVIII to aldehydes of Formula XXIX, (b) base-induced aldol condensation and dehydration to give enones of Formula XXX, and (c) selective reduction of the olefinic bond of enones XXX to provide the desired products.

Equation 25

(a)

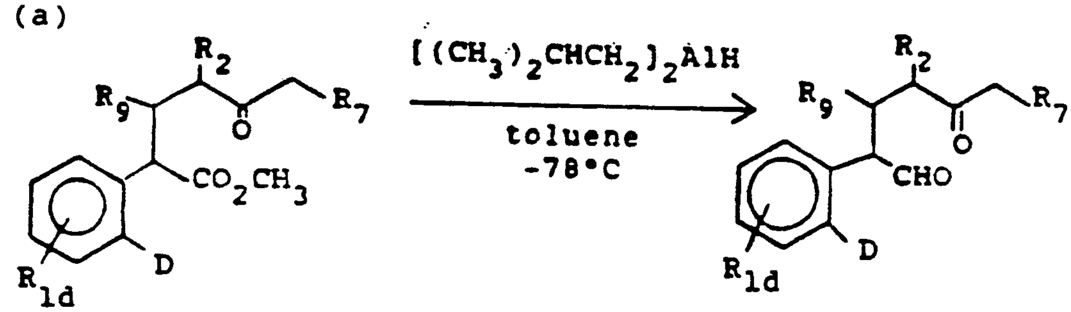


XXVIII

XXIX

(b)

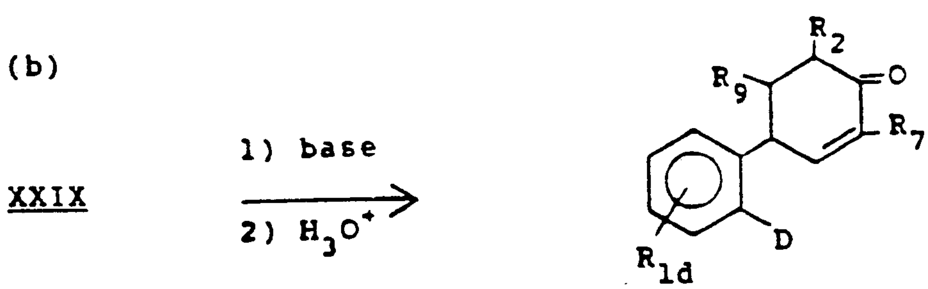


XXIX

XXX

(c)

XXX

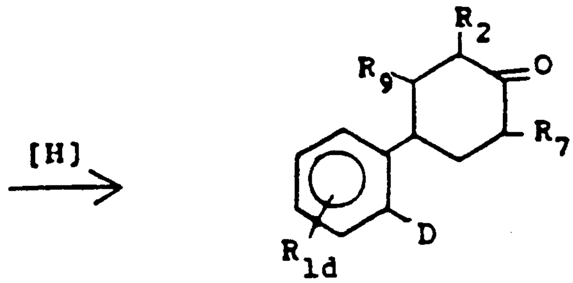


XXIIIe

wherein

$R_{1d}$, $R_2$, $R_7$ and $R_9$ are as previously defined, D is Br. $SR_{12}$, or $NO_2$, and $R_{12}$ is $C_2$—$C_4$ alkyl or benzyl.

The selective reduction of carboxylic esters, such as those of Formula XXVIII, to the corresponding aldehydes of Formula XXIX as shown in Equation 25(a) can be achieved with diisobutylaluminum hydride (DIBAL) at low temperatures as described by E. J. Corey, K. C. Nicolaou and T. Toru, *J. Am. Chem. Soc.*, *97*, 2287 (1975). The intramolecular aldol condensation depicted in Equation 25(b) is effectively carried out by treating the compounds of Formula XXIX with a catalytic amount of a suitable base such as sodium methoxide or potassium *tert*-butoxide. Subsequent aqueous acid workup results in dehydration of the intermediate aldols to give the enones of Formula XXX. Alternatively, the aldol condensation can be achieved under conditions of acid catalysis, in which case the enones XXX are obtained directly. For a comprehensive review of this well-known reaction, see A. T. Nielsen and W. J. Houlihan, *Org. Reactions*, *16*, 1 (1968). Equation 25(c) represents a selective reduction of the olefinic bond of α,β-unsaturated ketones XXX, and can be accomplished by any one of several methods. Two such methods are catalytic hydrogenation (see H. O. House, "Modern Synthetic Methods", 2nd Ed., W. A. Benjamin, Inc., Menlo Park, 1972, pp. 26—28), and dissolving metal reduction with lithium in liquid ammonia (H. O. House, *ibid*, pp. 174—176).

Ketones of Formula XXIIIf, where $R_8$ is as defined in the Summary of the Invention, can be easily synthesized by the 1,4-conjugate addition of appropriate cuprate reagents to enones of Formula XXX, as shown in Equation 26.

Equation 26

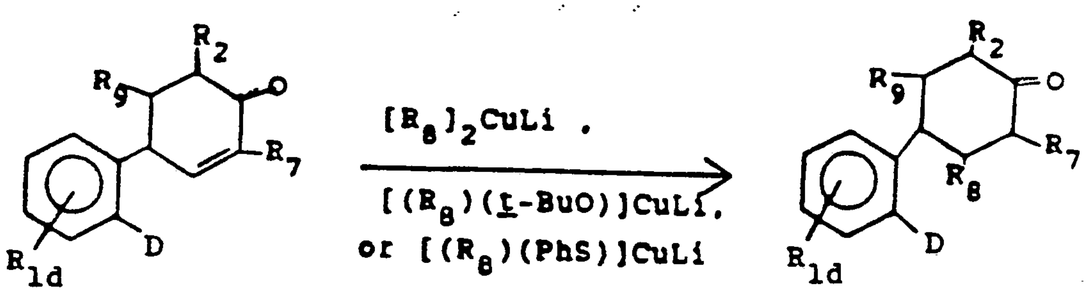


XXX XXIIIf

wherein

$R_{1d}$, $R_2$, $R_7$, $R_8$ and $R_9$ are as previously defined, D is Br, $SR_{12}$, or $NO_2$, and $R_{12}$ is $C_2$—$C_4$ alkyl or benzyl.

The 1,4-conjugate addition reaction of Equation 26 can be conveniently carried out according to the procedure described by House, Respess and Whitesides, *J. Org. Chem.*, *31*, 3128 (1966) when the copper reagent is of the form $[R_8]CuLi$. For use of "mixed" copper reagents, see Posner and Whitten, *Tetrahedron Lett.*, 1815 (1973) (for the reagent $[(R_8)(t\text{-BuO})]CuLi$), or Posner, Whitten and Sterling, *J. Am. Chem. Soc.*, *95*, 7788 (1973) (for the reagent $[(R_8)(PhS)]CuLi$).

It should be recognized that compounds of Formulas XXIIIe and XXIIIf can be treated according to one or both of the methods described in Equations 11 and 13 to afford the corresponding sulfonyl chlorides of Formula IX, where Q is Q—61. Similarly, removal of the *tert*-butyl group from compounds of Formulas XXIIIa-d by one or more of the procedures outlined in Equation 8 will furnish the primary sulfonamides of Formula IVb, where Q is Q—8, Q—60, Q—9 or Q—62, which can then be converted to compounds of Formula 1 with the corresponding Q substituents.

The requisite unsaturated carboxylic acids of Formula XXII can be conveniently synthesized by dehydration of the appropriate hydroxy esters of Formula XXIa, followed by saponification as shown in Equation 29.

Equation 29

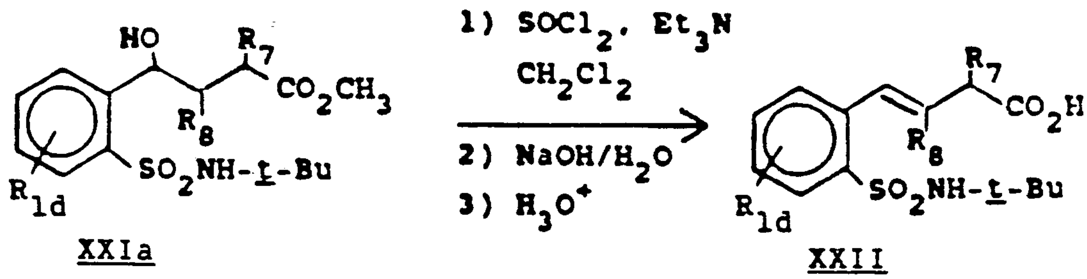


XXIa    XXII

wherein

$R_{1d}$, $R_7$ and $R_8$ are as previously defined.

The first step of Equation 29, dehydration of alcohols of Formula XXIa to give the corresponding olefins, can be carried out in a manner identical to that described for the second step in Equations 23(a) and 23(b). The saponification shown in Equation 29 (steps 2 and 3) can be accomplished as described for the second step in Equations 17(a) and 17(b).

The carboxylic esters of Formula XXVIII can be prepared by treatment of the anions derived from arylacetic esters of Formula XXXV with the appropriate α,β-unsaturated ketones as shown below in Equation 30.

Equation 30

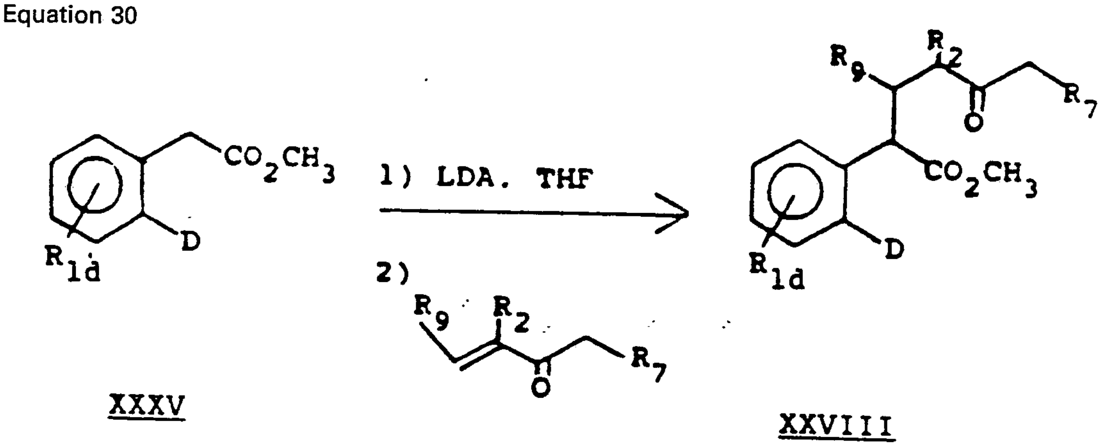


XXXV    XXVIII

wherein

$R_{1d}$, $R_2$, $R_7$ and $R_9$ are as previously defined, D is Br, $SR_{12}$, or $NO_2$, and $R_{12}$ is $C_2$—$C_4$ alkyl or benzyl.

The reaction of Equation 30 is conveniently carried out by adding a solution of the arylacetic ester XXXV in a suitable solvent such as tetrahydrofuran to a solution of a strong base such as lithium diisopropylamide (LDA) at −78° to 0°C under an inert atmosphere. The mixture is stirred at temperatures below 0°C for 0.5—1 hour to ensure complete anion formation and is then treated with an equimolar quantity of the appropriately substituted, α,β-unsaturated ketone, which is prone to undergo reaction in a 1,4-conjugate manner. For a compilation of references dealing with this type of reaction, see Bergmann, Ginsburg, and Pappo, *Org. Reactions, 10,* 179 (1959).

The requisite N-*t*-butylbenzenesulfonamides of Formulas XVIIIa, XVIIIb, XVIIIc and XIX can all be synthesized from common arylacetic esters of Formula XXXVI via a sequence of reactions that entails the same seven basic processes. Equation 31 outlines this sequence of reactions leading to compounds of Formula XVIIIa (where $R_7$ is H): (a) alkylation of the anions derived from the appropriate arylacetic esters of Formula XXXVI with aldehydes of Formula $R_8CHO$ to give β-hydroxy arylacetic esters of Formula XXXVII, (b) protection of the alcohol with a suitable protecting group such as the benzyl ether of Formula XXXVIII, (c) reduction of the esters of Formula XXXVIII to afford primary alcohols of Formula XXXIX, (d) conversion of the hyroxyl group to a good leaving group such as the alkyl bromides of Formula XL, (e) conversion of the *ortho* substituent J to N-*t*-butylsulfamoyl group by one of the methods described previously, (f) displacement of the bromides of Formula XLI to give the corresponding nitriles of Formula XLII, and (g) hydrolysis of the nitriles XLII to afford the carboxylic acids of Formula XVIIIa, where $R_7$ is H.

Equation 31

(a)

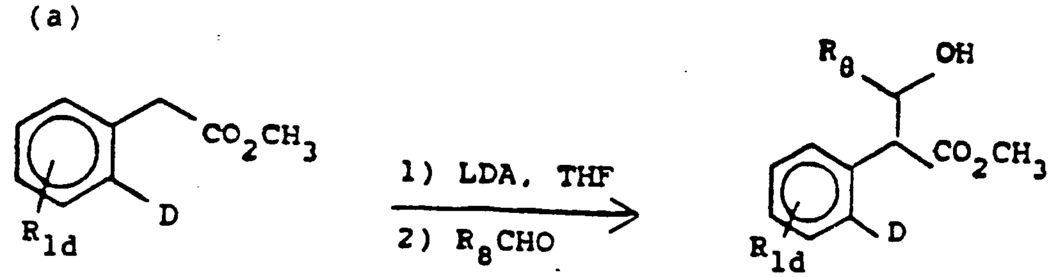


XXXVI

XXXVII

(b)

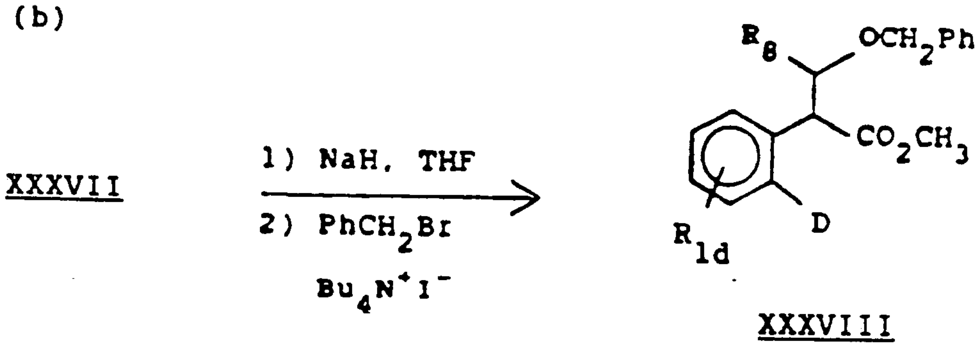


XXXVII

XXXVIII

(c)

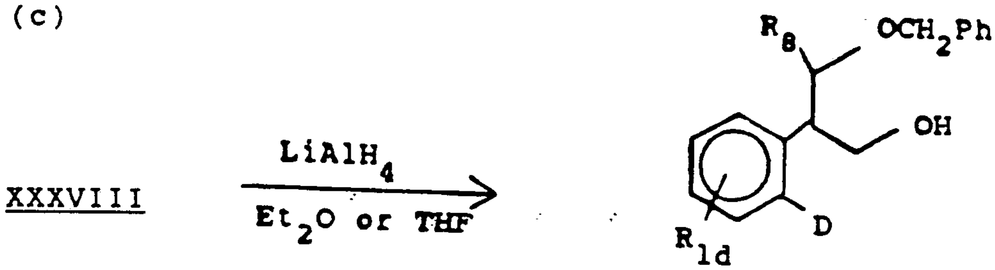


XXXVIII

XXXIX

(d)

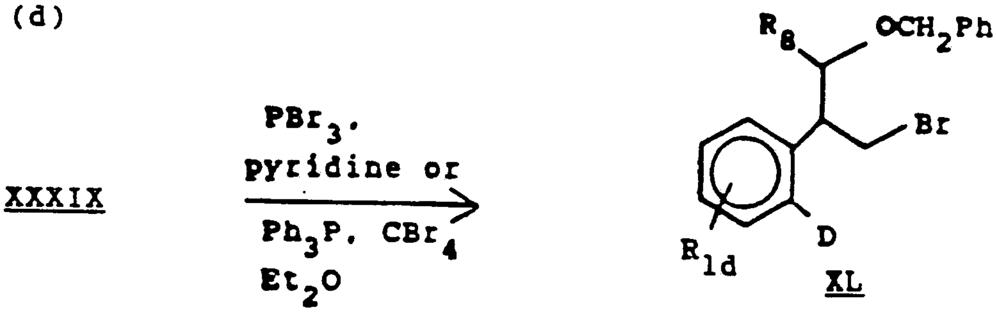


XXXIX

XL

(e)

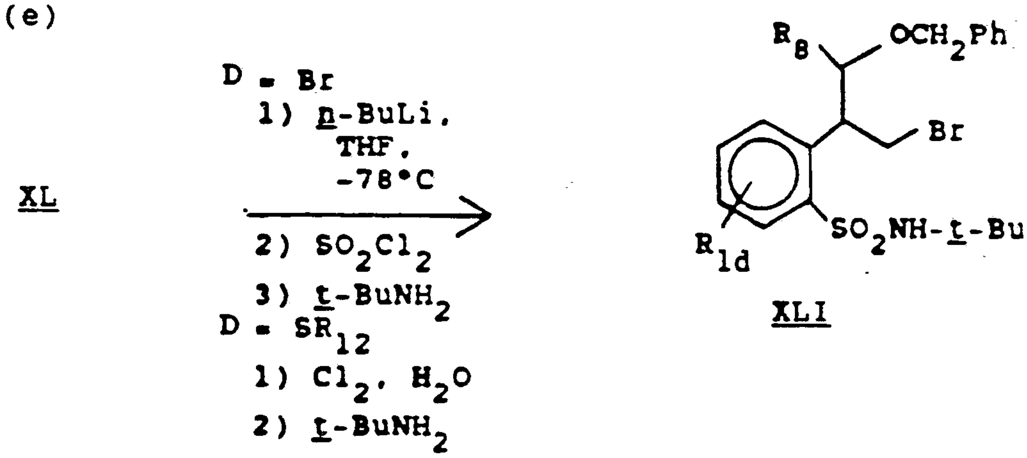


XL

XLI

(f)

XLI

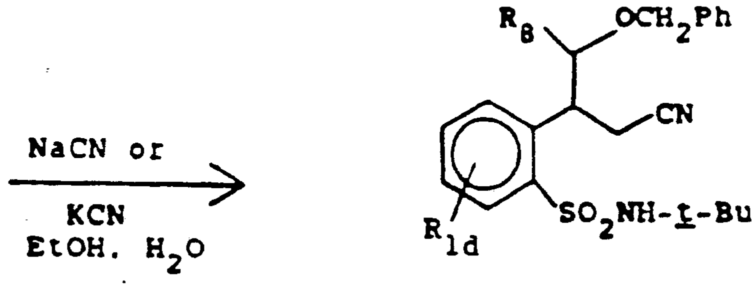


XLII

(g)

XLII

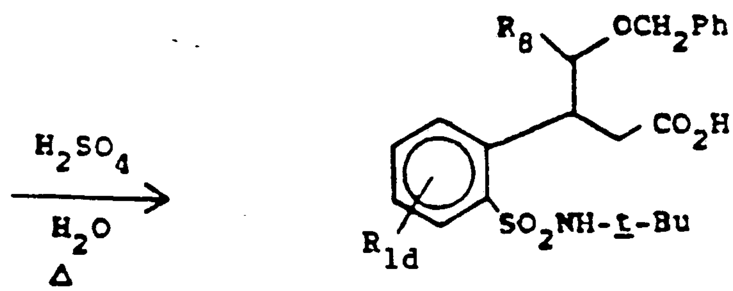


XVIIIa

wherein

$R_{1d}$ and $R_8$ are as previously defined, D is Br or $SR_{12}$, and $R_{12}$ is $C_2$—$C_4$ alkyl or benzyl.

Equation 31(a)

Anions of arylacetic esters of Formula XXXVI can be formed as shown in Equation 31(a) by treatment with a suitable base such as lithium diisopropylamide (LDA). For a description of this procedure, see Equation 30. Other methods for the preparation and reaction of aliphatic ester enolates have been described by M. W. Rathke, *J. Am. Chem. Soc., 92,* 3222 (1970), M. W. Rathke and D. F. Sullivan, *ibid., 95,* 3050 (1973), and M. W. Rathke and A. Lindert, *ibid., 93,* 2318 (1971). Addition of the appropriate aldehydes of structure $R_8CHO$ to these anions gives the desired β-hyroxy esters XXXVII.

Equation 31(b)

The reaction of Equation 31(b) is accomplished by formation of the sodium alkoxide of alcohols XXXVII with sodium hydride, and treatment with benzyl bromide in the presence of a phase-transfer catalyst such as tetrabutylammonium iodide. For a description of this procedure, see S. Czernecki, C. Georgoulis and C. Provelenghiou, *Tetrahedron Lett.,* 3535 (1976).

Equation 31(c)

The reduction of carboxylic esters such as those of Formula XXXVIII with lithium aluminum hydride as shown in Equation 31(c) is a well-known process and can be carried out according to the procedures described by Gaylord, "Reduction with Complex Metal Hydrides", Interscience Publishers, Inc., New York, 1956, pp. 391—531.

Equation 31(d)

The transformation shown in Equation 31(d) can be achieved by one or more of the following procedures: treatment of alcohols of Formula XXXIX with phosphorus tribromide in pyridine (Shone, *et al., J. Am. Chem. Soc., 58,* 585 (1936)), or with triphenyl phosphine-carbon tetrabromide (Lee and Downie, *Tetrahedron, 23,* 2789 (1967); Hooz and Gilani, *Can. J. Chem., 46,* 86, (1968)).

Equation 31(e)

Compounds of Formula XL, where D is Br, can be converted to the corresponding sulfonyl chlorides as described for Equation 12. Compounds of Formula XL, where D is $SR_{12}$, are efficiently converted to the corresponding sulfonyl chlorides in a manner identical to that described in Equation 13.

Equation 31(f)

The nucleophilic displacement reaction depicted in Equation 31(f) can be accomplished by treatment of bromides of Formula XLI with sodium or potassium cyanide according to the procedure of J. R. Ruhoff, *Org. Syntheses,* Coll. Vol. II, 292 (1943).

Equation 31(g)

Nitriles of Formula XLII can be conveniently converted to the corresponding acids of Formula XVIIIa, where $R_7$ is H, by treatment with sulfuric acid in the presence of water as described by Adams and Thal. *Org. Syntheses,* Coll, Vol. I, 436 (1941), and Wenner, *J. Org. Chem., 15,* 548 (1950).

Compounds of Formula XVIIIa, where $R_7$ is other than H, can be conveniently prepared from the corresponding unsubstituted acids of Formula XVIIIa, where $R_7$ = H, by formation of the O,α-dianion and subsequent trapping with the appropriate electrophile as shown in Equation 32.

Equation 32

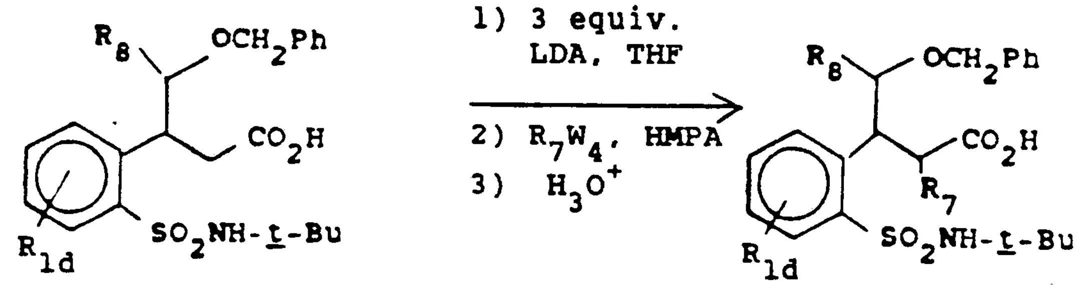


XVIIIa XVIIIa

wherein

$R_{1d}$, $R_7$ and $R_8$ are as defined above except $R_7$ is other than H, and $W_4$ is Cl, Br or I.

The alkylation of Equation 32 can be accomplished in a manner analogous to that described in Equation 31(a), except that 3 equivalents of a strong base such as LDA are required, and an electrophile of Formula $R_7W_4$, where $R_7$ is other than H and $W_4$ is Cl, Br, or I, is used to trap the enolate in lieu of an aldehyde. For relevant references, see J. C. Stowell, "Carbanions in Organic Synthesis", John Wiley and Sons, Inc., New York, 1979, pp. 157—161.

As mentioned above, N-*t*-butylbenzenesulfonamides of Formulas XVIIIb, XVIIIc, and XIX can all be synthesized from the appropriate arylacetic esters of Formula XXXVI in multi-step reaction schemes analogous to that described in Equation 31 for compounds of Formula XVIIIa. The minor modifications in reaction conditions necessary to achieve these syntheses would be obvious to one who is skilled in the art.

Butenolides of Formula XLIIIa can be prepared as shown below in Equation 33 by oxidation of the α-phenylthioethers XLIVa and subsequent thermolytic elimination.

Equation 33

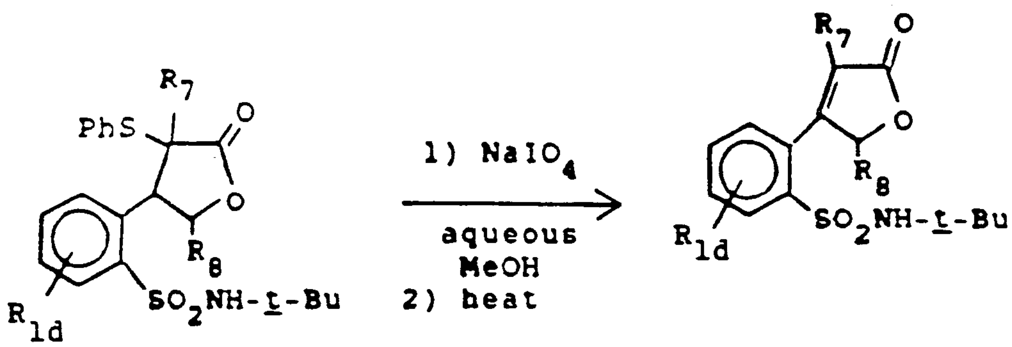


XLIVa XLIIIa

wherein

$R_{1d}$, $R_7$ and $R_8$ are as previously defined.

The reactions of Equation 33 can be carried out according to the procedure of B. M. Trost and T. N Salzmann *J. AM. Chem. Soc., 95,* 6840 (1973).

The requisite α-phenylthioethers XLIVa are readily obtained by treatment of the corresponding lactones of Formula XVIIa with a suitable base to generate the enolates, followed by trapping with diphenyl disulfide or phenylsulfenyl chloride as outlined in Equation 34.

Equation 34

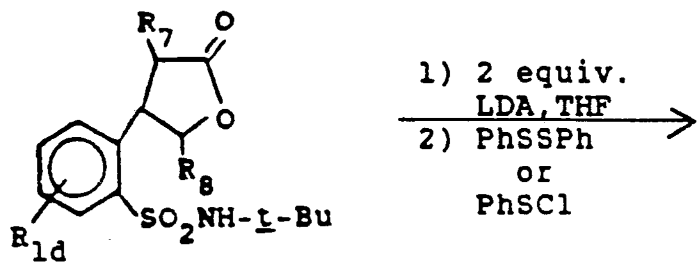


XVIIa

XLIVa

wherein

$R_{1d}$, $R_7$ and $R_8$ are as previously defined.

The α-alkylation of lactone enolates is a well-known process and can be successfully carried out according to G. H. Posner and G. L. Loomis, *Chem. Comm.*, 892 (1972), and K. Iwai, *et al., Chem Letters,* 385 (1974). For use of diphenyl disulfide as the electrophile, see the reference cited for Equation 33. In the case of N-*t*-butylbenzenesulfonamides of Formula XVIIa, it is necessary to use two molar equivalents of base. The first equivalent of base removes the acidic N—H proton, and the second equivalent forms the lactone enolate.

By using processes analogous to those described above in Equations 33 and 34, or modifications thereof,it is possible for one skilled in the art to prepare α,β-unsaturated lactones of Formulas XLIIIb-XLIIe from the appropriate saturated precursors of Formulas XVIIb-XVIIe as represented in Equations 35(a-d).

Equation 35

(a)

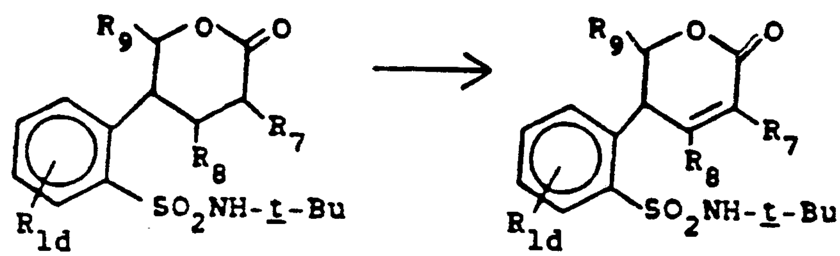

XVIIb

XLIIIb

(b)

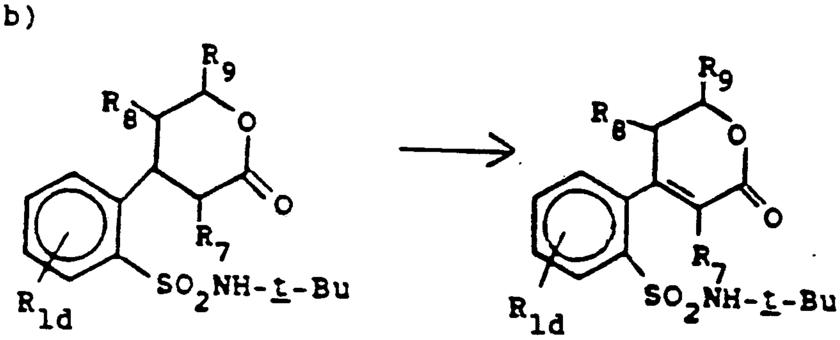

XVIIc

XLIIIc

(c)

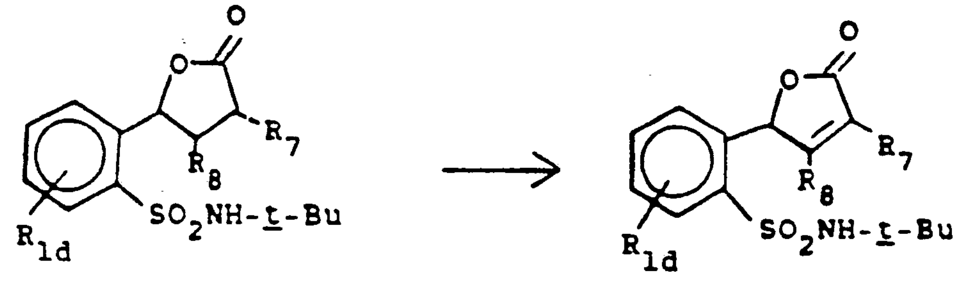

XVIId XLIIId

(d)

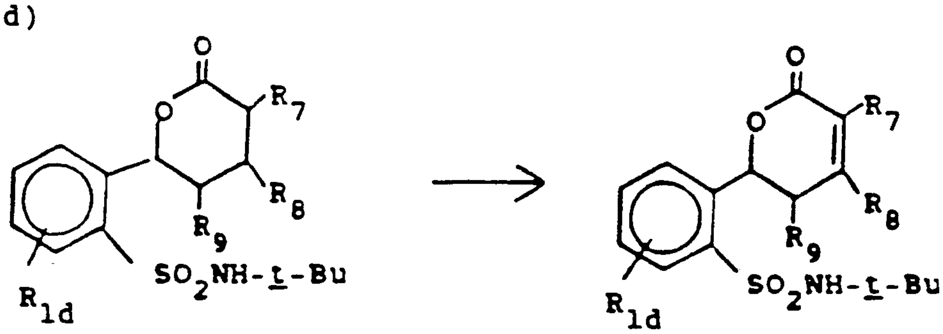

XVIIe XLIIIe

wherein

$R_{1d}$, $R_7$, $R_8$ and $R_9$ are as previously defined.

An alternative route to the lactones of Formula XLIIIe above where $R_8$ is $C_1$—$C_4$ alkyl and $R_9$ is H involves the addition of the dienolates of the substituted crotonate esters of Formula XLIIIf to the aldehyde XLIIIg according to the procedure of R. W. Dugger and C. H. Heathcock, *J. Org. Chem.*, *45*, 1181 (1980), as shown in Equation 35e. Aldehyde XLIIIg, which exists mostly as its cyclized tautomer, can be synthesized by the addition of N,N-dimethylformamide (DMF) to the dianions of sulfonamides of Formula XX.

Equation 35e

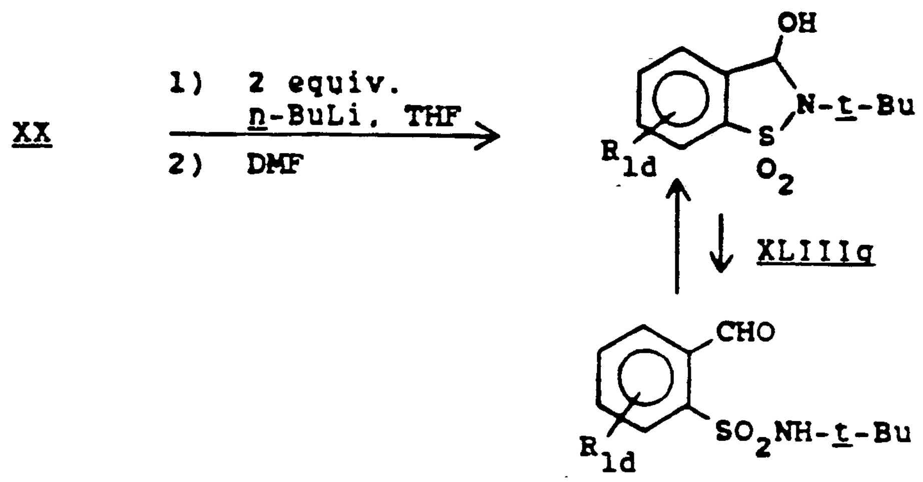


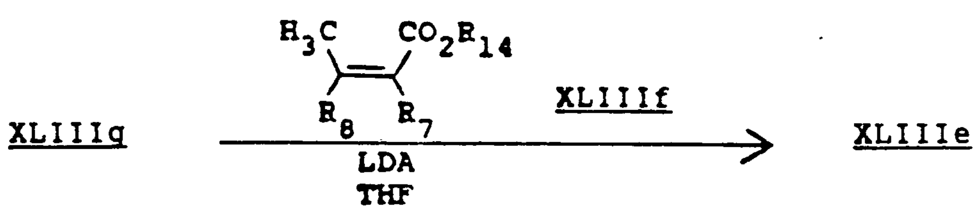

wherein

$R_{1d}$ and $R_7$ are as previously defined, $R_8$ and $R_{14}$ are independently $C_1$—$C_4$ alkyl, and $R_9$ is H.

In a similar fashion the lactams of Formulas XXIVa-XXIVg, where $R_3$ is other than H, can be converted to the corresponding α,β-unsaturated lactams of Formulas XLVa-XLVg as outlined in Equations 36(a-g).

Equation 36

(a)

XXIVa

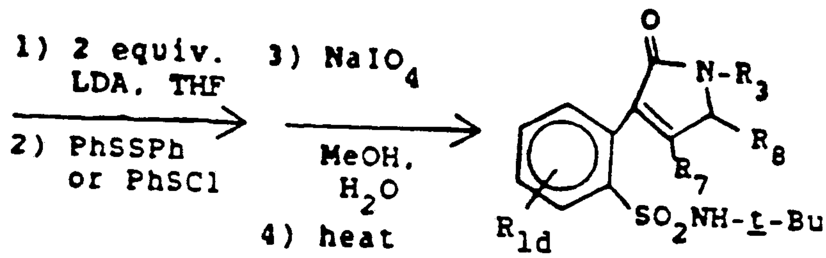


XLVa

(b)

XXIVb

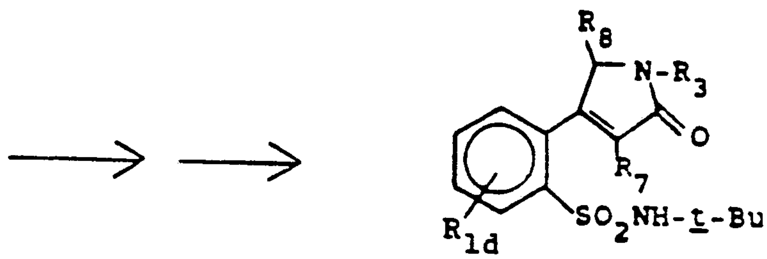

XLVb

(c)

XXIVc

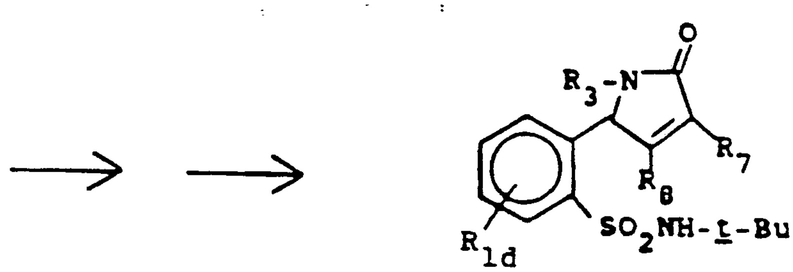

XLVc

(d)

XXIVd

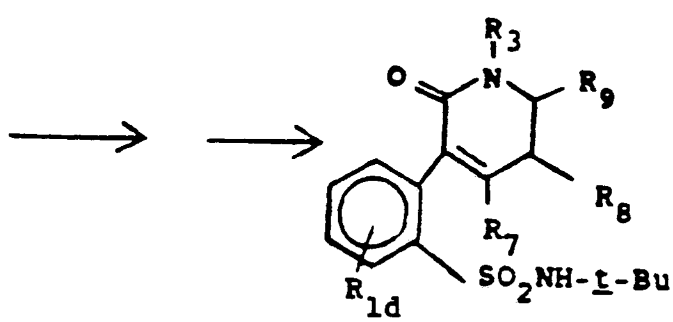

XLVd

(e)

XXIVe

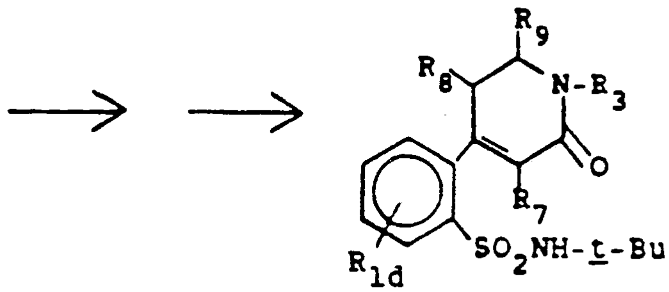

XLVe

(f)

XXIVf

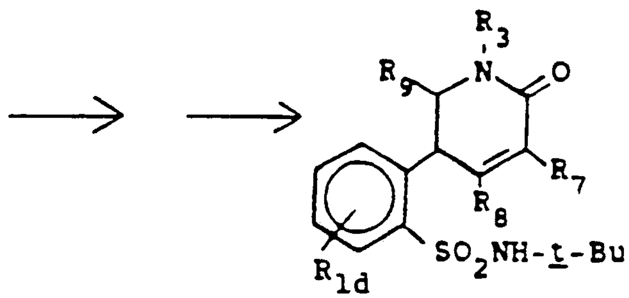

XLVf

(g)

XXIVg

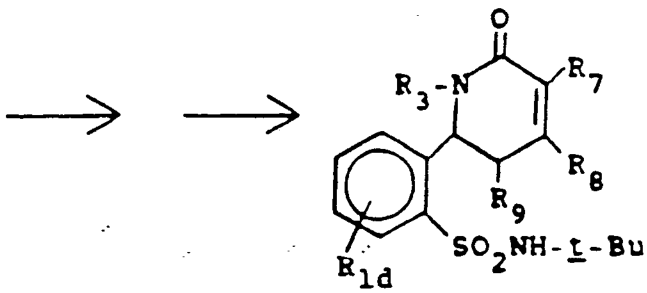

XLVg

wherein

$R_{1d}$, $R_3$, $R_7$, $R_8$, $R_9$ and $R_{10}$ are as previously defined except $R_3$ is other than H.

Unsaturated lactams of Formulas XLVa-XLVg, where $R_3$ is H, can be prepared by methods similar to those described in Equation 36. However, it is necessary to use one extra equivalent of a base such as lithium diisopropylamide (LDA) to generate the N,α-dianions XLVI, which can then be treated with diphenyl disulfide or phenylsulfenyl chloride in a manner identical to that described above to afford the desired products of Formulas XLVa-XLVg, where $R_3$ is H. Equation 37 depicts this procedure as it applies to the preparation of lactams of Formula XLVa ($R_3$ = H) from the appropriate saturated precursor of Formula XXIVa.

Equation 37

XXIVa

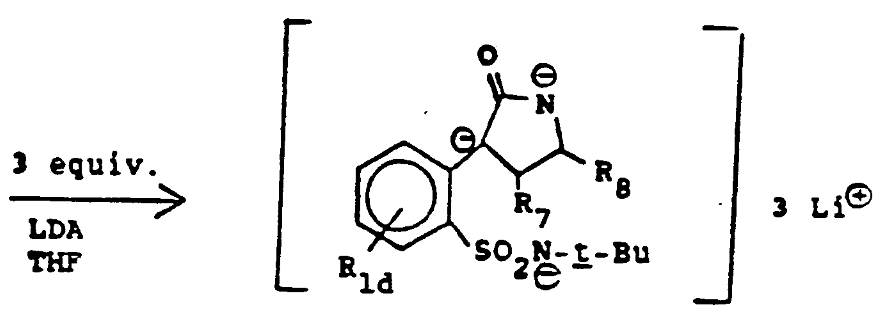


XLVI

[XLVI]

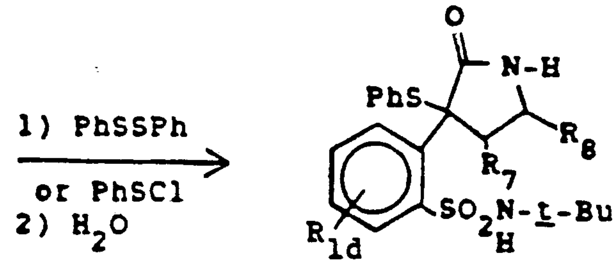


XLVII

XLVII

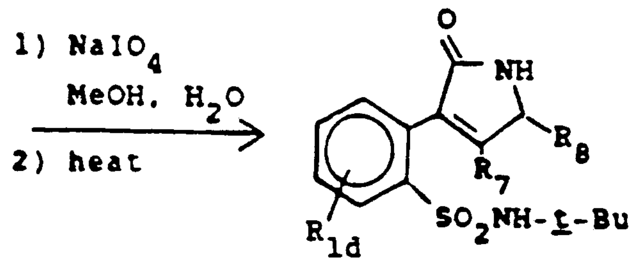


XLVa

wherein

$R_{1d}$, $R_7$ and $R_8$ are as previously defined.

For procedures dealing with the formation and alkylation of lactam α-anions such as those described in Equation 36, see P. A. Zoretic and F. Barcelos *Tetrahedron Lett.,* 529 (1977), or B. M. Trost and R. A. Kunz, *J.Org. Chem., 39,* 2475 (1974).

The method shown in Equation 37 can be applied to the synthesis of unsaturated lactams of Formulas XLVb-XLVg, where $R_3$ is H. For a relevant reference see J—P. Deprés, A. E. Greene and P. Crabbe, *Tetrahedron Lett.,* 2191 (1978).

Removal of the *tert*-butyl protecting group from compounds of Formula XLIIIa-XLIIIe and XLVa-XLVg by one or more of the procedures described in Equation 8 will give the primary sulfonamides of Formula IVb, where Q is Q—30, Q—97, Q—96, Q—31, Q—98, Q—32, Q—33, Q—34, Q—99, Q—100, Q—101 or Q—102. These sulfonamides can then be converted to compounds of Formula I with the corresponding substituents.

Enones of Formulas XLVIIIa and XLVIIIb can be conveniently prepared as shown below in Equation 38 by an intramolecular aldol condensation of the appropriate carbonyl compounds of Formulas XLIXa and XLIXb.

Equation 38

(a)

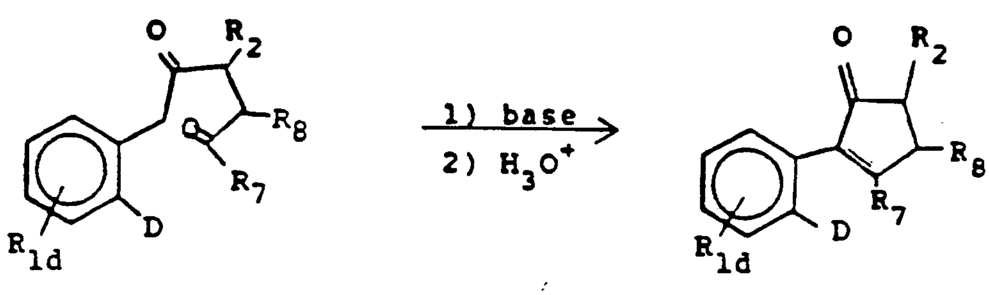


XLIXa XLVIIIa

(b)

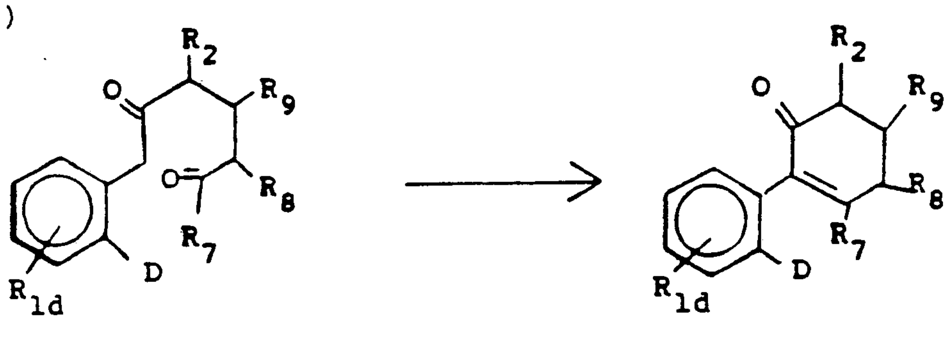

XLIXb XLVIIIb

wherein

$R_{1d}$, $R_2$, $R_7$, $R_8$ and $R_9$ are as previously defined, D is Br, $SR_{12}$ or $NO_2$, and $R_{12}$ is $C_2$—$C_4$ alkyl or benzyl.

The aldol condensation depicted in Equations 38(a) and 38(b) can be carried out in a manner analogous to that described for Equation 25(b).

In a similar fashion, enones of Formulas XLVIIIc—XLVIIIi can be prepared from the appropriate carbonyl compounds of Formulas XLIXc—XLIXi as outlined below in Equation 39 (a-g).

Equation 39

(a)

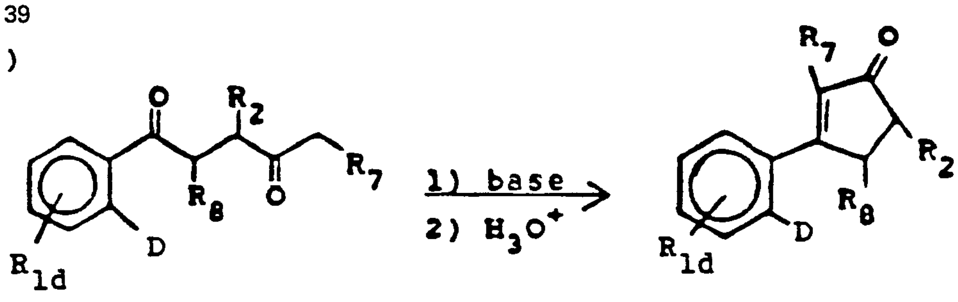


XLIXc XLVIIIc

(b)

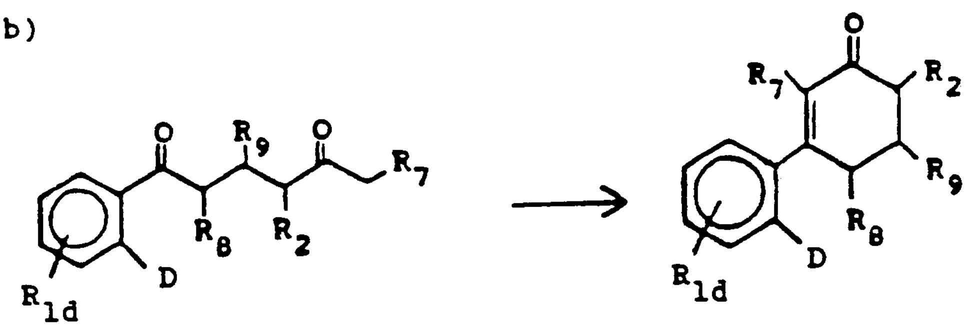

XLIXd XLVIIId

(c)

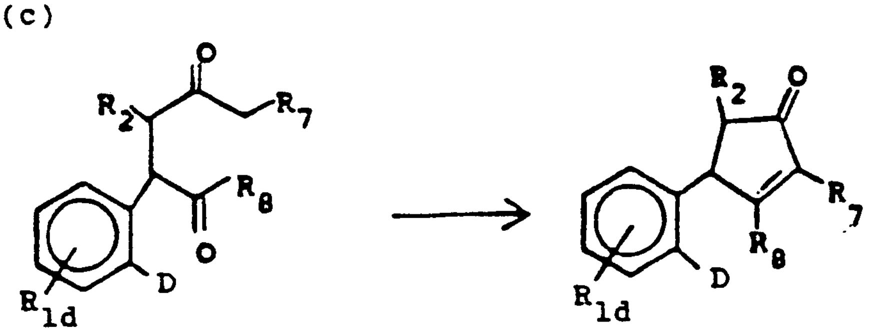

XLIXe XLVIIIe

(d)

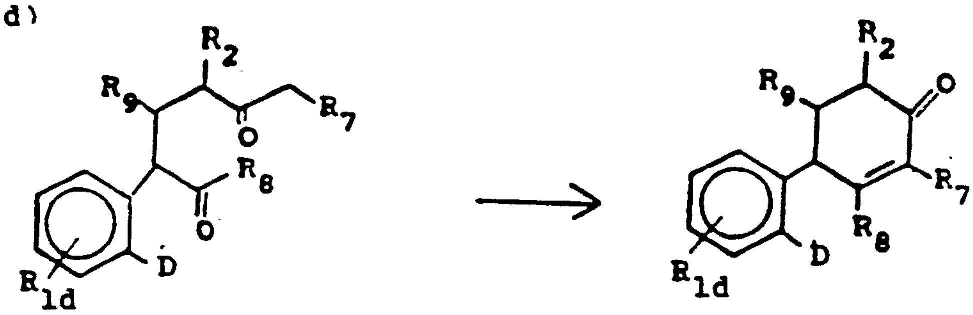

XLIXf XLVIIIf

(e)

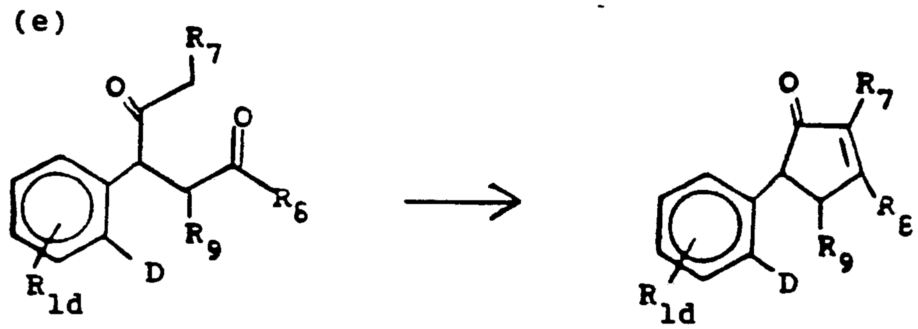

XLIXg XLVIIIg

(f)

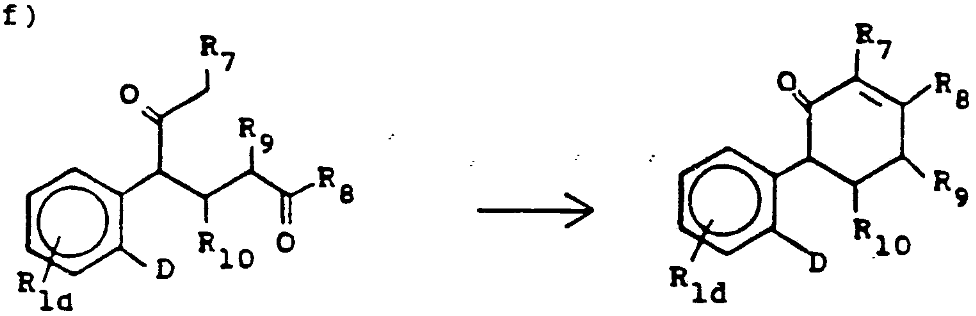

XLIXh XLVIIIh

(g)

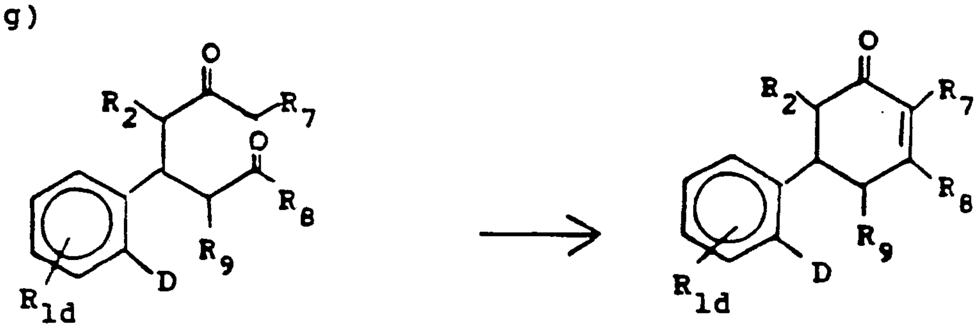

XLIXi XLVIIIi

wherein

$R_{1d}$, $R_2$, $R_7$, $R_8$ and $R_9$ are as previously defined, D is Br, $SR_{12}$ or $NO_2$, and $R_{12}$ is $C_2$—$C_4$ alkyl or benzyl.

Another route to the enones of Formula XLVIIIc and XLVIIId above is depicted in Equation 39h. The addition of 3-ethoxy-2-cyclohexenone or 3-ethoxy-2-cyclopentenone derivatives of Formula XLVIIIs and XLVIIIt respectively, to the dianions of sulfonamides of Formula XX followed by mild hydrolysis during work-up affords the desired enones directly.

XX

1) 2 equiv. n-BuLi, THF
2) XLVIIIs or XLVIIIt
3) $H_3O^+$

XLVIIId or XLVIIIc

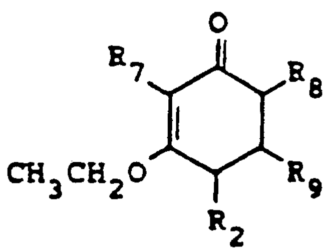

XLVIIIs

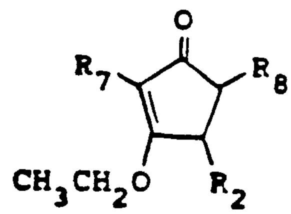

XLVIIIt

wherein

$R_{1d}$, $R_2$, $R_7$, $R_8$ and $R_9$ are as previously defined, D is $SO_2NH$-*t*-Bu.

Alternatively, many of the enones of Formulas XLVIIIj—XLVIIIr can be prepared from the correspondiong saturated ketones of Formulas XXIIIa—XXIIIf via treatment with the appropriate base to form the α-carbanions, trapping with diphenyl disulfide or phenylsulfenyl chloride, and subsequent oxidative elimination. This sequence is depicted below in Equation 40 for the preparation of enones XLVIIIj or XLVIIIp from ketones of Formula XXIIIa or XXIIIa'.

Equation 40

(a)

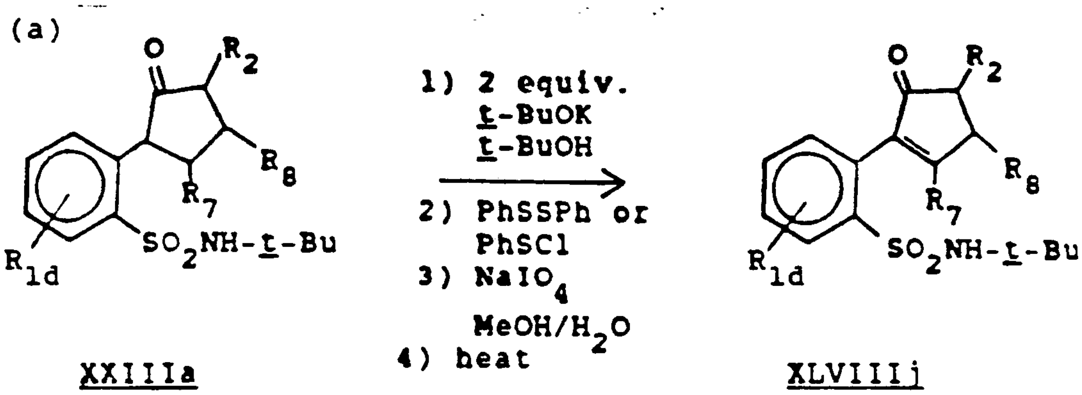


XXIIIa XLVIIIj

(b)

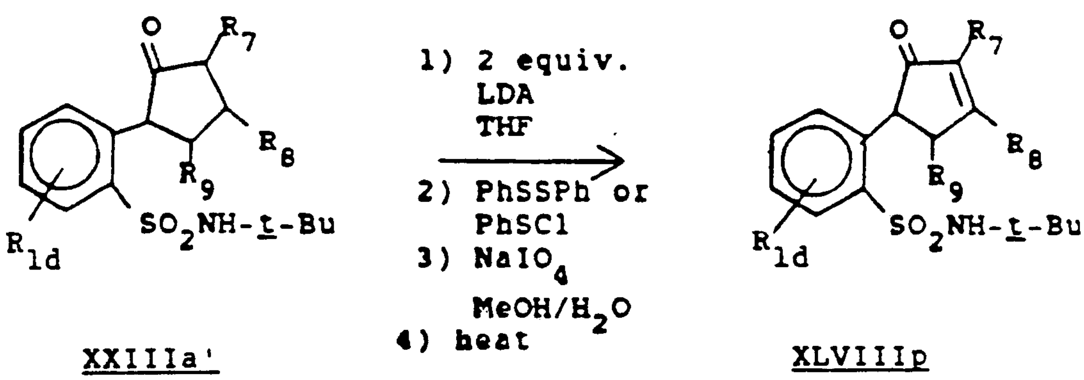


XXIIIa' XLVIIIp

wherein

$R_{1d}$, $R_2$, $R_7$, $R_8$ and $R_9$ are as previously defined.

Equation 40(a)

The transformation shown above in Equation 40(a) can be effectively carried out by treating ketones of Formula XXIIIa with two molar equivalents of a strong base such as potassium *tert*-butoxide (*t*-BuOK) in a suitable solvent such as *t*-butanol under an inert atmosphere at 0°—25°C. Such conditions are conducive to formation of the more stable, or thermodynamic, enolates. These anions are then treated in a manner analogous to that described in Equations 33 and 34.

Equation 40(b)

The transformation shown above in Equation 40(b) can be effectively carried out by treating ketones of Formula XXIIIa' with two molar equivalents of a hindered base such as a lithium diisopropylamide (LDA) in a suitable solvent such as tetrahydrofuran at low temperature (−78°C) under an inert atmosphere. Such conditions are conducive to formation of the less stable, or kinetic, enolates. These anions are then treated in a manner analogous to that described in Equations 33 and 34.

For a discussion of the optimal conditions required for selectively generating thermodynamic or kinetic enolates, see J. C. Stowell, "Carbanions in Organic Synthesis", John Wiley and Sons, Inc., New York, 1979, pp. 8—11, and references cited therein.

In a similar fashion, enones of Formulas XLVIIIk, XLVIIIl, XLVIIIm, XLVIIIn, XLVIIIo, XLVIIIq, and XLVIIIr can be prepared from the corresponding ketones of Formulas XXIIIb—XXIIIf by selection of the appropriate conditions for enolate formation.

Many of the requisite carbonyl compounds of Formulas XLIXa-XLIXi are known in the literature or can be prepared from known intermediates by methods obvious to one skilled in the art. The appropriately substituted arylacetic esters of Formula XXXVI will serve as useful precursors for most of the desired compounds of Formulas XLIXa—XLIXi, and can be transformed by methods similar to those described in Equation 31 or modifications thereof. Such methods would be obvious to one skilled in the art.

Sulfonamides of Formulas La and Lb can be prepared as shown in Equation 41 by treatment of compounds of Formulas LIa and LIb with base.

Equation 41

(a)

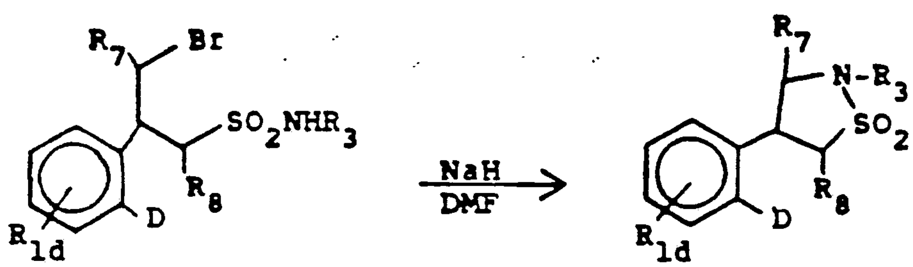


LIa La

(b)

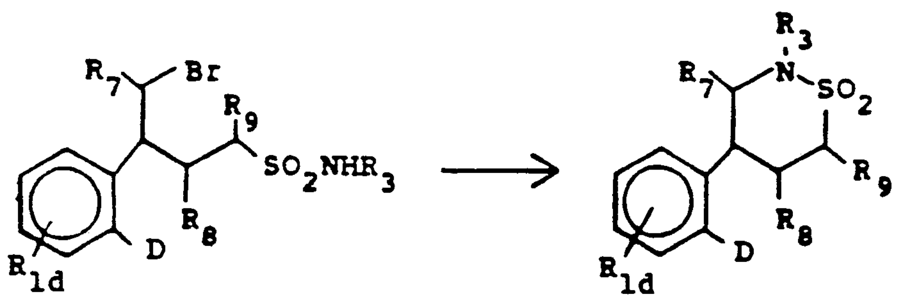

LIb Lb

wherein

$R_{1d}$, $R_3$, $R_7$, $R_8$ and $R_9$ are as previously defined, D is Br, $SR_{12}$ or $NO_2$, and $R_{12}$ is $C_2$—$C_4$ alkyl or benzyl.

The reactions of Equations 41(a) and 41(b) can be accomplished by adding a solution of sulfonamide LIa or LIb in a suitable solvent such as N,N-dimethylformamide (DMF) to a stirred suspension of a base such as sodium hydride at 0°—25°C under an inert atmosphere. After being stirred at 25°—100°C for several

hours, or until all of the starting material has disappeared, the reaction mixture is cooled and poured into ice-water. The desired products of Formula La or Lb are then isolated by filtration or extraction with a suitable solvent such as diethyl ether, methylene chloride, or ethyl acetate, followed by drying and evaporation of the volatile components.

The sulfonamides of Formulas Lc-Lg can be synthesized in a manner identical to that described above in Equation 41 from the appropriate compounds of Formula LIc-LIg as shown in Equations 42(a-e).

Equation 42

(a)

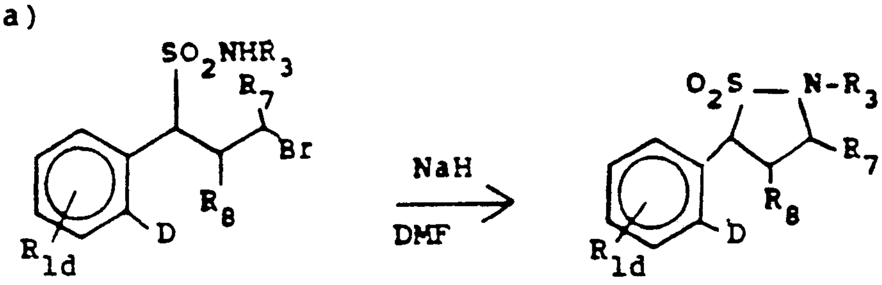


LIc Lc

(b)

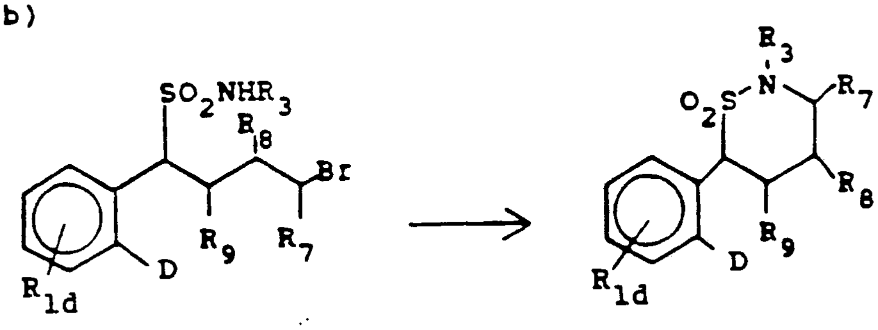

LId Ld

(c)

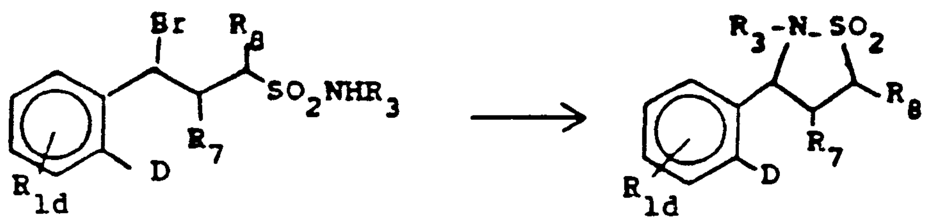

LIe Le

(d)

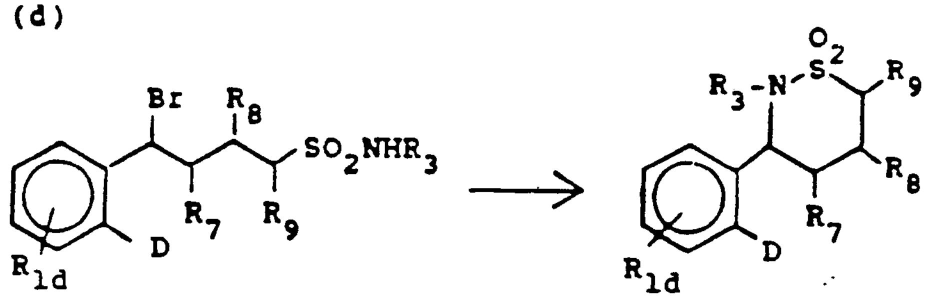

LIf Lf

(e)

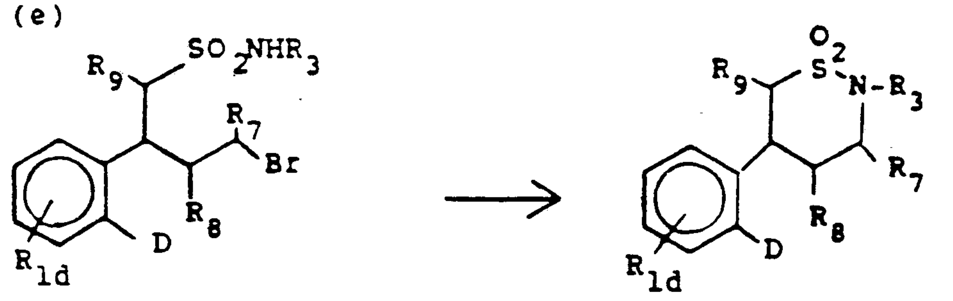

LIg Lg

wherein

$R_{1d}$, $R_2$, $R_7$, $R_8$ and $R_9$ are as previously defined, D is Br, $NO_2$, or $SR_{12}$, and $R_{12}$ is $C_2$—$C_4$ alkyl or benzyl.

The requisite compounds of Formulas LIa-LIg can be synthesized via the same basic four-step sequence of reactions. Equation 44 depicts this synthetic scheme for the preparation of sulfonamides LIa, starting from the appropriate alkyl bromides of Formula LVIIa as a representative example.

Equation 44

(a)

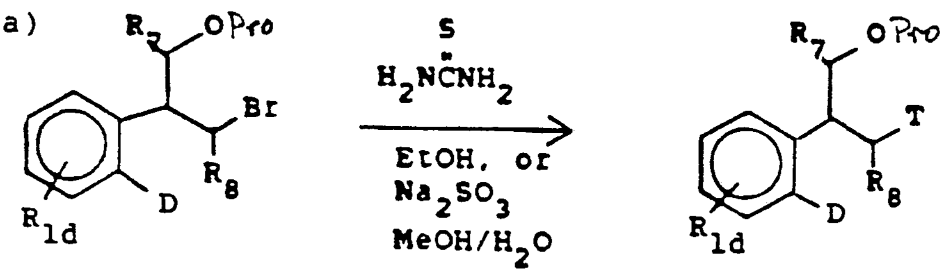


LVIIa LVIa

(T=-SC(=NH)NH$_2$•HBr, or -$SO_3$Na)

(b)

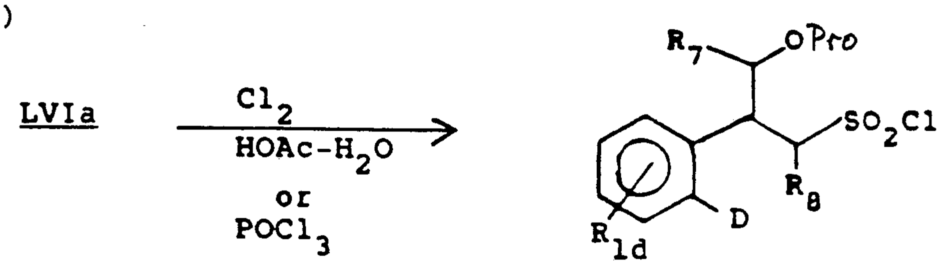


LVa

(c)

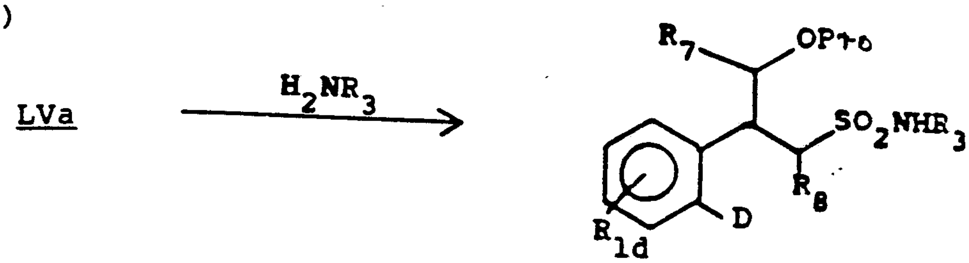


LIVa

(d)

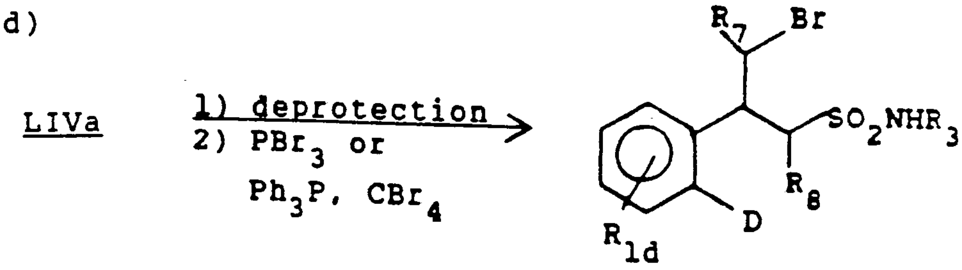


LIa

wherein

$R_{1d}$, $R_2$, $R_7$, $R_8$ and $R_9$ are as previously defined, D is Br, $SR_{12}$ or $NO_2$, $R_{12}$ is $C_2$—$C_4$ alkyl or benzyl, and Pro is an appropriate alcohol protecting group such as $CH_2Ph$, $CH_3$, *t*-butyl, $Si(CH_3)_2$(*t*-Bu), etc.

Equation 44(a)

The displacement reaction of Equation 44(a) can be effected by treating the alkyl bromides of Formula LVIIa with either one molar equivalent of thiourea to give the corresponding isothiouronium salts LVIa, where T is $—SC(NH_2)=NH·HBr$, or with sodium sulfite to yield the sodium sulfonate salts LVIa, where T is $—SO_3Na$. For detailed procedures relating to the preparation of isothiouronium salts, see Urquhart, Gates and Connor, *Org. syntheses, 21,* 36 (1941), or Vogel *J. Chem. Soc.,* 1822 (1948). For the use of sodium sulfite in the preparation of sodium sulfonate salts, see Reed and Tarter, *J. Am. Chem. Soc., 57,* 571 (1935), or Latimer and Bost. *J. Org. Chem., 5,* 24 (1940).

Equation 44(b)

The choice of chlorination conditions to be employed in the reacton of Equation 4e4(b) depends upon the nature of the substituent T. When T is $—SC(NH_2)=NH·HBr$, the process can be effected with chlorine in an aqueous medium according to Johnson and Sprague, *J. Am. Chem. Soc., 58,* 1348 (1936); *59,* 1837, 2439 (1937). When T is $—SO_3Na$, the reaction can be carried out using phosphorus oxychloride according to the procedure of Westlake and Dougherty, *J. Am. Chem. Soc., 63,* 658 (1941).

Equation 44(c)

The reaction shown in Equation 44(c) is conveniently accomplished in manner identical to that described for Equation 7. For useful references, see Huntress and Carter, *J. Am. Chem. Soc., 62,* 511 (1940), or Huntress and Autenrieth, *ibid., 63,* 3446 (1941).

Equation 44(d)

The first step of Equation 44(d) involves removal of the hydroxyl protecting group to release an alcohol substituent. Selection of a suitable protecting group must take into account the nature of other substituents in the molecule and would be obvious to one skilled in the art. For a compilation of references describing the wide variety of protecting groups available for alcohols, see T. W. Greene, "Protective Groups in Organic Synthesis". John Wiley and Sons, Inc., New York, 1981, pp. 10—72. The second step shown in Equation 44(d) involves the preparation of the desired products of Formula LIa from the corresponding alcohols with either phosphorous tribromide or triphenylphosphine-carbon tetrabromide. For relevant procedures, see Equation 31(d).

The requisite alkyl bromides of Formula LVIIa, where $R_8$ is H, can be synthesized in a manner analogous to that described in Equation 31 for compounds of Formula XL. Alternatively, the alkyl bromides of Formula LVIIa, where $R_8$ is other than H, are conveniently obtained as shown in Equation 45, starting from the appropriate esters of Formula XXXVIIIa.

Equation 45

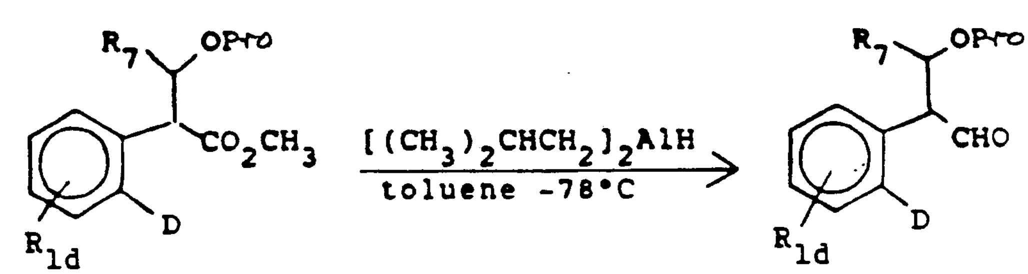


XXXVIIIa LIXa

LIXa

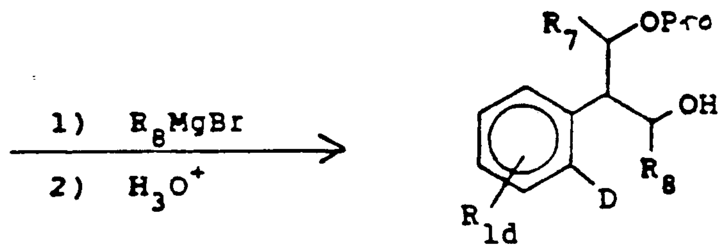


LIXb

LIXb

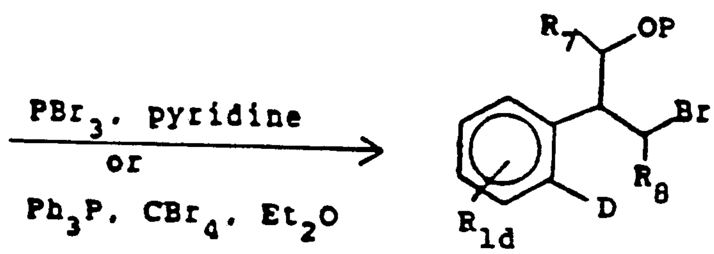


LVIIa

wherein

$R_{1d}$, $R_7$ and $R_8$ are as previously defined, D is Br, $SR_{12}$ or $NO_2$, $R_{12}$ is $C_2$—$C_4$ alkyl or benzyl, and Pro is a protectiong group as defined above in Equation 44.

The reduction of carboxylic esters such as those of Formula XXXVIIIa to the corresponding aldehydes with diisobutylaluminum hydride can be carried out as described in Equation 25(a). The second step of Equation 45 involves the addition of appropriate Grignard reagents, $R_3MgBr$, to aldehydes of Formula LIXa to afford the corresponding alcohols LIXb after aqueous workup. This is a well-known reaction and can be accomplished by following the procedures compiled in Patai, "The Chemistry of the Carbonyl Group", Vol. 1, Interscience Publishers, New York, 1969, pp. 621—693, or Kharasch and Reinmuth, "Grignard Reactions of Nonmetallic Substances", Prentice-Hall, Inc., Englewood Cliffs, N.J., 1954, pp. 138—528. The third step shown in Equation 45 involves the conversion of alcohols LIXb to the corresponding alkyl bromides LVIIa, and has been described above in Equation 31(d).

Compounds of Formulas LIb-LIg can be prepared by methods analogous to those described in Equations 44 and 45 with suitable modifications which would be obvious to one skilled in the art.

Equation 46 depicts the intramolecular reaction of γ- and δ-hydroxysulfonyl chlorides of Formulas LXa and LXb to afford the sulfonates of Formulas LXIa and LXIb.

Equation 46

(a)

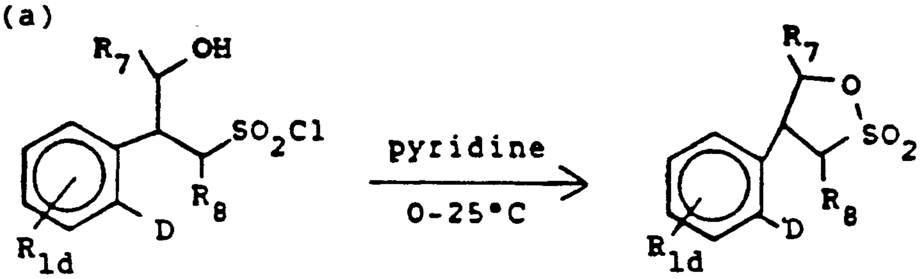


LXa LXIa

(b)

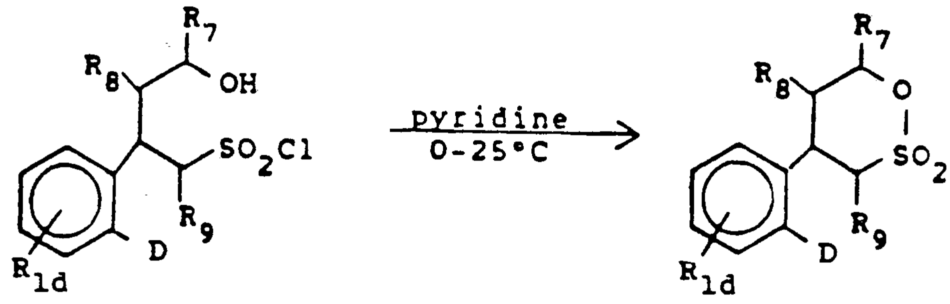


LXb LXIb

wherein

$R_{1d}$, $R_7$ and $R_9$ are as previously defined, D is Br, $NO_2$ or $SR_{12}$, and $R_{12}$ is $C_2$—$C_4$ alkyl or benzyl.

The cyclization shown in Equations 46(a) and 46(b) can be achieved according to the procedures of Tipson, *J. Org. Chem., 9,* 235 (1944), Marvel and Sekera, *Org. Syntheses, 20,* 50 (1940), or Sekera and Marvel, *J. Am. Chem. Soc., 55,* 346 (1933).

In a similar fashion, sulfonates of Formulas LXIc—LXIg can be prepared by base-induced cyclization of the appropriate or γ- or δ-hydroxysulfonyl chlorides of Formulas LXc—LXg, as shown below in Equations 47(a-e).

Equation 47

(a)

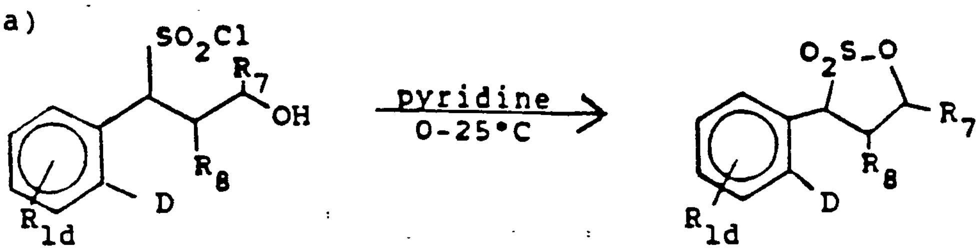


LXc LXIc

(b)

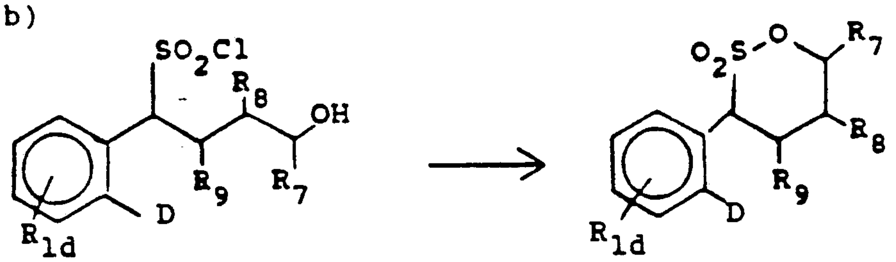

LXd LXId

(c)

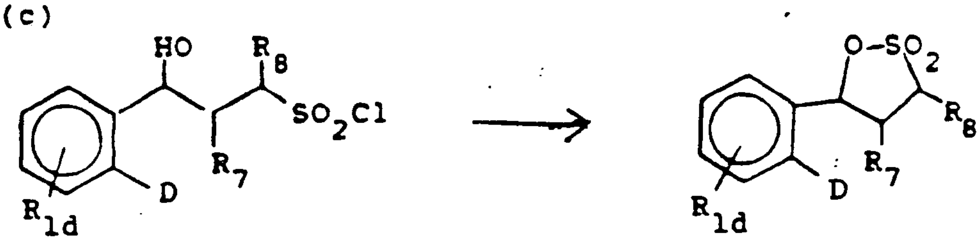

LXe LXIe

(d)

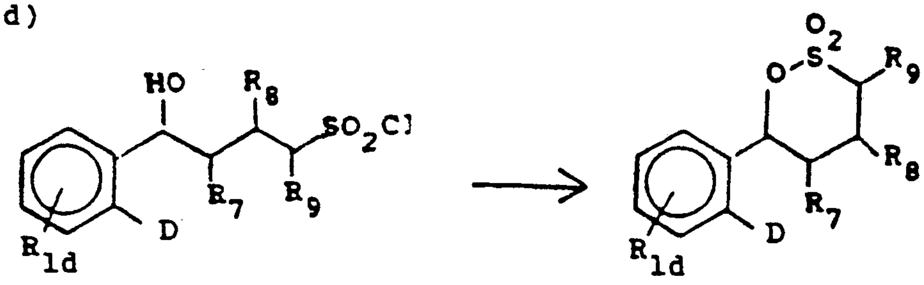

LXf LXIf

(e)

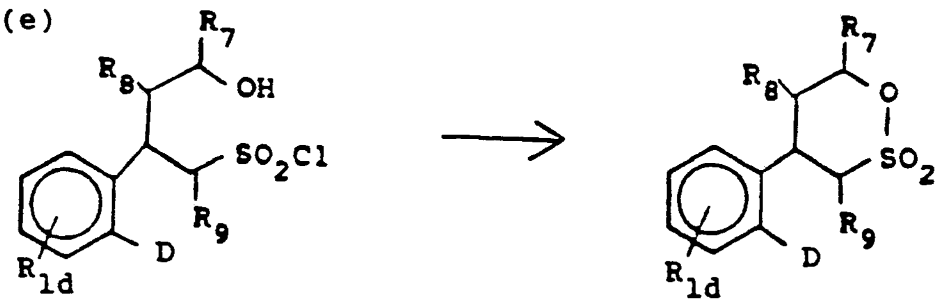

LXg LXIg

wherein

$R_{1d}$, $R_7$, $R_8$ and $R_9$ are as previously defined, D is Br, $NO_2$ or $SR_{12}$, and $R_{12}$ is $C_2$—$C_4$ alkyl or benzyl.

The synthesis of the requisite hydroxysulfonyl chlorides has been described previously. For example, γ-hydroxysulfonyl chlorides of Formula LXa can be obtained from the corresponding protected compounds of Formula LVa (Equation 44). In a similar fashion, the requisite hydroxysulfonyl chlorides LXb—LXg can be prepared from the appropriate protected alcohols by methods which would be obvious to one skilled in the art.

Sulfones of Formulas LXIIa—LXIIe can be conveniently prepared as shown in Equation 48 by oxidation of the appropriate 5- and 6-membered ring thioethers of Formulas LXIIIa—LXIIIe.

Equation 48

(a)

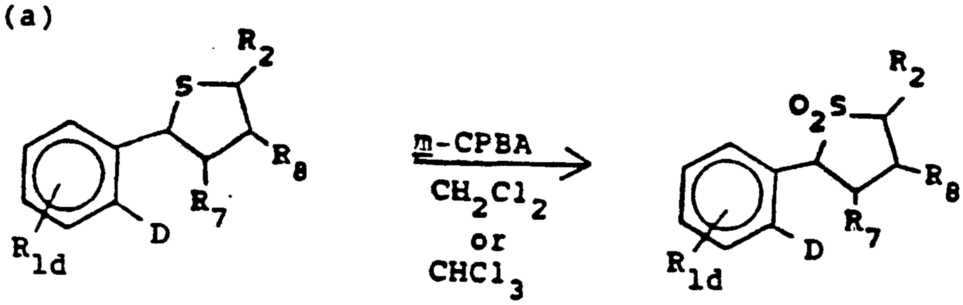


LXIIIa LXIIa

(b)

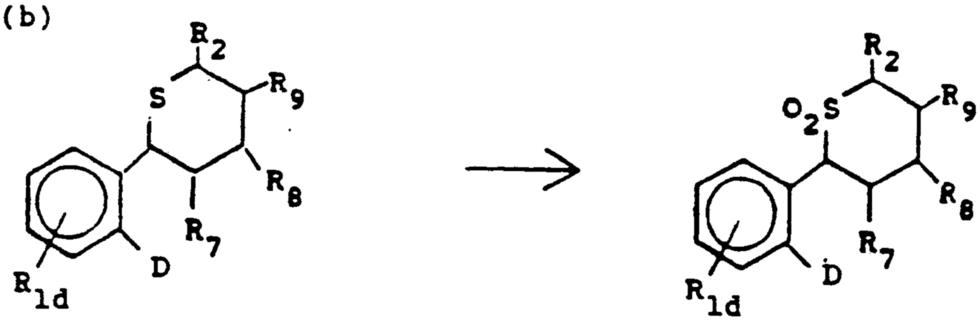

LXIIIb LXIIb

(c)

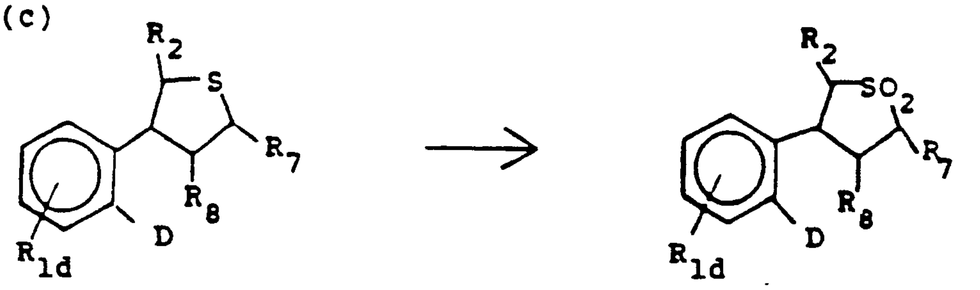

LXIIIc LXIIc

(d)

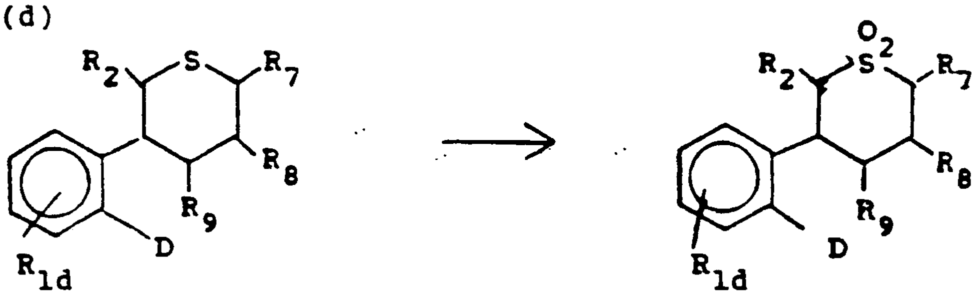

LXIIId LXIId

(e)

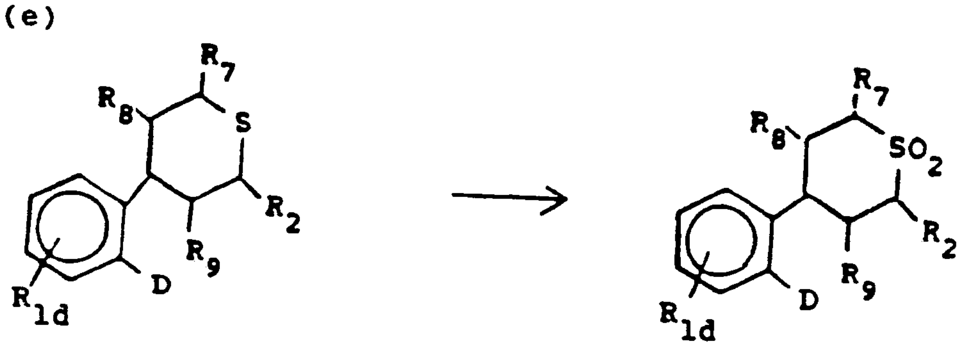

LXIIIe LXIIe

wherein

$R_{1d}$, $R_2$, $R_7$, $R_8$ and $R_9$ are as previously defined, and D is $NO_2$ or $SO_2NH$-*t*-Bu.

The oxidation shown in Equation 48 is most conveniently carried out by adding a solution of at least two molar equivalents of *m*-chloroperoxybenzoic acid (*m*-CPBA) in a suitable solvent such as methylene chloride or chloroform to a solution of the sulfide LXIII(a-e) in the same solvent at 0°—25°C. After the reaction mixture has been stirred at about 25°C for 1—4 hours, excess oxidant is destroyed by the addition

of saturated aqueous sodium bisulfite (ice-water cooling). The reaction mixture is then filtered to remove the by-product *m*-chlorobenzoic acid, and the filtrate is washed several times with portions of saturated aqueous sodium bicarbonate. The desired products of Formula LXII(a-e) are isolated by drying and evaporation of the organic layer, and are often sufficiently pure to be carried directly on to the next step.

The requisite sulfides of Formulas LXIIIa and LXIIIb can be prepared as shown in Equation 49, by conversion of the appropriate diols of Formulas LXIVa and LXIVb to the corresponding dibromides LXVa and LXVb, respectively, and subsequent treatment with sodium sulfide to effect cyclization.

Equation 49

(a)

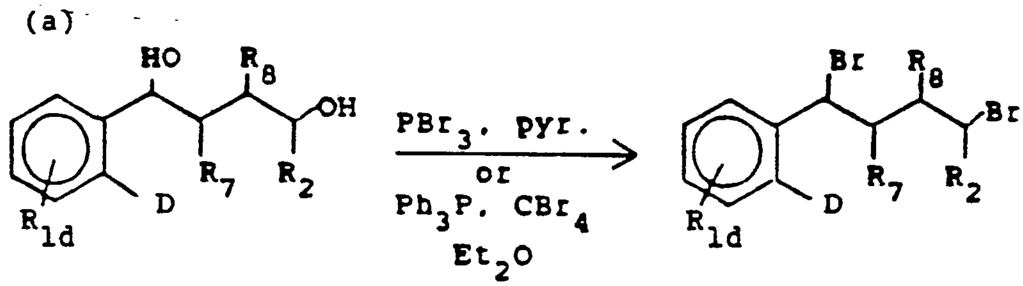


LXIVa LXVa

LXVa

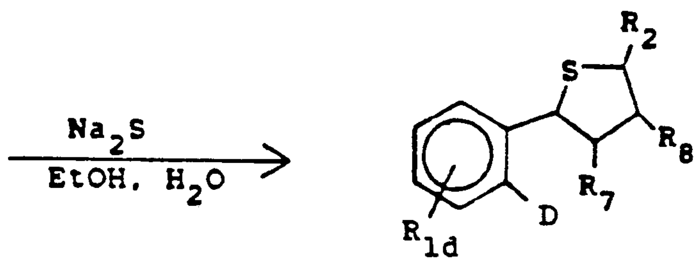


LXIIIa

(b)

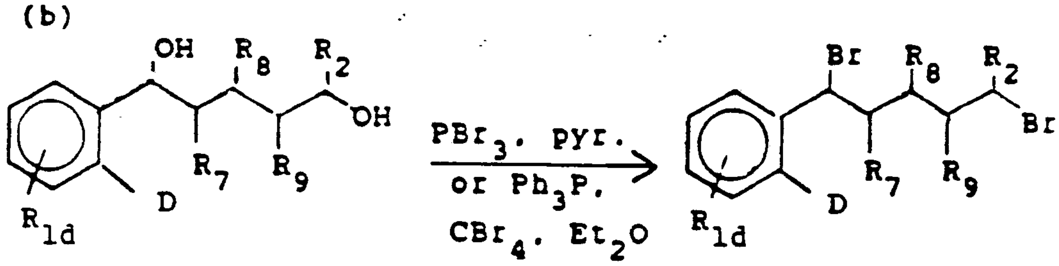


LXIVb LXVb

LXVb

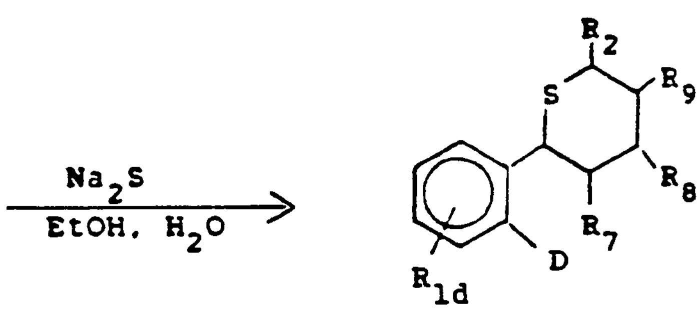


LXIIIb

wherein

$R_{1d}$, $R_2$, $R_7$, $R_8$ and $R_9$ are as previously defined, and D is $NO_2$ or $SO_2NH$-*t*-Bu.

The first step of Equations 49(a) and 49(b) can be effected in a manner analogous to that described for Equation 31(d) except that two molar equivalents of the appropriate brominating agent are required. The second step shown above in Equations 49(a) and 49(b) can be conveniently carried out according to the method of Tarbell and Weaver, *J. Am. Chem. Soc., 63,* 2940 (1941), or Naylor, *J. Chem. Soc.,* 1107 (1947).

In a similar manner, the requisite sulfides of Formulas LXIIIc—LXIIIe can be prepared via conversion of

the appropriate diols to the corresponding dibromides, and subsequent cyclization by treatment with sodium sulfide.

Diols such as those of Formulas LXIVa and LXIVb can be prepared from the appropriately substituted intermediates, many of which have already been described. Methods needed to effect these transformations would be obvious to one skilled in the art.

The α,β-unsaturated sulfonamides, represented by Formula LXVIIa, can be prepared by a procedure identical to that described for the synthesis of α,β-unsaturated lactams of Formulas XLVa-XLVg as shown above in Equations 36 and 37. For example, treatment of sulfonamides of Formula La with base, addition of diphenyl disulfide or phenylsulfenyl chloride, and subsequent oxidative elimination gives the unsaturated compounds of Formula LXVIIa as shown in Equation 50.

Equation 50

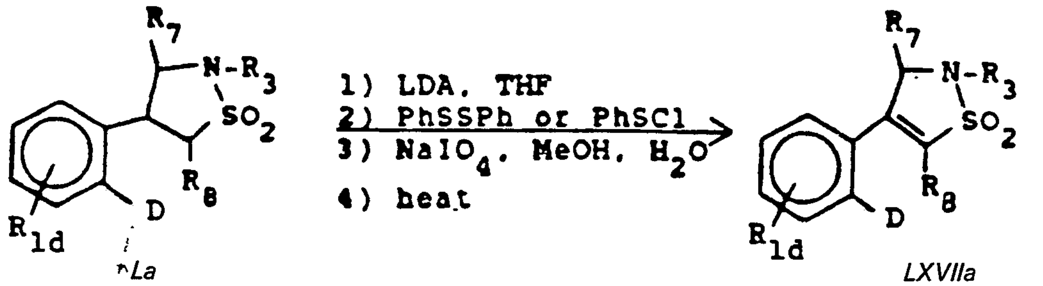


La LXVIIa

wherein

$R_{1d}$, $R_3$, $R_7$, and $R_8$ are as previously defined, and D is Br or $NO_2$.

The transformation depicted in Equation 50 is conveniently carried out in a manner analogous to that described in Equations 36 and 37. When $R_3$ is H in Equation 50, it is necessary to use one extra molar equivalent of lithium diisopropylamide (LDA) to form the α,N-dianions of sulfonamides La-Lg. This procedure can be applied to the preparation of the remaining α,β-unsaturated analogues of sulfonamides Lb-Lg.

The α,β-unsaturated sulfonates of Formulas LXVIIIa-LXVIIIg can be synthesized via the four-step sequence of reactions shown below in Equations 51(a-g), starting from the saturated compounds of Formulas LXIa-LXIg.

Equation 51

(a)

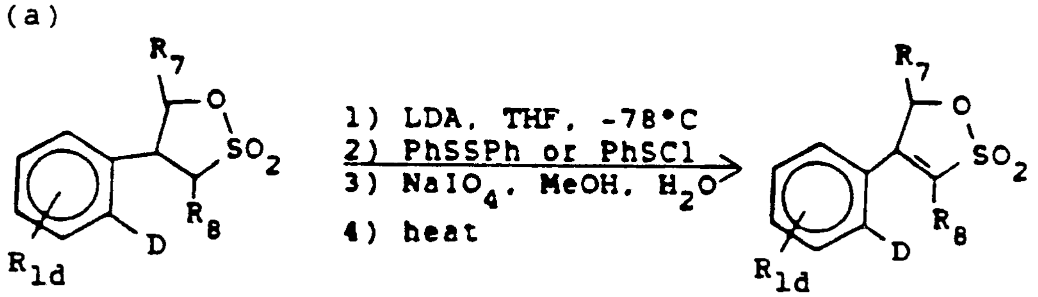


LXIa LXVIIIa

(b)

LXIb 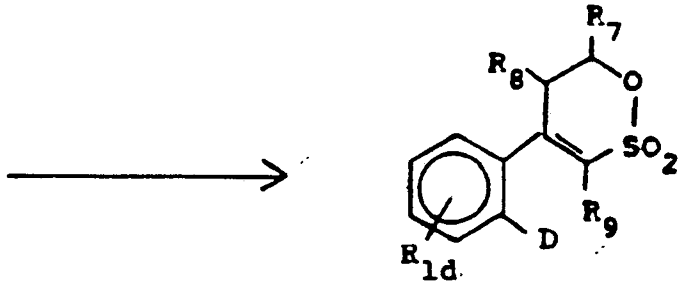

LXVIIIb

(c)

LXIc

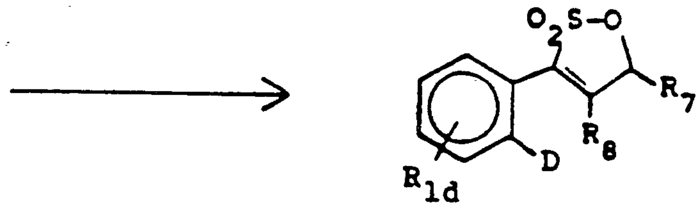

LXVIIIc

(d)

LXId

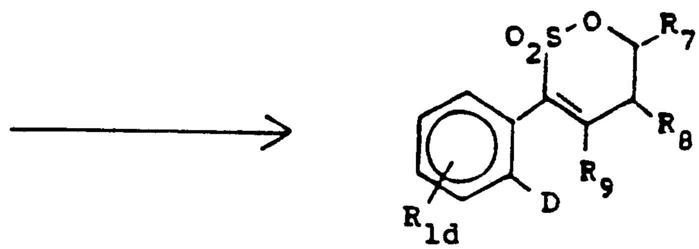

LXVIIId

(e)

LXIe

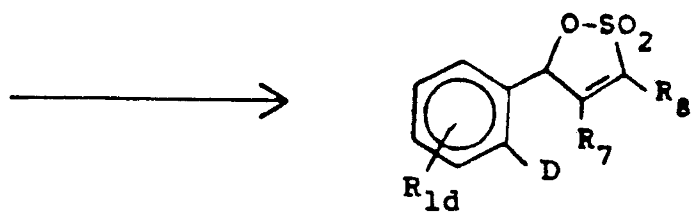

LXVIIIe

(f)

LXIf

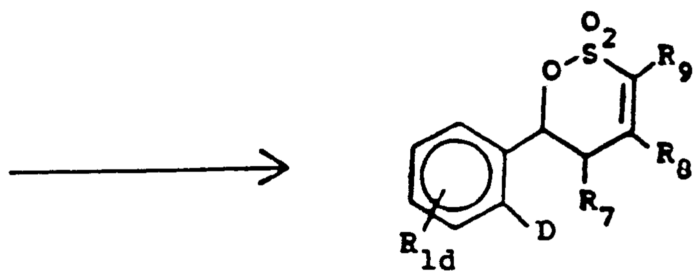

LXVIIIf

(g)

LXIg

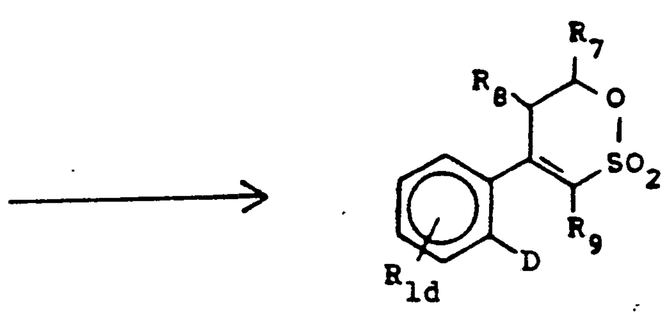

LXVIIIg

wherein

$R_{1d}$, $R_7$, $R_8$ and $R_9$ are as previously defined and D is Br, or $NO_2$.

The formation of anions *alpha* to the sulfonyl group of alkyl sulfonates such as those of Formulas LXI*a*–LXIg is a process with considerable precedent in the literature. For related examples refer to Truce, Hollister, Lindy and Parr, *J. Org. Chem., 33,* 43 (1968); Truce and Vrencur, *Can. J. Chem., 47,* 860 (1969), *J. Org. Chem., 35,* 1226 (1970); Julia and Arnould, *Bull. Soc. Chim. Fr.,* 743, 746 (1973); and Bird and Stirling, *J. Chem. Soc. (B),* 111 (1968). Reaction of these sulfonate anions with diphenyl disulfide or phenylsulfenyl chloride and subsequent oxidative elimination can be effected as described for Equations 36 and 37.

Compounds of Formulas LXXa—LXXd can be synthesized as shown in Equation 52 by the reaction of appropriate α,β-unsaturated carbonyl compounds LXXIa—LXXId with α-chloroketone acetals of Formulas LXXIIa or LXXIIb.

Equation 52

(a)

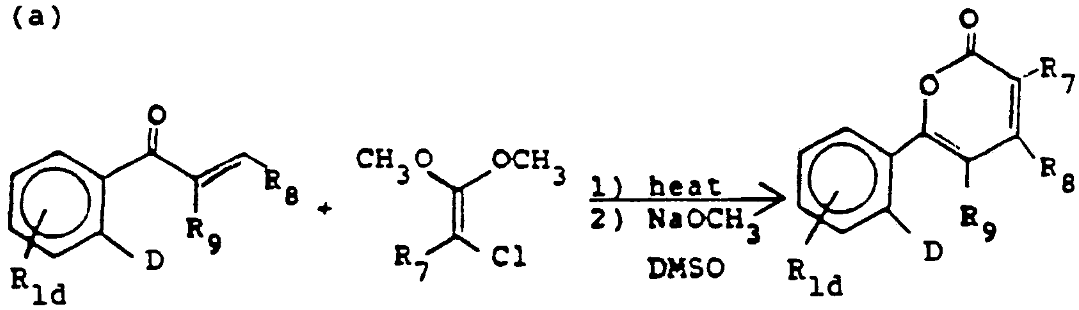


LXXIa LXXIIa LXXa

(b)

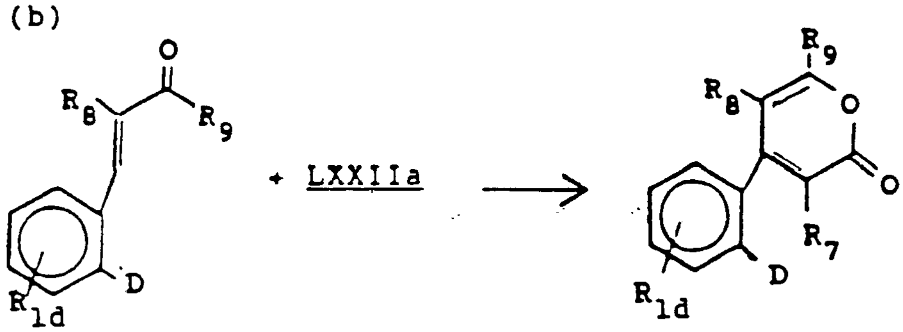


LXXIb LXXb

(c)

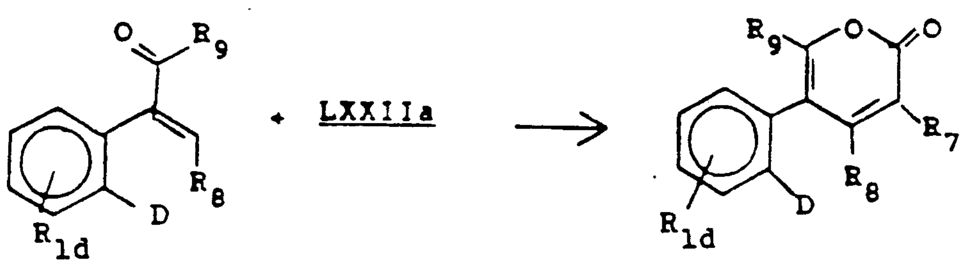


LXXIc LXXc

(d)

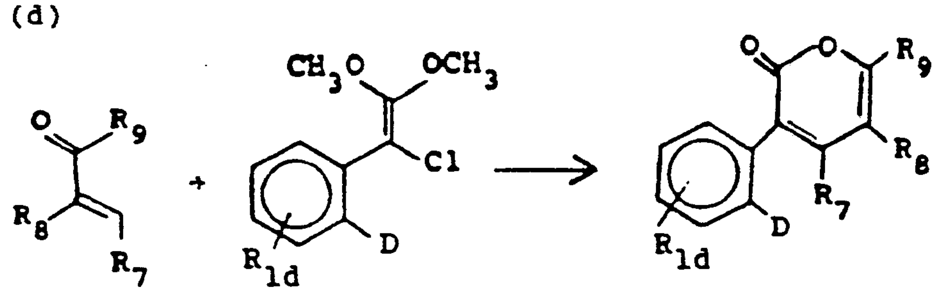

LXXId LXXIIb LXXd

wherein

$R_{1d}$, $R_7$, $R_8$ and $R_9$ are as previously defined, D is Br, $NO_2$, or $SR_{12}$, and $R_{12}$ is $C_2$—$C_4$ alkyl or benzyl.

The transformations outlined in Equation 52 can be conveniently carried out by the procedure of A. Belanger and P. Brassard, *Chem. Comm.,* 863 (1972). Other syntheses of α-pyrones are known in the literature and have been reviewed by J. Fried in "Heterocyclic Compounds", ed. R. C. Elderfield, Wiley, New York, 1950, Vol. I, pp. 358—370, and L. F. Cavalieri, *Chem. Rev., 41,* 525 (1947).

The requisite α,β-unsaturated carbonyl compounds of Formulas LXXIa—LXXId and the α-chloroketone acetals of Formula LXXIIb can be prepared by methods known to one skilled in the art.

The γ-pyrones of Formulas LXXIIIa and LXXIIIb can be prepared by one or more of several methods which have been reviewed by J. Fried in "Heterocyclic Compounds", ed. R. C. Elderfield, Wiley, New York, Vol I, pp. 379—391. A more recent procedure involves the reaction of suitable potassium enolates of Formulas LXXIVa and LXXIVb with the appropriate acid chlorides of Formulas LXXVa and LXXVb, as shown in Equation 53.

Equation 53

(a)

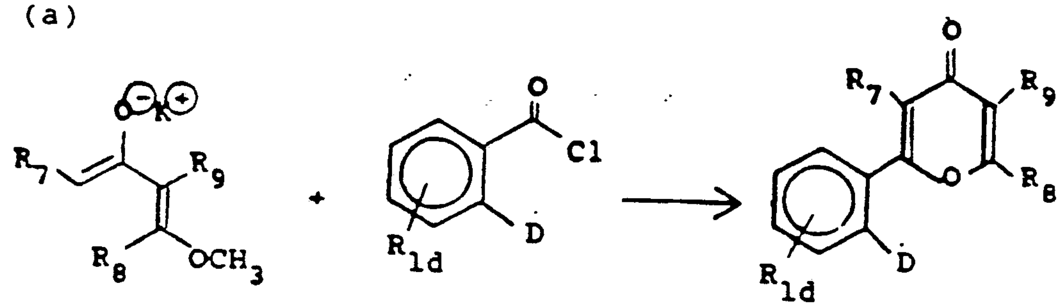

LXXIVa LXXVa LXXIIIa

(b)

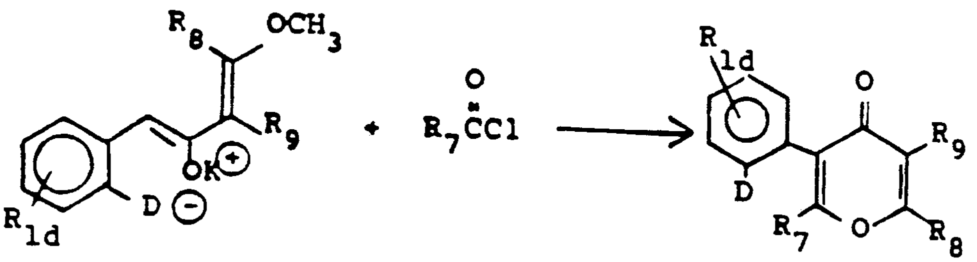

LXXIVb LXXVb LXXIIIb

wherein

$R_{1d}$, $R_7$, $R_8$ and $R_9$ are as previously defined, D is Br, $NO_2$ or $SR_{12}$, and $R_{12}$ is $C_2$—$C_4$ alkyl or benzyl.

The reactions shown above in Equations 53(a) and 53(b) can be carried out according to the procedures described by T. A. Morgan and B. Ganem, *Tetrahedron Lett., 21,* 2773 (1980). For a closely-related method,

refer to M. Koreeda and H. Akagi, *Tetrahedron Lett.*, *21*, 1197 (1980).

The requisite potassium enolates of Formulas LXXIVa and LXXIVB can be most conveniently prepared by treatment of the appropriate unsaturated ketones of Formulas LXXVIa and LXXVIb with a suitable base such as potassium *tert*-butoxide as shown in Equation 54.

Equation 54

(a)

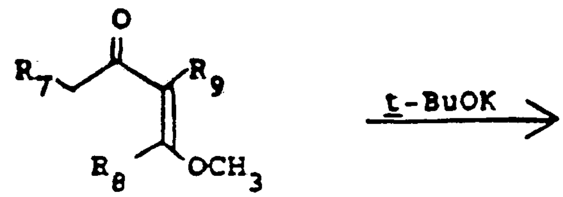


LXXIVa

LXXVIa

(b)

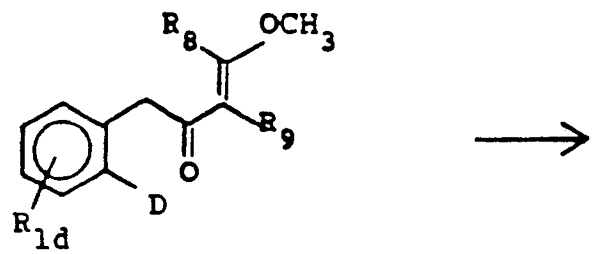

LXXIVb

LXXVIb

wherein

$R_{1d}$, $R_7$, $R_8$ and $R_9$ are as previously defined, D is Br, $NO_2$ or $SR_{12}$ and $R_{12}$ is $C_2$—$C_4$ alkyl or benzyl.

The reaction shown in Equation 54 can best be carried out according to the method described in Morgan and Ganem, *Tetrahedron Lett.*, *21*, 2773 (footnote 6) (1980).

The requisite unsaturated ketones of Formulas LXXVIa and LXXVIb, as well as the acid chlorides of Formula LXXVa, can be synthesized by methods known to one skilled in the art.

α-Pyridones of Formulas LXXVIIa—LXXVIId can be prepared in a straightforward manner by treatment of the appropriate α-pyrones of Formulas LXXa—LXXd with suitable amines, $H_2NR_{11}$, as represented below in Equations 55(a-d).

Equation 55

(a)

LXXa

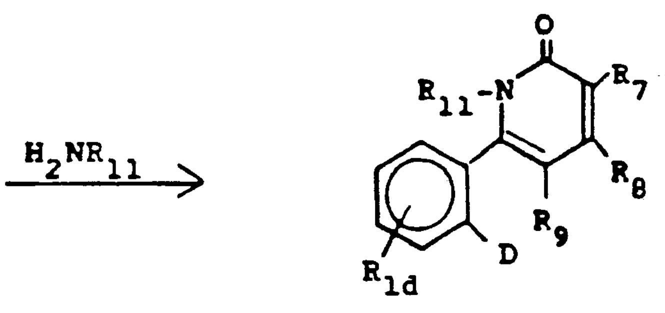


LXXVIIa

(b)

LXXb 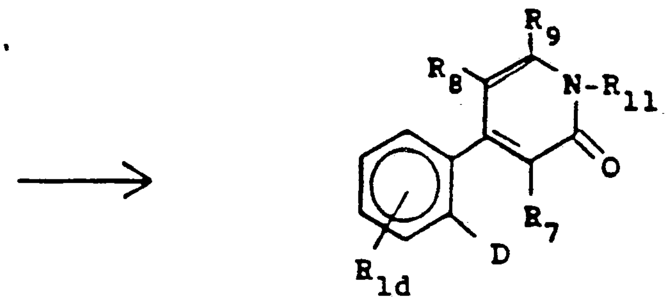

LXXVIIb

(c)

LXXc 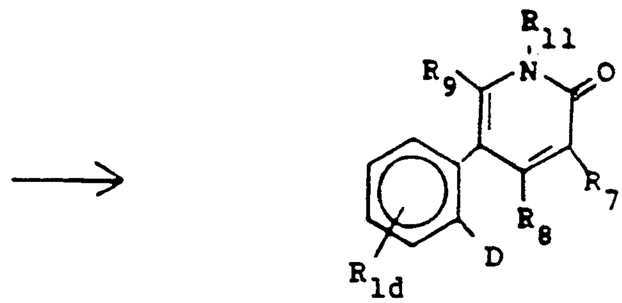

LXXVIIc

(d)

LXXd 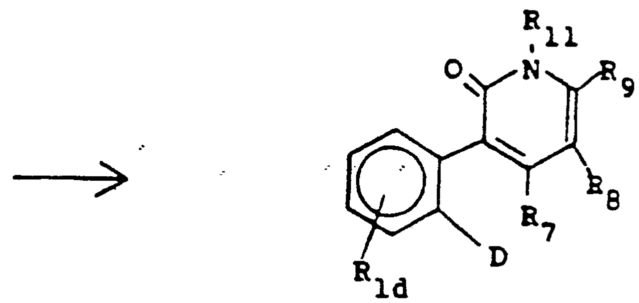

LXXVIId

wherein

$R_{1d}$, $R_7$, $R_8$, $R_9$ and $R_{11}$ are as previously defined, D is Br, $NO_2$ or $SR_{12}$, and $R_{12}$ is $C_2$—$C_4$ alkyl or benzyl.

The conversion of α-pyrones to the corresponding pyridones as shown in Equation 55 is a well-precedented process in the literature. For detailed descriptions of the procedure, see the following references: J. A. Leben, *Ber., 29,* 1673, (1896); von Pechmann and W. Welsh, *Ber., 17,* 2391 (1884); and J. H. Boyer and W. Schoen, *Org. Syntheses,* Coll. Vol. IV, 532 (1963).

γ-Pyridones of Formulas LXXXIa and LXXXIb can be synthesized from the corresponding γ-pyrones of Formulas LXXIIIa and LXXIIIb by treatment with the appropriate amines, $H_2NR_{11}$. This transformation is depicted in Equation 57.

Equation 57

(a) LXXIIIa 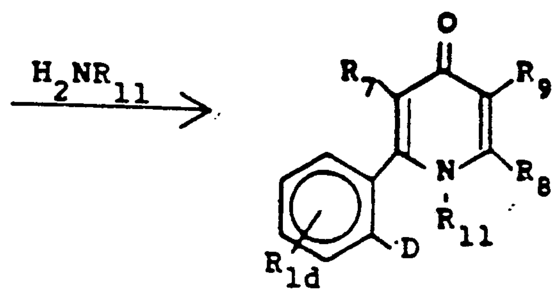


LXXXIa

(b) LXXIIIb

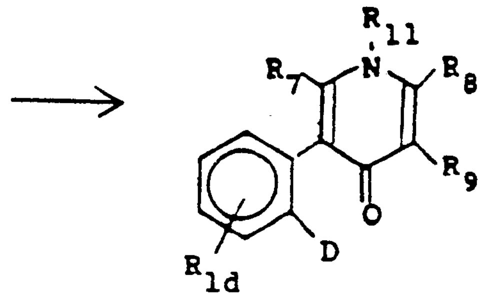

LXXXIb

wherein

$R_{1d}$, $R_7$, $R_8$, $R_9$ and $R_{11}$ are as previously defined, D is Br, $NO_2$ or $SR_{12}$, and $R_{12}$ is $C_2$—$C_4$ alkyl or benzyl.

The reactions of Equations 57(a) and 57(b) can be conveniently carried out as described by C. F. Rassweiler and R. Adams, *J. Am. Chem. Soc., 46,* 2758 (1924).

γ-Pyridones of Formula LXXXIc can be prepared as shown in Equation 58. Thus, addition of anions of Formula LXXIX to γ-pyrones of Formula LXXXII affords the desired products of Formula LXXXIc.

Equation 58

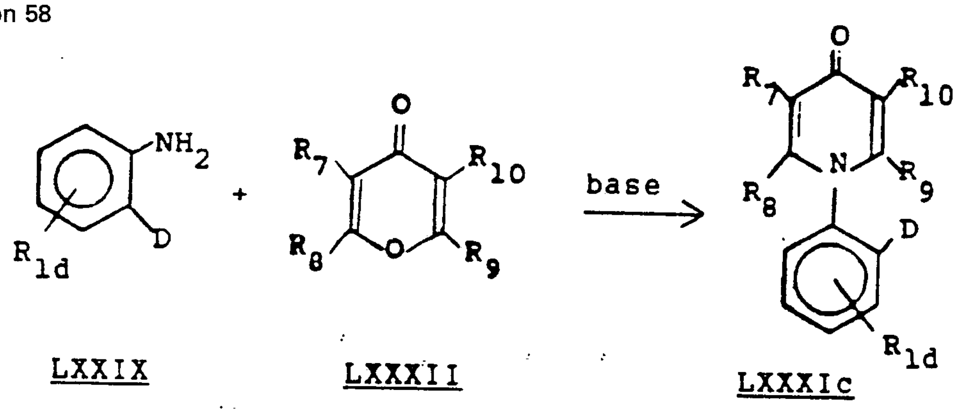


LXXIX LXXXII LXXXIc

wherein

$R_{1d}$, $R_7$, $R_8$, $R_9$ and $R_{10}$ are as previously defined, and D is Br, $NO_2$ or $SR_{12}$ and $R_2$ is $C_2$—$C_4$ alkyl or benzyl.

Compounds of Formula LXXXIIIa, where $R_8$ is H, can be prepared as shown in Equation 59 by the three-step sequence of reactions involving: (a) conversion of suitable protected carboxylic acids of Formula LXXXVI to the acid chlorides LXXXV, (b) treatment with diazomethane to give diazoketones of Formula LXXXIV, and (c) acid-induced cyclization.

Equation 59

(a)

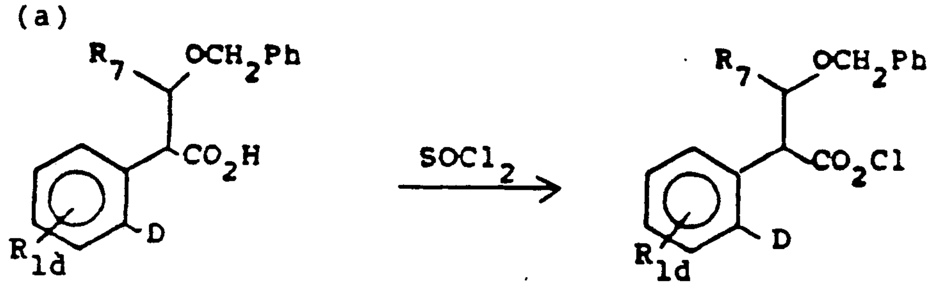


LXXXVI LXXXV

(b) LXXXV 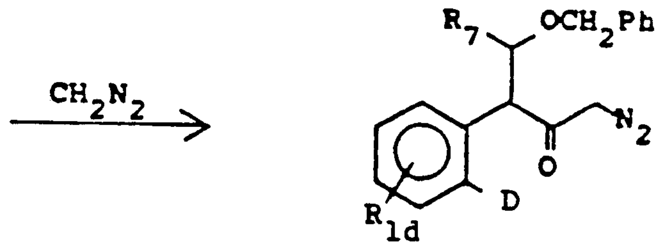


LXXXIV

(c) LXXXIV 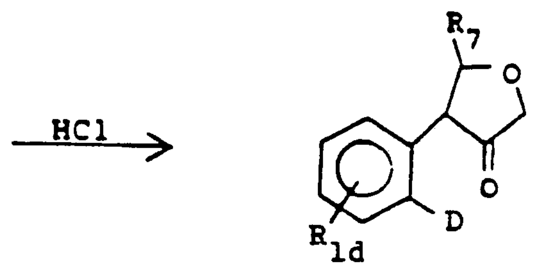


LXXXIIIa

wherein

$R_{1d}$ and $R_7$ are are as previously defined, D is Br, $NO_2$ or $SR_{12}$, and $R_{12}$ is $C_2$—$C_4$ alkyl or benzyl.

The transformations outlined in Equations 59(a-c) can be achieved according to the procedure of V. Luhmann and W. Luttke, *Chem. Ber., 105,* 1350 (1972).

Compounds of Formula LXXXIIIB can be prepared via an intramolecular epoxide opening reaction followed by oxidation of the resulting alcohol as depicted in Equation 60.

Equation 60

(a)

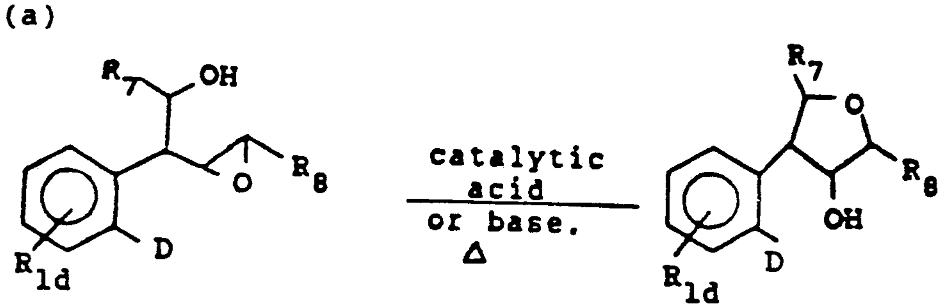


LXXXVIII LXXXVII

(b) LXXXVII 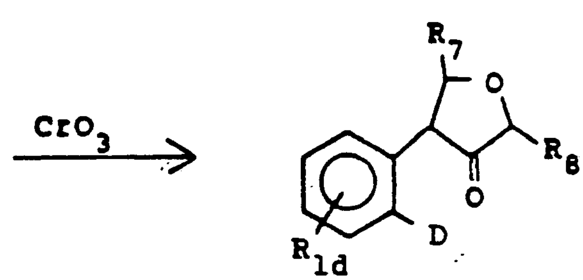


LXXXIIIb

wherein

$R_{1d}$, $R_7$ and $R_8$ are as previously defined, D is Br, $NO_2$ or $SR_{12}$, and $R_{12}$ is $C_2$—$C_4$ alkyl or benzyl.

Equation 60(a)

The reaction of Equation 60(a) involves the intramolecular alcoholysis of epoxides of Formula LXXXVIII, and can be accomplished by treatment with either a catalytic amount of a suitable base such as potassium *tert*-butoxide, or with a catalytic amount of a suitable acid such as sulfuric acid. For relevant procedures, refer to Chitwood and Freure, *J. Am. Chem. Soc., 68,* 680 (1946); Sexton and Britton, *ibid., 70* 3606 (1948); or Winstein and Henderson, *ibid., 65,* 2196 (1943).

Equation 60(b)

The oxidation of 3-hydroxytetrahydrofurans, such as those of Formula LXXXVII, to the corresponding furanones of Formula LXXXIIIb, can be accomplished with chromium trioxide as described by V. Luhmann and W. Luttke, *Chem. Ber., 105,* 1350 (1972).

Furanones of Formula LXXXIIIc can be synthesized as depicted in Equation 61 via a three-step process involving: (a) *o*-alkylation of the appropriate alcohols of Formula XCI, (b) Dieckmann cyclization to give α-carboethoxy furanones LXXXIX, and (c) hydrolysis and decarboxylation of esters LXXXIX with sulfuric acid.

Equation 61

(a)

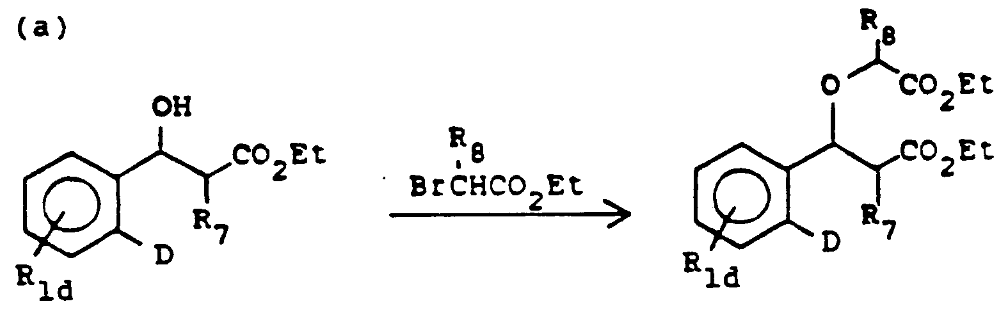


XCI

XC

(b) XC

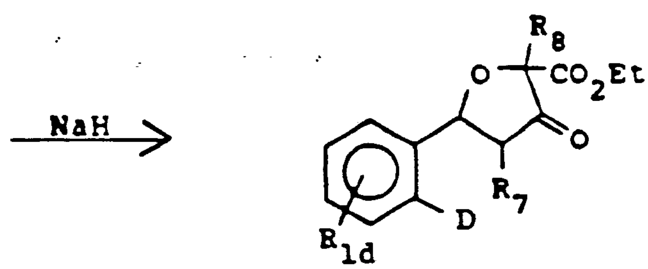


LXXXIX

(c) LXXXIX

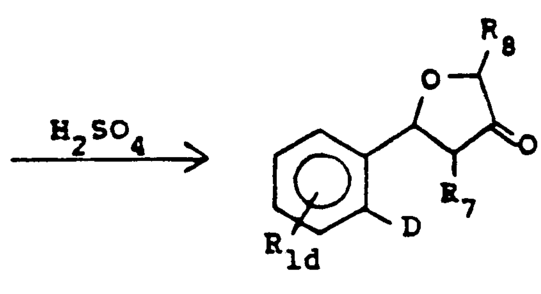


LXXXIIIc

wherein

$R_{1d}$, $R_7$ and $R_8$ are as previously defined, D is Br, $NO_2$ or $SR_{12}$, and $R_{12}$ is $C_2$—$C_4$ alkyl or benzyl.

The transformations outlined above in Equations 61(a-c) can be effectively accomplished by the procedure of V. Luhmann and W. Luttke, *Chem. Ber., 105,* 1350 (1972).

Furanones of Formula LXXXIIId can be prepared as shown in Equation 62 by addition of anions of Formula XCIII to the appropriate aldehydes XCIV, and subsequent acid-induced cyclization of the resultant α-hydroxyenones of Formula XCII.

Equation 62

(a)

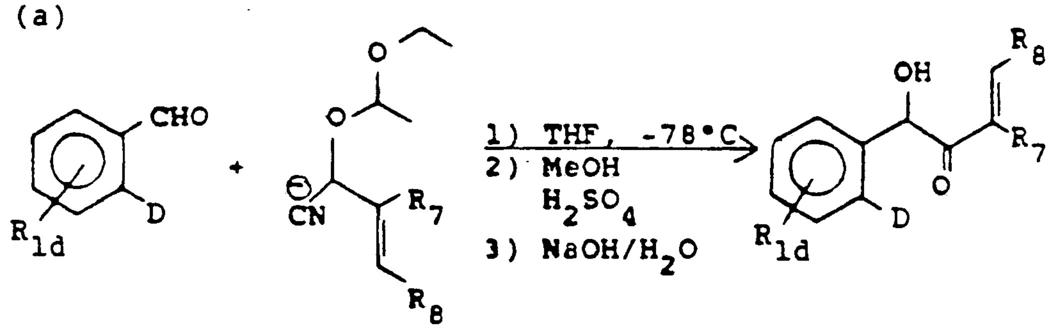


XCIV XCIII XCII

(b) XCII

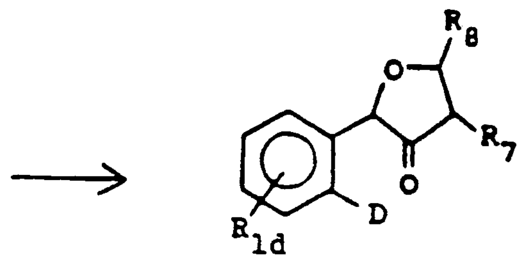

LXXXIIId

wherein

$R_{1d}$, $R_7$ and $R_8$ are as previously defined, D is Br, $NO_2$ or $SR_{12}$, and $R_{12}$ is $C_2$—$C_4$ alkyl or benzyl.

Equation 62(a)

The addition of protected cyanohydrin anions of Formula XCIII to aldehydes of Formula XCIV and subsequent hydrolysis to afford α-hydroxyenones of Formula XCII can be carried out according to the procedure of G. Stork and L. Maldonado, *J. Am. Chem. Soc., 93,* 5286 (1971). Also, refer to Stork and Maldonado, *ibid., 96,* 5272 (1974).

Equation 62(b)

The cyclization of Equation 62(b) can be carried out by heating a solution of the hydroxyenone of Formula XCII in a suitable solvent such as tetrahydrofuran in the presence of a catalytic amount of an appropriate acid such as hydrochloric or sulfuric acid. Alternatively, compounds of Formula XCII can be heated in a suitable solvent such as toluene at reflux temperature in the presence of a catalytic amount of *p*-toluenesulfonic acid.

Compounds of Formulas LXXXIIIe—LXXXIIIh, which are homologs of furanones LXXXIIIa—LXXXIIId, can be prepared by methods analogous to those described above in Equations 59, 60, 61 and 62. The minor modifications needed to implement these syntheses would be obvious to one skilled in the art. Equations 63(a)-63(d) depict the procedures for the preparation of compounds LXXXIIIe-LXXXIIIh.

Equation 63

(a)

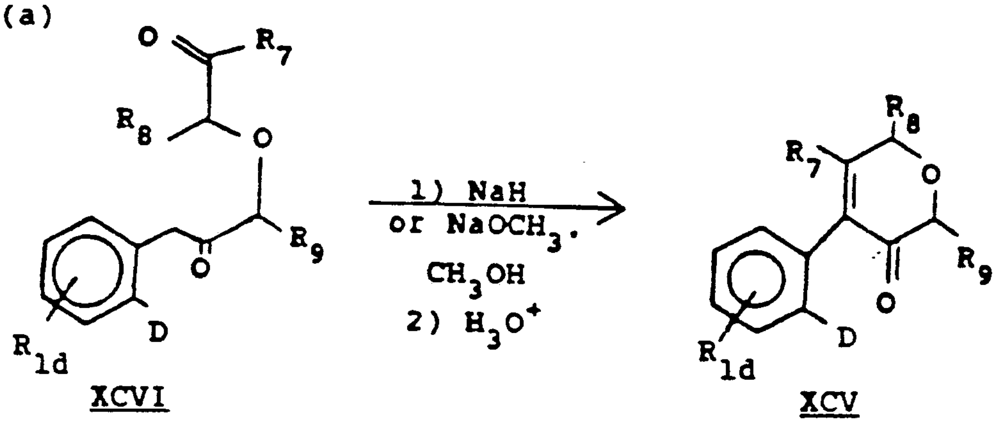


XCVI XCV

XCV

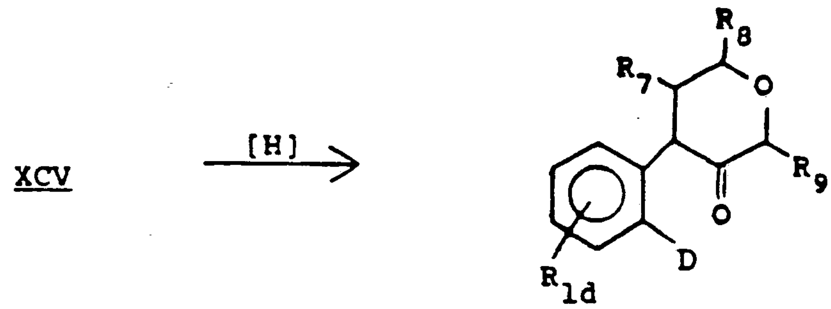


LXXXIIIf

(b)

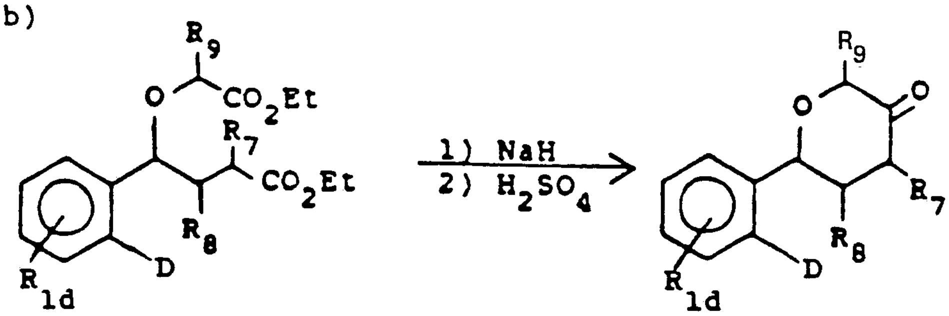


XCVII LXXXIIIf

(c)

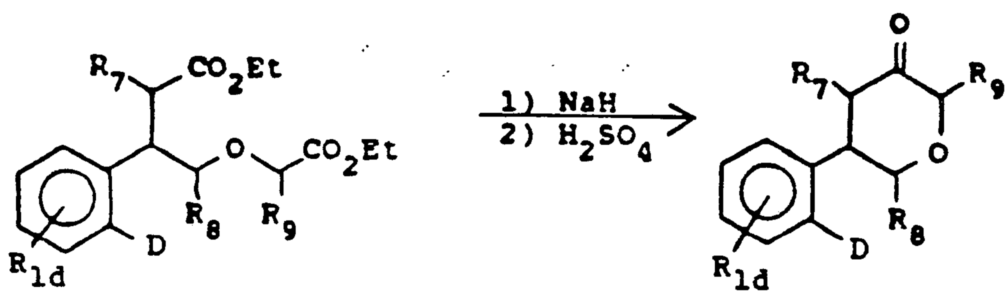


XCVIII LXXXIIIg

(d)

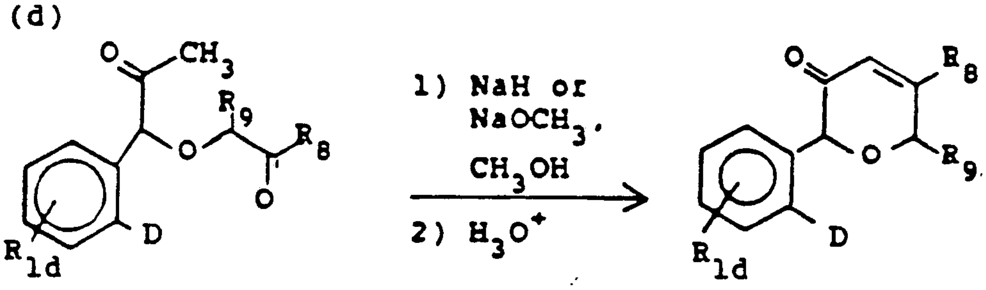


C XCIX

XCIX

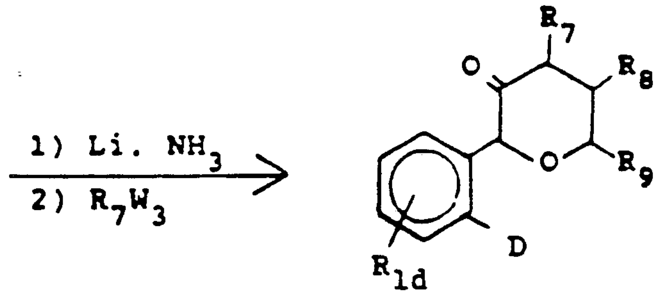


LXXXIIIh

wherein

$R_{1d}$, $R_7$, $R_8$ and $R_9$ are as previously defined, D is Br, $NO_2$ or $SR_{12}$, $R_2$ is $C_2$—$C_4$ alkyl or benzyl, and $W_3$ is Cl, Br, or I.

Equation 63(a)

The first step of Equation 63(a) is an intramolecular aldol condensation and can be carried out in a manner analogous to that described for Equation 25(b). The second step of Equation 63(a) involves a selective 1,4-reduction of an α,β-unsaturated ketone and can best be achieved by methods described in Equation 25(c).

Equations 63(b)-63(d)

For a description of the procedures for carrying out the reactions of Equations 63(b)-63(d), see the reference cited for Equation 61. The reductive alkylation process shown in the second step of Equation 63(d), whereby unsaturated ketones of Formula XCIX are converted to the desired products of Formula LXXXIIIh, can be conveniently effected according to the method of V. I. Mel'nikova and K. K. Pivnitskii, *J. Org. Chem., USSR (Engl. Trans.)*, *6*, 2635 (1970).

The pyrrolidones of Formulas CIa-CId can be synthesized as shown in Equation 64 via hydroboration/oxidation of the appropriate $\Delta^2$-pyrrolines of Formulas CIIa—CIIb.

Equation 64

(a)

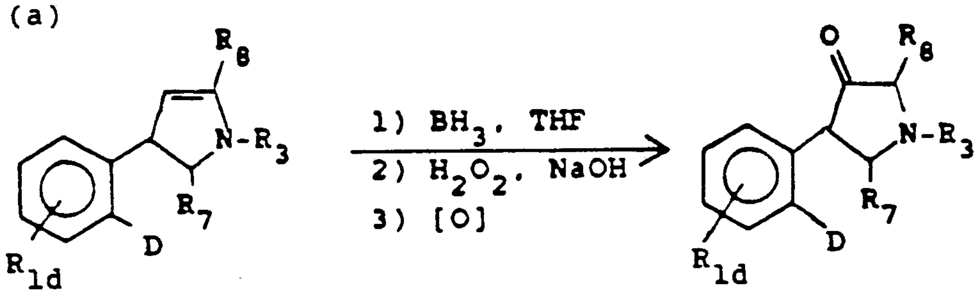


CIIa CIa

(b)

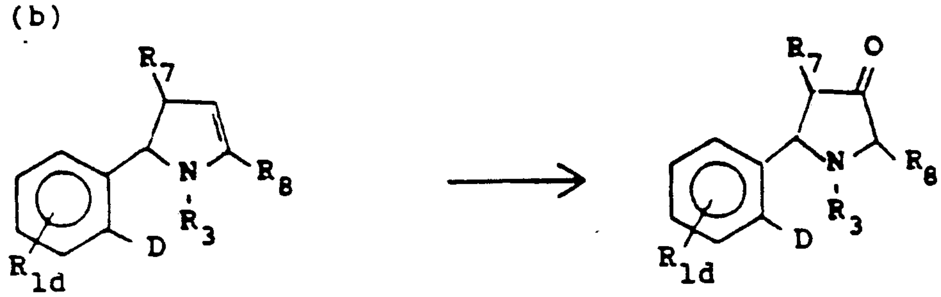

CIIb CIb

(c)

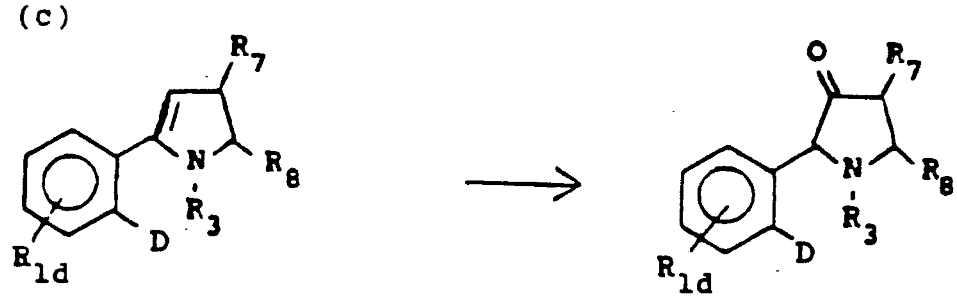

CIIc CIc

(d)

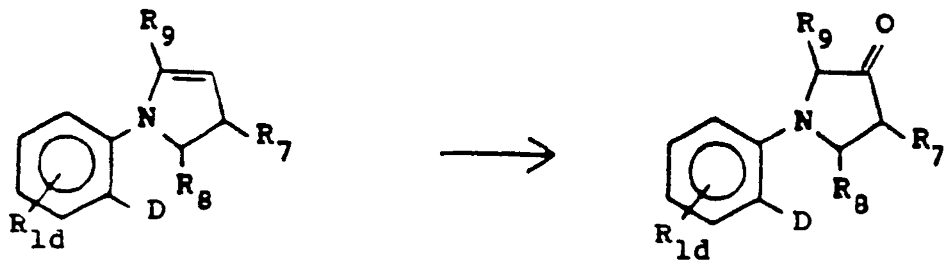

CIId CId

wherein

$R_{1d}$, $R_3$, $R_7$, $R_8$ and $R_9$ are as previously defined, and D is Br or $NO_2$.

The reactions of Equations 64(a)—(d) can be conveniently carried out according to the method of I. J. Borowitz and G. J. Williams, *J. Org. Chem., 32,* 4157 (1967).

In a similar fashion, the compounds of Formulas CIe—CIi can be prepared from the appropriate enamines of Formulas CIIIa—CIIIe as represented in Equations 65 (a—e).

Equation 65

(a)

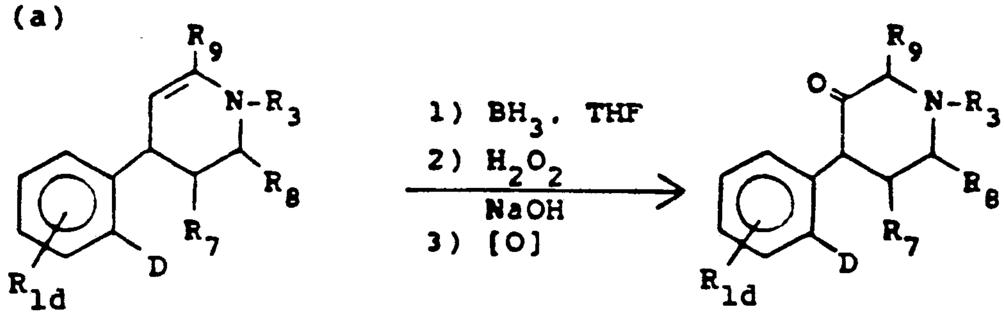


CIIIa CIe

(b)

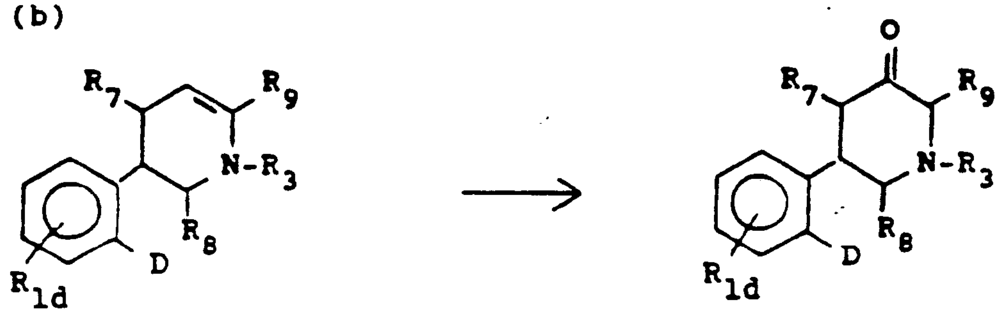

CIIIb CIf

(c)

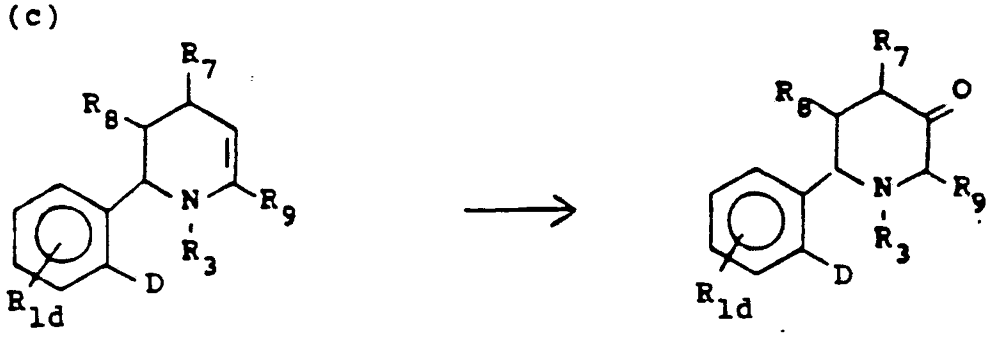

CIIIc CIg

(d)

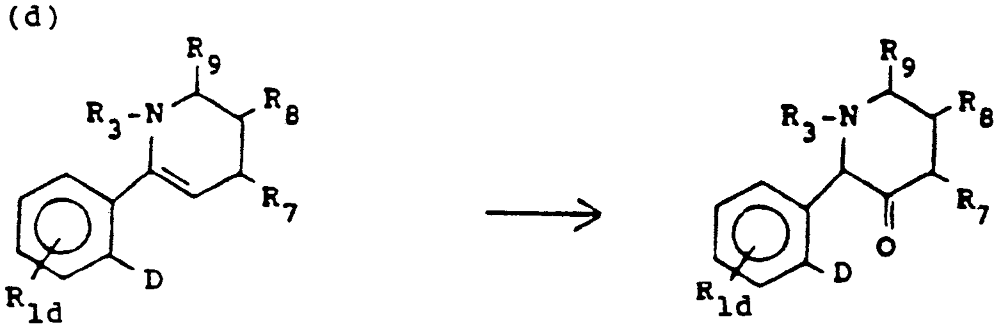

CIIId CIh

(e)

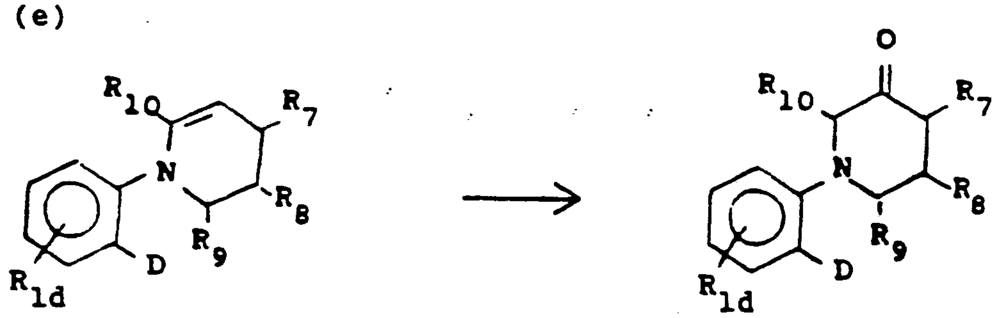

CIIIe CIi

wherein

$R_{1d}$, $R_3$, $R_7$, $R_8$, $R_9$ and $R_{10}$ are as previously defined, and D is Br or $NO_2$.

The requisite compounds of Formulas CIIa—CIId and CIIIa—CIIIe can be prepared by the method shown in Equation 66. For example, treatment of the appropriate compounds of Formulas CIVa or CIVb with suitable amines, $H_2NR_3$, affords the desired pyrrolines or tetrahydropyridines of Formulas CIIa and CIIIa, respectively.

Equation 66

(a)

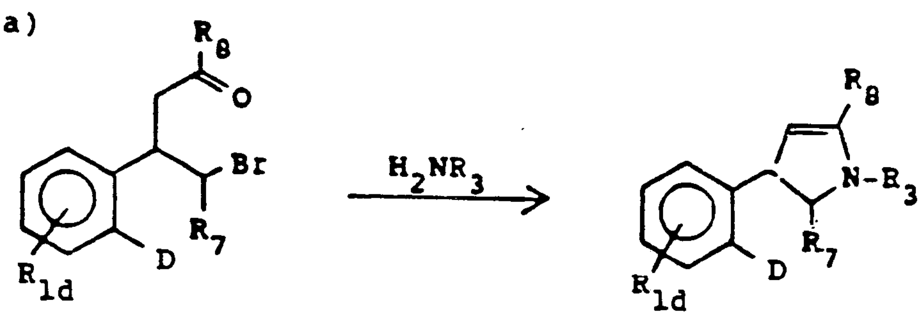


CIVa CIIa

(b)

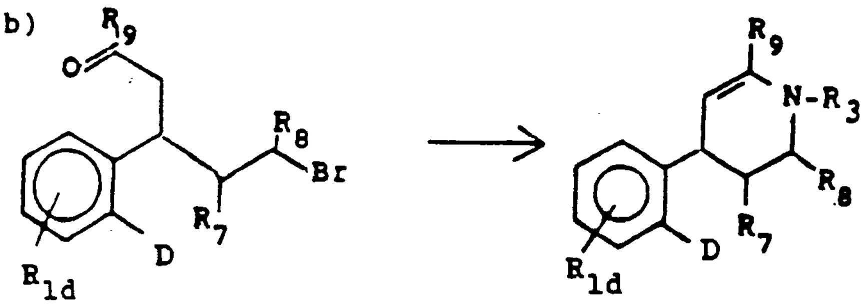

CIVb CIIIa

wherein

$R_{1d}$, $R_3$, $R_7$, $R_8$ and $R_9$ are as previously defined, and D is Br or $NO_2$.

The reactions of Equations 66(a) and 66(b) can be conveniently carried out according to procedures described in the following references: J. Cloke, *J. Am. Chem. Soc., 51,* 1174 (1929); A. Wohl, *Ber., 34,* 1914 (1901); A. Kipp, *Ber., 18,* 3284 (1895), and *25,* 2190 (1892); and S. Gabriel, Ber., *41,* 2010 (1908).

The transformations depicted above in Equation 66 can be applied in a straightforward manner to the synthesis of the related compounds of Formulas CIIB—CIId and CIIIb—CIIIe.

The α,β-unsaturated compounds of Formulas CVa can be prepared by the same type of process described above in Equations 33 and 34 to synthesize unsaturated lactones of Formula XLIII. Thus, treatment of compounds CIe with a kinetic base such as lithium diisopropylamide (LDA) will generate the corresponding enolates of Formula CVI. Trapping with diphenyl disulfide or phenylsulfenyl chloride, and subsequent oxidative elimination gives the desired compounds of Formula CVa, as shown in Equation 67.

Equation 67

CIe

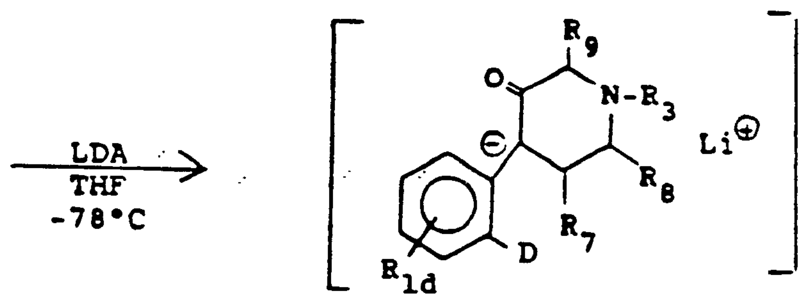


CVI

[CVI]

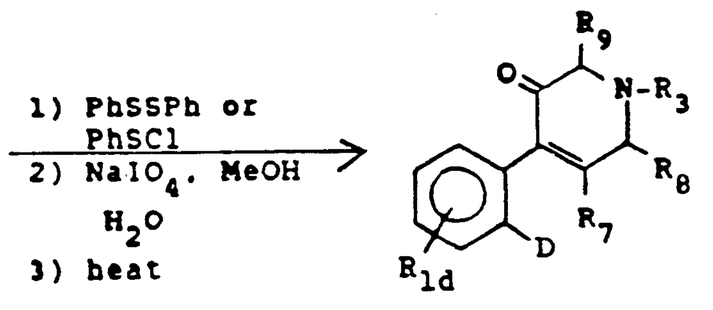


CVa

wherein

$R_{1d}$, $R_3$, $R_7$, $R_8$ and $R_9$ are as previously defined, and D is Br or $NO_2$, or $SO_2NH$-*t*-Bu.

It should be noted in Equation 67 that for compounds of Formula CIe, where $R_3$ is H, one extra molar equivalent of base must be employed to generate the dianions. For a procedure describing the formation of enolates of 3-piperidones such as those of Formulas CIe—CIi, see McElvain, *J. Amer. Chem. Soc., 55,* 1233 (1933), and McElvain and Vozza, *ibid., 71,* 896 (1949).

The procedure shown in Equation 67 can be quite easily applied to compounds of Formulas CIf—CIi. In this manner, the corresponding α,β-unsaturated derivatives can be synthesized and further transformed into the appropriate sulfonamides of Formula IVa or IVb, where Q is Q—128, Q—129, Q—131, and Q—130.

In an analogous fashion, compounds of Formulas LXXXIIIe—LXXXIIIh can be treated according to the procedures described in Equations 33 and 34 to give the corresponding α,β-unsaturated derivatives.

Further elaboration of these compounds then affords the primary sulfonamides of Formula IVa or IVb, where Q is Q—123, Q—125, Q—124, and Q—126.

Compounds of Formulas CVIIa and CVIIb can be conveniently prepared as shown in Equation 66(a and b) by a Dieckmann cyclization of the appropriate sulfides of Formulas CVIIIa and CVIIIb, followed by hydrolysis and decarboxylation.

Equation 68

(a)

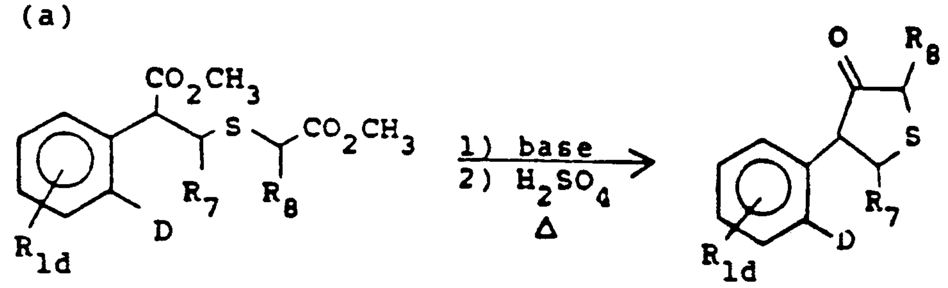


CVIIIa CVIIa

(b)

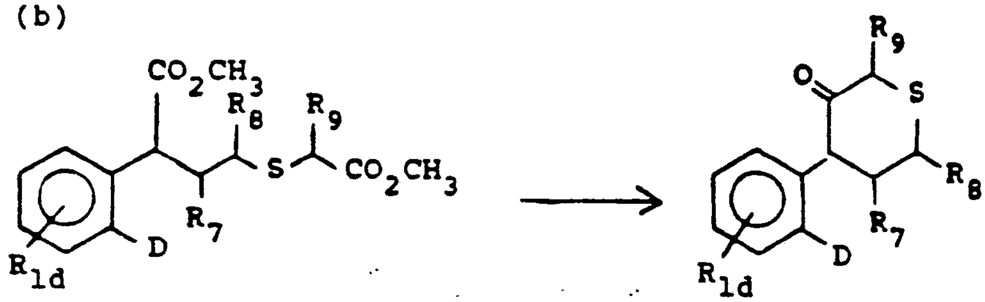

CVIIIb CVIIb

wherein

$R_{1d}$, $R_7$, $R_8$ and $R_9$ are as previously defined, and D is Br, $NO_2$, or $SO_2NH$-*t*-Bu.

The reactions of Equations 68(a) and 68(b) can be carried out according to the procedure of Woodward and Eastman, *J. Am. Chem. Soc., 68,* 2229 (1946), and Woodward and Eastman, *ibid., 66,* 849 (1944). For a review of syntheses of these ring systems, see Wolf and Folkers, *Org. Reactions,* Vol. *6,* 1951, pp. 443—468.

In a similar fashion, the 3-ketothiolanes of Formulas CVIIc—CVIIg can be prepared via Dieckmann cyclization of the appropriate sulfides of Formulas CVIIIc—CVIIIg as described below in Equations 69 (a—e).

Equation 69

(a)

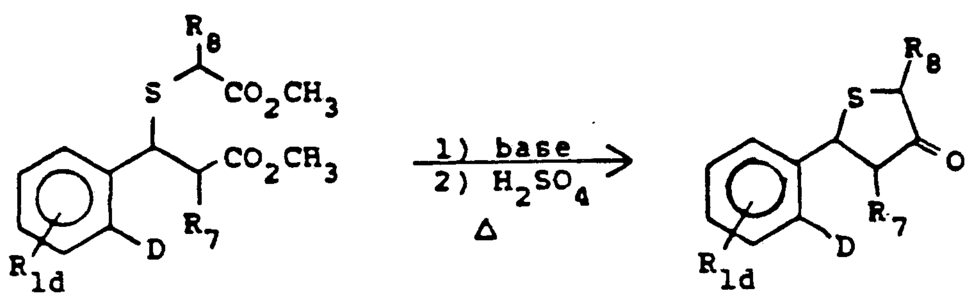


CVIIIc CVIIc

(b)

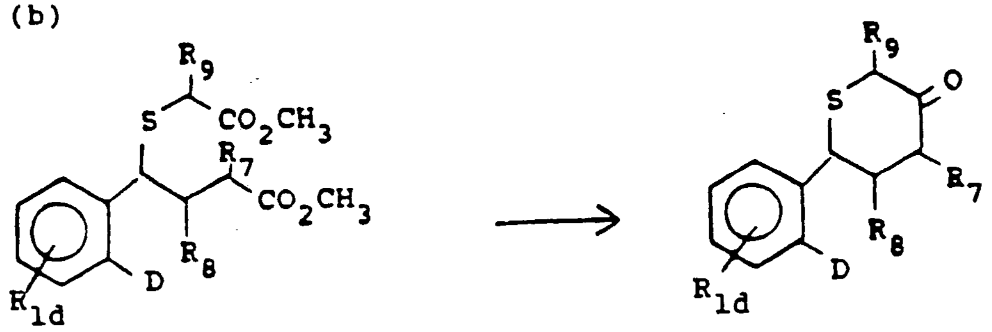

CVIIId CVIId

(c)

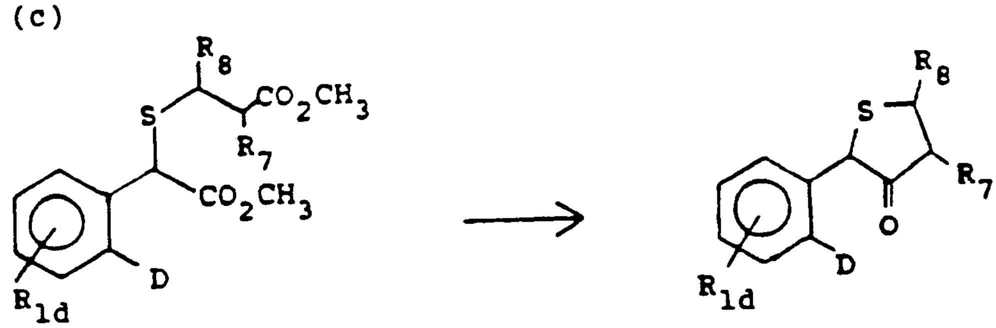

CVIIIe CVIIe

(d)

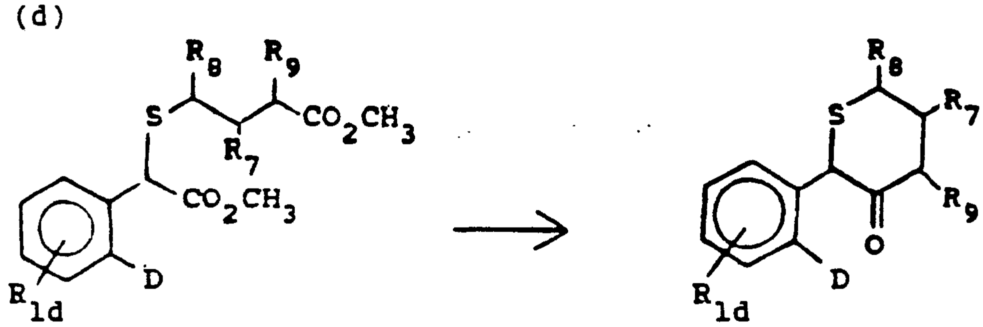

CVIIIf CVIIf

(e)

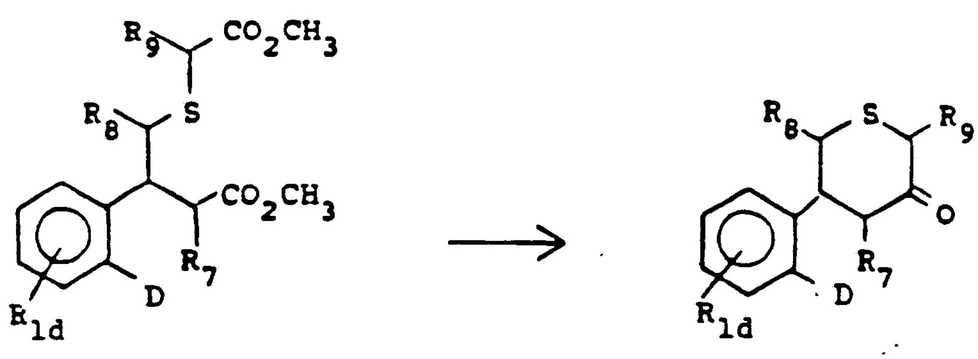

CVIIIg CVIIg

wherein

$R_{1d}$, $R_7$, $R_8$ and $R_9$ are as previously defined, and D is Br, $NO_2$, or $SO_2NH$-*t*-Bu.

The requisite sulfides of Formulas CVIIIa—CVIIIg can be prepared by the reaction of appropriate alkyl bromides of Formula CIX with substituted mercaptans of Formula CX. Equation 70 shows this reaction for the example CVIIIa.

Equation 70

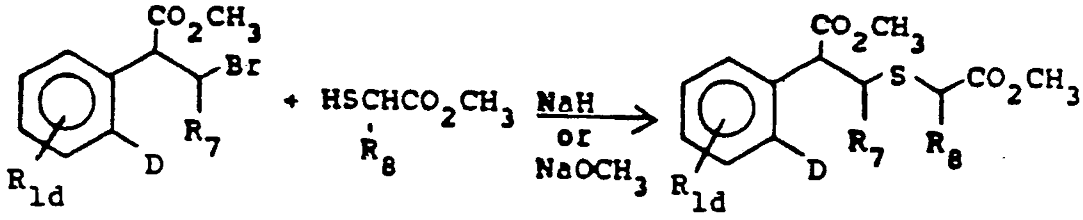


CIXa CXa CVIIIa

wherein

$R_{1d}$, $R_7$ and $R_8$ are as previously defined, and D is Br, $NO_2$, or $SO_2NH$-*t*-Bu.

The alkylation of mercaptans such as those of Formula CXa with alkyl halides is a well-known process with considerable precedent in the literature. For relevant references, see Shriner, Struck and Jorison, *J. Am. Chem. Soc., 52,* 2066 (1930); Kirner and Richter, *ibid., 51,* 3135 (1929); Kipnis and Ornfelt, *ibid., 71,* 3571 (1949) and Fehnel and Carmack, *ibid., 71,* 92 (1949).

By applying the procedures described above in Equation 70, one skilled in the art can prepare the requisite sulfides of Formulas CVIIIb—CVIIIg from the appropriate alkyl bromides and mercaptans.

In an analogous manner, the requisite ethers of Formulas XC, XCVI, SCVII, XCVIII and C can be prepared via treatment of the appropriate alkyl bromides of Formula CXI with the sodium salt of the appropriate alcohols of Formula CXII. This reaction is shown below in Equation 71 for the synthesis of compounds XCVI as a representative example.

Equation 71

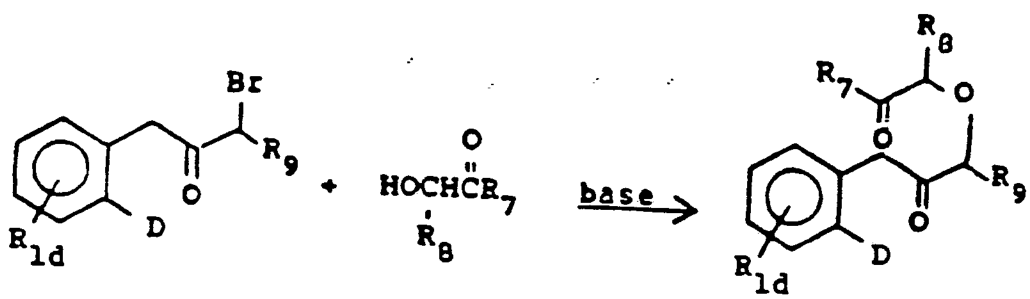


CXIa CXIIa XCVI

wherein

$R_{1d}$, $R_7$, $R_8$ and $R_9$ are as previously defined, D is Br, $NO_2$ or $SR_{12}$ and $R_2$ is $C_2$—$C_4$ alkyl or benzyl.

For a compilation of references dealing with the reaction of Equation 71, see R. B. Wagner and H. D. Zook, "Synthetic Organic Chemistry", John Wiley and Sons, Inc., New York, 1953, pp. 226—228.

Unsaturated sulfones of Formulas CXIIIa and CXIIIb can be prepared as shown in Equation 72 by a three-step sequence of reactions involving: (1) addition of the appropriate organometallic reagents, $R_8M$ or $R_7M$, to compounds of Formula CVIIa or CVIIb, (2) oxidation of the adducts of Formula CXVa or CXVb, and (3) dehydration of the sulfones CXVIa or CXVIb to the corresponding products of Formula CXIIIa or CXIIIb.

Equation 72

(a)

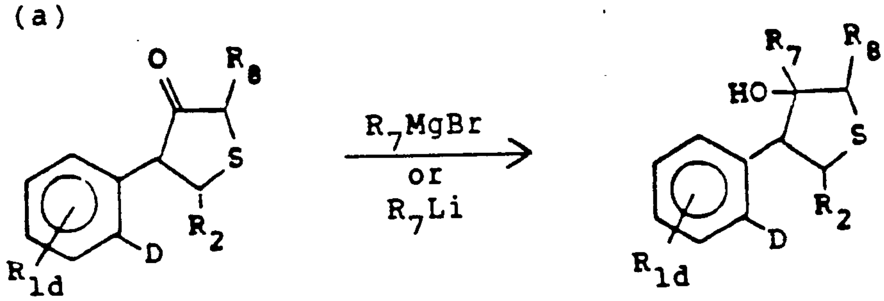


CVIIa CXVa

CXVa

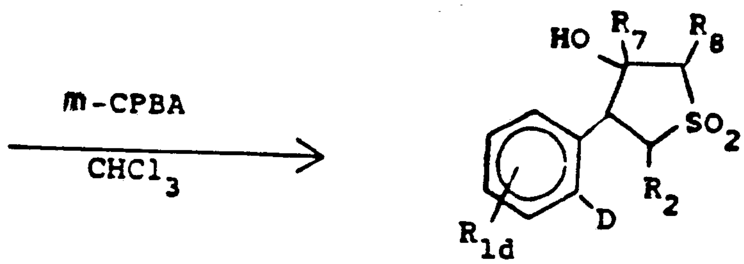


CXVIa

CXVIa

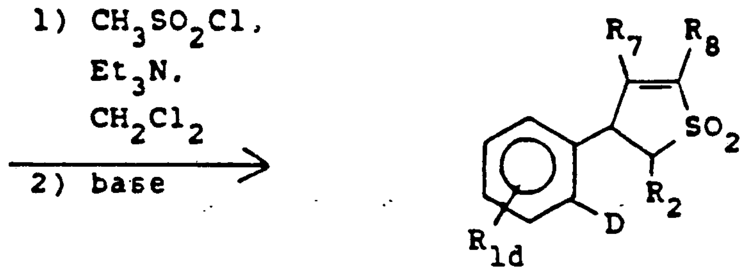


CXIIIa

(b)

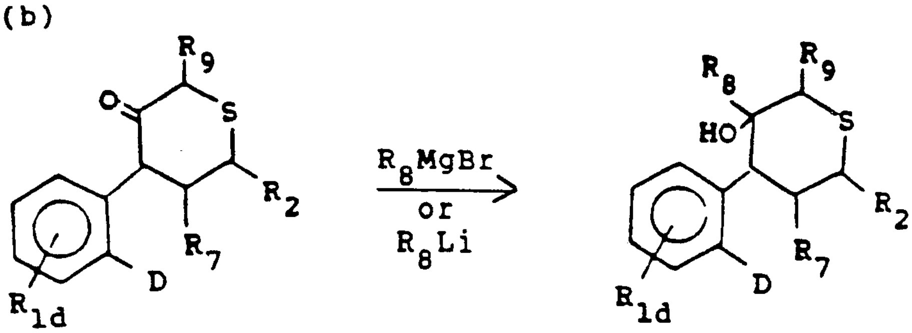


CVIIb CXVa

CXVb

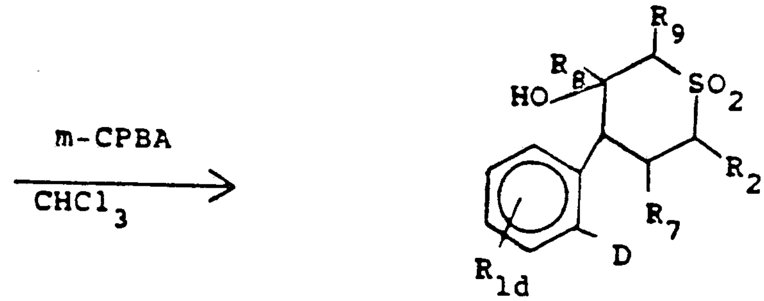


CXVIb

CXVIb

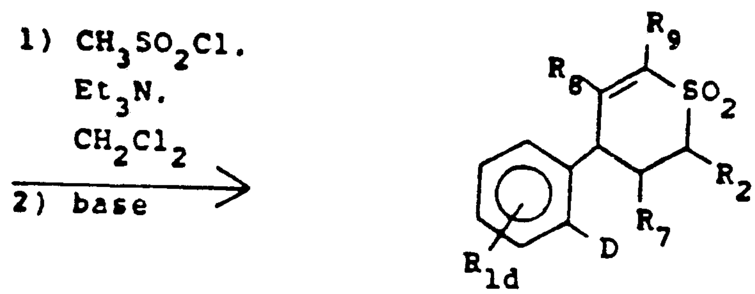


CXIIIb

wherein

$R_{1d}$, $R_2$, $R_7$, $R_8$ and $R_9$ are as previously defined and D is Br or $NO_2$.

The transformations shown above in Equations 72(a) and 72(b) can be effected according to the method of T. Takaya, *et al., Bull. Chem. Soc. Japan, 41,* 2086 (1968).

In a similar fashion, compounds of Formulas CVIIc—CVIIg can be treated according to the procedures described above in Equations 72(a) and 72(b) to afford the corresponding unsaturated sulfones. Further elaboration of the unsaturated sulfones derived from the compounds of Formulas CVIIa—CVIIg by methods described previously yields the primary sulfonamides of Formula IVa or IVb, where Q is Q—49, Q—120, Q—50, Q—118, Q—47, Q—122, and Q—119.

The unsaturated sulfones of Formulas CXIIIc and CXIIId can be prepared as shown in Equation 73 by: (1) addition of the dianions of N-*t*-butylbenzene sufonamides XX to the appropriately substituted compounds of Formulas CXVIIa and CXVIIb, and (2) oxidation and dehydration of the resultant adducts of Formulas CXVc and CXVd.

Equation 73

(a)

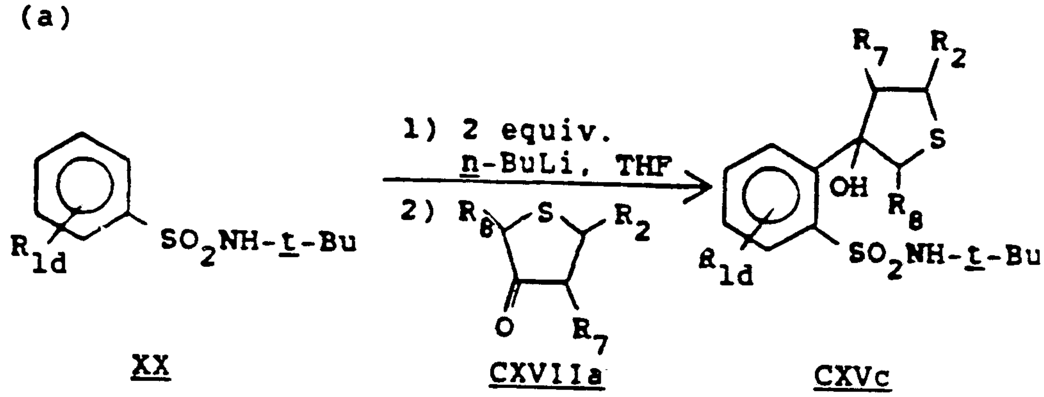


XX CXVIIa CXVc

XCVc

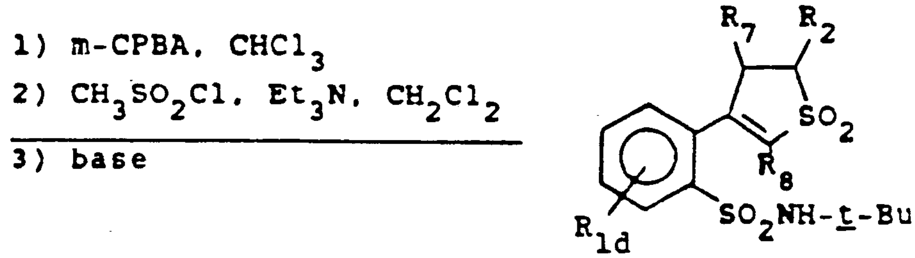


CXIIIc

(b)

XX

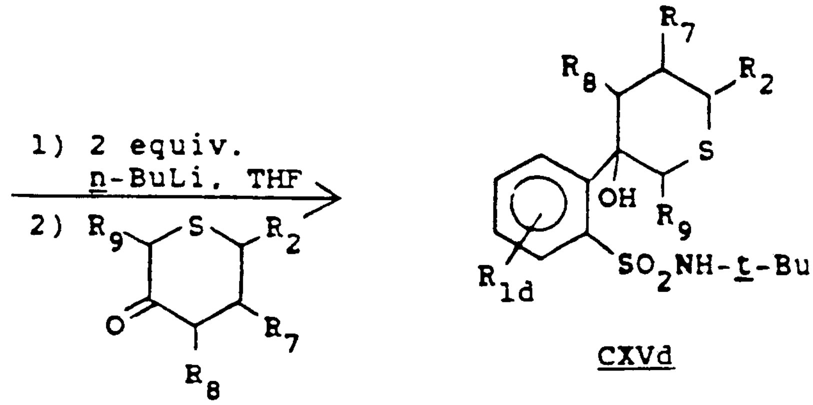


CXVIIb

CXVd

CXVd

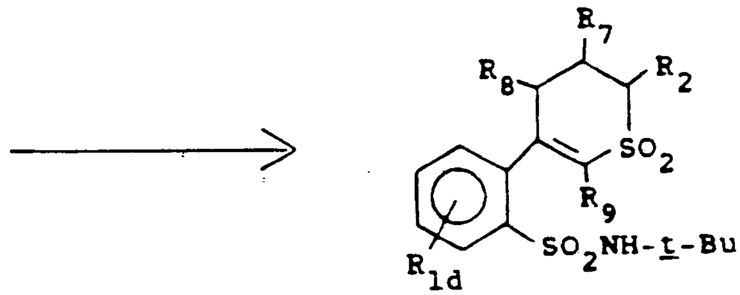

CXIIId

wherein

$R_{1d}$, $R_2$, $R_7$, $R_8$ and $R_9$ are as previously defined.

The first step of the reaction of Equations 73(a) and 73(b) can be conveniently carried out according to the procedure of J. G. Lombardino, *J. Org. Chem., 36,* 1843 (1971). The second step shown in Equations 73(a) and 73(b) is accomplished by the method described in Equation 72.

Dihydrothiopyran-3-ones of Formulas CXVIIIa—CXVIIId can be synthesized as depicted below in Equations 74 (a—d) via a Dieckmann-type cyclization of the appropriate sulfides of Formulas CXIXa—CXIXd, and subsequent acid-induced hydrolysis and decarboxylation.

Equation 74

(a)

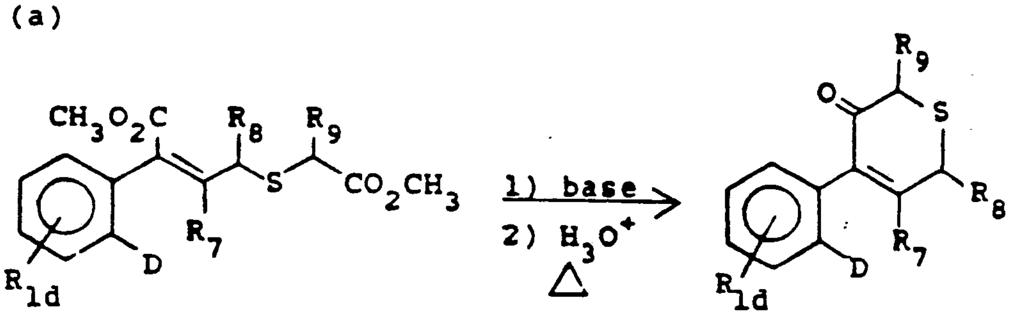


CXIXa

CXVIIIa

(b)

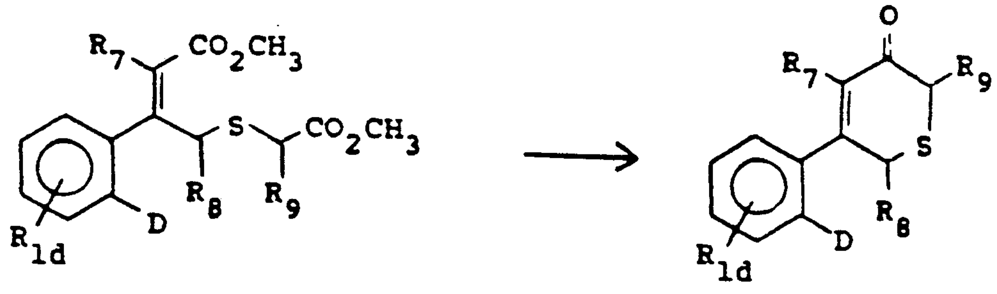

CXIXb CXVIIIb

(c)

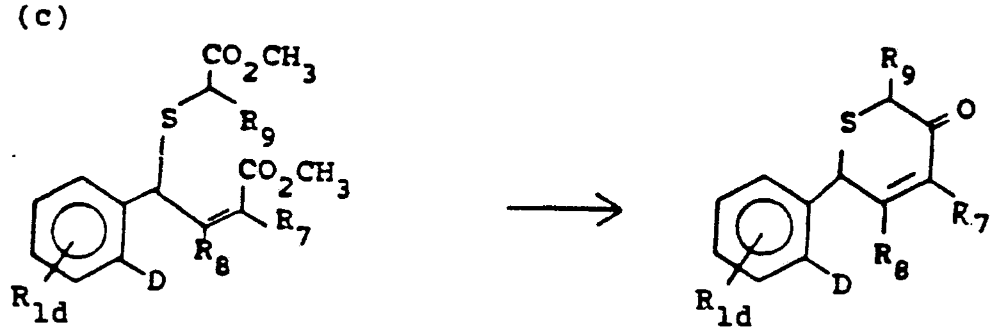

CXIXc CXVIIIc

(d)

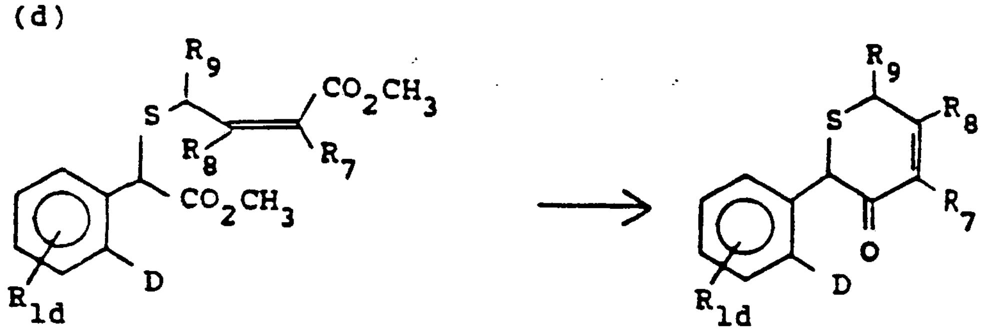

CXIXd CXVIIId

wherein

$R_{1d}$, $R_7$, $R_8$ and $R_9$ are as previously defined, and D is Br, $NO_2$ or $SO_2NH$-*t*-Bu.

The reaction of Equations 74(a—d) can be carried out according to the procedure of S. Rossi and G. Pagani, *Tetrahedron Lett.,* 2129 (1966).

The requisite sulfides of Formulas CXIXa—CXIXd can be prepared in a manner analogous to that described in Equation 70 for the preparation of compounds CVIIa.

Dihydropyrones of Formulas CXXa—CXXd can be conveniently prepared as shown in Equation 75 by reaction of the appropriate potassium enolates of Formulas CXXIa—CXXId with suitable acid chlorides of Formulas CXIIa—CXIIc.

Equation 75

(a)

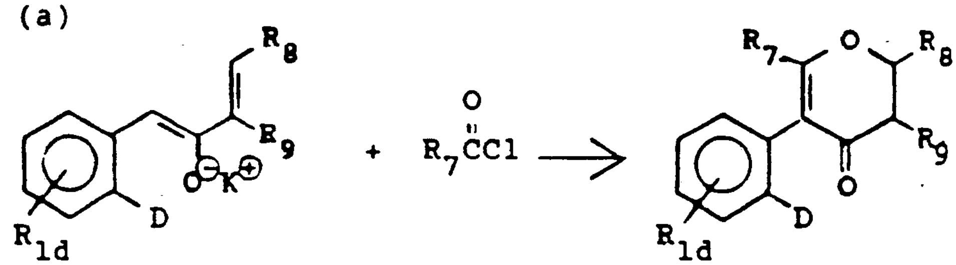

CXXIa CXXIIa CXXa

(b)

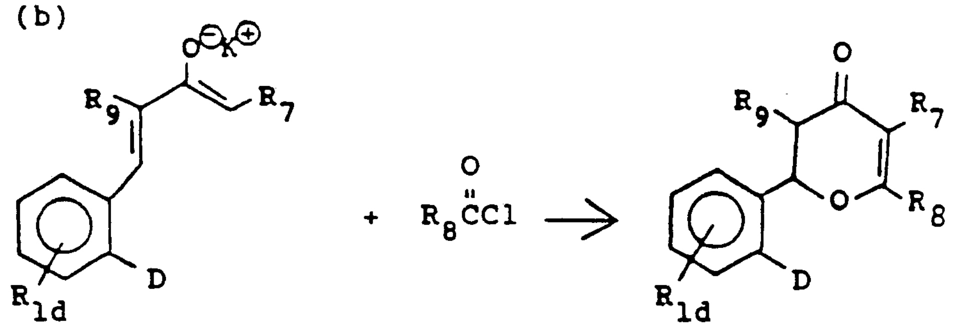

CXXIb CXXIIb CXXb

(c)

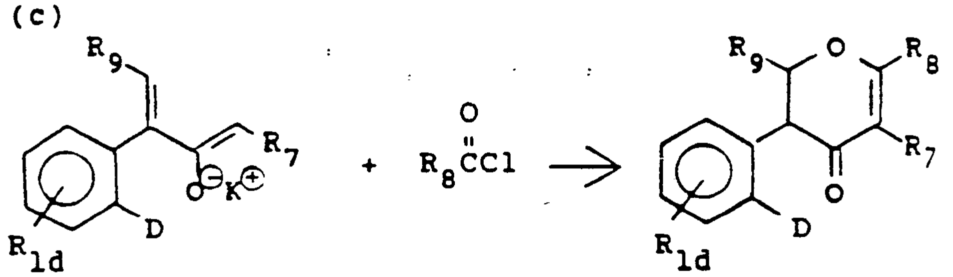

CXXIc CXXIIb CXXc

(d)

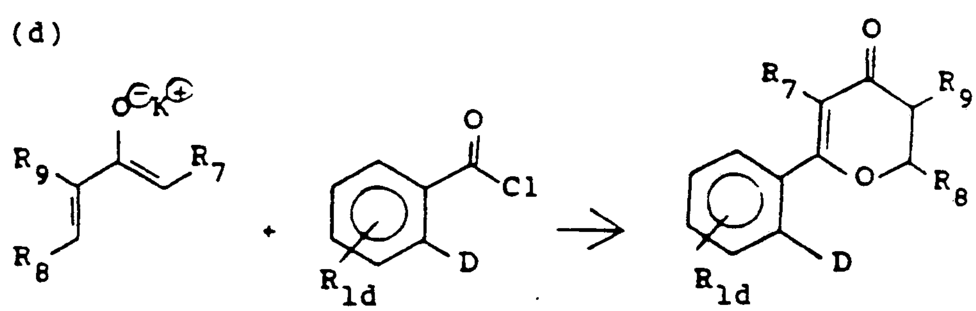

CXXId CXXIIc CXXd

wherein

$R_{1d}$, $R_7$, $R_8$ and $R_9$ are as previously defined, D is Br, $NO_2$ or $SR_{12}$, and $R_{12}$ is $C_2$—$C_4$ alkyl or benzyl.

The reactions of Equation 75 can be carried out in a manner analogous to that described for Equation 53.

An alternative method for the preparation of dihydropyrones of Formula CXXb above where $R_8$ is H involves the Lewis acid catalyzed hetero-Diels-Alder reaction of sulfonamide XCIV with dienes of Formula CXXe followed by mild hydrolysis as depicted in Equation 75e. Suitable Lewis acids include magnesium bromide, zinc chloride, and (6,6,7,7,8,8,8-heptafluoro-2,2-dimethyl-3,5-octanedionato)europium [Eu(fod)$_3$]. For typical procedures for carrying out the cyclocondensations, see M. Bednarski and S. Danishefsky, *J. Am. Chem. Soc.*, *105*, 5716 (1983).

Equation 75e

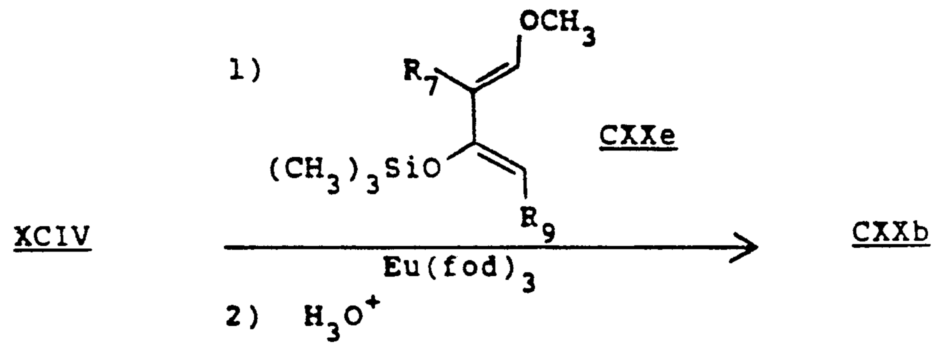


wherein

$R_{1d}$, $R_7$ and $R_9$ are as previously defined;

$R_8$ is H;

D is Br, $NO_2$, $SR_{12}$ or $SO_2NH$-*t*-Bu; and

$R_{12}$ is $C_2$—$C_4$ alkyl or benzyl.

Reduction of dihydropyrones of Formulas CXXa—CXXd gives the corresponding tetrahydropyrones as shown in Equation 76 for the specific example of CXXa.

Equation 76

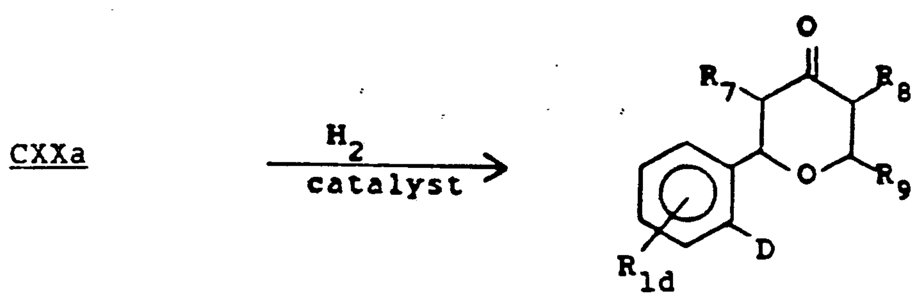


CXXIII

wherein

$R_{1d}$, $R_7$, $R_8$ and $R_9$ are as previously defined, D is Br, $NO_2$ or $SR_{12}$, and $R_{12}$ is $C_2$—$C_4$ alkyl or benzyl.

The reduction shown in Equation 76 can be carried out in the presence of a suitable catalyst such as colloidal palladium (*cf.*, Borsche, *Ber.*, *48*, 682 (1915); *56*, 2012, 213 (1923); *59*, 237 (1926)) or palladized strontium carbonate (see Cawley and Plant, *J. Chem. Soc.*, 1214 (1938); Attenburrow, *et al.*, *ibid.*, 571 (1945)).

In a similar fashion, compounds of Formulas CXXb—CXXd can be reduced to afford the corresesonding tetrahydropyrones. Further elaboration of the compounds by methods described previously then yields the primary sulfonamides IVa or IVb, where Q is Q—88 or Q—89.

Compounds of Formulas CXXIVa—CXXIVe can be synthesized as shown in Equation 77 by condensation of 1,3-diketones of Formulas CXXVa—CXXVe with the appropriate imines of Formulas CXXVIa—CXXVId.

Equation 77

(a)

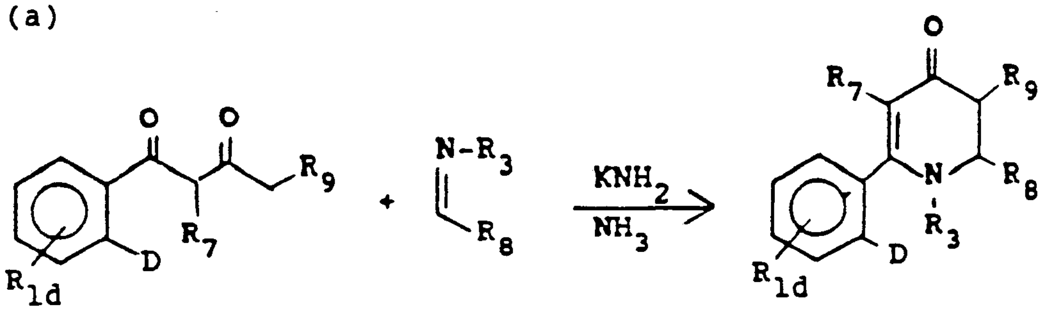


CXXVa CXXVIa CXXIVa

(b)

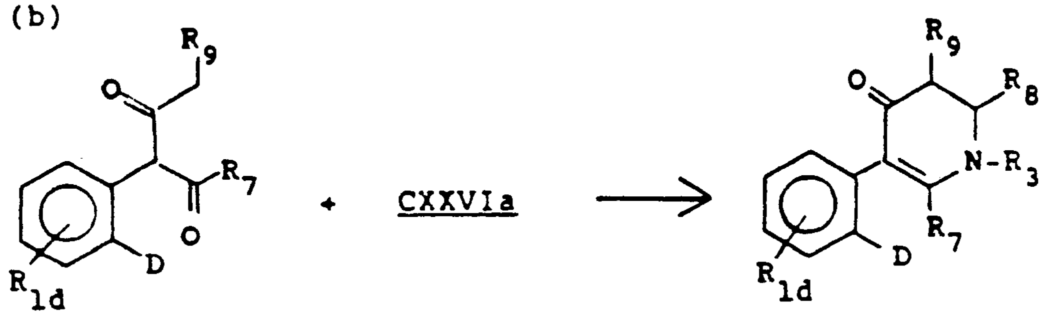

CXXVb CXXIVb

(c)

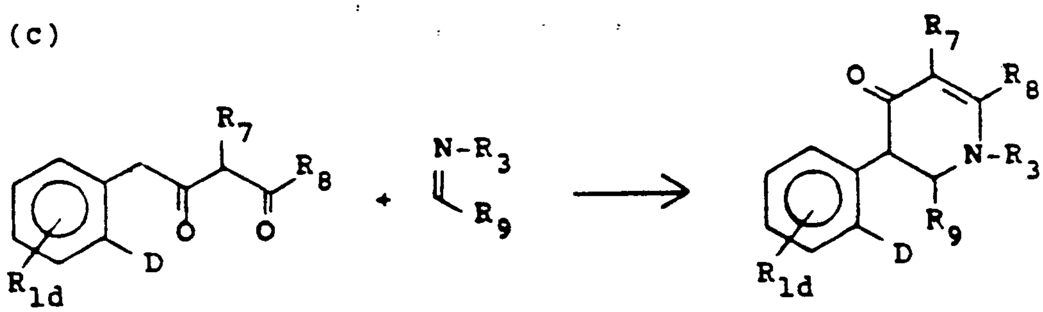

CXXVc CXXVIb CXXIVc

(d)

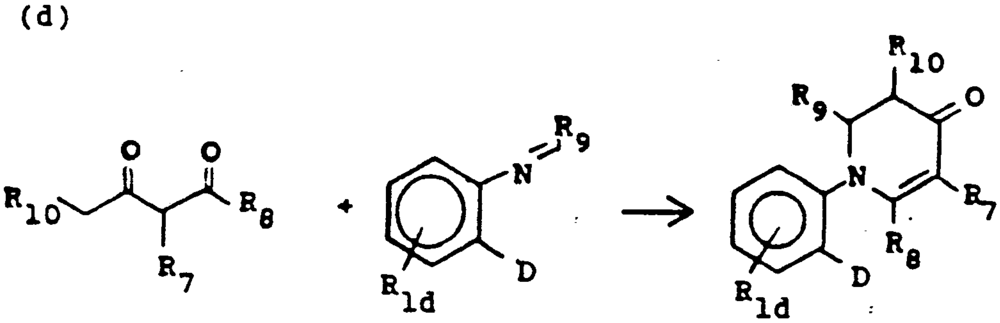

CXXVd CXXVIc CXXIVd

(e)

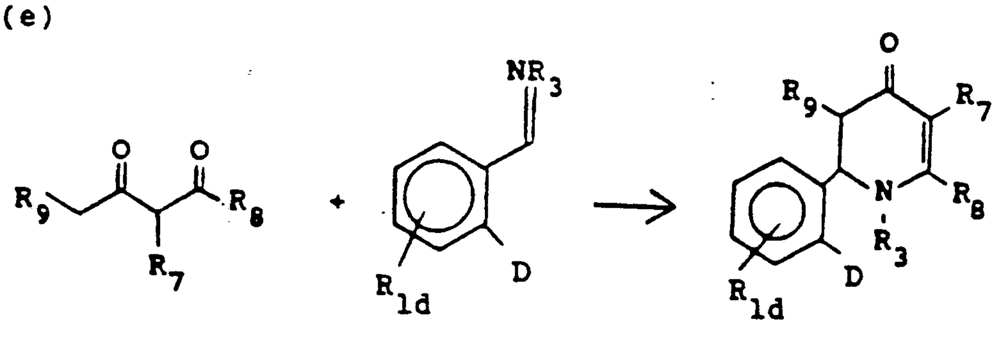

CXXVe CXXVId CXXIVe

wherein

$R_{1d}$, $R_3$, $R_7$, $R_8$, $R_9$ and $R_{10}$ are as previously defined, D is Br, $NO_2$ or $SR_{12}$, and $R_{12}$ is $C_2$—$C_4$ alkyl or benzyl.

The reactions of Equation 77 are caried out by the procedure described by N. Sugiyama, M. Yamamoto and C. Kasima, *Bull, Chem. Soc. Japan, 42,* 1357 (1969).

Dihydropyridone CXXIVe above (when $R_8$ is H) may also be prepared in an analogous manner to Equation 75e as depicted in Equation 77f. The Lewis acid catalyzed Diels-Alder reaction of imines of Formula CXXVId with dienes CXXe results in the desired dihydropyridones.

Equation 77f

CXXVId

CXXe

$ZnCl_2$

CXXIVe

wherein

$R_{1d}$, $R_3$, $R_7$, $R_9$ and $R_{10}$ are as previously defined;

$R_8$ is H; D is Br, $NO_2$, $SR_{12}$ or $SO_2NH$-*t*-Bu; and

$R_{12}$ is $C_2$—$C_4$ alkyl or benzyl.

Dihydropyridones, such as those of Formulas CXXIVa—CXXIVe, have also been prepared by reduction of the appropriate pyridones of Formulas LXXXIa and LXXXIb with lithium aluminum hydrride (E. Winterfeldt, *Ber. deutsch Chem. Ges., 97,* 2463 (1964)), lithium triethoxyaluminum hydride (Y. Tamura, *et al., Chem. and Ind.,* 168 (1972)), and catalytic hydrogenation (see J. Hebky and J. Kejha, *CA, 50,* 15532c).

Piperidones of Formulas CXXVIIa—CXVIIc can be prepared in a straightforward manner via reduction of the appropriate dihydropyridones of Formulas CXXIVa—CXXIVe. Equation 78 depicts the reduction of compounds of Formula CXXIVa with lithium aluminum hydride to give piperidones of Formula CXXVIIa.

Equation 78

CXXIVa

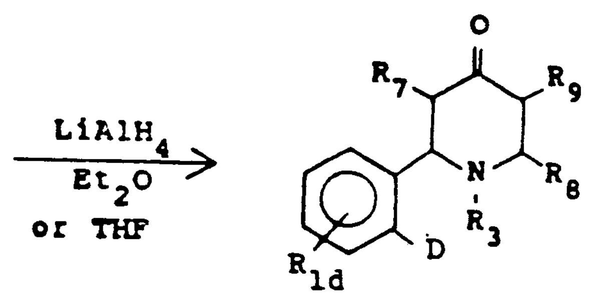


CXXVIIa

wherein

$R_{1d}$, $R_3$, $R_7$, $R_8$ and $R_9$ are as previously defined, D is Br, $NO_2$ or $SR_{12}$, and $R_{12}$ is $C_2$—$C_4$ alkyl or benzyl.

The 1,4-reduction of enaminones such as those of Formula CXXIVa can be achieved with lithium aluminum hydride and a variety of other reagents. For a review of these methods, see J. V. Greenhill, *Chem. Soc. Rev., 6,* 277 (1977).

The tetrahydrothiopyrones of Formulas CXXVIIIa and CXVIIIb can be prepared as shown in Equation 79 by a Dieckmann-type cyclization of the appropriate sulfides of Formulas CXXIXa and CXXIXb, followed by acid- or base-induced ester cleavage and decarboxylation.

Equation 79

(a)

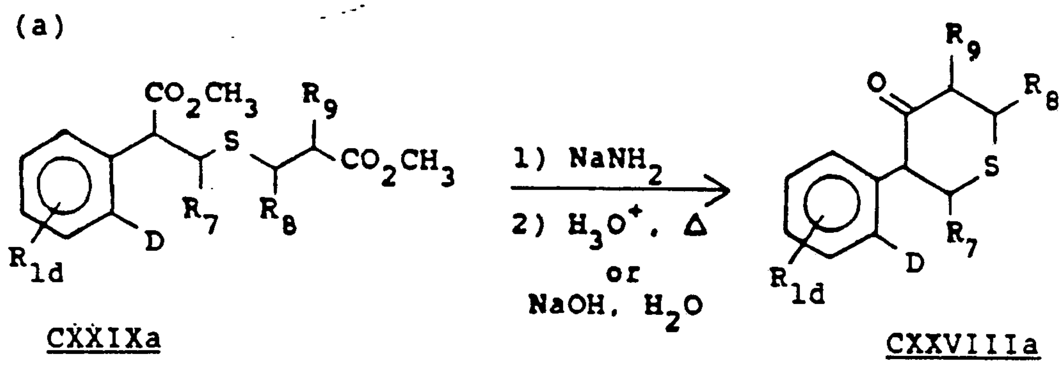


CXXIXa CXXVIIIa

(b)

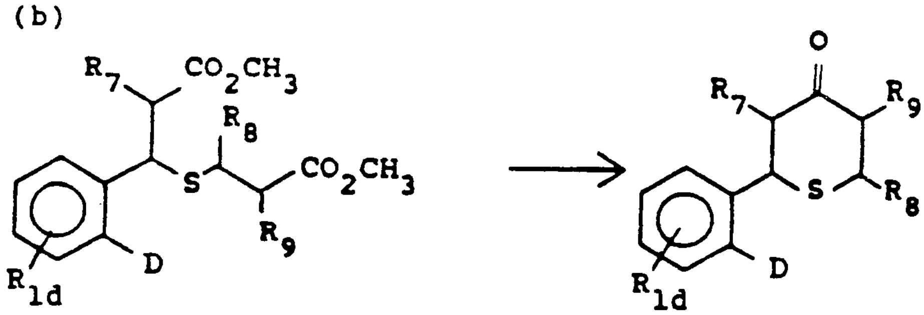

CXXIXb CXXVIIIb

wherein

$R_{1d}$, $R_7$, $R_8$ and $R_9$ are as previously defined, and D is Br, $NO_2$ or $SO_2NH$-*t*-Bu.

The reactions of Equations 79(a) and 79(b) can be effectively accomplished by the procedure of G. M. Bennett and L. V. D. Scorah, *J. Chem. Soc.*, 194 (1927).

The requisite sulfides of Formulas CXXIXa and CXXIXb can be readily synthesized by either of the methods shown in Equation 80. Thus, treatment of the mercaptide salts of Formula CXXX with either (a) α,β-unsaturated esters of Formula CXXXI, or (b) alkyl halides of Formula CXXXII affords the desired products of Formula CXXICa.

Equation 80

(a)

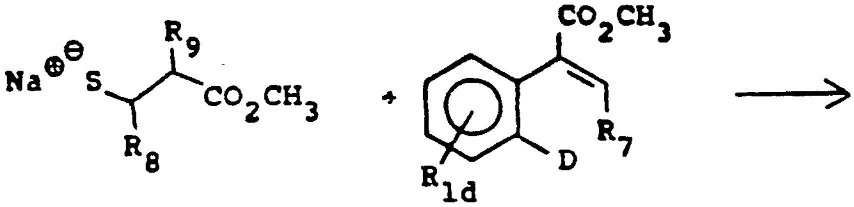

CXXIXa

CXXX CXXXI

(b)

CXXX +

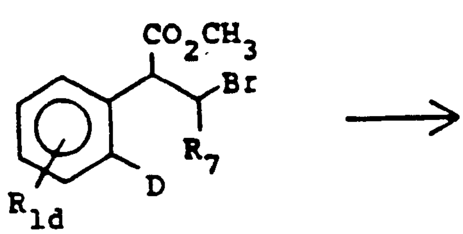

CXXIXa

CXXXII

wherein

$R_{1d}$, $R_7$, $R_8$ and $R_9$ are as previously defined, and D is Br, $NO_2$ or $SO_2NH$-*t*-Bu.

The reaction of Equation 80 can be carried out according to the procedures discussed by Bruson, *Org. Reactions, 5* (1949), pp. 95—97 and 129—130.

The dihydrothiopyrones of Formulas CXXXIIIa—CXXXIIId can be prepared from the appropriate tetrahydropyrones of Formulas CXXVII and CXXVIIb by oxidation with N-chlorosuccinimide (NCS) as shown in Equations 81(a) and 81(b).

Equation 81

(a)

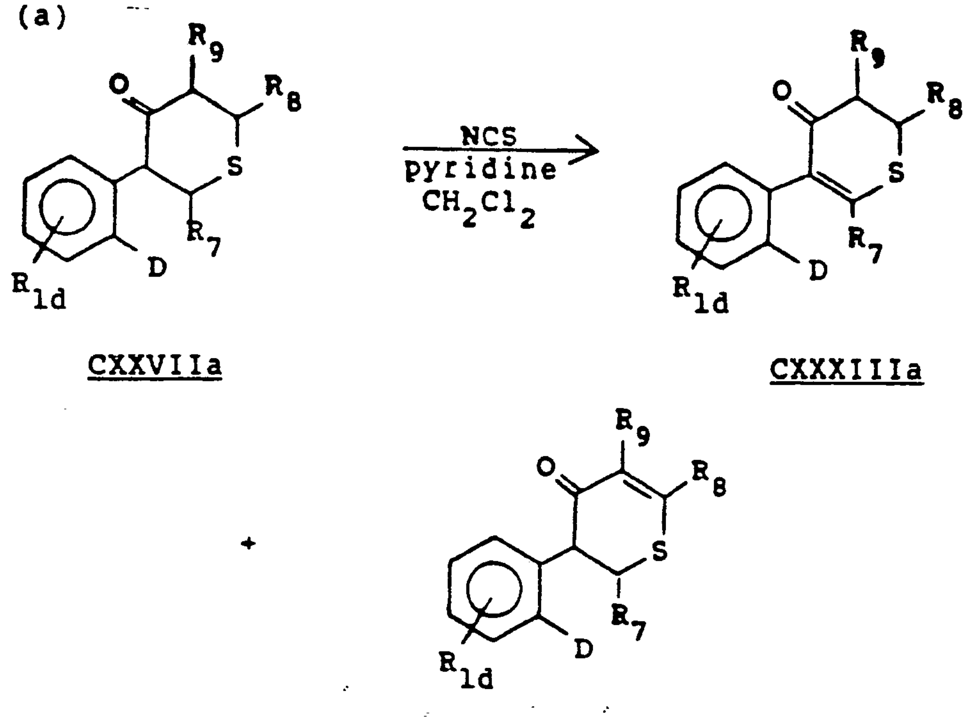


CXXVIIa

CXXXIIIa

+

CXXXIIIb

(b)

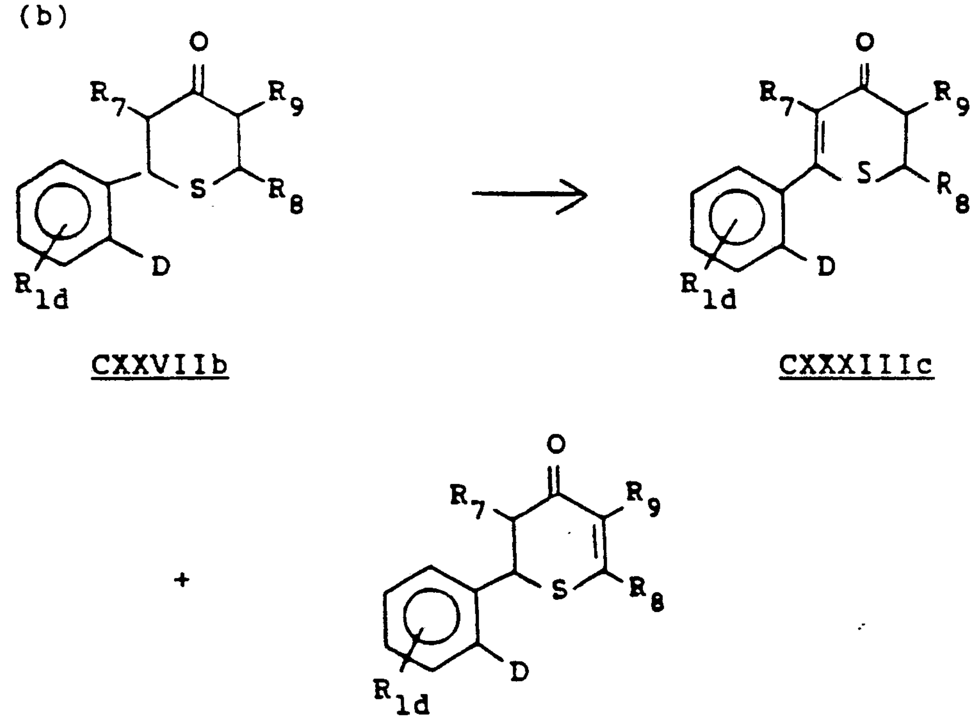

CXXVIIb

CXXXIIIc

+

CXXXIIId

wherein

$R_{1d}$, $R_7$, $R_8$ and $R_9$ are as previously defined, and D is Br, $NO_2$ or $SO_2NH$-*t*-Bu.

The reaction of Equation 81 can be carried out according to the procedure of C. H. Chen, G. A. Reynolds and J. A. Van Allan, *J. Org. Chem.*, *42*, 2777 (1977). It should be noted that the reaction shown in Equation 81, when applied to assymmetrical tetrahydropyrones such as those of Formulas CXXVIIa and CXXVIIb, gives mixtures of isomers. These compounds can be separated by recrystallization from a suitable solvent such as diethyl ether, benzene, or ethyl acetate, or by column chromatography.

The synthesis of heterocyclic amines such as those represented by Formula III has been reviewed in "The Chemistry of Heterocyclic Compounds", a series published by Interscience Publ., New York and London. Aminopyrimidines are described by D. J. Brown in "The Pyrimidines", Vol. XVI of the series mentioned above which is herein incorporated by reference. The 2-amino-1,3,5-triazines of Formula III, where A is A—1 and Z is N, can be prepared according to methods described by E. M. Smolin and L. Rapaport in "*s*-Triazines and Derivatives", Vol. XIII.

Pyrimidines of Formula III, where is A is A—1 and Y is an acetal or thioacetal substituent, can be prepared by methods taught in European Patent Application No. 84,224 (published July 27, 1983).

Pyrimidines of Formula III, where A is A—1 and Y is cyclopropyl or $OCF_2H$, can be synthesized according to the methods taught in South African Patent Application No. 83/7434 and South African Publication No. 82/5045, respectively.

Compounds of Formula III, where A is A—2 or A—3, can be prepared by procedures disclosed in United States Patent 4,339,267.

Compounds of Formula III, where A is A—4, can be prepared by methods taught in European Patent Application No. 46,677 (published March 3, 1982).

Additional references dealing with the synthesis of bicyclic pyrimidines of Formula III, where A is A—2, A—3, or A—4 are Braker, Sheehan, Spitzmiller and Lott, *J. Am. Chem. Soc.*, *69*, 3072 (1947); Mitler and Bhattachanya, *Quart. J. Indian Chem. Soc.*, *4*, 152 (1927); Shrage and Hitchings, *J. Org. Chem.*, *16*, 1153 (1951); Caldwell, Kornfeld and Donnell, *J. Am. Chem. Soc.*, *63*, 2188 (1941); and Frissekis, Myles and Brown, *J. Org. Chem.*, *29*, 2670 (1964).

Compounds of Formula III, where A is A—5, can be prepared by methods taught in United States Patent 4,421,550.

Compounds of Formula III, where A is A—6, can be prepared by methods taught in European Patent Application No. 94,260 (published November 16, 1983).

Agriculturally suitable salts of compounds of Formula I are also useful herbicides and can be prepared in a number of ways known to the art. For example, metal salts can be made by treating compounds of Formula I with a solution of an alkali or alkaline earth metal salt having a sufficiently basic anion (e.g., hydroxide, alkoxide, carbonate or hydride). Quaternary amine salts can be made by similar techniques. Detailed examples of such techniques are given in United States Patent 4,127,405.

The compounds of this invention and their preparation are further illustrated by the followina examples.

## Example 1

2H-1,2-Benzothiazin-3(4H)-one, 1,1-dioxide

A solution of 18 g of 2 sulfamoylphenylacetic acid, methyl ester, in 125 g of 10% aqueous sodium hydroxide was stirred at room temperature for 4 hours. The mixture was filtered and the filtrate acidified by the addition of concentrated hydrochloric acid with ice-water cooling. The resulting precipitate was collected by filtration, washed with water, and dried to afford 12.5 g of 2H-1,2-benzothiazin-3(4H)-one, 1,1-dioxide as an off-white powder, m.p. 190—195°C. NMC ($CDCl_3$/DMSO-$d_6$): δ 11.3 (1H, br s, NH), 7.85 1H, br d), 7.3—7.7 (3H, m) and 4.0(2H, s).

## Example 2

2-(Tetrahydro-2-oxo-3-furanyl)benzenesulfonamide

A solution of 5.0 g of the product from Example 1 in 125 mL dry tetrahydrofuran was cooled to −78°C under an atmosphere of nitrogen and treated with 33 mL of 1.6 M *n*-butyllithium in hexanes, added over a period of about 15 minutes. After completion of the addition, the temperature was allowed to rise to −30°C over 30 minutes, and the solution was then recooled to −78°C and treated with 2.5 mL of ethylene oxide. The reaction mixture was stirred to room temperature overnight, and was then cooled to 0°C and quenched with 5% aqueous hydrochloric acid. The layers were separated and the water layer extracted with ether. Drying and concentration of the combined organic extracts gave an orange oil which was immediately dissolved in 30 mL of tetrahydrofuran. Concentrated hydrochloric acid (5 mL) was added and the mixture was heated at reflux temperature for one hour. Tetrahydrofuran was removed *in vacuo*, and the residue was dissolved in ether and washed with water. Drying and concentration of the organic layer gave an orange oil which was purified by silica gel chromatography. Elution with 4:1 ethyl acetate-hexanes containing 1% methanol gave a foam which was crystallized from ether to afford 1.7 g of 2-(tetrahydro-2-oxo-3-furanyl)benzenesulfonamide as a white powder, m.p. 141—146°C. IR(KBr): 3370, 3220, 1750, 1335, 1170 $cm^{-1}$; NMR ($CDCl_3$): δ 8.15 (1H, dd, J=2, 8 Hz), 7.6 (1H, td, J=2, 8 Hz), 7.48 (1H, td, J=2, 8 Hz), 7.4 (1H, 33, J=2, 8 Hz), 5.35 (2H, br s, $NH_2$), 5.0 (1H, br t, J=9 Hz), 4.40—4.68 (2H, m) and 2.64—2.80 (2H, m).

Example 3

N-[(4,6-dimethoxypyrimidin-2-yl)aminocarbonyl]-2-(tetrahydro-2-oxo-3-furanyl)benzenesulfonamide

A solution of 240 mg of the product from Example 2 and 270 mg of (4,6-dimethoxy-2-pyrimidyl)carbamic acid, phenyl ester in 5 mL dry acetonitrile was treated at room temperature under an atmosphere of nitrogen with 0.15 mL of 1,8-diazabicyclo[5.4.0]undec-7-ene. The reaction mixture was stirred at room temperature for one hour, diluted with 3 mL water and acidified with 5% aqueous hydrochloric acid. The resulting precipitate was collected by filtration, and washed with water and ether. The yield of N-[(4,6-dimethoxypyrimidin-2-yl)aminocarbonyl]-2-(tetrahydro-2-oxo-3-furanyl)benzenesulfonamide was 290 mg as a white powder, m.p. 163.5—166.5°C. IR(KBr): 1775 (lactone C=O), 1710 $cm^{-1}$; NMR($CDCl_3$): δ 12.74 (1H, br s, NH), 8.24 (1H, br d, J=8 Hz), 7.65 (1H, br t, J=8 Hz), 7.49 (1H, br t, J=8 HZ), 7.36 (1H, br d, J=8 Hz), 7.24 (1H, br s, NH), 5.78 (1H s), 5.13 (1H, dd, J=8,10 Hz), 4.29—4.56 (2H, m), 3.94 (6H, s), 2.86—3.02 (1H, m) and 2.26—2.48 (1H, m).

Example 4

2-(Tetrahydro-4-methyl-2-oxo-3-furanyl)benzenesulfonamide

A solution of 5.0 g of the product from Example 1 in 125 mL of tetrahydrofuran was cooled to −78°C under an atmosphere of nitrogen and treated with 56 mL of 0.95 M *n*-butyllithium in hexane, added over a period of about 15 minutes. After completion of the addition, the temperature was allowed to rise to −30°C over 30 minutes and the solution was recooled to −78°C and treated with 3.7 mL of propylene oxide. The reaction mixture was stirred at room temperature overnight, and was then cooled to 0°C and quenched with 5% aqueous hydrochloric acid. The layers were separated and the water layer extracted with ether. Drying and concentration of the combined organic extracts gave an orange oil which was immediately disssolved in 30 mL tetrahydrofuran. Concentrated hydrochloric acid (10 mL) was added and the mixture was heated at reflux temperature for 1.25 hr. Tetrahydrofuran was removed *in vacuo*, and the residue was dissolved in ether and washed with water. Drying and concentration of the organic layer gave an orange oil which was purified by silica gel chromatography. Elution with 4:1 ethyl acetate-hexanes containing 1% methanol gave 1.6 g of the title compound (mixture of diastereomers) as a white powder, m.p. 144.5—153°C. IR(KBr): 3350, 3250, 1720, 1335, 1160 $cm^{-1}$; NMR($CDCl_3$/DMSO-$d_6$): δ 8.05 (1H, br d, J=7 Hz), 7.2—7.7 (3H, m), 6.9 (2H, br s, $NH_2$), 4.55—5.20 (2H, m), 2.8—3.2 (~2/3H, m), 2.4—2.6(~1/3H, m), 1.75—2.15 (1H, m) and 1.5 (3H, 3, J=6 Hz).

Example 5

N-[4-methyl-6-methoxypyrimidin-2-yl)aminocarbonyl]-2-(tetrahydro-4-methyl-2-oxo-3-furanyl)-benzenesulfonamide

A solution of 200 mg of the product from Example 4 and 200 mg of (4-methyl-6-methoxy-2-pyrimidinyl)carbamic acid, phenyl ester in 4 mL dry acetonitrile under an atmosphere of dry nitrogen was treated at room temperature with 0.12 mL of 1,8-diazabicyclo-[5.4.0]undec-7-ene. The reaction mixture was stirred at room temperature for one hour, diluted with 2 mL water and acidified with 5% aqueous hydrochloric acid. The resulting precipitate was collected by filtration, and washed well with water and ether. The yield of N-[(4-methyl-6-methoxypyrimidin-2-yl)-aminocarbonyl]-2-(tetrahydro-4-methyl-2-oxo-3-furanyl)-benzenesulfonamide (mixture of diastereomers) was 230 mg as a white powder, m.p. 188—188.5°C. IR(KBr): 1770 (lactone C=O), 1700, 1350 $cm^{-1}$; NMR($CDCl_3$): δ 13.32 (1H, br s, NH), 8.25 (1H, m), 7.64 (1H, br t), 7.48 (1H, br t), 7.35 (1H, br t), 7.27 (1H, br s, NH), 6.28 (1H, s), 5.12—5.24 (1H, m), 4.76—4.87 (~1/3H, m), 4.60—4.72 (~2/3H, m), 3.92 (3H, s), 2.96—3.10 (~2/3H, m), 2.44—2.60 (~1/3H, m), 2.41 (3H, s), 1.84—2.00 (1H, m), 1.50 and 1.46 (3H, overlapping doublets, J=6Hz).

Example 6

N-(1,1-Dimethylethyl)-2-(3-oxo-1-cyclohexen-1-yl)-3-thiophenesulfonamide

A solution of 3-*t*-butylthiophenesulfonamide (5.0 g, 0.0228 mole) in 100 mL THF was cooled to −78°C and stirred while *n*-butyllithium (1.37 M, 35 mL, 0.046 mole) was added rapidly in a dropwise manner. The solution was warmed to 0°C for 1 hour, stirred at room temperature for 1 hour, and was recooled to −10°C and treated with a solution of 3-ethoxy-2-cyclohexenone in 10 mL THF. After being warmed to room temperature and stirred for 4 hours, the mixture was treated with 100 mL of ice-water followed by 1*N* HCl to bring the pH to *ca* 4. The mixture was then extracted with ether (3 × 200 mL) and the combined ether extracts were washed with brine and dried ($MgSO_4$). Concentration gave an oil that was crystallized with 20% ethyl acetate in hexane to yield 4.37 g white solid, m.p. 157—158°C.

Example 7

2-(3-Oxo-1-cyclohexen-1-yl)-3-thiophenesulfonamide

A solution of the product from Example 6 (6.0 g, 0.0192 mole) in trifluoroacetic acid (20 mL) was stirred at room temperature overnight, was stripped to an oil, and crystallized with ethyl acetate/ether to yield 2.87 g solid, m.p. 140—147°C.

Example 8

N-[(4,6-Dimethylpyrimidin-2-yl)aminocarbonyl]-2-(3-oxo-1-cyclohexen-1-yl)-3-thiophenesulfonamide

A mixture of the product from Example 7 (0.30 g, 1.17 mmole) and N-(4,6-dimethylpyrimidin-2-yl)phenyl carbamate (0.30 g, 1.23 mmole) in acetonitrile (30 mL) was treated with DBU (184 µl, 1.23 mmole) at room temperature overnight. The solution was poured into water and acidified with acetic acid. The solid which precipitated was collected on a glass funnel and washed with water, butylchloride, and ether to yield 0.15 g white solid; m.p. 190—192°C; NMR ($CDCl_3$) δ: 2.0—2.2 (m, 2H), 2.3—2.5 (m, with s at δ 2.44, 8H), 2.7—2.8 (m, 2H), 6.25 (s, 1H), 6.77 (s, 1H), 7.36 and 7.66 (AB q, J=5.4 Hz, 2H); IR $cm^{-1}$ (nujol): 1730, 1700 (shoulder at 1685 and 1675), 1605, 1550, 1170, 1140.

Example 9

2-Chloro-3-pyridinesulfonamide

A solution of 50.4 g of sodium nitrite in 112 mL of water was added to 93.8 g of 3-amino-2-chloropyridine suspended at 0°C in 112 mL of acetic acid, 56 mL of water and 280 mL of concentrated HCl. After the addition was complete, the mixture was stirred at 0 to 5°C for 45 minutes. The resulting slurry was added dropwise to a suspension of 7.5 g of cuprous chloride in acetic acid (280 mL), concentrated HCl (140 mL), and sulfur dioxide (75 mL) at 0 to 5°C. The mixture was stirred 1 hour at 0°C, then 1 hour at 25°C, was poured into water, and the resultant solution was extracted with methylene chloride. The organic layer was washed and then dried ($Na_2SO_4$).

This methylene chloride solution was cooled to 0°C and an excess of ammonia was added in a dropwise manner. The suspension was stirred at 10—15°C for 1 hour and was then allowed to warm to 25°C for an additional hour. The solid was collected and washed with water to furnish 55 g of the desired sulfonamide, mp. 184—186°C.

Example 10

2-(4-Thiomorpholinyl)-3-pyridinesulfonamide

In a dry flask under an inert nitrogen atmosphere was mixed 2.5 g of 2-chloro-3-pyridinesulfonamide, 100 mL of dry N,N-dimethylformamide (DMF), and 2.7 mL of thiomorpholine. The mixture was heated at 80°C overnight at which time an additional .3 mL of thiomorpholine was added and the heating was continued. After 24 hours the mixture was concentrated and the residue was taken up in THF. The solids were filtered and the filtrate was concentrated and chromatographed on silica gel using 1:1 ethyl acetate/hexane as eluent. The pure subject compound was isolated as a solid, which melted at 176—177°C; NMR (200 MHz, $CDCl_3$): δ 2.89 (m, $CH_2S$, 4H), 3.45 (m, $CH_2N$, 4H), 5.52 (2, $HN_2$, 2H), 7.26 (t, ArH, 1H), 8.2—8.6 (m, ArH, 2H).

Example 11

2-(4-Thiomorpholinyl)-3-pyridinesulfonamide, S,S-dioxide

To a solution of 2-(4-thiomorpholinyl)-3-pyridinesulfonamide in 50 mL of trifluoroacetic acid was added 0.23 mL of methanesulfonic acid. Hydrogen peroxide (0.79 mL) was added dropwise and the resultant mixture was stirred at 25°C for 4 hours. An additional 0.3 mL of hydrogen peroxide was added and the mixture was stirred 72 hours. The volatiles were removed and the residue was partitioned between a saturated aqueous sodium bicarbonate solution and ethyl acetate. The organic layer was dried over magnesium sulfate, filtered and concentrated. The residue was chromatographed on silica gel using 40% ethyl acetate in hexane as eluent. The desired subject compound was isolated and crystallized from chloroform to give 0.6 g of a solid, m.p. 210—211°C; NMR (90 MHz, DMSO-$d_6$): δ 3.3—3.5 (m, $CH_2$, 4H), 3.55—3.75 (m, $CH_2$, 4H), 7.2—7.5 (m, ArH & $SO_2NH_2$, 3H), 8.2—8.4 & 8.5—8.7 (m, ArH, 2H).

Example 12

N-[(4,6-Dimethoxypyrimidin-2-yl)aminocarbonyl]-2-(4-thiomorpholinyl)-3-pyridinesulfonamide, S,S-dioxide

In a dry flask under an inert nitrogen atmosphere was mixed 0.13 g of 2-(4-thiomorpholine, S,S-dioxide)-3-pyridinesulfonamide and 0.132 g of phenyl 4,6-dimethoxy-1,3-pyrimidin-2-ylcarbamate. To this mixture was added 0.072 mL of 1,8-diazabicyclo[5.4.0]-undec-7-ene and the resultant mixture was stirred for 0.5 hour. Water (50 mL) was added followed by 1N HCl dropwise until a pH of 5 was obtained. The solid was filtered and washed with water and then 1:1 etherhexane to furnish 0.1 g of the title compound as a solid, m.p. 204—205°C; IR (nujol) 1700 $cm^{-1}$; NMR (200 MHz, DMSO-$d_6$): δ 3.19 (m, 4H), 3.54 (m, 4H), 3.8 (s, $OCH_3$, 6H), 6.06 (2, CH, 1H), 7.4—8.7 (m, ArH, 3H), 10.6 & 12.9 (bs, NH, 2H).

By applying the procedures of Examples 1—12 and Equations 1 through 81, the compounds shown in Tables 1 through 7 can be prepared by one skilled in the art.

General Formulas for Tables

General Formula 1

$W_1$ is O unless indicated by *, wherein $W_1$ is S.

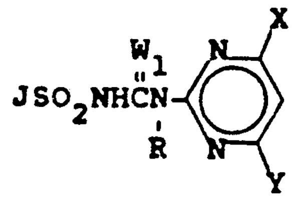

General Formula 2

$W_1$ is O unless indicated by *, wherein $W_1$ is S.

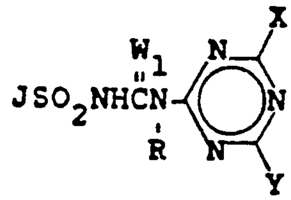

General Formula 3

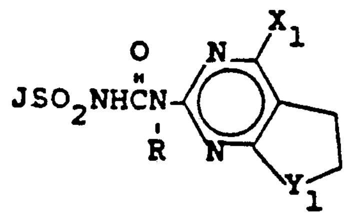

General Formula 4

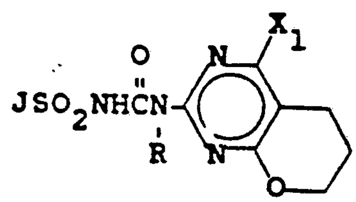

General Formula 5

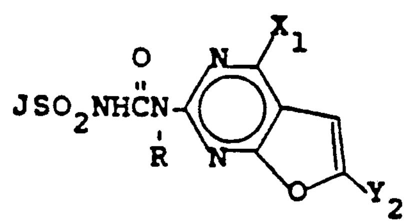

General Formula 6

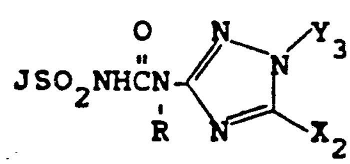

General Formula 7

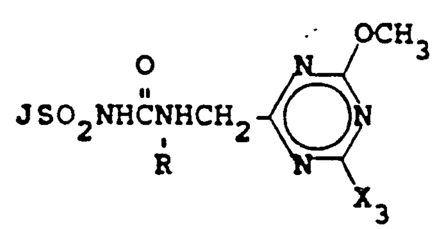

TABLE 1
General Formula 1

| J | $W_3$ | Q | R | $R_{1b}$ | X | Y |
|---|---|---|---|---|---|---|
| J-5 | S | Q-1 ($R_7$=H, $R_8$=H) | H | H | $CH_3$ | $CH_3$ |
| J-6 | S | Q-1 ($R_7$=H, $R_8$=H) | H | H | $OCH_3$ | $CH_3$ |
| J-5 | S | Q-1 ($R_7$=H, $R_8$=H) | H | H | $OCH_3$ | $OCH_3$ |
| J-6 | S | Q-1 ($R_7$=H, $R_8$=H) | $CH_3$ | H | $OCH_3$ | $OCH_3$ |
| J-5 | S | Q-1 ($R_7$=H, $R_8$=H) | $CH_3$ | H | $OCH_3$ | $CH_3$ |
| J-6 | S | Q-1 ($R_7$=H, $R_8$=H) | H | 5-$CH_3$ | $OCH_3$ | $OCH_3$ |
| J-5 | S | Q-1 ($R_7$=H, $R_8$=H) | H | 5-$CH_2F$ | $OCH_3$ | $OCH_3$ |
| J-6 | S | Q-1 ($R_7$=H, $R_8$=H) | H | 5-$CH_2CH_2Br$ | $OCH_3$ | $OCH_3$ |
| J-7 | S | Q-1 ($R_7$=H, $R_8$=H) | H | 5-$CH(CH_3)(CH_2Cl)$ | $OCH_3$ | $OCH_3$ |
| J-5 | O | Q-1 ($R_7$=H, $R_8$=H) | H | 4-$NH_2$ | $OCH_3$ | $OCH_3$ |
| J-6 | S | Q-1 ($R_7$=H, $R_8$=H) | H | 4-$N(CH_3)_2$ | $OCH_3$ | $OCH_3$ |
| J-5 | S | Q-1 ($R_7$=H, $R_8$=H) | H | 5-Cl | $OCH_3$ | $OCH_3$ |
| J-6 | S | Q-1 ($R_7$=H, $R_8$=H) | H | 4-F | $OCH_3$ | $OCH_3$ |
| J-5 | S | Q-1 ($R_7$=H, $R_8$=H) | H | 5-I | $OCH_3$ | $OCH_3$ |
| J-6 | S | Q-1 ($R_7$=H, $R_8$=H) | H | 4-$NO_2$ | $OCH_3$ | $OCH_3$ |
| J-5 | NH | Q-1 ($R_7$=H, $R_8$=H) | H | 5-$CF_3$ | $OCH_3$ | $OCH_3$ |
| J-6 | S | Q-1 ($R_7$=H, $R_8$=H) | H | H | $OCH_3$ | $CH_2CH_3$ |
| J-5 | S | Q-1 ($R_7$=H, $R_8$=H) | H | H | $OCH_3$ | $CH(CH_3)_2$ |
| J-6 | S | Q-1 ($R_7$=H, $R_8$=H) | H | H | $OCH_3$ | $(CH_2)_3CH_3$ |
| J-7 | O | Q-1 ($R_7$=H, $R_8$=H) | H | H | $OCH_3$ | $OCH_2CH_3$ |
| J-5 | S | Q-1 ($R_7$=H, $R_8$=H) | H | H | $OCH_3$ | $OCH(CH_3)_2$ |
| J-6 | S | Q-1 ($R_7$=H, $R_8$=H) | H | H | $OCH_3$ | $O(CH_2)_3CH_3$ |
| J-5 | S | Q-1 ($R_7$=H, $R_8$=H) | H | H | $OCF_2H$ | $OCH_3$ |
| J-6 | S | Q-1 ($R_7$=H, $R_8$=H) | H | H | $OCH_2CH_2F$ | $OCH_3$ |
| J-5 | S | Q-1 ($R_7$=H, $R_8$=H) | H | H | $OCH_2CHF_2$ | $OCH_3$ |
| J-6 | S | Q-1 ($R_7$=H, $R_8$=H) | H | H | $OCH_2CF_3$ | $OCH_3$ |
| J-5 | S | Q-1 ($R_7$=H, $R_8$=H) | H | H | $OCH(CH_3)(CH_2Cl)$ | $OCH_3$ |
| J-6 | S | Q-1 ($R_7$=H, $R_8$=H) | H | H | $O(CH_2)_3CH_2Br$ | $OCH_3$ |
| J-5 | S | Q-1 ($R_7$=H, $R_8$=H) | H | H | $CH_2F$ | $OCH_3$ |
| J-6 | O | Q-1 ($R_7$=H, $R_8$=H) | H | H | $CH_2Cl$ | $OCH_3$ |
| J-7 | S | Q-1 ($R_7$=H, $R_8$=H) | H | H | $CH_2Br$ | $OCH_3$ |

Table 1 (continued)

| J | $W_3$ | Q | R | $R_{1b}$ | X | Y |
|---|---|---|---|---|---|---|
| J-5 | S | Q-1 ($R_7$=H, $R_8$=H) | H | H | $CF_3$ | $OCH_3$ |
| J-6 | S | Q-1 ($R_7$=H, $R_8$=H) | H | H | $CH_2CH_2Br$ | $OCH_3$ |
| J-5 | S | Q-1 ($R_7$=H, $R_8$=H) | H | H | $CH(CH_3)(CH_2Cl)$ | $OCH_3$ |
| J-6 | S | Q-1 ($R_7$=H, $R_8$=H) | H | H | $(CH_2)_3CH_2I$ | $OCH_3$ |
| J-5 | S | Q-1 ($R_7$=H, $R_8$=H) | H | H | $SCH_3$ | $OCH_3$ |
| J-6 | S | Q-1 ($R_7$=H, $R_8$=H) | H | H | $SCH_2CH_3$ | $OCH_3$ |
| J-5 | S | Q-1 ($R_7$=H, $R_8$=H) | H | H | $SCH(CH_3)_2$ | $OCH_3$ |
| J-6 | S | Q-1 ($R_7$=H, $R_8$=H) | H | H | $S(CH_2)_3CH_3$ | $OCH_3$ |
| J-5 | O | Q-1 ($R_7$=H, $R_8$=H) | H | H | $SCHF_2$ | $OCH_3$ |
| J-6 | S | Q-1 ($R_7$=H, $R_8$=H) | H | H | $SCH_2CH_2Br$ | $OCH_3$ |
| J-7 | S | Q-1 ($R_7$=H, $R_8$=H) | H | H | $SCH(CH_3)(CH_2Cl)$ | $OCH_3$ |
| J-5 | S | Q-1 ($R_7$=H, $R_8$=H) | H | H | $S(CH_2)_3CH_2F$ | $OCH_3$ |
| J-6 | S | Q-1 ($R_7$=H, $R_8$=H) | H | H | Cl | $OCH_3$ |
| J-5 | S | Q-1 ($R_7$=H, $R_8$=H) | H | H | Br | $OCH_3$ |
| J-6 | S | Q-1 ($R_7$=H, $R_8$=H) | H | H | F | $OCH_3$ |
| J-5 | S | Q-1 ($R_7$=H, $R_8$=H) | H | H | I | $OCH_3$ |
| J-6 | S | Q-1 ($R_7$=H, $R_8$=H) | H | H | $OCH_3$ | $CH_2OCH_3$ |
| J-5 | S | Q-1 ($R_7$=H, $R_8$=H) | H | H | $OCH_3$ | $CH_2CH_2OCH(CH_3)_2$ |
| J-6 | O | Q-1 ($R_7$=H, $R_8$=H) | H | H | $OCH_3$ | $CH(CH_3)(CH_2OCH_3)$ |
| J-7 | S | Q-1 ($R_7$=H, $R_8$=H) | H | H | $OCH_3$ | $(CH_2)_4CH_2OCH_2CH_3$ |
| J-5 | S | Q-1 ($R_7$=H, $R_8$=H) | H | H | $OCH_2O(CH_2)_3CH_3$ | $OCH_3$ |
| J-6 | S | Q-1 ($R_7$=H, $R_8$=H) | H | H | $OCH_2CH_2OCH(CH_3)_2$ | $OCH_3$ |
| J-5 | S | Q-1 ($R_7$=H, $R_8$=H) | H | H | $OCH(CH_3)(CH_2OCH_3)$ | $OCH_3$ |
| J-6 | S | Q-1 ($R_7$=H, $R_8$=H) | H | H | $O(CH_2)_4CH_2OCH_2CH_3$ | $OCH_3$ |
| J-5 | S | Q-1 ($R_7$=H, $R_8$=H) | H | H | $OCH_3$ | $NH_2$ |
| J-6 | S | Q-1 ($R_7$=H, $R_8$=H) | H | H | $OCH_3$ | $NHCH_3$ |
| J-5 | S | Q-1 ($R_7$=H, $R_8$=H) | H | H | $OCH_3$ | $NHCH_2CH_3$ |
| J-6 | S | Q-1 ($R_7$=H, $R_8$=H) | H | H | $OCH_3$ | $NHCH(CH_3)_2$ |
| J-5 | O | Q-1 ($R_7$=H, $R_8$=H) | H | H | $OCH_3$ | $N(CH_3)_2$ |
| J-6 | S | Q-1 ($R_7$=H, $R_8$=H) | H | H | $OCH_3$ | $N(CH_3)(CH_2CH_3)$ |
| J-7 | S | Q-1 ($R_7$=H, $R_8$=H) | H | H | $OCH_3$ | $N(CH_3)(CH(CH_3)_2)$ |
| J-5 | S | Q-1 ($R_7$=H, $R_8$=H) | H | H | $OCH_3$ | $(CH_2)_4CH_2OCH_2CH_3$ |
| J-6 | S | Q-1 ($R_7$=H, $R_8$=H) | H | H | H | $OCH_3$ |

Table 1 (continued)

| J | $W_3$ | Q | R | $R_{1b}$ | X | Y |
|---|---|---|---|---|---|---|
| J-5 | S | Q-1 ($R_7$=H, $R_8$=H) | H | H | $OCH_3$ | $OCH_2CH=CH_2$ |
| J-6 | S | Q-1 ($R_7$=H, $R_8$=H) | H | H | $OCH_3$ | $OCH_2C(CH_3)=CH_2$ |
| J-5 | S | Q-1 ($R_7$=H, $R_8$=H) | H | H | $OCH_3$ | $OCH_2C\equiv CH$ |
| J-6 | S | Q-1 ($R_7$=H, $R_8$=H) | H | H | $OCH_3$ | $OCH_2C\equiv CCH_3$ |
| J-5 | S | Q-1 ($R_7$=H, $R_8$=H) | H | H | $OCH_3$ | $CH_2S(CH_2)_3CH_3$ |
| J-6 | S | Q-1 ($R_7$=H, $R_8$=H) | H | H | $OCH_3$ | $CH_2CH_2SCH(CH_3)_2$ |
| J-5 | O | Q-1 ($R_7$=H, $R_8$=H) | H | H | $OCH_3$ | $CH(CH_3)(CH_2SCH_3)$ |
| J-6 | S | Q-1 ($R_7$=H, $R_8$=H) | H | H | $OCH_3$ | $(CH_2)_4CH_2SCH_2CH_3$ |
| J-7 | S | Q-1 ($R_7$=H, $R_8$=H) | H | H | $OCH_3$ | cyclopropyl |
| J-5 | S | Q-1 ($R_7$=H, $R_8$=H) | H | H | $OCH_3$ | 2-methylcyclopropyl |
| J-6 | S | Q-1 ($R_7$=H, $R_8$=H) | H | H | $OCH_3$ | cyclopentyl |
| J-5 | S | Q-1 ($R_7$=H, $R_8$=H) | H | H | $OCH_3$ | $C\equiv CH$ |
| J-6 | S | Q-1 ($R_7$=H, $R_8$=H) | H | H | $OCH_3$ | $C\equiv CCH_3$ |
| J-5 | S | Q-1 ($R_7$=H, $R_8$=H) | H | H | $OCH_3$ | -CHO |
| J-6 | S | Q-1 ($R_7$=H, $R_8$=H) | H | H | $OCH_3$ | $-COCH_3$ |
| J-5 | S | Q-1 ($R_7$=H, $R_8$=H) | H | H | $OCH_3$ | $-CH(OCH_3)_2$ |
| J-6 | O | Q-1 ($R_7$=H, $R_8$=H) | H | H | $OCH_3$ | $-CH(SCH_3)(OCH_2CH_3)$ |
| J-5 | S | Q-1 ($R_7$=H, $R_8$=H) | H | H | $OCH_3$ | $-C(CH_3)(SCH_3)_2$ |
| J-6 | S | Q-1 ($R_7$=H, $R_8$=H) | H | H | $OCH_3$ | $-CH(SCH_2CH_3)_2$ |
| J-7 | S | Q-1 ($R_7$=H, $R_8$=H) | H | H | $OCH_3$ | 1,3-dioxolan-2-yl |
| J-5 | S | Q-1 ($R_7$=H, $R_8$=H) | H | H | $OCH_3$ | 2-methyl-1,3-oxa-thiolan-2-yl |
| J-6 | S | Q-1 ($R_7$=H, $R_8$=H) | H | H | $OCH_3$ | 1,3-oxathian-2-yl |
| J-5 | S | Q-1 ($R_7$=H, $R_8$=H) | H | H | $OCH_3$ | 2-methyl-1,3-dithian-2-yl |
| J-6 | S | Q-1 ($R_7$=H, $R_8$=H) | H | H | $OCH_3$ | 4-methyl-1,3-dioxolan-2-yl |
| J-5 | S | Q-1 ($R_7$=H, $R_8$=H) | H | H | $OCH_3$ | 4-methyl-1,3-oxathiolan-2-yl |
| J-6 | S | Q-1 ($R_7$=H, $R_8$=H) | H | H | $OCH_3$ | 2,4-dimethyl-1,3-dithiolan-2-yl |
| J-5 | O | Q-1 ($R_7$=H, $R_8$=H) | H | H | $OCH_3$ | $N(OCH_3)(CH_3)_2$ |
| J-6 | S | Q-1 ($R_7$=H, $R_8$=$CH_3$) | H | H | $OCH_3$ | $OCH_3$ |
| J-5 | S | Q-1 ($R_7$=H, $R_8$=$CH_3$) | H | H | $CH_3$ | $CH_3$ |

Table 1 (continued)

| J | $W_3$ | Q | R | $R_{1b}$ | X | Y |
|---|---|---|---|---|---|---|
| J-6 | S | Q-1 ($R_7$=H, $R_8$=$CH_3$) | H | H | $CH_3$ | $OCH_3$ |
| J-7 | S | Q-1 ($R_7$=H, $R_8$=$CH_2CH_3$) | H | H | $OCH_3$ | $OCH_3$ |
| J-5 | S | Q-1 ($R_7$=H, $R_8$=$CH(CH_3)_2$) | H | H | $OCH_3$ | $OCH_3$ |
| J-6 | S | Q-1 ($R_7$=H, $R_8$=$(CH_2)_3CH_3$) | H | H | $OCH_3$ | $OCH_3$ |
| J-5 | S | Q-1 ($R_7$=$CH_3$, $R_8$=$CH_3$) | H | H | $OCH_3$ | $OCH_3$ |
| J-5 | S | Q-2 ($R_7$=H, $R_8$=H) | H | H | $OCH_3$ | $OCH_3$ |
| J-5 | S | Q-2 ($R_7$=H, $R_8$=H) | $CH_3$ | H | $OCH_3$ | $OCH_3$ |
| J-6 | O | Q-2 ($R_7$=H, $R_8$=$CH_3$) | H | 5-$CH_3$ | $OCH_3$ | $OCH_3$ |
| J-5 | S | Q-3 ($R_7$=H, $R_8$=H) | H | H | $OCH_3$ | $OCH_3$ |
| J-6 | S | Q-3 ($R_7$=$C_2H_5$, $R_8$=H) | H | H | $OCF_2H$ | $CH_3$ |
| J-5 | S | Q-3 ($R_7$=$CH_3$, $R_8$=H) | H | 5-Cl | $OCH_3$ | $CH_3$ |
| J-6 | S | Q-4 ($R_7$=H, $R_8$=H, $R_3$=$CH_3$) | H | H | $CH_3$ | $OCH_3$ |
| J-7 | S | Q-4 ($R_7$=H, $R_8$=H, $R_3$=$C_2H_5$) | H | H | $CH_3$ | $OCH_3$ |
| J-5 | S | Q-4 ($R_7$=H, $R_8$=H, $R_3$=$\underline{i}$-$C_3H_7$) | H | 5-$CH_3$ | $OCH_3$ | $OCH_3$ |
| J-6 | S | Q-5 ($R_7$-H, $R_8$=H, $R_3$=H) | H | H | $OCH_3$ | $OCH_3$ |
| J-5 | S | Q-5 ($R_7$=H, $R_8$=H, $R_3$=$CH_3$) | H | H | Cl | $OCH_3$ |
| J-6 | S | Q-5 ($R_7$=H, $R_8$=$CH_3$, $R_3$=$CH_3$) | H | H | $CH_3$ | $OCH_3$ |
| J-5 | O | Q-6 ($R_7$=$CH_3$, $R_8$=H, $R_3$=$CH_3$) | H | H | $CH_3$ | $OCH_3$ |
| J-6 | S | Q-6 ($R_7$-H, $R_8$=H, $R_3$=$C_2H_5$) | $CH_3$ | H | $CH_3$ | $OCH_3$ |
| J-5 | S | Q-6 ($R_7$=$C_2H_5$, $R_8$=H, $R_3$=H) | H | H | $OCH_3$ | $OCH_3$ |
| J-7 | S | Q-8 ($R_7$=H, $R_8$=H, $R_2$=H) | H | H | $OCH_3$ | $OCH_3$ |
| J-5 | S | Q-8 ($R_7$=$CH_3$, $R_8$=H, $R_2$=$OCH_3$) | H | H | $OCH_3$ | $OCF_2H$ |
| J-5 | S | Q-8 ($R_7$=H, $R_8$=H, $R_2$=$OCH(CH_3)CH_2CH_3$) | H | 5-Cl | $OCH_3$ | $OCH_3$ |
| J-5 | S | Q-9 ($R_7$=H, $R_8$=H, $R_2$=H) | H | H | $OCH_3$ | $OCH_3$ |
| J-6 | O | Q-9 ($R_7$=$CH_3$, $R_8$=H, $R_2$=$CH_3$) | $CH_3$ | H | $OCH_3$ | $OCH_3$ |
| J-5 | S | Q-9 ($R_7$=H, $R_8$=H, $R_2$=Cl) | H | 5-$CH_3$ | $OCH_3$ | $OCH_3$ |
| J-6 | S | Q-10 ($R_7$=H, $R_8$=H) | H | H | $OCH_3$ | $OCH_3$ |
| J-5 | S | Q-10 ($R_7$=$CH_3$, $R_8$=H) | H | H | $OCF_2H$ | $CH_3$ |
| J-6 | S | Q-10 ($R_7$=H, $R_8$=$CH_3$) | H | 5-Cl | $OCH_3$ | $CH_3$ |
| J-5 | S | Q-11 ($R_7$=H, $R_8$=H) | H | H | $CH_3$ | $OCH_3$ |

Table 1 (continued)

| J | $W_3$ | Q | R | $R_{1b}$ | X | Y |
|---|---|---|---|---|---|---|
| J-6 | S | Q-11 ($R_7$=n-$C_4H_9$, $R_8$=H) | H | H | $CH_3$ | $OCH_3$ |
| J-7 | S | Q-11 ($R_7$=$CH_3$, $R_8$=$CH_3$) | H | 5-$CH_3$ | $OCH_3$ | $OCH_3$ |
| J-5 | S | Q-12 ($R_7$=H, $R_8$=H) | H | H | $OCH_3$ | $OCH_3$ |
| J-6 | S | Q-12 ($R_7$=$CH_3$, $R_8$=H) | H | H | Cl | $OCH_3$ |
| J-5 | O | Q-12 ($R_7$=H, $R_8$=$C_2H_5$) | H | H | $CH_3$ | $OCH_3$ |
| J-6 | S | Q-13 ($R_7$=H, $R_8$=H, $R_3$=H) | H | H | $CH_3$ | $OCH_3$ |
| J-5 | S | Q-13 ($R_7$=H, $R_8$=H, $R_3$=$CH_3$) | $CH_3$ | H | $CH_3$ | $OCH_3$ |
| J-6 | S | Q-13 ($R_7$=$CH_3$, $R_8$=H, $R_3$=$CH_3$) | H | H | $OCH_3$ | $OCH_3$ |
| J-5 | S | Q-14 ($R_7$=H, $R_8$=H, $R_3$=$CH_3$) | H | H | $OCH_3$ | $OCH_3$ |
| J-6 | S | Q-14 ($R_7$=$CH_3$, $R_8$=H, $R_3$=$C_2H_5$) | H | H | $OCH_3$ | $OCH_3$ |
| J-7 | S | Q-14 ($R_7$=H, $R_8$=$CH_3$, $R_3$=$CH_3$) | H | 5-$CH_3$ | $OCH_3$ | $OCH_3$ |
| J-5 | S | Q-15 ($R_7$=H, $R_8$=H, $R_3$=H) | H | H | $OCH_3$ | $OCH_3$ |
| J-6 | S | Q-15 ($R_7$=$C_2H_5$, $R_8$=H, $R_3$=$CH_3$) | H | H | $OCH_3$ | $OCF_2H$ |
| J-5 | S | Q-15 ($R_7$=$CH_3$, $R_8$=$CH_3$, $R_3$=$CH_3$) | H | 5-Cl | $OCH_3$ | $OCH_3$ |
| J-5 | S | Q-17 ($R_7$=H, $R_8$=H, $R_2$=H) | H | H | $OCH_3$ | $OCH_3$ |
| J-6 | S | Q-17 ($R_7$=$CH_3$, $R_8$=H, $R_2$=$CH_3$) | H | H | $OCF_2H$ | $CH_3$ |
| J-5 | S | Q-17 ($R_7$=H, $R_8$=H, $R_2$=$C_2H_5$) | H | 5-Cl | $OCH_3$ | $CH_3$ |
| J-7 | S | Q-18 ($R_7$=H, $R_8$=H, $R_2$=H) | H | H | $CH_3$ | $OCH_3$ |
| J-5 | S | Q-18 ($R_7$=H, $R_8$=$OC_2H_5$, $R_2$=H) | H | H | $CH_3$ | $OCH_3$ |
| J-6 | S | Q-18 ($R_7$=$CH_3$, $R_8$=$OCH(CH_3)_2$, $R_2$=$CH_3$) | H | 5-$CH_3$ | $OCH_3$ | $OCH_3$ |
| J-5 | O | Q-19 ($R_7$=H, $R_8$=H) | H | H | $OCH_3$ | $OCH_3$ |
| J-6 | S | Q-19 ($R_7$=$CH_3$, $R_8$=H) | H | H | Cl | $OCH_3$ |
| J-5 | S | Q-19 ($R_7$=$CH_3$, $R_8$=$CH_3$) | H | H | $CH_3$ | $OCH_3$ |
| J-5 | S | Q-20 ($R_7$=H, $R_8$=H) | H | H | $CH_3$ | $OCH_3$ |
| J-6 | S | Q-20 ($R_7$=$CH_3$, $R_8$=H) | $CH_3$ | H | $CH_3$ | $OCH_3$ |
| J-5 | S | Q-20 ($R_7$=H, $R_8$=$CH_3$) | H | H | $OCH_3$ | $OCH_3$ |
| J-6 | S | Q-21 ($R_7$=H, $R_8$=H) | H | H | $OCH_3$ | $OCH_3$ |
| J-5 | S | Q-21 ($R_7$=$CH_3$, $R_8$=H) | H | H | $OCH_3$ | $OCH_3$ |
| J-6 | S | Q-21 ($R_7$=$CH_3$, $R_8$=$CH_3$) | H | 5-$CH_3$ | $OCH_3$ | $OCH_3$ |
| J-7 | S | Q-22 ($R_7$=H, $R_8$=H, $R_3$=H) | H | H | $OCH_3$ | $OCH_3$ |

Table 1 (continued)

| J | $W_3$ | Q | R | $R_{1b}$ | X | Y |
|---|---|---|---|---|---|---|
| J-5 | S | Q-22 ($R_7$=H, $R_8$=H, $R_3$=$CH_3$) | H | H | $OCH_3$ | $OCF_2H$ |
| J-6 | O | Q-22 ($R_7$=$CH_3$, $R_8$=$CH_3$, $R_3$=$C_2H_5$) | H | 5-Cl | $OCH_3$ | $OCH_3$ |
| J-5 | S | Q-23 ($R_7$=H, $R_8$=H, $R_3$=H) | H | H | $OCH_3$ | $OCH_3$ |
| J-6 | S | Q-23 ($R_7$=H, $R_8$=H, $R_3$=$CH_3$) | $CH_3$ | H | $OCH_3$ | $OCH_3$ |
| J-5 | S | Q-23 ($R_7$=$CH_3$, $R_8$=H, $R_3$=n-$C_4H_9$) | H | 5-$CH_3$ | $OCH_3$ | $OCH_3$ |
| J-6 | S | Q-24 ($R_7$=H, $R_8$=H, $R_3$=$CH_3$) | H | H | $OCH_3$ | $OCH_3$ |
| J-5 | S | Q-24 ($R_7$=$CH_3$, $R_8$=$CH_3$, $R_3$=$C_2H_5$) | H | H | $OCF_2H$ | $CH_3$ |
| J-6 | S | Q-24 ($R_7$=$CH_3$, $R_8$=$CH_3$, $R_3$=i-$C_3H_7$) | H | 5-Cl | $OCH_3$ | $CH_3$ |
| J-5 | S | Q-25 ($R_7$=H, $R_8$=H, $R_9$=H) | H | H | $CH_3$ | $OCH_3$ |
| J-6 | S | Q-25 ($R_7$=$CH_3$, $R_8$=H, $R_9$=H) | H | H | $CH_3$ | $OCH_3$ |
| J-7 | S | Q-25 ($R_7$=H, $R_8$=H, $R_9$=$C_2H_5$) | H | 5-$CH_3$ | $OCH_3$ | $OCH_3$ |
| J-5 | O | Q-26 ($R_7$=H, $R_8$=H) | H | H | $OCH_3$ | $OCH_3$ |
| J-6 | S | Q-26 ($R_7$=$CH_3$, $R_8$=H) | H | H | Cl | $OCH_3$ |
| J-5 | S | Q-26 ($R_7$=$CH_3$, $R_8$=$CH_3$) | H | H | $CH_3$ | $OCH_3$ |
| J-6 | S | Q-27 ($R_7$=H, $R_8$=H) | H | H | $CH_3$ | $OCH_3$ |
| J-5 | S | Q-27 ($R_7$=$CH_3$, $R_8$=H) | $CH_3$ | H | $CH_3$ | $OCH_3$ |
| J-6 | S | Q-27 ($R_7$=H, $R_8$=$CH_3$) | H | H | $OCH_3$ | $OCH_3$ |
| J-5 | S | Q-28 ($R_7$=H, $R_8$=H) | H | H | $OCH_3$ | $OCH_3$ |
| J-6 | S | Q-28 ($R_7$=$CH_3$, $R_8$=H) | H | H | $OCH_3$ | $OCH_3$ |
| J-5 | S | Q-28 ($R_7$=$CH_3$, $R_8$=$CH_3$) | H | 5-$CH_3$ | $OCH_3$ | $OCH_3$ |
| J-6 | S | Q-29 ($R_7$=H, $R_8$=H) | H | H | $OCH_3$ | $OCH_3$ |
| J-7 | O | Q-29 ($R_7$=$CH_3$, $R_8$=H) | H | H | $OCH_3$ | $OCF_2H$ |
| J-5 | S | Q-29 ($R_7$=H, $R_8$=$CH_3$) | H | 5-Cl | $OCH_3$ | $OCH_3$ |
| J-6 | S | Q-30 ($R_7$=H, $R_8$=H) | H | H | $OCH_3$ | $OCH_3$ |
| J-5 | S | Q-30 ($R_7$=$CH_3$, $R_8$=$CH_3$) | $CH_3$ | H | $OCH_3$ | $CH_3$ |
| J-6 | S | Q-30 ($R_7$=$C_2H_5$, $R_8$=H) | H | 5-$CH_3$ | $OCH_3$ | $OCH_3$ |
| J-5 | S | Q-31 ($R_7$=H, $R_8$=H) | H | H | $OCH_3$ | $OCH_3$ |
| J-6 | S | Q-31 ($R_7$=i-$C_3H_7$, $R_8$=H) | H | H | $OCF_2H$ | $CH_3$ |
| J-5 | S | Q-31 ($R_7$=H, $R_8$=n-$C_4H_9$) | H | 5-Cl | $OCH_3$ | $CH_3$ |
| J-6 | S | Q-32 ($R_7$=H, $R_8$=H, $R_3$=H) | H | H | $CH_3$ | $OCH_3$ |
| J-5 | S | Q-32 ($R_7$=$CH_3$, $R_8$=H, $R_3$=$CH_3$) | H | H | $CH_3$ | $OCH_3$ |
| J-6 | O | Q-32 ($R_7$=H, $R_8$=$CH_3$, $R_3$=$C_2H_5$) | H | 5-$CH_3$ | $OCH_3$ | $OCH_3$ |
| J-7 | S | Q-33 ($R_7$=H, $R_8$=H, $R_8$=$CH_3$) | H | H | $OCH_3$ | $OCH_3$ |

Table 1 (continued)

| J | $W_3$ | Q | R | $R_{1b}$ | X | Y | m.p.(°C) |
|---|---|---|---|---|---|---|---|
| J-5 | S | Q-33 ($R_7$=$CH_3$, $R_8$=$CH_3$, $R_3$=i-$C_3H_7$) | H | H | Cl | $OCH_3$ | |
| J-6 | S | Q-33 ($R_7$=$C_2H_5$, $R_8$=H, $R_3$=n-$C_4H_9$) | H | H | $CH_3$ | $OCH_3$ | |
| J-5 | S | Q-34 ($R_7$=H, $R_8$=H, $R_3$=H) | H | H | $CH_3$ | $OCH_3$ | |
| J-6 | S | Q-34 ($R_7$=i-$C_3H_7$, $R_8$=H, $R_3$=$CH_3$) | $CH_3$ | H | $CH_3$ | $OCH_3$ | |
| J-5 | S | Q-34 ($R_7$=H, $R_8$=n-$C_4H_9$, $R_3$=$CH_3$) | H | H | $OCH_3$ | $OCH_3$ | |
| J-5 | O | Q-36 ($R_7$=H, $R_8$=H, $R_2$=H) | H | H | $OCH_3$ | $OCH_3$ | |
| J-6 | S | Q-36 ($R_7$=$CH_3$, $R_8$=H, $R_2$=$CH_3$) | H | H | $OCH_3$ | $OCF_2H$ | |
| J-6 | S | Q-36 ($R_7$=H, $R_8$=H, $R_2$=$C_2H_5$) | H | 5-Cl | $OCH_3$ | $OCH_3$ | |
| J-5 | S | Q-37 ($R_7$=H, $R_8$=H, $R_2$=H) | H | H | $OCH_3$ | $OCH_3$ | |
| J-6 | S | Q-37 ($R_7$=H, $R_8$=H, $R_2$=H) | H | H | $CH_3$ | $CH_3$ | 196-199 |
| J-6 | S | Q-37 ($R_7$=H, $R_8$=H, $R_2$=H) | H | H | $OCH_3$ | $CH_3$ | 202-204 |
| J-6 | S | Q-37 ($R_7$=H, $R_8$=H, $R_2$=H) | H | H | $OCH_3$ | $OCH_3$ | 160-163 |
| J-6 | S | Q-37 ($R_7$=H, $R_8$=H, $R_2$=H) | H | H | $OCH_3$ | Cl | 208-210 |
| J-7 | S | Q-37 ($R_7$=H, $R_8$=H, $R_2$=H) | H | H | $OCH_3$ | $OCH_3$ | 144-147 |
| J-6 | S | Q-37 ($R_7$=H, $R_8$=H, $R_2$=i-$C_3H_7$) | $CH_3$ | H | $OCH_3$ | $OCH_3$ | |
| J-5 | S | Q-37 ($R_7$=H, $R_8$=H, $R_2$=n-$C_4H_9$) | H | 5-$CH_3$ | $OCH_3$ | $OCH_3$ | |
| J-6 | S | Q-38 ($R_7$=H, $R_8$=H, $R_2$=$OCF_2H$) | H | H | $OCH_3$ | $OCH_3$ | |
| J-5 | S | Q-38 ($R_7$=$CH_3$, $R_8$=H, $R_2$=$OCH_2CH_2F$) | H | H | $OCF_2H$ | $CH_3$ | |
| J-6 | S | Q-38 ($R_7$=H, $R_8$=H, $R_2$=Cl) | H | 5-Cl | $OCH_3$ | $CH_3$ | |
| J-5 | S | Q-39 ($R_7$=H, $R_8$=H, $R_9$=H) | H | H | $CH_3$ | $OCH_3$ | |
| J-6 | O | Q-39 ($R_7$=$C_2H_5$, $R_8$=H, $R_9$=H) | H | H | $CH_3$ | $OCH_3$ | |
| J-5 | S | Q-39 ($R_7$=H, $R_8$=H, $R_9$=$CH_3$) | H | 5-$CH_3$ | $OCH_3$ | $OCH_3$ | |
| J-6 | S | Q-40 ($R_7$=H, $R_8$=H) | H | H | $OCH_3$ | $OCH_3$ | |
| J-7 | NH | Q-40 ($R_7$=$CH_3$, $R_8$=H) | H | H | Cl | $OCH_3$ | |
| J-5 | S | Q-40 ($R_7$=H, $R_8$=$CH_3$) | H | H | $CH_3$ | $OCH_3$ | |
| J-6 | S | Q-41 ($R_7$=H, $R_8$=H) | H | H | $CH_3$ | $OCH_3$ | |
| J-5 | S | Q-41 ($R_7$=$CH_3$, $R_8$=$CH_3$) | $CH_3$ | H | $CH_3$ | $OCH_3$ | |
| J-6 | S | Q-41 ($R_7$=H, $R_8$=$C_2H_5$) | H | H | $OCH_3$ | $OCH_3$ | |
| J-5 | S | Q-42 ($R_7$=H, $R_8$=H) | H | H | $OCH_3$ | $OCH_3$ | |
| J-6 | S | Q-42 ($R_7$=$CH_3$, $R_8$=$CH_3$) | H | H | $OCH_3$ | $OCH_3$ | |

Table 1 (continued)

| J | $W_3$ | Q | R | $R_{1b}$ | X | Y |
|---|---|---|---|---|---|---|
| J-5 | O | Q-42 ($R_7$=$C_2H_5$, $R_8$=$C_2H_5$) | H | 5-$CH_3$ | $OCH_3$ | $OCH_3$ |
| J-6 | S | Q-43 ($R_7$=H, $R_8$=H, $R_3$=H) | H | H | $OCH_3$ | $OCH_3$ |
| J-5 | S | Q-43 ($R_7$=H, $R_8$=H, $R_3$=$CH_3$) | H | H | $OCH_3$ | $OCF_2H$ |
| J-6 | S | Q-43 ($R_7$=$CH_3$, $R_8$=H, $R_3$=$CH_3$) | H | 5-Cl | $OCH_3$ | $OCH_3$ |
| J-7 | S | Q-44 ($R_7$=H, $R_8$=H, $R_3$=$CH_3$) | H | H | $OCH_3$ | $OCH_3$ |
| J-5 | S | Q-44 ($R_7$=$CH_3$, $R_8$=H, $R_3$=$C_2H_5$) | $CH_3$ | H | $OCH_3$ | $CH_3$ |
| J-6 | S | Q-44 ($R_7$=H, $R_8$=$CH_3$, $R_3$=$CH_3$) | H | 5-$CH_3$ | $OCH_3$ | $OCH_3$ |
| J-5 | S | Q-45 ($R_7$=H, $R_8$=H, $R_3$=H) | H | H | $OCH_3$ | $OCH_3$ |
| J-6 | S | Q-45 ($R_7$=$C_2H_5$, $R_8$=H, $R_3$=$CH_3$) | H | H | $OCF_2H$ | $CH_3$ |
| J-5 | S | Q-45 ($R_7$=$CH_3$, $R_8$=$CH_3$, $R_3$=$CH_3$) | H | 5-Cl | $OCH_3$ | $CH_3$ |
| J-5 | S | Q-47 ($R_7$=H, $R_8$=H, $R_2$=H) | H | H | $OCH_3$ | $OCH_3$ |
| J-6 | S | Q-47 ($R_7$=H, $R_8$=$CH_3$, $R_2$=$CH_3$) | H | H | Cl | $OCH_3$ |
| J-7 | S | Q-47 ($R_7$=H, $R_8$=H, $R_2$=$C_2H_5$) | H | H | $CH_3$ | $OCH_3$ |
| J-5 | S | Q-48 ($R_7$=H, $R_8$=H, $R_2$=H) | H | H | $CH_3$ | $OCH_3$ |
| J-6 | S | Q-48 ($R_7$=$CH_3$, $R_8$=$CH_3$, $R_2$=$CH_3$) | $CH_3$ | H | $CH_3$ | $OCH_3$ |
| J-5 | S | Q-48 ($R_7$=H, $R_8$=$C_2H_5$, $R_2$=$C_2H_5$) | H | H | $OCH_3$ | $OCH_3$ |
| J-6 | S | Q-49 ($R_7$=H, $R_8$=H, $R_2$=H) | H | H | $OCH_3$ | $OCH_3$ |
| J-5 | O | Q-49 ($R_7$=$CH_3$, $R_8$=$CH_3$, $R_2$=H) | H | H | $OCH_3$ | $OCH_3$ |
| J-6 | S | Q-49 ($R_7$=$CH_3$, $R_8$=$CH_3$, $R_2$=$CH_3$) | H | 5-$CH_3$ | $OCH_3$ | $OCH_3$ |
| J-5 | S | Q-50 ($R_7$=H, $R_8$=H, $R_2$=H) | H | H | $OCH_3$ | $OCH_3$ |
| J-6 | S | Q-50 ($R_7$=$CH_3$, $R_8$=$CH_3$, $R_2$=H) | H | H | $OCH_3$ | $OCF_2H$ |
| J-5 | S | Q-50 ($R_7$=$CH_3$, $R_8$=$CH_3$, $R_2$=$CH_3$) | H | 5-Cl | $OCH_3$ | $OCH_3$ |
| J-6 | S | Q-51 ($R_7$=H, $R_8$=H, $R_9$=H) | H | H | $OCH_3$ | $OCH_3$ |
| J-7 | S | Q-51 ($R_7$=H, $R_8$=H, $R_9$=$CH_3$) | $CH_3$ | H | $OCH_3$ | $OCH_3$ |
| J-5 | S | Q-51 ($R_7$=H, $R_8$=H, $R_9$=s-$C_4H_9$) | H | 5-$CH_3$ | $OCH_3$ | $OCH_3$ |
| J-6 | S | Q-52 ($R_7$=H, $R_8$=H, $R_9$=H) | H | H | $OCH_3$ | $OCH_3$ |
| J-5 | S | Q-52 ($R_7$=$CH_3$, $R_8$=H, $R_9$=H) | H | H | $OCF_2H$ | $CH_3$ |
| J-6 | O | Q-52 ($R_7$=H, $R_8$=H, $R_9$=$CH_3$) | H | 5-Cl | $OCH_3$ | $CH_3$ |
| J-5 | S | Q-53 ($R_7$=H, $R_8$=H, $R_9$=H) | H | H | $CH_3$ | $OCH_3$ |

Table 1 (continued)

| J | $W_3$ | Q | R | $R_{1b}$ | X | Y | m.p.(°C) |
|---|---|---|---|---|---|---|---|
| J-6 | S | Q-53 ($R_7$=$CH_3$, $R_8$=$CH_3$, $R_9$=$CH_3$) | H | H | $CH_3$ | $OCH_3$ | |
| J-5 | S | Q-53 ($R_7$=H, $R_8$=H, $R_9$=$CH_3$) | H | 5-$CH_3$ | $OCH_3$ | $OCH_3$ | |
| J-6 | S | Q-54 ($R_7$=H, $R_8$=H, $R_9$=H) | H | H | $OCH_3$ | $OCH_3$ | |
| J-7 | S | Q-54 ($R_7$=$CH_3$, $R_8$=H, $R_9$=H) | H | H | Cl | $OCH_3$ | |
| J-5 | S | Q-54 ($R_7$=H, $R_8$=H, $R_9$=i-$C_3H_7$) | H | H | $CH_3$ | $OCH_3$ | |
| J-6 | S | Q-55 ($R_7$=H, $R_8$=H, $R_9$=H, $R_3$=H) | H | H | $CH_3$ | $OCH_3$ | |
| J-5 | S | Q-55 ($R_7$=H, $R_8$=H, $R_9$=$CH_3$, $R_3$=$CH_3$) | $CH_3$ | H | $CH_3$ | $OCH_3$ | |
| J-6 | S | Q-55 ($R_7$=H, $R_8$=H, $R_9$=s-$C_4H_9$, $R_3$=H) | H | H | $OCH_3$ | $OCH_3$ | |
| J-5 | O | Q-56 ($R_7$=H, $R_8$=H, $R_9$=H, $R_3$=$C_2H_5$) | H | H | $OCH_3$ | $OCH_3$ | |
| J-6 | S | Q-56 ($R_7$=$CH_3$, $R_8$=H, $R_9$=H, $R_3$=H) | H | H | $OCH_3$ | $OCH_3$ | |
| J-5 | S | Q-56 ($R_7$=H, $R_8$=H, $R_9$=$CH_3$, $R_3$=$CH_3$) | H | 5-$CH_3$ | $OCH_3$ | $OCH_3$ | |
| J-6 | S | Q-57 ($R_7$=H, $R_8$=H, $R_9$=H, $R_3$=i-$C_3H_7$) | H | H | $OCH_3$ | $OCH_3$ | |
| J-5 | S | Q-57 ($R_7$=$CH_3$, $R_8$=$CH_3$, $R_9$=$CH_3$, $R_3$=H) | H | H | $OCH_3$ | $OCF_2H$ | |
| J-6 | S | Q-57 ($R_7$=H, $R_8$=H, $R_9$=$CH_3$, $R_3$=$CH_3$) | H | 5-Cl | $OCH_3$ | $OCH_3$ | |
| J-7 | S | Q-58 ($R_7$=H, $R_8$=H, $R_9$=H, $R_3$=H) | H | H | $OCH_3$ | $OCH_3$ | |
| J-5 | S | Q-58 ($R_7$=$CH_3$, $R_8$=H, $R_9$=H, $R_3$=$CH_3$) | $CH_3$ | H | $OCH_3$ | $CH_3$ | |
| J-6 | S | Q-58 ($R_7$=H, $R_8$=H, $R_9$=i-$C_3H_7$, $R_3$=$CH_3$) | H | 5-$CH_3$ | $OCH_3$ | $OCH_3$ | |
| J-6 | S | Q-60 ($R_7$=H, $R_8$=H, $R_9$=H, $R_2$=H) | H | H | $CH_3$ | $CH_3$ | 110-117 |
| J-6 | S | Q-60 ($R_7$=H, $R_8$=H, $R_9$=H, $R_2$=H) | H | H | $CH_3$ | $OCH_3$ | 123-133 |
| J-6 | S | Q-60 ($R_7$=H, $R_8$=H, $R_9$=H, $R_2$=H) | H | H | $OCH_3$ | $OCH_3$ | 124-132 |
| J-6 | S | Q-60 ($R_7$=H, $R_8$=H, $R_9$=H, $R_2$=H) | H | H | $OCH_3$ | Cl | 150-162 |
| J-5 | S | Q-60 ($R_7$=$CH_3$, $R_8$=H, $R_9$=H, $R_2$=$O(CH_2)_3CH_2Br$) | H | H | $CH_3$ | $OCH_3$ | |
| J-6 | S | Q-60 ($R_7$=H, $R_8$=H, $R_9$=H, $R_2$=Br) | H | 5-$CH_3$ | $OCH_3$ | $OCH_3$ | |
| J-5 | S | Q-61 ($R_7$=H, $R_8$=H, $R_9$=H, $R_2$=H) | H | H | $OCH_3$ | $OCH_3$ | |
| J-6 | S | Q-61 ($R_7$=$CH_3$, $R_8$=H, $R_9$=H, $R_2$=$OCH_2F$) | H | H | Cl | $OCH_3$ | |
| J-7 | S | Q-61 ($R_7$=H, $R_8$=$CH_3$, $R_9$=H, $R_2$=$O(CH_2)_3CH_3$) | H | H | $CH_3$ | $OCH_3$ | |
| J-5 | S | Q-62 ($R_7$=H, $R_8$=H, $R_9$=H, $R_2$=H) | H | H | $CH_3$ | $OCH_3$ | |
| J-6 | S | Q-62 ($R_7$=$CH_3$, $R_8$=H, $R_9$=H, $R_2$=$CH_3$) | $CH_3$ | H | $CH_3$ | $OCH_3$ | |
| J-5 | O | Q-62 ($R_7$=Cl, $R_8$=H, $R_9$=H, $R_2$=H) | H | H | $OCH_3$ | $OCH_3$ | |
| J-6 | S | Q-63 ($R_7$=H, $R_8$=H, $R_9$=H) | H | H | $OCH_3$ | $OCH_3$ | |

Table 1 (continued)

| J | $W_3$ | Q | R | $R_{1b}$ | X | Y |
|---|---|---|---|---|---|---|
| J-5 | S | Q-63 ($R_7$=$CH_3$, $R_8$=H, $R_9$=H) | H | H | $OCH_3$ | $OCH_3$ |
| J-6 | S | Q-63 ($R_7$=H, $R_8$=H, $R_9$=$CH_3$) | H | 5-$CH_3$ | $OCH_3$ | $OCH_3$ |
| J-5 | S | Q-64 ($R_7$=H, $R_8$=H, $R_9$=H) | H | H | $OCH_3$ | $OCH_3$ |
| J-6 | S | Q-64 ($R_7$=$C_2H_5$, $R_8$=H, $R_9$=H) | H | H | $OCH_3$ | $OCF_2H$ |
| J-5 | S | Q-64 ($R_7$=H, $R_8$=H, $R_9$=$C_2H_5$) | H | 5-Cl | $OCH_3$ | $OCH_3$ |
| J-6 | S | Q-65 ($R_7$=H, $R_8$=H, $R_9$=H) | H | H | $OCH_3$ | $OCH_3$ |
| J-7 | S | Q-65 ($R_7$=n-$C_4H_9$, $R_8$=H, $R_9$=H) | $CH_3$ | H | $OCH_3$ | $CH_3$ |
| J-5 | S | Q-65 ($R_7$=H, $R_8$=$CH_3$, $R_9$=i-$C_3H_7$) | H | 5-$CH_3$ | $OCH_3$ | $OCH_3$ |
| J-6 | O | Q-66 ($R_7$=H, $R_8$=H, $R_9$=H) | H | H | $OCH_3$ | $OCH_3$ |
| J-5 | S | Q-66 ($R_7$=i-$C_3H_7$, $R_8$=H, $R_9$=H) | H | H | $OCF_2H$ | $CH_3$ |
| J-6 | S | Q-66 ($R_7$=H, $R_8$=$C_2H_5$, $R_9$=$CH_3$) | H | 5-Cl | $OCH_3$ | $CH_3$ |
| J-5 | S | Q-67 ($R_7$=H, $R_8$=H, $R_9$=H, $R_3$=H) | H | H | $CH_3$ | $OCH_3$ |
| J-6 | S | Q-67 ($R_7$=$CH_3$, $R_8$=H, $R_9$=H, $R_3$=$CH_3$) | H | H | $CH_3$ | $OCH_3$ |
| J-5 | S | Q-67 ($R_7$=H, $R_8$=H, $R_9$=$CH_3$, $R_3$=$C_2H_5$) | H | 5-$CH_3$ | $OCH_3$ | $OCH_3$ |
| J-6 | S | Q-68 ($R_7$=H, $R_8$=H, $R_9$=H, $R_3$=$CH_3$) | H | H | $OCH_3$ | $OCH_3$ |
| J-5 | S | Q-68 ($R_7$=$C_2H_5$, $R_8$=H, $R_9$=H, $R_3$=i-$C_3H_7$) | H | H | Cl | $OCH_3$ |
| J-6 | S | Q-68 ($R_7$=H, $R_8$=H, $R_9$=$C_2H_5$, $R_3$=n-$C_4H_9$) | H | H | $CH_3$ | OCH |
| J-7 | S | Q-69 ($R_7$=H, $R_8$=H, $R_9$=H, $R_3$=$CH_3$) | H | H | $CH_3$ | $OCH_3$ |
| J-5 | O | Q-69 ($R_7$=n-$C_4H_9$, $R_8$=H, $R_9$=H, $R_3$=$C_2H_5$) | $CH_3$ | H | $CH_3$ | $OCH_3$ |
| J-6 | S | Q-69 ($R_7$=H, $R_8$=$CH_3$, $R_9$=i-$C_3H_7$, $R_3$=H) | H | H | $OCH_3$ | OCH |
| J-5 | S | Q-70 ($R_7$=H, $R_8$=H, $R_9$=H, $R_3$=$CH_3$) | H | H | $OCH_3$ | $OCH_3$ |
| J-6 | S | Q-70 ($R_7$=i-$C_3H_7$, $R_8$=H, $R_9$=H, $R_3$=$CH_3$) | H | H | $OCH_3$ | $OCH_3$ |
| J-5 | S | Q-70 ($R_7$=H, $R_8$=$C_2H_5$, $R_9$=$CH_3$, $R_3$=H) | H | 5-$CH_3$ | $OCH_3$ | OCH |
| J-5 | S | Q-72 ($R_7$=H, $R_8$=H, $R_9$=H, $R_2$=H) | H | H | $OCH_3$ | $OCH_3$ |
| J-6 | S | Q-72 ($R_7$=H, $R_8$=H, $R_9$=H, $R_2$=$CH_3$) | $CH_3$ | H | $OCH_3$ | $CH_3$ |
| J-7 | O | Q-72 ($R_7$=$CH_3$, $R_8$=H, $R_9$=H, $R_2$=H) | H | 5-$CH_3$ | $OCH_3$ | $OCH_3$ |
| J-5 | S | Q-73 ($R_7$=H, $R_8$=H, $R_9$=H, $R_2$=H) | H | H | $OCH_3$ | $OCH_3$ |
| J-6 | S | Q-73 ($R_7$=H, $R_8$=$CH_3$, $R_9$=H, $R_2$=$C_2H_5$) | H | H | $OCF_2H$ | $CH_3$ |
| J-5 | S | Q-73 ($R_7$=$CH_3$, $R_8$=H, $R_9$=$CH_3$, $R_2$=H) | H | 5-Cl | $OCH_3$ | $CH_3$ |
| J-6 | S | Q-74 ($R_7$=H, $R_8$=H, $R_9$=H, $R_2$=H) | H | H | $CH_3$ | $OCH_3$ |

Table 1 (continued)

| J | $W_3$ | Q | R | $R_{1b}$ | X | Y |
|---|---|---|---|---|---|---|
| J-5 | S | Q-74 ($R_7$=$CH_3$, $R_8$=H, $R_9$=H, $R_2$=H) | H | H | $CH_3$ | $OCH_3$ |
| J-6 | S | Q-74 ($R_7$=H, $R_8$=H, $R_9$=H, $R_2$=$CH_3$) | H | 5-$CH_3$ | $OCH_3$ | $OCH_3$ |
| J-5 | S | Q-75 ($R_7$=H, $R_8$=H, $R_9$=H) | H | H | $OCH_3$ | $OCH_3$ |
| J-6 | S | Q-75 ($R_7$=H, $R_8$=H, $R_9$=$CH_3$) | H | H | Cl | $OCH_3$ |
| J-5 | S | Q-75 ($R_7$=$CH_3$, $R_8$=H, $R_9$=$CH_3$) | H | H | $CH_3$ | $OCH_3$ |
| J-6 | O | Q-76 ($R_7$=H, $R_8$=H, $R_9$=H) | H | H | $CH_3$ | $OCH_3$ |
| J-7 | S | Q-76 ($R_7$=H, $R_8$=H, $R_9$=$CH_3$) | $CH_3$ | H | $CH_3$ | $OCH_3$ |
| J-5 | S | Q-76 ($R_7$=$C_2H_5$, $R_8$=H, $R_9$=$C_2H_5$) | H | H | $OCH_3$ | $OCH_3$ |
| J-6 | S | Q-77 ($R_7$=H, $R_8$=H, $R_9$=H) | H | H | $OCH_3$ | $OCH_3$ |
| J-5 | S | Q-77 ($R_7$=H, $R_8$=H, $R_9$=n-$C_3H_7$) | H | H | $OCH_3$ | $OCH_3$ |
| J-6 | S | Q-77 ($R_7$=H, $R_8$=$CH_3$, $R_9$=H) | H | 5-$CH_3$ | $OCH_3$ | $OCH_3$ |
| J-5 | S | Q-78 ($R_7$=H, $R_8$=H, $R_9$=H) | H | H | $OCH_3$ | $OCH_3$ |
| J-6 | S | Q-78 ($R_7$=H, $R_8$=H, $R_9$=s-$C_4H_9$) | H | H | $OCH_3$ | $OCF_2H$ |
| J-5 | S | Q-78 ($R_7$=n-$C_3H_7$, $R_8$=H, $R_9$=H) | H | 5-Cl | $OCH_3$ | $OCH_3$ |
| J-6 | S | Q-79 ($R_7$=H, $R_8$=H, $R_9$=H, $R_3$=H) | H | H | $OCH_3$ | $OCH_3$ |
| J-5 | O | Q-79 ($R_7$=H, $R_8$=H, $R_9$=H, $R_3$=$CH_3$) | $CH_3$ | H | $OCH_3$ | $OCH_3$ |
| J-6 | S | Q-79 ($R_7$=$CH_3$, $R_8$=H, $R_9$=H, $R_3$=$C_2H_5$) | H | 5-$CH_3$ | $OCH_3$ | $OCH_3$ |
| J-7 | S | Q-80 ($R_7$=H, $R_8$=H, $R_9$=H, $R_3$=$CH_3$) | H | H | $OCH_3$ | $OCH_3$ |
| J-5 | S | Q-80 ($R_7$=$CH_3$, $R_8$=H, $R_9$=H, $R_3$=$CH_3$) | H | H | $OCF_2H$ | $CH_3$ |
| J-6 | S | Q-80 ($R_7$=H, $R_8$=H, $R_9$=$CH_3$, $R_3$=$C_2H_5$) | H | 5-Cl | $OCH_3$ | $CH_3$ |
| J-5 | S | Q-81 ($R_7$=H, $R_8$=H, $R_9$=H, $R_3$=$CH_3$) | H | H | $CH_3$ | $OCH_3$ |
| J-6 | S | Q-81 ($R_7$=H, $R_8$=$CH_3$, $R_9$=H, $R_3$=i-$C_3H_7$) | H | H | $CH_3$ | $OCH_3$ |
| J-5 | S | Q-81 ($R_7$=$CH_3$, $R_8$=H, $R_9$=$CH_3$, $R_3$=$CH_3$) | H | 5-$CH_3$ | $OCH_3$ | $OCH_3$ |
| J-6 | S | Q-82 ($R_7$=H, $R_8$=H, $R_9$=H, $R_3$=$CH_3$) | H | H | $OCH_3$ | $OCH_3$ |
| J-5 | S | Q-82 ($R_7$=$CH_3$, $R_8$=H, $R_9$=$CH_3$, $R_3$=H) | H | H | Cl | $OCH_3$ |
| J-6 | O | Q-82 ($R_7$=H, $R_8$=H, $R_9$=$C_2H_5$, $R_3$=$CH_3$) | H | H | $CH_3$ | $OCH_3$ |
| J-5 | S | Q-83 ($R_7$=H, $R_8$=H, $R_9$=H, $R_{10}$=H) | H | H | $CH_3$ | $OCH_3$ |
| J-6 | S | Q-83 ($R_7$=$CH_3$, $R_8$=H, $R_9$=H, $R_{10}$=$CH_3$) | $CH_3$ | H | $CH_3$ | $OCH_3$ |
| J-7 | S | Q-83 ($R_7$=$CH_3$, $R_8$=$CH_3$, $R_9$=H, $R_{10}$=H) | H | H | $OCH_3$ | $OCH_3$ |
| J-5 | S | Q-84 ($R_7$=H, $R_8$=H, $R_9$=H) | H | H | $OCH_3$ | $OCH_3$ |
| J-6 | S | Q-84 ($R_7$=H, $R_8$=H, $R_9$=$CH_3$) | H | H | $OCH_3$ | $OCH_3$ |
| J-5 | S | Q-84 ($R_7$=H, $R_8$=$C_2H_5$, $R_9$=H) | H | 5-$CH_3$ | $OCH_3$ | $OCH_3$ |
| J-6 | S | Q-85 ($R_7$=H, $R_8$=H, $R_9$=H) | H | H | $OCH_3$ | $OCH_3$ |

Table 1 (continued)

| J | $W_3$ | Q | R | $R_{1b}$ | X | Y |
|---|---|---|---|---|---|---|
| J-5 | S | Q-85 ($R_7$=$CH_3$, $R_8$=H, $R_9$=H) | H | H | $OCH_3$ | $OCF_2H$ |
| J-6 | S | Q-85 ($R_7$=H, $R_8$=H, $R_9$=$C_2H_5$) | H | 5-Cl | $OCH_3$ | $OCH_3$ |
| J-5 | O | Q-86 ($R_7$=H, $R_8$=H, $R_9$=H) | H | H | $OCH_3$ | $OCH_3$ |
| J-6 | S | Q-86 ($R_7$=$CH_3$, $R_8$=H, $R_9$=H) | $CH_3$ | H | $OCH_3$ | $OCH_3$ |
| J-5 | S | Q-86 ($R_7$=H, $R_8$=H, $R_9$=$C_2H_5$) | H | 5-$CH_3$ | $OCH_3$ | $OCH_3$ |
| J-6 | S | Q-87 ($R_7$=H, $R_8$=H, $R_9$=H) | H | H | $OCH_3$ | $OCH_3$ |
| J-7 | S | Q-87 ($R_7$=H, $R_8$=H, $R_9$=$CH_3$) | H | H | $OCF_2H$ | $CH_3$ |
| J-5 | S | Q-87 ($R_7$=$CH_3$, $R_8$=H, $R_9$=H) | H | 5-Cl | $OCH_3$ | $CH_3$ |
| J-6 | S | Q-88 ($R_7$=H, $R_8$=H, $R_9$=H) | H | H | $CH_3$ | $OCH_3$ |
| J-5 | S | Q-88 ($R_7$=$CH_3$, $R_8$=H, $R_9$=H) | H | H | $CH_3$ | $OCH_3$ |
| J-6 | S | Q-88 ($R_7$=H, $R_8$=H, $R_9$=$CH_3$) | H | 5-$CH_3$ | $OCH_3$ | OCH |
| J-5 | S | Q-89 ($R_7$=H, $R_8$=H, $R_9$=H) | H | H | $OCH_3$ | $OCH_3$ |
| J-6 | O | Q-89 ($R_7$=$CH_3$, $R_8$=H, $R_9$=$C_2H_5$) | H | H | Cl | $OCH_3$ |
| J-5 | S | Q-89 ($R_7$=H, $R_8$=$CH_3$, $R_9$=H) | H | H | $CH_3$ | OCH |
| J-6 | S | Q-90 ($R_7$=H, $R_8$=H, $R_9$=H, $R_3$=$CH_3$) | H | H | $CH_3$ | $OCH_3$ |
| J-5 | S | Q-90 ($R_7$=$CH_3$, $R_8$=H, $R_9$=H, $R_3$=$C_2H_5$) | $CH_3$ | H | $CH_3$ | $OCH_3$ |
| J-6 | S | Q-90 ($R_7$=H, $R_8$=H, $R_9$=$CH_3$, $R_3$=$CH_3$) | H | H | $OCH_3$ | $OCH_3$ |
| J-7 | S | Q-91 ($R_7$=H, $R_8$=H, $R_9$=H, $R_3$=$CH_3$) | H | H | $OCH_3$ | $OCH_3$ |
| J-5 | S | Q-91 ($R_7$=$CH_3$, $R_8$=H, $R_9$=H, $R_3$=H) | H | H | $OCH_3$ | $OCH_3$ |
| J-6 | S | Q-91 ($R_7$=H, $R_8$=H, $R_9$=$CH_3$, $R_3$=$CH_3$) | H | 5-$CH_3$ | $OCH_3$ | $OCH_3$ |
| J-5 | S | Q-92 ($R_7$=H, $R_8$=H, $R_9$=H, $R_{10}$=H) | H | H | $OCH_3$ | $OCH_3$ |
| J-6 | S | Q-92 ($R_7$=H, $R_8$=H, $R_9$=H, $R_{10}$=$CH_3$) | H | H | $OCH_3$ | $OCF_2H$ |
| J-5 | O | Q-92 ($R_7$=H, $R_8$=$CH_3$, $R_9$=H, $R_{10}$=H) | H | 5-Cl | $OCH_3$ | $OCH_3$ |
| J-6 | S | Q-93 ($R_7$=H, $R_8$=H, $R_9$=H) | H | H | $OCH_3$ | $OCH_3$ |
| J-5 | S | Q-93 ($R_7$=H, $R_8$=H, $R_9$=$CH_3$) | $CH_3$ | H | $OCH_3$ | $OCH_3$ |
| J-6 | S | Q-93 ($R_7$=$CH_3$, $R_8$=H, $R_9$=H) | H | 5-$CH_3$ | $OCH_3$ | $OCH_3$ |
| J-5 | S | Q-94 ($R_7$=H, $R_8$=H, $R_9$=H) | H | H | $OCH_3$ | $OCH_3$ |
| J-6 | NH | Q-94 ($R_7$=$CH_3$, $R_8$=H, $R_9$=H) | H | H | $OCF_2H$ | $CH_3$ |
| J-7 | S | Q-94 ($R_7$=H, $R_8$=H, $R_9$=n-$C_4H_9$) | H | 5-Cl | $OCH_3$ | $CH_3$ |
| J-5 | S | Q-95 ($R_7$=H, $R_8$=H, $R_9$=H) | H | H | $CH_3$ | $OCH_3$ |
| J-6 | S | Q-95 ($R_7$=H, $R_8$=H, $R_9$=$CH_3$) | H | H | $CH_3$ | $OCH_3$ |
| J-5 | S | Q-95 ($R_7$=H, $R_8$=H, $R_9$=s-$C_4H_9$) | H | 5-$CH_3$ | $OCH_3$ | $OCH_3$ |
| J-6 | O | Q-96 ($R_7$=H, $R_8$=H, $R_9$=H) | H | H | $OCH_3$ | $OCH_3$ |

Table 1 (continued)

| J | $W_3$ | Q | R | $R_{1b}$ | X | Y | m.p.(°C) |
|---|---|---|---|---|---|---|---|
| J-5 | S | Q-96 ($R_7$=$CH_3$, $R_8$=H, $R_9$=H) | H | H | Cl | $OCH_3$ | |
| J-6 | S | Q-96 ($R_7$=H, $R_8$=H, $R_9$=$CH_3$) | H | H | $CH_3$ | $OCH_3$ | |
| J-5 | S | Q-97 ($R_7$=H, $R_8$=H, $R_9$=H) | H | H | $CH_3$ | $OCH_3$ | |
| J-6 | S | Q-97 ($R_7$=$CH_3$, $R_8$=$CH_3$, $R_9$=$CH_3$) | $CH_3$ | H | $CH_3$ | $OCH_3$ | |
| J-5 | S | Q-97 ($R_7$=H, $R_8$=H, $R_9$=$CH_3$) | H | H | $OCH_3$ | $OCH_3$ | |
| J-6 | S | Q-98 ($R_7$=H, $R_8$=H, $R_9$=H) | H | H | $OCH_3$ | $OCH_3$ | |
| J-7 | S | Q-98 ($R_7$=$CH_3$, $R_8$=H, $R_9$=H) | H | H | $OCH_3$ | $OCH_3$ | |
| J-5 | S | Q-98 ($R_7$=H, $R_8$=H, $R_9$=$\underline{i}$-$C_3H_7$) | H | 5-$CH_3$ | $OCH_3$ | $OCH_3$ | |
| J-6 | S | Q-99 ($R_7$=H, $R_8$=H, $R_9$=H, $R_3$=H) | H | H | $OCH_3$ | $OCH_3$ | |
| J-5 | O | Q-99 ($R_7$=H, $R_8$=H, $R_9$=$CH_3$, $R_3$=$CH_3$) | H | H | $OCH_3$ | $OCF_2H$ | |
| J-6 | S | Q-99 ($R_7$=H, $R_8$=H, $R_9$=$\underline{s}$-$C_4H_9$, $R_3$=H) | H | 5-Cl | $OCH_3$ | OCH | |
| J-5 | S | Q-100 ($R_7$=H, $R_8$=H, $R_9$=H, $R_3$=$C_2H_5$) | H | H | $OCH_3$ | $OCH_3$ | |
| J-6 | S | Q-100 ($R_7$=$CH_3$, $R_8$=H, $R_9$=H, $R_3$=H) | $CH_3$ | H | $OCH_3$ | $CH_3$ | |
| J-5 | S | Q-100 ($R_7$=H, $R_8$=H, $R_9$=$CH_3$, $R_3$=$CH_3$) | H | 5-$CH_3$ | $OCH_3$ | $OCH_3$ | |
| J-6 | S | Q-101 ($R_7$=H, $R_8$=H, $R_9$=H, $R_3$=$\underline{i}$-$C_3H_7$) | H | H | $OCH_3$ | $OCH_3$ | |
| J-5 | S | Q-101 ($R_7$=$CH_3$, $R_8$=$CH_3$, $R_9$=$CH_3$, $R_3$=H) | H | H | $OCF_2H$ | $CH_3$ | |
| J-6 | S | Q-101 ($R_7$=H, $R_8$=H, $R_9$=$CH_3$, $R_3$=$CH_3$) | H | 5-Cl | $OCH_3$ | $CH_3$ | |
| J-7 | S | Q-102 ($R_7$=H, $R_8$=H, $R_9$=H, $R_3$=H) | H | H | $CH_3$ | $OCH_3$ | |
| J-5 | S | Q-102 ($R_7$=$CH_3$, $R_8$=H, $R_9$=H, $R_3$=$CH_3$) | H | H | $CH_3$ | $OCH_3$ | |
| J-6 | O | Q-102 ($R_7$=H, $R_8$=H, $R_9$=$\underline{i}$-$C_3H_7$, $R_3$=$CH_3$) | H | 5-$CH_3$ | $OCH_3$ | $OCH_3$ | |
| J-6 | S | Q-104 ($R_7$=H, $R_8$=H, $R_9$=H, $R_2$=H) | H | H | $CH_3$ | $OCH_3$ | |
| J-5 | S | Q-104 ($R_7$=$CH_3$, $R_8$=H, $R_9$=H, $R_2$=$OC_2H_5$) | $CH_3$ | H | $CH_3$ | $OCH_3$ | |
| J-6 | S | Q-104 ($R_7$=H, $R_8$=H, $R_9$=H, $R_2$=Br) | H | H | $OCH_3$ | $OCH_3$ | |
| J-5 | S | Q-105 ($R_7$=H, $R_8$=H, $R_9$=H, $R_2$=H) | H | H | $OCH_3$ | $OCH_3$ | |
| J-6 | S | Q-105 ($R_7$=$CH_3$, $R_8$=H, $R_9$=H, $R_2$=$CH_3$) | H | H | $OCH_3$ | $OCH_3$ | |
| J-7 | S | Q-105 ($R_7$=H, $R_8$=$CH_3$, $R_9$=H, $R_2$=$C_2H_5$) | H | 5-$CH_3$ | $OCH_3$ | $OCH_3$ | |
| J-6 | S | Q-105 ($R_7$=H, $R_8$=H, $R_9$=H, $R_2$=H) | H | H | $CH_3$ | $CH_3$ | 190-192 |
| J-6 | S | Q-105 ($R_7$=H, $R_8$=H, $R_9$=H, $R_2$=H) | H | H | $OCH_3$ | $CH_3$ | 186-188 |
| J-6 | S | Q-105 ($R_7$=H, $R_8$=H, $R_9$=H, $R_2$=H) | H | H | $OCH_3$ | $OCH_3$ | 182-183 |
| J-6 | S | Q-105 ($R_7$=H, $R_8$=H, $R_9$=H, $R_2$=H) | H | H | $OCH_3$ | Cl | 170-173 |

Table 1 (continued)

| J | $W_3$ | Q | R | $R_{1b}$ | X | Y |
|---|---|---|---|---|---|---|
| J-5 | O | Q-106 ($R_7$=H, $R_8$=H, $R_9$=H, $R_2$=H) | H | H | $OCH_3$ | $OCH_3$ |
| J-6 | S | Q-106 ($R_7$=$CH_3$, $R_8$=H, $R_9$=H, $R_2$=$CH_3$) | H | H | $OCH_3$ | $OCF_2H$ |
| J-5 | S | Q-106 ($R_7$=Cl, $R_8$=H, $R_9$=H, $R_2$=H) | H | 5-Cl | $OCH_3$ | $OCH_3$ |
| J-6 | S | Q-107 ($R_7$=H, $R_8$=H, $R_9$=H, $R_2$=H) | H | H | $OCH_3$ | $OCH_3$ |
| J-5 | S | Q-107 ($R_7$=$CH_3$, $R_8$=H, $R_9$=H, $R_2$=H) | $CH_3$ | H | $OCH_3$ | $OCH_3$ |
| J-6 | S | Q-107 ($R_7$=H, $R_8$=H, $R_9$=H, $R_2$=$CH_3$) | H | 5-$CH_3$ | $OCH_3$ | $OCH_3$ |
| J-5 | S | Q-108 ($R_7$=H, $R_8$=H, $R_9$=H, $R_2$=H) | H | H | $OCH_3$ | $OCH_3$ |
| J-6 | S | Q-108 ($R_7$=$CH_3$, $R_8$=H, $R_9$=H, $R_2$=Cl) | H | H | $OCF_2H$ | $CH_3$ |
| J-5 | S | Q-108 ($R_7$=H, $R_8$=H, $R_9$=H, $R_2$=$CH_3$) | H | 5-Cl | $OCH_3$ | $CH_3$ |
| J-6 | S | Q-109 ($R_7$=H, $R_8$=H, $R_9$=H) | H | H | $CH_3$ | $OCH_3$ |
| J-7 | O | Q-109 ($R_7$=$CH_3$, $R_8$=H, $R_9$=H) | H | H | $CH_3$ | $OCH_3$ |
| J-5 | S | Q-109 ($R_7$=H, $R_8$=H, $R_9$=$CH_3$) | H | 5-$CH_3$ | $OCH_3$ | $OCH_3$ |
| J-6 | S | Q-110 ($R_7$=H, $R_8$=H, $R_9$=H) | H | H | $OCH_3$ | $OCH_3$ |
| J-5 | S | Q-110 ($R_7$=$CH_3$, $R_8$=H, $R_9$=H) | H | H | Cl | $OCH_3$ |
| J-6 | S | Q-110 ($R_7$=H, $R_8$=$CH_3$, $R_9$=H) | H | H | $CH_3$ | $OCH_3$ |
| J-5 | S | Q-111 ($R_7$=H, $R_8$=H, $R_9$=H) | H | H | $CH_3$ | $OCH_3$ |
| J-6 | S | Q-111 ($R_7$=$CH_3$, $R_8$=H, $R_9$=H) | $CH_3$ | H | $CH_3$ | $OCH_3$ |
| J-5 | S | Q-111 ($R_7$=H, $R_8$=H, $R_9$=$CH_3$) | H | H | $OCH_3$ | OCH |
| J-6 | S | Q-112 ($R_7$=H, $R_8$=H, $R_9$=H) | H | H | $OCH_3$ | $OCH_3$ |
| J-5 | S | Q-112 ($R_7$=$CH_3$, $R_8$=H, $R_9$=H) | H | H | $OCH_3$ | $OCH_3$ |
| J-6 | O | Q-112 ($R_7$=H, $R_8$=$CH_3$, $R_9$=$CH_3$) | H | 5-$CH_3$ | $OCH_3$ | $OCH_3$ |
| J-7 | S | Q-113 ($R_7$=H, $R_8$=H, $R_9$=H, $R_3$=$CH_3$) | H | H | $OCH_3$ | $OCH_3$ |
| J-5 | S | Q-113 ($R_7$=$C_2H_5$, $R_8$=H, $R_9$=H, $R_3$=i-$C_3H_7$) | H | H | $OCH_3$ | $OCF_2H$ |
| J-6 | S | Q-113 ($R_7$=H, $R_8$=H, $R_9$=$C_2H_5$, $R_3$=n-$C_4H_9$) | H | 5-Cl | $OCH_3$ | $OCH_3$ |
| J-5 | S | Q-114 ($R_7$=H, $R_8$=H, $R_9$=H, $R_3$=$CH_3$) | H | H | $OCH_3$ | $OCH_3$ |
| J-6 | S | Q-114 ($R_7$=n-$C_4H_9$, $R_8$=H, $R_9$=H, $R_3$=$C_2H_5$) | $CH_3$ | H | $OCH_3$ | $CH_3$ |
| J-5 | S | Q-114 ($R_7$=H, $R_8$=$CH_3$, $R_8$=i-$C_3H_7$, $R_3$=H) | H | 5-$CH_3$ | $OCH_3$ | $OCH_3$ |
| J-6 | S | Q-115 ($R_7$=H, $R_8$=H, $R_9$=H, $R_3$=$CH_3$) | H | H | $OCH_3$ | $OCH_3$ |
| J-5 | S | Q-115 ($R_7$=i-$C_3H_7$, $R_8$=H, $R_9$=H, $R_3$=$CH_3$) | H | H | $OCF_2H$ | $CH_3$ |
| J-6 | S | Q-115 ($R_7$=H, $R_8$=$C_2H_5$, $R_9$=$CH_3$, $R_3$=H) | H | 5-Cl | $OCH_3$ | $CH_3$ |
| J-5 | O | Q-116 ($R_7$=H, $R_8$=H, $R_9$=H, $R_{10}$=H) | H | H | $CH_3$ | $OCH_3$ |
| J-6 | S | Q-116 ($R_7$=$CH_3$, $R_8$=H, $R_9$=H, $R_{10}$=H) | H | H | $CH_3$ | $OCH_3$ |
| J-7 | $NCH_3$ | Q-116 ($R_7$=H, $R_8$=H, $R_9$=H, $R_{10}$=$CH_3$) | H | 5-$CH_3$ | $OCH_3$ | $OCH_3$ |

Table 1 (continued)

| J | $W_3$ | Q | R | $R_{1b}$ | X | Y |
|---|---|---|---|---|---|---|
| J-6 | S | Q-118 ($R_7$=H, $R_8$=H, $R_9$=H, $R_{10}$=H) | H | H | $CH_3$ | $OCH_3$ |
| J-5 | S | Q-118 ($R_7$=$CH_3$, $R_8$=H, $R_9$=H, $R_{10}$=H) | $CH_3$ | H | $CH_3$ | $OCH_3$ |
| J-6 | S | Q-118 ($R_7$=H, $R_8$=H, $R_9$=H, $R_{10}$=$CH_3$) | H | H | $OCH_3$ | OCH |
| J-5 | S | Q-119 ($R_7$=H, $R_8$=H, $R_9$=H, $R_2$=H) | H | H | $OCH_3$ | $OCH_3$ |
| J-6 | O | Q-119 ($R_7$=H, $R_8$=H, $R_9$=H, $R_2$=$CH_3$) | H | H | $OCH_3$ | $OCH_3$ |
| J-5 | S | Q-119 ($R_7$=H, $R_8$=$CH_3$, $R_9$=H, $R_2$=$C_2H_5$) | H | 5-$CH_3$ | $OCH_3$ | $OCH_3$ |
| J-6 | S | Q-120 ($R_7$=H, $R_8$=H, $R_9$=H, $R_2$=H) | H | H | $OCH_3$ | $OCH_3$ |
| J-7 | S | Q-120 ($R_7$=H, $R_8$=H, $R_9$=H, $R_2$=$CH_3$) | H | H | $OCH_3$ | $OCF_2H$ |
| J-5 | S | Q-120 ($R_7$=H, $R_8$=H, $R_9$=$CH_3$, $R_2$=$CH_3$) | H | 5-Cl | $OCH_3$ | $OCH_3$ |
| J-6 | S | Q-121 ($R_7$=H, $R_8$=H, $R_9$=H, $R_2$=H) | H | H | $OCH_3$ | $OCH_3$ |
| J-5 | S | Q-121 ($R_7$=H, $R_8$=H, $R_9$=H, $R_2$=$C_2H_5$) | $CH_3$ | H | $OCH_3$ | $CH_3$ |
| J-6 | S | Q-121 ($R_7$=$CH_3$, $R_8$=$CH_3$, $R_9$=H, $R_2$=H) | H | 5-$CH_3$ | $OCH_3$ | $OCH_3$ |
| J-5 | S | Q-122 ($R_7$=H, $R_8$=H, $R_9$=H, $R_2$=H) | H | H | $OCH_3$ | $OCH_3$ |
| J-6 | O | Q-122 ($R_7$=H, $R_8$=H, $R_9$=H, $R_2$=$CH_3$) | H | H | $OCF_2H$ | $CH_3$ |
| J-5 | S | Q-122 ($R_7$=$CH_3$, $R_8$=H, $R_9$=H, $R_2$=H) | H | 5-Cl | $OCH_3$ | $CH_3$ |
| J-6 | S | Q-123 ($R_7$=H, $R_8$=H, $R_9$=H) | H | H | $CH_3$ | $OCH_3$ |
| J-5 | S | Q-123 ($R_7$=H, $R_8$=H, $R_9$=$CH_3$) | H | H | $CH_3$ | $OCH_3$ |
| J-6 | S | Q-123 ($R_7$=$CH_3$, $R_8$=H, $R_9$=$CH_3$) | H | 5-$CH_3$ | $OCH_3$ | $OCH_3$ |
| J-7 | S | Q-124 ($R_7$=H, $R_8$=H, $R_9$=H) | H | H | $OCH_3$ | $OCH_3$ |
| J-5 | S | Q-124 ($R_7$=H, $R_8$=H, $R_9$=$CH_3$) | H | H | Cl | $OCH_3$ |
| J-6 | S | Q-124 ($R_7$=$C_2H_5$, $R_8$=H, $R_9$=$C_2H_5$) | H | H | $CH_3$ | $OCH_3$ |
| J-5 | S | Q-125 ($R_7$=H, $R_8$=H, $R_9$=H) | H | H | $CH_3$ | $OCH_3$ |
| J-6 | S | Q-125 ($R_7$=H, $R_8$=H, $R_9$=$\underline{n}$-$C_4H_9$) | $CH_3$ | H | $CH_3$ | $OCH_3$ |
| J-5 | O | Q-125 ($R_7$=H, $R_8$=$CH_3$, $R_9$=H) | H | H | $OCH_3$ | $OCH_3$ |
| J-6 | S | Q-126 ($R_7$=H, $R_8$=H, $R_9$=H) | H | H | $OCH_3$ | $OCH_3$ |
| J-5 | S | Q-126 ($R_7$=H, $R_8$=H, $R_9$=$\underline{s}$-$C_3H_7$) | H | H | $OCH_3$ | $OCH_3$ |
| J-6 | S | Q-126 ($R_7$=$\underline{n}$-$C_3H_7$, $R_8$=H, $R_9$=H) | H | 5-$CH_3$ | $OCH_3$ | $OCH_3$ |
| J-5 | S | Q-127 ($R_7$=H, $R_8$=H, $R_9$=H, $R_3$=H) | H | H | $OCH_3$ | $OCH_3$ |
| J-6 | S | Q-127 ($R_7$=H, $R_8$=H, $R_9$=H, $R_3$=$CH_3$) | H | H | $OCH_3$ | $OCF_2H$ |
| J-7 | S | Q-127 ($R_7$=$CH_3$, $R_8$=H, $R_9$=H, $R_3$=$C_2H_5$) | H | 5-Cl | $OCH_3$ | $OCH_3$ |

Table 1 (continued)

| J | $W_3$ | Q | R | $R_{1b}$ | X | Y |
|---|---|---|---|---|---|---|
| J-5 | S | Q-128 ($R_7$=H, $R_8$=H, $R_9$=H, $R_3$=$CH_3$) | H | H | $OCH_3$ | $OCH_3$ |
| J-6 | S | Q-128 ($R_7$=$CH_3$, $R_8$=H, $R_9$=H, $R_3$=$CH_3$) | $CH_3$ | H | $OCH_3$ | $OCH_3$ |
| J-5 | S | Q-128 ($R_7$=H, $R_8$=H, $R_9$=$CH_3$, $R_3$=$C_2H_5$) | H | 5-$CH_3$ | $OCH_3$ | $OCH_3$ |
| J-6 | O | Q-129 ($R_7$=H, $R_8$=H, $R_9$=H, $R_3$=$CH_3$) | H | H | $OCH_3$ | $OCH_3$ |
| J-5 | S | Q-129 ($R_7$=H, $R_8$=$CH_3$, $R_9$=H, $R_3$=$\underline{i}$-$C_3H_7$) | H | H | $OCF_2H$ | $CH_3$ |
| J-6 | S | Q-129 ($R_7$=$CH_3$, $R_8$=H, $R_9$=$CH_3$, $R_3$=$CH_3$) | H | 5-Cl | $OCH_3$ | $CH_3$ |
| J-5 | S | Q-130 ($R_7$=H, $R_8$=H, $R_9$=H, $R_3$=$CH_3$) | H | H | $CH_3$ | $OCH_3$ |
| J-6 | S | Q-130 ($R_7$=$CH_3$, $R_8$=H, $R_9$=$CH_3$, $R_3$=H) | H | H | $CH_3$ | $OCH_3$ |
| J-5 | S | Q-130 ($R_7$=H, $R_8$=H, $R_9$=$C_2H_5$, $R_3$=$CH_3$) | H | 5-$CH_3$ | $OCH_3$ | $OCH_3$ |
| J-6 | S | Q-131 ($R_7$=H, $R_8$=H, $R_9$=H, $R_{10}$=H) | H | H | $OCH_3$ | $OCH_3$ |
| J-7 | S | Q-131 ($R_7$=$CH_3$, $R_8$=H, $R_9$=H, $R_{10}$=$CH_3$) | H | H | Cl | $OCH_3$ |
| J-5 | S | Q-131 ($R_7$=$CH_3$, $R_8$=$CH_3$, $R_9$=H, $R_{10}$=H) | H | H | $CH_3$ | $OCH_3$ |
| J-6 | S | Q-132 ($R_7$=H, $R_8$=H, $R_9$=H) | H | H | $CH_3$ | $OCH_3$ |
| J-5 | O | Q-132 ($R_7$=H, $R_8$=H, $R_9$=$CH_3$) | $CH_3$ | H | $CH_3$ | $OCH_3$ |
| J-6 | S | Q-132 ($R_7$=H, $R_8$=$C_2H_5$, $R_9$=H) | H | H | $OCH_3$ | $OCH_3$ |
| J-5 | S | Q-133 ($R_7$=H, $R_8$=H, $R_9$=H) | H | H | $OCH_3$ | $OCH_3$ |
| J-6 | S | Q-133 ($R_7$=$CH_3$, $R_8$=H, $R_9$=H) | H | H | $OCH_3$ | $OCH_3$ |
| J-5 | S | Q-133 ($R_7$=H, $R_8$=H, $R_9$=$C_2H_5$) | H | 5-$CH_3$ | $OCH_3$ | $OCH_3$ |
| J-6 | S | Q-134 ($R_7$=H, $R_8$=H, $R_9$=H) | H | H | $OCH_3$ | $OCH_3$ |
| J-5 | S | Q-134 ($R_7$=$CH_3$, $R_8$=H, $R_9$=H) | H | H | $OCH_3$ | $OCF_2H$ |
| J-6 | S | Q-134 ($R_7$=H, $R_8$=H, $R_9$=$C_2H_5$) | H | 5-Cl | $OCH_3$ | $OCH_3$ |
| J-7 | S | Q-135 ($R_7$=H, $R_8$=H, R =H) | H | H | $OCH_3$ | $OCH_3$ |
| J-5 | S | Q-135 ($R_7$=H, $R_8$=H, R =$CH_3$) | $CH_3$ | H | $OCH_3$ | $OCH_3$ |
| J-6 | O | Q-135 ($R_7$=$CH_3$, $R_8$=H, $R_9$=H) | H | 5-$CH_3$ | $OCH_3$ | $OCH_3$ |
| J-5 | S | Q-136 ($R_7$=H, $R_8$=H, $R_9$=H) | H | H | $OCH_3$ | $OCH_3$ |
| J-6 | S | Q-136 ($R_7$=H, $R_8$=H, $R_9$=$CH_3$) | H | H | $OCF_2H$ | $CH_3$ |
| J-5 | S | Q-136 ($R_7$=H, $R_8$=$CH_3$, $R_9$=$C_2H_5$) | H | 5-Cl | $OCH_3$ | $CH_3$ |
| J-6 | S | Q-137 ($R_7$=H, $R_8$=H, $R_9$=H) | H | H | $CH_3$ | $OCH_3$ |
| J-5 | S | Q-137 ($R_7$=$CH_3$, $R_8$=H, $R_9$=H) | H | H | $CH_3$ | $OCH_3$ |
| J-6 | S | Q-137 ($R_7$=H, $R_8$=H, $R_9$=$CH_3$) | H | 5-$CH_3$ | $OCH_3$ | $OCH_3$ |
| J-5 | S | Q-138 ($R_7$=H, $R_8$=H, $R_9$=H) | H | H | $OCH_3$ | $OCH_3$ |

Table 1 (continued)

| J | $W_3$ | Q | R | $R_{1b}$ | X | Y | m.p.(°C) |
|---|---|---|---|---|---|---|---|
| J-6 | S | Q-138 ($R_7$=H, $R_8$=H, $R_9$=H) | H | H | $CH_3$ | $CH_3$ | 87-95 |
| J-6 | S | Q-138 ($R_7$=H, $R_8$=H, $R_9$=H) | H | H | $OCH_3$ | $CH_3$ | 73-84 |
| J-6 | S | Q-138 ($R_7$=H, $R_8$=H, $R_9$=H) | H | H | $OCH_3$ | $OCH_3$ | 89-93 |
| J-6 | S | Q-138 ($R_7$=H, $R_8$=H, $R_9$=H) | H | H | $OCH_3$ | Cl | 85-93 |
| J-6 | S | Q-138 ($R_7$=$CH_3$, $R_8$=H, $R_9$=H) | H | H | Cl | $OCH_3$ | |
| J-7 | S | Q-138 ($R_7$=H, $R_8$=H, $R_9$=$CH_3$) | H | H | $CH_3$ | $OCH_3$ | |
| J-5 | O | Q-139 ($R_7$=H, $R_8$=H, $R_9$=H) | H | H | $CH_3$ | $OCH_3$ | |
| J-6 | S | Q-139 ($R_7$=$CH_3$, $R_8$=H, $R_9$=H) | $CH_3$ | H | $CH_3$ | $OCH_3$ | |
| J-5 | S | Q-139 ($R_7$=$CH_3$, $R_8$=$CH_3$, $R_9$=H) | H | H | $OCH_3$ | $OCH_3$ | |
| J-6 | S | Q-140 ($R_7$=H, $R_8$=H, $R_9$=H, $R_3$=$CH_3$) | H | H | $OCH_3$ | $OCH_3$ | |
| J-5 | S | Q-140 ($R_7$=H, $R_8$=H, $R_9$=$CH_3$, $R_3$=H) | H | H | $OCH_3$ | $OCH_3$ | |
| J-6 | S | Q-140 ($R_7$=H, $R_8$=$CH_3$, $R_9$=H, $R_3$=$C_2H_5$) | H | 5-$CH_3$ | $OCH_3$ | $OCH_3$ | |
| J-5 | S | Q-141 ($R_7$=H, $R_8$=H, $R_9$=H, $R_3$=$CH_3$) | H | H | $OCH_3$ | $OCH_3$ | |
| J-6 | S | Q-141 ($R_7$=H, $R_8$=H, $R_9$=H, $R_3$=H) | H | H | $OCH_3$ | $OCF_2H$ | |
| J-5 | S | Q-141 ($R_7$=H, $R_8$=H, $R_9$=$CH_3$, $R_3$=$CH_3$) | H | 5-Cl | $OCH_3$ | $OCH_3$ | |
| J-6 | S | Q-142 ($R_7$=H, $R_8$=H, $R_9$=H, $R_{10}$=H) | H | H | $OCH_3$ | $OCH_3$ | |
| J-7 | O | Q-142 ($R_7$=$CH_3$, $R_8$=H, $R_9$=H, $R_{10}$=H) | $CH_3$ | H | $OCH_3$ | $CH_3$ | |
| J-5 | S | Q-142 ($R_7$=H, $R_8$=H, $R_9$=H, $R_{10}$=$CH_3$) | H | 5-$CH_3$ | $OCH_3$ | $OCH_3$ | |
| J-6 | S | Q-143 ($R_7$=H, $R_8$=H, $R_9$=H, $R_3$=$CH_3$) | H | H | $OCH_3$ | $OCH_3$ | |
| J-5 | S | Q-143 ($R_7$=H, $R_8$=H, $R_9$=H, $R_3$=H) | H | H | $OCF_2H$ | $CH_3$ | |
| J-6 | S | Q-143 ($R_7$=$CH_3$, $R_8$=H, $R_9$=$CH_3$, $R_3$=$CH_3$) | H | 5-Cl | $OCH_3$ | $CH_3$ | |
| J-5 | NH | Q-144 ($R_7$=H, $R_8$=H, $R_9$=H, $R_3$=$CH_3$) | H | H | $CH_3$ | $OCH_3$ | |
| J-6 | S | Q-144 ($R_7$=$CH_3$, $R_8$=H, $R_9$=H, $R_3$=$CH_3$) | H | H | $CH_3$ | $OCH_3$ | |
| J-5 | S | Q-144 ($R_7$=H, $R_8$=$C_2H_5$, $R_9$=H, $R_3$=$CH_3$) | H | 5-$CH_3$ | $OCH_3$ | $OCH_3$ | |
| J-6 | S | Q-145 ($R_7$=H, $R_8$=H, $R_9$=H) | H | H | $OCH_3$ | $OCH_3$ | |
| J-5 | S | Q-145 ($R_7$=H, $R_8$=H, $R_9$=$CH_3$) | H | H | Cl | $OCH_3$ | |
| J-6 | O | Q-145 ($R_7$=$CH_3$, $R_8$=H, $R_9$=H) | H | H | $CH_3$ | $OCH_3$ | |
| J-7 | S | Q-146 ($R_7$=H, $R_8$=H, $R_9$=H) | H | H | $CH_3$ | $OCH_3$ | |
| J-5 | S | Q-146 ($R_7$=$CH_3$, $R_8$=H, $R_9$=H) | $CH_3$ | H | $CH_3$ | $OCH_3$ | |
| J-6 | S | Q-146 ($R_7$=H, $R_8$=H, $R_9$=n-$C_4H_9$) | H | H | $OCH_3$ | $OCH_3$ | |
| J-5 | S | Q-147 ($R_7$=H, $R_8$=H, $R_9$=H) | H | H | $OCH_3$ | $OCH_3$ | |
| J-6 | S | Q-147 ($R_7$=$CH_3$, $R_8$=H, $R_9$=H) | H | H | $OCH_3$ | $OCH_3$ | |
| J-5 | S | Q-147 ($R_7$=H, $R_8$=H, $R_9$=n-$C_4H_9$) | H | 5-$CH_3$ | $OCH_3$ | $OCH_3$ | |

Table 1 (continued)

| J | $W_3$ | Q | R | $R_{1b}$ | X | Y |
|---|---|---|---|---|---|---|
| J-6 | S | Q-148 ($R_7$=H, $R_8$=H, $R_9$=H) | H | H | $OCH_3$ | $OCH_3$ |
| J-5 | S | Q-148 ($R_7$=H, $R_8$=H, $R_9$=$CH_3$) | H | H | $OCH_3$ | $OCF_2H$ |
| J-6 | S | Q-148 ($R_7$=$CH_3$, $R_8$=H, $R_9$=H) | H | 5-Cl | $OCH_3$ | $OCH_3$ |
| J-5 | O | Q-149 ($R_7$=H, $R_8$=H, $R_9$=H) | H | H | $OCH_3$ | $OCH_3$ |
| J-6 | S | Q-149 ($R_7$=H, $R_8$=H, $R_9$=$CH_3$) | $CH_3$ | H | $OCH_3$ | $OCH_3$ |
| J-7 | S | Q-149 ($R_7$=$CH_3$, $R_8$=H, $R_9$=H) | H | 5-$CH_3$ | $OCH_3$ | $OCH_3$ |
| J-5 | S | Q-150 ($R_7$=H, $R_8$=H, $R_9$=H) | H | H | $OCH_3$ | $OCH_3$ |
| J-6 | S | Q-150 ($R_7$=$CH_3$, $R_8$=H, $R_9$=H) | H | H | $OCF_2H$ | $CH_3$ |
| J-5 | S | Q-150 ($R_7$=H, $R_8$=$CH_3$, $R_9$=$CH_3$) | H | 5-Cl | $OCH_3$ | $CH_3$ |
| J-6 | S | Q-151 ($R_7$=H, $R_8$=H, $R_9$=H) | H | H | $CH_3$ | $OCH_3$ |
| J-5 | S | Q-151 ($R_7$=$CH_3$, $R_8$=H, $R_9$=H) | H | H | $CH_3$ | $OCH_3$ |
| J-6 | S | Q-151 ($R_7$=$CH_3$, $R_8$=$CH_3$, $R_9$=H) | H | 5-$CH_3$ | $OCH_3$ | $OCH_3$ |
| J-5 | S | Q-152 ($R_7$=H, $R_8$=H, $R_9$=H) | H | H | $OCH_3$ | $OCH_3$ |
| J-6 | O | Q-152 ($R_7$=$C_2H_5$, $R_8$=H, $R_9$=H) | H | H | Cl | $OCH_3$ |
| J-5 | S | Q-152 ($R_7$=$CH_3$, $R_8$=$CH_3$, $R_9$=H) | H | H | $CH_3$ | $OCH_3$ |
| J-6 | S | Q-153 ($R_7$=H, $R_8$=H, $R_9$=H, $R_{11}$=$CH_3$) | H | H | $CH_3$ | $OCH_3$ |
| J-7 | S | Q-153 ($R_7$=H, $R_8$=H, $R_9$=H, $R_{11}$=H) | $CH_3$ | H | $CH_3$ | $OCH_3$ |
| J-5 | S | Q-153 ($R_7$=$CH_3$, $R_8$=H, $R_9$=H, $R_{11}$=$CH_3$) | H | H | $OCH_3$ | $OCH_3$ |
| J-6 | S | Q-154 ($R_7$=H, $R_8$=H, $R_9$=H, $R_{11}$=$CH_3$) | H | H | $OCH_3$ | $OCH_3$ |
| J-5 | S | Q-154 ($R_7$=$CH_3$, $R_8$=H, $R_9$=H) | H | H | $OCH_3$ | $OCH_3$ |
| J-6 | S | Q-154 ($R_7$=H, $R_8$=$CH_3$, $R_9$=$CH_3$) | H | 5-$CH_3$ | $OCH_3$ | $OCH_3$ |
| J-5 | S | Q-155 ($R_7$=H, $R_8$=H, $R_9$=H) | H | H | $OCH_3$ | $OCH_3$ |
| J-6 | S | Q-155 ($R_7$=$CH_3$, $R_8$=H, $R_9$=H) | H | H | $OCH_3$ | $OCF_2H$ |
| J-5 | O | Q-155 ($R_7$=$CH_3$, $R_8$=$CH_3$, $R_9$=H) | H | 5-Cl | $OCH_3$ | $OCH_3$ |
| J-6 | S | Q-156 ($R_7$=H, $R_8$=H, $R_9$=H) | H | H | $OCH_3$ | $OCH_3$ |
| J-5 | S | Q-156 ($R_7$=$C_2H_5$, $R_8$=H, $R_9$=H) | $CH_3$ | H | $OCH_3$ | $OCH_3$ |
| J-6 | $NCH_3$ | Q-156 ($R_7$=$CH_3$, $R_8$=$CH_3$, $R_9$=H) | H | 5-$CH_3$ | $OCH_3$ | $OCH_3$ |
| J-5 | S | Q-158 ($R_7$=H, $R_8$=H, $R_9$=H) | H | H | $CH_3$ | $OCH_3$ |
| J-6 | S | Q-158 ($R_7$=H, $R_8$=H, $R_9$=$CH_3$) | H | H | $CH_3$ | $OCH_3$ |
| J-5 | S | Q-158 ($R_7$=$CH_3$, $R_8$=H, $R_9$=H) | H | 5-$CH_3$ | $OCH_3$ | $OCH_3$ |

Table 1 (continued)

| J | $W_3$ | Q | R | $R_{1b}$ | X | Y |
|---|---|---|---|---|---|---|
| J-6 | O | Q-159 ($R_7$=H, $R_8$=H, $R_9$=H) | H | H | $OCH_3$ | $OCH_3$ |
| J-5 | S | Q-159 ($R_7$=$CH_3$, $R_8$=H, $R_9$=H) | H | H | Cl | $OCH_3$ |
| J-6 | S | Q-159 ($R_7$=H, $R_8$=H, $R_9$=$CH_3$) | H | H | $CH_3$ | $OCH_3$ |
| J-5 | S | Q-160 ($R_7$=H, $R_8$=H, $R_9$=H, $R_{11}$=$CH_3$) | H | H | $CH_3$ | $OCH_3$ |
| J-6 | S | Q-160 ($R_7$=H, $R_8$=H, $R_9$=$CH_3$, $R_{11}$=H) | $CH_3$ | H | $CH_3$ | $OCH_3$ |
| J-7 | S | Q-160 ($R_7$=$CH_3$, $R_8$=H, $R_9$=H, $R_{11}$=$\underline{i}$-$C_3H_7$) | H | H | $OCH_3$ | $OCH_3$ |
| J-5 | S | Q-161 ($R_7$=H, $R_8$=H, $R_9$=H, $R_{11}$=$CH_3$) | H | H | $OCH_3$ | $OCH_3$ |
| J-6 | S | Q-161 ($R_7$=$CH_3$, $R_8$=H, $R_9$=H, $R_{11}$=H) | H | H | $OCH_3$ | $OCH_3$ |
| J-5 | S | Q-161 ($R_7$=H, $R_8$=H, $R_9$=$CH_3$, $R_{11}$=$CH_3$) | H | 5-$CH_3$ | $OCH_3$ | $OCH_3$ |
| J-6 | S | Q-162 ($R_7$=H, $R_8$=H, $R_9$=H, $R_{10}$=H) | H | H | $OCH_3$ | $OCH_3$ |
| J-5 | O | Q-162 ($R_7$=$CH_3$, $R_8$=H, $R_9$=H, $R_{10}$=H) | H | H | $OCH_3$ | $OCF_2H$ |
| J-6 | S | Q-162 ($R_7$=H, $R_8$=H, $R_9$=H, $R_{10}$=$CH_3$) | H | 5-Cl | $OCH_3$ | $OCH_3$ |
| J-5 | S | Q-163 ($R_7$=H, $R_8$=H, $R_9$=H, $R_2$=H) | H | H | $CH_3$ | $OCH_3$ |
| J-6 | O | Q-163 ($R_7$=H, $R_8$=H, $R_9$=H, $R_2$=H) | H | H | $OCH_3$ | $OCH_3$ |
| J-6 | S | Q-163 ($R_7$=H, $R_8$=H, $R_9$=H, $R_2$=H) | H | H | $OCH_3$ | $OCH_3$ |
| J-5 | S | Q-164 ($R_7$=H) | H | H | $OCH_3$ | $OCH_3$ |
| J-6 | O | Q-164 ($R_7$=H) | H | H | $OCH_3$ | $OCH_3$ |
| J-6 | S | Q-164 ($R_7$=H) | H | H | $OCH_3$ | $OCH_3$ |
| J-5 | S | Q-165 ($R_7$=H, $R_3$=$CH_3$) | H | H | $OCH_3$ | $OCH_3$ |
| J-6 | O | Q-165 ($R_7$=H, $R_3$=$CH_3$) | H | H | $OCH_3$ | $OCH_3$ |
| J-6 | S | Q-165 ($R_7$=H, $R_3$=$CH_3$) | H | H | $OCH_3$ | $OCH_3$ |
| J-5 | S | Q-166 ($R_7$=H, $R_3$=$CH_3$) | H | H | $OCH_3$ | $OCH_3$ |
| J-6 | O | Q-166 ($R_7$=H, $R_3$=$CH_3$) | H | H | $OCH_3$ | $OCH_3$ |
| J-6 | S | Q-166 ($R_7$=H, $R_3$=$CH_3$) | H | H | $OCH_3$ | $OCH_3$ |
| J-5* | S | Q-1($R_7$=H, $R_8$=H) | H | H | $OCH_3$ | $OCH_3$ |
| J-5* | S | Q-1($R_7$=H, $R_8$=H) | H | H | $OCH_3$ | $CH_3$ |
| J-6* | S | Q-1($R_7$=H, $R_8$=H) | H | H | $OCH_3$ | $OCH_3$ |
| J-7* | S | Q-1($R_7$=H, $R_8$=H) | H | H | $OCH_3$ | $OCH_3$ |
| J-6* | S | Q-37($R_7$=H, $R_8$=H, $R_2$=H) | H | H | $OCH_3$ | $OCH_3$ |
| J-6* | S | Q-60($R_7$=H, $R_8$=H, $R_9$=H, $R_2$=H) | H | H | $OCH_3$ | $OCH_3$ |
| J-6* | S | Q-105($R_7$=H, $R_8$=H, $R_9$=H, $R_2$=H) | H | H | $OCH_3$ | $OCH_3$ |
| J-6* | S | Q-138($R_7$=H, $R_8$=H, $R_9$=H) | H | H | $OCH_3$ | $OCH_3$ |

Table 1 (continued)

| J | Q | R | $R_{1c}$ | X | Y |
|---|---|---|---|---|---|
| J-8 | Q-1 ($R_7$=H, $R_8$=H) | H | H | $CH_3$ | $CH_3$ |
| J-8 | Q-1 ($R_7$=H, $R_8$=H) | H | H | $OCH_3$ | $CH_3$ |
| J-8 | Q-1 ($R_7$=H, $R_8$=H) | H | H | $OCH_3$ | $OCH_3$ |
| J-8 | Q-1 ($R_7$=H, $R_8$=H) | H | 5-$CH_3$ | $OCH_3$ | $OCH_3$ |
| J-8 | Q-1 ($R_7$=H, $R_8$=H) | H | 5-$OCH_3$ | $OCH_3$ | $OCH_3$ |
| J-8 | Q-1 ($R_7$=H, $R_8$=H) | H | 6-$OCH_2CH_2Br$ | $OCH_3$ | $OCH_3$ |
| J-8 | Q-1 ($R_7$=H, $R_8$=H) | H | 5-$CH_2F$ | $OCH_3$ | $OCH_3$ |
| J-8 | Q-1 ($R_7$=H, $R_8$=H) | H | 5-$SCH_3$ | $OCH_3$ | $OCH_3$ |
| J-8 | Q-1 ($R_7$=H, $R_8$=H) | H | 5-$SCH_2F$ | $OCH_3$ | $OCH_3$ |
| J-8 | Q-1 ($R_7$=H, $R_8$=H) | H | 5-$NHCH_3$ | $OCH_3$ | $OCH_3$ |
| J-8 | Q-1 ($R_7$=H, $R_8$=H) | H | 5-Cl | $OCH_3$ | $OCH_3$ |
| J-8 | Q-1 ($R_7$=H, $R_8$=H) | H | 4-$NO_2$ | $OCH_3$ | $OCH_3$ |
| J-8 | Q-1 ($R_7$=H, $R_8$=H) | H | 5-$CF_3$ | $OCH_3$ | $OCH_3$ |
| J-8 | Q-1 ($R_7$=H, $R_8$=H) | H | H | $OCH_3$ | $CH_2CH_3$ |
| J-8 | Q-1 ($R_7$=H, $R_8$=H) | H | H | $OCH_3$ | $OCH_2CH_3$ |
| J-8 | Q-1 ($R_7$=H, $R_8$=H) | H | H | $OCF_2H$ | $OCH_3$ |
| J-8 | Q-1 ($R_7$=H, $R_8$=H) | H | H | $OCH_2CF_3$ | $OCH_3$ |
| J-8 | Q-1 ($R_7$=H, $R_8$=H) | H | H | $CF_3$ | $OCH_3$ |
| J-8 | Q-1 ($R_7$=H, $R_8$=H) | H | H | $SCH_3$ | $OCH_3$ |
| J-8 | Q-1 ($R_7$=H, $R_8$=H) | H | H | $SCHF_2$ | $OCH_3$ |
| J-8 | Q-1 ($R_7$=H, $R_8$=H) | H | H | Cl | $OCH_3$ |
| J-8 | Q-1 ($R_7$=H, $R_8$=H) | H | H | $OCH_3$ | $CH_2OCH_3$ |
| J-8 | Q-1 ($R_7$=H, $R_8$=H) | H | H | $OCH_3$ | $NH_2$ |
| J-8 | Q-1 ($R_7$=H, $R_8$=H) | H | H | $OCH_3$ | $NHCH_3$ |
| J-8 | Q-1 ($R_7$=H, $R_8$=H) | H | H | H | $OCH_3$ |
| J-8 | Q-1 ($R_7$=H, $R_8$=H) | H | H | $OCH_3$ | $OCH_2C{\equiv}CH$ |
| J-8 | Q-1 ($R_7$=H, $R_8$=H) | H | H | $OCH_3$ | $CH_2S(CH_2)_3CH_3$ |
| J-8 | Q-1 ($R_7$=H, $R_8$=H) | H | H | $OCH_3$ | cyclopropyl |
| J-8 | Q-1 ($R_7$=H, $R_8$=H) | H | H | $OCH_3$ | $C{\equiv}CH$ |
| J-8 | Q-1 ($R_7$=H, $R_8$=H) | H | H | $OCH_3$ | $-CH(OCH_3)_2$ |
| J-8 | Q-2 ($R_7$=H, $R_8$=H) | H | H | $OCH_3$ | $OCH_3$ |

Table 1 (continued)

| J | Q | R | $R_{1c}$ | X | Y |
|---|---|---|---|---|---|
| J-8 | Q-3 ($R_7$=H, $R_8$=H) | H | H | $OCH_3$ | $OCH_3$ |
| J-8 | Q-4 ($R_7$=H, $R_8$=H, $R_3$=$CH_3$) | H | H | $CH_3$ | $OCH_3$ |
| J-8 | Q-5 ($R_7$-H, $R_8$=H, $R_3$=H) | H | H | $OCH_3$ | $OCH_3$ |
| J-8 | Q-6 ($R_7$=$CH_3$, $R_8$=H, $R_3$=$CH_3$) | H | H | $CH_3$ | $OCH_3$ |
| J-8 | Q-8 ($R_7$=H, $R_8$=H, $R_2$=H) | H | H | $OCH_3$ | $OCH_3$ |
| J-8 | Q-9 ($R_7$=H, $R_8$=H, $R_2$=H) | H | H | $OCH_3$ | $OCH_3$ |
| J-8 | Q-10 ($R_7$=H, $R_8$=H) | H | H | $OCH_3$ | $OCH_3$ |
| J-8 | Q-11 ($R_7$=H, $R_8$=H) | H | H | $CH_3$ | $OCH_3$ |
| J-8 | Q-12 ($R_7$=H, $R_8$=H) | H | H | $OCH_3$ | $OCH_3$ |
| J-8 | Q-13 ($R_7$=H, $R_8$=H, $R_3$=H) | H | H | $CH_3$ | $OCH_3$ |
| J-8 | Q-14 ($R_7$=H, $R_8$=H, $R_3$=$CH_3$) | H | H | $OCH_3$ | $OCH_3$ |
| J-8 | Q-15 ($R_7$=CH , $R_8$=H, $R_3$=H) | H | H | $OCH_3$ | $OCH_3$ |
| J-8 | Q-17 ($R_7$=H, $R_8$=H, $R_2$=H) | H | H | $OCH_3$ | $OCH_3$ |
| J-8 | Q-18 ($R_7$=H, $R_8$=H, $R_2$=H) | H | H | $CH_3$ | $OCH_3$ |
| J-8 | Q-19 ($R_7$=H, $R_8$=H) | H | H | $OCH_3$ | $OCH_3$ |
| J-8 | Q-20 ($R_7$=H, $R_8$=H) | H | H | $CH_3$ | $OCH_3$ |
| J-8 | Q-21 ($R_7$=H, $R_8$=H) | H | H | $OCH_3$ | $OCH_3$ |
| J-8 | Q-22 ($R_7$=H, $R_8$=H, $R_3$=H) | H | H | $OCH_3$ | $OCH_3$ |
| J-8 | Q-23 ($R_7$=H, $R_8$=H, $R_3$=H) | H | H | $OCH_3$ | $OCH_3$ |
| J-8 | Q-24 ($R_7$=H, $R_8$=H, $R_3$=$CH_3$) | H | H | $OCH_3$ | $OCH_3$ |
| J-8 | Q-25 ($R_7$=H, $R_8$=H, $R_9$=H) | H | H | $CH_3$ | $OCH_3$ |
| J-8 | Q-26 ($R_7$=H, $R_8$=H) | H | H | $OCH_3$ | $OCH_3$ |
| J-8 | Q-27 ($R_7$=H, $R_8$=H) | H | H | $CH_3$ | $OCH_3$ |
| J-8 | Q-28 ($R_7$=H, $R_8$=H) | H | H | $OCH_3$ | $OCH_3$ |
| J-8 | Q-29 ($R_7$=H, $R_8$=H) | H | H | $OCH_3$ | $OCH_3$ |
| J-8 | Q-30 ($R_7$=H, $R_8$=H) | H | H | $OCH_3$ | $OCH_3$ |
| J-8 | Q-31 ($R_7$=H, $R_8$=H) | H | H | $OCH_3$ | $OCH_3$ |
| J-8 | Q-32 ($R_7$=H, $R_8$=H, $R_3$=H) | H | H | $CH_3$ | $OCH_3$ |
| J-8 | Q-33 ($R_7$=H, $R_8$=H, $R_8$=$CH_3$) | H | H | $OCH_3$ | $OCH_3$ |
| J-8 | Q-34 ($R_7$=H, $R_8$=H, $R_3$=H) | H | H | $CH_3$ | $OCH_3$ |

Table 1 (continued)

| J | Q | R | $R_{1c}$ | X | Y |
|---|---|---|---|---|---|
| J-8 | Q-36 ($R_7$=H, $R_8$=H, $R_2$=H) | H | H | $OCH_3$ | $OCH_3$ |
| J-8 | Q-37 ($R_7$=H, $R_8$=H, $R_2$=H) | H | H | $OCH_3$ | $OCH_3$ |
| J-8 | Q-38 ($R_7$=H, $R_8$=H, $R_2$=H) | H | H | $OCH_3$ | $OCH_3$ |
| J-8 | Q-39 ($R_7$=H, $R_8$=H, $R_9$=H) | H | H | $CH_3$ | $OCH_3$ |
| J-8 | Q-40 ($R_7$=H, $R_8$=H) | H | H | $OCH_3$ | $OCH_3$ |
| J-8 | Q-41 ($R_7$=H, $R_8$=H) | H | H | $CH_3$ | $OCH_3$ |
| J-8 | Q-42 ($R_7$=H, $R_8$=H) | H | H | $OCH_3$ | $OCH_3$ |
| J-8 | Q-43 ($R_7$=H, $R_8$=H, $R_3$=H) | H | H | $OCH_3$ | $OCH_3$ |
| J-8 | Q-44 ($R_7$=H, $R_8$=H, $R_3$=$CH_3$) | H | H | $OCH_3$ | $OCH_3$ |
| J-8 | Q-45 ($R_7$=H, $R_8$=H, $R_3$=H) | H | H | $OCH_3$ | $OCH_3$ |
| J-8 | Q-47 ($R_7$=H, $R_8$=H, $R_2$=H) | H | H | $OCH_3$ | $OCH_3$ |
| J-8 | Q-48 ($R_7$=H, $R_8$=H, $R_2$=H) | H | H | $CH_3$ | $OCH_3$ |
| J-8 | Q-49 ($R_7$=H, $R_8$=H, $R_2$=H) | H | H | $OCH_3$ | $OCH_3$ |
| J-8 | Q-50 ($R_7$=H, $R_8$=H, $R_2$=H) | H | H | $OCH_3$ | $OCH_3$ |
| J-8 | Q-51 ($R_7$=H, $R_8$=H, $R_9$=H) | H | H | $OCH_3$ | $OCH_3$ |
| J-8 | Q-52 ($R_7$=H, $R_8$=H, $R_9$=H) | H | H | $OCH_3$ | $OCH_3$ |
| J-8 | Q-53 ($R_7$=H, $R_8$=H, $R_9$=H) | H | H | $CH_3$ | $OCH_3$ |
| J-8 | Q-54 ($R_7$=H, $R_8$=H, $R_9$=H) | H | H | $OCH_3$ | $OCH_3$ |
| J-8 | Q-55 ($R_7$=H, $R_8$=H, $R_9$=H, $R_3$=H) | H | H | $CH_3$ | $OCH_3$ |
| J-8 | Q-56 ($R_7$=H, $R_8$=H, $R_9$=H, $R_3$=$C_2H_5$) | H | H | $CH_3$ | $OCH_3$ |
| J-8 | Q-57 ($R_7$=H, $R_8$=H, $R_9$=H, $R_3$=i-$C_3H_7$) | H | H | $OCH_3$ | $OCH_3$ |
| J-8 | Q-58 ($R_7$=H, $R_8$=H, $R_9$=H, $R_3$=H) | H | H | $OCH_3$ | $OCH_3$ |
| J-8 | Q-60 ($R_7$=H, $R_8$=H, $R_9$=H, $R_2$=H) | H | H | $CH_3$ | $OCH_3$ |
| J-8 | Q-61 ($R_7$=H, $R_8$=H, $R_9$=H, $R_2$=H) | H | H | $OCH_3$ | $OCH_3$ |
| J-8 | Q-62 ($R_7$=H, $R_8$=H, $R_9$=H, $R_2$=H) | H | H | $CH_3$ | $OCH_3$ |
| J-8 | Q-63 ($R_7$=H, $R_8$=H, $R_9$=H) | H | H | $OCH_3$ | $OCH_3$ |
| J-8 | Q-64 ($R_7$=H, $R_8$=H, $R_9$=H) | H | H | $OCH_3$ | $OCH_3$ |
| J-8 | Q-65 ($R_7$=H, $R_8$=H, $R_9$=H) | H | H | $OCH_3$ | $OCH_3$ |
| J-8 | Q-66 ($R_7$=H, $R_8$=H, $R_9$=H) | H | H | $OCH_3$ | $OCH_3$ |

Table 1 (continued)

| J | Q | R | $R_{1c}$ | X | Y |
|---|---|---|---|---|---|
| J-8 | Q-67 ($R_7$=H, $R_8$=H, $R_9$=H, $R_3$=H) | H | H | $CH_3$ | $OCH_3$ |
| J-8 | Q-68 ($R_7$=H, $R_8$=H, $R_9$=H, $R_3$=$CH_3$) | H | H | $OCH_3$ | $OCH_3$ |
| J-8 | Q-69 ($R_7$=H, $R_8$=H, $R_9$=H, $R_3$=$CH_3$) | H | H | $CH_3$ | $OCH_3$ |
| J-8 | Q-70 ($R_7$=H, $R_8$=H, $R_9$=H, $R_3$=$CH_3$) | H | H | $OCH_3$ | $OCH_3$ |
| J-8 | Q-72 ($R_7$=H, $R_8$=H, $R_9$=H, $R_2$=H) | H | H | $OCH_3$ | $OCH_3$ |
| J-8 | Q-73 ($R_7$=H, $R_8$=H, $R_9$=H, $R_2$=H) | H | H | $OCH_3$ | $OCH_3$ |
| J-8 | Q-74 ($R_7$=H, $R_8$=H, $R_9$=H, $R_2$=H) | H | H | $CH_3$ | $OCH_3$ |
| J-8 | Q-75 ($R_7$=H, $R_8$=H, $R_9$=H) | H | H | $OCH_3$ | $OCH_3$ |
| J-8 | Q-76 ($R_7$=H, $R_8$=H, $R_9$=H) | H | H | $CH_3$ | $OCH_3$ |
| J-8 | Q-77 ($R_7$=H, $R_8$=H, $R_9$=H) | H | H | $OCH_3$ | $OCH_3$ |
| J-8 | Q-78 ($R_7$=H, $R_8$=H, $R_9$=H) | H | H | $OCH_3$ | $OCH_3$ |
| J-8 | Q-79 ($R_7$=H, $R_8$=H, $R_9$=H, $R_3$=H) | H | H | $OCH_3$ | $OCH_3$ |
| J-8 | Q-80 ($R_7$=H, $R_8$=H, $R_9$=H, $R_3$=$CH_3$) | H | H | $OCH_3$ | $OCH_3$ |
| J-8 | Q-81 ($R_7$=H, $R_8$=H, $R_9$=H, $R_3$=$CH_3$) | H | H | $CH_3$ | $OCH_3$ |
| J-8 | Q-82 ($R_7$=H, $R_8$=H, $R_9$=H, $R_3$=$CH_3$) | H | H | $OCH_3$ | $OCH_3$ |
| J-8 | Q-83 ($R_7$=H, $R_8$=H, $R_9$=H, $R_{10}$=H) | H | H | $CH_3$ | $OCH_3$ |
| J-8 | Q-84 ($R_7$=H, $R_8$=H, $R_9$=H) | H | H | $OCH_3$ | $OCH_3$ |
| J-8 | Q-85 ($R_7$=H, $R_8$=H, $R_9$=H) | H | H | $OCH_3$ | $OCH_3$ |
| J-8 | Q-86 ($R_7$=H, $R_8$=H, $R_9$=H) | H | H | $OCH_3$ | $OCH_3$ |
| J-8 | Q-87 ($R_7$=H, $R_8$=H, $R_9$=H) | H | H | $OCH_3$ | $OCH_3$ |
| J-8 | Q-88 ($R_7$=H, $R_8$=H, $R_9$=H) | H | H | $CH_3$ | $OCH_3$ |
| J-8 | Q-89 ($R_7$=H, $R_8$=H, $R_9$=H) | H | H | $OCH_3$ | $OCH_3$ |
| J-8 | Q-90 ($R_7$=H, $R_8$=H, $R_9$=H, $R_3$=$CH_3$) | H | H | $CH_3$ | $OCH_3$ |
| J-8 | Q-91 ($R_7$=H, $R_8$=H, $R_9$=H, $R_3$=$CH_3$) | H | H | $OCH_3$ | $OCH_3$ |
| J-8 | Q-92 ($R_7$=H, $R_8$=H, $R_9$=H, $R_{10}$=H) | H | H | $OCH_3$ | $OCH_3$ |
| J-8 | Q-93 ($R_7$=H, $R_8$=H, $R_9$=H) | H | H | $OCH_3$ | $OCH_3$ |
| J-8 | Q-94 ($R_7$=H, $R_8$=H, $R_9$=H) | H | H | $OCH_3$ | $OCH_3$ |
| J-8 | Q-95 ($R_7$=H, $R_8$=H, $R_9$=H) | H | H | $CH_3$ | $OCH_3$ |
| J-8 | Q-96 ($R_7$=H, $R_8$=H, $R_9$=H) | H | H | $OCH_3$ | $OCH_3$ |
| J-8 | Q-97 ($R_7$=H, $R_8$=H, $R_9$=H) | H | H | $CH_3$ | $OCH_3$ |
| J-8 | Q-98 ($R_7$=H, $R_8$=H, $R_9$=H) | H | H | $OCH_3$ | $OCH_3$ |

Table 1 (continued)

| J | Q | R | $R_{1c}$ | X | Y |
|---|---|---|---|---|---|
| J-8 | Q-99 ($R_7$=H, $R_8$=H, $R_9$=H, $R_3$=H) | H | H | $OCH_3$ | $OCH_3$ |
| J-8 | Q-100 ($R_7$=H, $R_8$=H, $R_9$=H, $R_3$=$C_2H_5$) | H | H | $OCH_3$ | $OCH_3$ |
| J-8 | Q-101 ($R_7$=H, $R_8$=H, $R_9$=H, $R_3$=i-$C_2H_5$) | H | H | $OCH_3$ | $OCH_3$ |
| J-8 | Q-102 ($R_7$=H, $R_8$=H, $R_9$=H, $R_3$=H) | H | H | $CH_3$ | $OCH_3$ |
| J-8 | Q-104 ($R_7$=H, $R_8$=H, $R_9$=H, $R_2$=H) | H | H | $CH_3$ | $OCH_3$ |
| J-8 | Q-105 ($R_7$=H, $R_8$=H, $R_9$=H, $R_2$=H) | H | H | $OCH_3$ | $OCH_3$ |
| J-8 | Q-106 ($R_7$=H, $R_8$=H, $R_9$=H, $R_2$=H) | H | H | $OCH_3$ | $OCH_3$ |
| J-8 | Q-107 ($R_7$=H, $R_8$=H, $R_9$=H, $R_2$=H) | H | H | $OCH_3$ | $OCH_3$ |
| J-8 | Q-108 ($R_7$=H, $R_8$=H, $R_9$=H, $R_2$=H) | H | H | $OCH_3$ | $OCH_3$ |
| J-8 | Q-109 ($R_7$=H, $R_8$=H, $R_9$=H) | H | H | $CH_3$ | $OCH_3$ |
| J-8 | Q-110 ($R_7$=H, $R_8$=H, $R_9$=H) | H | H | $OCH_3$ | $OCH_3$ |
| J-8 | Q-111 ($R_7$=H, $R_8$=H, $R_9$=H) | H | H | $CH_3$ | $OCH_3$ |
| J-8 | Q-112 ($R_7$=H, $R_8$=H, $R_9$=H) | H | H | $OCH_3$ | $OCH_3$ |
| J-8 | Q-113 ($R_7$=H, $R_8$=H, $R_9$=H, $R_3$=$CH_3$) | H | H | $OCH_3$ | $OCH_3$ |
| J-8 | Q-114 ($R_7$=H, $R_8$=H, $R_9$=H, $R_3$=$CH_3$) | H | H | $OCH_3$ | $OCH_3$ |
| J-8 | Q-115 ($R_7$=H, $R_8$=H, $R_9$=H, $R_3$=$CH_3$) | H | H | $OCH_3$ | $OCH_3$ |
| J-8 | Q-116 ($R_7$=H, $R_8$=H, $R_9$=H, $R_{10}$=H) | H | H | $CH_3$ | $OCH_3$ |
| J-8 | Q-118 ($R_7$=H, $R_8$=H, $R_9$=H, $R_{10}$=H) | H | H | $CH_3$ | $OCH_3$ |
| J-8 | Q-119 ($R_7$=H, $R_8$=H, $R_9$=H, $R_2$=H) | H | H | $OCH_3$ | $OCH_3$ |
| J-8 | Q-120 ($R_7$=H, $R_8$=H, $R_9$=H, $R_2$=H) | H | H | $OCH_3$ | $OCH_3$ |
| J-8 | Q-121 ($R_7$=H, $R_8$=H, $R_9$=H, $R_2$=H) | H | H | $OCH_3$ | $OCH_3$ |
| J-8 | Q-122 ($R_7$=H, $R_8$=H, $R_9$=H, $R_2$=H) | H | H | $OCH_3$ | $OCH_3$ |
| J-8 | Q-123 ($R_7$=H, $R_8$=H, $R_9$=H) | H | H | $CH_3$ | $OCH_3$ |
| J-8 | Q-124 ($R_7$=H, $R_8$=H, $R_9$=H) | H | H | $OCH_3$ | $OCH_3$ |
| J-8 | Q-125 ($R_7$=H, $R_8$=H, $R_9$=H) | H | H | $CH_3$ | $OCH_3$ |
| J-8 | Q-126 ($R_7$=H, $R_8$=H, $R_9$=H) | H | H | $OCH_3$ | $OCH_3$ |
| J-8 | Q-127 ($R_7$=H, $R_8$=H, $R_9$=H, $R_3$=H) | H | H | $OCH_3$ | $OCH_3$ |
| J-8 | Q-128 ($R_7$=H, $R_8$=H, $R_9$=H, $R_3$=$CH_3$) | H | H | $OCH_3$ | $OCH_3$ |
| J-8 | Q-129 ($R_7$=H, $R_8$=H, $R_9$=H, $R_3$=$CH_3$) | H | H | $OCH_3$ | $OCH_3$ |
| J-8 | Q-130 ($R_7$=H, $R_8$=H, $R_9$=H, $R_3$=$CH_3$) | H | H | $CH_3$ | $OCH_3$ |

Table 1 (continued)

| J | Q | R | $R_{1c}$ | X | Y |
|---|---|---|---|---|---|
| J-8 | Q-131 ($R_7$=H, $R_8$=H, $R_9$=H, $R_{10}$=H) | H | H | $OCH_3$ | $OCH_3$ |
| J-8 | Q-132 ($R_7$=H, $R_8$=H, $R_9$=H) | H | H | $CH_3$ | $OCH_3$ |
| J-8 | Q-133 ($R_7$=H, $R_8$=H, $R_9$=H) | H | H | $OCH_3$ | $OCH_3$ |
| J-8 | Q-134 ($R_7$=H, $R_8$=H, $R_9$=H) | H | H | $OCH_3$ | $OCH_3$ |
| J-8 | Q-135 ($R_7$=H, $R_8$=H, $R_9$=H) | H | H | $OCH_3$ | $OCH_3$ |
| J-8 | Q-136 ($R_7$=H, $R_8$=H, $R_9$=H) | H | H | $OCH_3$ | $OCH_3$ |
| J-8 | Q-137 ($R_7$=H, $R_8$=H, $R_9$=H) | H | H | $CH_3$ | $OCH_3$ |
| J-8 | Q-138 ($R_7$=H, $R_8$=H, $R_9$=H) | H | H | $OCH_3$ | $OCH_3$ |
| J-8 | Q-139 ($R_7$=H, $R_8$=H, $R_9$=H) | H | H | $CH_3$ | $OCH_3$ |
| J-8 | Q-140 ($R_7$=H, $R_8$=H, $R_9$=H, $R_3$=$CH_3$) | H | H | $OCH_3$ | $OCH_3$ |
| J-8 | Q-141 ($R_7$=H, $R_8$=H, $R_9$=H, $R_3$=$CH_3$) | H | H | $OCH_3$ | $OCH_3$ |
| J-8 | Q-142 ($R_7$=H, $R_8$=H, $R_9$=H, $R_{10}$=H) | H | H | $OCH_3$ | $OCH_3$ |
| J-8 | Q-143 ($R_7$=H, $R_8$=H, $R_9$=H, $R_3$=$CH_3$) | H | H | $OCH_3$ | $OCH_3$ |
| J-8 | Q-144 ($R_7$=H, $R_8$=H, $R_9$=H, $R_3$=$CH_3$) | H | H | $CH_3$ | $OCH_3$ |
| J-8 | Q-145 ($R_7$=H, $R_8$=H, $R_9$=H) | H | H | $OCH_3$ | $OCH_3$ |
| J-8 | Q-146 ($R_7$=H, $R_8$=H, $R_9$=H) | H | H | $CH_3$ | $OCH_3$ |
| J-8 | Q-147 ($R_7$=H, $R_8$=H, $R_9$=H) | H | H | $OCH_3$ | $OCH_3$ |
| J-8 | Q-148 ($R_7$=H, $R_8$=H, $R_9$=H) | H | H | $OCH_3$ | $OCH_3$ |
| J-8 | Q-149 ($R_7$=H, $R_8$=H, $R_9$=H) | H | H | $OCH_3$ | $OCH_3$ |
| J-8 | Q-150 ($R_7$=H, $R_8$=H, $R_9$=H) | H | H | $OCH_3$ | $OCH_3$ |
| J-8 | Q-151 ($R_7$=H, $R_8$=H, $R_9$=H) | H | H | $CH_3$ | $OCH_3$ |
| J-8 | Q-152 ($R_7$=H, $R_8$=H, $R_9$=H) | H | H | $OCH_3$ | $OCH_3$ |
| J-8 | Q-153 ($R_7$=H, $R_8$=H, $R_9$=H, $R_{11}$=$CH_3$) | H | H | $CH_3$ | $OCH_3$ |
| J-8 | Q-154 ($R_7$=H, $R_8$=H, $R_9$=H, $R_{11}$=$CH_3$) | H | H | $OCH_3$ | $OCH_3$ |
| J-8 | Q-155 ($R_7$=H, $R_8$=H, $R_9$=H) | H | H | $OCH_3$ | $OCH_3$ |
| J-8 | Q-156 ($R_7$=H, $R_8$=H, $R_9$=H,) | H | H | $OCH_3$ | $OCH_3$ |
| J-8 | Q-158 ($R_7$=H, $R_8$=H, $R_9$=H) | H | H | $CH_3$ | $OCH_3$ |
| J-8 | Q-159 ($R_7$=H, $R_8$=H, $R_9$=H) | H | H | $OCH_3$ | $OCH_3$ |
| J-8 | Q-160 ($R_7$=H, $R_8$=H, $R_9$=H, $R_{11}$=$CH_3$) | H | H | $CH_3$ | $OCH_3$ |
| J-8 | Q-161 ($R_7$=H, $R_8$=H, $R_9$=H, $R_{11}$=$CH_3$) | H | H | $OCH_3$ | $OCH_3$ |
| J-8 | Q-162 ($R_7$=H, $R_8$=H, $R_9$=H, $R_{10}$=H) | H | H | $OCH_3$ | $OCH_3$ |
| J-8 | Q-163 ($R_2$=H, $R_7$=H, $R_8$=H, $R_9$=H) | H | H | $OCH_3$ | $OCH_3$ |
| J-8 | Q-164 ($R_7$=H) | H | H | $OCH_3$ | $OCH_3$ |

Table 1 (continued)

| J | Q | R | $R_{1c}$ | X | Y |
|---|---|---|---|---|---|
| J-8 | Q-165 ($R_3$=H, $R_7$=H) | H | H | $OCH_3$ | $OCH_3$ |
| J-8 | Q-166 ($R_3$=H, $R_7$=H) | H | H | $OCH_3$ | $OCH_3$ |
| J-9 | Q-1 ($R_7$=H, $R_8$=H) | H | 5-$SOCH_3$ | $OCH_3$ | $OCH_3$ |
| J-9 | Q-1 ($R_7$=H, $R_8$=H) | H | 5-$SO_2CH_3$ | $OCH_3$ | $OCH_3$ |
| J-9 | Q-1 ($R_7$=H, $R_8$=H) | H | 5-$CO_2CH_3$ | $OCH_3$ | $OCH_3$ |
| J-9 | Q-1 ($R_7$=H, $R_8$=H) | H | 4-$SO_2N(CH_3)_2$ | $OCH_3$ | $OCH_3$ |
| J-9 | Q-1 ($R_7$=H, $R_8$=H) | H | 6-Cl | $OCH_3$ | $OCH_3$ |
| J-9 | Q-1 ($R_7$=H, $R_8$=H) | H | 6-$COOCH_3$ | $OCH_3$ | $OCH_3$ |
| J-9 | Q-1 ($R_7$=H, $R_8$=H) | H | 4-$SO_2CH_3$ | $OCH_3$ | $OCH_3$ |
| J-9 | Q-1 ($R_7$=H, $R_8$=H) | H | H | $CH_3$ | $CH_3$ |
| J-9 | Q-1 ($R_7$=H, $R_8$=H) | H | H | $OCH_3$ | $CH_3$ |
| J-9 | Q-1 ($R_7$=H, $R_8$=H) | H | H | $OCH_3$ | $OCH_3$ |
| J-9 | Q-1 ($R_7$=H, $R_8$=H) | $CH_3$ | H | $OCH_3$ | $OCH_3$ |
| J-9 | Q-1 ($R_7$=H, $R_8$=H) | $CH_3$ | H | $OCH_3$ | $CH_3$ |
| J-9 | Q-1 ($R_7$=H, $R_8$=H) | H | 5-$CH_3$ | $OCH_3$ | $OCH_3$ |
| J-9 | Q-1 ($R_7$=H, $R_8$=H) | H | 6-$CH_2CH_3$ | $OCH_3$ | $OCH_3$ |
| J-9 | Q-1 ($R_7$=H, $R_8$=H) | H | 5-$CH(CH_3)_2$ | $OCH_3$ | $OCH_3$ |
| J-9 | Q-1 ($R_7$=H, $R_8$=H) | H | 5-$OCH_3$ | $OCH_3$ | $OCH_3$ |
| J-9 | Q-1 ($R_7$=H, $R_8$=H) | H | 5-$OCH_2CH_3$ | $OCH_3$ | $OCH_3$ |
| J-9 | Q-1 ($R_7$=H, $R_8$=H) | H | 5-$OCH(CH_3)_2$ | $OCH_3$ | $OCH_3$ |
| J-9 | Q-1 ($R_7$=H, $R_8$=H) | H | 5-$OCF_2H$ | $OCH_3$ | $OCH_3$ |
| J-9 | Q-1 ($R_7$=H, $R_8$=H) | H | 6-$OCH_2CH_2Br$ | $OCH_3$ | $OCH_3$ |
| J-9 | Q-1 ($R_7$=H, $R_8$=H) | H | 5-$OCH(CH_3)(CH_2Cl)$ | $OCH_3$ | $OCH_3$ |
| J-9 | Q-1 ($R_7$=H, $R_8$=H) | H | 5-$CH_2F$ | $OCH_3$ | $OCH_3$ |
| J-9 | Q-1 ($R_7$=H, $R_8$=H) | H | 5-$CH_2CH_2Br$ | $OCH_3$ | $OCH_3$ |
| J-9 | Q-1 ($R_7$=H, $R_8$=H) | H | 5-$CH(CH_3)(CH_2Cl)$ | $OCH_3$ | $OCH_3$ |
| J-9 | Q-1 ($R_7$=H, $R_8$=H) | H | 5-$SCH_3$ | $OCH_3$ | $OCH_3$ |
| J-9 | Q-1 ($R_7$=H, $R_8$=H) | H | 6-$SCH_2CH_3$ | $OCH_3$ | $OCH_3$ |
| J-9 | Q-1 ($R_7$=H, $R_8$=H) | H | 5-$SCH(CH_3)_2$ | $OCH_3$ | $OCH_3$ |
| J-9 | Q-1 ($R_7$=H, $R_8$=H) | H | 5-$SCH_2F$ | $OCH_3$ | $OCH_3$ |
| J-9 | Q-1 ($R_7$=H, $R_8$=H) | H | 5-$SCH_2CH_2Br$ | $OCH_3$ | $OCH_3$ |
| J-9 | Q-1 ($R_7$=H, $R_8$=H) | H | 5-$SCH(CH_3)(CH_2Cl)$ | $OCH_3$ | $OCH_3$ |
| J-9 | Q-1 ($R_7$=H, $R_8$=H) | H | 4-$NH_2$ | $OCH_3$ | $OCH_3$ |
| J-9 | Q-1 ($R_7$=H, $R_8$=H) | H | 5-$NHCH_3$ | $OCH_3$ | $OCH_3$ |

Table 1 (continued)

| J | Q | R | $R_{1c}$ | X | Y |
|---|---|---|---|---|---|
| J-9 | Q-1 ($R_7$=H, $R_8$=H) | H | 6-$NHCH_2CH_3$ | $OCH_3$ | $OCH_3$ |
| J-9 | Q-1 ($R_7$=H, $R_8$=H) | H | 5-$NHCH(CH_3)_2$ | $OCH_3$ | $OCH_3$ |
| J-9 | Q-1 ($R_7$=H, $R_8$=H) | H | 4-$N(CH_3)_2$ | $OCH_3$ | $OCH_3$ |
| J-9 | Q-1 ($R_7$=H, $R_8$=H) | H | 6-$N(CH_3)(CH_2CH_3)$ | $OCH_3$ | $OCH_3$ |
| J-9 | Q-1 ($R_7$=H, $R_8$=H) | H | 5-$N(CH_3)(CH(CH_3)_2)$ | $OCH_3$ | $OCH_3$ |
| J-9 | Q-1 ($R_7$=H, $R_8$=H) | H | 5-Cl | $OCH_3$ | $OCH_3$ |
| J-9 | Q-1 ($R_7$=H, $R_8$=H) | H | 5-Br | $OCH_3$ | $OCH_3$ |
| J-9 | Q-1 ($R_7$=H, $R_8$=H) | H | 4-F | $OCH_3$ | $OCH_3$ |
| J-9 | Q-1 ($R_7$=H, $R_8$=H) | H | 5-I | $OCH_3$ | $OCH_3$ |
| J-9 | Q-1 ($R_7$=H, $R_8$=H) | H | 4-$NO_2$ | $OCH_3$ | $OCH_3$ |
| J-9 | Q-1 ($R_7$=H, $R_8$=H) | H | 5-$CF_3$ | $OCH_3$ | $OCH_3$ |
| J-9 | Q-1 ($R_7$=H, $R_8$=H) | H | 5-$OCF_2H$ | $OCH_3$ | $OCH_3$ |
| J-9 | Q-1 ($R_7$=H, $R_8$=H) | H | H | $OCH_3$ | $CH_2CH_3$ |
| J-9 | Q-1 ($R_7$=H, $R_8$=H) | H | H | $OCH_3$ | $CH(CH_3)_2$ |
| J-9 | Q-1 ($R_7$=H, $R_8$=H) | H | H | $OCH_3$ | $(CH_2)_3CH_3$ |
| J-9 | Q-1 ($R_7$=H, $R_8$=H) | H | H | $OCH_3$ | $OCH_2CH_3$ |
| J-9 | Q-1 ($R_7$=H, $R_8$=H) | H | H | $OCH_3$ | $OCH(CH_3)_2$ |
| J-9 | Q-1 ($R_7$=H, $R_8$=H) | H | H | $OCH_3$ | $O(CH_2)_3CH_3$ |
| J-9 | Q-1 ($R_7$=H, $R_8$=H) | H | H | $OCF_2H$ | $OCH_3$ |
| J-9 | Q-1 ($R_7$=H, $R_8$=H) | H | H | $OCH_2CH_2F$ | $OCH_3$ |
| J-9 | Q-1 ($R_7$=H, $R_8$=H) | H | H | $OCH_2CHF_2$ | $OCH_3$ |
| J-9 | Q-1 ($R_7$=H, $R_8$=H) | H | H | $OCH_2CF_3$ | $OCH_3$ |
| J-9 | Q-1 ($R_7$=H, $R_8$=H) | H | H | $OCH(CH_3)(CH_2Cl)$ | $OCH_3$ |
| J-9 | Q-1 ($R_7$=H, $R_8$=H) | H | H | $O(CH_2)_3CH_2Br$ | $OCH_3$ |
| J-9 | Q-1 ($R_7$=H, $R_8$=H) | H | H | $CH_2F$ | $OCH_3$ |
| J-9 | Q-1 ($R_7$=H, $R_8$=H) | H | H | $CH_2Cl$ | $OCH_3$ |
| J-9 | Q-1 ($R_7$=H, $R_8$=H) | H | H | $CH_2Br$ | $OCH_3$ |
| J-9 | Q-1 ($R_7$=H, $R_8$=H) | H | H | $CF_3$ | $OCH_3$ |
| J-9 | Q-1 ($R_7$=H, $R_8$=H) | H | H | $CH_2CH_2Br$ | $OCH_3$ |
| J-9 | Q-1 ($R_7$=H, $R_8$=H) | H | H | $CH(CH_3)(CH_2Cl)$ | $OCH_3$ |
| J-9 | Q-1 ($R_7$=H, $R_8$=H) | H | H | $(CH_2)_3CH_2I$ | $OCH_3$ |
| J-9 | Q-1 ($R_7$=H, $R_8$=H) | H | H | $SCH_3$ | $OCH_3$ |
| J-9 | Q-1 ($R_7$=H, $R_8$=H) | H | H | $SCH_2CH_3$ | $OCH_3$ |
| J-9 | Q-1 ($R_7$=H, $R_8$=H) | H | H | $SCH(CH_3)_2$ | $OCH_3$ |
| J-9 | Q-1 ($R_7$=H, $R_8$=H) | H | H | $S(CH_2)_3CH_3$ | $OCH_3$ |

Table 1 (continued)

| J | Q | R | $R_{1c}$ | X | Y |
|---|---|---|---|---|---|
| J-9 | Q-1 ($R_7$=H, $R_8$=H) | H | H | $SCHF_2$ | $OCH_3$ |
| J-9 | Q-1 ($R_7$=H, $R_8$=H) | H | H | $SCH_2CH_2Br$ | $OCH_3$ |
| J-9 | Q-1 ($R_7$=H, $R_8$=H) | H | H | $SCH(CH_3)(CH_2Cl)$ | $OCH_3$ |
| J-9 | Q-1 ($R_7$=H, $R_8$=H) | H | H | $S(CH_2)_3CH_2F$ | $OCH_3$ |
| J-9 | Q-1 ($R_7$=H, $R_8$=H) | H | H | Cl | $OCH_3$ |
| J-9 | Q-1 ($R_7$=H, $R_8$=H) | H | H | Br | $OCH_3$ |
| J-9 | Q-1 ($R_7$=H, $R_8$=H) | H | H | F | $OCH_3$ |
| J-9 | Q-1 ($R_7$=H, $R_8$=H) | H | H | I | $OCH_3$ |
| J-9 | Q-1 ($R_7$=H, $R_8$=H) | H | H | $OCH_3$ | $CH_2OCH_3$ |
| J-9 | Q-1 ($R_7$=H, $R_8$=H) | H | H | $OCH_3$ | $CH_2CH_2OCH(CH_3)_2$ |
| J-9 | Q-1 ($R_7$=H, $R_8$=H) | H | H | $OCH_3$ | $CH(CH_3)(CH_2OCH_3)$ |
| J-9 | Q-1 ($R_7$=H, $R_8$=H) | H | H | $OCH_3$ | $(CH_2)_4CH_2OCH_2CH_3$ |
| J-9 | Q-1 ($R_7$=H, $R_8$=H) | H | H | $OCH_2O(CH_2)_3CH_3$ | $OCH_3$ |
| J-9 | Q-1 ($R_7$=H, $R_8$=H) | H | H | $OCH_2CH_2OCH(CH_3)_2$ | $OCH_3$ |
| J-9 | Q-1 ($R_7$=H, $R_8$=H) | H | H | $OCH(CH_3)(CH_2OCH_3)$ | $OCH_3$ |
| J-9 | Q-1 ($R_7$=H, $R_8$=H) | H | H | $O(CH_2)_4CH_2OCH_2CH_3$ | $OCH_3$ |
| J-9 | Q-1 ($R_7$=H, $R_8$=H) | H | H | $OCH_3$ | $NH_2$ |
| J-9 | Q-1 ($R_7$=H, $R_8$=H) | H | H | $OCH_3$ | $NHCH_3$ |
| J-9 | Q-1 ($R_7$=H, $R_8$=H) | H | H | $OCH_3$ | $NHCH_2CH_3$ |
| J-9 | Q-1 ($R_7$=H, $R_8$=H) | H | H | $OCH_3$ | $NHCH(CH_3)_2$ |
| J-9 | Q-1 ($R_7$=H, $R_8$=H) | H | H | $OCH_3$ | $N(CH_3)_2$ |
| J-9 | Q-1 ($R_7$=H, $R_8$=H) | H | H | $OCH_3$ | $N(CH_3)(CH_2CH_3)$ |
| J-9 | Q-1 ($R_7$=H, $R_8$=H) | H | H | $OCH_3$ | $N(CH_3)(CH(CH_3)_2)$ |
| J-9 | Q-1 ($R_7$=H, $R_8$=H) | H | H | $OCH_3$ | $(CH_2)_4CH_2OCH_2CH_3$ |
| J-9 | Q-1 ($R_7$=H, $R_8$=H) | H | H | H | $OCH_3$ |
| J-9 | Q-1 ($R_7$=H, $R_8$=H) | H | H | $OCH_3$ | $OCH_2CH=CH_2$ |
| J-9 | Q-1 ($R_7$=H, $R_8$=H) | H | H | $OCH_3$ | $OCH_2C(CH_3)=CH_2$ |
| J-9 | Q-1 ($R_7$=H, $R_8$=H) | H | H | $OCH_3$ | $OCH_2C\equiv CH$ |
| J-9 | Q-1 ($R_7$=H, $R_8$=H) | H | H | $OCH_3$ | $OCH_2C\equiv CCH_3$ |
| J-9 | Q-1 ($R_7$=H, $R_8$=H) | H | H | $OCH_3$ | $CH_2S(CH_2)_3CH_3$ |
| J-9 | Q-1 ($R_7$=H, $R_8$=H) | H | H | $OCH_3$ | $CH_2CH_2SCH(CH_3)_2$ |
| J-9 | Q-1 ($R_7$=H, $R_8$=H) | H | H | $OCH_3$ | $CH(CH_3)(CH_2SCH_3)$ |
| J-9 | Q-1 ($R_7$=H, $R_8$=H) | H | H | $OCH_3$ | $(CH_2)_4CH_2SCH_2CH_3$ |
| J-9 | Q-1 ($R_7$=H, $R_8$=H) | H | H | $OCH_3$ | cyclopropyl |
| J-9 | Q-1 ($R_7$=H, $R_8$=H) | H | H | $OCH_3$ | 2-methylcyclopropyl |

Table 1 (continued)

| J | Q | R | $R_{1c}$ | X | Y |
|---|---|---|---|---|---|
| J-9 | Q-1 ($R_7$=H, $R_8$=H) | H | H | $OCH_3$ | cyclopentyl |
| J-9 | Q-1 ($R_7$=H, $R_8$=H) | H | H | $OCH_3$ | C≡CH |
| J-9 | Q-1 ($R_7$=H, $R_8$=H) | H | H | $OCH_3$ | $C{\equiv}CCH_3$ |
| J-9 | Q-1 ($R_7$=H, $R_8$=H) | H | H | $OCH_3$ | -CHO |
| J-9 | Q-1 ($R_7$=H, $R_8$=H) | H | H | $OCH_3$ | $-COCH_3$ |
| J-9 | Q-1 ($R_7$=H, $R_8$=H) | H | H | $OCH_3$ | $-CH(OCH_3)_2$ |
| J-9 | Q-1 ($R_7$=H, $R_8$=H) | H | H | $OCH_3$ | $-CH(SCH_3)(OCH_2CH_3)$ |
| J-9 | Q-1 ($R_7$=H, $R_8$=H) | H | H | $OCH_3$ | $-C(CH_3)(SCH_3)_2$ |
| J-9 | Q-1 ($R_7$=H, $R_8$=H) | H | H | $OCH_3$ | $-CH(SCH_2CH_3)_2$ |
| J-9 | Q-1 ($R_7$=H, $R_8$=H) | H | H | $OCH_3$ | 1,3-dioxolan-2-yl |
| J-9 | Q-1 ($R_7$=H, $R_8$=H) | H | H | $OCH_3$ | 2-methyl-1,3-oxathiolan-2-yl |
| J-9 | Q-1 ($R_7$=H, $R_8$=H) | H | H | $OCH_3$ | 1,3-oxathian-2-yl |
| J-9 | Q-1 ($R_7$=H, $R_8$=H) | H | H | $OCH_3$ | 2-methyl-1,3-dithian-2-yl |
| J-9 | Q-1 ($R_7$=H, $R_8$=H) | H | H | $OCH_3$ | 4-methyl-1,3-dioxolan-2-yl |
| J-9 | Q-1 ($R_7$=H, $R_8$=H) | H | H | $OCH_3$ | 4-methyl-1,3-oxathiolan-2-yl |
| J-9 | Q-1 ($R_7$=H, $R_8$=H) | H | H | $OCH_3$ | 2,4-dimethyl-1,3-dithiolan-2-yl |
| J-9 | Q-1 ($R_7$=H, $R_8$=H) | H | H | $OCH_3$ | $N(OCH_3)(CH_3)_2$ |
| J-9 | Q-1 ($R_7$=H, $R_8$=$CH_3$) | H | H | $OCH_3$ | $OCH_3$ |
| J-9 | Q-1 ($R_7$=H, $R_8$=$CH_3$) | H | H | $CH_3$ | $CH_3$ |
| J-9 | Q-1 ($R_7$=H, $R_8$=$CH_3$) | H | H | $CH_3$ | $OCH_3$ |
| J-9 | Q-1 ($R_7$=H, $R_8$=$CH_2CH_3$) | H | H | $OCH_3$ | $OCH_3$ |
| J-9 | Q-1 ($R_7$=H, $R_8$=$CH(CH_3)_2$) | H | H | $OCH_3$ | $OCH_3$ |
| J-9 | Q-1 ($R_7$=H, $R_8$=$(CH_2)_3CH_3$) | H | H | $OCH_3$ | $OCH_3$ |
| J-9 | Q-1 ($R_7$=$CH_3$, $R_8$=$CH_3$) | H | H | $OCH_3$ | $OCH_3$ |
| J-9 | Q-2 ($R_7$=H, $R_8$=H) | H | H | $OCH_3$ | $OCH_3$ |
| J-9 | Q-2 ($R_7$=H, $R_8$=H) | $CH_3$ | H | $OCH_3$ | $OCH_3$ |
| J-9 | Q-2 ($R_7$=H, $R_8$=$CH_3$) | H | 5-$OCH_3$ | $OCH_3$ | $OCH_3$ |
| J-9 | Q-3 ($R_7$=H, $R_8$=H) | H | H | $OCH_3$ | $OCH_3$ |
| J-9 | Q-3 ($R_7$=$C_2H_5$, $R_8$=H) | H | H | $OCF_2H$ | $CH_3$ |
| J-9 | Q-3 ($R_7$=$CH_3$, $R_8$=H) | H | 5-Cl | $OCH_3$ | $CH_3$ |
| J-9 | Q-4 ($R_7$=H, $R_8$=H, $R_3$=$CH_3$) | H | H | $CH_3$ | $OCH_3$ |
| J-9 | Q-4 ($R_7$=H, $R_8$=H, $R_3$=$C_2H_5$) | H | H | $CH_3$ | $OCH_3$ |
| J-9 | Q-4 ($R_7$=H, $R_8$=H, $R_3$=<u>i</u>-$C_3H_7$) | H | 5-$CH_3$ | $OCH_3$ | $OCH_3$ |

Table 1 (continued)

| J | Q | R | $R_{1c}$ | X | Y |
|---|---|---|---|---|---|
| J-9 | Q-5 ($R_7$=H, $R_8$=H, $R_3$=H) | H | H | $OCH_3$ | $OCH_3$ |
| J-9 | Q-5 ($R_7$=H, $R_8$=H, $R_3$=$CH_3$) | H | H | Cl | $OCH_3$ |
| J-9 | Q-5 ($R_7$=H, $R_8$=$CH_3$, $R_3$=$CH_3$) | H | H | $CH_3$ | $OCH_3$ |
| J-9 | Q-6 ($R_7$=$CH_3$, $R_8$=H, $R_3$=$CH_3$) | H | H | $CH_3$ | $OCH_3$ |
| J-9 | Q-6 ($R_7$=H, $R_8$=H, $R_3$=$C_2H_5$) | $CH_3$ | H | $CH_3$ | $OCH_3$ |
| J-9 | Q-6 ($R_7$=$C_2H_5$, $R_8$=H, $R_3$=H) | H | H | $OCH_3$ | $OCH_3$ |
| J-9 | Q-8 ($R_7$=H, $R_8$=H, $R_2$=H) | H | H | $OCH_3$ | $OCH_3$ |
| J-9 | Q-8 ($R_7$=$CH_3$, $R_8$=H, $R_2$=$OCH_3$) | H | H | $OCH_3$ | $OCF_2H$ |
| J-9 | Q-8 ($R_7$=H, $R_8$=H, $R_2$=$OCH(CH_3)CH_2CH_3$) | H | 5-$Cl_3$ | $OCH_3$ | OCH |
| J-9 | Q-9 ($R_7$=H, $R_8$=H, $R_2$=H) | H | H | $OCH_3$ | $OCH_3$ |
| J-9 | Q-9 ($R_7$=$CH_3$, $R_8$=H, $R_2$=$CH_3$) | $CH_3$ | H | $OCH_3$ | $OCH_3$ |
| J-9 | Q-9 ($R_7$=H, $R_8$=H, $R_2$=Cl) | H | 5-$OCH_3$ | $OCH_3$ | $OCH_3$ |
| J-9 | Q-10 ($R_7$=H, $R_8$=H) | H | H | $OCH_3$ | $OCH_3$ |
| J-9 | Q-10 ($R_7$=$CH_3$, $R_8$=H) | H | H | $OCF_2H$ | $CH_3$ |
| J-9 | Q-10 ($R_7$=H, $R_8$=$CH_3$) | H | 5-Cl | $OCH_3$ | $CH_3$ |
| J-9 | Q-11 ($R_7$=H, $R_8$=H) | H | H | $CH_3$ | $OCH_3$ |
| J-9 | Q-11 ($R_7$=<u>n</u>-$C_4H_9$, $R_8$=H) | H | H | $CH_3$ | $OCH_3$ |
| J-9 | Q-11 ($R_7$=$CH_3$, $R_8$=$CH_3$) | H | 5-$CH_3$ | $OCH_3$ | $OCH_3$ |
| J-9 | Q-12 ($R_7$=H, $R_8$=H) | H | H | $OCH_3$ | $OCH_3$ |
| J-9 | Q-12 ($R_7$=$CH_3$, $R_8$=H) | H | H | Cl | $OCH_3$ |
| J-9 | Q-12 ($R_7$=H, $R_8$=$C_2H_5$) | H | H | $CH_3$ | $OCH_3$ |
| J-9 | Q-13 ($R_7$=H, $R_8$=H, $R_3$=H) | H | H | $CH_3$ | $OCH_3$ |
| J-9 | Q-13 ($R_7$=H, $R_8$=H, $R_3$=$CH_3$) | $CH_3$ | H | $CH_3$ | $OCH_3$ |
| J-9 | Q-13 ($R_7$=$CH_3$, $R_8$=H, $R_3$=$CH_3$) | H | H | $OCH_3$ | $OCH_3$ |
| J-9 | Q-14 ($R_7$=H, $R_8$=H, $R_3$=$CH_3$) | H | H | $OCH_3$ | $OCH_3$ |
| J-9 | Q-14 ($R_7$=$CH_3$, $R_8$=H, $R_3$=$C_2H_5$) | H | H | $OCH_3$ | $OCH_3$ |
| J-9 | Q-14 ($R_7$=H, $R_8$=$CH_3$, $R_3$=$CH_3$) | H | 5-$OCH_3$ | $OCH_3$ | $OCH_3$ |
| J-9 | Q-15 ($R_7$=H, $R_8$=H, $R_3$=H) | H | H | $OCH_3$ | $OCH_3$ |
| J-9 | Q-15 ($R_7$=$C_2H_5$, $R_8$=H, $R_3$=$CH_3$) | H | H | $OCH_3$ | $OCF_2H$ |
| J-9 | Q-15 ($R_7$=$CH_3$, $R_8$=$CH_3$, $R_3$=$CH_3$) | H | 5-Cl | $OCH_3$ | $OCH_3$ |
| J-9 | Q-17 ($R_7$=H, $R_8$=H, $R_2$=H) | H | H | $OCH_3$ | $OCH_3$ |
| J-9 | Q-17 ($R_7$=$CH_3$, $R_8$=H, $R_2$=$CH_3$) | H | H | $OCF_2H$ | $CH_3$ |
| J-9 | Q-17 ($R_7$=H, $R_8$=H, $R_2$=$C_2H_5$) | H | 5-Cl | $OCH_3$ | $CH_3$ |
| J-9 | Q-18 ($R_7$=H, $R_8$=H, $R_2$=H) | H | H | $CH_3$ | $OCH_3$ |
| J-9 | Q-18 ($R_7$=H, $R_8$=$OC_2H_5$, $R_2$=H) | H | H | $CH_3$ | $OCH_3$ |
| J-9 | Q-18 ($R_7$=$CH_3$, $R_8$=$OCH(CH_3)_2$, $R_2$=$CH_3$) | H | 5-$CH_3$ | $OCH_3$ | $OCH_3$ |

Table 1 (continued)

| J | Q | R | $R_{1c}$ | X | Y |
|---|---|---|---|---|---|
| J-9 | Q-19 ($R_7$=H, $R_8$=H) | H | H | $OCH_3$ | $OCH_3$ |
| J-9 | Q-19 ($R_7$=$CH_3$, $R_8$=H) | H | H | Cl | $OCH_3$ |
| J-9 | Q-19 ($R_7$=$CH_3$, $R_8$=$CH_3$) | H | H | $CH_3$ | $OCH_3$ |
| J-9 | Q-20 ($R_7$=H, $R_8$=H) | H | H | $CH_3$ | $OCH_3$ |
| J-9 | Q-20 ($R_7$=$CH_3$, $R_8$=H) | $CH_3$ | H | $CH_3$ | $OCH_3$ |
| J-9 | Q-20 ($R_7$=H, $R_8$=$CH_3$) | H | H | $OCH_3$ | $OCH_3$ |
| J-9 | Q-21 ($R_7$=H, $R_8$=H) | H | H | $OCH_3$ | $OCH_3$ |
| J-9 | Q-21 ($R_7$=$CH_3$, $R_8$=H) | H | H | $OCH_3$ | $OCH_3$ |
| J-9 | Q-21 ($R_7$=$CH_3$, $R_8$=$CH_3$) | H | 5-$OCH_3$ | $OCH_3$ | $OCH_3$ |
| J-9 | Q-22 ($R_7$=H, $R_8$=H, $R_3$=H) | H | H | $OCH_3$ | $OCH_3$ |
| J-9 | Q-22 ($R_7$=H, $R_8$=H, $R_3$=$CH_3$) | H | H | $OCH_3$ | $OCF_2H$ |
| J-9 | Q-22 ($R_7$=$CH_3$, $R_8$=$CH_3$, $R_3$=$C_2H_5$) | H | 5-Cl | $OCH_3$ | $OCH_3$ |
| J-9 | Q-23 ($R_7$=H, $R_8$=H, $R_3$=H) | H | H | $OCH_3$ | $OCH_3$ |
| J-9 | Q-23 ($R_7$=H, $R_8$=H, $R_3$=$CH_3$) | $CH_3$ | H | $OCH_3$ | $OCH_3$ |
| J-9 | Q-23 ($R_7$=$CH_3$, $R_8$=H, $R_3$=n-$C_4H_9$) | H | 5-$OCH_3$ | $OCH_3$ | $OCH_3$ |
| J-9 | Q-24 ($R_7$=H, $R_8$=H, $R_3$=$CH_3$) | H | H | $OCH_3$ | $OCH_3$ |
| J-9 | Q-24 ($R_7$=$CH_3$, $R_8$=$CH_3$, $R_3$=$C_2H_5$) | H | H | $OCF_2H$ | $CH_3$ |
| J-9 | Q-24 ($R_7$=$CH_3$, $R_8$=$CH_3$, $R_3$=i-$C_3H_7$) | H | 5-Cl | $OCH_3$ | $CH_3$ |
| J-9 | Q-25 ($R_7$=H, $R_8$=H, $R_9$=H) | H | H | $CH_3$ | $OCH_3$ |
| J-9 | Q-25 ($R_7$=$CH_3$, $R_8$=H, $R_9$=H) | H | H | $CH_3$ | $OCH_3$ |
| J-9 | Q-25 ($R_7$=H, $R_8$=H, $R_9$=$C_2H_5$) | H | 5-$CH_3$ | $OCH_3$ | $OCH_3$ |
| J-9 | Q-26 ($R_7$=H, $R_8$=H) | H | H | $OCH_3$ | $OCH_3$ |
| J-9 | Q-26 ($R_7$=$CH_3$, $R_8$=H) | H | H | Cl | $OCH_3$ |
| J-9 | Q-26 ($R_7$=$CH_3$, $R_8$=$CH_3$) | H | H | $CH_3$ | $OCH_3$ |
| J-9 | Q-27 ($R_7$=H, $R_8$=H) | H | H | $CH_3$ | $OCH_3$ |
| J-9 | Q-27 ($R_7$=$CH_3$, $R_8$=H) | $CH_3$ | H | $CH_3$ | $OCH_3$ |
| J-9 | Q-27 ($R_7$=H, $R_8$=$CH_3$) | H | H | $OCH_3$ | $OCH_3$ |
| J-9 | Q-28 ($R_7$=H, $R_8$=H) | H | H | $OCH_3$ | $OCH_3$ |
| J-9 | Q-28 ($R_7$=$CH_3$, $R_8$=H) | H | H | $OCH_3$ | $OCH_3$ |
| J-9 | Q-28 ($R_7$=$CH_3$, $R_8$=$CH_3$) | H | 5-$OCH_3$ | $OCH_3$ | $OCH_3$ |
| J-9 | Q-29 ($R_7$=H, $R_8$=H) | H | H | $OCH_3$ | $OCH_3$ |
| J-9 | Q-29 ($R_7$=$CH_3$, $R_8$=H) | H | H | $OCH_3$ | $OCF_2H$ |
| J-9 | Q-29 ($R_7$=H, $R_8$=$CH_3$) | H | 5-Cl | $OCH_3$ | $OCH_3$ |
| J-9 | Q-30 ($R_7$=H, $R_8$=H) | H | H | $OCH_3$ | $OCH_3$ |
| J-9 | Q-30 ($R_7$=$CH_3$, $R_8$=$CH_3$) | $CH_3$ | H | $OCH_3$ | $CH_3$ |
| J-9 | Q-30 ($R_7$=$C_2H_5$, $R_8$=H) | H | 5-$OCH_3$ | $OCH_3$ | $OCH_3$ |

Table 1 (continued)

| J | Q | R | $R_{1c}$ | X | Y |
|---|---|---|---|---|---|
| J-9 | Q-31 ($R_7$=H, $R_8$=H) | H | H | $OCH_3$ | $OCH_3$ |
| J-9 | Q-31 ($R_7$=i-$C_3H_7$, $R_8$=H) | H | H | $OCF_2H$ | $CH_3$ |
| J-9 | Q-31 ($R_7$=H, $R_8$=n-$C_4H_9$) | H | 5-Cl | $OCH_3$ | $CH_3$ |
| J-9 | Q-32 ($R_7$=H, $R_8$=H, $R_3$=H) | H | H | $CH_3$ | $OCH_3$ |
| J-9 | Q-32 ($R_7$=$CH_3$, $R_8$=H, $R_3$=$CH_3$) | H | H | $CH_3$ | $OCH_3$ |
| J-9 | Q-32 ($R_7$=H, $R_8$=$CH_3$, $R_3$=$C_2H_5$) | H | 5-$CH_3$ | $OCH_3$ | $OCH_3$ |
| J-9 | Q-33 ($R_7$=H, $R_8$=H, $R_8$=$CH_3$) | H | H | $OCH_3$ | $OCH_3$ |
| J-9 | Q-33 ($R_7$=$CH_3$, $R_8$=$CH_3$, $R_3$=i-$C_3H_7$) | H | H | Cl | $OCH_3$ |
| J-9 | Q-33 ($R_7$=$C_2H_5$, $R_8$=H, $R_3$=n-$C_4H_9$) | H | H | $CH_3$ | $OCH_3$ |
| J-9 | Q-34 ($R_7$=H, $R_8$=H, $R_3$=H) | H | H | $CH_3$ | $OCH_3$ |
| J-9 | Q-34 ($R_7$=i-$C_3H_7$, $R_8$=H, $R_3$=$CH_3$) | $CH_3$ | H | $CH_3$ | $OCH_3$ |
| J-9 | Q-34 ($R_7$=H, $R_8$=n-$C_4H_9$, $R_3$=$CH_3$) | H | H | $OCH_3$ | $OCH_3$ |
| J-9 | Q-36 ($R_7$=H, $R_8$=H, $R_2$=H) | H | H | $OCH_3$ | $OCH_3$ |
| J-9 | Q-36 ($R_7$=$CH_3$, $R_8$=H, $R_2$=$CH_3$) | H | H | $OCH_3$ | $OCF_2H$ |
| J-9 | Q-36 ($R_7$=H, $R_8$=H, $R_2$=$C_2H_5$) | H | 5-Cl | $OCH_3$ | $OCH_3$ |
| J-9 | Q-37 ($R_7$=H, $R_8$=H, $R_2$=H) | H | H | $OCH_3$ | $OCH_3$ |
| J-9 | Q-37 ($R_7$=H, $R_8$=H, $R_2$=i-$C_3H_7$) | $CH_3$ | H | $OCH_3$ | $OCH_3$ |
| J-9 | Q-37 ($R_7$=H, $R_8$=H, $R_2$=n-$C_4H_9$) | H | 5-$OCH_3$ | $OCH_3$ | $OCH_3$ |
| J-9 | Q-38 ($R_7$=H, $R_8$=H, $R_2$=$OCF_2H$) | H | H | $OCH_3$ | $OCH_3$ |
| J-9 | Q-38 ($R_7$=$CH_3$, $R_8$=H, $R_2$=$OCH_2CH_2F$) | H | H | $OCF_2H$ | $CH_3$ |
| J-9 | Q-38 ($R_7$=H, $R_8$=H, $R_2$=Cl) | H | 5-Cl | $OCH_3$ | $CH_3$ |
| J-9 | Q-39 ($R_7$=H, $R_8$=H, $R_9$=H) | H | H | $CH_3$ | $OCH_3$ |
| J-9 | Q-39 ($R_7$=$C_2H_5$, $R_8$=H, $R_9$=H) | H | H | $CH_3$ | $OCH_3$ |
| J-9 | Q-39 ($R_7$=H, $R_8$=H, $R_9$=$CH_3$) | H | 5-$CH_3$ | $OCH_3$ | $OCH_3$ |
| J-9 | Q-40 ($R_7$=H, $R_8$=H) | H | H | $OCH_3$ | $OCH_3$ |
| J-9 | Q-40 ($R_7$=$CH_3$, $R_8$=H) | H | H | Cl | $OCH_3$ |
| J-9 | Q-40 ($R_7$=H, $R_8$=$CH_3$) | H | H | $CH_3$ | $OCH_3$ |
| J-9 | Q-41 ($R_7$=H, $R_8$=H) | H | H | $CH_3$ | $OCH_3$ |
| J-9 | Q-41 ($R_7$=$CH_3$, $R_8$=$CH_3$) | $CH_3$ | H | $CH_3$ | $OCH_3$ |
| J-9 | Q-41 ($R_7$=H, $R_8$=$C_2H_5$) | H | H | $OCH_3$ | $OCH_3$ |
| J-9 | Q-42 ($R_7$=H, $R_8$=H) | H | H | $OCH_3$ | $OCH_3$ |
| J-9 | Q-42 ($R_7$=$CH_3$, $R_8$=$CH_3$) | H | H | $OCH_3$ | $OCH_3$ |
| J-9 | Q-42 ($R_7$=$C_2H_5$, $R_8$=$C_2H_5$) | H | 5-$OCH_3$ | $OCH_3$ | $OCH_3$ |
| J-9 | Q-43 ($R_7$=H, $R_8$=H, $R_3$=H) | H | H | $OCH_3$ | $OCH_3$ |
| J-9 | Q-43 ($R_7$=H, $R_8$=H, $R_3$=$CH_3$) | H | H | $OCH_3$ | $OCF_2H$ |
| J-9 | Q-43 ($R_7$=$CH_3$, $R_8$=H, $R_3$=$CH_3$) | H | 5-Cl | $OCH_3$ | $OCH_3$ |

Table 1 (continued)

| J | Q | R | $R_{1c}$ | X | Y |
|---|---|---|---|---|---|
| J-9 | Q-44 ($R_7$=H, $R_8$=H, $R_3$=$CH_3$) | H | H | $OCH_3$ | $OCH_3$ |
| J-9 | Q-44 ($R_7$=$CH_3$, $R_8$=H, $R_3$=$C_2H_5$) | $CH_3$ | H | $OCH_3$ | $CH_3$ |
| J-9 | Q-44 ($R_7$=H, $R_8$=$CH_3$, $R_3$=$CH_3$) | H | 5-$OCH_3$ | $OCH_3$ | $OCH_3$ |
| J-9 | Q-45 ($R_7$=H, $R_8$=H, $R_3$=H) | H | H | $OCH_3$ | $OCH_3$ |
| J-9 | Q-45 ($R_7$=$C_2H_5$, $R_8$=H, $R_3$=$CH_3$) | H | H | $OCF_2H$ | $CH_3$ |
| J-9 | Q-45 ($R_7$=$CH_3$, $R_8$=$CH_3$, $R_3$=$CH_3$) | H | 5-Cl | $OCH_3$ | $CH_3$ |
| J-9 | Q-47 ($R_7$=H, $R_8$=H, $R_2$=H) | H | H | $OCH_3$ | $OCH_3$ |
| J-9 | Q-47 ($R_7$=H, $R_8$=$CH_3$, $R_2$=$CH_3$) | H | H | Cl | $OCH_3$ |
| J-9 | Q-47 ($R_7$=H, $R_8$=H, $R_2$=$C_2H_5$) | H | H | $CH_3$ | $OCH_3$ |
| J-9 | Q-48 ($R_7$=H, $R_8$=H, $R_2$=H) | H | H | $CH_3$ | $OCH_3$ |
| J-9 | Q-48 ($R_7$=$CH_3$, $R_8$=$CH_3$, $R_2$=$CH_3$) | $CH_3$ | H | $CH_3$ | $OCH_3$ |
| J-9 | Q-48 ($R_7$=H, $R_8$=$C_2H_5$, $R_2$=$C_2H_5$) | H | H | $OCH_3$ | $OCH_3$ |
| J-9 | Q-49 ($R_7$=H, $R_8$=H, $R_2$=H) | H | H | $OCH_3$ | $OCH_3$ |
| J-9 | Q-49 ($R_7$=$CH_3$, $R_8$=$CH_3$, $R_2$=H) | H | H | $OCH_3$ | $OCH_3$ |
| J-9 | Q-49 ($R_7$=$CH_3$, $R_8$=$CH_3$, $R_2$=$CH_3$) | H | 5-$OCH_3$ | $OCH_3$ | $OCH_3$ |
| J-9 | Q-50 ($R_7$=H, $R_8$=H, $R_2$=H) | H | H | $OCH_3$ | $OCH_3$ |
| J-9 | Q-50 ($R_7$=$CH_3$, $R_8$=$CH_3$, $R_2$=H) | H | H | $OCH_3$ | $OCF_2H$ |
| J-9 | Q-50 ($R_7$=$CH_3$, $R_8$=$CH_3$, $R_2$=$CH_3$) | H | 5-Cl | $OCH_3$ | $OCH_3$ |
| J-9 | Q-51 ($R_7$=H, $R_8$=H, $R_9$=H) | H | H | $OCH_3$ | $OCH_3$ |
| J-9 | Q-51 ($R_7$=H, $R_8$=H, $R_9$=$CH_3$) | $CH_3$ | H | $OCH_3$ | $OCH_3$ |
| J-9 | Q-51 ($R_7$=H, $R_8$=H, $R_9$=<u>s</u>-$C_4H_9$) | H | 5-$OCH_3$ | $OCH_3$ | $OCH_3$ |
| J-9 | Q-52 ($R_7$=H, $R_8$=H, $R_9$=H) | H | H | $OCH_3$ | $OCH_3$ |
| J-9 | Q-52 ($R_7$=$CH_3$, $R_8$=H, $R_9$=H) | H | H | $OCF_2H$ | $CH_3$ |
| J-9 | Q-52 ($R_7$=H, $R_8$=H, $R_9$=$CH_3$) | H | 5-Cl | $OCH_3$ | $CH_3$ |
| J-9 | Q-53 ($R_7$=H, $R_8$=H, $R_9$=H) | H | H | $CH_3$ | $OCH_3$ |
| J-9 | Q-53 ($R_7$=$CH_3$, $R_8$=$CH_3$, $R_9$=$CH_3$) | H | H | $CH_3$ | $OCH_3$ |
| J-9 | Q-53 ($R_7$=H, $R_8$=H, $R_9$=$CH_3$) | H | 5-$CH_3$ | $OCH_3$ | $OCH_3$ |
| J-9 | Q-54 ($R_7$=H, $R_8$=H, $R_9$=H) | H | H | $OCH_3$ | $OCH_3$ |
| J-9 | Q-54 ($R_7$=$CH_3$, $R_8$=H, $R_9$=H) | H | H | Cl | $OCH_3$ |
| J-9 | Q-54 ($R_7$=H, $R_8$=H, $R_9$=<u>i</u>-$C_3H_7$) | H | H | $CH_3$ | $OCH_3$ |

Table 1 (continued)

| J | Q | R | $R_{1c}$ | X | Y |
|---|---|---|---|---|---|
| J-9 | Q-55 ($R_7$=H, $R_8$=H, $R_9$=H, $R_3$=H) | H | H | $CH_3$ | $OCH_3$ |
| J-9 | Q-55 ($R_7$=H, $R_8$=H, $R_9$=$CH_3$, $R_3$=$CH_3$) | $CH_3$ | H | $CH_3$ | $OCH_3$ |
| J-9 | Q-55 ($R_7$=H, $R_8$=H, $R_9$=s-$C_4H_9$, $R_3$=H) | H | H | $OCH_3$ | $OCH_3$ |
| J-9 | Q-56 ($R_7$=H, $R_8$=H, $R_9$=H, $R_3$=$C_2H_5$) | H | H | $OCH_3$ | $OCH_3$ |
| J-9 | Q-56 ($R_7$=$CH_3$, $R_8$=H, $R_9$=H, $R_3$=H) | H | H | $OCH_3$ | $OCH_3$ |
| J-9 | Q-56 ($R_7$=H, $R_8$=H, $R_9$=$CH_3$, $R_3$=$CH_3$) | H | 5-$OCH_3$ | $OCH_3$ | $OCH_3$ |
| J-9 | Q-57 ($R_7$=H, $R_8$=H, $R_9$=H, $R_3$=i-$C_3H_7$) | H | H | $OCH_3$ | $OCH_3$ |
| J-9 | Q-57 ($R_7$=$CH_3$, $R_8$=$CH_3$, $R_9$=$CH_3$, $R_3$=H) | H | H | $OCH_3$ | $OCF_2H$ |
| J-9 | Q-57 ($R_7$=H, $R_8$=H, $R_9$=$CH_3$, $R_3$=$CH_3$) | H | 5-Cl | $OCH_3$ | $OCH_3$ |
| J-9 | Q-58 ($R_7$=H, $R_8$=H, $R_9$=H, $R_3$=H) | H | H | $OCH_3$ | $OCH_3$ |
| J-9 | Q-58 ($R_7$=$CH_3$, $R_8$=H, $R_9$=H, $R_3$=$CH_3$) | $CH_3$ | H | $OCH_3$ | $CH_3$ |
| J-9 | Q-58 ($R_7$=H, $R_8$=H, $R_9$=i-$C_3H_7$, $R_3$=$CH_3$) | H | 5-$OCH_3$ | $OCH_3$ | $OCH_3$ |
| J-9 | Q-60 ($R_7$=H, $R_8$=H, $R_9$=H, $R_2$=H) | H | H | $CH_3$ | $OCH_3$ |
| J-9 | Q-60 ($R_7$=$CH_3$, $R_8$=H, $R_9$=H, $R_2$=$O(CH_2)_3CH_2Br$) | H | H | $CH_3$ | $OCH_3$ |
| J-9 | Q-60 ($R_7$=H, $R_8$=H, $R_9$=H, $R_2$=Br) | H | 5-$CH_3$ | $OCH_3$ | $OCH_3$ |
| J-9 | Q-61 ($R_7$=H, $R_8$=H, $R_9$=H, $R_2$=H) | H | H | $OCH_3$ | $OCH_3$ |
| J-9 | Q-61 ($R_7$=$CH_3$, $R_8$=H, $R_9$=H, $R_2$=$OCH_2F$) | H | H | Cl | $OCH_3$ |
| J-9 | Q-61 ($R_7$=H, $R_8$=$CH_3$, $R_9$=H, $R_2$=$O(CH_2)_3CH_3$) | H | H | $CH_3$ | $OCH_3$ |
| J-9 | Q-62 ($R_7$=H, $R_8$=H, $R_9$=H, $R_2$=H) | H | H | $CH_3$ | $OCH_3$ |
| J-9 | Q-62 ($R_7$=$CH_3$, $R_8$=H, $R_9$=H, $R_2$=$CH_3$) | $CH_3$ | H | $CH_3$ | $OCH_3$ |
| J-9 | Q-62 ($R_7$=Cl, $R_8$=H, $R_9$=H, $R_2$=H) | H | H | $OCH_3$ | $OCH_3$ |
| J-9 | Q-63 ($R_7$=H, $R_8$=H, $R_9$=H) | H | H | $OCH_3$ | $OCH_3$ |
| J-9 | Q-63 ($R_7$=$CH_3$, $R_8$=H, $R_9$=H) | H | H | $OCH_3$ | $OCH_3$ |
| J-9 | Q-63 ($R_7$=H, $R_8$=H, $R_9$=$CH_3$) | H | 5-$OCH_3$ | $OCH_3$ | $OCH_3$ |
| J-9 | Q-64 ($R_7$=H, $R_8$=H, $R_9$=H) | H | H | $OCH_3$ | $OCH_3$ |
| J-9 | Q-64 ($R_7$=$C_2H_5$, $R_8$=H, $R_9$=H) | H | H | $OCH_3$ | $OCF_2H$ |
| J-9 | Q-64 ($R_7$=H, $R_8$=H, $R_9$=$C_2H_5$) | H | 5-Cl | $OCH_3$ | $OCH_3$ |
| J-9 | Q-65 ($R_7$=H, $R_8$=H, $R_9$=H) | H | H | $OCH_3$ | $OCH_3$ |
| J-9 | Q-65 ($R_7$=n-$C_4H_9$, $R_8$=H, $R_9$=H) | $CH_3$ | H | $OCH_3$ | $CH_3$ |

Table 1 (continued)

| J | Q | R | $R_{1c}$ | X | Y |
|---|---|---|---|---|---|
| J-9 | Q-65 ($R_7$=H, $R_8$=$CH_3$, $R_9$=i-$C_3H_7$) | H | 5-$OCH_3$ | $OCH_3$ | $OCH_3$ |
| J-9 | Q-66 ($R_7$=H, $R_8$=H, $R_9$=H) | H | H | $OCH_3$ | $OCH_3$ |
| J-9 | Q-66 ($R_7$=i-$C_3H_7$, $R_8$=H, $R_9$=H) | H | H | $OCF_2H$ | $CH_3$ |
| J-9 | Q-66 ($R_7$=H, $R_8$=$C_2H_5$, $R_9$=$CH_3$) | H | 5-Cl | $OCH_3$ | $CH_3$ |
| J-9 | Q-67 ($R_7$=H, $R_8$=H, $R_9$=H, $R_3$=H) | H | H | $CH_3$ | $OCH_3$ |
| J-9 | Q-67 ($R_7$=$CH_3$, $R_8$=H, $R_9$=H, $R_3$=$CH_3$) | H | H | $CH_3$ | $OCH_3$ |
| J-9 | Q-67 ($R_7$=H, $R_8$=H, $R_9$=$CH_3$, $R_3$=$C_2H_5$) | H | 5-$CH_3$ | $OCH_3$ | $OCH_3$ |
| J-9 | Q-68 ($R_7$=H, $R_8$=H, $R_9$=H, $R_3$=$CH_3$) | H | H | $OCH_3$ | $OCH_3$ |
| J-9 | Q-68 ($R_7$=$C_2H_5$, $R_8$=H, $R_9$=H, $R_3$=i-$C_3H_7$) | H | H | Cl | $OCH_3$ |
| J-9 | Q-68 ($R_7$=H, $R_8$=H, $R_9$=$C_2H_5$, $R_3$=n-$C_4H_9$) | H | H | $CH_3$ | $OCH_3$ |
| J-9 | Q-69 ($R_7$=H, $R_8$=H, $R_9$=H, $R_3$=$CH_3$) | H | H | $CH_3$ | $OCH_3$ |
| J-9 | Q-69 ($R_7$=n-$C_4H_9$, $R_8$=H, $R_9$=H, $R_3$=$C_2H_5$) | $CH_3$ | H | $CH_3$ | $OCH_3$ |
| J-9 | Q-69 ($R_7$=H, $R_8$=$CH_3$, $R_9$=i-$C_3H_7$, $R_3$=H) | H | H | OCH | $OCH_3$ |
| J-9 | Q-70 ($R_7$=H, $R_8$=H, $R_9$=H, $R_3$=$CH_3$) | H | H | $OCH_3$ | $OCH_3$ |
| J-9 | Q-70 ($R_7$=i-$C_3H_7$, $R_8$=H, $R_9$=H, $R_3$=$CH_3$) | H | H | $OCH_3$ | $OCH_3$ |
| J-9 | Q-70 ($R_7$=H, $R_8$=$C_2H_5$, $R_9$=$CH_3$, $R_3$=H) | H | 5-$OCH_3$ | $OCH_3$ | $OCH_3$ |
| J-9 | Q-72 ($R_7$=H, $R_8$=H, $R_9$=H, $R_2$=H) | H | H | $OCH_3$ | $OCH_3$ |
| J-9 | Q-72 ($R_7$=H, $R_8$=H, $R_9$=H, $R_2$=$CH_3$) | $CH_3$ | H | $OCH_3$ | $CH_3$ |
| J-9 | Q-72 ($R_7$=$CH_3$, $R_8$=H, $R_9$=H, $R_2$=H) | H | 5-$OCH_3$ | $OCH_3$ | $OCH_3$ |
| J-9 | Q-73 ($R_7$=H, $R_8$=H, $R_9$=H, $R_2$=H) | H | H | $OCH_3$ | $OCH_3$ |
| J-9 | Q-73 ($R_7$=H, $R_8$=$CH_3$, $R_9$=H, $R_2$=$C_2H_5$) | H | H | $OCF_2H$ | $CH_3$ |
| J-9 | Q-73 ($R_7$=$CH_3$, $R_8$=H, $R_9$=$CH_3$, $R_2$=H) | H | 5-Cl | $OCH_3$ | $CH_3$ |
| J-9 | Q-74 ($R_7$=H, $R_8$=H, $R_9$=H, $R_2$=H) | H | H | $CH_3$ | $OCH_3$ |
| J-9 | Q-74 ($R_7$=$CH_3$, $R_8$=H, $R_9$=H, $R_2$=H) | H | H | $CH_3$ | $OCH_3$ |
| J-9 | Q-74 ($R_7$=H, $R_8$=H, $R_9$=H, $R_2$=$CH_3$) | H | 5-$CH_3$ | $OCH_3$ | $OCH_3$ |
| J-9 | Q-75 ($R_7$=H, $R_8$=H, $R_9$=H) | H | H | $OCH_3$ | $OCH_3$ |
| J-9 | Q-75 ($R_7$=H, $R_8$=H, $R_9$=$CH_3$) | H | H | Cl | $OCH_3$ |
| J-9 | Q-75 ($R_7$=$CH_3$, $R_8$=H, $R_9$=$CH_3$) | H | H | $CH_3$ | $OCH_3$ |
| J-9 | Q-76 ($R_7$=H, $R_8$=H, $R_9$=H) | H | H | $CH_3$ | $OCH_3$ |

Table 1 (continued)

| J | Q | R | $R_{1c}$ | X | Y |
|---|---|---|---|---|---|
| J-9 | Q-76 ($R_7$=H, $R_8$=H, $R_9$=$CH_3$) | $CH_3$ | H | $CH_3$ | $OCH_3$ |
| J-9 | Q-76 ($R_7$=$C_2H_5$, $R_8$=H, $R_9$=$C_2H_5$) | H | H | $OCH_3$ | $OCH_3$ |
| J-9 | Q-77 ($R_7$=H, $R_8$=H, $R_9$=H) | H | H | $OCH_3$ | $OCH_3$ |
| J-9 | Q-77 ($R_7$=H, $R_8$=H, $R_9$=n-$C_3H_7$) | H | H | $OCH_3$ | $OCH_3$ |
| J-9 | Q-77 ($R_7$=H, $R_8$=$CH_3$, $R_9$=H) | H | 5-$OCH_3$ | $OCH_3$ | OCH |
| J-9 | Q-78 ($R_7$=H, $R_8$=H, $R_9$=H) | H | H | $OCH_3$ | $OCH_3$ |
| J-9 | Q-78 ($R_7$=H, $R_8$=H, $R_9$=s-$C_4H_9$) | H | H | $OCH_3$ | $OCF_2H$ |
| J-9 | Q-78 ($R_7$=n-$C_3H_7$, $R_8$=H, $R_9$=H) | H | 5-Cl | $OCH_3$ | $OCH_3$ |
| J-9 | Q-79 ($R_7$=H, $R_8$=H, $R_9$=H, $R_3$=H) | H | H | $OCH_3$ | $OCH_3$ |
| J-9 | Q-79 ($R_7$=H, $R_8$=H, $R_9$=H, $R_3$=$CH_3$) | $CH_3$ | H | $OCH_3$ | $OCH_3$ |
| J-9 | Q-79 ($R_7$=$CH_3$, $R_8$=H, $R_9$=H, $R_3$=$C_2H_5$) | H | 5-$OCH_3$ | $OCH_3$ | $OCH_3$ |
| J-9 | Q-80 ($R_7$=H, $R_8$=H, $R_9$=H, $R_3$=$CH_3$) | H | H | $OCH_3$ | $OCH_3$ |
| J-9 | Q-80 ($R_7$=$CH_3$, $R_8$=H, $R_9$=H, $R_3$=$CH_3$) | H | H | $OCF_2H$ | $CH_3$ |
| J-9 | Q-80 ($R_7$=H, $R_8$=H, $R_9$=$CH_3$, $R_3$=$C_2H_5$) | H | 5-Cl | $OCH_3$ | $CH_3$ |
| J-9 | Q-81 ($R_7$=H, $R_8$=H, $R_9$=H, $R_3$=$CH_3$) | H | H | $CH_3$ | $OCH_3$ |
| J-9 | Q-81 ($R_7$=H, $R_8$=$CH_3$, $R_9$=H, $R_3$=i-$C_3H_7$) | H | H | $CH_3$ | $OCH_3$ |
| J-9 | Q-81 ($R_7$=$CH_3$, $R_8$=H, $R_9$=$CH_3$, $R_3$=$CH_3$) | H | 5-$CH_3$ | $OCH_3$ | OCH |
| J-9 | Q-82 ($R_7$=H, $R_8$=H, $R_9$=H, $R_3$=$CH_3$) | H | H | $OCH_3$ | $OCH_3$ |
| J-9 | Q-82 ($R_7$=$CH_3$, $R_8$=H, $R_9$=$CH_3$, $R_3$=H) | H | H | Cl | $OCH_3$ |
| J-9 | Q-82 ($R_7$=H, $R_8$=H, $R_9$=$C_2H_5$, $R_3$=$CH_3$) | H | H | $CH_3$ | $OCH_3$ |
| J-9 | Q-83 ($R_7$=H, $R_8$=H, $R_9$=H, $R_{10}$=H) | H | H | $CH_3$ | $OCH_3$ |
| J-9 | Q-83 ($R_7$=$CH_3$, $R_8$=H, $R_9$=H, $R_{10}$=$CH_3$) | $CH_3$ | H | $CH_3$ | $OCH_3$ |
| J-9 | Q-83 ($R_7$=$CH_3$, $R_8$=$CH_3$, $R_9$=H, $R_{10}$=H) | H | H | $OCH_3$ | $OCH_3$ |
| J-9 | Q-84 ($R_7$=H, $R_8$=H, $R_9$=H) | H | H | $OCH_3$ | $OCH_3$ |
| J-9 | Q-84 ($R_7$=H, $R_8$=H, $R_9$=$CH_3$) | H | H | $OCH_3$ | $OCH_3$ |
| J-9 | Q-84 ($R_7$=H, $R_8$=$C_2H_5$, $R_9$=H) | H | 5-$OCH_3$ | $OCH_3$ | $OCH_3$ |
| J-9 | Q-85 ($R_7$=H, $R_8$=H, $R_9$=H) | H | H | $OCH_3$ | $OCH_3$ |
| J-9 | Q-85 ($R_7$=$CH_3$, $R_8$=H, $R_9$=H) | H | H | $OCH_3$ | $OCF_2H$ |
| J-9 | Q-85 ($R_7$=H, $R_8$=H, $R_9$=$C_2H_5$) | H | 5-Cl | $OCH_3$ | $OCH_3$ |
| J-9 | Q-86 ($R_7$=H, $R_8$=H, $R_9$=H) | H | H | $OCH_3$ | $OCH_3$ |
| J-9 | Q-86 ($R_7$=$CH_3$, $R_8$=H, $R_9$=H) | $CH_3$ | H | $OCH_3$ | $OCH_3$ |
| J-9 | Q-86 ($R_7$=H, $R_8$=H, $R_9$=$C_2H_5$) | H | 5-$OCH_3$ | $OCH_3$ | $OCH_3$ |

Table 1 (continued)

| J | Q | R | $R_{1c}$ | X | Y |
|---|---|---|---|---|---|
| J-9 | Q-87 ($R_7$=H, $R_8$=H, $R_9$=H) | H | H | $OCH_3$ | $OCH_3$ |
| J-9 | Q-87 ($R_7$=H, $R_8$=H, $R_9$=$CH_3$) | H | H | $OCF_2H$ | $CH_3$ |
| J-9 | Q-87 ($R_7$=$CH_3$, $R_8$=H, $R_9$=H) | H | 5-Cl | $OCH_3$ | $CH_3$ |
| J-9 | Q-88 ($R_7$=H, $R_8$=H, $R_9$=H) | H | H | $CH_3$ | $OCH_3$ |
| J-9 | Q-88 ($R_7$=$CH_3$, $R_8$=H, $R_9$=H) | H | H | $CH_3$ | $OCH_3$ |
| J-9 | Q-88 ($R_7$=H, $R_8$=H, $R_9$=$CH_3$) | H | 5-$CH_3$ | $OCH_3$ | OCH |
| J-9 | Q-89 ($R_7$=H, $R_8$=H, $R_9$=H) | H | H | $OCH_3$ | $OCH_3$ |
| J-9 | Q-89 ($R_7$=$CH_3$, $R_8$=H, $R_9$=$C_2H_5$) | H | H | Cl | $OCH_3$ |
| J-9 | Q-89 ($R_7$=H, $R_8$=$CH_3$, $R_9$=H) | H | H | $CH_3$ | OCH |
| J-9 | Q-90 ($R_7$=H, $R_8$=H, $R_9$=H, $R_3$=$CH_3$) | H | H | $CH_3$ | $OCH_3$ |
| J-9 | Q-90 ($R_7$=$CH_3$, $R_8$=H, $R_9$=H, $R_3$=$C_2H_5$) | $CH_3$ | H | $CH_3$ | $OCH_3$ |
| J-9 | Q-90 ($R_7$=H, $R_8$=H, $R_9$=$CH_3$, $R_3$=$CH_3$) | H | H | $OCH_3$ | $OCH_3$ |
| J-9 | Q-91 ($R_7$=H, $R_8$=H, $R_9$=H, $R_3$=$CH_3$) | H | H | $OCH_3$ | $OCH_3$ |
| J-9 | Q-91 ($R_7$=$CH_3$, $R_8$=H, $R_9$=H, $R_3$=H) | H | H | $OCH_3$ | $OCH_3$ |
| J-9 | Q-91 ($R_7$=H, $R_8$=H, $R_9$=$CH_3$, $R_3$=$CH_3$) | H | 5-$OCH_3$ | $OCH_3$ | $OCH_3$ |
| J-9 | Q-92 ($R_7$=H, $R_8$=H, $R_9$=H, $R_{10}$=H) | H | H | $OCH_3$ | $OCH_3$ |
| J-9 | Q-92 ($R_7$=H, $R_8$=H, $R_9$=H, $R_{10}$=$CH_3$) | H | H | $OCH_3$ | $OCF_2H$ |
| J-9 | Q-92 ($R_7$=H, $R_8$=$CH_3$, $R_9$=H, $R_{10}$=H) | H | 5-Cl | $OCH_3$ | $OCH_3$ |
| J-9 | Q-93 ($R_7$=H, $R_8$=H, $R_9$=H) | H | H | $OCH_3$ | $OCH_3$ |
| J-9 | Q-93 ($R_7$=H, $R_8$=H, $R_9$=$CH_3$) | $CH_3$ | H | $OCH_3$ | $OCH_3$ |
| J-9 | Q-93 ($R_7$=$CH_3$, $R_8$=H, $R_9$=H) | H | 5-$OCH_3$ | $OCH_3$ | $OCH_3$ |
| J-9 | Q-94 ($R_7$=H, $R_8$=H, $R_9$=H) | H | H | $OCH_3$ | $OCH_3$ |
| J-9 | Q-94 ($R_7$=$CH_3$, $R_8$=H, $R_9$=H) | H | H | $OCF_2H$ | $CH_3$ |
| J-9 | Q-94 ($R_7$=H, $R_8$=H, $R_9$=$\underline{n}$-$C_4H_9$) | H | 5-Cl | $OCH_3$ | $CH_3$ |
| J-9 | Q-95 ($R_7$=H, $R_8$=H, $R_9$=H) | H | H | $CH_3$ | $OCH_3$ |
| J-9 | Q-95 ($R_7$=H, $R_8$=H, $R_9$=$CH_3$) | H | H | $CH_3$ | $OCH_3$ |
| J-9 | Q-95 ($R_7$=H, $R_8$=H, $R_9$=$\underline{s}$-$C_4H_9$) | H | 5-$CH_3$ | $OCH_3$ | $OCH_3$ |
| J-9 | Q-96 ($R_7$=H, $R_8$=H, $R_9$=H) | H | H | $OCH_3$ | $OCH_3$ |
| J-9 | Q-96 ($R_7$=$CH_3$, $R_8$=H, $R_9$=H) | H | H | Cl | $OCH_3$ |
| J-9 | Q-96 ($R_7$=H, $R_8$=H, $R_9$=$CH_3$) | H | H | $CH_3$ | $OCH_3$ |
| J-9 | Q-97 ($R_7$=H, $R_8$=H, $R_9$=H) | H | H | $CH_3$ | $OCH_3$ |
| J-9 | Q-97 ($R_7$=$CH_3$, $R_8$=$CH_3$, $R_9$=$CH_3$) | $CH_3$ | H | $CH_3$ | $OCH_3$ |

Table 1 (continued)

| J | Q | R | $R_{1c}$ | X | Y |
|---|---|---|---|---|---|
| J-9 | Q-97 ($R_7$=H, $R_8$=H, $R_9$=$CH_3$) | H | H | $OCH_3$ | $OCH_3$ |
| J-9 | Q-98 ($R_7$=H, $R_8$=H, $R_9$=H) | H | H | $OCH_3$ | $OCH_3$ |
| J-9 | Q-98 ($R_7$=$CH_3$, $R_8$=H, $R_9$=H) | H | H | $OCH_3$ | $OCH_3$ |
| J-9 | Q-98 ($R_7$=H, $R_8$=H, $R_9$=i-$C_3H_7$) | H | 5-$OCH_3$ | $OCH_3$ | $OCH_3$ |
| J-9 | Q-99 ($R_7$=H, $R_8$=H, $R_9$=H, $R_3$=H) | H | H | $OCH_3$ | $OCH_3$ |
| J-9 | Q-99 ($R_7$=H, $R_8$=H, $R_9$=$CH_3$, $R_3$=$CH_3$) | H | H | $OCH_3$ | $OCF_2H$ |
| J-9 | Q-100 ($R_7$=H, $R_8$=H, $R_9$=H, $R_3$=$C_2H_5$) | H | H | $OCH_3$ | $OCH_3$ |
| J-9 | Q-100 ($R_7$=$CH_3$, $R_8$=H, $R_9$=H, $R_3$=H) | $CH_3$ | H | $OCH_3$ | $CH_3$ |
| J-9 | Q-100 ($R_7$=H, $R_8$=H, $R_9$=$CH_3$, $R_3$=$CH_3$) | H | 5-$OCH_3$ | $OCH_3$ | $OCH_3$ |
| J-9 | Q-101 ($R_7$=H, $R_8$=H, $R_9$=H, $R_3$=i-$C_3H_7$) | H | H | $OCH_3$ | $OCH_3$ |
| J-9 | Q-101 ($R_7$=$CH_3$, $R_8$=$CH_3$, $R_9$=$CH_3$, $R_3$=H) | H | H | $OCF_2H$ | $CH_3$ |
| J-9 | Q-101 ($R_7$=H, $R_8$=H, $R_9$=$CH_3$, $R_3$=$CH_3$) | H | 5-Cl | $OCH_3$ | $CH_3$ |
| J-9 | Q-102 ($R_7$=H, $R_8$=H, $R_9$=H, $R_3$=H) | H | H | $CH_3$ | $OCH_3$ |
| J-9 | Q-102 ($R_7$=$CH_3$, $R_8$=H, $R_9$=H, $R_3$=$CH_3$) | H | H | $CH_3$ | $OCH_3$ |
| J-9 | Q-102 ($R_7$=H, $R_8$=H, $R_9$=i-$C_3H_7$, $R_3$=$CH_3$) | H | 5-$CH_3$ | $OCH_3$ | $OCH_3$ |
| J-9 | Q-104 ($R_7$=H, $R_8$=H, $R_9$=H, $R_2$=H) | H | H | $CH_3$ | $OCH_3$ |
| J-9 | Q-104 ($R_7$=$CH_3$, $R_8$=H, $R_9$=H, $R_2$=$OC_2H_5$) | $CH_3$ | H | $CH_3$ | $OCH_3$ |
| J-9 | Q-104 ($R_7$=H, $R_8$=H, $R_9$=H, $R_2$=Br) | H | H | $OCH_3$ | $OCH_3$ |
| J-9 | Q-105 ($R_7$=H, $R_8$=H, $R_9$=H, $R_2$=H) | H | H | $OCH_3$ | $OCH_3$ |
| J-9 | Q-105 ($R_7$=$CH_3$, $R_8$=H, $R_9$=H, $R_2$=$CH_3$) | H | H | $OCH_3$ | $OCH_3$ |
| J-9 | Q-105 ($R_7$=H, $R_8$=$CH_3$, $R_9$=H, $R_2$=$C_2H_5$) | H | 5-$OCH_3$ | $OCH_3$ | $OCH_3$ |
| J-9 | Q-106 ($R_7$=H, $R_8$=H, $R_9$=H, $R_2$=H) | H | H | $OCH_3$ | $OCH_3$ |
| J-9 | Q-106 ($R_7$=$CH_3$, $R_8$=H, $R_9$=H, $R_2$=$CH_3$) | H | H | $OCH_3$ | $OCF_2H$ |
| J-9 | Q-106 ($R_7$=Cl, $R_8$=H, $R_9$=H, $R_2$=H) | H | 5-Cl | $OCH_3$ | $OCH_3$ |
| J-9 | Q-107 ($R_7$=H, $R_8$=H, $R_9$=H, $R_2$=H) | H | H | $OCH_3$ | $OCH_3$ |
| J-9 | Q-107 ($R_7$=$CH_3$, $R_8$=H, $R_9$=H, $R_2$=H) | $CH_3$ | H | $OCH_3$ | $OCH_3$ |
| J-9 | Q-107 ($R_7$=H, $R_8$=H, $R_9$=H, $R_2$=$CH_3$) | H | 5-$OCH_3$ | $OCH_3$ | $OCH_3$ |
| J-9 | Q-108 ($R_7$=H, $R_8$=H, $R_9$=H, $R_2$=H) | H | H | $OCH_3$ | $OCH_3$ |
| J-9 | Q-108 ($R_7$=$CH_3$, $R_8$=H, $R_9$=H, $R_2$=Cl) | H | H | $OCF_2H$ | $CH_3$ |

Table 1 (continued)

| J | Q | R | $R_{1c}$ | X | Y |
|---|---|---|---|---|---|
| J-9 | Q-108 ($R_7$=H, $R_8$=H, $R_9$=H, $R_2$=$CH_3$) | H | 5-Cl | $OCH_3$ | $CH_3$ |
| J-9 | Q-109 ($R_7$=H, $R_8$=H, $R_9$=H) | H | H | $CH_3$ | $OCH_3$ |
| J-9 | Q-109 ($R_7$=$CH_3$, $R_8$=H, $R_9$=H) | H | H | $CH_3$ | $OCH_3$ |
| J-9 | Q-109 ($R_7$=H, $R_8$=H, $R_9$=$CH_3$) | H | 5-$CH_3$ | $OCH_3$ | $OCH_3$ |
| J-9 | Q-110 ($R_7$=H, $R_8$=H, $R_9$=H) | H | H | $OCH_3$ | $OCH_3$ |
| J-9 | Q-110 ($R_7$=$CH_3$, $R_8$=H, $R_9$=H) | H | H | Cl | $OCH_3$ |
| J-9 | Q-110 ($R_7$=H, $R_8$=$CH_3$, $R_9$=H) | H | H | $CH_3$ | $OCH_3$ |
| J-9 | Q-111 ($R_7$=H, $R_8$=H, $R_9$=H) | H | H | $CH_3$ | $OCH_3$ |
| J-9 | Q-111 ($R_7$=$CH_3$, $R_8$=H, $R_9$=H) | $CH_3$ | H | $CH_3$ | $OCH_3$ |
| J-9 | Q-111 ($R_7$=H, $R_8$=H, $R_9$=$CH_3$) | H | H | $OCH_3$ | $OCH_3$ |
| J-9 | Q-112 ($R_7$=H, $R_8$=H, $R_9$=H) | H | H | $OCH_3$ | $OCH_3$ |
| J-9 | Q-112 ($R_7$=$CH_3$, $R_8$=H, $R_9$=H) | H | H | $OCH_3$ | $OCH_3$ |
| J-9 | Q-112 ($R_7$=H, $R_8$=$CH_3$, $R_9$=$CH_3$) | H | 5-$OCH_3$ | $OCH_3$ | $OCH_3$ |
| J-9 | Q-113 ($R_7$=H, $R_8$=H, $R_9$=H, $R_3$=$CH_3$) | H | H | $OCH_3$ | $OCH_3$ |
| J-9 | Q-113 ($R_7$=$C_2H_5$, $R_8$=H, $R_9$=H, $R_3$=$\underline{i}$-$C_3H_7$) | H | H | $OCH_3$ | $OCF_2H$ |
| J-9 | Q-113 ($R_7$=H, $R_8$=H, $R_9$=$C_2H_5$, $R_3$=$\underline{n}$-$C_4H_9$) | H | 5-Cl | $OCH_3$ | $OCH_3$ |
| J-9 | Q-114 ($R_7$=H, $R_8$=H, $R_9$=H, $R_3$=$CH_3$) | H | H | $OCH_3$ | $OCH_3$ |
| J-9 | Q-114 ($R_7$=$\underline{n}$-$C_4H_9$, $R_8$=H, $R_9$=H, $R_3$=$C_2H_5$) | $CH_3$ | H | $OCH_3$ | $CH_3$ |
| J-9 | Q-114 ($R_7$=H, $R_8$=$CH_3$, $R_8$=$\underline{i}$-$C_3H_7$, $R_3$=H) | H | 5-$OCH_3$ | $OCH_3$ | $OCH_3$ |
| J-9 | Q-115 ($R_7$=H, $R_8$=H, $R_9$=H, $R_3$=$CH_3$) | H | H | $OCH_3$ | $OCH_3$ |
| J-9 | Q-115 ($R_7$=$\underline{i}$-$C_3H_7$, $R_8$=H, $R_9$=H, $R_3$=$CH_3$) | H | H | $OCF_2H$ | $CH_3$ |
| J-9 | Q-115 ($R_7$=H, $R_8$=$C_2H_5$, $R_9$=$CH_3$, $R_3$=H) | H | 5-Cl | $OCH_3$ | $CH_3$ |
| J-9 | Q-116 ($R_7$=H, $R_8$=H, $R_9$=H, $R_{10}$=H) | H | H | $CH_3$ | $OCH_3$ |
| J-9 | Q-116 ($R_7$=$CH_3$, $R_8$=H, $R_9$=H, $R_{10}$=H) | H | H | $CH_3$ | $OCH_3$ |
| J-9 | Q-116 ($R_7$=H, $R_8$=H, $R_9$=H, $R_{10}$=$CH_3$) | H | 5-$CH_3$ | $OCH_3$ | $OCH_3$ |
| J-9 | Q-118 ($R_7$=H, $R_8$=H, $R_9$=H, $R_{10}$=H) | H | H | $CH_3$ | $OCH_3$ |
| J-9 | Q-118 ($R_7$=$CH_3$, $R_8$=H, $R_9$=H, $R_{10}$=H) | $CH_3$ | H | $CH_3$ | $OCH_3$ |
| J-9 | Q-118 ($R_7$=H, $R_8$=H, $R_9$=H, $R_{10}$=$CH_3$) | H | H | $OCH_3$ | $OCH_3$ |
| J-9 | Q-119 ($R_7$=H, $R_8$=H, $R_9$=H, $R_2$=H) | H | H | $OCH_3$ | $OCH_3$ |

Table 1 (continued)

| J | Q | R | $R_{1c}$ | X | Y |
|---|---|---|---|---|---|
| J-9 | Q-119 ($R_7$=H, $R_8$=H, $R_9$=H, $R_2$=$CH_3$) | H | H | $OCH_3$ | $OCH_3$ |
| J-9 | Q-119 ($R_7$=H, $R_8$=$CH_3$, $R_9$=H, $R_2$=$C_2H_5$) | H | 5-$OCH_3$ | $OCH_3$ | $OCH_3$ |
| J-9 | Q-120 ($R_7$=H, $R_8$=H, $R_9$=H, $R_2$=H) | H | H | $OCH_3$ | $OCH_3$ |
| J-9 | Q-120 ($R_7$=H, $R_8$=H, $R_9$=H, $R_2$=$CH_3$) | H | H | $OCH_3$ | $OCF_2H$ |
| J-9 | Q-120 ($R_7$=H, $R_8$=H, $R_9$=$CH_3$, $R_2$=$CH_3$) | H | 5-Cl | $OCH_3$ | $OCH_3$ |
| J-9 | Q-121 ($R_7$=H, $R_8$=H, $R_9$=H, $R_2$=H) | H | H | $OCH_3$ | $OCH_3$ |
| J-9 | Q-121 ($R_7$=H, $R_8$=H, $R_9$=H, $R_2$=$C_2H_5$) | $CH_3$ | H | $OCH_3$ | $CH_3$ |
| J-9 | Q-121 ($R_7$=$CH_3$, $R_8$=$CH_3$, $R_9$=H, $R_2$=H) | H | 5-$OCH_3$ | $OCH_3$ | $OCH_3$ |
| J-9 | Q-122 ($R_7$=H, $R_8$=H, $R_9$=H, $R_2$=H) | H | H | $OCH_3$ | $OCH_3$ |
| J-9 | Q-122 ($R_7$=H, $R_8$=H, $R_9$=H, $R_2$=$CH_3$) | H | H | $OCF_2H$ | $CH_3$ |
| J-9 | Q-122 ($R_7$=$CH_3$, $R_8$=H, $R_9$=H, $R_2$=H) | H | 5-Cl | $OCH_3$ | $CH_3$ |
| J-9 | Q-123 ($R_7$=H, $R_8$=H, $R_9$=H) | H | H | $CH_3$ | $OCH_3$ |
| J-9 | Q-123 ($R_7$=H, $R_8$=H, $R_9$=$CH_3$) | H | H | $CH_3$ | $OCH_3$ |
| J-9 | Q-123 ($R_7$=$CH_3$, $R_8$=H, $R_9$=$CH_3$) | H | 5-$CH_3$ | $OCH_3$ | $OCH_3$ |
| J-9 | Q-124 ($R_7$=H, $R_8$=H, $R_9$=H) | H | H | $OCH_3$ | $OCH_3$ |
| J-9 | Q-124 ($R_7$=H, $R_8$=H, $R_9$=$CH_3$) | H | H | Cl | $OCH_3$ |
| J-9 | Q-124 ($R_7$=$C_2H_5$, $R_8$=H, $R_9$=$C_2H_5$) | H | H | $CH_3$ | $OCH_3$ |
| J-9 | Q-125 ($R_7$=H, $R_8$=H, $R_9$=H) | H | H | $CH_3$ | $OCH_3$ |
| J-9 | Q-125 ($R_7$=H, $R_8$=H, $R_9$=n-$C_4H_9$) | $CH_3$ | H | $CH_3$ | $OCH_3$ |
| J-9 | Q-125 ($R_7$=H, $R_8$=$CH_3$, $R_9$=H) | H | H | $OCH_3$ | $OCH_3$ |
| J-9 | Q-126 ($R_7$=H, $R_8$=H, $R_9$=H) | H | H | $OCH_3$ | $OCH_3$ |
| J-9 | Q-126 ($R_7$=H, $R_8$=H, $R_9$=s-$C_3H_7$) | H | H | $OCH_3$ | $OCH_3$ |
| J-9 | Q-126 ($R_7$=n-$C_3H_7$, $R_8$=H, $R_9$=H) | H | 5-$OCH_3$ | $OCH_3$ | $OCH_3$ |
| J-9 | Q-127 ($R_7$=H, $R_8$=H, $R_9$=H, $R_3$=H) | H | H | $OCH_3$ | $OCH_3$ |
| J-9 | Q-127 ($R_7$=H, $R_8$=H, $R_9$=H, $R_3$=$CH_3$) | H | H | $OCH_3$ | $OCF_2H$ |
| J-9 | Q-127 ($R_7$=$CH_3$, $R_8$=H, $R_9$=H, $R_3$=$C_2H_5$) | H | 5-Cl | $OCH_3$ | $OCH_3$ |
| J-9 | Q-128 ($R_7$=H, $R_8$=H, $R_9$=H, $R_3$=$CH_3$) | H | H | $OCH_3$ | $OCH_3$ |
| J-9 | Q-128 ($R_7$=$CH_3$, $R_8$=H, $R_9$=H, $R_3$=$CH_3$) | $CH_3$ | H | $OCH_3$ | $OCH_3$ |
| J-9 | Q-128 ($R_7$=H, $R_8$=H, $R_9$=$CH_3$, $R_3$=$C_2H_5$) | H | 5-$OCH_3$ | $OCH_3$ | $OCH_3$ |
| J-9 | Q-129 ($R_7$=H, $R_8$=H, $R_9$=H, $R_3$=$CH_3$) | H | H | $OCH_3$ | $OCH_3$ |
| J-9 | Q-129 ($R_7$=H, $R_8$=$CH_3$, $R_9$=H, $R_3$=i-$C_3H_7$) | H | H | $OCF_2H$ | $CH_3$ |
| J-9 | Q-129 ($R_7$=$CH_3$, $R_8$=H, $R_9$=$CH_3$, $R_3$=$CH_3$) | H | 5-Cl | $OCH_3$ | $CH_3$ |

Table 1 (continued)

| J | Q | R | $R_{1c}$ | X | Y |
|---|---|---|---|---|---|
| J-9 | Q-130 ($R_7$=H, $R_8$=H, $R_9$=H, $R_3$=$CH_3$) | H | H | $CH_3$ | $OCH_3$ |
| J-9 | Q-130 ($R_7$=$CH_3$, $R_8$=H, $R_9$=$CH_3$, $R_3$=H) | H | H | $CH_3$ | $OCH_3$ |
| J-9 | Q-130 ($R_7$=H, $R_8$=H, $R_9$=$C_2H_5$, $R_3$=$CH_3$) | H | 5-$CH_3$ | $OCH_3$ | $OCH_3$ |
| J-9 | Q-131 ($R_7$=H, $R_8$=H, $R_9$=H, $R_{10}$=H) | H | H | $OCH_3$ | $OCH_3$ |
| J-9 | Q-131 ($R_7$=$CH_3$, $R_8$=H, $R_9$=H, $R_{10}$=$CH_3$) | H | H | Cl | $OCH_3$ |
| J-9 | Q-131 ($R_7$=$CH_3$, $R_8$=$CH_3$, $R_9$=H, $R_{10}$=H) | H | H | $CH_3$ | $OCH_3$ |
| J-9 | Q-132 ($R_7$=H, $R_8$=H, $R_9$=H) | H | H | $CH_3$ | $OCH_3$ |
| J-9 | Q-132 ($R_7$=H, $R_8$=H, $R_9$=$CH_3$) | $CH_3$ | H | $CH_3$ | $OCH_3$ |
| J-9 | Q-132 ($R_7$=H, $R_8$=$C_2H_5$, $R_9$=H) | H | H | $OCH_3$ | $OCH_3$ |
| J-9 | Q-133 ($R_7$=H, $R_8$=H, $R_9$=H) | H | H | $OCH_3$ | $OCH_3$ |
| J-9 | Q-133 ($R_7$=$CH_3$, $R_8$=H, $R_9$=H) | H | H | $OCH_3$ | $OCH_3$ |
| J-9 | Q-133 ($R_7$=H, $R_8$=H, $R_9$=$C_2H_5$) | H | 5-$OCH_3$ | $OCH_3$ | $OCH_3$ |
| J-9 | Q-134 ($R_7$=H, $R_8$=H, $R_9$=H) | H | H | $OCH_3$ | $OCH_3$ |
| J-9 | Q-134 ($R_7$=$CH_3$, $R_8$=H, $R_9$=H) | H | H | $OCH_3$ | $OCF_2H$ |
| J-9 | Q-134 ($R_7$=H, $R_8$=H, $R_9$=$C_2H_5$) | H | 5-Cl | $OCH_3$ | $OCH_3$ |
| J-9 | Q-135 ($R_7$=H, $R_8$=H, $R_9$=H) | H | H | $OCH_3$ | $OCH_3$ |
| J-9 | Q-135 ($R_7$=H, $R_8$=H, $R_9$=$CH_3$) | $CH_3$ | H | $OCH_3$ | $OCH_3$ |
| J-9 | Q-135 ($R_7$=$CH_3$, $R_8$=H, $R_9$=H) | H | 5-$OCH_3$ | $OCH_3$ | $OCH_3$ |
| J-9 | Q-136 ($R_7$=H, $R_8$=H, $R_9$=H) | H | H | $OCH_3$ | $OCH_3$ |
| J-9 | Q-136 ($R_7$=H, $R_8$=H, $R_9$=$CH_3$) | H | H | $OCF_2H$ | $CH_3$ |
| J-9 | Q-136 ($R_7$=H, $R_8$=$CH_3$, $R_9$=$C_2H_5$) | H | 5-Cl | $OCH_3$ | $CH_3$ |
| J-9 | Q-137 ($R_7$=H, $R_8$=H, $R_9$=H) | H | H | $CH_3$ | $OCH_3$ |
| J-9 | Q-137 ($R_7$=$CH_3$, $R_8$=H, $R_9$=H) | H | H | $CH_3$ | $OCH_3$ |
| J-9 | Q-137 ($R_7$=H, $R_8$=H, $R_9$=$CH_3$) | H | 5-$CH_3$ | $OCH_3$ | $OCH_3$ |
| J-9 | Q-138 ($R_7$=H, $R_8$=H, $R_9$=H) | H | H | $OCH_3$ | $OCH_3$ |
| J-9 | Q-138 ($R_7$=$CH_3$, $R_8$=H, $R_9$=H) | H | H | Cl | $OCH_3$ |
| J-9 | Q-138 ($R_7$=H, $R_8$=H, $R_9$=$CH_3$) | H | H | $CH_3$ | $OCH_3$ |
| J-9 | Q-139 ($R_7$=H, $R_8$=H, $R_9$=H) | H | H | $CH_3$ | $OCH_3$ |
| J-9 | Q-139 ($R_7$=$CH_3$, $R_8$=H, $R_9$=H) | $CH_3$ | H | $CH_3$ | $OCH_3$ |
| J-9 | Q-139 ($R_7$=$CH_3$, $R_8$=$CH_3$, $R_9$=H) | H | H | $OCH_3$ | $OCH_3$ |
| J-9 | Q-140 ($R_7$=H, $R_8$=H, $R_9$=H, $R_3$=$CH_3$) | H | H | $OCH_3$ | $OCH_3$ |
| J-9 | Q-140 ($R_7$=H, $R_8$=H, $R_9$=$CH_3$, $R_3$=H) | H | H | $OCH_3$ | $OCH_3$ |

Table 1 (continued)

| J | Q | R | $R_{1c}$ | X | Y |
|---|---|---|---|---|---|
| J-9 | Q-140 ($R_7$=H, $R_8$=$CH_3$, $R_9$=H, $R_3$=$C_2H_5$) | H | 5-$OCH_3$ | $OCH_3$ | $OCH_3$ |
| J-9 | Q-141 ($R_7$=H, $R_8$=H, $R_9$=H, $R_3$=$CH_3$) | H | H | $OCH_3$ | $OCH_3$ |
| J-9 | Q-141 ($R_7$=H, $R_8$=H, $R_9$=H, $R_3$=H) | H | H | $OCH_3$ | $OCF_2H$ |
| J-9 | Q-141 ($R_7$=H, $R_8$=H, $R_9$=$CH_3$, $R_3$=$CH_3$) | H | 5-Cl | $OCH_3$ | $OCH_3$ |
| J-9 | Q-142 ($R_7$=H, $R_8$=H, $R_9$=H, $R_{10}$=H) | H | H | $OCH_3$ | $OCH_3$ |
| J-9 | Q-142 ($R_7$=$CH_3$, $R_8$=H, $R_9$=H, $R_{10}$=H) | $CH_3$ | H | $OCH_3$ | $CH_3$ |
| J-9 | Q-142 ($R_7$=H, $R_8$=H, $R_9$=H, $R_{10}$=$CH_3$) | H | 5-$OCH_3$ | $OCH_3$ | $OCH_3$ |
| J-9 | Q-143 ($R_7$=H, $R_8$=H, $R_9$=H, $R_3$=$CH_3$) | H | H | $OCH_3$ | $OCH_3$ |
| J-9 | Q-143 ($R_7$=H, $R_8$=H, $R_9$=H, $R_3$=H) | H | H | $OCF_2H$ | $CH_3$ |
| J-9 | Q-143 ($R_7$=$CH_3$, $R_8$=H, $R_9$=$CH_3$, $R_3$=$CH_3$) | H | 5-Cl | $OCH_3$ | $CH_3$ |
| J-9 | Q-144 ($R_7$=H, $R_8$=H, $R_9$=H, $R_3$=$CH_3$) | H | H | $CH_3$ | $OCH_3$ |
| J-9 | Q-144 ($R_7$=$CH_3$, $R_8$=H, $R_9$=H, $R_3$=$CH_3$) | H | H | $CH_3$ | $OCH_3$ |
| J-9 | Q-144 ($R_7$=H, $R_8$=$C_2H_5$, $R_9$=H, $R_3$=$CH_3$) | H | 5-$CH_3$ | $OCH_3$ | $OCH_3$ |
| J-9 | Q-145 ($R_7$=H, $R_8$=H, $R_9$=H) | H | H | $OCH_3$ | $OCH_3$ |
| J-9 | Q-145 ($R_7$=H, $R_8$=H, $R_9$=$CH_3$) | H | H | Cl | $OCH_3$ |
| J-9 | Q-145 ($R_7$=$CH_3$, $R_8$=H, $R_9$=H) | H | H | $CH_3$ | $OCH_3$ |
| J-9 | Q-146 ($R_7$=H, $R_8$=H, $R_9$=H) | H | H | $CH_3$ | $OCH_3$ |
| J-9 | Q-146 ($R_7$=$CH_3$, $R_8$=H, $R_9$=H) | $CH_3$ | H | $CH_3$ | $OCH_3$ |
| J-9 | Q-146 ($R_7$=H, $R_8$=H, $R_9$=<u>n</u>-$C_4H_9$) | H | H | $OCH_3$ | $OCH_3$ |
| J-9 | Q-147 ($R_7$=H, $R_8$=H, $R_9$=H) | H | H | $OCH_3$ | $OCH_3$ |
| J-9 | Q-147 ($R_7$=$CH_3$, $R_8$=H, $R_9$=H) | H | H | $OCH_3$ | $OCH_3$ |
| J-9 | Q-147 ($R_7$=H, $R_8$=H, $R_9$=<u>n</u>-$C_4H_9$) | H | 5-$OCH_3$ | $OCH_3$ | $OCH_3$ |
| J-9 | Q-148 ($R_7$=H, $R_8$=H, $R_9$=H) | H | H | $OCH_3$ | $OCH_3$ |
| J-9 | Q-148 ($R_7$=H, $R_8$=H, $R_9$=$CH_3$) | H | H | $OCH_3$ | $OCF_2H$ |
| J-9 | Q-148 ($R_7$=$CH_3$, $R_8$=H, $R_9$=H) | H | 5-Cl | $OCH_3$ | $OCH_3$ |
| J-9 | Q-149 ($R_7$=H, $R_8$=H, $R_9$=H) | H | H | $OCH_3$ | $OCH_3$ |
| J-9 | Q-149 ($R_7$=H, $R_8$=H, $R_9$=$CH_3$) | $CH_3$ | H | $OCH_3$ | $OCH_3$ |
| J-9 | Q-149 ($R_7$=$CH_3$, $R_8$=H, $R_9$=H) | H | 5-$OCH_3$ | $OCH_3$ | $OCH_3$ |
| J-9 | Q-150 ($R_7$=H, $R_8$=H, $R_9$=H) | H | H | $OCH_3$ | $OCH_3$ |
| J-9 | Q-150 ($R_7$=$CH_3$, $R_8$=H, $R_9$=H) | H | H | $OCF_2H$ | $CH_3$ |
| J-9 | Q-150 ($R_7$=H, $R_8$=$CH_3$, $R_9$=$CH_3$) | H | 5-Cl | $OCH_3$ | $CH_3$ |
| J-9 | Q-151 ($R_7$=H, $R_8$=H, $R_9$=H) | H | H | $CH_3$ | $OCH_3$ |

Table 1 (continued)

| J | Q | R | $R_{1c}$ | X | Y |
|---|---|---|---|---|---|
| J-9 | Q-151 ($R_7$=$CH_3$, $R_8$=H, $R_9$=H) | H | H | $CH_3$ | $OCH_3$ |
| J-9 | Q-151 ($R_7$=$CH_3$, $R_8$=$CH_3$, $R_9$=H) | H | 5-$CH_3$ | $OCH_3$ | $OCH_3$ |
| J-9 | Q-152 ($R_7$=H, $R_8$=H, $R_9$=H) | H | H | $OCH_3$ | $OCH_3$ |
| J-9 | Q-152 ($R_7$=$C_2H_5$, $R_8$=H, $R_9$=H) | H | H | Cl | $OCH_3$ |
| J-9 | Q-152 ($R_7$=$CH_3$, $R_8$=$CH_3$, $R_9$=H) | H | H | $CH_3$ | $OCH_3$ |
| J-9 | Q-153 ($R_7$=H, $R_8$=H, $R_9$=H, $R_{11}$=$CH_3$) | H | H | $CH_3$ | $OCH_3$ |
| J-9 | Q-153 ($R_7$=H, $R_8$=H, $R_9$=H, $R_{11}$=H) | $CH_3$ | H | $CH_3$ | $OCH_3$ |
| J-9 | Q-153 ($R_7$=$CH_3$, $R_8$=H, $R_9$=H, $R_{11}$=$CH_3$) | H | H | $OCH_3$ | $OCH_3$ |
| J-9 | Q-154 ($R_7$=H, $R_8$=H, $R_9$=H, $R_{11}$=$CH_3$) | H | H | $OCH_3$ | $OCH_3$ |
| J-9 | Q-154 ($R_7$=$CH_3$, $R_8$=H, $R_9$=H) | H | H | $OCH_3$ | $OCH_3$ |
| J-9 | Q-154 ($R_7$=H, $R_8$=$CH_3$, $R_9$=$CH_3$) | H | 5-$OCH_3$ | $OCH_3$ | $OCH_3$ |
| J-9 | Q-155 ($R_7$=H, $R_8$=H, $R_9$=H) | H | H | $OCH_3$ | $OCH_3$ |
| J-9 | Q-155 ($R_7$=$CH_3$, $R_8$=H, $R_9$=H) | H | H | $OCH_3$ | $OCF_2H$ |
| J-9 | Q-155 ($R_7$=$CH_3$, $R_8$=$CH_3$, $R_9$=H) | H | 5-Cl | $OCH_3$ | $OCH_3$ |
| J-9 | Q-156 ($R_7$=H, $R_8$=H, $R_9$=H) | H | H | $OCH_3$ | $OCH_3$ |
| J-9 | Q-156 ($R_7$=$C_2H_5$, $R_8$=H, $R_9$=H) | $CH_3$ | H | $OCH_3$ | $OCH_3$ |
| J-9 | Q-156 ($R_7$=$CH_3$, $R_8$=$CH_3$, $R_9$=H) | H | 5-$OCH_3$ | $OCH_3$ | $OCH_3$ |
| J-9 | Q-158 ($R_7$=H, $R_8$=H, $R_9$=H) | H | H | $CH_3$ | $OCH_3$ |
| J-9 | Q-158 ($R_7$=H, $R_8$=H, $R_9$=$CH_3$) | H | H | $CH_3$ | $OCH_3$ |
| J-9 | Q-158 ($R_7$=$CH_3$, $R_8$=H, $R_9$=H) | H | 5-$CH_3$ | $OCH_3$ | $OCH_3$ |
| J-9 | Q-159 ($R_7$=H, $R_8$=H, $R_9$=H) | H | H | $OCH_3$ | $OCH_3$ |
| J-9 | Q-159 ($R_7$=$CH_3$, $R_8$=H, $R_9$=H) | H | H | Cl | $OCH_3$ |
| J-9 | Q-159 ($R_7$=H, $R_8$=H, $R_9$=$CH_3$) | H | H | $CH_3$ | $OCH_3$ |
| J-9 | Q-160 ($R_7$=H, $R_8$=H, $R_9$=H, $R_{11}$=$CH_3$) | H | H | $CH_3$ | $OCH_3$ |
| J-9 | Q-160 ($R_7$=H, $R_8$=H, $R_9$=$CH_3$, $R_{11}$=H) | $CH_3$ | H | $CH_3$ | $OCH_3$ |
| J-9 | Q-160 ($R_7$=$CH_3$, $R_8$=H, $R_9$=H, $R_{11}$=<u>i</u>-$C_3H_7$) | H | H | $OCH_3$ | $OCH_3$ |
| J-9 | Q-161 ($R_7$=H, $R_8$=H, $R_9$=H, $R_{11}$=$CH_3$) | H | H | $OCH_3$ | $OCH_3$ |
| J-9 | Q-161 ($R_7$=$CH_3$, $R_8$=H, $R_9$=H, $R_{11}$=H) | H | H | $OCH_3$ | $OCH_3$ |
| J-9 | Q-161 ($R_7$=H, $R_8$=H, $R_9$=$CH_3$, $R_{11}$=$CH_3$) | H | 5-$OCH_3$ | $OCH_3$ | $OCH_3$ |

Table 1 (continued)

| J | Q | R | $R_{1c}$ | X | Y | m.p.(°C) |
|---|---|---|---|---|---|---|
| J-9 | Q-162 ($R_7$=H, $R_8$=H, $R_9$=H, $R_{10}$=H) | H | H | $OCH_3$ | $OCH_3$ | |
| J-9 | Q-162 ($R_7$=$CH_3$, $R_8$=H, $R_9$=H, $R_{10}$=H) | H | H | $OCH_3$ | $OCF_2H$ | |
| J-9 | Q-162 ($R_7$=H, $R_8$=H, $R_9$=H, $R_{10}$=$CH_3$) | H | 5-Cl | $OCH_3$ | $OCH_3$ | |
| J-9 | Q-163 ($R_2$=H, $R_7$=H, $R_8$=H, $R_9$=H) | H | H | $OCH_3$ | $OCH_3$ | |
| J-9 | Q-164 ($R_7$=H) | H | H | $OCH_3$ | $OCH_3$ | |
| J-9 | Q-165 ($R_3$=H, $R_7$=H) | H | H | $OCH_3$ | $OCH_3$ | |
| J-9 | Q-166 ($R_3$=H, $R_7$=H) | H | H | $OCH_3$ | $OCH_3$ | |
| J-9 | Q-167 ($R_7$=H, $R_8$=H, $R_9$=H, $R_{10}$=H) | H | H | $OCH_3$ | $OCH_3$ | 204-205 |
| J-9 | Q-167 ($R_7$=H, $R_8$=H, $R_9$=H, $R_{10}$=H) | H | H | $CH_3$ | $CH_3$ | 224-225(d) |
| J-10 | Q-1 ($R_7$=H, $R_8$=H) | H | H | $CH_3$ | $CH_3$ | |
| J-10 | Q-1 ($R_7$=H, $R_8$=H) | H | H | $OCH_3$ | $CH_3$ | |
| J-10 | Q-1 ($R_7$=H, $R_8$=H) | H | H | $OCH_3$ | $OCH_3$ | |
| J-10 | Q-1 ($R_7$=H, $R_8$=H) | H | 5-$CH_3$ | $OCH_3$ | $OCH_3$ | |
| J-10 | Q-1 ($R_7$=H, $R_8$=H) | H | 5-$OCH_3$ | $OCH_3$ | $OCH_3$ | |
| J-10 | Q-1 ($R_7$=H, $R_8$=H) | H | 6-$OCH_2CH_2Br$ | $OCH_3$ | $OCH_3$ | |
| J-10 | Q-1 ($R_7$=H, $R_8$=H) | H | 2-$CH_2F$ | $OCH_3$ | $OCH_3$ | |
| J-10 | Q-1 ($R_7$=H, $R_8$=H) | H | 2-$SCH_3$ | $OCH_3$ | $OCH_3$ | |
| J-10 | Q-1 ($R_7$=H, $R_8$=H) | H | 2-$SCH_2F$ | $OCH_3$ | $OCH_3$ | |
| J-10 | Q-1 ($R_7$=H, $R_8$=H) | H | 2-$NHCH_3$ | $OCH_3$ | $OCH_3$ | |
| J-10 | Q-1 ($R_7$=H, $R_8$=H) | H | 2-Cl | $OCH_3$ | $OCH_3$ | |
| J-10 | Q-1 ($R_7$=H, $R_8$=H) | H | 2-$NO_2$ | $OCH_3$ | $OCH_3$ | |
| J-10 | Q-1 ($R_7$=H, $R_8$=H) | H | 2-$OCF_2H$ | $OCH_3$ | $OCH_3$ | |
| J-10 | Q-1 ($R_7$=H, $R_8$=H) | H | H | $OCH_3$ | $CH_2CH_3$ | |
| J-10 | Q-1 ($R_7$=H, $R_8$=H) | H | H | $OCH_3$ | $OCH_2CH_3$ | |
| J-10 | Q-1 ($R_7$=H, $R_8$=H) | H | H | $OCF_2H$ | $OCH_3$ | |
| J-10 | Q-1 ($R_7$=H, $R_8$=H) | H | H | $OCH_2CF_3$ | $OCH_3$ | |
| J-10 | Q-1 ($R_7$=H, $R_8$=H) | H | H | $CF_3$ | $OCH_3$ | |
| J-10 | Q-1 ($R_7$=H, $R_8$=H) | H | H | $SCH_3$ | $OCH_3$ | |
| J-10 | Q-1 ($R_7$=H, $R_8$=H) | H | H | $SCHF_2$ | $OCH_3$ | |
| J-10 | Q-1 ($R_7$=H, $R_8$=H) | H | H | Cl | $OCH_3$ | |
| J-10 | Q-1 ($R_7$=H, $R_8$=H) | H | H | $OCH_3$ | $CH_2OCH_3$ | |
| J-10 | Q-1 ($R_7$=H, $R_8$=H) | H | H | $OCH_3$ | $NH_2$ | |
| J-10 | Q-1 ($R_7$=H, $R_8$=H) | H | H | $OCH_3$ | $NHCH_3$ | |

Table 1 (continued)

| J | Q | R | $R_{1c}$ | X | Y |
|---|---|---|---|---|---|
| J-10 | Q-1 ($R_7$=H, $R_8$=H) | H | H | H | $OCH_3$ |
| J-10 | Q-1 ($R_7$=H, $R_8$=H) | H | H | $OCH_3$ | $OCH_2C{\equiv}CH$ |
| J-10 | Q-1 ($R_7$=H, $R_8$=H) | H | H | $OCH_3$ | $CH_2S(CH_2)_3CH_3$ |
| J-10 | Q-1 ($R_7$=H, $R_8$=H) | H | H | $OCH_3$ | cyclopropyl |
| J-10 | Q-1 ($R_7$=H, $R_8$=H) | H | H | $OCH_3$ | $C{\equiv}CH$ |
| J-10 | Q-1 ($R_7$=H, $R_8$=H) | H | H | $OCH_3$ | $-CH(OCH_3)_2$ |
| J-10 | Q-2 ($R_7$=H, $R_8$=H) | H | H | $OCH_3$ | $OCH_3$ |
| J-10 | Q-3 ($R_7$=H, $R_8$=H) | H | H | $OCH_3$ | $OCH_3$ |
| J-10 | Q-4 ($R_7$=H, $R_8$=H, $R_3$=$CH_3$) | H | H | $CH_3$ | $OCH_3$ |
| J-10 | Q-5 ($R_7$-H, $R_8$=H, $R_3$=H) | H | H | $OCH_3$ | $OCH_3$ |
| J-10 | Q-6 ($R_7$=$CH_3$, $R_8$=H, $R_3$=$CH_3$) | H | H | $CH_3$ | $OCH_3$ |
| J-10 | Q-8 ($R_7$=H, $R_8$=H, $R_2$=H) | H | H | $OCH_3$ | $OCH_3$ |
| J-10 | Q-9 ($R_7$=H, $R_8$=H, $R_2$=H) | H | H | $OCH_3$ | $OCH_3$ |
| J-10 | Q-10 ($R_7$=H, $R_8$=H) | H | H | $OCH_3$ | $OCH_3$ |
| J-10 | Q-11 ($R_7$=H, $R_8$=H) | H | H | $CH_3$ | $OCH_3$ |
| J-10 | Q-12 ($R_7$=H, $R_8$=H) | H | H | $OCH_3$ | $OCH_3$ |
| J-10 | Q-13 ($R_7$=H, $R_8$=H, $R_3$=H) | H | H | $CH_3$ | $OCH_3$ |
| J-10 | Q-14 ($R_7$=H, $R_8$=H, $R_3$=$CH_3$) | H | H | $OCH_3$ | $OCH_3$ |
| J-10 | Q-15 ($R_7$=CH , $R_8$=H, $R_3$=H) | H | H | $OCH_3$ | $OCH_3$ |
| J-10 | Q-17 ($R_7$=H, $R_8$=H, $R_2$=H) | H | H | $OCH_3$ | $OCH_3$ |
| J-10 | Q-18 ($R_7$=H, $R_8$=H, $R_2$=H) | H | H | $CH_3$ | $OCH_3$ |
| J-10 | Q-19 ($R_7$=H, $R_8$=H) | H | H | $OCH_3$ | $OCH_3$ |
| J-10 | Q-20 ($R_7$=H, $R_8$=H) | H | H | $CH_3$ | $OCH_3$ |
| J-10 | Q-21 ($R_7$=H, $R_8$=H) | H | H | $OCH_3$ | $OCH_3$ |
| J-10 | Q-22 ($R_7$=H, $R_8$=H, $R_3$=H) | H | H | $OCH_3$ | $OCH_3$ |
| J-10 | Q-23 ($R_7$=H, $R_8$=H, $R_3$=H) | H | H | $OCH_3$ | $OCH_3$ |
| J-10 | Q-24 ($R_7$=H, $R_8$=H, $R_3$=$CH_3$) | H | H | $OCH_3$ | $OCH_3$ |
| J-10 | Q-25 ($R_7$=H, $R_8$=H, $R_9$=H) | H | H | $CH_3$ | $OCH_3$ |
| J-10 | Q-26 ($R_7$=H, $R_8$=H) | H | H | $OCH_3$ | $OCH_3$ |
| J-10 | Q-27 ($R_7$=H, $R_8$=H) | H | H | $CH_3$ | $OCH_3$ |

Table 1 (continued)

| J | Q | R | $R_{1c}$ | X | Y |
|---|---|---|---|---|---|
| J-10 | Q-28 ($R_7$=H, $R_8$=H) | H | H | $OCH_3$ | $OCH_3$ |
| J-10 | Q-29 ($R_7$=H, $R_8$=H) | H | H | $OCH_3$ | $OCH_3$ |
| J-10 | Q-30 ($R_7$=H, $R_8$=H) | H | H | $OCH_3$ | $OCH_3$ |
| J-10 | Q-31 ($R_7$=H, $R_8$=H) | H | H | $OCH_3$ | $OCH_3$ |
| J-10 | Q-32 ($R_7$=H, $R_8$=H, $R_3$=H) | H | H | $CH_3$ | $OCH_3$ |
| J-10 | Q-33 ($R_7$=H, $R_8$=H, $R_8$=$CH_3$) | H | H | $OCH_3$ | $OCH_3$ |
| J-10 | Q-34 ($R_7$=H, $R_8$=H, $R_3$=H) | H | H | $CH_3$ | $OCH_3$ |
| J-10 | Q-36 ($R_7$=H, $R_8$=H, $R_2$=H) | H | H | $OCH_3$ | $OCH_3$ |
| J-10 | Q-37 ($R_7$=H, $R_8$=H, $R_2$=H) | H | H | $OCH_3$ | $OCH_3$ |
| J-10 | Q-38 ($R_7$=H, $R_8$=H, $R_2$=$OCF_2H$) | H | H | $OCH_3$ | $OCH_3$ |
| J-10 | Q-39 ($R_7$=H, $R_8$=H, $R_9$=H) | H | H | $CH_3$ | $OCH_3$ |
| J-10 | Q-40 ($R_7$=H, $R_8$=H) | H | H | $OCH_3$ | $OCH_3$ |
| J-10 | Q-41 ($R_7$=H, $R_8$=H) | H | H | $CH_3$ | $OCH_3$ |
| J-10 | Q-42 ($R_7$=H, $R_8$=H) | H | H | $OCH_3$ | $OCH_3$ |
| J-10 | Q-43 ($R_7$=H, $R_8$=H, $R_3$=H) | H | H | $OCH_3$ | $OCH_3$ |
| J-10 | Q-44 ($R_7$=H, $R_8$=H, $R_3$=$CH_3$) | H | H | $OCH_3$ | $OCH_3$ |
| J-10 | Q-45 ($R_7$=H, $R_8$=H, $R_3$=H) | H | H | $OCH_3$ | $OCH_3$ |
| J-10 | Q-47 ($R_7$=H, $R_8$=H, $R_2$=H) | H | H | $OCH_3$ | $OCH_3$ |
| J-10 | Q-48 ($R_7$=H, $R_8$=H, $R_2$=H) | H | H | $CH_3$ | $OCH_3$ |
| J-10 | Q-49 ($R_7$=H, $R_8$=H, $R_2$=H) | H | H | $OCH_3$ | $OCH_3$ |
| J-10 | Q-50 ($R_7$=H, $R_8$=H, $R_2$=H) | H | H | $OCH_3$ | $OCH_3$ |
| J-10 | Q-51 ($R_7$=H, $R_8$=H, $R_9$=H) | H | H | $OCH_3$ | $OCH_3$ |
| J-10 | Q-52 ($R_7$=H, $R_8$=H, $R_9$=H) | H | H | $OCH_3$ | $OCH_3$ |
| J-10 | Q-53 ($R_7$=H, $R_8$=H, $R_9$=H) | H | H | $CH_3$ | $OCH_3$ |
| J-10 | Q-54 ($R_7$=H, $R_8$=H, $R_9$=H) | H | H | $OCH_3$ | $OCH_3$ |
| J-10 | Q-55 ($R_7$=H, $R_8$=H, $R_9$=H, $R_3$=H) | H | H | $CH_3$ | $OCH_3$ |
| J-10 | Q-56 ($R_7$=H, $R_8$=H, $R_9$=H, $R_3$=$C_2H_5$) | H | H | $CH_3$ | $OCH_3$ |
| J-10 | Q-57 ($R_7$=H, $R_8$=H, $R_9$=H, $R_3$=$\underline{i}$-$C_3H_7$) | H | H | $OCH_3$ | $OCH_3$ |
| J-10 | Q-58 ($R_7$=H, $R_8$=H, $R_9$=H, $R_3$=H) | H | H | $OCH_3$ | $OCH_3$ |
| J-10 | Q-60 ($R_7$=H, $R_8$=H, $R_9$=H, $R_2$=H) | H | H | $CH_3$ | $OCH_3$ |

Table 1 (continued)

| J | Q | R | $R_{1c}$ | X | Y |
|---|---|---|---|---|---|
| J-10 | Q-61 ($R_7$=H, $R_8$=H, $R_9$=H, $R_2$=H) | H | H | $OCH_3$ | $OCH_3$ |
| J-10 | Q-62 ($R_7$=H, $R_8$=H, $R_9$=H, $R_2$=H) | H | H | $CH_3$ | $OCH_3$ |
| J-10 | Q-63 ($R_7$=H, $R_8$=H, $R_9$=H) | H | H | $OCH_3$ | $OCH_3$ |
| J-10 | Q-64 ($R_7$=H, $R_8$=H, $R_9$=H) | H | H | $OCH_3$ | $OCH_3$ |
| J-10 | Q-65 ($R_7$=H, $R_8$=H, $R_9$=H) | H | H | $OCH_3$ | $OCH_3$ |
| J-10 | Q-66 ($R_7$=H, $R_8$=H, $R_9$=H) | H | H | $OCH_3$ | $OCH_3$ |
| J-10 | Q-67 ($R_7$=H, $R_8$=H, $R_9$=H, $R_3$=H) | H | H | $CH_3$ | $OCH_3$ |
| J-10 | Q-68 ($R_7$=H, $R_8$=H, $R_9$=H, $R_3$=$CH_3$) | H | H | $OCH_3$ | $OCH_3$ |
| J-10 | Q-69 ($R_7$=H, $R_8$=H, $R_9$=H, $R_3$=$CH_3$) | H | H | $CH_3$ | $OCH_3$ |
| J-10 | Q-70 ($R_7$=H, $R_8$=H, $R_9$=H, $R_3$=$CH_3$) | H | H | $OCH_3$ | $OCH_3$ |
| J-10 | Q-72 ($R_7$=H, $R_8$=H, $R_9$=H, $R_2$=H) | H | H | $OCH_3$ | $OCH_3$ |
| J-10 | Q-73 ($R_7$=H, $R_8$=H, $R_9$=H, $R_2$=H) | H | H | $OCH_3$ | $OCH_3$ |
| J-10 | Q-74 ($R_7$=H, $R_8$=H, $R_9$=H, $R_2$=H) | H | H | $CH_3$ | $OCH_3$ |
| J-10 | Q-75 ($R_7$=H, $R_8$=H, $R_9$=H) | H | H | $OCH_3$ | $OCH_3$ |
| J-10 | Q-76 ($R_7$=H, $R_8$=H, $R_9$=H) | H | H | $CH_3$ | $OCH_3$ |
| J-10 | Q-77 ($R_7$=H, $R_8$=H, $R_9$=H) | H | H | $OCH_3$ | $OCH_3$ |
| J-10 | Q-78 ($R_7$=H, $R_8$=H, $R_9$=H) | H | H | $OCH_3$ | $OCH_3$ |
| J-10 | Q-79 ($R_7$=H, $R_8$=H, $R_9$=H, $R_3$=H) | H | H | $OCH_3$ | $OCH_3$ |
| J-10 | Q-80 ($R_7$=H, $R_8$=H, $R_9$=H, $R_3$=$CH_3$) | H | H | $OCH_3$ | $OCH_3$ |
| J-10 | Q-81 ($R_7$=H, $R_8$=H, $R_9$=H, $R_3$=$CH_3$) | H | H | $CH_3$ | $OCH_3$ |
| J-10 | Q-82 ($R_7$=H, $R_8$=H, $R_9$=H, $R_3$=$CH_3$) | H | H | $OCH_3$ | $OCH_3$ |
| J-10 | Q-83 ($R_7$=H, $R_8$=H, $R_9$=H, $R_{10}$=H) | H | H | $CH_3$ | $OCH_3$ |
| J-10 | Q-84 ($R_7$=H, $R_8$=H, $R_9$=H) | H | H | $OCH_3$ | $OCH_3$ |
| J-10 | Q-85 ($R_7$=H, $R_8$=H, $R_9$=H) | H | H | $OCH_3$ | $OCH_3$ |
| J-10 | Q-86 ($R_7$=H, $R_8$=H, $R_9$=H) | H | H | $OCH_3$ | $OCH_3$ |
| J-10 | Q-87 ($R_7$=H, $R_8$=H, $R_9$=H) | H | H | $OCH_3$ | $OCH_3$ |
| J-10 | Q-88 ($R_7$=H, $R_8$=H, $R_9$=H) | H | H | $CH_3$ | $OCH_3$ |
| J-10 | Q-89 ($R_7$=H, $R_8$=H, $R_9$=H) | H | H | $OCH_3$ | $OCH_3$ |
| J-10 | Q-90 ($R_7$=H, $R_8$=H, $R_9$=H, $R_3$=$CH_3$) | H | H | $CH_3$ | $OCH_3$ |
| J-10 | Q-91 ($R_7$=H, $R_8$=H, $R_9$=H, $R_3$=$CH_3$) | H | H | $OCH_3$ | $OCH_3$ |
| J-10 | Q-92 ($R_7$=H, $R_8$=H, $R_9$=H, $R_{10}$=H) | H | H | $OCH_3$ | $OCH_3$ |
| J-10 | Q-93 ($R_7$=H, $R_8$=H, $R_9$=H) | H | H | $OCH_3$ | $OCH_3$ |

Table 1 (continued)

| J | Q | R | $R_{1c}$ | X | Y |
|---|---|---|---|---|---|
| J-10 | Q-94 ($R_7$=H, $R_8$=H, $R_9$=H) | H | H | $OCH_3$ | $OCH_3$ |
| J-10 | Q-95 ($R_7$=H, $R_8$=H, $R_9$=H) | H | H | $CH_3$ | $OCH_3$ |
| J-10 | Q-96 ($R_7$=H, $R_8$=H, $R_9$=H) | H | H | $OCH_3$ | $OCH_3$ |
| J-10 | Q-97 ($R_7$=H, $R_8$=H, $R_9$=H) | H | H | $CH_3$ | $OCH_3$ |
| J-10 | Q-98 ($R_7$=H, $R_8$=H, $R_9$=H) | H | H | $OCH_3$ | $OCH_3$ |
| J-10 | Q-99 ($R_7$=H, $R_8$=H, $R_9$=H, $R_3$=H) | H | H | $OCH_3$ | $OCH_3$ |
| J-10 | Q-100 ($R_7$=H, $R_8$=H, $R_9$=H, $R_3$=$C_2H_5$) | H | H | $OCH_3$ | $OCH_3$ |
| J-10 | Q-101 ($R_7$=H, $R_8$=H, $R_9$=H, $R_3$=i-$C_2H_5$) | H | H | $OCH_3$ | $OCH_3$ |
| J-10 | Q-102 ($R_7$=H, $R_8$=H, $R_9$=H, $R_3$=H) | H | H | $CH_3$ | $OCH_3$ |
| J-10 | Q-104 ($R_7$=H, $R_8$=H, $R_9$=H, $R_2$=H) | H | H | $CH_3$ | $OCH_3$ |
| J-10 | Q-105 ($R_7$=H, $R_8$=H, $R_9$=H, $R_2$=H) | H | H | $OCH_3$ | $OCH_3$ |
| J-10 | Q-106 ($R_7$=H, $R_8$=H, $R_9$=H, $R_2$=H) | H | H | $OCH_3$ | $OCH_3$ |
| J-10 | Q-107 ($R_7$=H, $R_8$=H, $R_9$=H, $R_2$=H) | H | H | $OCH_3$ | $OCH_3$ |
| J-10 | Q-108 ($R_7$=H, $R_8$=H, $R_9$=H, $R_2$=H) | H | H | $OCH_3$ | $OCH_3$ |
| J-10 | Q-109 ($R_7$=H, $R_8$=H, $R_9$=H) | H | H | $CH_3$ | $OCH_3$ |
| J-10 | Q-110 ($R_7$=H, $R_8$=H, $R_9$=H) | H | H | $OCH_3$ | $OCH_3$ |
| J-10 | Q-111 ($R_7$=H, $R_8$=H, $R_9$=H) | H | H | $CH_3$ | $OCH_3$ |
| J-10 | Q-112 ($R_7$=H, $R_8$=H, $R_9$=H) | H | H | $OCH_3$ | $OCH_3$ |
| J-10 | Q-113 ($R_7$=H, $R_8$=H, $R_9$=H, $R_3$=$CH_3$) | H | H | $OCH_3$ | $OCH_3$ |
| J-10 | Q-114 ($R_7$=H, $R_8$=H, $R_9$=H, $R_3$=$CH_3$) | H | H | $OCH_3$ | $OCH_3$ |
| J-10 | Q-115 ($R_7$=H, $R_8$=H, $R_9$=H, $R_3$=$CH_3$) | H | H | $OCH_3$ | $OCH_3$ |
| J-10 | Q-116 ($R_7$=H, $R_8$=H, $R_9$=H, $R_{10}$=H) | H | H | $CH_3$ | $OCH_3$ |
| J-10 | Q-118 ($R_7$=H, $R_8$=H, $R_9$=H, $R_{10}$=H) | H | H | $CH_3$ | $OCH_3$ |
| J-10 | Q-119 ($R_7$=H, $R_8$=H, $R_9$=H, $R_2$=H) | H | H | $OCH_3$ | $OCH_3$ |
| J-10 | Q-120 ($R_7$=H, $R_8$=H, $R_9$=H, $R_2$=H) | H | H | $OCH_3$ | $OCH_3$ |
| J-10 | Q-121 ($R_7$=H, $R_8$=H, $R_9$=H, $R_2$=H) | H | H | $OCH_3$ | $OCH_3$ |
| J-10 | Q-122 ($R_7$=H, $R_8$=H, $R_9$=H, $R_2$=H) | H | H | $OCH_3$ | $OCH_3$ |
| J-10 | Q-123 ($R_7$=H, $R_8$=H, $R_9$=H) | H | H | $CH_3$ | $OCH_3$ |
| J-10 | Q-124 ($R_7$=H, $R_8$=H, $R_9$=H) | H | H | $OCH_3$ | $OCH_3$ |
| J-10 | Q-125 ($R_7$=H, $R_8$=H, $R_9$=H) | H | H | $CH_3$ | $OCH_3$ |

Table 1 (continued)

| J | Q | R | $R_{1c}$ | X | Y |
|---|---|---|---|---|---|
| J-10 | Q-126 ($R_7$=H, $R_8$=H, $R_9$=H) | H | H | $OCH_3$ | $OCH_3$ |
| J-10 | Q-127 ($R_7$=H, $R_8$=H, $R_9$=H, $R_3$=H) | H | H | $OCH_3$ | $OCH_3$ |
| J-10 | Q-128 ($R_7$=H, $R_8$=H, $R_9$=H, $R_3$=$CH_3$) | H | H | $OCH_3$ | $OCH_3$ |
| J-10 | Q-129 ($R_7$=H, $R_8$=H, $R_9$=H, $R_3$=$CH_3$) | H | H | $OCH_3$ | $OCH_3$ |
| J-10 | Q-130 ($R_7$=H, $R_8$=H, $R_9$=H, $R_3$=$CH_3$) | H | H | $CH_3$ | $OCH_3$ |
| J-10 | Q-131 ($R_7$=H, $R_8$=H, $R_9$=H, $R_{10}$=H) | H | H | $OCH_3$ | $OCH_3$ |
| J-10 | Q-132 ($R_7$=H, $R_8$=H, $R_9$=H) | H | H | $CH_3$ | $OCH_3$ |
| J-10 | Q-133 ($R_7$=H, $R_8$=H, $R_9$=H) | H | H | $OCH_3$ | $OCH_3$ |
| J-10 | Q-134 ($R_7$=H, $R_8$=H, $R_9$=H) | H | H | $OCH_3$ | $OCH_3$ |
| J-10 | Q-135 ($R_7$=H, $R_8$=H, $R_9$=H) | H | H | $OCH_3$ | $OCH_3$ |
| J-10 | Q-136 ($R_7$=H, $R_8$=H, $R_9$=H) | H | H | $OCH_3$ | $OCH_3$ |
| J-10 | Q-137 ($R_7$=H, $R_8$=H, $R_9$=H) | H | H | $CH_3$ | $OCH_3$ |
| J-10 | Q-138 ($R_7$=H, $R_8$=H, $R_9$=H) | H | H | $OCH_3$ | $OCH_3$ |
| J-10 | Q-139 ($R_7$=H, $R_8$=H, $R_9$=H) | H | H | $CH_3$ | $OCH_3$ |
| J-10 | Q-140 ($R_7$=H, $R_8$=H, $R_9$=H, $R_3$=$CH_3$) | H | H | $OCH_3$ | $OCH_3$ |
| J-10 | Q-141 ($R_7$=H, $R_8$=H, $R_9$=H, $R_3$=$CH_3$) | H | H | $OCH_3$ | $OCH_3$ |
| J-10 | Q-142 ($R_7$=H, $R_8$=H, $R_9$=H, $R_{10}$=H) | H | H | $OCH_3$ | $OCH_3$ |
| J-10 | Q-143 ($R_7$=H, $R_8$=H, $R_9$=H, $R_3$=$CH_3$) | H | H | $OCH_3$ | $OCH_3$ |
| J-10 | Q-144 ($R_7$=H, $R_8$=H, $R_9$=H, $R_3$=$CH_3$) | H | H | $CH_3$ | $OCH_3$ |
| J-10 | Q-145 ($R_7$=H, $R_8$=H, $R_9$=H) | H | H | $OCH_3$ | $OCH_3$ |
| J-10 | Q-146 ($R_7$=H, $R_8$=H, $R_9$=H) | H | H | $CH_3$ | $OCH_3$ |
| J-10 | Q-147 ($R_7$=H, $R_8$=H, $R_9$=H) | H | H | $OCH_3$ | $OCH_3$ |
| J-10 | Q-148 ($R_7$=H, $R_8$=H, $R_9$=H) | H | H | $OCH_3$ | $OCH_3$ |
| J-10 | Q-149 ($R_7$=H, $R_8$=H, $R_9$=H) | H | H | $OCH_3$ | $OCH_3$ |
| J-10 | Q-150 ($R_7$=H, $R_8$=H, $R_9$=H) | H | H | $OCH_3$ | $OCH_3$ |
| J-10 | Q-151 ($R_7$=H, $R_8$=H, $R_9$=H) | H | H | $CH_3$ | $OCH_3$ |
| J-10 | Q-152 ($R_7$=H, $R_8$=H, $R_9$=H) | H | H | $OCH_3$ | $OCH_3$ |
| J-10 | Q-153 ($R_7$=H, $R_8$=H, $R_9$=H, $R_{11}$=$CH_3$) | H | H | $CH_3$ | $OCH_3$ |
| J-10 | Q-154 ($R_7$=H, $R_8$=H, $R_9$=H, $R_{11}$=$CH_3$) | H | H | $OCH_3$ | $OCH_3$ |
| J-10 | Q-155 ($R_7$=H, $R_8$=H, $R_9$=H) | H | H | $OCH_3$ | $OCH_3$ |
| J-10 | Q-156 ($R_7$=H, $R_8$=H, $R_9$=H) | H | H | $OCH_3$ | $OCH_3$ |

Table 1 (continued)

| J | Q | R | $R_{1c}$ | X | Y |
|---|---|---|---|---|---|
| J-10 | Q-158 ($R_7$=H, $R_8$=H, $R_9$=H) | H | H | $CH_3$ | $OCH_3$ |
| J-10 | Q-159 ($R_7$=H, $R_8$=H, $R_9$=H) | H | H | $OCH_3$ | $OCH_3$ |
| J-10 | Q-160 ($R_7$=H, $R_8$=H, $R_9$=H, $R_{11}$=$CH_3$) | H | H | $CH_3$ | $OCH_3$ |
| J-10 | Q-161 ($R_7$=H, $R_8$=H, $R_9$=H, $R_{11}$=$CH_3$) | H | H | $OCH_3$ | $OCH_3$ |
| J-10 | Q-162 ($R_7$=H, $R_8$=H, $R_9$=H, $R_{10}$=H) | H | H | $OCH_3$ | $OCH_3$ |
| J-8* | Q-1 ($R_7$=H, $R_8$=H) | H | H | $OCH_3$ | $OCH_3$ |
| J-8* | Q-1 ($R_7$=H, $R_8$=H) | H | H | $OCH_3$ | $CH_3$ |
| J-9* | Q-1 ($R_7$=H, $R_8$=H) | H | H | $OCH_3$ | $OCH_3$ |
| J-10* | Q-1 ($R_7$=H, $R_8$=H) | H | H | $OCH_3$ | $OCH_3$ |
| J-9* | Q-167 ($R_7$=H, $R_8$=H, $R_9$=H, $R_{10}$=H) | H | H | $OCH_3$ | $OCH_3$ |
| J-9* | Q-167 ($R_7$=H, $R_8$=H, $R_9$=H, $R_{10}$=H) | H | H | OCH | CH |

Table 1 (continued)

| J | Q | R | $R_{1d}$ | X | Y | m.p.(°C) |
|---|---|---|---|---|---|---|
| J-11 | Q-1 ($R_7$=H, $R_8$=H) | H | H | $CH_3$ | $CH_3$ | 168.5-171 |
| J-11 | Q-1 ($R_7$=H, $R_8$=H) | H | H | $OCH_3$ | $CH_3$ | 165-167 |
| J-11 | Q-1 ($R_7$=H, $R_8$=H) | H | H | $OCH_3$ | $OCH_3$ | 163.5-166.5 |
| J-11 | Q-1 ($R_7$=H, $R_8$=H) | $CH_3$ | H | $OCH_3$ | $OCH_3$ | |
| J-11 | Q-1 ($R_7$=H, $R_8$=H) | $CH_3$ | H | $OCH_3$ | $CH_3$ | |
| J-11 | Q-1*($R_7$=H, $R_8$=H) | H | 5-$CH_3$ | $OCH_3$ | $OCH_3$ | |
| J-11 | Q-1 ($R_7$=H, $R_8$=H) | H | 6-$CH_2CH_3$ | $OCH_3$ | $OCH_3$ | |
| J-11 | Q-1 ($R_7$=H, $R_8$=H) | H | 3-$CH(CH_3)_2$ | $OCH_3$ | $OCH_3$ | |
| J-11 | Q-1 ($R_7$=H, $R_8$=H) | H | 5-$OCH_3$ | $OCH_3$ | $OCH_3$ | |
| J-11 | Q-1 ($R_7$=H, $R_8$=H) | H | 5-$OCH_2CH_3$ | $OCH_3$ | $OCH_3$ | |
| J-11 | Q-1 ($R_7$=H, $R_8$=H) | H | 3-$OCH(CH_3)_2$ | $OCH_3$ | $OCH_3$ | |
| J-11 | Q-1 ($R_7$=H, $R_8$=H) | H | 5-$OCF_2H$ | $OCH_3$ | $OCH_3$ | |
| J-11 | Q-1 ($R_7$=H, $R_8$=H) | H | 6-$OCH_2CH_2Br$ | $OCH_3$ | $OCH_3$ | |
| J-11 | Q-1 ($R_7$=H, $R_8$=H) | H | 5-$OCH(CH_3)(CH_2Cl)$ | $OCH_3$ | $OCH_3$ | |
| J-11 | Q-1 ($R_7$=H, $R_8$=H) | H | 5-$CH_2F$ | $OCH_3$ | $OCH_3$ | |
| J-11 | Q-1 ($R_7$=H, $R_8$=H) | H | 5-$CH_2CH_2Br$ | $OCH_3$ | $OCH_3$ | |
| J-11 | Q-1 ($R_7$=H, $R_8$=H) | H | 5-$CH(CH_3)(CH_2Cl)$ | $OCH_3$ | $OCH_3$ | |
| J-11 | Q-1 ($R_7$=H, $R_8$=H) | H | 5-$SCH_3$ | $OCH_3$ | $OCH_3$ | |
| J-11 | Q-1*($R_7$=H, $R_8$=H) | H | 6-$SCH_2CH_3$ | $OCH_3$ | $OCH_3$ | |
| J-11 | Q-1 ($R_7$=H, $R_8$=H) | H | 5-$SCH(CH_3)_2$ | $OCH_3$ | $OCH_3$ | |
| J-11 | Q-1 ($R_7$=H, $R_8$=H) | H | 5-$SCH_2F$ | $OCH_3$ | $OCH_3$ | |
| J-11 | Q-1 ($R_7$=H, $R_8$=H) | H | 5-$SCH_2CH_2Br$ | $OCH_3$ | $OCH_3$ | |
| J-11 | Q-1 ($R_7$=H, $R_8$=H) | H | 5-$SCH(CH_3)(CH_2Cl)$ | $OCH_3$ | $OCH_3$ | |
| J-11 | Q-1 ($R_7$=H, $R_8$=H) | H | 4-$NH_2$ | $OCH_3$ | $OCH_3$ | |
| J-11 | Q-1 ($R_7$=H, $R_8$=H) | H | 5-$NHCH_3$ | $OCH_3$ | $OCH_3$ | |
| J-11 | Q-1 ($R_7$=H, $R_8$=H) | H | 6-$NHCH_2CH_3$ | $OCH_3$ | $OCH_3$ | |
| J-11 | Q-1 ($R_7$=H, $R_8$=H) | H | 3-$NHCH(CH_3)_2$ | $OCH_3$ | $OCH_3$ | |
| J-11 | Q-1 ($R_7$=H, $R_8$=H) | H | 4-$N(CH_3)_2$ | $OCH_3$ | $OCH_3$ | |
| J-11 | Q-1 ($R_7$=H, $R_8$=H) | H | 6-$N(CH_3)(CH_2CH_3)$ | $OCH_3$ | $OCH_3$ | |
| J-11 | Q-1 ($R_7$=H, $R_8$=H) | H | 5-$N(CH_3)(CH(CH_3)_2)$ | $OCH_3$ | $OCH_3$ | |
| J-11 | Q-1 ($R_7$=H, $R_8$=H) | H | 5-Cl | $OCH_3$ | $OCH_3$ | |
| J-11 | Q-1 ($R_7$=H, $R_8$=H) | H | 5-$CH_2CN$ | $CH_3$ | $OCH_3$ | |

Table 1 (continued)

| J | Q | R | $R_{1d}$ | X | Y | m.p.(°C) |
|---|---|---|---|---|---|---|
| J-11 | Q-1 ($R_7$=H, $R_8$=H) | H | 5-$CH_2OCH_3$ | $OCH_3$ | $OCH_3$ | |
| J-11 | Q-1 ($R_7$=H, $R_8$=H) | H | 5-$CH_2SCH_3$ | Cl | $OCH_3$ | |
| J-11 | Q-1 ($R_7$=H, $R_8$=H) | H | 3-Br | $OCH_3$ | $OCH_3$ | |
| J-11 | Q-1 ($R_7$=H, $R_8$=H) | H | 4-F | $OCH_3$ | $OCH_3$ | |
| J-11 | Q-1 ($R_7$=H, $R_8$=H) | H | 5-I | $OCH_3$ | $OCH_3$ | |
| J-11 | Q-1 ($R_7$=H, $R_8$=H) | H | 4-$NO_2$ | $OCH_3$ | $OCH_3$ | |
| J-11 | Q-1 ($R_7$=H, $R_8$=H) | H | 5-$CF_3$ | $OCH_3$ | $OCH_3$ | |
| J-11 | Q-1 ($R_7$=H, $R_8$=H) | H | 5-$OCF_2H$ | $OCH_3$ | $OCH_3$ | |
| J-11 | Q-1 ($R_7$=H, $R_8$=H) | H | H | $OCH_3$ | $CH_2CH_3$ | |
| J-11 | Q-1 ($R_7$=H, $R_8$=H) | H | H | $OCH_3$ | $CH(CH_3)_2$ | |
| J-11 | Q-1 ($R_7$=H, $R_8$=H) | H | H | $OCH_3$ | $(CH_2)_3CH_3$ | |
| J-11 | Q-1 ($R_7$=H, $R_8$=H) | H | H | $OCH_3$ | $OCH_2CH_3$ | |
| J-11 | Q-1 ($R_7$=H, $R_8$=H) | H | H | $OCH_3$ | $OCH(CH_3)_2$ | |
| J-11 | Q-1 ($R_7$=H, $R_8$=H) | H | H | $OCH_3$ | $O(CH_2)_3CH_3$ | |
| J-11 | Q-1 ($R_7$=H, $R_8$=H) | H | H | $OCF_2H$ | $OCH_3$ | 207.5-209 |
| J-11 | Q-1 ($R_7$=H, $R_8$=H) | H | H | $OCH_2CH_2F$ | $OCH_3$ | |
| J-11 | Q-1 ($R_7$=H, $R_8$=H) | H | H | $OCH_2CHF_2$ | $OCH_3$ | |
| J-11 | Q-1 ($R_7$=H, $R_8$=H) | H | H | $OCH_2CF_3$ | $OCH_3$ | |
| J-11 | Q-1 ($R_7$=H, $R_8$=H) | H | H | $OCH(CH_3)(CH_2Cl)$ | $OCH_3$ | |
| J-11 | Q-1 ($R_7$=H, $R_8$=H) | H | H | $O(CH_2)_3CH_2Br$ | $OCH_3$ | |
| J-11 | Q-1 ($R_7$=H, $R_8$=H) | H | H | $CH_2F$ | $OCH_3$ | |
| J-11 | Q-1 ($R_7$=H, $R_8$=H) | H | H | $CH_2Cl$ | $OCH_3$ | |
| J-11 | Q-1 ($R_7$=H, $R_8$=H) | H | H | $CH_2Br$ | $OCH_3$ | |
| J-11 | Q-1 ($R_7$=H, $R_8$=H) | H | H | $CF_3$ | $OCH_3$ | |
| J-11 | Q-1 ($R_7$=H, $R_8$=H) | H | H | $CH_2CH_2Br$ | $OCH_3$ | |
| J-11 | Q-1 ($R_7$=H, $R_8$=H) | H | H | $CH(CH_3)(CH_2Cl)$ | $OCH_3$ | |
| J-11 | Q-1 ($R_7$=H, $R_8$=H) | H | H | $(CH_2)_3CH_2I$ | $OCH_3$ | |
| J-11 | Q-1 ($R_7$=H, $R_8$=H) | H | H | $SCH_3$ | $OCH_3$ | |
| J-11 | Q-1*($R_7$=H, $R_8$=H) | H | H | $SCH_2CH_3$ | $OCH_3$ | |
| J-11 | Q-1 ($R_7$=H, $R_8$=H) | H | H | $SCH(CH_3)_2$ | $OCH_3$ | |
| J-11 | Q-1 ($R_7$=H, $R_8$=H) | H | H | $S(CH_2)_3CH_3$ | $OCH_3$ | |
| J-11 | Q-1 ($R_7$=H, $R_8$=H) | H | H | $SCHF_2$ | $OCH_3$ | |
| J-11 | Q-1 ($R_7$=H, $R_8$=H) | H | H | $SCH_2CH_2Br$ | $OCH_3$ | |

Table 1 (continued)

| J | Q | R | $R_{1d}$ | X | Y | m.p.(°C) |
|---|---|---|---|---|---|---|
| J-11 | Q-1 ($R_7$=H, $R_8$=H) | H | H | $SCH(CH_3)(CH_2Cl)$ | $OCH_3$ | |
| J-11 | Q-1 ($R_7$=H, $R_8$=H) | H | H | $S(CH_2)_3CH_2F$ | $OCH_3$ | |
| J-11 | Q-1 ($R_7$=H, $R_8$=H) | H | H | Cl | $OCH_3$ | 186.5-187 |
| J-11 | Q-1 ($R_7$=H, $R_8$=H) | H | H | Br | $OCH_3$ | |
| J-11 | Q-1 ($R_7$=H, $R_8$=H) | H | H | F | $OCH_3$ | |
| J-11 | Q-1 ($R_7$=H, $R_8$=H) | H | H | I | $OCH_3$ | |
| J-11 | Q-1*($R_7$=H, $R_8$=H) | H | H | $OCH_3$ | $CH_2OCH_3$ | |
| J-11 | Q-1 ($R_7$=H, $R_8$=H) | H | H | $OCH_3$ | $CH_2CH_2OCH(CH_3)_2$ | |
| J-11 | Q-1 ($R_7$=H, $R_8$=H) | H | H | $OCH_3$ | $CH(CH_3)(CH_2OCH_3)$ | |
| J-11 | Q-1 ($R_7$=H, $R_8$=H) | H | H | $OCH_3$ | $(CH_2)_4CH_2OCH_2CH_3$ | |
| J-11 | Q-1 ($R_7$=H, $R_8$=H) | H | H | $OCH_2O(CH_2)_3CH_3$ | $OCH_3$ | |
| J-11 | Q-1 ($R_7$=H, $R_8$=H) | H | H | $OCH_2CH_2OCH(CH_3)_2$ | $OCH_3$ | |
| J-11 | Q-1 ($R_7$=H, $R_8$=H) | H | H | $OCH(CH_3)(CH_2OCH_3)$ | $OCH_3$ | |
| J-11 | Q-1 ($R_7$=H, $R_8$=H) | H | H | $O(CH_2)_4CH_2OCH_2CH_3$ | $OCH_3$ | |
| J-11 | Q-1 ($R_7$=H, $R_8$=H) | H | H | $OCH_3$ | $NH_2$ | |
| J-11 | Q-1 ($R_7$=H, $R_8$=H) | H | H | $OCH_3$ | $NHCH_3$ | |
| J-11 | Q-1 ($R_7$=H, $R_8$=H) | H | H | $OCH_3$ | $NHCH_2CH_3$ | |
| J-11 | Q-1 ($R_7$=H, $R_8$=H) | H | H | $OCH_3$ | $NHCH(CH_3)_2$ | |
| J-11 | Q-1 ($R_7$=H, $R_8$=H) | H | H | $OCH_3$ | $N(CH_3)_2$ | |
| J-11 | Q-1 ($R_7$=H, $R_8$=H) | H | H | $OCH_3$ | $N(CH_3)(CH_2CH_3)$ | |
| J-11 | Q-1 ($R_7$=H, $R_8$=H) | H | H | $OCH_3$ | $N(CH_3)(CH(CH_3)_2)$ | |
| J-11 | Q-1 ($R_7$=H, $R_8$=H) | H | H | $OCH_3$ | $(CH_2)_4CH_2OCH_2CH_3$ | |
| J-11 | Q-1 ($R_7$=H, $R_8$=H) | H | H | H | $OCH_3$ | |
| J-11 | Q-1 ($R_7$=H, $R_8$=H) | H | H | $OCH_3$ | $OCH_2CH{=}CH_2$ | |
| J-11 | Q-1 ($R_7$=H, $R_8$=H) | H | H | $OCH_3$ | $OCH_2C(CH_3){=}CH_2$ | |
| J-11 | Q-1 ($R_7$=H, $R_8$=H) | H | H | $OCH_3$ | $OCH_2C{\equiv}CH$ | |
| J-11 | Q-1*($R_7$=H, $R_8$=H) | H | H | $OCH_3$ | $OCH_2C{\equiv}CCH_3$ | |
| J-11 | Q-1 ($R_7$=H, $R_8$=H) | H | H | $OCH_3$ | $CH_2S(CH_2)_3CH_3$ | |
| J-11 | Q-1 ($R_7$=H, $R_8$=H) | H | H | $OCH_3$ | $CH_2CH_2SCH(CH_3)_2$ | |
| J-11 | Q-1 ($R_7$=H, $R_8$=H) | H | H | $OCH_3$ | $CH(CH_3)(CH_2SCH_3)$ | |
| J-11 | Q-1 ($R_7$=H, $R_8$=H) | H | H | $OCH_3$ | $(CH_2)_4CH_2SCH_2CH_3$ | |
| J-11 | Q-1 ($R_7$=H, $R_8$=H) | H | H | $OCH_3$ | cyclopropyl | |
| J-11 | Q-1 ($R_7$=H, $R_8$=H) | H | H | $OCH_3$ | 2-methylcyclopropyl | |

Table 1 (continued)

| J | Q | R | $R_{1d}$ | X | Y | m.p.(°C) |
|---|---|---|---|---|---|---|
| J-11 | Q-1 ($R_7$=H, $R_8$=H) | H | H | $OCH_3$ | cyclopentyl | |
| J-11 | Q-1 ($R_7$=H, $R_8$=H) | H | H | $OCH_3$ | C≡CH | |
| J-11 | Q-1 ($R_7$=H, $R_8$=H) | H | H | $OCH_3$ | $C{\equiv}CCH_3$ | |
| J-11 | Q-1 ($R_7$=H, $R_8$=H) | H | H | $OCH_3$ | -CHO | |
| J-11 | Q-1 ($R_7$=H, $R_8$=H) | H | H | $OCH_3$ | $-COCH_3$ | |
| J-11 | Q-1*($R_7$=H, $R_8$=H) | H | H | $OCH_3$ | $-CH(OCH_3)_2$ | |
| J-11 | Q-1 ($R_7$=H, $R_8$=H) | H | H | $OCH_3$ | $-CH(SCH_3)(OCH_2CH_3)$ | |
| J-11 | Q-1 ($R_7$=H, $R_8$=H) | H | H | $OCH_3$ | $-C(CH_3)(SCH_3)_2$ | |
| J-11 | Q-1 ($R_7$=H, $R_8$=H) | H | H | $OCH_3$ | $-CH(SCH_2CH_3)_2$ | |
| J-11 | Q-1 ($R_7$=H, $R_8$=H) | H | H | $OCH_3$ | 1,3-dioxolan-2-yl | |
| J-11 | Q-1 ($R_7$=H, $R_8$=H) | H | H | $OCH_3$ | 2-methyl-1,3-oxa-thiolan-2-yl | |
| J-11 | Q-1 ($R_7$=H, $R_8$=H) | H | H | $OCH_3$ | 1,3-oxathian-2-yl | |
| J-11 | Q-1 ($R_7$=H, $R_8$=H) | H | H | $OCH_3$ | 2-methyl-1,3-dithian-2-yl | |
| J-11 | Q-1 ($R_7$=H, $R_8$=H) | H | H | $OCH_3$ | 4-methyl-1,3-dioxolan-2-yl | |
| J-11 | Q-1 ($R_7$=H, $R_8$=H) | H | H | $OCH_3$ | 4-methyl-1,3-oxathiolan-2-yl | |
| J-11 | Q-1 ($R_7$=H, $R_8$=H) | H | H | $OCH_3$ | 2,4-dimethyl-1,3-dithiolan-2-yl | |
| J-11 | Q-1*($R_7$=H, $R_8$=H) | H | H | $OCH_3$ | $N(OCH_3)(CH_3)_2$ | |
| J-11 | Q-1 ($R_7$=H, $R_8$=H) | H | $6-SO_2N(CH_3)_2$ | $OCH_3$ | $OCH_3$ | |
| J-11 | Q-1 ($R_7$=H, $R_8$=H) | H | $6-SO_2CH_3$ | $OCH_3$ | $OCH_3$ | |
| J-11 | Q-1 ($R_7$=H, $R_8$=H) | H | $6-SO_2CH(CH_3)_2$ | $OCH_3$ | $OCH_3$ | |
| J-11 | Q-1 ($R_7$=H, $R_8$=H) | H | $6-CO_2CH_3$ | $OCH_3$ | $OCH_3$ | |
| J-11 | Q-1 ($R_7$=H, $R_8$=$CH_3$) | H | H | $OCH_3$ | $OCH_3$ | 182-184 |
| J-11 | Q-1 ($R_7$=H, $R_8$=$CH_3$) | H | H | $CH_3$ | $CH_3$ | 191-192.5 |
| J-11 | Q-1 ($R_7$=H, $R_8$=$CH_3$) | H | H | $CH_3$ | $OCH_3$ | 188-188.5 |
| J-11 | Q-1 ($R_7$=H, $R_8$=$CH_2CH_3$) | H | H | $OCH_3$ | $OCH_3$ | |
| J-11 | Q-1 ($R_7$=H, $R_8$=$CH(CH_3)_2$) | H | H | $OCH_3$ | $OCH_3$ | |
| J-11 | Q-1 ($R_7$=H, $R_8$=$(CH_2)_3CH_3$) | H | H | $OCH_3$ | $OCH_3$ | |

Table 1 (continued)

| J | Q | R | $R_{1d}$ | X | Y | m.p. (°C) |
|---|---|---|---|---|---|---|
| J-11 | Q-1 ($R_7$=$CH_3$, $R_8$=$CH_3$) | H | H | $OCH_3$ | $OCH_3$ | |
| J-11 | Q-2 ($R_7$=H, $R_8$=H) | H | H | $OCH_3$ | $OCH_3$ | |
| J-11 | Q-2 ($R_7$=H, $R_8$=H) | $CH_3$ | H | $OCH_3$ | $OCH_3$ | |
| J-11 | Q-2 ($R_7$=H, $R_8$=$CH_3$) | H | 5-$OCH_3$ | $OCH_3$ | $OCH_3$ | |
| J-11 | Q-3 ($R_7$=H, $R_8$=H) | H | H | $OCH_3$ | $OCH_3$ | |
| J-11 | Q-3 ($R_7$=$C_2H_5$, $R_8$=H) | H | H | $OCF_2H$ | $CH_3$ | |
| J-11 | Q-3 ($R_7$=$CH_3$, $R_8$=H) | H | 5-Cl | $OCH_3$ | $CH_3$ | |
| J-11 | Q-4 ($R_7$=H, $R_8$=H, $R_3$=$CH_3$) | H | H | $CH_3$ | $OCH_3$ | |
| J-11 | Q-4 ($R_7$=H, $R_8$=H, $R_3$=$C_2H_5$) | H | H | $CH_3$ | $OCH_3$ | |
| J-11 | Q-4 ($R_7$=H, $R_8$=H, $R_3$=i-$C_3H_7$) | H | 5-$CH_3$ | $OCH_3$ | $OCH_3$ | |
| J-11 | Q-5 ($R_7$-H, $R_8$=H, $R_3$=H) | H | H | $OCH_3$ | $OCH_3$ | |
| J-11 | Q-5 ($R_7$=H, $R_8$=H, $R_3$=$CH_3$) | H | H | Cl | $OCH_3$ | |
| J-11 | Q-5 ($R_7$=H, $R_8$=$CH_3$, $R_3$=$CH_3$) | H | H | $CH_3$ | $OCH_3$ | |
| J-11 | Q-6 ($R_7$=$CH_3$, $R_8$=H, $R_3$=$CH_3$) | H | H | $CH_3$ | $OCH_3$ | |
| J-11 | Q-6 ($R_7$-H, $R_8$=H, $R_3$=$C_2H_5$) | $CH_3$ | H | $CH_3$ | $OCH_3$ | |
| J-11 | Q-6 ($R_7$=$C_2H_5$, $R_8$=H, $R_3$=H) | H | H | $OCH_3$ | $OCH_3$ | |
| J-11 | Q-8 ($R_7$=H, $R_8$=H, $R_2$=H) | H | H | $OCH_3$ | $OCH_3$ | |
| J-11 | Q-8 ($R_7$=$CH_3$, $R_8$=H, $R_2$=$OCH_3$) | H | H | $OCH_3$ | $OCF_2H$ | |
| J-11 | Q-8 ($R_7$=H, $R_8$=H, $R_2$=$OCH(CH_3)CH_2CH_3$) | H | 5-Cl | $OCH_3$ | $OCH_3$ | |
| J-11 | Q-9 ($R_7$=H, $R_8$=H, $R_2$=H) | H | H | $OCH_3$ | $OCH_3$ | |
| J-11 | Q-9 ($R_7$=$CH_3$, $R_8$=H, $R_2$=$CH_3$) | $CH_3$ | H | $OCH_3$ | $OCH_3$ | |
| J-11 | Q-9 ($R_7$=H, $R_8$=H, $R_2$=Cl) | H | 5-$OCH_3$ | $OCH_3$ | $OCH_3$ | |
| J-11 | Q-10 ($R_7$=H, $R_8$=H) | H | H | $OCH_3$ | $OCH_3$ | |
| J-11 | Q-10 ($R_7$=$CH_3$, $R_8$=H) | H | H | $OCF_2H$ | $CH_3$ | |
| J-11 | Q-10 ($R_7$=H, $R_8$=$CH_3$) | H | 5-Cl | $OCH_3$ | $CH_3$ | |
| J-11 | Q-11 ($R_7$=H, $R_8$=H) | H | H | $CH_3$ | $OCH_3$ | |
| J-11 | Q-11 ($R_7$=n-$C_4H_9$, $R_8$=H) | H | H | $CH_3$ | $OCH_3$ | |
| J-11 | Q-11 ($R_7$=$CH_3$, $R_8$=$CH_3$) | H | 5-$CH_3$ | $OCH_3$ | $OCH_3$ | |
| J-11 | Q-12 ($R_7$=H, $R_8$=H) | H | H | $OCH_3$ | $OCH_3$ | |

Table 1 (continued)

| J | Q | R | $R_{1d}$ | X | Y | m.p. (°C) |
|---|---|---|---|---|---|---|
| J-11 | Q-12 ($R_7$=$CH_3$, $R_8$=H) | H | H | Cl | $OCH_3$ | |
| J-11 | Q-12 ($R_7$=H, $R_8$=$C_2H_5$) | H | H | $CH_3$ | $OCH_3$ | |
| J-11 | Q-13 ($R_7$=H, $R_8$=H, $R_3$=H) | H | H | $CH_3$ | $OCH_3$ | |
| J-11 | Q-13 ($R_7$=H, $R_8$=H, $R_3$=$CH_3$) | $CH_3$ | H | $CH_3$ | $OCH_3$ | |
| J-11 | Q-13 ($R_7$=$CH_3$, $R_8$=H, $R_3$=$CH_3$) | H | H | $OCH_3$ | $OCH_3$ | |
| J-11 | Q-14 ($R_7$=H, $R_8$=H, $R_3$=$CH_3$) | H | H | $OCH_3$ | $OCH_3$ | |
| J-11 | Q-14 ($R_7$=$CH_3$, $R_8$=H, $R_3$=$C_2H_5$) | H | H | $OCH_3$ | $OCH_3$ | |
| J-11 | Q-14 ($R_7$=H, $R_8$=$CH_3$, $R_3$=$CH_3$) | H | 5-$OCH_3$ | $OCH_3$ | $OCH_3$ | |
| J-11 | Q-15 ($R_7$=H, $R_8$=H, $R_3$=H) | H | H | $OCH_3$ | $OCH_3$ | |
| J-11 | Q-15 ($R_7$=$C_2H_5$, $R_8$=H, $R_3$=$CH_3$) | H | H | $OCH_3$ | $OCF_2H$ | |
| J-11 | Q-15 ($R_7$=$CH_3$, $R_8$=$CH_3$, $R_3$=$CH_3$) | H | 5-Cl | $OCH_3$ | $OCH_3$ | |
| J-11 | Q-17 ($R_7$=H, $R_8$=H, $R_2$=H) | H | H | $OCH_3$ | $OCH_3$ | |
| J-11 | Q-17 ($R_7$=$CH_3$, $R_8$=H, $R_2$=$CH_3$) | H | H | $OCF_2H$ | $CH_3$ | |
| J-11 | Q-17 ($R_7$=H, $R_8$=H, $R_2$=$C_2H_5$) | H | 5-Cl | $OCH_3$ | $CH_3$ | |
| J-11 | Q-18 ($R_7$=H, $R_8$=H, $R_2$=H) | H | H | $CH_3$ | $OCH_3$ | |
| J-11 | Q-18 ($R_7$=H, $R_8$=$OC_2H_5$, $R_2$=H) | H | H | $CH_3$ | $OCH_3$ | |
| J-11 | Q-18 ($R_7$=$CH_3$, $R_8$=$OCH(CH_3)_2$, $R_2$=$CH_3$) | H | 5-$CH_3$ | $OCH_3$ | $OCH_3$ | |
| J-11 | Q-19 ($R_7$=H, $R_8$=H) | H | H | $OCH_3$ | $OCH_3$ | |
| J-11 | Q-19 ($R_7$=$CH_3$, $R_8$=H) | H | H | Cl | $OCH_3$ | |
| J-11 | Q-19 ($R_7$=$CH_3$, $R_8$=$CH_3$) | H | H | $CH_3$ | $OCH_3$ | |
| J-11 | Q-20 ($R_7$=H, $R_8$=H) | H | H | $CH_3$ | $OCH_3$ | |
| J-11 | Q-20 ($R_7$=$CH_3$, $R_8$=H) | $CH_3$ | H | $CH_3$ | $OCH_3$ | |
| J-11 | Q-20 ($R_7$=H, $R_8$=$CH_3$) | H | H | $OCH_3$ | $OCH_3$ | |
| J-11 | Q-21 ($R_7$=H, $R_8$=H) | H | H | $OCH_3$ | $OCH_3$ | |
| J-11 | Q-21 ($R_7$=$CH_3$, $R_8$=H) | H | H | $OCH_3$ | $OCH_3$ | |
| J-11 | Q-21 ($R_7$=$CH_3$, $R_8$=$CH_3$) | H | 5-$OCH_3$ | $OCH_3$ | $OCH_3$ | |
| J-11 | Q-22 ($R_7$=H, $R_8$=H, $R_3$=H) | H | H | $OCH_3$ | $OCH_3$ | |
| J-11 | Q-22 ($R_7$=H, $R_8$=H, $R_3$=$CH_3$) | H | H | $OCH_3$ | $OCF_2H$ | |
| J-11 | Q-22 ($R_7$=$CH_3$, $R_8$=$CH_3$, $R_3$=$C_2H_5$) | H | 5-Cl | $OCH_3$ | $OCH_3$ | |

Table 1 (continued)

| J | Q | R | $R_{1d}$ | X | Y | m.p. (°C) |
|---|---|---|---|---|---|---|
| J-11 | Q-23 ($R_7$=H, $R_8$=H, $R_3$=H) | H | H | $OCH_3$ | $OCH_3$ | |
| J-11 | Q-23 ($R_7$=H, $R_8$=H, $R_3$=$CH_3$) | $CH_3$ | H | $OCH_3$ | $OCH_3$ | |
| J-11 | Q-23 ($R_7$=$CH_3$, $R_8$=H, $R_3$=n-$C_4H_9$) | H | 5-$OCH_3$ | $OCH_3$ | $OCH_3$ | |
| J-11 | Q-24 ($R_7$=H, $R_8$=H, $R_3$=$CH_3$) | H | H | $OCH_3$ | $OCH_3$ | |
| J-11 | Q-24 ($R_7$=$CH_3$, $R_8$=$CH_3$, $R_3$=$C_2H_5$) | H | H | $OCF_2H$ | $CH_3$ | |
| J-11 | Q-24 ($R_7$=$CH_3$, $R_8$=$CH_3$, $R_3$=i-$C_3H_7$) | H | 5-Cl | $OCH_3$ | $CH_3$ | |
| J-11 | Q-25 ($R_7$=H, $R_8$=H, $R_9$=H) | H | H | $CH_3$ | $OCH_3$ | |
| J-11 | Q-25 ($R_7$=$CH_3$, $R_8$=H, $R_9$=H) | H | H | $CH_3$ | $OCH_3$ | |
| J-11 | Q-25 ($R_7$=H, $R_8$=H, $R_9$=$C_2H_5$) | H | 5-$CH_3$ | $OCH_3$ | $OCH_3$ | |
| J-11 | Q-26 ($R_7$=H, $R_8$=H) | H | H | $OCH_3$ | $OCH_3$ | |
| J-11 | Q-26 ($R_7$=$CH_3$, $R_8$=H) | H | H | Cl | $OCH_3$ | |
| J-11 | Q-26 ($R_7$=$CH_3$, $R_8$=$CH_3$) | H | H | $CH_3$ | $OCH_3$ | |
| J-11 | Q-27 ($R_7$=H, $R_8$=H) | H | H | $CH_3$ | $OCH_3$ | |
| J-11 | Q-27 ($R_7$=$CH_3$, $R_8$=H) | $CH_3$ | H | $CH_3$ | $OCH_3$ | |
| J-11 | Q-27 ($R_7$=H, $R_8$=$CH_3$) | H | H | $OCH_3$ | $OCH_3$ | |
| J-11 | Q-28*($R_7$=H, $R_8$=H) | H | H | $OCH_3$ | $OCH_3$ | |
| J-11 | Q-28 ($R_7$=$CH_3$, $R_8$=H) | H | H | $OCH_3$ | $OCH_3$ | |
| J-11 | Q-28 ($R_7$=$CH_3$, $R_8$=$CH_3$) | H | 5-$OCH_3$ | $OCH_3$ | $OCH_3$ | |
| J-11 | Q-29 ($R_7$=H, $R_8$=H) | H | H | $OCH_3$ | $OCH_3$ | |
| J-11 | Q-29 ($R_7$=$CH_3$, $R_8$=H) | H | H | $OCH_3$ | $OCF_2H$ | |
| J-11 | Q-29 ($R_7$=H, $R_8$=$CH_3$) | H | 5-Cl | $OCH_3$ | $OCH_3$ | |
| J-11 | Q-30 ($R_7$=H, $R_8$=H) | H | H | $OCH_3$ | $OCH_3$ | |
| J-11 | Q-30 ($R_7$=$CH_3$, $R_8$=$CH_3$) | $CH_3$ | H | $OCH_3$ | $CH_3$ | |
| J-11 | Q-30 ($R_7$=$C_2H_5$, $R_8$=H) | H | 5-$OCH_3$ | $OCH_3$ | $OCH_3$ | |
| J-11 | Q-31 ($R_7$=H, $R_8$=H) | H | H | $OCH_3$ | $OCH_3$ | |
| J-11 | Q-31 ($R_7$=i-$C_3H_7$, $R_8$=H) | H | H | $OCF_2H$ | $CH_3$ | |
| J-11 | Q-31 ($R_7$=H, $R_8$=n-$C_4H_9$) | H | 5-Cl | $OCH_3$ | $CH_3$ | |
| J-11 | Q-32 ($R_7$=H, $R_8$=H, $R_3$=H) | H | H | $CH_3$ | $OCH_3$ | |
| J-11 | Q-32 ($R_7$=$CH_3$, $R_8$=H, $R_3$=$CH_3$) | H | H | $CH_3$ | $OCH_3$ | |
| J-11 | Q-32 ($R_7$=H, $R_8$=$CH_3$, $R_3$=$C_2H_5$) | H | 5-$CH_3$ | $OCH_3$ | $OCH_3$ | |
| J-11 | Q-33 ($R_7$=H, $R_8$=H, $R_8$=$CH_3$) | H | H | $OCH_3$ | $OCH_3$ | |
| J-11 | Q-33 ($R_7$=$CH_3$, $R_8$=$CH_3$, $R_3$=i-$C_3H_7$) | H | H | Cl | $OCH_3$ | |

Table 1 (continued)

| J | Q | R | $R_{1d}$ | X | Y | m.p.(°C) |
|---|---|---|---|---|---|---|
| J-11 | Q-33 ($R_7=C_2H_5$, $R_8$=H, $R_3$=n-$C_4H_9$) | H | H | $CH_3$ | $OCH_3$ | |
| J-11 | Q-34 ($R_7$=H, $R_8$=H, $R_3$=H) | H | H | $CH_3$ | $OCH_3$ | |
| J-11 | Q-34 ($R_7$=i-$C_3H_7$, $R_8$=H, $R_3=CH_3$) | $CH_3$ | H | $CH_3$ | $OCH_3$ | |
| J-11 | Q-34 ($R_7$=H, $R_8$=n-$C_4H_9$, $R_3=CH_3$) | H | H | $OCH_3$ | $OCH_3$ | |
| J-11 | Q-36 ($R_7$=H, $R_8$=H, $R_2$=H) | H | H | $OCH_3$ | $OCH_3$ | |
| J-11 | Q-36 ($R_7=CH_3$, $R_8$=H, $R_2=CH_3$) | H | H | $OCH_3$ | $OCF_2H$ | |
| J-11 | Q-36 ($R_7$=H, $R_8$=H, $R_2=C_2H_5$) | H | 5-Cl | $OCH_3$ | $OCH_3$ | |
| J-11 | Q-37 ($R_7$=H, $R_8$=H, $R_2$=H) | H | H | $OCH_3$ | $OCH_3$ | 197-202 |
| J-11 | Q-37 ($R_7$=H, $R_8$=H, $R_2$=H) | H | H | $OCH_3$ | $CH_3$ | 226-227 |
| J-11 | Q-37 ($R_7$=H, $R_8$=H, $R_2$=H) | H | H | $OCH_3$ | Cl | 200-205 |
| J-11 | Q-37 ($R_7$=H, $R_8$=H, $R_2$=H) | H | H | $CH_3$ | $CH_3$ | 210 |
| J-11 | Q-37 ($R_7$=H, $R_8$=H, $R_2$=i-$C_3H_7$) | $CH_3$ | H | $OCH_3$ | $OCH_3$ | |
| J-11 | Q-37 ($R_7$=H, $R_8$=H, $R_2$=n-$C_4H_9$) | H | 5-$OCH_3$ | $OCH_3$ | $OCH_3$ | |
| J-11 | Q-38 ($R_7$=H, $R_8$=H, $R_2=OCF_2H$) | H | H | $OCH_3$ | $OCH_3$ | |
| J-11 | Q-38 ($R_7=CH_3$, $R_8$=H, $R_2=OCH_2CH_2F$) | H | H | $OCF_2H$ | $CH_3$ | |
| J-11 | Q-38 ($R_7$=H, $R_8$=H, $R_2$=Cl) | H | 5-Cl | $OCH_3$ | $CH_3$ | |
| J-11 | Q-39 ($R_7$=H, $R_8$=H, $R_9$=H) | H | H | $CH_3$ | $OCH_3$ | |
| J-11 | Q-39 ($R_7=C_2H_5$, $R_8$=H, $R_9$=H) | H | H | $CH_3$ | $OCH_3$ | |
| J-11 | Q-39 ($R_7$=H, $R_8$=H, $R_9=CH_3$) | H | 5-$CH_3$ | $OCH_3$ | $OCH_3$ | |
| J-11 | Q-40 ($R_7$=H, $R_8$=H) | H | H | $OCH_3$ | $OCH_3$ | |
| J-11 | Q-40 ($R_7=CH_3$, $R_8$=H) | H | H | Cl | $OCH_3$ | |
| J-11 | Q-40 ($R_7$=H, $R_8=CH_3$) | H | H | $CH_3$ | $OCH_3$ | |
| J-11 | Q-41 ($R_7$=H, $R_8$=H) | H | H | $CH_3$ | $OCH_3$ | |
| J-11 | Q-41 ($R_7=CH_3$, $R_8=CH_3$) | $CH_3$ | H | $CH_3$ | $OCH_3$ | |
| J-11 | Q-41 ($R_7$=H, $R_8=C_2H_5$) | H | H | $OCH_3$ | $OCH_3$ | |
| J-11 | Q-42 ($R_7$=H, $R_8$=H) | H | H | $OCH_3$ | $OCH_3$ | |
| J-11 | Q-42 ($R_7=CH_3$, $R_8=CH_3$) | H | H | $OCH_3$ | $OCH_3$ | |
| J-11 | Q-42 ($R_7=C_2H_5$, $R_8=C_2H_5$) | H | 5-$OCH_3$ | $OCH_3$ | $OCH_3$ | |
| J-11 | Q-43 ($R_7$=H, $R_8$=H, $R_3$=H) | H | H | $OCH_3$ | $OCH_3$ | |

Table 1 (continued)

| J | Q | R | $R_{1d}$ | X | Y | m.p. (°C) |
|---|---|---|---|---|---|---|
| J-11 | Q-43 ($R_7$=H, $R_8$=H, $R_3$=$CH_3$) | H | H | $OCH_3$ | $OCF_2H$ | |
| J-11 | Q-43 ($R_7$=$CH_3$, $R_8$=H, $R_3$=$CH_3$) | H | 5-Cl | $OCH_3$ | $OCH_3$ | |
| J-11 | Q-44 ($R_7$=H, $R_8$=H, $R_3$=$CH_3$) | H | H | $OCH_3$ | $OCH_3$ | |
| J-11 | Q-44 ($R_7$=$CH_3$, $R_8$=H, $R_3$=$C_2H_5$) | $CH_3$ | H | $OCH_3$ | $CH_3$ | |
| J-11 | Q-44 ($R_7$=H, $R_8$=$CH_3$, $R_3$=$CH_3$) | H | 5-$OCH_3$ | $OCH_3$ | $OCH_3$ | |
| J-11 | Q-45 ($R_7$=H, $R_8$=H, $R_3$=H) | H | H | $OCH_3$ | $OCH_3$ | |
| J-11 | Q-45 ($R_7$=$C_2H_5$, $R_8$=H, $R_3$=$CH_3$) | H | H | $OCF_2H$ | $CH_3$ | |
| J-11 | Q-45 ($R_7$=$CH_3$, $R_8$=$CH_3$, $R_3$=$CH_3$) | H | 5-Cl | $OCH_3$ | $CH_3$ | |
| J-11 | Q-47 ($R_7$=H, $R_8$=H, $R_2$=H) | H | H | $OCH_3$ | $OCH_3$ | |
| J-11 | Q-47 ($R_7$=H, $R_8$=$CH_3$, $R_2$=$CH_3$) | H | H | Cl | $OCH_3$ | |
| J-11 | Q-47 ($R_7$=H, $R_8$=H, $R_2$=$C_2H_5$) | H | H | $CH_3$ | $OCH_3$ | |
| J-11 | Q-48 ($R_7$=H, $R_8$=H, $R_2$=H) | H | H | $CH_3$ | $OCH_3$ | |
| J-11 | Q-48 ($R_7$=$CH_3$, $R_8$=$CH_3$, $R_2$=$CH_3$) | $CH_3$ | H | $CH_3$ | $OCH_3$ | |
| J-11 | Q-48 ($R_7$=H, $R_8$=$C_2H_5$, $R_2$=$C_2H_5$) | H | H | $OCH_3$ | $OCH_3$ | |
| J-11 | Q-49*($R_7$=H, $R_8$=H, $R_2$=H) | H | H | $OCH_3$ | $OCH_3$ | |
| J-11 | Q-49 ($R_7$=$CH_3$, $R_8$=$CH_3$, $R_2$=H) | H | H | $OCH_3$ | $OCH_3$ | |
| J-11 | Q-49 ($R_7$=$CH_3$, $R_8$=$CH_3$, $R_2$=$CH_3$) | H | 5-$OCH_3$ | $OCH_3$ | $OCH_3$ | |
| J-11 | Q-50 ($R_7$=H, $R_8$=H, $R_2$=H) | H | H | $OCH_3$ | $OCH_3$ | |
| J-11 | Q-50 ($R_7$=$CH_3$, $R_8$=$CH_3$, $R_2$=H) | H | H | $OCH_3$ | $OCF_2H$ | |
| J-11 | Q-50 ($R_7$=$CH_3$, $R_8$=$CH_3$, $R_2$=$CH_3$) | H | 5-Cl | $OCH_3$ | $OCH_3$ | |
| J-11 | Q-51 ($R_7$=H, $R_8$=H, $R_9$=H) | H | H | $OCH_3$ | $OCH_3$ | |
| J-11 | Q-51 ($R_7$=H, $R_8$=H, $R_9$=$CH_3$) | $CH_3$ | H | $OCH_3$ | $OCH_3$ | |
| J-11 | Q-51 ($R_7$=H, $R_8$=H, $R_9$=s-$C_4H_9$) | H | 5-$OCH_3$ | $OCH_3$ | $OCH_3$ | |
| J-11 | Q-52 ($R_7$=H, $R_8$=H, $R_9$=H) | H | H | $OCH_3$ | $OCH_3$ | |
| J-11 | Q-52 ($R_7$=$CH_3$, $R_8$=H, $R_9$=H) | H | H | $OCF_2H$ | $CH_3$ | |
| J-11 | Q-52 ($R_7$=H, $R_8$=H, $R_9$=$CH_3$) | H | 5-Cl | $OCH_3$ | $CH_3$ | |
| J-11 | Q-53 ($R_7$=H, $R_8$=H, $R_9$=H) | H | H | $CH_3$ | $OCH_3$ | |
| J-11 | Q-53 ($R_7$=$CH_3$, $R_8$=$CH_3$, $R_9$=$CH_3$) | H | H | $CH_3$ | $OCH_3$ | |
| J-11 | Q-53 ($R_7$=H, $R_8$=H, $R_9$=$CH_3$) | H | 5-$CH_3$ | $OCH_3$ | $OCH_3$ | |

Table 1 (continued)

| J | Q | R | $R_{1d}$ | X | Y | m.p.(°C) |
|---|---|---|---|---|---|---|
| J-11 | Q-54 ($R_7$=H, $R_8$=H, $R_9$=H) | H | H | $OCH_3$ | $OCH_3$ | |
| J-11 | Q-54 ($R_7$=$CH_3$, $R_8$=H, $R_9$=H) | H | H | Cl | $OCH_3$ | |
| J-11 | Q-54 ($R_7$=H, $R_8$=H, $R_9$=i-$C_3H_7$) | H | H | $CH_3$ | $OCH_3$ | |
| J-11 | Q-55 ($R_7$=H, $R_8$=H, $R_9$=H, $R_3$=H) | H | H | $CH_3$ | $OCH_3$ | |
| J-11 | Q-55 ($R_7$=H, $R_8$=H, $R_9$=$CH_3$, $R_3$=$CH_3$) | $CH_3$ | H | $CH_3$ | $OCH_3$ | |
| J-11 | Q-55 ($R_7$=H, $R_8$=H, $R_9$=s-$C_4H_9$, $R_3$=H) | H | H | $OCH_3$ | $OCH_3$ | |
| J-11 | Q-56 ($R_7$=H, $R_8$=H, $R_9$=H, $R_3$=$C_2H_5$) | H | H | $OCH_3$ | $OCH_3$ | |
| J-11 | Q-56 ($R_7$=$CH_3$, $R_8$=H, $R_9$=H, $R_3$=H) | H | H | $OCH_3$ | $OCH_3$ | |
| J-11 | Q-56 ($R_7$=H, $R_8$=H, $R_9$=$CH_3$, $R_3$=$CH_3$) | H | 5-$OCH_3$ | $OCH_3$ | $OCH_3$ | |
| J-11 | Q-57 ($R_7$=H, $R_8$=H, $R_9$=H, $R_3$=i-$C_3H_7$) | H | H | $OCH_3$ | $OCH_3$ | |
| J-11 | Q-57 ($R_7$=$CH_3$, $R_8$=$CH_3$, $R_9$=$CH_3$, $R_3$=H) | H | H | $OCH_3$ | $OCF_2H$ | |
| J-11 | Q-57 ($R_7$=H, $R_8$=H, $R_9$=$CH_3$, $R_3$=$CH_3$) | H | 5-Cl | $OCH_3$ | $OCH_3$ | |
| J-11 | Q-58 ($R_7$=H, $R_8$=H, $R_9$=H, $R_3$=H) | H | H | $OCH_3$ | $OCH_3$ | |
| J-11 | Q-58 ($R_7$=$CH_3$, $R_8$=H, $R_9$=H, $R_3$=$CH_3$) | $CH_3$ | H | $OCH_3$ | $CH_3$ | |
| J-11 | Q-58 ($R_7$=H, $R_8$=H, $R_9$=i-$C_3H_7$, $R_3$=$CH_3$) | H | 5-$OCH_3$ | $OCH_3$ | $OCH_3$ | |
| J-11 | Q-60 ($R_7$=H, $R_8$=H, $R_9$=H, $R_2$=H) | H | H | $CH_3$ | $OCH_3$ | |
| J-11 | Q-60 ($R_7$=$CH_3$, $R_8$=H, $R_9$=H, $R_2$=$O(CH_2)_3CH_2Br$) | H | H | $CH_3$ | $OCH_3$ | |
| J-11 | Q-60 ($R_7$=H, $R_8$=H, $R_9$=H, $R_2$=Br) | H | 5-$CH_3$ | $OCH_3$ | $OCH_3$ | |
| J-11 | Q-61 ($R_7$=H, $R_8$=H, $R_9$=H, $R_2$=H) | H | H | $OCH_3$ | $OCH_3$ | |
| J-11 | Q-61 ($R_7$=$CH_3$, $R_8$=H, $R_9$=H, $R_2$=$OCH_2F$) | H | H | Cl | $OCH_3$ | |
| J-11 | Q-61 ($R_7$=H, $R_8$=$CH_3$, $R_9$=H, $R_2$=$O(CH_2)_3CH_3$) | H | H | $CH_3$ | $OCH_3$ | |
| J-11 | Q-62 ($R_7$=H, $R_8$=H, $R_9$=H, $R_2$=H) | H | H | $OCH_3$ | $OCH_3$ | 142-145 |
| J-11 | Q-62 ($R_7$=$CH_3$, $R_8$=H, $R_9$=H, $R_2$=$CH_3$) | $CH_3$ | H | $CH_3$ | $OCH_3$ | |
| J-11 | Q-62 ($R_7$=Cl, $R_8$=H, $R_9$=H, $R_2$=H) | H | H | $OCH_3$ | $OCH_3$ | |
| J-11 | Q-63 ($R_7$=H, $R_8$=H, $R_9$=H) | H | H | $OCH_3$ | $OCH_3$ | |
| J-11 | Q-63 ($R_7$=$CH_3$, $R_8$=H, $R_9$=H) | H | H | $OCH_3$ | $OCH_3$ | |
| J-11 | Q-63 ($R_7$=H, $R_8$=H, $R_9$=$CH_3$) | H | 5-$OCH_3$ | $OCH_3$ | $OCH_3$ | |
| J-11 | Q-64 ($R_7$=H, $R_8$=H, $R_9$=H) | H | H | $OCH_3$ | $OCH_3$ | |
| J-11 | Q-64 ($R_7$=$C_2H_5$, $R_8$=H, $R_9$=H) | H | H | $OCH_3$ | $OCF_2H$ | |

Table 1 (continued)

| J | Q | R | $R_{1d}$ | X | Y | m.p. (°C) |
|---|---|---|---|---|---|---|
| J-11 | Q-64 ($R_7$=H, $R_8$=H, $R_9$=$C_2H_5$) | H | 5-Cl | $OCH_3$ | $OCH_3$ | |
| J-11 | Q-65 ($R_7$=H, $R_8$=H, $R_9$=H) | H | H | $OCH_3$ | $OCH_3$ | |
| J-11 | Q-65 ($R_7$=n-$C_4H_9$, $R_8$=H, $R_9$=H) | $CH_3$ | H | $OCH_3$ | $CH_3$ | |
| J-11 | Q-65 ($R_7$=H, $R_8$=$CH_3$, $R_9$=i-$C_3H_7$) | H | 5-$OCH_3$ | $OCH_3$ | $OCH_3$ | |
| J-11 | Q-66 ($R_7$=H, $R_8$=H, $R_9$=H) | H | H | $OCH_3$ | $OCH_3$ | |
| J-11 | Q-66 ($R_7$=i-$C_3H_7$, $R_8$=H, $R_9$=H) | H | H | $OCF_2H$ | $CH_3$ | |
| J-11 | Q-66 ($R_7$=H, $R_8$=$C_2H_5$, $R_9$=$CH_3$) | H | 5-Cl | $OCH_3$ | $CH_3$ | |
| J-11 | Q-67 ($R_7$=H, $R_8$=H, $R_9$=H, $R_3$=H) | H | H | $CH_3$ | $OCH_3$ | |
| J-11 | Q-67 ($R_7$=$CH_3$, $R_8$=H, $R_9$=H, $R_3$=$CH_3$) | H | H | $CH_3$ | $OCH_3$ | |
| J-11 | Q-67 ($R_7$=H, $R_8$=H, $R_9$=$CH_3$, $R_3$=$C_2H_5$) | H | 5-$CH_3$ | $OCH_3$ | $OCH_3$ | |
| J-11 | Q-68 ($R_7$=H, $R_8$=H, $R_9$=H, $R_3$=$CH_3$) | H | H | $OCH_3$ | $OCH_3$ | |
| J-11 | Q-68 ($R_7$=$C_2H_5$, $R_8$=H, $R_9$=H, $R_3$=i-$C_3H_7$) | H | H | Cl | $OCH_3$ | |
| J-11 | Q-68 ($R_7$=H, $R_8$=H, $R_9$=$C_2H_5$, $R_3$=n-$C_4H_9$) | H | H | $CH_3$ | $OCH_3$ | |
| J-11 | Q-69 ($R_7$=H, $R_8$=H, $R_9$=H, $R_3$=$CH_3$) | H | H | $CH_3$ | $OCH_3$ | |
| J-11 | Q-69 ($R_7$=n-$C_4H_9$, $R_8$=H, $R_9$=H, $R_3$=$C_2H_5$) | $CH_3$ | H | $CH_3$ | $OCH_3$ | |
| J-11 | Q-69 ($R_7$=H, $R_8$=$CH_3$, $R_9$=i-$C_3H_7$, $R_3$=H) | H | H | $OCH_3$ | $OCH_3$ | |
| J-11 | Q-70 ($R_7$=H, $R_8$=H, $R_9$=H, $R_3$=$CH_3$) | H | H | $OCH_3$ | $OCH_3$ | |
| J-11 | Q-70 ($R_7$=i-$C_3H_7$, $R_8$=H, $R_9$=H, $R_3$=$CH_3$) | H | H | $OCH_3$ | $OCH_3$ | |
| J-11 | Q-70 ($R_7$=H, $R_8$=$C_2H_5$, $R_9$=$CH_3$, $R_3$=H) | H | 5-$OCH_3$ | $OCH_3$ | $OCH_3$ | |
| J-11 | Q-72 ($R_7$=H, $R_8$=H, $R_9$=H, $R_2$=H) | H | H | $OCH_3$ | $OCH_3$ | |
| J-11 | Q-72 ($R_7$=H, $R_8$=H, $R_9$=H, $R_2$=$CH_3$) | $CH_3$ | H | $OCH_3$ | $CH_3$ | |
| J-11 | Q-72 ($R_7$=$CH_3$, $R_8$=H, $R_9$=H, $R_2$=H) | H | 5-$OCH_3$ | $OCH_3$ | $OCH_3$ | |
| J-11 | Q-73 ($R_7$=H, $R_8$=H, $R_9$=H, $R_2$=H) | H | H | $OCH_3$ | $OCH_3$ | |
| J-11 | Q-73 ($R_7$=H, $R_8$=$CH_3$, $R_9$=H, $R_2$=$C_2H_5$) | H | H | $OCF_2H$ | $CH_3$ | |
| J-11 | Q-73 ($R_7$=$CH_3$, $R_8$=H, $R_9$=$CH_3$, $R_2$=H) | H | 5-Cl | $OCH_3$ | $CH_3$ | |
| J-11 | Q-74 ($R_7$=H, $R_8$=H, $R_9$=H, $R_2$=H) | H | H | $CH_3$ | $OCH_3$ | |
| J-11 | Q-74 ($R_7$=$CH_3$, $R_8$=H, $R_9$=H, $R_2$=H) | H | H | $CH_3$ | $OCH_3$ | |
| J-11 | Q-74 ($R_7$=H, $R_8$=H, $R_9$=H, $R_2$=$CH_3$) | H | 5-$CH_3$ | $OCH_3$ | $OCH_3$ | |
| J-11 | Q-75 ($R_7$=H, $R_8$=H, $R_9$=H) | H | H | $OCH_3$ | $OCH_3$ | |

Table 1 (continued)

| J | Q | R | $R_{1d}$ | X | Y | m.p. (°C) |
|---|---|---|---|---|---|---|
| J-11 | Q-75 ($R_7$=H, $R_8$=H, $R_9$=$CH_3$) | H | H | Cl | $OCH_3$ | |
| J-11 | Q-75 ($R_7$=$CH_3$, $R_8$=H, $R_9$=$CH_3$) | H | H | $CH_3$ | $OCH_3$ | |
| J-11 | Q-76 ($R_7$=H, $R_8$=H, $R_9$=H) | H | H | $CH_3$ | $OCH_3$ | |
| J-11 | Q-76 ($R_7$=H, $R_8$=H, $R_9$=$CH_3$) | $CH_3$ | H | $CH_3$ | $OCH_3$ | |
| J-11 | Q-76 ($R_7$=$C_2H_5$, $R_8$=H, $R_9$=$C_2H_5$) | H | H | $OCH_3$ | $OCH_3$ | |
| J-11 | Q-77 ($R_7$=H, $R_8$=H, $R_9$=H) | H | H | $OCH_3$ | $OCH_3$ | |
| J-11 | Q-77 ($R_7$=H, $R_8$=H, $R_9$=n-$C_3H_7$) | H | H | $OCH_3$ | $OCH_3$ | |
| J-11 | Q-77 ($R_7$=H, $R_8$=$CH_3$, $R_9$=H) | H | 5-$OCH_3$ | $OCH_3$ | $OCH_3$ | |
| J-11 | Q-78 ($R_7$=H, $R_8$=H, $R_9$=H) | H | H | $OCH_3$ | $OCH_3$ | |
| J-11 | Q-78 ($R_7$=H, $R_8$=H, $R_9$=s-$C_4H_9$) | H | H | $OCH_3$ | $OCF_2H$ | |
| J-11 | Q-78 ($R_7$=n-$C_3H_7$, $R_8$=H, $R_9$=H) | H | 5-Cl | $OCH_3$ | $OCH_3$ | |
| J-11 | Q-79 ($R_7$=H, $R_8$=H, $R_9$=H, $R_3$=H) | H | H | $OCH_3$ | $OCH_3$ | |
| J-11 | Q-79 ($R_7$=H, $R_8$=H, $R_9$=H, $R_3$=$CH_3$) | $CH_3$ | H | $OCH_3$ | $OCH_3$ | |
| J-11 | Q-79 ($R_7$=$CH_3$, $R_8$=H, $R_9$=H, $R_3$=$C_2H_5$) | H | 5-$OCH_3$ | $OCH_3$ | $OCH_3$ | |
| J-11 | Q-80 ($R_7$=H, $R_8$=H, $R_9$=H, $R_3$=$CH_3$) | H | H | $OCH_3$ | $OCH_3$ | |
| J-11 | Q-80 ($R_7$=$CH_3$, $R_8$=H, $R_9$=H, $R_3$=$CH_3$) | H | H | $OCF_2H$ | $CH_3$ | |
| J-11 | Q-80 ($R_7$=H, $R_8$=H, $R_9$=$CH_3$, $R_3$=$C_2H_5$) | H | 5-Cl | $OCH_3$ | $CH_3$ | |
| J-11 | Q-81 ($R_7$=H, $R_8$=H, $R_9$=H, $R_3$=$CH_3$) | H | H | $CH_3$ | $OCH_3$ | |
| J-11 | Q-81 ($R_7$=H, $R_8$=$CH_3$, $R_9$=H, $R_3$=i-$C_3H_7$) | H | H | $CH_3$ | $OCH_3$ | |
| J-11 | Q-81 ($R_7$=$CH_3$, $R_8$=H, $R_9$=$CH_3$, $R_3$=$CH_3$) | H | 5-$CH_3$ | $OCH_3$ | $OCH_3$ | |
| J-11 | Q-82 ($R_7$=H, $R_8$=H, $R_9$=H, $R_3$=$CH_3$) | H | H | $OCH_3$ | $OCH_3$ | |
| J-11 | Q-82 ($R_7$=$CH_3$, $R_8$=H, $R_9$=$CH_3$, $R_3$=H) | H | H | Cl | $OCH_3$ | |
| J-11 | Q-82 ($R_7$=H, $R_8$=H, $R_9$=$C_2H_5$, $R_3$=$CH_3$) | H | H | $CH_3$ | $OCH_3$ | |
| J-11 | Q-83 ($R_7$=H, $R_8$=H, $R_9$=H, $R_{10}$=H) | H | H | $CH_3$ | $OCH_3$ | |
| J-11 | Q-83 ($R_7$=$CH_3$, $R_8$=H, $R_9$=H, $R_{10}$=$CH_3$) | $CH_3$ | H | $CH_3$ | $OCH_3$ | |
| J-11 | Q-83 ($R_7$=$CH_3$, $R_8$=$CH_3$, $R_9$=H, $R_{10}$=H) | H | H | $OCH_3$ | $OCH_3$ | |
| J-11 | Q-84 ($R_7$=H, $R_8$=H, $R_9$=H) | H | H | $OCH_3$ | $OCH_3$ | |
| J-11 | Q-84 ($R_7$=H, $R_8$=H, $R_9$=$CH_3$) | H | H | $OCH_3$ | $OCH_3$ | |
| J-11 | Q-84 ($R_7$=H, $R_8$=$C_2H_5$, $R_9$=H) | H | 5-$OCH_3$ | $OCH_3$ | $OCH_3$ | |
| J-11 | Q-85 ($R_7$=H, $R_8$=H, $R_9$=H) | H | H | $OCH_3$ | $OCH_3$ | |
| J-11 | Q-85 ($R_7$=$CH_3$, $R_8$=H, $R_9$=H) | H | H | $OCH_3$ | $OCF_2H$ | |
| J-11 | Q-85 ($R_7$=H, $R_8$=H, $R_9$=$C_2H_5$) | H | 5-Cl | $OCH_3$ | $OCH_3$ | |

Table 1 (continued)

| J | Q | R | $R_{1d}$ | X | Y | m.p. (°C) |
|---|---|---|---|---|---|---|
| J-11 | Q-86 ($R_7$=H, $R_8$=H, $R_9$=H) | H | H | $OCH_3$ | $OCH_3$ | |
| J-11 | Q-86 ($R_7$=$CH_3$, $R_8$=H, $R_9$=H) | $CH_3$ | H | $OCH_3$ | $OCH_3$ | |
| J-11 | Q-86 ($R_7$=H, $R_8$=H, $R_9$=$C_2H_5$) | H | 5-$OCH_3$ | $OCH_3$ | $OCH_3$ | |
| J-11 | Q-87 ($R_7$=H, $R_8$=H, $R_9$=H) | H | H | $OCH_3$ | $OCH_3$ | |
| J-11 | Q-87 ($R_7$=H, $R_8$=H, $R_9$=$CH_3$) | H | H | $OCF_2H$ | $CH_3$ | |
| J-11 | Q-87 ($R_7$=$CH_3$, $R_8$=H, $R_9$=H) | H | 5-Cl | $OCH_3$ | $CH_3$ | |
| J-11 | Q-88 ($R_7$=H, $R_8$=H, $R_9$=H) | H | H | $CH_3$ | $OCH_3$ | |
| J-11 | Q-88 ($R_7$=$CH_3$, $R_8$=H, $R_9$=H) | H | H | $CH_3$ | $OCH_3$ | |
| J-11 | Q-88 ($R_7$=H, $R_8$=H, $R_9$=$CH_3$) | H | 5-$CH_3$ | $OCH_3$ | $OCH_3$ | |
| J-11 | Q-89 ($R_7$=H, $R_8$=H, $R_9$=H) | H | H | $OCH_3$ | $OCH_3$ | |
| J-11 | Q-89 ($R_7$=$CH_3$, $R_8$=H, $R_9$=$C_2H_5$) | H | H | Cl | $OCH_3$ | |
| J-11 | Q-89 ($R_7$=H, $R_8$=$CH_3$, $R_9$=H) | H | H | $CH_3$ | $OCH_3$ | |
| J-11 | Q-90 ($R_7$=H, $R_8$=H, $R_9$=H, $R_3$=$CH_3$) | H | H | $CH_3$ | $OCH_3$ | |
| J-11 | Q-90 ($R_7$=$CH_3$, $R_8$=H, $R_9$=H, $R_3$=$C_2H_5$) | $CH_3$ | H | $CH_3$ | $OCH_3$ | |
| J-11 | Q-90 ($R_7$=H, $R_8$=H, $R_9$=$CH_3$, $R_3$=$CH_3$) | H | H | $OCH_3$ | $OCH_3$ | |
| J-11 | Q-91 ($R_7$=H, $R_8$=H, $R_9$=H, $R_3$=$CH_3$) | H | H | $OCH_3$ | $OCH_3$ | |
| J-11 | Q-91 ($R_7$=$CH_3$, $R_8$=H, $R_9$=H, $R_3$=H) | H | H | $OCH_3$ | $OCH_3$ | |
| J-11 | Q-91 ($R_7$=H, $R_8$=H, $R_9$=$CH_3$, $R_3$=$CH_3$) | H | 5-$OCH_3$ | $OCH_3$ | $OCH_3$ | |
| J-11 | Q-92 ($R_7$=H, $R_8$=H, $R_9$=H, $R_{10}$=H) | H | H | $OCH_3$ | $OCH_3$ | |
| J-11 | Q-92 ($R_7$=H, $R_8$=H, $R_9$=H, $R_{10}$=$CH_3$) | H | H | $OCH_3$ | $OCF_2H$ | |
| J-11 | Q-92 ($R_7$=H, $R_8$=$CH_3$, $R_9$=H, $R_{10}$=H) | H | 5-Cl | $OCH_3$ | $OCH_3$ | |
| J-11 | Q-93 ($R_7$=H, $R_8$=H, $R_9$=H) | H | H | $OCH_3$ | $OCH_3$ | |
| J-11 | Q-93 ($R_7$=H, $R_8$=H, $R_9$=$CH_3$) | $CH_3$ | H | $OCH_3$ | $OCH_3$ | |
| J-11 | Q-93 ($R_7$=$CH_3$, $R_8$=H, $R_9$=H) | H | 5-$OCH_3$ | $OCH_3$ | $OCH_3$ | |
| J-11 | Q-94 ($R_7$=H, $R_8$=H, $R_9$=H) | H | H | $OCH_3$ | $OCH_3$ | |
| J-11 | Q-94 ($R_7$=$CH_3$, $R_8$=H, $R_9$=H) | H | H | $OCF_2H$ | $CH_3$ | |
| J-11 | Q-94 ($R_7$=H, $R_8$=H, $R_9$=n-$C_4H_9$) | H | 5-Cl | $OCH_3$ | $CH_3$ | |
| J-11 | Q-95 ($R_7$=H, $R_8$=H, $R_9$=H) | H | H | $CH_3$ | $OCH_3$ | |
| J-11 | Q-95 ($R_7$=H, $R_8$=H, $R_9$=$CH_3$) | H | H | $CH_3$ | $OCH_3$ | |
| J-11 | Q-95 ($R_7$=H, $R_8$=H, $R_9$=s-$C_4H_9$) | H | 5-$CH_3$ | $OCH_3$ | $OCH_3$ | |
| J-11 | Q-96 ($R_7$=H, $R_8$=H, $R_9$=H) | H | H | $OCH_3$ | $OCH_3$ | |
| J-11 | Q-96 ($R_7$=$CH_3$, $R_8$=H, $R_9$=H) | H | H | Cl | $OCH_3$ | |

Table 1 (continued)

| J | Q | R | $R_{1d}$ | X | Y | m.p.(°C) |
|---|---|---|---|---|---|---|
| J-11 | Q-96 ($R_7$=H, $R_8$=H, $R_9$=$CH_3$) | H | H | $CH_3$ | $OCH_3$ | |
| J-11 | Q-97 ($R_7$=H, $R_8$=H, $R_9$=H) | H | H | $CH_3$ | $OCH_3$ | |
| J-11 | Q-97 ($R_7$=$CH_3$, $R_8$=$CH_3$, $R_9$=$CH_3$) | $CH_3$ | H | $CH_3$ | $OCH_3$ | |
| J-11 | Q-97 ($R_7$=H, $R_8$=H, $R_9$=$CH_3$) | H | H | $OCH_3$ | $OCH_3$ | |
| J-11 | Q-98 ($R_7$=H, $R_8$=H, $R_9$=H) | H | H | $OCH_3$ | $OCH_3$ | |
| J-11 | Q-98 ($R_7$=$CH_3$, $R_8$=H, $R_9$=H) | H | H | $OCH_3$ | $OCH_3$ | |
| J-11 | Q-98 ($R_7$=H, $R_8$=H, $R_9$=i-$C_3H_7$) | H | 5-$OCH_3$ | $OCH_3$ | $OCH_3$ | |
| J-11 | Q-99 ($R_7$=H, $R_8$=H, $R_9$=H, $R_3$=H) | H | H | $OCH_3$ | $OCH_3$ | |
| J-11 | Q-99 ($R_7$=H, $R_8$=H, $R_9$=$CH_3$, $R_3$=$CH_3$) | H | H | $OCH_3$ | $OCF_2H$ | |
| J-11 | Q-99 ($R_7$=H, $R_8$=H, $R_9$=s-$C_4H_9$, $R_3$=H) | H | 5-Cl | $OCH_3$ | $OCH_3$ | |
| J-11 | Q-100 ($R_7$=H, $R_8$=H, $R_9$=H, $R_3$=$C_2H_5$) | H | H | $OCH_3$ | $OCH_3$ | |
| J-11 | Q-100 ($R_7$=$CH_3$, $R_8$=H, $R_9$=H, $R_3$=H) | $CH_3$ | H | $OCH_3$ | $CH_3$ | |
| J-11 | Q-100 ($R_7$=H, $R_8$=H, $R_9$=$CH_3$, $R_3$=$CH_3$) | H | 5-$OCH_3$ | $OCH_3$ | $OCH_3$ | |
| J-11 | Q-101 ($R_7$=H, $R_8$=H, $R_9$=H, $R_3$=i-$C_3H_7$) | H | H | $OCH_3$ | $OCH_3$ | |
| J-11 | Q-101 ($R_7$=$CH_3$, $R_8$=$CH_3$, $R_9$=$CH_3$, $R_3$=H) | H | H | $OCF_2H$ | $CH_3$ | |
| J-11 | Q-101 ($R_7$=H, $R_8$=H, $R_9$=$CH_3$, $R_3$=$CH_3$) | H | 5-Cl | $OCH_3$ | $CH_3$ | |
| J-11 | Q-102 ($R_7$=H, $R_8$=H, $R_9$=H, $R_3$=H) | H | H | $CH_3$ | $OCH_3$ | |
| J-11 | Q-102 ($R_7$=$CH_3$, $R_8$=H, $R_9$=H, $R_3$=$CH_3$) | H | H | $CH_3$ | $OCH_3$ | |
| J-11 | Q-102 ($R_7$=H, $R_8$=H, $R_9$=i-$C_3H_7$, $R_3$=$CH_3$) | H | 5-$CH_3$ | $OCH_3$ | $OCH_3$ | |
| J-11 | Q-104 ($R_7$=H, $R_8$=H, $R_9$=H, $R_2$=H) | H | H | $CH_3$ | $OCH_3$ | |
| J-11 | Q-104 ($R_7$=$CH_3$, $R_8$=H, $R_9$=H, $R_2$=$OC_2H_5$) | $CH_3$ | H | $CH_3$ | $OCH_3$ | |
| J-11 | Q-104 ($R_7$=H, $R_8$=H, $R_9$=H, $R_2$=Br) | H | H | $OCH_3$ | $OCH_3$ | |
| J-11 | Q-105 ($R_7$=H, $R_8$=H, $R_9$=H, $R_2$=H) | H | H | $OCH_3$ | $OCH_3$ | 195-200 |
| J-11 | Q-105 ($R_7$=H, $R_8$=H, $R_9$=H, $R_2$=H) | H | H | $OCH_3$ | $CH_3$ | 185 |
| J-11 | Q-105 ($R_7$=H, $R_8$=H, $R_9$=H, $R_2$=H) | H | H | $OCH_3$ | Cl | 172-202 |
| J-11 | Q-105 ($R_7$=H, $R_8$=H, $R_9$=H, $R_2$=H) | H | H | $CH_3$ | $CH_3$ | 205 |
| J-11 | Q-105 ($R_7$=$CH_3$, $R_8$=H, $R_9$=H, $R_2$=$CH_3$) | H | H | $OCH_3$ | $OCH_3$ | |
| J-11 | Q-105 ($R_7$=H, $R_8$=$CH_3$, $R_9$=H, $R_2$=$C_2H_5$) | H | 5-$OCH_3$ | $OCH_3$ | $OCH_3$ | |
| J-11 | Q-106 ($R_7$=H, $R_8$=H, $R_9$=H, $R_2$=H) | H | H | $OCH_3$ | $OCH_3$ | |

Table 1 (continued)

| J | Q | R | $R_{1d}$ | X | Y | m.p. (°C) |
|---|---|---|---|---|---|---|
| J-11 | Q-106 ($R_7=CH_3$, $R_8=H$, $R_9=H$, $R_2=CH_3$) | H | H | $OCH_3$ | $OCF_2H$ | |
| J-11 | Q-106 ($R_7=Cl$, $R_8=H$, $R_9=H$, $R_2=H$) | H | 5-Cl | $OCH_3$ | $OCH_3$ | |
| J-11 | Q-107 ($R_7=H$, $R_8=H$, $R_9=H$, $R_2=H$) | H | H | $OCH_3$ | $OCH_3$ | |
| J-11 | Q-107 ($R_7=CH_3$, $R_8=H$, $R_9=H$, $R_2=H$) | $CH_3$ | H | $OCH_3$ | $OCH_3$ | |
| J-11 | Q-107 ($R_7=H$, $R_8=H$, $R_9=H$, $R_2=CH_3$) | H | 5-$OCH_3$ | $OCH_3$ | $OCH_3$ | |
| J-11 | Q-108 ($R_7=H$, $R_8=H$, $R_9=H$, $R_2=H$) | H | H | $OCH_3$ | $OCH_3$ | |
| J-11 | Q-108 ($R_7=CH_3$, $R_8=H$, $R_9=H$, $R_2=Cl$) | H | H | $OCF_2H$ | $CH_3$ | |
| J-11 | Q-108 ($R_7=H$, $R_8=H$, $R_9=H$, $R_2=CH_3$) | H | 5-Cl | $OCH_3$ | $CH_3$ | |
| J-11 | Q-109 ($R_7=H$, $R_8=H$, $R_9=H$) | H | H | $CH_3$ | $OCH_3$ | |
| J-11 | Q-109 ($R_7=CH_3$, $R_8=H$, $R_9=H$) | H | H | $CH_3$ | $OCH_3$ | |
| J-11 | Q-109 ($R_7=H$, $R_8=H$, $R_9=CH_3$) | H | 5-$CH_3$ | $OCH_3$ | $OCH_3$ | |
| J-11 | Q-110*($R_7=H$, $R_8=H$, $R_9=H$) | H | H | $OCH_3$ | $OCH_3$ | |
| J-11 | Q-110 ($R_7=CH_3$, $R_8=H$, $R_9=H$) | H | H | Cl | $OCH_3$ | |
| J-11 | Q-110 ($R_7=H$, $R_8=CH_3$, $R_9=H$) | H | H | $CH_3$ | $OCH_3$ | |
| J-11 | Q-111 ($R_7=H$, $R_8=H$, $R_9=H$) | H | H | $CH_3$ | $OCH_3$ | |
| J-11 | Q-111 ($R_7=CH_3$, $R_8=H$, $R_9=H$) | $CH_3$ | H | $CH_3$ | $OCH_3$ | |
| J-11 | Q-111 ($R_7=H$, $R_8=H$, $R_9=CH_3$) | H | H | $OCH_3$ | $OCH_3$ | |
| J-11 | Q-112 ($R_7=H$, $R_8=H$, $R_9=H$) | H | H | $OCH_3$ | $OCH_3$ | |
| J-11 | Q-112 ($R_7=CH_3$, $R_8=H$, $R_9=H$) | H | H | $OCH_3$ | $OCH_3$ | |
| J-11 | Q-112 ($R_7=H$, $R_8=CH_3$, $R_9=CH_3$) | H | 5-$OCH_3$ | $OCH_3$ | $OCH_3$ | |
| J-11 | Q-113 ($R_7=H$, $R_8=H$, $R_9=H$, $R_3=CH_3$) | H | H | $OCH_3$ | $OCH_3$ | |
| J-11 | Q-113 ($R_7=C_2H_5$, $R_8=H$, $R_9=H$, $R_3$=<u>i</u>-$C_3H_7$) | H | H | $OCH_3$ | $OCF_2H$ | |
| J-11 | Q-113 ($R_7=H$, $R_8=H$, $R_9=C_2H_5$, $R_3$=<u>n</u>-$C_4H_9$) | H | 5-Cl | $OCH_3$ | $OCH_3$ | |
| J-11 | Q-114 ($R_7=H$, $R_8=H$, $R_9=H$, $R_3=CH_3$) | H | H | $OCH_3$ | $OCH_3$ | |
| J-11 | Q-114 ($R_7$=<u>n</u>-$C_4H_9$, $R_8=H$, $R_9=H$, $R_3=C_2H_5$) | $CH_3$ | H | $OCH_3$ | $CH_3$ | |
| J-11 | Q-114 ($R_7=H$, $R_8=CH_3$, $R_8$=<u>i</u>-$C_3H_7$, $R_3=H$) | H | 5-$OCH_3$ | $OCH_3$ | $OCH_3$ | |
| J-11 | Q-115 ($R_7=H$, $R_8=H$, $R_9=H$, $R_3=CH_3$) | H | H | $OCH_3$ | $OCH_3$ | |
| J-11 | Q-115 ($R_7$=<u>i</u>-$C_3H_7$, $R_8=H$, $R_9=H$, $R_3=CH_3$) | H | H | $OCF_2H$ | $CH_3$ | |
| J-11 | Q-115 ($R_7=H$, $R_8=C_2H_5$, $R_9=CH_3$, $R_3=H$) | H | 5-Cl | $OCH_3$ | $CH_3$ | |
| J-11 | Q-116 ($R_7=H$, $R_8=H$, $R_9=H$, $R_{10}=H$) | H | H | $CH_3$ | $OCH_3$ | |
| J-11 | Q-116 ($R_7=CH_3$, $R_8=H$, $R_9=H$, $R_{10}=H$) | H | H | $CH_3$ | $OCH_3$ | |
| J-11 | Q-116 ($R_7=H$, $R_8=H$, $R_9=H$, $R_{10}=CH_3$) | H | 5-$CH_3$ | $OCH_3$ | $OCH_3$ | |

Table 1 (continued)

| J | Q | R | $R_{1d}$ | X | Y | m.p. (°C) |
|---|---|---|---|---|---|---|
| J-11 | Q-118 ($R_7$=H, $R_8$=H, $R_9$=H, $R_{10}$=H) | H | H | $CH_3$ | $OCH_3$ | |
| J-11 | Q-118 ($R_7$=$CH_3$, $R_8$=H, $R_9$=H, $R_{10}$=H) | $CH_3$ | H | $CH_3$ | $OCH_3$ | |
| J-11 | Q-118 ($R_7$=H, $R_8$=H, $R_9$=H, $R_{10}$=$CH_3$) | H | H | $OCH_3$ | $OCH_3$ | |
| J-11 | Q-119 ($R_7$=H, $R_8$=H, $R_9$=H, $R_2$=H) | H | H | $OCH_3$ | $OCH_3$ | |
| J-11 | Q-119 ($R_7$=H, $R_8$=H, $R_9$=H, $R_2$=$CH_3$) | H | H | $OCH_3$ | $OCH_3$ | |
| J-11 | Q-119 ($R_7$=H, $R_8$=$CH_3$, $R_9$=H, $R_2$=$C_2H_5$) | H | 5-$OCH_3$ | $OCH_3$ | $OCH_3$ | |
| J-11 | Q-120 ($R_7$=H, $R_8$=H, $R_9$=H, $R_2$=H) | H | H | $OCH_3$ | $OCH_3$ | |
| J-11 | Q-120 ($R_7$=H, $R_8$=H, $R_9$=H, $R_2$=$CH_3$) | H | H | $OCH_3$ | $OCF_2H$ | |
| J-11 | Q-120 ($R_7$=H, $R_8$=H, $R_9$=$CH_3$, $R_2$=$CH_3$) | H | 5-Cl | $OCH_3$ | $OCH_3$ | |
| J-11 | Q-121 ($R_7$=H, $R_8$=H, $R_9$=H, $R_2$=H) | H | H | $OCH_3$ | $OCH_3$ | |
| J-11 | Q-121 ($R_7$=H, $R_8$=H, $R_9$=H, $R_2$=$C_2H_5$) | $CH_3$ | H | $OCH_3$ | $CH_3$ | |
| J-11 | Q-121 ($R_7$=$CH_3$, $R_8$=$CH_3$, $R_9$=H, $R_2$=H) | H | 5-$OCH_3$ | $OCH_3$ | $OCH_3$ | |
| J-11 | Q-122 ($R_7$=H, $R_8$=H, $R_9$=H, $R_2$=H) | H | H | $OCH_3$ | $OCH_3$ | |
| J-11 | Q-122 ($R_7$=H, $R_8$=H, $R_9$=H, $R_2$=$CH_3$) | H | H | $OCF_2H$ | $CH_3$ | |
| J-11 | Q-122 ($R_7$=$CH_3$, $R_8$=H, $R_9$=H, $R_2$=H) | H | 5-Cl | $OCH_3$ | $CH_3$ | |
| J-11 | Q-123 ($R_7$=H, $R_8$=H, $R_9$=H) | H | H | $CH_3$ | $OCH_3$ | |
| J-11 | Q-123 ($R_7$=H, $R_8$=H, $R_9$=$CH_3$) | H | H | $CH_3$ | $OCH_3$ | |
| J-11 | Q-123 ($R_7$=$CH_3$, $R_8$=H, $R_9$=$CH_3$) | H | 5-$CH_3$ | $OCH_3$ | $OCH_3$ | |
| J-11 | Q-124 ($R_7$=H, $R_8$=H, $R_9$=H) | H | H | $OCH_3$ | $OCH_3$ | |
| J-11 | Q-124 ($R_7$=H, $R_8$=H, $R_9$=$CH_3$) | H | H | Cl | $OCH_3$ | |
| J-11 | Q-124 ($R_7$=$C_2H_5$, $R_8$=H, $R_9$=$C_2H_5$) | H | H | $CH_3$ | $OCH_3$ | |
| J-11 | Q-125 ($R_7$=H, $R_8$=H, $R_9$=H) | H | H | $CH_3$ | $OCH_3$ | |
| J-11 | Q-125 ($R_7$=H, $R_8$=H, $R_9$=<u>n</u>-$C_4H_9$) | $CH_3$ | H | $CH_3$ | $OCH_3$ | |
| J-11 | Q-125 ($R_7$=H, $R_8$=$CH_3$, $R_9$=H) | H | H | $OCH_3$ | $OCH_3$ | |
| J-11 | Q-126 ($R_7$=H, $R_8$=H, $R_9$=H) | H | H | $OCH_3$ | $OCH_3$ | |
| J-11 | Q-126 ($R_7$=H, $R_8$=H, $R_9$=<u>s</u>-$C_3H_7$) | H | H | $OCH_3$ | $OCH_3$ | |
| J-11 | Q-126 ($R_7$=<u>n</u>-$C_3H_7$, $R_8$=H, $R_9$=H) | H | 5-$OCH_3$ | $OCH_3$ | $OCH_3$ | |
| J-11 | Q-127 ($R_7$=H, $R_8$=H, $R_9$=H, $R_3$=H) | H | H | $OCH_3$ | $OCH_3$ | |
| J-11 | Q-127 ($R_7$=H, $R_8$=H, $R_9$=H, $R_3$=$CH_3$) | H | H | $OCH_3$ | $OCF_2H$ | |

Table 1 (continued)

| J | Q | R | $R_{1d}$ | X | Y | m.p. (°C) |
|---|---|---|---|---|---|---|
| J-11 | Q-127 ($R_7=CH_3$, $R_8=H$, $R_9=H$, $R_3=C_2H_5$) | H | 5-Cl | $OCH_3$ | $OCH_3$ | |
| J-11 | Q-128 ($R_7=H$, $R_8=H$, $R_9=H$, $R_3=CH_3$) | H | H | $OCH_3$ | $OCH_3$ | |
| J-11 | Q-128 ($R_7=CH_3$, $R_8=H$, $R_9=H$, $R_3=CH_3$) | $CH_3$ | H | $OCH_3$ | $OCH_3$ | |
| J-11 | Q-128 ($R_7=H$, $R_8=H$, $R_9=CH_3$, $R_3=C_2H_5$) | H | 5-$OCH_3$ | $OCH_3$ | $OCH_3$ | |
| J-11 | Q-129 ($R_7=H$, $R_8=H$, $R_9=H$, $R_3=CH_3$) | H | H | $OCH_3$ | $OCH_3$ | |
| J-11 | Q-129 ($R_7=H$, $R_8=CH_3$, $R_9=H$, $R_3$=i-$C_3H_7$) | H | H | $OCF_2H$ | $CH_3$ | |
| J-11 | Q-129 ($R_7=CH_3$, $R_8=H$, $R_9=CH_3$, $R_3=CH_3$) | H | 5-Cl | $OCH_3$ | $CH_3$ | |
| J-11 | Q-130 ($R_7=H$, $R_8=H$, $R_9=H$, $R_3=CH_3$) | H | H | $CH_3$ | $OCH_3$ | |
| J-11 | Q-130 ($R_7=CH_3$, $R_8=H$, $R_9=CH_3$, $R_3=H$) | H | H | $CH_3$ | $OCH_3$ | |
| J-11 | Q-130 ($R_7=H$, $R_8=H$, $R_9=C_2H_5$, $R_3=CH_3$) | H | 5-$CH_3$ | $OCH_3$ | $OCH_3$ | |
| J-11 | Q-131 ($R_7=H$, $R_8=H$, $R_9=H$, $R_{10}=H$) | H | H | $OCH_3$ | $OCH_3$ | |
| J-11 | Q-131 ($R_7=CH_3$, $R_8=H$, $R_9=H$, $R_{10}=CH_3$) | H | H | Cl | $OCH_3$ | |
| J-11 | Q-131 ($R_7=CH_3$, $R_8=CH_3$, $R_9=H$, $R_{10}=H$) | H | H | $CH_3$ | $OCH_3$ | |
| J-11 | Q-132 ($R_7=H$, $R_8=H$, $R_9=H$) | H | H | $CH_3$ | $OCH_3$ | |
| J-11 | Q-132 ($R_7=H$, $R_8=H$, $R_9=CH_3$) | $CH_3$ | H | $CH_3$ | $OCH_3$ | |
| J-11 | Q-132 ($R_7=H$, $R_8=C_2H_5$, $R_9=H$) | H | H | $OCH_3$ | $OCH_3$ | |
| J-11 | Q-133 ($R_7=H$, $R_8=H$, $R_9=H$) | H | H | $OCH_3$ | $OCH_3$ | |
| J-11 | Q-133 ($R_7=CH_3$, $R_8=H$, $R_9=H$) | H | H | $OCH_3$ | $OCH_3$ | |
| J-11 | Q-133 ($R_7=H$, $R_8=H$, $R_9=C_2H_5$) | H | 5-$OCH_3$ | $OCH_3$ | $OCH_3$ | |
| J-11 | Q-134 ($R_7=H$, $R_8=H$, $R_9=H$) | H | H | $OCH_3$ | $OCH_3$ | |
| J-11 | Q-134 ($R_7=CH_3$, $R_8=H$, $R_9=H$) | H | H | $OCH_3$ | $OCF_2H$ | |
| J-11 | Q-134 ($R_7=H$, $R_8=H$, $R_9=C_2H_5$) | H | 5-Cl | $OCH_3$ | $OCH_3$ | |
| J-11 | Q-135 ($R_7=H$, $R_8=H$, R =H) | H | H | $OCH_3$ | $OCH_3$ | |
| J-11 | Q-135 ($R_7=H$, $R_8=H$, R $=CH_3$) | $CH_3$ | H | $OCH_3$ | $OCH_3$ | |
| J-11 | Q-135 ($R_7=CH_3$, $R_8=H$, $R_9=H$) | H | 5-$OCH_3$ | $OCH_3$ | $OCH_3$ | |
| J-11 | Q-136 ($R_7=H$, $R_8=H$, $R_9=H$) | H | H | $OCH_3$ | $OCH_3$ | |
| J-11 | Q-136 ($R_7=H$, $R_8=H$, $R_9=CH_3$) | H | H | $OCF_2H$ | $CH_3$ | |
| J-11 | Q-136 ($R_7=H$, $R_8=CH_3$, $R_9=C_2H_5$) | H | 5-Cl | $OCH_3$ | $CH_3$ | |
| J-11 | Q-137 ($R_7=H$, $R_8=H$, $R_9=H$) | H | H | $CH_3$ | $OCH_3$ | |
| J-11 | Q-137 ($R_7=CH_3$, $R_8=H$, $R_9=H$) | H | H | $CH_3$ | $OCH_3$ | |
| J-11 | Q-137 ($R_7=H$, $R_8=H$, $R_9=CH_3$) | H | 5-$CH_3$ | $OCH_3$ | $OCH_3$ | |
| J-11 | Q-138 ($R_7=H$, $R_8=H$, $R_9=H$) | H | H | $OCH_3$ | $OCH_3$ | 195 |

Table 1 (continued)

| J | Q | R | $R_{1d}$ | X | Y | m.p.(°C) |
|---|---|---|---|---|---|---|
| J-11 | Q-138 ($R_7$=H, $R_8$=H, $R_9$=H) | H | H | $OCH_3$ | $CH_3$ | 201 |
| J-11 | Q-138 ($R_7$=H, $R_8$=H, $R_9$=H) | H | H | $OCH_3$ | Cl | 198 |
| J-11 | Q-138 ($R_7$=H, $R_8$=H, $R_9$=H) | H | H | $CH_3$ | $CH_3$ | 201-202 |
| J-11 | Q-138 ($R_7$=$CH_3$, $R_8$=H, $R_9$=H) | H | H | Cl | $OCH_3$ | |
| J-11 | Q-138 ($R_7$=H, $R_8$=H, $R_9$=$CH_3$) | H | H | $CH_3$ | $OCH_3$ | |
| J-11 | Q-139 ($R_7$=H, $R_8$=H, $R_9$=H) | H | H | $CH_3$ | $OCH_3$ | |
| J-11 | Q-139 ($R_7$=$CH_3$, $R_8$=H, $R_9$=H) | $CH_3$ | H | $CH_3$ | $OCH_3$ | |
| J-11 | Q-139 ($R_7$=$CH_3$, $R_8$=$CH_3$, $R_9$=H) | H | H | $OCH_3$ | $OCH_3$ | |
| J-11 | Q-140 ($R_7$=H, $R_8$=H, $R_9$=H, $R_3$=$CH_3$) | H | H | $OCH_3$ | $OCH_3$ | |
| J-11 | Q-140 ($R_7$=H, $R_8$=H, $R_9$=$CH_3$, $R_3$=H) | H | H | $OCH_3$ | $OCH_3$ | |
| J-11 | Q-140 ($R_7$=H, $R_8$=$CH_3$, $R_9$=H, $R_3$=$C_2H_5$) | H | 5-$OCH_3$ | $OCH_3$ | $OCH_3$ | |
| J-11 | Q-141 ($R_7$=H, $R_8$=H, $R_9$=H, $R_3$=$CH_3$) | H | H | $OCH_3$ | $OCH_3$ | |
| J-11 | Q-141 ($R_7$=H, $R_8$=H, $R_9$=H, $R_3$=H) | H | H | $OCH_3$ | $OCF_2H$ | |
| J-11 | Q-141 ($R_7$=H, $R_8$=H, $R_9$=$CH_3$, $R_3$=$CH_3$) | H | 5-Cl | $OCH_3$ | $OCH_3$ | |
| J-11 | Q-142 ($R_7$=H, $R_8$=H, $R_9$=H, $R_{10}$=H) | H | H | $OCH_3$ | $OCH_3$ | |
| J-11 | Q-142 ($R_7$=$CH_3$, $R_8$=H, $R_9$=H, $R_{10}$=H) | $CH_3$ | H | $OCH_3$ | $CH_3$ | |
| J-11 | Q-142 ($R_7$=H, $R_8$=H, $R_9$=H, $R_{10}$=$CH_3$) | H | 5-$OCH_3$ | $OCH_3$ | $OCH_3$ | |
| J-11 | Q-143 ($R_7$=H, $R_8$=H, $R_9$=H, $R_3$=$CH_3$) | H | H | $OCH_3$ | $OCH_3$ | |
| J-11 | Q-143 ($R_7$=H, $R_8$=H, $R_9$=H, $R_3$=H) | H | H | $OCF_2H$ | $CH_3$ | |
| J-11 | Q-143 ($R_7$=$CH_3$, $R_8$=H, $R_9$=$CH_3$, $R_3$=$CH_3$) | H | 5-Cl | $OCH_3$ | $CH_3$ | |
| J-11 | Q-143 ($R_7$=H, $R_8$=H, $R_9$=H, $R_3$=n-$C_3H_7$) | H | H | $OCH_3$ | $OCH_3$ | 167-173 |
| J-11 | Q-143 ($R_7$=H, $R_8$=H, $R_9$=H, $R_3$=n-$C_3H_7$) | H | H | $OCH_3$ | $CH_3$ | 163-165 |
| J-11 | Q-143 ($R_7$=H, $R_8$=H, $R_9$=H, $R_3$=n-$C_3H_7$) | H | H | Cl | $OCH_3$ | 143-150 |
| J-11 | Q-143 ($R_7$=H, $R_8$=H, $R_9$=H, $R_3$=n-$C_3H_7$) | H | H | $CH_3$ | $CH_3$ | 148-153 |
| J-11 | Q-144 ($R_7$=H, $R_8$=H, $R_9$=H, $R_3$=$CH_3$) | H | H | $CH_3$ | $OCH_3$ | |
| J-11 | Q-144 ($R_7$=$CH_3$, $R_8$=H, $R_9$=H, $R_3$=$CH_3$) | H | H | $CH_3$ | $OCH_3$ | |
| J-11 | Q-144 ($R_7$=H, $R_8$=$C_2H_5$, $R_9$=H, $R_3$=$CH_3$) | H | 5-$CH_3$ | $OCH_3$ | $OCH_3$ | |
| J-11 | Q-145 ($R_7$=H, $R_8$=H, $R_9$=H) | H | H | $OCH_3$ | $OCH_3$ | |
| J-11 | Q-145 ($R_7$=H, $R_8$=H, $R_9$=$CH_3$) | H | H | Cl | $OCH_3$ | |
| J-11 | Q-145 ($R_7$=$CH_3$, $R_8$=H, $R_9$=H) | H | H | $CH_3$ | $OCH_3$ | |
| J-11 | Q-146 ($R_7$=H, $R_8$=H, $R_9$=H) | H | H | $CH_3$ | $OCH_3$ | |
| J-11 | Q-146 ($R_7$=$CH_3$, $R_8$=H, $R_9$=H) | $CH_3$ | H | $CH_3$ | $OCH_3$ | |
| J-11 | Q-146 ($R_7$=H, $R_8$=H, $R_9$=n-$C_4H_9$) | H | H | $OCH_3$ | $OCH_3$ | |

Table 1 (continued)

| J | Q | R | $R_{1d}$ | X | Y | m.p. (°C) |
|---|---|---|---|---|---|---|
| J-11 | Q-147 ($R_7$=H, $R_8$=H, $R_9$=H) | H | H | $OCH_3$ | $OCH_3$ | |
| J-11 | Q-147 ($R_7$=$CH_3$, $R_8$=H, $R_9$=H) | H | H | $OCH_3$ | $OCH_3$ | |
| J-11 | Q-147 ($R_7$=H, $R_8$=H, $R_9$=n-$C_4H_9$) | H | 5-$OCH_3$ | $OCH_3$ | $OCH_3$ | |
| J-11 | Q-148 ($R_7$=H, $R_8$=H, $R_9$=H) | H | H | $OCH_3$ | $OCH_3$ | |
| J-11 | Q-148 ($R_7$=H, $R_8$=H, $R_9$=$CH_3$) | H | H | $OCH_3$ | $OCF_2H$ | |
| J-11 | Q-148 ($R_7$=$CH_3$, $R_8$=H, $R_9$=H) | H | 5-Cl | $OCH_3$ | $OCH_3$ | |
| J-11 | Q-149 ($R_7$=H, $R_8$=H, $R_9$=H) | H | H | $OCH_3$ | $OCH_3$ | |
| J-11 | Q-149 ($R_7$=H, $R_8$=H, $R_9$=$CH_3$) | $CH_3$ | H | $OCH_3$ | $OCH_3$ | |
| J-11 | Q-149 ($R_7$=$CH_3$, $R_8$=H, $R_9$=H) | H | 5-$OCH_3$ | $OCH_3$ | $OCH_3$ | |
| J-11 | Q-150 ($R_7$=H, $R_8$=H, $R_9$=H) | H | H | $OCH_3$ | $OCH_3$ | |
| J-11 | Q-150 ($R_7$=$CH_3$, $R_8$=H, $R_9$=H) | H | H | $OCF_2H$ | $CH_3$ | |
| J-11 | Q-150 ($R_7$=H, $R_8$=$CH_3$, $R_9$=$CH_3$) | H | 5-Cl | $OCH_3$ | $CH_3$ | |
| J-11 | Q-151 ($R_7$=H, $R_8$=H, $R_9$=H) | H | H | $CH_3$ | $OCH_3$ | |
| J-11 | Q-151 ($R_7$=$CH_3$, $R_8$=H, $R_9$=H) | H | H | $CH_3$ | $OCH_3$ | |
| J-11 | Q-151 ($R_7$=$CH_3$, $R_8$=$CH_3$, $R_9$=H) | H | 5-$CH_3$ | $OCH_3$ | $OCH_3$ | |
| J-11 | Q-152 ($R_7$=H, $R_8$=H, $R_9$=H) | H | H | $OCH_3$ | $OCH_3$ | |
| J-11 | Q-152 ($R_7$=$C_2H_5$, $R_8$=H, $R_9$=H) | H | H | Cl | $OCH_3$ | |
| J-11 | Q-152 ($R_7$=$CH_3$, $R_8$=$CH_3$, $R_9$=H) | H | H | $CH_3$ | $OCH_3$ | |
| J-11 | Q-153 ($R_7$=H, $R_8$=H, $R_9$=H, $R_{11}$=$CH_3$) | H | H | $CH_3$ | $OCH_3$ | |
| J-11 | Q-153 ($R_7$=H, $R_8$=H, $R_9$=H, $R_{11}$=H) | $CH_3$ | H | $CH_3$ | $OCH_3$ | |
| J-11 | Q-153 ($R_7$=$CH_3$, $R_8$=H, $R_9$=H, $R_{11}$=$CH_3$) | H | H | $OCH_3$ | $OCH_3$ | |
| J-11 | Q-154 ($R_7$=H, $R_8$=H, $R_9$=H, $R_{11}$=$CH_3$) | H | H | $OCH_3$ | $OCH_3$ | |
| J-11 | Q-154 ($R_7$=$CH_3$, $R_8$=H, $R_9$=H) | H | H | $OCH_3$ | $OCH_3$ | |
| J-11 | Q-154 ($R_7$=H, $R_8$=$CH_3$, $R_9$=$CH_3$) | H | 5-$OCH_3$ | $OCH_3$ | $OCH_3$ | |
| J-11 | Q-155 ($R_7$=H, $R_8$=H, $R_9$=H) | H | H | $OCH_3$ | $OCH_3$ | |
| J-11 | Q-155 ($R_7$=$CH_3$, $R_8$=H, $R_9$=H) | H | H | $OCH_3$ | $OCF_2H$ | |
| J-11 | Q-155 ($R_7$=$CH_3$, $R_8$=$CH_3$, $R_9$=H) | H | 5-Cl | $OCH_3$ | $OCH_3$ | |
| J-11 | Q-156 ($R_7$=H, $R_8$=H, $R_9$=H) | H | H | $OCH_3$ | $OCH_3$ | |
| J-11 | Q-156 ($R_7$=$C_2H_5$, $R_8$=H, $R_9$=H) | $CH_3$ | H | $OCH_3$ | $OCH_3$ | |
| J-11 | Q-156 ($R_7$=$CH_3$, $R_8$=$CH_3$, $R_9$=H) | H | 5-$OCH_3$ | $OCH_3$ | $OCH_3$ | |

Table 1 (continued)

| J | Q | R | $R_{1d}$ | X | Y | m.p. (°C) |
|---|---|---|---|---|---|---|
| J-11 | Q-158 ($R_7$=H, $R_8$=H, $R_9$=H) | H | H | $CH_3$ | $OCH_3$ | |
| J-11 | Q-158 ($R_7$=H, $R_8$=H, $R_9$=$CH_3$) | H | H | $CH_3$ | $OCH_3$ | |
| J-11 | Q-158 ($R_7$=$CH_3$, $R_8$=H, $R_9$=H) | H | 5-$CH_3$ | $OCH_3$ | $OCH_3$ | |
| J-11 | Q-159 ($R_7$=H, $R_8$=H, $R_9$=H) | H | H | $OCH_3$ | $OCH_3$ | |
| J-11 | Q-159 ($R_7$=$CH_3$, $R_8$=H, $R_9$=H) | H | H | Cl | $OCH_3$ | |
| J-11 | Q-159 ($R_7$=H, $R_8$=H, $R_9$=$CH_3$) | H | H | $CH_3$ | $OCH_3$ | |
| J-11 | Q-160 ($R_7$=H, $R_8$=H, $R_9$=H, $R_{11}$=$CH_3$) | H | H | $CH_3$ | $OCH_3$ | |
| J-11 | Q-160 ($R_7$=H, $R_8$=H, $R_9$=$CH_3$, $R_{11}$=H) | $CH_3$ | H | $CH_3$ | $OCH_3$ | |
| J-11 | Q-160 ($R_7$=$CH_3$, $R_8$=H, $R_9$=H, $R_{11}$=i-$C_3H_7$) | H | H | $OCH_3$ | $OCH_3$ | |
| J-11 | Q-161 ($R_7$=H, $R_8$=H, $R_9$=H, $R_{11}$=$CH_3$) | H | H | $OCH_3$ | $OCH_3$ | |
| J-11 | Q-161 ($R_7$=$CH_3$, $R_8$=H, $R_9$=H, $R_{11}$=H) | H | H | $OCH_3$ | $OCH_3$ | |
| J-11 | Q-161 ($R_7$=H, $R_8$=H, $R_9$=$CH_3$, $R_{11}$=$CH_3$) | H | 5-$OCH_3$ | $OCH_3$ | $OCH_3$ | |
| J-11 | Q-161 ($R_7$=H, $R_8$=H, $R_9$=H, $R_{11}$=H) | H | H | $OCH_3$ | $OCH_3$ | 203-206 |
| J-11 | Q-162 ($R_7$=H, $R_8$=H, $R_9$=H, $R_{10}$=H) | H | H | $OCH_3$ | $OCH_3$ | |
| J-11 | Q-162 ($R_7$=$CH_3$, $R_8$=H, $R_9$=H, $R_{10}$=H) | H | H | $OCH_3$ | $OCF_2H$ | |
| J-11 | Q-162 ($R_7$=H, $R_8$=H, $R_9$=H, $R_{10}$=$CH_3$) | H | 5-Cl | $OCH_3$ | $OCH_3$ | |

TABLE 2
General Formula 2

| J | $W_3$ | Q | R | $R_{1b}$ | X | Y |
|---|---|---|---|---|---|---|
| J-5 | S | Q-1 ($R_7$=H, $R_8$=H) | H | H | $CH_3$ | $CH_3$ |
| J-6 | S | Q-1 ($R_7$=H, $R_8$=H) | H | H | $OCH_3$ | $CH_3$ |
| J-5 | S | Q-1 ($R_7$=H, $R_8$=H) | H | H | $OCH_3$ | $OCH_3$ |
| J-6 | S | Q-1 ($R_7$=H, $R_8$=H) | $CH_3$ | H | $OCH_3$ | $OCH_3$ |
| J-5 | S | Q-1 ($R_7$=H, $R_8$=H) | $CH_3$ | H | $OCH_3$ | $CH_3$ |
| J-6 | S | Q-1 ($R_7$=H, $R_8$=H) | H | 5-$CH_3$ | $OCH_3$ | $OCH_3$ |
| J-5 | S | Q-1 ($R_7$=H, $R_8$=H) | H | 5-$CH_2F$ | $OCH_3$ | $OCH_3$ |
| J-6 | S | Q-1 ($R_7$=H, $R_8$=H) | H | 5-$CH_2CH_2Br$ | $OCH_3$ | $OCH_3$ |
| J-5 | S | Q-1 ($R_7$=H, $R_8$=H) | H | 5-$CH(CH_3)(CH_2Cl)$ | $OCH_3$ | $OCH_3$ |
| J-6 | O | Q-1 ($R_7$=H, $R_8$=H) | H | 4-$NH_2$ | $OCH_3$ | $OCH_3$ |
| J-5 | S | Q-1 ($R_7$=H, $R_8$=H) | H | 4-$N(CH_3)_2$ | $OCH_3$ | $OCH_3$ |
| J-7 | S | Q-1 ($R_7$=H, $R_8$=H) | H | 5-Cl | $OCH_3$ | $OCH_3$ |
| J-5 | S | Q-1 ($R_7$=H, $R_8$=H) | H | 4-F | $OCH_3$ | $OCH_3$ |
| J-6 | S | Q-1 ($R_7$=H, $R_8$=H) | H | 5-I | $OCH_3$ | $OCH_3$ |
| J-5 | S | Q-1 ($R_7$=H, $R_8$=H) | H | 4-$NO_2$ | $OCH_3$ | $OCH_3$ |
| J-6 | S | Q-1 ($R_7$=H, $R_8$=H) | H | 5-$CF_3$ | $OCH_3$ | $OCH_3$ |
| J-5 | S | Q-1 ($R_7$=H, $R_8$=H) | H | H | $OCH_3$ | $CH_2CH_3$ |
| J-6 | S | Q-1 ($R_7$=H, $R_8$=H) | H | H | $OCH_3$ | $CH(CH_3)_2$ |
| J-5 | S | Q-1 ($R_7$=H, $R_8$=H) | H | H | $OCH_3$ | $(CH_2)_3CH_3$ |
| J-6 | O | Q-1 ($R_7$=H, $R_8$=H) | H | H | $OCH_3$ | $OCH_2CH_3$ |
| J-5 | S | Q-1 ($R_7$=H, $R_8$=H) | H | H | $OCH_3$ | $OCH(CH_3)_2$ |
| J-6 | S | Q-1 ($R_7$=H, $R_8$=H) | H | H | $OCH_3$ | $O(CH_2)_3CH_3$ |
| J-5 | S | Q-1 ($R_7$=H, $R_8$=H) | H | H | $OCH_2CH_2F$ | $OCH_3$ |
| J-6 | S | Q-1 ($R_7$=H, $R_8$=H) | H | H | $OCH_2CHF_2$ | $OCH_3$ |
| J-5 | S | Q-1 ($R_7$=H, $R_8$=H) | H | H | $OCH_2CF_3$ | $OCH_3$ |
| J-6 | S | Q-1 ($R_7$=H, $R_8$=H) | H | H | $OCH(CH_3)(CH_2Cl)$ | $OCH_3$ |
| J-5 | S | Q-1 ($R_7$=H, $R_8$=H) | H | H | $O(CH_2)_3CH_2Br$ | $OCH_3$ |
| J-6 | S | Q-1 ($R_7$=H, $R_8$=H) | H | H | $CH_2F$ | $OCH_3$ |
| J-5 | O | Q-1 ($R_7$=H, $R_8$=H) | H | H | $CH_2Cl$ | $OCH_3$ |
| J-6 | S | Q-1 ($R_7$=H, $R_8$=H) | H | H | $CH_2Br$ | $OCH_3$ |
| J-5 | S | Q-1 ($R_7$=H, $R_8$=H) | H | H | $CF_3$ | $OCH_3$ |

Table 2 (continued)

| J | $W_3$ | Q | R | $R_{1b}$ | X | Y |
|---|---|---|---|---|---|---|
| J-6 | S | Q-1 ($R_7$=H, $R_8$=H) | H | H | $CH_2CH_2Br$ | $OCH_3$ |
| J-7 | S | Q-1 ($R_7$=H, $R_8$=H) | H | H | $CH(CH_3)(CH_2Cl)$ | $OCH_3$ |
| J-5 | S | Q-1 ($R_7$=H, $R_8$=H) | H | H | $(CH_2)_3CH_2I$ | $OCH_3$ |
| J-6 | S | Q-1 ($R_7$=H, $R_8$=H) | H | H | $SCH_3$ | $OCH_3$ |
| J-5 | NH | Q-1 ($R_7$=H, $R_8$=H) | H | H | $SCH_2CH_3$ | $OCH_3$ |
| J-6 | S | Q-1 ($R_7$=H, $R_8$=H) | H | H | $SCH(CH_3)_2$ | $OCH_3$ |
| J-5 | S | Q-1 ($R_7$=H, $R_8$=H) | H | H | $S(CH_2)_3CH_3$ | $OCH_3$ |
| J-6 | O | Q-1 ($R_7$=H, $R_8$=H) | H | H | $SCHF_2$ | $OCH_3$ |
| J-5 | S | Q-1 ($R_7$=H, $R_8$=H) | H | H | $SCH_2CH_2Br$ | $OCH_3$ |
| J-6 | S | Q-1 ($R_7$=H, $R_8$=H) | H | H | $SCH(CH_3)(CH_2Cl)$ | $OCH_3$ |
| J-5 | S | Q-1 ($R_7$=H, $R_8$=H) | H | H | $S(CH_2)_3CH_2F$ | $OCH_3$ |
| J-6 | S | Q-1 ($R_7$=H, $R_8$=H) | H | H | $OCH_3$ | $CH_2OCH_3$ |
| J-7 | S | Q-1 ($R_7$=H, $R_8$=H) | H | H | $OCH_3$ | $CH_2CH_2OCH(CH_3)_2$ |
| J-5 | S | Q-1 ($R_7$=H, $R_8$=H) | H | H | $OCH_3$ | $CH(CH_3)(CH_2OCH_3)$ |
| J-6 | S | Q-1 ($R_7$=H, $R_8$=H) | H | H | $OCH_3$ | $(CH_2)_4CH_2OCH_2CH_3$ |
| J-5 | S | Q-1 ($R_7$=H, $R_8$=H) | H | H | $OCH_2O(CH_2)_3CH_3$ | $OCH_3$ |
| J-6 | S | Q-1 ($R_7$=H, $R_8$=H) | H | H | $OCH_2CH_2OCH(CH_3)_2$ | $OCH_3$ |
| J-5 | O | Q-1 ($R_7$=H, $R_8$=H) | H | H | $OCH(CH_3)(CH_2OCH_3)$ | $OCH_3$ |
| J-6 | S | Q-1 ($R_7$=H, $R_8$=H) | H | H | $O(CH_2)_4CH_2OCH_2CH_3$ | $OCH_3$ |
| J-5 | S | Q-1 ($R_7$=H, $R_8$=H) | H | H | $OCH_3$ | $NH_2$ |
| J-6 | S | Q-1 ($R_7$=H, $R_8$=H) | H | H | $OCH_3$ | $NHCH_3$ |
| J-5 | S | Q-1 ($R_7$=H, $R_8$=H) | H | H | $OCH_3$ | $NHCH_2CH_3$ |
| J-6 | S | Q-1 ($R_7$=H, $R_8$=H) | H | H | $OCH_3$ | $NHCH(CH_3)_2$ |
| J-7 | S | Q-1 ($R_7$=H, $R_8$=H) | H | H | $OCH_3$ | $N(CH_3)_2$ |
| J-5 | S | Q-1 ($R_7$=H, $R_8$=H) | H | H | $OCH_3$ | $N(CH_3)(CH_2CH_3)$ |
| J-6 | S | Q-1 ($R_7$=H, $R_8$=H) | H | H | $OCH_3$ | $N(CH_3)(CH(CH_3)_2)$ |
| J-5 | S | Q-1 ($R_7$=H, $R_8$=H) | H | H | $OCH_3$ | $(CH_2)_4CH_2OCH_2CH_3$ |
| J-6 | O | Q-1 ($R_7$=H, $R_8$=H) | H | H | H | $OCH_3$ |
| J-5 | S | Q-1 ($R_7$=H, $R_8$=H) | H | H | $OCH_3$ | $OCH_2CH{=}CH_2$ |
| J-6 | S | Q-1 ($R_7$=H, $R_8$=H) | H | H | $OCH_3$ | $OCH_2C(CH_3){=}CH_2$ |
| J-5 | S | Q-1 ($R_7$=H, $R_8$=H) | H | H | $OCH_3$ | $OCH_2C{\equiv}CH$ |
| J-6 | S | Q-1 ($R_7$=H, $R_8$=H) | H | H | $OCH_3$ | $OCH_2C{\equiv}CCH_3$ |
| J-5 | S | Q-1 ($R_7$=H, $R_8$=H) | H | H | $OCH_3$ | $CH_2S(CH_2)_3CH_3$ |

Table 2 (continued)

| J | $W_3$ | Q | R | $R_{1b}$ | X | Y |
|---|---|---|---|---|---|---|
| J-6 | S | Q-1 ($R_7$=H, $R_8$=H) | H | H | $OCH_3$ | $CH_2CH_2SCH(CH_3)_2$ |
| J-7 | S | Q-1 ($R_7$=H, $R_8$=H) | H | H | $OCH_3$ | $CH(CH_3)(CH_2SCH_3)$ |
| J-5 | S | Q-1 ($R_7$=H, $R_8$=H) | H | H | $OCH_3$ | $(CH_2)_4CH_2SCH_2CH_3$ |
| J-6 | S | Q-1 ($R_7$=H, $R_8$=H) | H | H | $OCH_3$ | cyclopropyl |
| J-5 | O | Q-1 ($R_7$=H, $R_8$=H) | H | H | $OCH_3$ | 2-methylcyclopropyl |
| J-6 | S | Q-1 ($R_7$=H, $R_8$=H) | H | H | $OCH_3$ | cyclopentyl |
| J-5 | S | Q-1 ($R_7$=H, $R_8$=H) | H | H | $OCH_3$ | $C\equiv CH$ |
| J-6 | S | Q-1 ($R_7$=H, $R_8$=H) | H | H | $OCH_3$ | $C\equiv CCH_3$ |
| J-5 | S | Q-1 ($R_7$=H, $R_8$=H) | H | H | $OCH_3$ | -CHO |
| J-6 | S | Q-1 ($R_7$=H, $R_8$=H) | H | H | $OCH_3$ | $-COCH_3$ |
| J-5 | S | Q-1 ($R_7$=H, $R_8$=H) | H | H | $OCH_3$ | $-CH(OCH_3)_2$ |
| J-6 | S | Q-1 ($R_7$=H, $R_8$=H) | H | H | $OCH_3$ | $-CH(SCH_3)(OCH_2CH_3)$ |
| J-7 | S | Q-1 ($R_7$=H, $R_8$=H) | H | H | $OCH_3$ | $-C(CH_3)(SCH_3)_2$ |
| J-5 | S | Q-1 ($R_7$=H, $R_8$=H) | H | H | $OCH_3$ | $-CH(SCH_2CH_3)_2$ |
| J-6 | O | Q-1 ($R_7$=H, $R_8$=H) | H | H | $OCH_3$ | 1,3-dioxolan-2-yl |
| J-5 | S | Q-1 ($R_7$=H, $R_8$=H) | H | H | $OCH_3$ | 2-methyl-1,3-oxa-thiolan-2-yl |
| J-6 | S | Q-1 ($R_7$=H, $R_8$=H) | H | H | $OCH_3$ | 1,3-oxathian-2-yl |
| J-5 | S | Q-1 ($R_7$=H, $R_8$=H) | H | H | $OCH_3$ | 2-methyl-1,3-dithian-2-yl |
| J-6 | S | Q-1 ($R_7$=H, $R_8$=H) | H | H | $OCH_3$ | 4-methyl-1,3-dioxolan-2-yl |
| J-5 | S | Q-1 ($R_7$=H, $R_8$=H) | H | H | $OCH_3$ | 4-methyl-1,3-oxathiolan-2-yl |
| J-6 | S | Q-1 ($R_7$=H, $R_8$=H) | H | H | $OCH_3$ | 2,4-dimethyl-1,3-dithiolan-2-yl |
| J-5 | S | Q-1 ($R_7$=H, $R_8$=H) | H | H | $OCH_3$ | $N(OCH_3)(CH_3)_2$ |
| J-6 | S | Q-1 ($R_7$=H, $R_8$=$CH_3$) | H | H | $OCH_3$ | $OCH_3$ |
| J-5 | S | Q-1 ($R_7$=H, $R_8$=$CH_3$) | H | H | $CH_3$ | $CH_3$ |
| J-6 | O | Q-1 ($R_7$=H, $R_8$=$CH_3$) | H | H | $CH_3$ | $OCH_3$ |
| J-7 | S | Q-1 ($R_7$=H, $R_8$=$CH_2CH_3$) | H | H | $OCH_3$ | $OCH_3$ |
| J-5 | S | Q-1 ($R_7$=H, $R_8$=$CH(CH_3)_2$) | H | H | $OCH_3$ | $OCH_3$ |
| J-6 | S | Q-1 ($R_7$=H, $R_8$=$(CH_2)_3CH_3$) | H | H | $OCH_3$ | $OCH_3$ |
| J-5 | S | Q-1 ($R_7$=$CH_3$, $R_8$=$CH_3$) | H | H | $OCH_3$ | $OCH_3$ |

Table 2 (continued)

| J | $W_3$ | Q | R | $R_{1b}$ | X | Y |
|---|---|---|---|---|---|---|
| J-6 | S | Q-2 ($R_7$=H, $R_8$=H) | H | H | $OCH_3$ | $OCH_3$ |
| J-5 | S | Q-2 ($R_7$=H, $R_8$=H) | $CH_3$ | H | $OCH_3$ | $OCH_3$ |
| J-6 | S | Q-2 ($R_7$=H, $R_8$=$CH_3$) | H | 5-$CH_3$ | $OCH_3$ | $OCH_3$ |
| J-5 | S | Q-3 ($R_7$=H, $R_8$=H) | H | H | $OCH_3$ | $OCH_3$ |
| J-6 | S | Q-3 ($R_7$=$C_2H_5$, $R_8$=H) | H | H | $OCH_3$ | $CH_3$ |
| J-5 | O | Q-3 ($R_7$=$CH_3$, $R_8$=H) | H | 5-Cl | $OCH_3$ | $CH_3$ |
| J-6 | S | Q-4 ($R_7$=H, $R_8$=H, $R_3$=$CH_3$) | H | H | $CH_3$ | $OCH_3$ |
| J-7 | S | Q-4 ($R_7$=H, $R_8$=H, $R_3$=$C_2H_5$) | H | H | $CH_3$ | $OCH_3$ |
| J-5 | S | Q-4 ($R_7$=H, $R_8$=H, $R_3$=i-$C_3H_7$) | H | 5-$CH_3$ | $OCH_3$ | $OCH_3$ |
| J-6 | S | Q-5 ($R_7$-H, $R_8$=H, $R_3$=H) | H | H | $OCH_3$ | $OCH_3$ |
| J-5 | S | Q-5 ($R_7$=H, $R_8$=H, $R_3$=$CH_3$) | H | H | $CH_3$ | $OCH_3$ |
| J-6 | S | Q-5 ($R_7$=H, $R_8$=$CH_3$, $R_3$=$CH_3$) | H | H | $CH_3$ | $OCH_3$ |
| J-5 | S | Q-6 ($R_7$=$CH_3$, $R_8$=H, $R_3$=$CH_3$) | H | H | $CH_3$ | $OCH_3$ |
| J-6 | S | Q-6 ($R_7$-H, $R_8$=H, $R_3$=$C_2H_5$) | $CH_3$ | H | $CH_3$ | $OCH_3$ |
| J-5 | S | Q-6 ($R_7$=$C_2H_5$, $R_8$=H, $R_3$=H) | H | H | $OCH_3$ | $OCH_3$ |
| J-7 | S | Q-8 ($R_7$=H, $R_8$=H, $R_2$=H) | H | H | $OCH_3$ | $OCH_3$ |
| J-5 | S | Q-8 ($R_7$=$CH_3$, $R_8$=H, $R_2$=$OCH_3$) | H | H | $OCH_3$ | $OCH_3$ |
| J-6 | S | Q-8 ($R_7$=H, $R_8$=H, $R_2$=$OCH(CH_3)CH_2CH_3$) | H | 5-Cl | $OCH_3$ | $OCH_3$ |
| J-5 | S | Q-9 ($R_7$=H, $R_8$=H, $R_2$=H) | H | H | $OCH_3$ | $OCH_3$ |
| J-6 | S | Q-9 ($R_7$=$CH_3$, $R_8$=H, $R_2$=$CH_3$) | $CH_3$ | H | $OCH_3$ | $OCH_3$ |
| J-5 | S | Q-9 ($R_7$=H, $R_8$=H, $R_2$=Cl) | H | 5-$CH_3$ | $OCH_3$ | $OCH_3$ |
| J-6 | S | Q-10 ($R_7$=H, $R_8$=H) | H | H | $OCH_3$ | $OCH_3$ |
| J-5 | O | Q-10 ($R_7$=$CH_3$, $R_8$=H) | H | H | $OCH_3$ | $CH_3$ |
| J-6 | S | Q-10 ($R_7$=H, $R_8$=$CH_3$) | H | 5-Cl | $OCH_3$ | $CH_3$ |
| J-5 | S | Q-11 ($R_7$=H, $R_8$=H) | H | H | $CH_3$ | $OCH_3$ |
| J-6 | S | Q-11 ($R_7$=n-$C_4H_9$, $R_8$=H) | H | H | $CH_3$ | $OCH_3$ |

Table 2 (continued)

| J | $W_3$ | Q | R | $R_{1b}$ | X | Y |
|---|---|---|---|---|---|---|
| J-7 | S | Q-11 ($R_7=CH_3$, $R_8=CH_3$) | H | 5-$CH_3$ | $OCH_3$ | $OCH_3$ |
| J-5 | S | Q-12 ($R_7=H$, $R_8=H$) | H | H | $OCH_3$ | $OCH_3$ |
| J-6 | S | Q-12 ($R_7=CH_3$, $R_8=H$) | H | H | $CH_3$ | $OCH_3$ |
| J-5 | S | Q-12 ($R_7=H$, $R_8=C_2H_5$) | H | H | $CH_3$ | $OCH_3$ |
| J-6 | S | Q-13 ($R_7=H$, $R_8=H$, $R_3=H$) | H | H | $CH_3$ | $OCH_3$ |
| J-5 | S | Q-13 ($R_7=H$, $R_8=H$, $R_3=CH_3$) | $CH_3$ | H | $CH_3$ | $OCH_3$ |
| J-6 | O | Q-13 ($R_7=CH_3$, $R_8=H$, $R_3=CH_3$) | H | H | $OCH_3$ | $OCH_3$ |
| J-5 | S | Q-14 ($R_7=H$, $R_8=H$, $R_3=CH_3$) | H | H | $OCH_3$ | $OCH_3$ |
| J-6 | S | Q-14 ($R_7=CH_3$, $R_8=H$, $R_3=C_2H_5$) | H | H | $OCH_3$ | $OCH_3$ |
| J-5 | S | Q-14 ($R_7=H$, $R_8=CH_3$, $R_3=CH_3$) | H | 5-$CH_3$ | $OCH_3$ | $OCH_3$ |
| J-6 | S | Q-15 ($R_7=H$, $R_8=H$, $R_3=H$) | H | H | $OCH_3$ | $OCH_3$ |
| J-7 | S | Q-15 ($R_7=C_2H_5$, $R_8=H$, $R_3=CH_3$) | H | H | $OCH_3$ | $OCH_3$ |
| J-5 | S | Q-15 ($R_7=CH_3$, $R_8=CH_3$, $R_3=CH_3$) | H | 5-Cl | $OCH_3$ | $OCH_3$ |
| J-5 | O | Q-17 ($R_7=H$, $R_8=H$, $R_2=H$) | H | H | $OCH_3$ | $OCH_3$ |
| J-6 | S | Q-17 ($R_7=CH_3$, $R_8=H$, $R_2=CH_3$) | H | H | $OCH_3$ | $CH_3$ |
| J-5 | S | Q-17 ($R_7=H$, $R_8=H$, $R_2=C_2H_5$) | H | 5-Cl | $OCH_3$ | $CH_3$ |
| J-6 | S | Q-18 ($R_7=H$, $R_8=H$, $R_2=H$) | H | H | $CH_3$ | $OCH_3$ |
| J-5 | S | Q-18 ($R_7=H$, $R_8=OC_2H_5$, $R_2=H$) | H | H | $CH_3$ | $OCH_3$ |
| J-6 | S | Q-18 ($R_7=CH_3$, $R_8=OCH(CH_3)_2$, $R_2=CH_3$) | H | 5-$CH_3$ | $OCH_3$ | $OCH_3$ |
| J-7 | S | Q-19 ($R_7=H$, $R_8=H$) | H | H | $OCH_3$ | $OCH_3$ |
| J-5 | S | Q-19 ($R_7=CH_3$, $R_8=H$) | H | H | $CH_3$ | $OCH_3$ |
| J-6 | $NCH_3$ | Q-19 ($R_7=CH_3$, $R_8=CH_3$) | H | H | $CH_3$ | $OCH_3$ |
| J-5 | S | Q-20 ($R_7=H$, $R_8=H$) | H | H | $CH_3$ | $OCH_3$ |
| J-6 | O | Q-20 ($R_7=CH_3$, $R_8=H$) | $CH_3$ | H | $CH_3$ | $OCH_3$ |
| J-5 | S | Q-20 ($R_7=H$, $R_8=CH_3$) | H | H | $OCH_3$ | $OCH_3$ |
| J-6 | S | Q-21 ($R_7=H$, $R_8=H$) | H | H | $OCH_3$ | $OCH_3$ |
| J-5 | S | Q-21 ($R_7=CH_3$, $R_8=H$) | H | H | $OCH_3$ | $OCH_3$ |
| J-6 | S | Q-21 ($R_7=CH_3$, $R_8=CH_3$) | H | 5-$CH_3$ | $OCH_3$ | $OCH_3$ |
| J-5 | S | Q-22 ($R_7=H$, $R_8=H$, $R_3=H$) | H | H | $OCH_3$ | $OCH_3$ |

Table 2 (continued)

| J | $W_3$ | Q | R | $R_{1b}$ | X | Y |
|---|---|---|---|---|---|---|
| J-6 | S | Q-22 ($R_7$=H, $R_8$=H, $R_3$=$CH_3$) | H | H | $OCH_3$ | $OCH_3$ |
| J-7 | S | Q-22 ($R_7$=$CH_3$, $R_8$=$CH_3$, $R_3$=$C_2H_5$) | H | 5-Cl | $OCH_3$ | $OCH_3$ |
| J-5 | S | Q-23 ($R_7$=H, $R_8$=H, $R_3$=H) | H | H | $OCH_3$ | $OCH_3$ |
| J-6 | S | Q-23 ($R_7$=H, $R_8$=H, $R_3$=$CH_3$) | $CH_3$ | H | $OCH_3$ | $OCH_3$ |
| J-5 | O | Q-23 ($R_7$=$CH_3$, $R_8$=H, $R_3$=n-$C_4H_9$) | H | 5-$CH_3$ | $OCH_3$ | $OCH_3$ |
| J-6 | S | Q-24 ($R_7$=H, $R_8$=H, $R_3$=$CH_3$) | H | H | $OCH_3$ | $OCH_3$ |
| J-5 | S | Q-24 ($R_7$=$CH_3$, $R_8$=$CH_3$, $R_3$=$C_2H_5$) | H | H | $OCH_3$ | $CH_3$ |
| J-6 | S | Q-24 ($R_7$=$CH_3$, $R_8$=$CH_3$, $R_3$=i-$C_3H_7$) | H | 5-Cl | $OCH_3$ | $CH_3$ |
| J-5 | S | Q-25 ($R_7$=H, $R_8$=H, $R_9$=H) | H | H | $CH_3$ | $OCH_3$ |
| J-6 | S | Q-25 ($R_7$=$CH_3$, $R_8$=H, $R_9$=H) | H | H | $CH_3$ | $OCH_3$ |
| J-5 | S | Q-25 ($R_7$=H, $R_8$=H, $R_9$=$C_2H_5$) | H | 5-$CH_3$ | $OCH_3$ | $OCH_3$ |
| J-6 | S | Q-26 ($R_7$=H, $R_8$=H) | H | H | $OCH_3$ | $OCH_3$ |
| J-7 | S | Q-26 ($R_7$=$CH_3$, $R_8$=H) | H | H | $CH_3$ | $OCH_3$ |
| J-5 | S | Q-26 ($R_7$=$CH_3$, $R_8$=$CH_3$) | H | H | $CH_3$ | $OCH_3$ |
| J-6 | O | Q-27 ($R_7$=H, $R_8$=H) | H | H | $CH_3$ | $OCH_3$ |
| J-5 | S | Q-27 ($R_7$=$CH_3$, $R_8$=H) | $CH_3$ | H | $CH_3$ | $OCH_3$ |
| J-6 | S | Q-27 ($R_7$=H, $R_8$=$CH_3$) | H | H | $OCH_3$ | $OCH_3$ |
| J-5 | S | Q-28 ($R_7$=H, $R_8$=H) | H | H | $OCH_3$ | $OCH_3$ |
| J-6 | S | Q-28 ($R_7$=$CH_3$, $R_8$=H) | H | H | $OCH_3$ | $OCH_3$ |
| J-5 | S | Q-28 ($R_7$=$CH_3$, $R_8$=$CH_3$) | H | 5-$CH_3$ | $OCH_3$ | $OCH_3$ |
| J-6 | S | Q-29 ($R_7$=H, $R_8$=H) | H | H | $OCH_3$ | $OCH_3$ |
| J-5 | S | Q-29 ($R_7$=$CH_3$, $R_8$=H) | H | H | $OCH_3$ | $OCH_3$ |
| J-6 | S | Q-29 ($R_7$=H, $R_8$=$CH_3$) | H | 5-Cl | $OCH_3$ | $OCH_3$ |
| J-7 | S | Q-30 ($R_7$=H, $R_8$=H) | H | H | $OCH_3$ | $OCH_3$ |
| J-5 | O | Q-30 ($R_7$=$CH_3$, $R_8$=$CH_3$) | $CH_3$ | H | $OCH_3$ | $CH_3$ |
| J-6 | S | Q-30 ($R_7$=$C_2H_5$, $R_8$=H) | H | 5-$CH_3$ | $OCH_3$ | $OCH_3$ |
| J-5 | S | Q-31 ($R_7$=H, $R_8$=H) | H | H | $OCH_3$ | $OCH_3$ |
| J-6 | S | Q-31 ($R_7$=i-$C_3H_7$, $R_8$=H) | H | H | $OCH_3$ | $CH_3$ |
| J-5 | S | Q-31 ($R_7$=H, $R_8$=n-$C_4H_9$) | H | 5-Cl | $OCH_3$ | $CH_3$ |
| J-6 | S | Q-32 ($R_7$=H, $R_8$=H, $R_3$=H) | H | H | $CH_3$ | $OCH_3$ |
| J-5 | S | Q-32 ($R_7$=$CH_3$, $R_8$=H, $R_3$=$CH_3$) | H | H | $CH_3$ | $OCH_3$ |
| J-6 | S | Q-32 ($R_7$=H, $R_8$=$CH_3$, $R_3$=$C_2H_5$) | H | 5-$CH_3$ | $OCH_3$ | $OCH_3$ |
| J-5 | S | Q-33 ($R_7$=H, $R_8$=H, $R_8$=$CH_3$) | H | H | $OCH_3$ | $OCH_3$ |

Table 2 (continued)

| J | $W_3$ | Q | R | $R_{1b}$ | X | Y | m.p.(°) |
|---|---|---|---|---|---|---|---|
| J-6 | S | Q-33 ($R_7$=$CH_3$, $R_8$=$CH_3$, $R_3$=i-$C_3H_7$) | H | H | $CH_3$ | $OCH_3$ | |
| J-7 | O | Q-33 ($R_7$=$C_2H_5$, $R_8$=H, $R_3$=n-$C_4H_9$) | H | H | $CH_3$ | $OCH_3$ | |
| J-5 | S | Q-34 ($R_7$=H, $R_8$=H, $R_3$=H) | H | H | $CH_3$ | $OCH_3$ | |
| J-6 | S | Q-34 ($R_7$=i-$C_3H_7$, $R_8$=H, $R_3$=$CH_3$) | $CH_3$ | H | $CH_3$ | $OCH_3$ | |
| J-5 | S | Q-34 ($R_7$=H, $R_8$=n-$C_4H_9$, $R_3$=$CH_3$) | H | H | $OCH_3$ | $OCH_3$ | |
| J-5 | S | Q-36 ($R_7$=H, $R_8$=H, $R_2$=H) | H | H | $OCH_3$ | $OCH_3$ | |
| J-6 | S | Q-36 ($R_7$=$CH_3$, $R_8$=H, $R_2$=$CH_3$) | H | H | $OCH_3$ | $OCH_3$ | |
| J-5 | S | Q-36 ($R_7$=H, $R_8$=H, $R_2$=$C_2H_5$) | H | 5-Cl | $OCH_3$ | $OCH_3$ | |
| J-6 | O | Q-37 ($R_7$=H, $R_8$=H, $R_2$=H) | H | H | $OCH_3$ | $OCH_3$ | |
| J-6 | S | Q-37 ($R_7$=H, $R_8$=H, $R_2$=H) | H | H | $OCH_3$ | $CH_3$ | 162-165 |
| J-7 | S | Q-37 ($R_7$=H, $R_8$=H, $R_2$=i-$C_3H_7$) | $CH_3$ | H | $OCH_3$ | $OCH_3$ | |
| J-5 | S | Q-37 ($R_7$=H, $R_8$=H, $R_2$=n-$C_4H_9$) | H | 5-$CH_3$ | $OCH_3$ | $OCH_3$ | |
| J-6 | S | Q-38 ($R_7$=H, $R_8$=H, $R_2$=$OCF_2H$) | H | H | $OCH_3$ | $OCH_3$ | |
| J-5 | S | Q-38 ($R_7$=$CH_3$, $R_8$=H, $R_2$=$OCH_2CH_2F$) | H | H | $OCH_3$ | $CH_3$ | |
| J-6 | S | Q-38 ($R_7$=H, $R_8$=H, $R_2$=Cl) | H | 5-Cl | $OCH_3$ | $CH_3$ | |
| J-5 | S | Q-39 ($R_7$=H, $R_8$=H, $R_9$=H) | H | H | $CH_3$ | $OCH_3$ | |
| J-6 | S | Q-39 ($R_7$=$C_2H_5$, $R_8$=H, $R_9$=H) | H | H | $CH_3$ | $OCH_3$ | |
| J-5 | S | Q-39 ($R_7$=H, $R_8$=H, $R_9$=$CH_3$) | H | 5-$CH_3$ | $OCH_3$ | $OCH_3$ | |
| J-6 | S | Q-40 ($R_7$=H, $R_8$=H) | H | H | $OCH_3$ | $OCH_3$ | |
| J-5 | O | Q-40 ($R_7$=$CH_3$, $R_8$=H) | H | H | $CH_3$ | $OCH_3$ | |
| J-6 | S | Q-40 ($R_7$=H, $R_8$=$CH_3$) | H | H | $CH_3$ | $OCH_3$ | |
| J-7 | S | Q-41 ($R_7$=H, $R_8$=H) | H | H | $CH_3$ | $OCH_3$ | |
| J-5 | S | Q-41 ($R_7$=$CH_3$, $R_8$=$CH_3$) | $CH_3$ | H | $CH_3$ | $OCH_3$ | |
| J-6 | S | Q-41 ($R_7$=H, $R_8$=$C_2H_5$) | H | H | $OCH_3$ | $OCH_3$ | |
| J-5 | S | Q-42 ($R_7$=H, $R_8$=H) | H | H | $OCH_3$ | $OCH_3$ | |
| J-6 | S | Q-42 ($R_7$=$CH_3$, $R_8$=$CH_3$) | H | H | $OCH_3$ | $OCH_3$ | |
| J-5 | S | Q-42 ($R_7$=$C_2H_5$, $R_8$=$C_2H_5$) | H | 5-$CH_3$ | $OCH_3$ | $OCH_3$ | |
| J-6 | S | Q-43 ($R_7$=H, $R_8$=H, $R_3$=H) | H | H | $OCH_3$ | $OCH_3$ | |
| J-5 | S | Q-43 ($R_7$=H, $R_8$=H, $R_3$=$CH_3$) | H | H | $OCH_3$ | $OCH_3$ | |
| J-6 | O | Q-43 ($R_7$=$CH_3$, $R_8$=H, $R_3$=$CH_3$) | H | 5-Cl | $OCH_3$ | $OCH_3$ | |
| J-5 | S | Q-44 ($R_7$=H, $R_8$=H, $R_3$=$CH_3$) | H | H | $OCH_3$ | $OCH_3$ | |

Table 2 (continued)

| J | $W_3$ | Q | R | $R_{1b}$ | X | Y |
|---|---|---|---|---|---|---|
| J-6 | S | Q-44 ($R_7$=$CH_3$, $R_8$=H, $R_3$=$C_2H_5$) | $CH_3$ | H | $OCH_3$ | $CH_3$ |
| J-7 | S | Q-44 ($R_7$=H, $R_8$=$CH_3$, $R_3$=$CH_3$) | H | 5-$CH_3$ | $OCH_3$ | $OCH_3$ |
| J-5 | S | Q-45 ($R_7$=H, $R_8$=H, $R_3$=H) | H | H | $OCH_3$ | $OCH_3$ |
| J-6 | S | Q-45 ($R_7$=$C_2H_5$, $R_8$=H, $R_3$=$CH_3$) | H | H | $OCH_3$ | $CH_3$ |
| J-5 | S | Q-45 ($R_7$=$CH_3$, $R_8$=$CH_3$, $R_3$=$CH_3$) | H | 5-Cl | $OCH_3$ | $CH_3$ |
| J-5 | O | Q-47 ($R_7$=H, $R_8$=H, $R_2$=H) | H | H | $OCH_3$ | $OCH_3$ |
| J-6 | S | Q-47 ($R_7$=H, $R_8$=$CH_3$, $R_2$=$CH_3$) | H | H | $CH_3$ | $OCH_3$ |
| J-5 | S | Q-47 ($R_7$=H, $R_8$=H, $R_2$=$C_2H_5$) | H | H | $CH_3$ | $OCH_3$ |
| J-6 | S | Q-48 ($R_7$=H, $R_8$=H, $R_2$=H) | H | H | $CH_3$ | $OCH_3$ |
| J-7 | NH | Q-48 ($R_7$=$CH_3$, $R_8$=$CH_3$, $R_2$=$CH_3$) | $CH_3$ | H | $CH_3$ | $OCH_3$ |
| J-5 | S | Q-48 ($R_7$=H, $R_8$=$C_2H_5$, $R_2$=$C_2H_5$) | H | H | $OCH_3$ | $OCH_3$ |
| J-6 | S | Q-49 ($R_7$=H, $R_8$=H, $R_2$=H) | H | H | $OCH_3$ | $OCH_3$ |
| J-5 | S | Q-49 ($R_7$=$CH_3$, $R_8$=$CH_3$, $R_2$=H) | H | H | $OCH_3$ | $OCH_3$ |
| J-6 | S | Q-49 ($R_7$=$CH_3$, $R_8$=$CH_3$, $R_2$=$CH_3$) | H | 5-$CH_3$ | $OCH_3$ | $OCH_3$ |
| J-5 | S | Q-50 ($R_7$=H, $R_8$=H, $R_2$=H) | H | H | $OCH_3$ | $OCH_3$ |
| J-6 | O | Q-50 ($R_7$=$CH_3$, $R_8$=$CH_3$, $R_2$=H) | H | H | $OCH_3$ | $OCH_3$ |
| J-5 | S | Q-50 ($R_7$=$CH_3$, $R_8$=$CH_3$, $R_2$=$CH_3$) | H | 5-Cl | $OCH_3$ | $OCH_3$ |
| J-6 | S | Q-51 ($R_7$=H, $R_8$=H, $R_9$=H) | H | H | $OCH_3$ | $OCH_3$ |
| J-5 | S | Q-51 ($R_7$=H, $R_8$=H, $R_9$=$CH_3$) | $CH_3$ | H | $OCH_3$ | $OCH_3$ |
| J-6 | S | Q-51 ($R_7$=H, $R_8$=H, $R_9$=s-$C_4H_9$) | H | 5-$CH_3$ | $OCH_3$ | $OCH_3$ |
| J-7 | S | Q-52 ($R_7$=H, $R_8$=H, $R_9$=H) | H | H | $OCH_3$ | $OCH_3$ |
| J-5 | S | Q-52 ($R_7$=$CH_3$, $R_8$=H, $R_9$=H) | H | H | $OCH_3$ | $CH_3$ |
| J-6 | S | Q-52 ($R_7$=H, $R_8$=H, $R_9$=$CH_3$) | H | 5-Cl | $OCH_3$ | $CH_3$ |
| J-5 | S | Q-53 ($R_7$=H, $R_8$=H, $R_9$=H) | H | H | $CH_3$ | $OCH_3$ |
| J-6 | S | Q-53 ($R_7$=$CH_3$, $R_8$=$CH_3$, $R_9$=$CH_3$) | H | H | $CH_3$ | $OCH_3$ |
| J-5 | O | Q-53 ($R_7$=H, $R_8$=H, $R_9$=$CH_3$) | H | 5-$CH_3$ | $OCH_3$ | $OCH_3$ |
| J-6 | S | Q-54 ($R_7$=H, $R_8$=H, $R_9$=H) | H | H | $OCH_3$ | $OCH_3$ |
| J-5 | S | Q-54 ($R_7$=$CH_3$, $R_8$=H, $R_9$=H) | H | H | $CH_3$ | $OCH_3$ |
| J-6 | S | Q-54 ($R_7$=H, $R_8$=H, $R_9$=i-$C_3H_7$) | H | H | $CH_3$ | $OCH_3$ |
| J-5 | S | Q-55 ($R_7$=H, $R_8$=H, $R_9$=H, $R_3$=H) | H | H | $CH_3$ | $OCH_3$ |

Table 2 (continued)

| J | $W_3$ | Q | R | $R_{1b}$ | X | Y | m.p.(°C) |
|---|---|---|---|---|---|---|---|
| J-6 | S | Q-55 ($R_7$=H, $R_8$=H, $R_9$=$CH_3$, $R_3$=$CH_3$) | $CH_3$ | H | $CH_3$ | $OCH_3$ | |
| J-7 | S | Q-55 ($R_7$=H, $R_8$=H, $R_9$=s-$C_4H_9$, $R_3$=H) | H | H | $OCH_3$ | $OCH_3$ | |
| J-5 | S | Q-56 ($R_7$=H, $R_8$=H, $R_9$=H, $R_3$=$C_2H_5$) | H | H | $OCH_3$ | $OCH_3$ | |
| J-6 | S | Q-56 ($R_7$=$CH_3$, $R_8$=H, $R_9$=H, $R_3$=H) | H | H | $OCH_3$ | $OCH_3$ | |
| J-5 | S | Q-56 ($R_7$=H, $R_8$=H, $R_9$=$CH_3$, $R_3$=$CH_3$) | H | 5-$CH_3$ | $OCH_3$ | $OCH_3$ | |
| J-6 | O | Q-57 ($R_7$=H, $R_8$=H, $R_9$=H, $R_3$=i-$C_3H_7$) | H | H | $OCH_3$ | $OCH_3$ | |
| J-5 | S | Q-57 ($R_7$=$CH_3$, $R_8$=$CH_3$, $R_9$=$CH_3$, $R_3$=H) | H | H | $OCH_3$ | $OCH_3$ | |
| J-6 | S | Q-57 ($R_7$=H, $R_8$=H, $R_9$=$CH_3$, $R_3$=$CH_3$) | H | 5-Cl | $OCH_3$ | $OCH_3$ | |
| J-5 | S | Q-58 ($R_7$=H, $R_8$=H, $R_9$=H, $R_3$=H) | H | H | $OCH_3$ | $OCH_3$ | |
| J-6 | S | Q-58 ($R_7$=$CH_3$, $R_8$=H, $R_9$=H, $R_3$=$CH_3$) | $CH_3$ | H | $OCH_3$ | $CH_3$ | |
| J-5 | S | Q-58 ($R_7$=H, $R_8$=H, $R_9$=i-$C_3H_7$, $R_3$=$CH_3$) | H | 5-$CH_3$ | $OCH_3$ | $OCH_3$ | |
| J-6 | S | Q-60 ($R_7$=H, $R_8$=H, $R_9$=H, $R_2$=H) | H | H | $CH_3$ | $OCH_3$ | 118-128 |
| J-6 | S | Q-60 ($R_7$=H, $R_8$=H, $R_9$=H, $R_2$=H) | H | H | $OCH_3$ | $OCH_3$ | 120-133 |
| J-5 | O | Q-60 ($R_7$=$CH_3$, $R_8$=H, $R_9$=H, $R_2$=$O(CH_2)_3CH_2Br$) | H | H | $CH_3$ | $OCH_3$ | |
| J-6 | S | Q-60 ($R_7$=H, $R_8$=H, $R_9$=H, $R_2$=Br) | H | 5-$CH_3$ | $OCH_3$ | $OCH_3$ | |
| J-5 | S | Q-61 ($R_7$=H, $R_8$=H, $R_9$=H, $R_2$=H) | H | H | $OCH_3$ | $OCH_3$ | |
| J-6 | S | Q-61 ($R_7$=$CH_3$, $R_8$=H, $R_9$=H, $R_2$=$OCH_2F$) | H | H | $CH_3$ | $OCH_3$ | |
| J-5 | S | Q-61 ($R_7$=H, $R_8$=$CH_3$, $R_9$=H, $R_2$=$O(CH_2)_3CH_3$) | H | H | $CH_3$ | $OCH_3$ | |
| J-6 | S | Q-62 ($R_7$=H, $R_8$=H, $R_9$=H, $R_2$=H) | H | H | $CH_3$ | $OCH_3$ | |
| J-5 | S | Q-62 ($R_7$=$CH_3$, $R_8$=H, $R_9$=H, $R_2$=$CH_3$) | $CH_3$ | H | $CH_3$ | $OCH_3$ | |
| J-6 | S | Q-62 ($R_7$=Cl, $R_8$=H, $R_9$=H, $R_2$=H) | H | H | $OCH_3$ | $OCH_3$ | |
| J-7 | S | Q-63 ($R_7$=H, $R_8$=H, $R_9$=H) | H | H | $OCH_3$ | $OCH_3$ | |
| J-5 | S | Q-63 ($R_7$=$CH_3$, $R_8$=H, $R_9$=H) | H | H | $OCH_3$ | $OCH_3$ | |
| J-6 | O | Q-63 ($R_7$=H, $R_8$=H, $R_9$=$CH_3$) | H | 5-$CH_3$ | $OCH_3$ | $OCH_3$ | |
| J-5 | S | Q-64 ($R_7$=H, $R_8$=H, $R_9$=H) | H | H | $OCH_3$ | $OCH_3$ | |
| J-6 | S | Q-64 ($R_7$=$C_2H_5$, $R_8$=H, $R_9$=H) | H | H | $OCH_3$ | $OCH_3$ | |
| J-5 | S | Q-64 ($R_7$=H, $R_8$=H, $R_9$=$C_2H_5$) | H | 5-Cl | $OCH_3$ | $OCH_3$ | |
| J-6 | S | Q-65 ($R_7$=H, $R_8$=H, $R_9$=H) | H | H | $OCH_3$ | $OCH_3$ | |
| J-5 | S | Q-65 ($R_7$=n-$C_4H_9$, $R_8$=H, $R_9$=H) | $CH_3$ | H | $OCH_3$ | $CH_3$ | |
| J-6 | S | Q-65 ($R_7$=H, $R_8$=$CH_3$, $R_9$=i-$C_3H_7$) | H | 5-$CH_3$ | $OCH_3$ | $OCH_3$ | |
| J-5 | S | Q-66 ($R_7$=H, $R_8$=H, $R_9$=H) | H | H | $OCH_3$ | $OCH_3$ | |

Table 2 (continued)

| J | $W_3$ | Q | R | $R_{1b}$ | X | Y |
|---|---|---|---|---|---|---|
| J-6 | S | Q-66 ($R_7$=i-$C_3H_7$, $R_8$=H, $R_9$=H) | H | H | $OCH_3$ | $CH_3$ |
| J-7 | S | Q-66 ($R_7$=H, $R_8$=$C_2H_5$, $R_9$=$CH_3$) | H | 5-Cl | $OCH_3$ | $CH_3$ |
| J-5 | S | Q-67 ($R_7$=H, $R_8$=H, $R_9$=H, $R_3$=H) | H | H | $CH_3$ | $OCH_3$ |
| J-6 | S | Q-67 ($R_7$=$CH_3$, $R_8$=H, $R_9$=H, $R_3$=$CH_3$) | H | H | $CH_3$ | $OCH_3$ |
| J-5 | S | Q-67 ($R_7$=H, $R_8$=H, $R_9$=$CH_3$, $R_3$=$C_2H_5$) | H | 5-$CH_3$ | $OCH_3$ | $OCH_3$ |
| J-6 | S | Q-68 ($R_7$=H, $R_8$=H, $R_9$=H, $R_3$=$CH_3$) | H | H | $OCH_3$ | $OCH_3$ |
| J-5 | S | Q-68 ($R_7$=$C_2H_5$, $R_8$=H, $R_9$=H, $R_3$=i-$C_3H_7$) | H | H | $CH_3$ | $OCH_3$ |
| J-6 | S | Q-68 ($R_7$=H, $R_8$=H, $R_9$=$C_2H_5$, $R_3$=n-$C_4H_9$) | H | H | $CH_3$ | OCH |
| J-5 | S | Q-69 ($R_7$=H, $R_8$=H, $R_9$=H, $R_3$=$CH_3$) | H | H | $CH_3$ | $OCH_3$ |
| J-6 | S | Q-69 ($R_7$=n-$C_4H_9$, $R_8$=H, $R_9$=H, $R_3$=$C_2H_5$) | $CH_3$ | H | $CH_3$ | $OCH_3$ |
| J-5 | S | Q-69 ($R_7$=H, $R_8$=$CH_3$, $R_9$=i-$C_3H_7$, $R_3$=H) | H | H | $OCH_3$ | OCH |
| J-6 | S | Q-70 ($R_7$=H, $R_8$=H, $R_9$=H, $R_3$=$CH_3$) | H | H | $OCH_3$ | $OCH_3$ |
| J-7 | O | Q-70 ($R_7$=i-$C_3H_7$, $R_8$=H, $R_9$=H, $R_3$=$CH_3$) | H | H | $OCH_3$ | $OCH_3$ |
| J-5 | S | Q-70 ($R_7$=H, $R_8$=$C_2H_5$, $R_9$=$CH_3$, $R_3$=H) | H | 5-$CH_3$ | $OCH_3$ | OCH |
| J-5 | S | Q-72 ($R_7$=H, $R_8$=H, $R_9$=H, $R_2$=H) | H | H | $OCH_3$ | $OCH_3$ |
| J-6 | S | Q-72 ($R_7$=H, $R_8$=H, $R_9$=H, $R_2$=$CH_3$) | $CH_3$ | H | $OCH_3$ | $CH_3$ |
| J-5 | S | Q-72 ($R_7$=$CH_3$, $R_8$=H, $R_9$=H, $R_2$=H) | H | 5-$CH_3$ | $OCH_3$ | $OCH_3$ |
| J-6 | S | Q-73 ($R_7$=H, $R_8$=H, $R_9$=H, $R_2$=H) | H | H | $OCH_3$ | $OCH_3$ |
| J-5 | S | Q-73 ($R_7$=H, $R_8$=$CH_3$, $R_9$=H, $R_2$=$C_2H_5$) | H | H | $OCH_3$ | $CH_3$ |
| J-6 | O | Q-73 ($R_7$=$CH_3$, $R_8$=H, $R_9$=$CH_3$, $R_2$=H) | H | 5-Cl | $OCH_3$ | $CH_3$ |
| J-7 | S | Q-74 ($R_7$=H, $R_8$=H, $R_9$=H, $R_2$=H) | H | H | $CH_3$ | $OCH_3$ |
| J-5 | S | Q-74 ($R_7$=$CH_3$, $R_8$=H, $R_9$=H, $R_2$=H) | H | H | $CH_3$ | $OCH_3$ |
| J-6 | S | Q-74 ($R_7$=H, $R_8$=H, $R_9$=H, $R_2$=$CH_3$) | H | 5-$CH_3$ | $OCH_3$ | $OCH_3$ |
| J-5 | S | Q-75 ($R_7$=H, $R_8$=H, $R_9$=H) | H | H | $OCH_3$ | $OCH_3$ |
| J-6 | S | Q-75 ($R_7$=H, $R_8$=H, $R_9$=$CH_3$) | H | H | $CH_3$ | $OCH_3$ |
| J-5 | S | Q-75 ($R_7$=$CH_3$, $R_8$=H, $R_9$=$CH_3$) | H | H | $CH_3$ | $OCH_3$ |
| J-6 | S | Q-76 ($R_7$=H, $R_8$=H, $R_9$=H) | H | H | $CH_3$ | $OCH_3$ |
| J-5 | S | Q-76 ($R_7$=H, $R_8$=H, $R_9$=$CH_3$) | $CH_3$ | H | $CH_3$ | $OCH_3$ |
| J-6 | S | Q-76 ($R_7$=$C_2H_5$, $R_8$=H, $R_9$=$C_2H_5$) | H | H | $OCH_3$ | $OCH_3$ |
| J-5 | S | Q-77 ($R_7$=H, $R_8$=H, $R_9$=H) | H | H | $OCH_3$ | $OCH_3$ |

Table 2 (continued)

| J | $W_3$ | Q | R | $R_{1b}$ | X | Y |
|---|---|---|---|---|---|---|
| J-6 | O | Q-77 ($R_7$=H, $R_8$=H, $R_9$=n-$C_3H_7$) | H | H | $OCH_3$ | $OCH_3$ |
| J-7 | S | Q-77 ($R_7$=H, $R_8$=$CH_3$, $R_9$=H) | H | 5-$CH_3$ | $OCH_3$ | OCH |
| J-5 | S | Q-78 ($R_7$=H, $R_8$=H, $R_9$=H) | H | H | $OCH_3$ | $OCH_3$ |
| J-6 | S | Q-78 ($R_7$=H, $R_8$=H, $R_9$=s-$C_4H_9$) | H | H | $OCH_3$ | $OCH_3$ |
| J-5 | S | Q-78 ($R_7$=n-$C_3H_7$, $R_8$=H, $R_9$=H) | H | 5-Cl | $OCH_3$ | $OCH_3$ |
| J-6 | S | Q-79 ($R_7$=H, $R_8$=H, $R_9$=H, $R_3$=H) | H | H | $OCH_3$ | $OCH_3$ |
| J-5 | S | Q-79 ($R_7$=H, $R_8$=H, $R_9$=H, $R_3$=$CH_3$) | $CH_3$ | H | $OCH_3$ | $OCH_3$ |
| J-6 | S | Q-79 ($R_7$=$CH_3$, $R_8$=H, $R_9$=H, $R_3$=$C_2H_5$) | H | 5-$CH_3$ | $OCH_3$ | $OCH_3$ |
| J-5 | S | Q-80 ($R_7$=H, $R_8$=H, $R_9$=H, $R_3$=$CH_3$) | H | H | $OCH_3$ | $OCH_3$ |
| J-6 | S | Q-80 ($R_7$=$CH_3$, $R_8$=H, $R_9$=H, $R_3$=$CH_3$) | H | H | $OCH_3$ | $CH_3$ |
| J-5 | O | Q-80 ($R_7$=H, $R_8$=H, $R_9$=$CH_3$, $R_3$=$C_2H_5$) | H | 5-Cl | $OCH_3$ | $CH_3$ |
| J-6 | S | Q-81 ($R_7$=H, $R_8$=H, $R_9$=H, $R_3$=$CH_3$) | H | H | $CH_3$ | $OCH_3$ |
| J-7 | S | Q-81 ($R_7$=H, $R_8$=$CH_3$, $R_9$=H, $R_3$=i-$C_3H_7$) | H | H | $CH_3$ | $OCH_3$ |
| J-5 | S | Q-81 ($R_7$=$CH_3$, $R_8$=H, $R_9$=$CH_3$, $R_3$=$CH_3$) | H | 5-$CH_3$ | $OCH_3$ | OCH |
| J-6 | S | Q-82 ($R_7$=H, $R_8$=H, $R_9$=H, $R_3$=$CH_3$) | H | H | $OCH_3$ | $OCH_3$ |
| J-5 | S | Q-82 ($R_7$=$CH_3$, $R_8$=H, $R_9$=$CH_3$, $R_3$=H) | H | H | $CH_3$ | $OCH_3$ |
| J-6 | S | Q-82 ($R_7$=H, $R_8$=H, $R_9$=$C_2H_5$, $R_3$=$CH_3$) | H | H | $CH_3$ | $OCH_3$ |
| J-5 | $NCH_3$ | Q-83 ($R_7$=H, $R_8$=H, $R_9$=H, $R_{10}$=H) | H | H | $CH_3$ | $OCH_3$ |
| J-6 | S | Q-83 ($R_7$=$CH_3$, $R_8$=H, $R_9$=H, $R_{10}$=$CH_3$) | $CH_3$ | H | $CH_3$ | $OCH_3$ |
| J-5 | S | Q-83 ($R_7$=$CH_3$, $R_8$=$CH_3$, $R_9$=H, $R_{10}$=H) | H | H | $OCH_3$ | $OCH_3$ |
| J-6 | O | Q-84 ($R_7$=H, $R_8$=H, $R_9$=H) | H | H | $OCH_3$ | $OCH_3$ |
| J-5 | S | Q-84 ($R_7$=H, $R_8$=H, $R_9$=$CH_3$) | H | H | $OCH_3$ | $OCH_3$ |
| J-6 | S | Q-84 ($R_7$=H, $R_8$=$C_2H_5$, $R_9$=H) | H | 5-$CH_3$ | $OCH_3$ | $OCH_3$ |
| J-7 | S | Q-85 ($R_7$=H, $R_8$=H, $R_9$=H) | H | H | $OCH_3$ | $OCH_3$ |
| J-5 | S | Q-85 ($R_7$=$CH_3$, $R_8$=H, $R_9$=H) | H | H | $OCH_3$ | $OCH_3$ |
| J-6 | S | Q-85 ($R_7$=H, $R_8$=H, $R_9$=$C_2H_5$) | H | 5-Cl | $OCH_3$ | $OCH_3$ |
| J-5 | S | Q-86 ($R_7$=H, $R_8$=H, $R_9$=H) | H | H | $OCH_3$ | $OCH_3$ |
| J-6 | S | Q-86 ($R_7$=$CH_3$, $R_8$=H, $R_9$=H) | $CH_3$ | H | $OCH_3$ | $OCH_3$ |
| J-5 | S | Q-86 ($R_7$=H, $R_8$=H, $R_9$=$C_2H_5$) | H | 5-$CH_3$ | $OCH_3$ | $OCH_3$ |
| J-6 | S | Q-87 ($R_7$=H, $R_8$=H, $R_9$=H) | H | H | $OCH_3$ | $OCH_3$ |
| J-5 | O | Q-87 ($R_7$=H, $R_8$=H, $R_9$=$CH_3$) | H | H | $OCH_3$ | $CH_3$ |
| J-6 | S | Q-87 ($R_7$=$CH_3$, $R_8$=H, $R_9$=H) | H | 5-Cl | $OCH_3$ | $CH_3$ |
| J-5 | S | Q-88 ($R_7$=H, $R_8$=H, $R_9$=H) | H | H | $CH_3$ | $OCH_3$ |

Table 2 (continued)

| J | $W_3$ | Q | R | $R_{1b}$ | X | Y |
|---|---|---|---|---|---|---|
| J-6 | S | Q-88 ($R_7$=$CH_3$, $R_8$=H, $R_9$=H) | H | H | $CH_3$ | $OCH_3$ |
| J-7 | S | Q-88 ($R_7$=H, $R_8$=H, $R_9$=$CH_3$) | H | 5-$CH_3$ | $OCH_3$ | OCH |
| J-5 | S | Q-89 ($R_7$=H, $R_8$=H, $R_9$=H) | H | H | $OCH_3$ | $OCH_3$ |
| J-6 | S | Q-89 ($R_7$=$CH_3$, $R_8$=H, $R_9$=$C_2H_5$) | H | H | $CH_3$ | $OCH_3$ |
| J-5 | S | Q-89 ($R_7$=H, $R_8$=$CH_3$, $R_9$=H) | H | H | $CH_3$ | OCH |
| J-6 | S | Q-90 ($R_7$=H, $R_8$=H, $R_9$=H, $R_3$=$CH_3$) | H | H | $CH_3$ | $OCH_3$ |
| J-5 | S | Q-90 ($R_7$=$CH_3$, $R_8$=H, $R_9$=H, $R_3$=$C_2H_5$) | $CH_3$ | H | $CH_3$ | $OCH_3$ |
| J-6 | O | Q-90 ($R_7$=H, $R_8$=H, $R_9$=$CH_3$, $R_3$=$CH_3$) | H | H | $OCH_3$ | $OCH_3$ |
| J-5 | S | Q-91 ($R_7$=H, $R_8$=H, $R_9$=H, $R_3$=$CH_3$) | H | H | $OCH_3$ | $OCH_3$ |
| J-6 | S | Q-91 ($R_7$=$CH_3$, $R_8$=H, $R_9$=H, $R_3$=H) | H | H | $OCH_3$ | $OCH_3$ |
| J-5 | S | Q-91 ($R_7$=H, $R_8$=H, $R_9$=$CH_3$, $R_3$=$CH_3$) | H | 5-$CH_3$ | $OCH_3$ | $OCH_3$ |
| J-6 | S | Q-92 ($R_7$=H, $R_8$=H, $R_9$=H, $R_{10}$=H) | H | H | $OCH_3$ | $OCH_3$ |
| J-7 | S | Q-92 ($R_7$=H, $R_8$=H, $R_9$=H, $R_{10}$=$CH_3$) | H | H | $OCH_3$ | $OCH_3$ |
| J-5 | S | Q-92 ($R_7$=H, $R_8$=$CH_3$, $R_9$=H, $R_{10}$=H) | H | 5-Cl | $OCH_3$ | $OCH_3$ |
| J-6 | S | Q-93 ($R_7$=H, $R_8$=H, $R_9$=H) | H | H | $OCH_3$ | $OCH_3$ |
| J-5 | S | Q-93 ($R_7$=H, $R_8$=H, $R_9$=$CH_3$) | $CH_3$ | H | $OCH_3$ | $OCH_3$ |
| J-6 | S | Q-93 ($R_7$=$CH_3$, $R_8$=H, $R_9$=H) | H | 5-$CH_3$ | $OCH_3$ | $OCH_3$ |
| J-5 | O | Q-94 ($R_7$=H, $R_8$=H, $R_9$=H) | H | H | $OCH_3$ | $OCH_3$ |
| J-6 | S | Q-94 ($R_7$=$CH_3$, $R_8$=H, $R_9$=H) | H | H | $OCH_3$ | $CH_3$ |
| J-5 | S | Q-94 ($R_7$=H, $R_8$=H, $R_9$=n-$C_4H_9$) | H | 5-Cl | $OCH_3$ | $CH_3$ |
| J-6 | S | Q-95 ($R_7$=H, $R_8$=H, $R_9$=H) | H | H | $CH_3$ | $OCH_3$ |
| J-5 | S | Q-95 ($R_7$=H, $R_8$=H, $R_9$=$CH_3$) | H | H | $CH_3$ | $OCH_3$ |
| J-6 | S | Q-95 ($R_7$=H, $R_8$=H, $R_9$=s-$C_4H_9$) | H | 5-$CH_3$ | $OCH_3$ | $OCH_3$ |
| J-7 | S | Q-96 ($R_7$=H, $R_8$=H, $R_9$=H) | H | H | $OCH_3$ | $OCH_3$ |
| J-5 | S | Q-96 ($R_7$=$CH_3$, $R_8$=H, $R_9$=H) | H | H | $CH_3$ | $OCH_3$ |
| J-6 | S | Q-96 ($R_7$=H, $R_8$=H, $R_9$=$CH_3$) | H | H | $CH_3$ | $OCH_3$ |
| J-5 | S | Q-97 ($R_7$=H, $R_8$=H, $R_9$=H) | H | H | $CH_3$ | $OCH_3$ |
| J-6 | O | Q-97 ($R_7$=$CH_3$, $R_8$=$CH_3$, $R_9$=$CH_3$) | $CH_3$ | H | $CH_3$ | $OCH_3$ |
| J-5 | O | Q-97 ($R_7$=H, $R_8$=H, $R_9$=$CH_3$) | H | H | $OCH_3$ | $OCH_3$ |
| J-6 | S | Q-98 ($R_7$=H, $R_8$=H, $R_9$=H) | H | H | $OCH_3$ | $OCH_3$ |
| J-5 | S | Q-98 ($R_7$=$CH_3$, $R_8$=H, $R_9$=H) | H | H | $OCH_3$ | $OCH_3$ |
| J-6 | S | Q-98 ($R_7$=H, $R_8$=H, $R_9$=i-$C_3H_7$) | H | 5-$CH_3$ | $OCH_3$ | $OCH_3$ |
| J-5 | S | Q-99 ($R_7$=H, $R_8$=H, $R_9$=H, $R_3$=H) | H | H | $OCH_3$ | $OCH_3$ |

Table 2 (continued)

| J | $W_3$ | Q | R | $R_{1b}$ | X | Y | m.p.(°C) |
|---|---|---|---|---|---|---|---|
| J-6 | S | Q-99 ($R_7$=H, $R_8$=H, $R_9$=$CH_3$, $R_3$=$CH_3$) | H | H | $OCH_3$ | $OCH_3$ | |
| J-7 | S | Q-99 ($R_7$=H, $R_8$=H, $R_9$=s-$C_4H_9$, $R_3$=H) | H | 5-Cl | $OCH_3$ | OCH | |
| J-5 | S | Q-100 ($R_7$=H, $R_8$=H, $R_9$=H, $R_3$=$C_2H_5$) | H | H | $OCH_3$ | $OCH_3$ | |
| J-6 | S | Q-100 ($R_7$=$CH_3$, $R_8$=H, $R_9$=H, $R_3$=H) | $CH_3$ | H | $OCH_3$ | $CH_3$ | |
| J-5 | O | Q-100 ($R_7$=H, $R_8$=H, $R_9$=$CH_3$, $R_3$=$CH_3$) | H | 5-$CH_3$ | $OCH_3$ | $OCH_3$ | |
| J-6 | S | Q-101 ($R_7$=H, $R_8$=H, $R_9$=H, $R_3$=i-$C_3H_7$) | H | H | $OCH_3$ | $OCH_3$ | |
| J-5 | S | Q-101 ($R_7$=$CH_3$, $R_8$=$CH_3$, $R_9$=$CH_3$, $R_3$=H) | H | H | $OCH_3$ | $CH_3$ | |
| J-6 | S | Q-101 ($R_7$=H, $R_8$=H, $R_9$=$CH_3$, $R_3$=$CH_3$) | H | 5-Cl | $OCH_3$ | $CH_3$ | |
| J-5 | S | Q-102 ($R_7$=H, $R_8$=H, $R_9$=H, $R_3$=H) | H | H | $CH_3$ | $OCH_3$ | |
| J-6 | S | Q-102 ($R_7$=$CH_3$, $R_8$=H, $R_9$=H, $R_3$=$CH_3$) | H | H | $CH_3$ | $OCH_3$ | |
| J-5 | S | Q-102 ($R_7$=H, $R_8$=H, $R_9$=i-$C_3H_7$, $R_3$=$CH_3$) | H | 5-$CH_3$ | $OCH_3$ | $OCH_3$ | |
| J-6 | O | Q-104 ($R_7$=H, $R_8$=H, $R_9$=H, $R_2$=H) | H | H | $CH_3$ | $OCH_3$ | |
| J-5 | S | Q-104 ($R_7$=$CH_3$, $R_8$=H, $R_9$=H, $R_2$=$OC_2H_5$) | $CH_3$ | H | $CH_3$ | $OCH_3$ | |
| J-6 | S | Q-104 ($R_7$=H, $R_8$=H, $R_9$=H, $R_2$=Br) | H | H | $OCH_3$ | $OCH_3$ | |
| J-5 | S | Q-105 ($R_7$=H, $R_8$=H, $R_9$=H, $R_2$=H) | H | H | $OCH_3$ | $OCH_3$ | |
| J-6 | S | Q-105 ($R_7$=H, $R_8$=H, $R_9$=H, $R_2$=H) | H | H | $OCH_3$ | $CH_3$ | 175-180 |
| J-6 | S | Q-105 ($R_7$=H, $R_8$=H, $R_9$=H, $R_2$=H) | H | H | $OCH_3$ | $OCH_3$ | 162-167 |
| J-6 | S | Q-105 ($R_7$=$CH_3$, $R_8$=H, $R_9$=H, $R_2$=$CH_3$) | H | H | $OCH_3$ | $OCH_3$ | |
| J-5 | S | Q-105 ($R_7$=H, $R_8$=$CH_3$, $R_9$=H, $R_2$=$C_2H_5$) | H | 5-$CH_3$ | $OCH_3$ | $OCH_3$ | |
| J-6 | S | Q-106 ($R_7$=H, $R_8$=H, $R_9$=H, $R_2$=H) | H | H | $OCH_3$ | $OCH_3$ | |
| J-5 | S | Q-106 ($R_7$=$CH_3$, $R_8$=H, $R_9$=H, $R_2$=$CH_3$) | H | H | $OCH_3$ | $OCH_3$ | |
| J-6 | S | Q-106 ($R_7$=Cl, $R_8$=H, $R_9$=H, $R_2$=H) | H | 5-Cl | $OCH_3$ | $OCH_3$ | |
| J-7 | S | Q-107 ($R_7$=H, $R_8$=H, $R_9$=H, $R_2$=H) | H | H | $OCH_3$ | $OCH_3$ | |
| J-5 | O | Q-107 ($R_7$=$CH_3$, $R_8$=H, $R_9$=H, $R_2$=H) | $CH_3$ | H | $OCH_3$ | $OCH_3$ | |
| J-6 | S | Q-107 ($R_7$=H, $R_8$=H, $R_9$=H, $R_2$=$CH_3$) | H | 5-$CH_3$ | $OCH_3$ | $OCH_3$ | |
| J-5 | S | Q-108 ($R_7$=H, $R_8$=H, $R_9$=H, $R_2$=H) | H | H | $OCH_3$ | $OCH_3$ | |
| J-6 | S | Q-108 ($R_7$=$CH_3$, $R_8$=H, $R_9$=H, $R_2$=Cl) | H | H | $OCH_3$ | $CH_3$ | |
| J-5 | S | Q-108 ($R_7$=H, $R_8$=H, $R_9$=H, $R_2$=$CH_3$) | H | 5-Cl | $OCH_3$ | $CH_3$ | |
| J-6 | S | Q-109 ($R_7$=H, $R_8$=H, $R_9$=H) | H | H | $CH_3$ | $OCH_3$ | |
| J-5 | S | Q-109 ($R_7$=$CH_3$, $R_8$=H, $R_9$=H) | H | H | $CH_3$ | $OCH_3$ | |

Table 2 (continued)

| J | $W_3$ | Q | R | $R_{1b}$ | X | Y |
|---|---|---|---|---|---|---|
| J-6 | S | Q-109 ($R_7$=H, $R_8$=H, $R_9$=$CH_3$) | H | 5-$CH_3$ | $OCH_3$ | $OCH_3$ |
| J-5 | S | Q-110 ($R_7$=H, $R_8$=H, $R_9$=H) | H | H | $OCH_3$ | $OCH_3$ |
| J-6 | S | Q-110 ($R_7$=$CH_3$, $R_8$=H, $R_9$=H) | H | H | $CH_3$ | $OCH_3$ |
| J-7 | O | Q-110 ($R_7$=H, $R_8$=$CH_3$, $R_9$=H) | H | H | $CH_3$ | $OCH_3$ |
| J-5 | S | Q-111 ($R_7$=H, $R_8$=H, $R_9$=H) | H | H | $CH_3$ | $OCH_3$ |
| J-6 | S | Q-111 ($R_7$=$CH_3$, $R_8$=H, $R_9$=H) | $CH_3$ | H | $CH_3$ | $OCH_3$ |
| J-5 | S | Q-111 ($R_7$=H, $R_8$=H, $R_9$=$CH_3$) | H | H | $OCH_3$ | OCH |
| J-6 | S | Q-112 ($R_7$=H, $R_8$=H, $R_9$=H) | H | H | $OCH_3$ | $OCH_3$ |
| J-5 | S | Q-112 ($R_7$=$CH_3$, $R_8$=H, $R_9$=H) | H | H | $OCH_3$ | $OCH_3$ |
| J-6 | S | Q-112 ($R_7$=H, $R_8$=$CH_3$, $R_9$=$CH_3$) | H | 5-$CH_3$ | $OCH_3$ | $OCH_3$ |
| J-5 | S | Q-113 ($R_7$=H, $R_8$=H, $R_9$=H, $R_3$=$CH_3$) | H | H | $OCH_3$ | $OCH_3$ |
| J-6 | S | Q-113 ($R_7$=$C_2H_5$, $R_8$=H, $R_9$=H, $R_3$=i-$C_3H_7$) | H | H | $OCH_3$ | $OCH_3$ |
| J-5 | S | Q-113 ($R_7$=H, $R_8$=H, $R_9$=$C_2H_5$, $R_3$=n-$C_4H_9$) | H | 5-Cl | $OCH_3$ | $OCH_3$ |
| J-6 | O | Q-114 ($R_7$=H, $R_8$=H, $R_9$=H, $R_3$=$CH_3$) | H | H | $OCH_3$ | $OCH_3$ |
| J-7 | S | Q-114 ($R_7$=n-$C_4H_9$, $R_8$=H, $R_9$=H, $R_3$=$C_2H_5$) | $CH_3$ | H | $OCH_3$ | $CH_3$ |
| J-5 | S | Q-114 ($R_7$=H, $R_8$=$CH_3$, $R_8$=i-$C_3H_7$, $R_3$=H) | H | 5-$CH_3$ | $OCH_3$ | $OCH_3$ |
| J-6 | S | Q-115 ($R_7$=H, $R_8$=H, $R_9$=H, $R_3$=$CH_3$) | H | H | $OCH_3$ | $OCH_3$ |
| J-5 | S | Q-115 ($R_7$=i-$C_3H_7$, $R_8$=H, $R_9$=H, $R_3$=$CH_3$) | H | H | $OCH_3$ | $CH_3$ |
| J-6 | S | Q-115 ($R_7$=H, $R_8$=$C_2H_5$, $R_9$=$CH_3$, $R_3$=H) | H | 5-Cl | $OCH_3$ | $CH_3$ |
| J-5 | S | Q-116 ($R_7$=H, $R_8$=H, $R_9$=H, $R_{10}$=H) | H | H | $CH_3$ | $OCH_3$ |
| J-6 | S | Q-116 ($R_7$=$CH_3$, $R_8$=H, $R_9$=H, $R_{10}$=H) | H | H | $CH_3$ | $OCH_3$ |
| J-5 | S | Q-116 ($R_7$=H, $R_8$=H, $R_9$=H, $R_{10}$=$CH_3$) | H | 5-$CH_3$ | $OCH_3$ | $OCH_3$ |
| J-7 | NH | Q-118 ($R_7$=H, $R_8$=H, $R_9$=H, $R_{10}$=H) | H | H | $CH_3$ | $OCH_3$ |
| J-5 | S | Q-118 ($R_7$=$CH_3$, $R_8$=H, $R_9$=H, $R_{10}$=H) | $CH_3$ | H | $CH_3$ | $OCH_3$ |
| J-6 | S | Q-118 ($R_7$=H, $R_8$=H, $R_9$=H, $R_{10}$=$CH_3$) | H | H | $OCH_3$ | OCH |
| J-5 | S | Q-119 ($R_7$=H, $R_8$=H, $R_9$=H, $R_2$=H) | H | H | $OCH_3$ | $OCH_3$ |
| J-6 | S | Q-119 ($R_7$=H, $R_8$=H, $R_9$=H, $R_2$=$CH_3$) | H | H | $OCH_3$ | $OCH_3$ |
| J-5 | S | Q-119 ($R_7$=H, $R_8$=$CH_3$, $R_9$=H, $R_2$=$C_2H_5$) | H | 5-$CH_3$ | $OCH_3$ | $OCH_3$ |
| J-6 | S | Q-120 ($R_7$=H, $R_8$=H, $R_9$=H, $R_2$=H) | H | H | $OCH_3$ | $OCH_3$ |
| J-5 | S | Q-120 ($R_7$=H, $R_8$=H, $R_9$=H, $R_2$=$CH_3$) | H | H | $OCH_3$ | $OCH_3$ |

Table 2 (continued)

| J | $W_3$ | Q | R | $R_{1b}$ | X | Y |
|---|---|---|---|---|---|---|
| J-6 | O | Q-120 ($R_7$=H, $R_8$=H, $R_9$=$CH_3$, $R_2$=$CH_3$) | H | 5-Cl | $OCH_3$ | $OCH_3$ |
| J-5 | S | Q-121 ($R_7$=H, $R_8$=H, $R_9$=H, $R_2$=H) | H | H | $OCH_3$ | $OCH_3$ |
| J-6 | S | Q-121 ($R_7$=H, $R_8$=H, $R_9$=H, $R_2$=$C_2H_5$) | $CH_3$ | H | $OCH_3$ | $CH_3$ |
| J-7 | S | Q-121 ($R_7$=$CH_3$, $R_8$=$CH_3$, $R_9$=H, $R_2$=H) | H | 5-$CH_3$ | $OCH_3$ | $OCH_3$ |
| J-5 | S | Q-122 ($R_7$=H, $R_8$=H, $R_9$=H, $R_2$=H) | H | H | $OCH_3$ | $OCH_3$ |
| J-6 | S | Q-122 ($R_7$=H, $R_8$=H, $R_9$=H, $R_2$=$CH_3$) | H | H | $OCH_3$ | $CH_3$ |
| J-5 | S | Q-122 ($R_7$=$CH_3$, $R_8$=H, $R_9$=H, $R_2$=H) | H | 5-Cl | $OCH_3$ | $CH_3$ |
| J-6 | S | Q-123 ($R_7$=H, $R_8$=H, $R_9$=H) | H | H | $CH_3$ | $OCH_3$ |
| J-5 | S | Q-123 ($R_7$=H, $R_8$=H, $R_9$=$CH_3$) | H | H | $CH_3$ | $OCH_3$ |
| J-6 | S | Q-123 ($R_7$=$CH_3$, $R_8$=H, $R_9$=$CH_3$) | H | 5-$CH_3$ | $OCH_3$ | $OCH_3$ |
| J-5 | O | Q-124 ($R_7$=H, $R_8$=H, $R_9$=H) | H | H | $OCH_3$ | $OCH_3$ |
| J-6 | S | Q-124 ($R_7$=H, $R_8$=H, $R_9$=$CH_3$) | H | H | $CH_3$ | $OCH_3$ |
| J-5 | S | Q-124 ($R_7$=$C_2H_5$, $R_8$=H, $R_9$=$C_2H_5$) | H | H | $CH_3$ | $OCH_3$ |
| J-6 | S | Q-125 ($R_7$=H, $R_8$=H, $R_9$=H) | H | H | $CH_3$ | $OCH_3$ |
| J-7 | S | Q-125 ($R_7$=H, $R_8$=H, $R_9$=n-$C_4H_9$) | $CH_3$ | H | $CH_3$ | $OCH_3$ |
| J-5 | S | Q-125 ($R_7$=H, $R_8$=$CH_3$, $R_9$=H) | H | H | $OCH_3$ | $OCH_3$ |
| J-6 | S | Q-126 ($R_7$=H, $R_8$=H, $R_9$=H) | H | H | $OCH_3$ | $OCH_3$ |
| J-5 | S | Q-126 ($R_7$=H, $R_8$=H, $R_9$=s-$C_3H_7$) | H | H | $OCH_3$ | $OCH_3$ |
| J-6 | S | Q-126 ($R_7$=n-$C_3H_7$, $R_8$=H, $R_9$=H) | H | 5-$CH_3$ | $OCH_3$ | $OCH_3$ |
| J-5 | S | Q-127 ($R_7$=H, $R_8$=H, $R_9$=H, $R_3$=H) | H | H | $OCH_3$ | $OCH_3$ |
| J-6 | O | Q-127 ($R_7$=H, $R_8$=H, $R_9$=H, $R_3$=$CH_3$) | H | H | $OCH_3$ | $OCH_3$ |
| J-5 | S | Q-127 ($R_7$=$CH_3$, $R_8$=H, $R_9$=H, $R_3$=$C_2H_5$) | H | 5-Cl | $OCH_3$ | $OCH_3$ |
| J-6 | S | Q-128 ($R_7$=H, $R_8$=H, $R_9$=H, $R_3$=$CH_3$) | H | H | $OCH_3$ | $OCH_3$ |
| J-5 | S | Q-128 ($R_7$=$CH_3$, $R_8$=H, $R_9$=H, $R_3$=$CH_3$) | $CH_3$ | H | $OCH_3$ | $OCH_3$ |
| J-6 | S | Q-128 ($R_7$=H, $R_8$=H, $R_9$=$CH_3$, $R_3$=$C_2H_5$) | H | 5-$CH_3$ | $OCH_3$ | $OCH_3$ |
| J-7 | S | Q-129 ($R_7$=H, $R_8$=H, $R_9$=H, $R_3$=$CH_3$) | H | H | $OCH_3$ | $OCH_3$ |
| J-5 | S | Q-129 ($R_7$=H, $R_8$=$CH_3$, $R_9$=H, $R_3$=i-$C_3H_7$) | H | H | $OCH_3$ | $CH_3$ |
| J-6 | S | Q-129 ($R_7$=$CH_3$, $R_8$=H, $R_9$=$CH_3$, $R_3$=$CH_3$) | H | 5-Cl | $OCH_3$ | $CH_3$ |
| J-5 | S | Q-130 ($R_7$=H, $R_8$=H, $R_9$=H, $R_3$=$CH_3$) | H | H | $CH_3$ | $OCH_3$ |
| J-6 | S | Q-130 ($R_7$=$CH_3$, $R_8$=H, $R_9$=$CH_3$, $R_3$=H) | H | H | $CH_3$ | $OCH_3$ |
| J-5 | O | Q-130 ($R_7$=H, $R_8$=H, $R_9$=$C_2H_5$, $R_3$=$CH_3$) | H | 5-$CH_3$ | $OCH_3$ | $OCH_3$ |
| J-6 | S | Q-131 ($R_7$=H, $R_8$=H, $R_9$=H, $R_{10}$=H) | H | H | $OCH_3$ | $OCH_3$ |

Table 2 (continued)

| J | $W_3$ | Q | R | $R_{1b}$ | X | Y | m.p.(°C) |
|---|---|---|---|---|---|---|---|
| J-5 | S | Q-131 ($R_7$=$CH_3$, $R_8$=H, $R_9$=H, $R_{10}$=$CH_3$) | H | H | $CH_3$ | $OCH_3$ | |
| J-6 | S | Q-131 ($R_7$=$CH_3$, $R_8$=$CH_3$, $R_9$=H, $R_{10}$=H) | H | H | $CH_3$ | $OCH_3$ | |
| J-5 | S | Q-132 ($R_7$=H, $R_8$=H, $R_9$=H) | H | H | $CH_3$ | $OCH_3$ | |
| J-6 | S | Q-132 ($R_7$=H, $R_8$=H, $R_9$=$CH_3$) | $CH_3$ | H | $CH_3$ | $OCH_3$ | |
| J-7 | S | Q-132 ($R_7$=H, $R_8$=$C_2H_5$, $R_9$=H) | H | H | $OCH_3$ | $OCH_3$ | |
| J-5 | S | Q-133 ($R_7$=H, $R_8$=H, $R_9$=H) | H | H | $OCH_3$ | $OCH_3$ | |
| J-6 | S | Q-133 ($R_7$=$CH_3$, $R_8$=H, $R_9$=H) | H | H | $OCH_3$ | $OCH_3$ | |
| J-5 | S | Q-133 ($R_7$=H, $R_8$=H, $R_9$=$C_2H_5$) | H | 5-$CH_3$ | $OCH_3$ | $OCH_3$ | |
| J-6 | O | Q-134 ($R_7$=H, $R_8$=H, $R_9$=H) | H | H | $OCH_3$ | $OCH_3$ | |
| J-5 | S | Q-134 ($R_7$=$CH_3$, $R_8$=H, $R_9$=H) | H | H | $OCH_3$ | $OCH_3$ | |
| J-6 | S | Q-134 ($R_7$=H, $R_8$=H, $R_9$=$C_2H_5$) | H | 5-Cl | $OCH_3$ | $OCH_3$ | |
| J-5 | S | Q-135 ($R_7$=H, $R_8$=H, R =H) | H | H | $OCH_3$ | $OCH_3$ | |
| J-6 | S | Q-135 ($R_7$=H, $R_8$=H, R =$CH_3$) | $CH_3$ | H | $OCH_3$ | $OCH_3$ | |
| J-5 | S | Q-135 ($R_7$=$CH_3$, $R_8$=H, $R_9$=H) | H | 5-$CH_3$ | $OCH_3$ | $OCH_3$ | |
| J-6 | S | Q-136 ($R_7$=H, $R_8$=H, $R_9$=H) | H | H | $OCH_3$ | $OCH_3$ | |
| J-7 | S | Q-136 ($R_7$=H, $R_8$=H, $R_9$=$CH_3$) | H | H | $OCH_3$ | $CH_3$ | |
| J-5 | S | Q-136 ($R_7$=H, $R_8$=$CH_3$, $R_9$=$C_2H_5$) | H | 5-Cl | $OCH_3$ | $CH_3$ | |
| J-6 | S | Q-137 ($R_7$=H, $R_8$=H, $R_9$=H) | H | H | $CH_3$ | $OCH_3$ | |
| J-5 | O | Q-137 ($R_7$=$CH_3$, $R_8$=H, $R_9$=H) | H | H | $CH_3$ | $OCH_3$ | |
| J-6 | S | Q-137 ($R_7$=H, $R_8$=H, $R_9$=$CH_3$) | H | 5-$CH_3$ | $OCH_3$ | $OCH_3$ | |
| J-5 | S | Q-138 ($R_7$=H, $R_8$=H, $R_9$=H) | H | H | $OCH_3$ | $OCH_3$ | |
| J-6 | S | Q-138 ($R_7$=H, $R_8$=H, $R_9$=H) | H | H | $CH_3$ | $OCH_3$ | 85-93 |
| J-6 | S | Q-138 ($R_7$=H, $R_8$=H, $R_9$=H) | H | H | $OCH_3$ | $OCH_3$ | 118-127 |
| J-6 | S | Q-138 ($R_7$=$CH_3$, $R_8$=H, $R_9$=H) | H | H | $CH_3$ | $OCH_3$ | |
| J-5 | $NCH_3$ | Q-138 ($R_7$=H, $R_8$=H, $R_9$=$CH_3$) | H | H | $CH_3$ | $OCH_3$ | |
| J-6 | S | Q-139 ($R_7$=H, $R_8$=H, $R_9$=H) | H | H | $CH_3$ | $OCH_3$ | |
| J-5 | S | Q-139 ($R_7$=$CH_3$, $R_8$=H, $R_9$=H) | $CH_3$ | H | $CH_3$ | $OCH_3$ | |
| J-6 | S | Q-139 ($R_7$=$CH_3$, $R_8$=$CH_3$, $R_9$=H) | H | H | $OCH_3$ | $OCH_3$ | |
| J-7 | S | Q-140 ($R_7$=H, $R_8$=H, $R_9$=H, $R_3$=$CH_3$) | H | H | $OCH_3$ | $OCH_3$ | |
| J-5 | S | Q-140 ($R_7$=H, $R_8$=H, $R_9$=$CH_3$, $R_3$=H) | H | H | $OCH_3$ | $OCH_3$ | |
| J-6 | O | Q-140 ($R_7$=H, $R_8$=$CH_3$, $R_9$=H, $R_3$=$C_2H_5$) | H | 5-$CH_3$ | $OCH_3$ | $OCH_3$ | |
| J-5 | S | Q-141 ($R_7$=H, $R_8$=H, $R_9$=H, $R_3$=$CH_3$) | H | H | $OCH_3$ | $OCH_3$ | |
| J-6 | S | Q-141 ($R_7$=H, $R_8$=H, $R_9$=H, $R_3$=H) | H | H | $OCH_3$ | $OCH_3$ | |
| J-5 | S | Q-141 ($R_7$=H, $R_8$=H, $R_9$=$CH_3$, $R_3$=$CH_3$) | H | 5-Cl | $OCH_3$ | $OCH_3$ | |

Table 2 (continued)

| J | $W_3$ | Q | R | $R_{1b}$ | X | Y |
|---|---|---|---|---|---|---|
| J-6 | S | Q-142 ($R_7$=H, $R_8$=H, $R_9$=H, $R_{10}$=H) | H | H | $OCH_3$ | $OCH_3$ |
| J-5 | S | Q-142 ($R_7$=$CH_3$, $R_8$=H, $R_9$=H, $R_{10}$=H) | $CH_3$ | H | $OCH_3$ | $CH_3$ |
| J-6 | S | Q-142 ($R_7$=H, $R_8$=H, $R_9$=H, $R_{10}$=$CH_3$) | H | 5-$CH_3$ | $OCH_3$ | $OCH_3$ |
| J-5 | S | Q-143 ($R_7$=H, $R_8$=H, $R_9$=H, $R_3$=$CH_3$) | H | H | $OCH_3$ | $OCH_3$ |
| J-6 | S | Q-143 ($R_7$=H, $R_8$=H, $R_9$=H, $R_3$=H) | H | H | $OCH_3$ | $CH_3$ |
| J-7 | S | Q-143 ($R_7$=$CH_3$, $R_8$=H, $R_9$=$CH_3$, $R_3$=$CH_3$) | H | 5-Cl | $OCH_3$ | $CH_3$ |
| J-5 | O | Q-144 ($R_7$=H, $R_8$=H, $R_9$=H, $R_3$=$CH_3$) | H | H | $CH_3$ | $OCH_3$ |
| J-6 | S | Q-144 ($R_7$=$CH_3$, $R_8$=H, $R_9$=H, $R_3$=$CH_3$) | H | H | $CH_3$ | $OCH_3$ |
| J-5 | S | Q-144 ($R_7$=H, $R_8$=$C_2H_5$, $R_9$=H, $R_3$=$CH_3$) | H | 5-$CH_3$ | $OCH_3$ | $OCH_3$ |
| J-6 | S | Q-145 ($R_7$=H, $R_8$=H, $R_9$=H) | H | H | $OCH_3$ | $OCH_3$ |
| J-5 | S | Q-145 ($R_7$=H, $R_8$=H, $R_9$=$CH_3$) | H | H | $CH_3$ | $OCH_3$ |
| J-6 | S | Q-145 ($R_7$=$CH_3$, $R_8$=H, $R_9$=H) | H | H | $CH_3$ | $OCH_3$ |
| J-5 | S | Q-146 ($R_7$=H, $R_8$=H, $R_9$=H) | H | H | $CH_3$ | $OCH_3$ |
| J-6 | S | Q-146 ($R_7$=$CH_3$, $R_8$=H, $R_9$=H) | $CH_3$ | H | $CH_3$ | $OCH_3$ |
| J-5 | S | Q-146 ($R_7$=H, $R_8$=H, $R_9$=<u>n</u>-$C_4H_9$) | H | H | $OCH_3$ | $OCH_3$ |
| J-6 | S | Q-147 ($R_7$=H, $R_8$=H, $R_9$=H) | H | H | $OCH_3$ | $OCH_3$ |
| J-7 | O | Q-147 ($R_7$=$CH_3$, $R_8$=H, $R_9$=H) | H | H | $OCH_3$ | $OCH_3$ |
| J-5 | S | Q-147 ($R_7$=H, $R_8$=H, $R_9$=<u>n</u>-$C_4H_9$) | H | 5-$CH_3$ | $OCH_3$ | $OCH_3$ |
| J-6 | S | Q-148 ($R_7$=H, $R_8$=H, $R_9$=H) | H | H | $OCH_3$ | $OCH_3$ |
| J-5 | S | Q-148 ($R_7$=H, $R_8$=H, $R_9$=$CH_3$) | H | H | $OCH_3$ | $OCH_3$ |
| J-6 | S | Q-148 ($R_7$=$CH_3$, $R_8$=H, $R_9$=H) | H | 5-Cl | $OCH_3$ | $OCH_3$ |
| J-5 | S | Q-149 ($R_7$=H, $R_8$=H, $R_9$=H) | H | H | $OCH_3$ | $OCH_3$ |
| J-6 | S | Q-149 ($R_7$=H, $R_8$=H, $R_9$=$CH_3$) | $CH_3$ | H | $OCH_3$ | $OCH_3$ |
| J-5 | S | Q-149 ($R_7$=$CH_3$, $R_8$=H, $R_9$=H) | H | 5-$CH_3$ | $OCH_3$ | $OCH_3$ |
| J-6 | S | Q-150 ($R_7$=H, $R_8$=H, $R_9$=H) | H | H | $OCH_3$ | $OCH_3$ |
| J-5 | S | Q-150 ($R_7$=$CH_3$, $R_8$=H, $R_9$=H) | H | H | $OCH_3$ | $CH_3$ |
| J-6 | O | Q-150 ($R_7$=H, $R_8$=$CH_3$, $R_9$=$CH_3$) | H | 5-Cl | $OCH_3$ | $CH_3$ |
| J-7 | S | Q-151 ($R_7$=H, $R_8$=H, $R_9$=H) | H | H | $CH_3$ | $OCH_3$ |
| J-5 | S | Q-151 ($R_7$=$CH_3$, $R_8$=H, $R_9$=H) | H | H | $CH_3$ | $OCH_3$ |
| J-6 | S | Q-151 ($R_7$=$CH_3$, $R_8$=$CH_3$, $R_9$=H) | H | 5-$CH_3$ | $OCH_3$ | $OCH_3$ |
| J-5 | S | Q-152 ($R_7$=H, $R_8$=H, $R_9$=H) | H | H | $OCH_3$ | $OCH_3$ |
| J-6 | S | Q-152 ($R_7$=$C_2H_5$, $R_8$=H, $R_9$=H) | H | H | $CH_3$ | $OCH_3$ |

Table 2 (continued)

| J | $W_3$ | Q | R | $R_{1b}$ | X | Y |
|---|---|---|---|---|---|---|
| J-5 | S | Q-152 ($R_7$=$CH_3$, $R_8$=$CH_3$, $R_9$=H) | H | H | $CH_3$ | $OCH_3$ |
| J-6 | S | Q-153 ($R_7$=H, $R_8$=H, $R_9$=H, $R_{11}$=$CH_3$) | H | H | $CH_3$ | $OCH_3$ |
| J-5 | S | Q-153 ($R_7$=H, $R_8$=H, $R_9$=H, $R_{11}$=H) | $CH_3$ | H | $CH_3$ | $OCH_3$ |
| J-6 | S | Q-153 ($R_7$=$CH_3$, $R_8$=H, $R_9$=H, $R_{11}$=$CH_3$) | H | H | $OCH_3$ | $OCH_3$ |
| J-5 | O | Q-154 ($R_7$=H, $R_8$=H, $R_9$=H, $R_{11}$=$CH_3$) | H | H | $OCH_3$ | $OCH_3$ |
| J-6 | S | Q-154 ($R_7$=$CH_3$, $R_8$=H, $R_9$=H) | H | H | $OCH_3$ | $OCH_3$ |
| J-7 | S | Q-154 ($R_7$=H, $R_8$=$CH_3$, $R_9$=$CH_3$) | H | 5-$CH_3$ | $OCH_3$ | $OCH_3$ |
| J-5 | S | Q-155 ($R_7$=H, $R_8$=H, $R_9$=H) | H | H | $OCH_3$ | $OCH_3$ |
| J-6 | S | Q-155 ($R_7$=$CH_3$, $R_8$=H, $R_9$=H) | H | H | $OCH_3$ | $OCH_3$ |
| J-5 | S | Q-155 ($R_7$=$CH_3$, $R_8$=$CH_3$, $R_9$=H) | H | 5-Cl | $OCH_3$ | $OCH_3$ |
| J-6 | S | Q-156 ($R_7$=H, $R_8$=H, $R_9$=H) | H | H | $OCH_3$ | $OCH_3$ |
| J-5 | S | Q-156 ($R_7$=$C_2H_5$, $R_8$=H, $R_9$=H) | $CH_3$ | H | $OCH_3$ | $OCH_3$ |
| J-6 | S | Q-156 ($R_7$=$CH_3$, $R_8$=$CH_3$, $R_9$=H) | H | 5-$CH_3$ | $OCH_3$ | $OCH_3$ |
| J-6 | S | Q-158 ($R_7$=H, $R_8$=H, $R_9$=H) | H | H | $CH_3$ | $OCH_3$ |
| J-7 | S | Q-158 ($R_7$=H, $R_8$=H, $R_9$=$CH_3$) | H | H | $CH_3$ | $OCH_3$ |
| J-5 | S | Q-158 ($R_7$=$CH_3$, $R_8$=H, $R_9$=H) | H | 5-$CH_3$ | $OCH_3$ | $OCH_3$ |
| J-6 | NH | Q-159 ($R_7$=H, $R_8$=H, $R_9$=H) | H | H | $OCH_3$ | $OCH_3$ |
| J-5 | S | Q-159 ($R_7$=$CH_3$, $R_8$=H, $R_9$=H) | H | H | $CH_3$ | $OCH_3$ |
| J-6 | S | Q-159 ($R_7$=H, $R_8$=H, $R_9$=$CH_3$) | H | H | $CH_3$ | $OCH_3$ |
| J-5 | S | Q-160 ($R_7$=H, $R_8$=H, $R_9$=H, $R_{11}$=$CH_3$) | H | H | $CH_3$ | $OCH_3$ |
| J-6 | S | Q-160 ($R_7$=H, $R_8$=H, $R_9$=$CH_3$, $R_{11}$=H) | $CH_3$ | H | $CH_3$ | $OCH_3$ |
| J-5 | O | Q-160 ($R_7$=$CH_3$, $R_8$=H, $R_9$=H, $R_{11}$=i-$C_3H_7$) | H | H | $OCH_3$ | $OCH_3$ |
| J-6 | S | Q-161 ($R_7$=H, $R_8$=H, $R_9$=H, $R_{11}$=$CH_3$) | H | H | $OCH_3$ | $OCH_3$ |
| J-5 | S | Q-161 ($R_7$=$CH_3$, $R_8$=H, $R_9$=H, $R_{11}$=H) | H | H | $OCH_3$ | $OCH_3$ |
| J-6 | S | Q-161 ($R_7$=H, $R_8$=H, $R_9$=$CH_3$, $R_{11}$=$CH_3$) | H | 5-$CH_3$ | $OCH_3$ | $OCH_3$ |
| J-7 | S | Q-162 ($R_7$=H, $R_8$=H, $R_9$=H, $R_{10}$=H) | H | H | $OCH_3$ | $OCH_3$ |
| J-5 | S | Q-162 ($R_7$=$CH_3$, $R_8$=H, $R_9$=H, $R_{10}$=H) | H | H | $OCH_3$ | $OCH_3$ |
| J-6 | S | Q-162 ($R_7$=H, $R_8$=H, $R_9$=H, $R_{10}$=$CH_3$) | H | 5-Cl | $OCH_3$ | $OCH_3$ |

Table 2 (continued)

| J | $W_3$ | Q | R | $R_{1b}$ | X | Y |
|---|---|---|---|---|---|---|
| J-5 | S | Q-163 ($R_7$=H, $R_8$=H, $R_9$=H, $R_2$=H) | H | H | $OCH_3$ | $OCH_3$ |
| J-6 | O | Q-163 ($R_7$=H, $R_8$=H, $R_9$=H, $R_2$=H) | H | H | $OCH_3$ | $OCH_3$ |
| J-6 | S | Q-163 ($R_7$=H, $R_8$=H, $R_9$=H, $R_2$=H) | H | H | $OCH_3$ | $OCH_3$ |
| J-5 | S | Q-164 ($R_7$=H) | H | H | $OCH_3$ | $OCH_3$ |
| J-6 | O | Q-164 ($R_7$=H) | H | H | $OCH_3$ | $OCH_3$ |
| J-6 | S | Q-164 ($R_7$=H) | H | H | $OCH_3$ | $OCH_3$ |
| J-5 | S | Q-165 ($R_7$=H, $R_3$=$CH_3$) | H | H | $OCH_3$ | $OCH_3$ |
| J-6 | O | Q-165 ($R_7$=H, $R_3$=$CH_3$) | H | H | $OCH_3$ | $OCH_3$ |
| J-6 | S | Q-165 ($R_7$=H, $R_3$=$CH_3$) | H | H | $OCH_3$ | $OCH_3$ |
| J-5 | S | Q-166 ($R_7$=H, $R_3$=$CH_3$) | H | H | $OCH_3$ | $OCH_3$ |
| J-6 | O | Q-166 ($R_7$=H, $R_3$=$CH_3$) | H | H | $OCH_3$ | $OCH_3$ |
| J-6 | S | Q-166 ($R_7$=H, $R_3$=$CH_3$) | H | H | $OCH_3$ | $OCH_3$ |
| J-5* | S | Q-1($R_7$=H, $R_8$=H) | H | H | $OCH_3$ | $OCH_3$ |
| J-5* | S | Q-1($R_7$=H, $R_8$=H) | H | H | $OCH_3$ | $CH_3$ |
| J-6* | S | Q-1($R_7$=H, $R_8$=H) | H | H | $OCH_3$ | $OCH_3$ |
| J-7* | S | Q-1($R_7$=H, $R_8$=H) | H | H | $OCH_3$ | $OCH_3$ |
| J-6* | S | Q-37($R_7$=H, $R_8$=H, $R_2$=H) | H | H | $OCH_3$ | $OCH_3$ |
| J-6* | S | Q-60($R_7$=H, $R_8$=H, $R_9$=H, $R_2$=H) | H | H | $OCH_3$ | $OCH_3$ |
| J-6* | S | Q-105($R_7$=H, $R_8$=H, $R_9$=H, $R_2$=H) | H | H | $OCH_3$ | $OCH_3$ |
| J-6* | S | Q-138($R_7$=H, $R_8$=H, $R_9$=H) | H | H | $OCH_3$ | $OCH_3$ |

Table 2 (continued)

| J | Q | R | $R_{1c}$ | X | Y |
|---|---|---|---|---|---|
| J-8 | Q-1 ($R_7$=H, $R_8$=H) | H | H | $CH_3$ | $CH_3$ |
| J-8 | Q-1 ($R_7$=H, $R_8$=H) | H | H | $OCH_3$ | $CH_3$ |
| J-8 | Q-1 ($R_7$=H, $R_8$=H) | H | H | $OCH_3$ | $OCH_3$ |
| J-8 | Q-1 ($R_7$=H, $R_8$=H) | H | 5-$CH_3$ | $OCH_3$ | $OCH_3$ |
| J-8 | Q-1 ($R_7$=H, $R_8$=H) | H | 5-$OCH_3$ | $OCH_3$ | $OCH_3$ |
| J-8 | Q-1 ($R_7$=H, $R_8$=H) | H | 6-$OCH_2CH_2Br$ | $OCH_3$ | $OCH_3$ |
| J-8 | Q-1 ($R_7$=H, $R_8$=H) | H | 5-$CH_2F$ | $OCH_3$ | $OCH_3$ |
| J-8 | Q-1 ($R_7$=H, $R_8$=H) | H | 5-$SCH_3$ | $OCH_3$ | $OCH_3$ |
| J-8 | Q-1 ($R_7$=H, $R_8$=H) | H | 5-$SCH_2F$ | $OCH_3$ | $OCH_3$ |
| J-8 | Q-1 ($R_7$=H, $R_8$=H) | H | 5-$NHCH_3$ | $OCH_3$ | $OCH_3$ |
| J-8 | Q-1 ($R_7$=H, $R_8$=H) | H | 5-Cl | $OCH_3$ | $OCH_3$ |
| J-8 | Q-1 ($R_7$=H, $R_8$=H) | H | 4-$NO_2$ | $OCH_3$ | $OCH_3$ |
| J-8 | Q-1 ($R_7$=H, $R_8$=H) | H | 5-$CF_3$ | $OCH_3$ | $OCH_3$ |
| J-8 | Q-1 ($R_7$=H, $R_8$=H) | H | H | $OCH_3$ | $CH_2CH_3$ |
| J-8 | Q-1 ($R_7$=H, $R_8$=H) | H | H | $OCH_3$ | $OCH_2CH_3$ |
| J-8 | Q-1 ($R_7$=H, $R_8$=H) | H | H | $OCH_2CF_3$ | $OCH_3$ |
| J-8 | Q-1 ($R_7$=H, $R_8$=H) | H | H | $CF_3$ | $OCH_3$ |
| J-8 | Q-1 ($R_7$=H, $R_8$=H) | H | H | $SCH_3$ | $OCH_3$ |
| J-8 | Q-1 ($R_7$=H, $R_8$=H) | H | H | $SCHF_2$ | $OCH_3$ |
| J-8 | Q-1 ($R_7$=H, $R_8$=H) | H | H | $OCH_3$ | $CH_2OCH_3$ |
| J-8 | Q-1 ($R_7$=H, $R_8$=H) | H | H | $OCH_3$ | $NH_2$ |
| J-8 | Q-1 ($R_7$=H, $R_8$=H) | H | H | $OCH_3$ | $NHCH_3$ |
| J-8 | Q-1 ($R_7$=H, $R_8$=H) | H | H | H | $OCH_3$ |
| J-8 | Q-1 ($R_7$=H, $R_8$=H) | H | H | $OCH_3$ | $OCH_2C{\equiv}CH$ |
| J-8 | Q-1 ($R_7$=H, $R_8$=H) | H | H | $OCH_3$ | $CH_2S(CH_2)_3CH_3$ |
| J-8 | Q-1 ($R_7$=H, $R_8$=H) | H | H | $OCH_3$ | cyclopropyl |
| J-8 | Q-1 ($R_7$=H, $R_8$=H) | H | H | $OCH_3$ | $C{\equiv}CH$ |
| J-8 | Q-1 ($R_7$=H, $R_8$=H) | H | H | $OCH_3$ | $-CH(OCH_3)_2$ |
| J-8 | Q-2 ($R_7$=H, $R_8$=H) | H | H | $OCH_3$ | $OCH_3$ |
| J-8 | Q-3 ($R_7$=H, $R_8$=H) | H | H | $OCH_3$ | $OCH_3$ |

Table 2 (continued)

| J | Q | R | $R_{1c}$ | X | Y |
|---|---|---|---|---|---|
| J-8 | Q-4 ($R_7$=H, $R_8$=H, $R_3$=$CH_3$) | H | H | $CH_3$ | $OCH_3$ |
| J-8 | Q-5 ($R_7$-H, $R_8$=H, $R_3$=H) | H | H | $OCH_3$ | $OCH_3$ |
| J-8 | Q-6 ($R_7$=$CH_3$, $R_8$=H, $R_3$=$CH_3$) | H | H | $CH_3$ | $OCH_3$ |
| J-8 | Q-8 ($R_7$=H, $R_8$=H, $R_2$=H) | H | H | $OCH_3$ | $OCH_3$ |
| J-8 | Q-9 ($R_7$=H, $R_8$=H, $R_2$=H) | H | H | $OCH_3$ | $OCH_3$ |
| J-8 | Q-10 ($R_7$=H, $R_8$=H) | H | H | $OCH_3$ | $OCH_3$ |
| J-8 | Q-11 ($R_7$=H, $R_8$=H) | H | H | $CH_3$ | $OCH_3$ |
| J-8 | Q-12 ($R_7$=H, $R_8$=H) | H | H | $OCH_3$ | $OCH_3$ |
| J-8 | Q-13 ($R_7$=H, $R_8$=H, $R_3$=H) | H | H | $CH_3$ | $OCH_3$ |
| J-8 | Q-14 ($R_7$=H, $R_8$=H, $R_3$=$CH_3$) | H | H | $OCH_3$ | $OCH_3$ |
| J-8 | Q-15 ($R_7$=CH , $R_8$=H, $R_3$=H) | H | H | $OCH_3$ | $OCH_3$ |
| J-8 | Q-17 ($R_7$=H, $R_8$=H, $R_2$=H) | H | H | $OCH_3$ | $OCH_3$ |
| J-8 | Q-18 ($R_7$=H, $R_8$=H, $R_2$=H) | H | H | $CH_3$ | $OCH_3$ |
| J-8 | Q-19 ($R_7$=H, $R_8$=H) | H | H | $OCH_3$ | $OCH_3$ |
| J-8 | Q-20 ($R_7$=H, $R_8$=H) | H | H | $CH_3$ | $OCH_3$ |
| J-8 | Q-21 ($R_7$=H, $R_8$=H) | H | H | $OCH_3$ | $OCH_3$ |
| J-8 | Q-22 ($R_7$=H, $R_8$=H, $R_3$=H) | H | H | $OCH_3$ | $OCH_3$ |
| J-8 | Q-23 ($R_7$=H, $R_8$=H, $R_3$=H) | H | H | $OCH_3$ | $OCH_3$ |
| J-8 | Q-24 ($R_7$=H, $R_8$=H, $R_3$=$CH_3$) | H | H | $OCH_3$ | $OCH_3$ |
| J-8 | Q-25 ($R_7$=H, $R_8$=H, $R_9$=H) | H | H | $CH_3$ | $OCH_3$ |
| J-8 | Q-26 ($R_7$=H, $R_8$=H) | H | H | $OCH_3$ | $OCH_3$ |
| J-8 | Q-27 ($R_7$=H, $R_8$=H) | H | H | $CH_3$ | $OCH_3$ |
| J-8 | Q-28 ($R_7$=H, $R_8$=H) | H | H | $OCH_3$ | $OCH_3$ |
| J-8 | Q-29 ($R_7$=H, $R_8$=H) | H | H | $OCH_3$ | $OCH_3$ |
| J-8 | Q-30 ($R_7$=H, $R_8$=H) | H | H | $OCH_3$ | $OCH_3$ |
| J-8 | Q-31 ($R_7$=H, $R_8$=H) | H | H | $OCH_3$ | $OCH_3$ |
| J-8 | Q-32 ($R_7$=H, $R_8$=H, $R_3$=H) | H | H | $CH_3$ | $OCH_3$ |
| J-8 | Q-33 ($R_7$=H, $R_8$=H, $R_8$=$CH_3$) | H | H | $OCH_3$ | $OCH_3$ |
| J-8 | Q-34 ($R_7$=H, $R_8$=H, $R_3$=H) | H | H | $CH_3$ | $OCH_3$ |

Table 2 (continued)

| J | Q | R | $R_{1c}$ | X | Y |
|---|---|---|---|---|---|
| J-8 | Q-36 ($R_7$=H, $R_8$=H, $R_2$=H) | H | H | $OCH_3$ | $OCH_3$ |
| J-8 | Q-37 ($R_7$=H, $R_8$=H, $R_2$=H) | H | H | $OCH_3$ | $OCH_3$ |
| J-8 | Q-38 ($R_7$=H, $R_8$=H, $R_2$=$OCF_2H$) | H | H | $OCH_3$ | $OCH_3$ |
| J-8 | Q-39 ($R_7$=H, $R_8$=H, $R_9$=H) | H | H | $CH_3$ | $OCH_3$ |
| J-8 | Q-40 ($R_7$=H, $R_8$=H) | H | H | $OCH_3$ | $OCH_3$ |
| J-8 | Q-41 ($R_7$=H, $R_8$=H) | H | H | $CH_3$ | $OCH_3$ |
| J-8 | Q-42 ($R_7$=H, $R_8$=H) | H | H | $OCH_3$ | $OCH_3$ |
| J-8 | Q-43 ($R_7$=H, $R_8$=H, $R_3$=H) | H | H | $OCH_3$ | $OCH_3$ |
| J-8 | Q-44 ($R_7$=H, $R_8$=H, $R_3$=$CH_3$) | H | H | $OCH_3$ | $OCH_3$ |
| J-8 | Q-45 ($R_7$=H, $R_8$=H, $R_3$=H) | H | H | $OCH_3$ | $OCH_3$ |
| J-8 | Q-47 ($R_7$=H, $R_8$=H, $R_2$=H) | H | H | $OCH_3$ | $OCH_3$ |
| J-8 | Q-48 ($R_7$=H, $R_8$=H, $R_2$=H) | H | H | $CH_3$ | $OCH_3$ |
| J-8 | Q-49 ($R_7$=H, $R_8$=H, $R_2$=H) | H | H | $OCH_3$ | $OCH_3$ |
| J-8 | Q-50 ($R_7$=H, $R_8$=H, $R_2$=H) | H | H | $OCH_3$ | $OCH_3$ |
| J-8 | Q-51 ($R_7$=H, $R_8$=H, $R_9$=H) | H | H | $OCH_3$ | $OCH_3$ |
| J-8 | Q-52 ($R_7$=H, $R_8$=H, $R_9$=H) | H | H | $OCH_3$ | $OCH_3$ |
| J-8 | Q-53 ($R_7$=H, $R_8$=H, $R_9$=H) | H | H | $CH_3$ | $OCH_3$ |
| J-8 | Q-54 ($R_7$=H, $R_8$=H, $R_9$=H) | H | H | $OCH_3$ | $OCH_3$ |
| J-8 | Q-55 ($R_7$=H, $R_8$=H, $R_9$=H, $R_3$=H) | H | H | $CH_3$ | $OCH_3$ |
| J-8 | Q-56 ($R_7$=H, $R_8$=H, $R_9$=H, $R_3$=$C_2H_5$) | H | H | $CH_3$ | $OCH_3$ |
| J-8 | Q-57 ($R_7$=H, $R_8$=H, $R_9$=H, $R_3$=$\underline{i}$-$C_3H_7$) | H | H | $OCH_3$ | $OCH_3$ |
| J-8 | Q-58 ($R_7$=H, $R_8$=H, $R_9$=H, $R_3$=H) | H | H | $OCH_3$ | $OCH_3$ |
| J-8 | Q-60 ($R_7$=H, $R_8$=H, $R_9$=H, $R_2$=H) | H | H | $CH_3$ | $OCH_3$ |
| J-8 | Q-61 ($R_7$=H, $R_8$=H, $R_9$=H, $R_2$=H) | H | H | $OCH_3$ | $OCH_3$ |
| J-8 | Q-62 ($R_7$=H, $R_8$=H, $R_9$=H, $R_2$=H) | H | H | $CH_3$ | $OCH_3$ |
| J-8 | Q-63 ($R_7$=H, $R_8$=H, $R_9$=H) | H | H | $OCH_3$ | $OCH_3$ |
| J-8 | Q-64 ($R_7$=H, $R_8$=H, $R_9$=H) | H | H | $OCH_3$ | $OCH_3$ |
| J-8 | Q-65 ($R_7$=H, $R_8$=H, $R_9$=H) | H | H | $OCH_3$ | $OCH_3$ |
| J-8 | Q-66 ($R_7$=H, $R_8$=H, $R_9$=H) | H | H | $OCH_3$ | $OCH_3$ |

Table 2 (continued)

| J | Q | R | $R_{1c}$ | X | Y |
|---|---|---|---|---|---|
| J-8 | Q-67 ($R_7$=H, $R_8$=H, $R_9$=H, $R_3$=H) | H | H | $CH_3$ | $OCH_3$ |
| J-8 | Q-68 ($R_7$=H, $R_8$=H, $R_9$=H, $R_3$=$CH_3$) | H | H | $OCH_3$ | $OCH_3$ |
| J-8 | Q-69 ($R_7$=H, $R_8$=H, $R_9$=H, $R_3$=$CH_3$) | H | H | $CH_3$ | $OCH_3$ |
| J-8 | Q-70 ($R_7$=H, $R_8$=H, $R_9$=H, $R_3$=$CH_3$) | H | H | $OCH_3$ | $OCH_3$ |
| J-8 | Q-72 ($R_7$=H, $R_8$=H, $R_9$=H, $R_2$=H) | H | H | $OCH_3$ | $OCH_3$ |
| J-8 | Q-73 ($R_7$=H, $R_8$=H, $R_9$=H, $R_2$=H) | H | H | $OCH_3$ | $OCH_3$ |
| J-8 | Q-74 ($R_7$=H, $R_8$=H, $R_9$=H, $R_2$=H) | H | H | $CH_3$ | $OCH_3$ |
| J-8 | Q-75 ($R_7$=H, $R_8$=H, $R_9$=H) | H | H | $OCH_3$ | $OCH_3$ |
| J-8 | Q-76 ($R_7$=H, $R_8$=H, $R_9$=H) | H | H | $CH_3$ | $OCH_3$ |
| J-8 | Q-77 ($R_7$=H, $R_8$=H, $R_9$=H) | H | H | $OCH_3$ | $OCH_3$ |
| J-8 | Q-78 ($R_7$=H, $R_8$=H, $R_9$=H) | H | H | $OCH_3$ | $OCH_3$ |
| J-8 | Q-79 ($R_7$=H, $R_8$=H, $R_9$=H, $R_3$=H) | H | H | $OCH_3$ | $OCH_3$ |
| J-8 | Q-80 ($R_7$=H, $R_8$=H, $R_9$=H, $R_3$=$CH_3$) | H | H | $OCH_3$ | $OCH_3$ |
| J-8 | Q-81 ($R_7$=H, $R_8$=H, $R_9$=H, $R_3$=$CH_3$) | H | H | $CH_3$ | $OCH_3$ |
| J-8 | Q-82 ($R_7$=H, $R_8$=H, $R_9$=H, $R_3$=$CH_3$) | H | H | $OCH_3$ | $OCH_3$ |
| J-8 | Q-83 ($R_7$=H, $R_8$=H, $R_9$=H, $R_{10}$=H) | H | H | $CH_3$ | $OCH_3$ |
| J-8 | Q-84 ($R_7$=H, $R_8$=H, $R_9$=H) | H | H | $OCH_3$ | $OCH_3$ |
| J-8 | Q-85 ($R_7$=H, $R_8$=H, $R_9$=H) | H | H | $OCH_3$ | $OCH_3$ |
| J-8 | Q-86 ($R_7$=H, $R_8$=H, $R_9$=H) | H | H | $OCH_3$ | $OCH_3$ |
| J-8 | Q-87 ($R_7$=H, $R_8$=H, $R_9$=H) | H | H | $OCH_3$ | $OCH_3$ |
| J-8 | Q-88 ($R_7$=H, $R_8$=H, $R_9$=H) | H | H | $CH_3$ | $OCH_3$ |
| J-8 | Q-89 ($R_7$=H, $R_8$=H, $R_9$=H) | H | H | $OCH_3$ | $OCH_3$ |
| J-8 | Q-90 ($R_7$=H, $R_8$=H, $R_9$=H, $R_3$=$CH_3$) | H | H | $CH_3$ | $OCH_3$ |
| J-8 | Q-91 ($R_7$=H, $R_8$=H, $R_9$=H, $R_3$=$CH_3$) | H | H | $OCH_3$ | $OCH_3$ |
| J-8 | Q-92 ($R_7$=H, $R_8$=H, $R_9$=H, $R_{10}$=H) | H | H | $OCH_3$ | $OCH_3$ |
| J-8 | Q-93 ($R_7$=H, $R_8$=H, $R_9$=H) | H | H | $OCH_3$ | $OCH_3$ |
| J-8 | Q-94 ($R_7$=H, $R_8$=H, $R_9$=H) | H | H | $OCH_3$ | $OCH_3$ |
| J-8 | Q-95 ($R_7$=H, $R_8$=H, $R_9$=H) | H | H | $CH_3$ | $OCH_3$ |
| J-8 | Q-96 ($R_7$=H, $R_8$=H, $R_9$=H) | H | H | $OCH_3$ | $OCH_3$ |
| J-8 | Q-97 ($R_7$=H, $R_8$=H, $R_9$=H) | H | H | $CH_3$ | $OCH_3$ |
| J-8 | Q-98 ($R_7$=H, $R_8$=H, $R_9$=H) | H | H | $OCH_3$ | $OCH_3$ |
| J-8 | Q-99 ($R_7$=H, $R_8$=H, $R_9$=H, $R_3$=H) | H | H | $OCH_3$ | $OCH_3$ |

Table 2 (continued)

| J | Q | R | $R_{1c}$ | X | Y |
|---|---|---|---|---|---|
| J-8 | Q-100 ($R_7$=H, $R_8$=H, $R_9$=H, $R_3$=$C_2H_5$) | H | H | $OCH_3$ | $OCH_3$ |
| J-8 | Q-101 ($R_7$=H, $R_8$=H, $R_9$=H, $R_3$=i-$C_2H_5$) | H | H | $OCH_3$ | $OCH_3$ |
| J-8 | Q-102 ($R_7$=H, $R_8$=H, $R_9$=H, $R_3$=H) | H | H | $CH_3$ | $OCH_3$ |
| J-8 | Q-104 ($R_7$=H, $R_8$=H, $R_9$=H, $R_2$=H) | H | H | $CH_3$ | $OCH_3$ |
| J-8 | Q-105 ($R_7$=H, $R_8$=H, $R_9$=H, $R_2$=H) | H | H | $OCH_3$ | $OCH_3$ |
| J-8 | Q-106 ($R_7$=H, $R_8$=H, $R_9$=H, $R_2$=H) | H | H | $OCH_3$ | $OCH_3$ |
| J-8 | Q-107 ($R_7$=H, $R_8$=H, $R_9$=H, $R_2$=H) | H | H | $OCH_3$ | $OCH_3$ |
| J-8 | Q-108 ($R_7$=H, $R_8$=H, $R_9$=H, $R_2$=H) | H | H | $OCH_3$ | $OCH_3$ |
| J-8 | Q-109 ($R_7$=H, $R_8$=H, $R_9$=H) | H | H | $CH_3$ | $OCH_3$ |
| J-8 | Q-110 ($R_7$=H, $R_8$=H, $R_9$=H) | H | H | $OCH_3$ | $OCH_3$ |
| J-8 | Q-111 ($R_7$=H, $R_8$=H, $R_9$=H) | H | H | $CH_3$ | $OCH_3$ |
| J-8 | Q-112 ($R_7$=H, $R_8$=H, $R_9$=H) | H | H | $OCH_3$ | $OCH_3$ |
| J-8 | Q-113 ($R_7$=H, $R_8$=H, $R_9$=H, $R_3$=$CH_3$) | H | H | $OCH_3$ | $OCH_3$ |
| J-8 | Q-114 ($R_7$=H, $R_8$=H, $R_9$=H, $R_3$=$CH_3$) | H | H | $OCH_3$ | $OCH_3$ |
| J-8 | Q-115 ($R_7$=H, $R_8$=H, $R_9$=H, $R_3$=$CH_3$) | H | H | $OCH_3$ | $OCH_3$ |
| J-8 | Q-116 ($R_7$=H, $R_8$=H, $R_9$=H, $R_{10}$=H) | H | H | $CH_3$ | $OCH_3$ |
| J-8 | Q-118 ($R_7$=H, $R_8$=H, $R_9$=H, $R_{10}$=H) | H | H | $CH_3$ | $OCH_3$ |
| J-8 | Q-119 ($R_7$=H, $R_8$=H, $R_9$=H, $R_2$=H) | H | H | $OCH_3$ | $OCH_3$ |
| J-8 | Q-120 ($R_7$=H, $R_8$=H, $R_9$=H, $R_2$=H) | H | H | $OCH_3$ | $OCH_3$ |
| J-8 | Q-121 ($R_7$=H, $R_8$=H, $R_9$=H, $R_2$=H) | H | H | $OCH_3$ | $OCH_3$ |
| J-8 | Q-122 ($R_7$=H, $R_8$=H, $R_9$=H, $R_2$=H) | H | H | $OCH_3$ | $OCH_3$ |
| J-8 | Q-123 ($R_7$=H, $R_8$=H, $R_9$=H) | H | H | $CH_3$ | $OCH_3$ |
| J-8 | Q-124 ($R_7$=H, $R_8$=H, $R_9$=H) | H | H | $OCH_3$ | $OCH_3$ |
| J-8 | Q-125 ($R_7$=H, $R_8$=H, $R_9$=H) | H | H | $CH_3$ | $OCH_3$ |
| J-8 | Q-126 ($R_7$=H, $R_8$=H, $R_9$=H) | H | H | $OCH_3$ | $OCH_3$ |
| J-8 | Q-127 ($R_7$=H, $R_8$=H, $R_9$=H, $R_3$=H) | H | H | $OCH_3$ | $OCH_3$ |
| J-8 | Q-128 ($R_7$=H, $R_8$=H, $R_9$=H, $R_3$=$CH_3$) | H | H | $OCH_3$ | $OCH_3$ |
| J-8 | Q-129 ($R_7$=H, $R_8$=H, $R_9$=H, $R_3$=$CH_3$) | H | H | $OCH_3$ | $OCH_3$ |
| J-8 | Q-130 ($R_7$=H, $R_8$=H, $R_9$=H, $R_3$=$CH_3$) | H | H | $CH_3$ | $OCH_3$ |
| J-8 | Q-131 ($R_7$=H, $R_8$=H, $R_9$=H, $R_{10}$=H) | H | H | $OCH_3$ | $OCH_3$ |

Table 2 (continued)

| J | Q | R | $R_{1c}$ | X | Y |
|---|---|---|---|---|---|
| J-8 | Q-132 ($R_7$=H, $R_8$=H, $R_9$=H) | H | H | $CH_3$ | $OCH_3$ |
| J-8 | Q-133 ($R_7$=H, $R_8$=H, $R_9$=H) | H | H | $OCH_3$ | $OCH_3$ |
| J-8 | Q-134 ($R_7$=H, $R_8$=H, $R_9$=H) | H | H | $OCH_3$ | $OCH_3$ |
| J-8 | Q-135 ($R_7$=H, $R_8$=H, $R_9$=H) | H | H | $OCH_3$ | $OCH_3$ |
| J-8 | Q-136 ($R_7$=H, $R_8$=H, $R_9$=H) | H | H | $OCH_3$ | $OCH_3$ |
| J-8 | Q-137 ($R_7$=H, $R_8$=H, $R_9$=H) | H | H | $CH_3$ | $OCH_3$ |
| J-8 | Q-138 ($R_7$=H, $R_8$=H, $R_9$=H) | H | H | $OCH_3$ | $OCH_3$ |
| J-8 | Q-139 ($R_7$=H, $R_8$=H, $R_9$=H) | H | H | $CH_3$ | $OCH_3$ |
| J-8 | Q-140 ($R_7$=H, $R_8$=H, $R_9$=H, $R_3$=$CH_3$) | H | H | $OCH_3$ | $OCH_3$ |
| J-8 | Q-141 ($R_7$=H, $R_8$=H, $R_9$=H, $R_3$=$CH_3$) | H | H | $OCH_3$ | $OCH_3$ |
| J-8 | Q-142 ($R_7$=H, $R_8$=H, $R_9$=H, $R_{10}$=H) | H | H | $OCH_3$ | $OCH_3$ |
| J-8 | Q-143 ($R_7$=H, $R_8$=H, $R_9$=H, $R_3$=$CH_3$) | H | H | $OCH_3$ | $OCH_3$ |
| J-8 | Q-144 ($R_7$=H, $R_8$=H, $R_9$=H, $R_3$=$CH_3$) | H | H | $CH_3$ | $OCH_3$ |
| J-8 | Q-145 ($R_7$=H, $R_8$=H, $R_9$=H) | H | H | $OCH_3$ | $OCH_3$ |
| J-8 | Q-146 ($R_7$=H, $R_8$=H, $R_9$=H) | H | H | $CH_3$ | $OCH_3$ |
| J-8 | Q-147 ($R_7$=H, $R_8$=H, $R_9$=H) | H | H | $OCH_3$ | $OCH_3$ |
| J-8 | Q-148 ($R_7$=H, $R_8$=H, $R_9$=H) | H | H | $OCH_3$ | $OCH_3$ |
| J-8 | Q-149 ($R_7$=H, $R_8$=H, $R_9$=H) | H | H | $OCH_3$ | $OCH_3$ |
| J-8 | Q-150 ($R_7$=H, $R_8$=H, $R_9$=H) | H | H | $OCH_3$ | $OCH_3$ |
| J-8 | Q-151 ($R_7$=H, $R_8$=H, $R_9$=H) | H | H | $CH_3$ | $OCH_3$ |
| J-8 | Q-152 ($R_7$=H, $R_8$=H, $R_9$=H) | H | H | $OCH_3$ | $OCH_3$ |
| J-8 | Q-153 ($R_7$=H, $R_8$=H, $R_9$=H, $R_{11}$=$CH_3$) | H | H | $CH_3$ | $OCH_3$ |
| J-8 | Q-154 ($R_7$=H, $R_8$=H, $R_9$=H, $R_{11}$=$CH_3$) | H | H | $OCH_3$ | $OCH_3$ |
| J-8 | Q-155 ($R_7$=H, $R_8$=H, $R_9$=H) | H | H | $OCH_3$ | $OCH_3$ |
| J-8 | Q-156 ($R_7$=H, $R_8$=H, $R_9$=H) | H | H | $OCH_3$ | $OCH_3$ |
| J-8 | Q-158 ($R_7$=H, $R_8$=H, $R_9$=H) | H | H | $CH_3$ | $OCH_3$ |
| J-8 | Q-159 ($R_7$=H, $R_8$=H, $R_9$=H) | H | H | $OCH_3$ | $OCH_3$ |
| J-8 | Q-160 ($R_7$=H, $R_8$=H, $R_9$=H, $R_{11}$=$CH_3$) | H | H | $CH_3$ | $OCH_3$ |
| J-8 | Q-161 ($R_7$=H, $R_8$=H, $R_9$=H, $R_{11}$=$CH_3$) | H | H | $OCH_3$ | $OCH_3$ |
| J-8 | Q-162 ($R_7$=H, $R_8$=H, $R_9$=H, $R_{10}$=H) | H | H | $OCH_3$ | $OCH_3$ |
| J-8 | Q-163 ($R_2$=H, $R_7$=H, $R_8$=H, $R_9$=H) | H | H | $OCH_3$ | $OCH_3$ |
| J-8 | Q-164 ($R_7$=H) | H | H | $OCH_3$ | $OCH_3$ |
| J-8 | Q-165 ($R_3$=H, $R_7$=H) | H | H | $OCH_3$ | $OCH_3$ |
| J-8 | Q-166 ($R_3$=H, $R_7$=H) | H | H | $OCH_3$ | $OCH_3$ |

Table 2 (continued)

| J | Q | R | $R_{1c}$ | X | Y |
|---|---|---|---|---|---|
| J-9 | Q-1 ($R_7$=H, $R_8$=H) | H | 5-$SCH_3$ | $OCH_3$ | $OCH_3$ |
| J-9 | Q-1 ($R_7$=H, $R_8$=H) | H | 5-$SOCH_3$ | $OCH_3$ | $OCH_3$ |
| J-9 | Q-1 ($R_7$=H, $R_8$=H) | H | 5-$SO_2CH_3$ | $OCH_3$ | $OCH_3$ |
| J-9 | Q-1 ($R_7$=H, $R_8$=H) | H | 5-$CO_2CH_3$ | $OCH_3$ | $OCH_3$ |
| J-9 | Q-1 ($R_7$=H, $R_8$=H) | H | 4-$SO_2N(CH_3)_2$ | $OCH_3$ | $OCH_3$ |
| J-9 | Q-1 ($R_7$=H, $R_8$=H) | H | 5-$NO_2$ | $OCH_3$ | $OCH_3$ |
| J-9 | Q-1 ($R_7$=H, $R_8$=H) | H | 5-Cl | $OCH_3$ | $OCH_3$ |
| J-9 | Q-1 ($R_7$=H, $R_8$=H) | H | 4-$NO_2$ | $OCH_3$ | $OCH_3$ |
| J-9 | Q-1 ($R_7$=H, $R_8$=H) | H | 6-Cl | $OCH_3$ | $OCH_3$ |
| J-9 | Q-1 ($R_7$=H, $R_8$=H) | H | 6-$COOCH_2$ | $OCH_3$ | $OCH_3$ |
| J-9 | Q-1 ($R_7$=H, $R_8$=H) | H | 6-$NO_2$ | $OCH_3$ | $OCH_3$ |
| J-9 | Q-1 ($R_7$=H, $R_8$=H) | H | 4-$SO_2CH_3$ | $OCH_3$ | $OCH_3$ |
| J-9 | Q-1 ($R_7$=H, $R_8$=H) | H | 6-$OCH_3$ | $OCH_3$ | $OCH_3$ |
| J-9 | Q-1 ($R_7$=H, $R_8$=H) | H | 5-$OCH_3$ | $OCH_3$ | $OCH_3$ |
| J-9 | Q-1 ($R_7$=H, $R_8$=H) | H | H | $CH_3$ | $CH_3$ |
| J-9 | Q-1 ($R_7$=H, $R_8$=H) | H | H | $OCH_3$ | $CH_3$ |
| J-9 | Q-1 ($R_7$=H, $R_8$=H) | H | H | $OCH_3$ | $OCH_3$ |
| J-9 | Q-1 ($R_7$=H, $R_8$=H) | $CH_3$ | H | $OCH_3$ | $OCH_3$ |
| J-9 | Q-1 ($R_7$=H, $R_8$=H) | $CH_3$ | H | $OCH_3$ | $CH_3$ |
| J-9 | Q-1 ($R_7$=H, $R_8$=H) | H | 5-$CH_3$ | $OCH_3$ | $OCH_3$ |
| J-9 | Q-1 ($R_7$=H, $R_8$=H) | H | 6-$CH_2CH_3$ | $OCH_3$ | $OCH_3$ |
| J-9 | Q-1 ($R_7$=H, $R_8$=H) | H | 5-$CH(CH_3)_2$ | $OCH_3$ | $OCH_3$ |
| J-9 | Q-1 ($R_7$=H, $R_8$=H) | H | 5-$OCH_3$ | $OCH_3$ | $OCH_3$ |
| J-9 | Q-1 ($R_7$=H, $R_8$=H) | H | 5-$OCH_2CH_3$ | $OCH_3$ | $OCH_3$ |
| J-9 | Q-1 ($R_7$=H, $R_8$=H) | H | 5-$OCH(CH_3)_2$ | $OCH_3$ | $OCH_3$ |
| J-9 | Q-1 ($R_7$=H, $R_8$=H) | H | 5-$OCF_2H$ | $OCH_3$ | $OCH_3$ |
| J-9 | Q-1 ($R_7$=H, $R_8$=H) | H | 6-$OCH_2CH_2Br$ | $OCH_3$ | $OCH_3$ |
| J-9 | Q-1 ($R_7$=H, $R_8$=H) | H | 5-$OCH(CH_3)(CH_2Cl)$ | $OCH_3$ | $OCH_3$ |
| J-9 | Q-1 ($R_7$=H, $R_8$=H) | H | 5-$CH_2F$ | $OCH_3$ | $OCH_3$ |
| J-9 | Q-1 ($R_7$=H, $R_8$=H) | H | 5-$CH_2CH_2Br$ | $OCH_3$ | $OCH_3$ |
| J-9 | Q-1 ($R_7$=H, $R_8$=H) | H | 5-$CH(CH_3)(CH_2Cl)$ | $OCH_3$ | $OCH_3$ |
| J-9 | Q-1 ($R_7$=H, $R_8$=H) | H | 6-$SCH_2CH_3$ | $OCH_3$ | $OCH_3$ |

Table 2 (continued)

| J | Q | R | $R_{1c}$ | X | Y |
|---|---|---|---|---|---|
| J-9 | Q-1 ($R_7$=H, $R_8$=H) | H | 5-$SCH(CH_3)_2$ | $OCH_3$ | $OCH_3$ |
| J-9 | Q-1 ($R_7$=H, $R_8$=H) | H | 5-$SCH_2F$ | $OCH_3$ | $OCH_3$ |
| J-9 | Q-1 ($R_7$=H, $R_8$=H) | H | 5-$SCH_2CH_2Br$ | $OCH_3$ | $OCH_3$ |
| J-9 | Q-1 ($R_7$=H, $R_8$=H) | H | 5-$SCH(CH_3)(CH_2Cl)$ | $OCH_3$ | $OCH_3$ |
| J-9 | Q-1 ($R_7$=H, $R_8$=H) | H | 4-$NH_2$ | $OCH_3$ | $OCH_3$ |
| J-9 | Q-1 ($R_7$=H, $R_8$=H) | H | 5-$NHCH_3$ | $OCH_3$ | $OCH_3$ |
| J-9 | Q-1 ($R_7$=H, $R_8$=H) | H | 6-$NHCH_2CH_3$ | $OCH_3$ | $OCH_3$ |
| J-9 | Q-1 ($R_7$=H, $R_8$=H) | H | 5-$NHCH(CH_3)_2$ | $OCH_3$ | $OCH_3$ |
| J-9 | Q-1 ($R_7$=H, $R_8$=H) | H | 4-$N(CH_3)_2$ | $OCH_3$ | $OCH_3$ |
| J-9 | Q-1 ($R_7$=H, $R_8$=H) | H | 6-$N(CH_3)(CH_2CH_3)$ | $OCH_3$ | $OCH_3$ |
| J-9 | Q-1 ($R_7$=H, $R_8$=H) | H | 5-$N(CH_3)(CH(CH_3)_2)$ | $OCH_3$ | $OCH_3$ |
| J-9 | Q-1 ($R_7$=H, $R_8$=H) | H | 5-Cl | $OCH_3$ | $OCH_3$ |
| J-9 | Q-1 ($R_7$=H, $R_8$=H) | H | 5-Br | $OCH_3$ | $OCH_3$ |
| J-9 | Q-1 ($R_7$=H, $R_8$=H) | H | 4-F | $OCH_3$ | $OCH_3$ |
| J-9 | Q-1 ($R_7$=H, $R_8$=H) | H | 5-$CF_3$ | $OCH_3$ | $OCH_3$ |
| J-9 | Q-1 ($R_7$=H, $R_8$=H) | H | 5-$OCF_2H$ | $OCH_3$ | $OCH_3$ |
| J-9 | Q-1 ($R_7$=H, $R_8$=H) | H | H | $OCH_3$ | $CH_2CH_3$ |
| J-9 | Q-1 ($R_7$=H, $R_8$=H) | H | H | $OCH_3$ | $CH(CH_3)_2$ |
| J-9 | Q-1 ($R_7$=H, $R_8$=H) | H | H | $OCH_3$ | $(CH_2)_3CH_3$ |
| J-9 | Q-1 ($R_7$=H, $R_8$=H) | H | H | $OCH_3$ | $OCH_2CH_3$ |
| J-9 | Q-1 ($R_7$=H, $R_8$=H) | H | H | $OCH_3$ | $OCH(CH_3)_2$ |
| J-9 | Q-1 ($R_7$=H, $R_8$=H) | H | H | $OCH_3$ | $O(CH_2)_3CH_3$ |
| J-9 | Q-1 ($R_7$=H, $R_8$=H) | H | H | $OCH_2CH_2F$ | $OCH_3$ |
| J-9 | Q-1 ($R_7$=H, $R_8$=H) | H | H | $OCH_2CHF_2$ | $OCH_3$ |
| J-9 | Q-1 ($R_7$=H, $R_8$=H) | H | H | $OCH_2CF_3$ | $OCH_3$ |
| J-9 | Q-1 ($R_7$=H, $R_8$=H) | H | H | $OCH(CH_3)(CH_2Cl)$ | $OCH_3$ |
| J-9 | Q-1 ($R_7$=H, $R_8$=H) | H | H | $O(CH_2)_3CH_2Br$ | $OCH_3$ |
| J-9 | Q-1 ($R_7$=H, $R_8$=H) | H | H | $CH_2F$ | $OCH_3$ |
| J-9 | Q-1 ($R_7$=H, $R_8$=H) | H | H | $CH_2Cl$ | $OCH_3$ |
| J-9 | Q-1 ($R_7$=H, $R_8$=H) | H | H | $CH_2Br$ | $OCH_3$ |
| J-9 | Q-1 ($R_7$=H, $R_8$=H) | H | H | $CF_3$ | $OCH_3$ |
| J-9 | Q-1 ($R_7$=H, $R_8$=H) | H | H | $CH_2CH_2Br$ | $OCH_3$ |

Table 2 (continued)

| J | Q | R | $R_{1c}$ | X | Y |
|---|---|---|---|---|---|
| J-9 | Q-1 ($R_7$=H, $R_8$=H) | H | H | $CH(CH_3)(CH_2Cl)$ | $OCH_3$ |
| J-9 | Q-1 ($R_7$=H, $R_8$=H) | H | H | $(CH_2)_3CH_2I$ | $OCH_3$ |
| J-9 | Q-1 ($R_7$=H, $R_8$=H) | H | H | $SCH_3$ | $OCH_3$ |
| J-9 | Q-1 ($R_7$=H, $R_8$=H) | H | H | $SCH_2CH_3$ | $OCH_3$ |
| J-9 | Q-1 ($R_7$=H, $R_8$=H) | H | H | $SCH(CH_3)_2$ | $OCH_3$ |
| J-9 | Q-1 ($R_7$=H, $R_8$=H) | H | H | $S(CH_2)_3CH_3$ | $OCH_3$ |
| J-9 | Q-1 ($R_7$=H, $R_8$=H) | H | H | $SCHF_2$ | $OCH_3$ |
| J-9 | Q-1 ($R_7$=H, $R_8$=H) | H | H | $SCH_2CH_2Br$ | $OCH_3$ |
| J-9 | Q-1 ($R_7$=H, $R_8$=H) | H | H | $SCH(CH_3)(CH_2Cl)$ | $OCH_3$ |
| J-9 | Q-1 ($R_7$=H, $R_8$=H) | H | H | $S(CH_2)_3CH_2F$ | $OCH_3$ |
| J-9 | Q-1 ($R_7$=H, $R_8$=H) | H | H | $OCH_3$ | $CH_2OCH_3$ |
| J-9 | Q-1 ($R_7$=H, $R_8$=H) | H | H | $OCH_3$ | $CH_2CH_2OCH(CH_3)_2$ |
| J-9 | Q-1 ($R_7$=H, $R_8$=H) | H | H | $OCH_3$ | $CH(CH_3)(CH_2OCH_3)$ |
| J-9 | Q-1 ($R_7$=H, $R_8$=H) | H | H | $OCH_3$ | $(CH_2)_4CH_2OCH_2CH_3$ |
| J-9 | Q-1 ($R_7$=H, $R_8$=H) | H | H | $OCH_2O(CH_2)_3CH_3$ | $OCH_3$ |
| J-9 | Q-1 ($R_7$=H, $R_8$=H) | H | H | $OCH_2CH_2OCH(CH_3)_2$ | $OCH_3$ |
| J-9 | Q-1 ($R_7$=H, $R_8$=H) | H | H | $OCH(CH_3)(CH_2OCH_3)$ | $OCH_3$ |
| J-9 | Q-1 ($R_7$=H, $R_8$=H) | H | H | $O(CH_2)_4CH_2OCH_2CH_3$ | $OCH_3$ |
| J-9 | Q-1 ($R_7$=H, $R_8$=H) | H | H | $OCH_3$ | $NH_2$ |
| J-9 | Q-1 ($R_7$=H, $R_8$=H) | H | H | $OCH_3$ | $NHCH_3$ |
| J-9 | Q-1 ($R_7$=H, $R_8$=H) | H | H | $OCH_3$ | $NHCH_2CH_3$ |
| J-9 | Q-1 ($R_7$=H, $R_8$=H) | H | H | $OCH_3$ | $NHCH(CH_3)_2$ |
| J-9 | Q-1 ($R_7$=H, $R_8$=H) | H | H | $OCH_3$ | $N(CH_3)_2$ |
| J-9 | Q-1 ($R_7$=H, $R_8$=H) | H | H | $OCH_3$ | $N(CH_3)(CH_2CH_3)$ |
| J-9 | Q-1 ($R_7$=H, $R_8$=H) | H | H | $OCH_3$ | $N(CH_3)(CH(CH_3)_2)$ |
| J-9 | Q-1 ($R_7$=H, $R_8$=H) | H | H | $OCH_3$ | $(CH_2)_4CH_2OCH_2CH_3$ |
| J-9 | Q-1 ($R_7$=H, $R_8$=H) | H | H | H | $OCH_3$ |
| J-9 | Q-1 ($R_7$=H, $R_8$=H) | H | H | $OCH_3$ | $OCH_2CH=CH_2$ |

Table 2 (continued)

| J | Q | R | $R_{1c}$ | X | Y |
|---|---|---|---|---|---|
| J-9 | Q-1 ($R_7$=H, $R_8$=H) | H | H | $OCH_3$ | $OCH_2C(CH_3)=CH_2$ |
| J-9 | Q-1 ($R_7$=H, $R_8$=H) | H | H | $OCH_3$ | $OCH_2C{\equiv}CH$ |
| J-9 | Q-1 ($R_7$=H, $R_8$=H) | H | H | $OCH_3$ | $OCH_2C{\equiv}CCH_3$ |
| J-9 | Q-1 ($R_7$=H, $R_8$=H) | H | H | $OCH_3$ | $CH_2S(CH_2)_3CH_3$ |
| J-9 | Q-1 ($R_7$=H, $R_8$=H) | H | H | $OCH_3$ | $CH_2CH_2SCH(CH_3)_2$ |
| J-9 | Q-1 ($R_7$=H, $R_8$=H) | H | H | $OCH_3$ | $CH(CH_3)(CH_2SCH_3)$ |
| J-9 | Q-1 ($R_7$=H, $R_8$=H) | H | H | $OCH_3$ | $(CH_2)_4CH_2SCH_2CH_3$ |
| J-9 | Q-1 ($R_7$=H, $R_8$=H) | H | H | $OCH_3$ | cyclopropyl |
| J-9 | Q-1 ($R_7$=H, $R_8$=H) | H | H | $OCH_3$ | 2-methylcyclopropyl |
| J-9 | Q-1 ($R_7$=H, $R_8$=H) | H | H | $OCH_3$ | cyclopentyl |
| J-9 | Q-1 ($R_7$=H, $R_8$=H) | H | H | $OCH_3$ | $C{\equiv}CH$ |
| J-9 | Q-1 ($R_7$=H, $R_8$=H) | H | H | $OCH_3$ | $C{\equiv}CCH_3$ |
| J-9 | Q-1 ($R_7$=H, $R_8$=H) | H | H | $OCH_3$ | -CHO |
| J-9 | Q-1 ($R_7$=H, $R_8$=H) | H | H | $OCH_3$ | $-COCH_3$ |
| J-9 | Q-1 ($R_7$=H, $R_8$=H) | H | H | $OCH_3$ | $-CH(OCH_3)_2$ |
| J-9 | Q-1 ($R_7$=H, $R_8$=H) | H | H | $OCH_3$ | $-CH(SCH_3)(OCH_2CH_3)$ |
| J-9 | Q-1 ($R_7$=H, $R_8$=H) | H | H | $OCH_3$ | $-C(CH_3)(SCH_3)_2$ |
| J-9 | Q-1 ($R_7$=H, $R_8$=H) | H | H | $OCH_3$ | $-CH(SCH_2CH_3)_2$ |
| J-9 | Q-1 ($R_7$=H, $R_8$=H) | H | H | $OCH_3$ | 1,3-dioxolan-2-yl |
| J-9 | Q-1 ($R_7$=H, $R_8$=H) | H | H | $OCH_3$ | 2-methyl-1,3-oxa-thiolan-2-yl |
| J-9 | Q-1 ($R_7$=H, $R_8$=H) | H | H | $OCH_3$ | 1,3-oxathian-2-yl |
| J-9 | Q-1 ($R_7$=H, $R_8$=H) | H | H | $OCH_3$ | 2-methyl-1,3-dithian-2-yl |
| J-9 | Q-1 ($R_7$=H, $R_8$=H) | H | H | $OCH_3$ | 4-methyl-1,3-dioxolan-2-yl |
| J-9 | Q-1 ($R_7$=H, $R_8$=H) | H | H | $OCH_3$ | 4-methyl-1,3-oxathiolan-2-yl |
| J-9 | Q-1 ($R_7$=H, $R_8$=H) | H | H | $OCH_3$ | 2,4-dimethyl-1,3-dithiolan-2-yl |
| J-9 | Q-1 ($R_7$=H, $R_8$=H) | H | H | $OCH_3$ | $N(OCH_3)(CH_3)_2$ |
| J-9 | Q-1 ($R_7$=H, $R_8$=$CH_3$) | H | H | $OCH_3$ | $OCH_3$ |
| J-9 | Q-1 ($R_7$=H, $R_8$=$CH_3$) | H | H | $CH_3$ | $CH_3$ |
| J-9 | Q-1 ($R_7$=H, $R_8$=$CH_3$) | H | H | $CH_3$ | $OCH_3$ |

Table 2 (continued)

| J | Q | R | $R_{1c}$ | X | Y |
|---|---|---|---|---|---|
| J-9 | Q-1 ($R_7$=H, $R_8$=$CH_2CH_3$) | H | H | $OCH_3$ | $OCH_3$ |
| J-9 | Q-1 ($R_7$=H, $R_8$=$CH(CH_3)_2$) | H | H | $OCH_3$ | $OCH_3$ |
| J-9 | Q-1 ($R_7$=H, $R_8$=$(CH_2)_3CH_3$) | H | H | $OCH_3$ | $OCH_3$ |
| J-9 | Q-1 ($R_7$=$CH_3$, $R_8$=$CH_3$) | H | H | $OCH_3$ | $OCH_3$ |
| J-9 | Q-2 ($R_7$=H, $R_8$=H) | H | H | $OCH_3$ | $OCH_3$ |
| J-9 | Q-2 ($R_7$=H, $R_8$=H) | $CH_3$ | H | $OCH_3$ | $OCH_3$ |
| J-9 | Q-2 ($R_7$=H, $R_8$=$CH_3)_3$ | H | 5-$OCH_3$ | $OCH_3$ | $OCH_3$ |
| J-9 | Q-3 ($R_7$=H, $R_8$=H) | H | H | $OCH_3$ | $OCH_3$ |
| J-9 | Q-3 ($R_7$=$CH_3$, $R_8$=H) | H | 5-Cl | $OCH_3$ | $CH_3$ |
| J-9 | Q-4 ($R_7$=H, $R_8$=H, $R_3$=$CH_3$) | H | H | $CH_3$ | $OCH_3$ |
| J-9 | Q-4 ($R_7$=H, $R_8$=H, $R_3$=$C_2H_5$) | H | H | $CH_3$ | $OCH_3$ |
| J-9 | Q-4 ($R_7$=H, $R_8$=H, $R_3$=i-$C_3H_7$) | H | 5-$CH_3$ | $OCH_3$ | $OCH_3$ |
| J-9 | Q-5 ($R_7$-H, $R_8$=H, $R_3$=H) | H | H | $OCH_3$ | $OCH_3$ |
| J-9 | Q-5 ($R_7$=H, $R_8$=$CH_3$, $R_3$=$CH_3$) | H | H | $CH_3$ | $OCH_3$ |
| J-9 | Q-6 ($R_7$=$CH_3$, $R_8$=H, $R_3$=$CH_3$) | H | H | $CH_3$ | $OCH_3$ |
| J-9 | Q-6 ($R_7$-H, $R_8$=H, $R_3$=$C_2H_5$) | $CH_3$ | H | $CH_3$ | $OCH_3$ |
| J-9 | Q-6 ($R_7$=$C_2H_5$, $R_8$=H, $R_3$=H) | H | H | $OCH_3$ | $OCH_3$ |
| J-9 | Q-8 ($R_7$=H, $R_8$=H, $R_2$=H) | H | H | $OCH_3$ | $OCH_3$ |
| J-9 | Q-8 ($R_7$=H, $R_8$=H, $R_2$=$OCH(CH_3)CH_2CH_3$) | H | 5-Cl | $OCH_3$ | $OCH_3$ |
| J-9 | Q-9 ($R_7$=H, $R_8$=H, $R_2$=H) | H | H | $OCH_3$ | $OCH_3$ |
| J-9 | Q-9 ($R_7$=$CH_3$, $R_8$=H, $R_2$=$CH_3$) | $CH_3$ | H | $OCH_3$ | $OCH_3$ |
| J-9 | Q-9 ($R_7$=H, $R_8$=H, $R_2$=Cl) | H | 5-$OCH_3$ | $OCH_3$ | $OCH_3$ |
| J-9 | Q-10 ($R_7$=H, $R_8$=H) | H | H | $OCH_3$ | $OCH_3$ |
| J-9 | Q-10 ($R_7$=H, $R_8$=$CH_3$) | H | 5-Cl | $OCH_3$ | $CH_3$ |
| J-9 | Q-11 ($R_7$=H, $R_8$=H) | H | H | $CH_3$ | $OCH_3$ |
| J-9 | Q-11 ($R_7$=n-$C_4H_9$, $R_8$=H) | H | H | $CH_3$ | $OCH_3$ |
| J-9 | Q-11 ($R_7$=$CH_3$, $R_8$=$CH_3$) | H | 5-$CH_3$ | $OCH_3$ | $OCH_3$ |
| J-9 | Q-12 ($R_7$=H, $R_8$=H) | H | H | $OCH_3$ | $OCH_3$ |

Table 2 (continued)

| J | Q | R | $R_{1c}$ | X | Y |
|---|---|---|---|---|---|
| J-9 | Q-12 ($R_7$=H, $R_8$=$C_2H_5$) | H | H | $CH_3$ | $OCH_3$ |
| J-9 | Q-13 ($R_7$=H, $R_8$=H, $R_3$=H) | H | H | $CH_3$ | $OCH_3$ |
| J-9 | Q-13 ($R_7$=H, $R_8$=H, $R_3$=$CH_3$) | $CH_3$ | H | $CH_3$ | $OCH_3$ |
| J-9 | Q-13 ($R_7$=$CH_3$, $R_8$=H, $R_3$=$CH_3$) | H | H | $OCH_3$ | $OCH_3$ |
| J-9 | Q-14 ($R_7$=H, $R_8$=H, $R_3$=$CH_3$) | H | H | $OCH_3$ | $OCH_3$ |
| J-9 | Q-14 ($R_7$=$CH_3$, $R_8$=H, $R_3$=$C_2H_5$) | H | H | $OCH_3$ | $OCH_3$ |
| J-9 | Q-14 ($R_7$=H, $R_8$=$CH_3$, $R_3$=$CH_3$) | H | 5-$OCH_3$ | $OCH_3$ | $OCH_3$ |
| J-9 | Q-15 ($R_7$=H, $R_8$=H, $R_3$=H) | H | H | $OCH_3$ | $OCH_3$ |
| J-9 | Q-15 ($R_7$=$CH_3$, $R_8$=$CH_3$, $R_3$=$CH_3$) | H | 5-Cl | $OCH_3$ | $OCH_3$ |
| J-9 | Q-17 ($R_7$=H, $R_8$=H, $R_2$=H) | H | H | $OCH_3$ | $OCH_3$ |
| J-9 | Q-17 ($R_7$=H, $R_8$=H, $R_2$=$C_2H_5$) | H | 5-Cl | $OCH_3$ | $CH_3$ |
| J-9 | Q-18 ($R_7$=H, $R_8$=H, $R_2$=H) | H | H | $CH_3$ | $OCH_3$ |
| J-9 | Q-18 ($R_7$=H, $R_8$=$OC_2H_5$, $R_2$=H) | H | H | $CH_3$ | $OCH_3$ |
| J-9 | Q-18 ($R_7$=$CH_3$, $R_8$=$OCH(CH_3)_2$, $R_2$=$CH_3$) | H | 5-$CH_3$ | $OCH_3$ | $OCH_3$ |
| J-9 | Q-19 ($R_7$=H, $R_8$=H) | H | H | $OCH_3$ | $OCH_3$ |
| J-9 | Q-19 ($R_7$=$CH_3$, $R_8$=$CH_3$) | H | H | $CH_3$ | $OCH_3$ |
| J-9 | Q-20 ($R_7$=H, $R_8$=H) | H | H | $CH_3$ | $OCH_3$ |
| J-9 | Q-20 ($R_7$=$CH_3$, $R_8$=H) | $CH_3$ | H | $CH_3$ | $OCH_3$ |
| J-9 | Q-20 ($R_7$=H, $R_8$=$CH_3$) | H | H | $OCH_3$ | $OCH_3$ |
| J-9 | Q-21 ($R_7$=H, $R_8$=H) | H | H | $OCH_3$ | $OCH_3$ |
| J-9 | Q-21 ($R_7$=$CH_3$, $R_8$=H) | H | H | $OCH_3$ | $OCH_3$ |
| J-9 | Q-21 ($R_7$=$CH_3$, $R_8$=$CH_3$) | H | 5-$OCH_3$ | $OCH_3$ | $OCH_3$ |
| J-9 | Q-22 ($R_7$=H, $R_8$=H, $R_3$=H) | H | H | $OCH_3$ | $OCH_3$ |
| J-9 | Q-22 ($R_7$=$CH_3$, $R_8$=$CH_3$, $R_3$=$C_2H_5$) | H | 5-Cl | $OCH_3$ | $OCH_3$ |
| J-9 | Q-23 ($R_7$=H, $R_8$=H, $R_3$=H) | H | H | $OCH_3$ | $OCH_3$ |
| J-9 | Q-23 ($R_7$=H, $R_8$=H, $R_3$=$CH_3$) | $CH_3$ | H | $OCH_3$ | $OCH_3$ |
| J-9 | Q-23 ($R_7$=$CH_3$, $R_8$=H, $R_3$=n-$C_4H_9$) | H | 5-$OCH_3$ | $OCH_3$ | $OCH_3$ |

Table 2 (continued)

| J | Q | R | $R_{1c}$ | X | Y |
|---|---|---|---|---|---|
| J-9 | Q-24 ($R_7$=H, $R_8$=H, $R_3$=$CH_3$) | H | H | $OCH_3$ | $OCH_3$ |
| J-9 | Q-24 ($R_7$=$CH_3$, $R_8$=$CH_3$, $R_3$=i-$C_3H_7$) | H | 5-Cl | $OCH_3$ | $CH_3$ |
| J-9 | Q-25 ($R_7$=H, $R_8$=H, $R_9$=H) | H | H | $CH_3$ | $OCH_3$ |
| J-9 | Q-25 ($R_7$=$CH_3$, $R_8$=H, $R_9$=H) | H | H | $CH_3$ | $OCH_3$ |
| J-9 | Q-25 ($R_7$=H, $R_8$=H, $R_9$=$C_2H_5$) | H | 5-$CH_3$ | $OCH_3$ | $OCH_3$ |
| J-9 | Q-26 ($R_7$=H, $R_8$=H) | H | H | $OCH_3$ | $OCH_3$ |
| J-9 | Q-26 ($R_7$=$CH_3$, $R_8$=$CH_3$) | H | H | $CH_3$ | $OCH_3$ |
| J-9 | Q-27 ($R_7$=H, $R_8$=H) | H | H | $CH_3$ | $OCH_3$ |
| J-9 | Q-27 ($R_7$=$CH_3$, $R_8$=H) | $CH_3$ | H | $CH_3$ | $OCH_3$ |
| J-9 | Q-27 ($R_7$=H, $R_8$=$CH_3$) | H | H | $OCH_3$ | $OCH_3$ |
| J-9 | Q-28 ($R_7$=H, $R_8$=H) | H | H | $OCH_3$ | $OCH_3$ |
| J-9 | Q-28 ($R_7$=$CH_3$, $R_8$=H) | H | H | $OCH_3$ | $OCH_3$ |
| J-9 | Q-28 ($R_7$=$CH_3$, $R_8$=$CH_3$) | H | 5-$OCH_3$ | $OCH_3$ | $OCH_3$ |
| J-9 | Q-29 ($R_7$=H, $R_8$=H) | H | H | $OCH_3$ | $OCH_3$ |
| J-9 | Q-29 ($R_7$=H, $R_8$=$CH_3$) | H | 5-Cl | $OCH_3$ | $OCH_3$ |
| J-9 | Q-30 ($R_7$=H, $R_8$=H) | H | H | $OCH_3$ | $OCH_3$ |
| J-9 | Q-30 ($R_7$=$CH_3$, $R_8$=$CH_3$) | $CH_3$ | H | $OCH_3$ | $CH_3$ |
| J-9 | Q-30 ($R_7$=$C_2H_5$, $R_8$=H) | H | 5-$OCH_3$ | $OCH_3$ | $OCH_3$ |
| J-9 | Q-31 ($R_7$=H, $R_8$=H) | H | H | $OCH_3$ | $OCH_3$ |
| J-9 | Q-31 ($R_7$=H, $R_8$=n-$C_4H_9$) | H | 5-Cl | $OCH_3$ | $CH_3$ |
| J-9 | Q-32 ($R_7$=H, $R_8$=H, $R_3$=H) | H | H | $CH_3$ | $OCH_3$ |
| J-9 | Q-32 ($R_7$=$CH_3$, $R_8$=H, $R_3$=$CH_3$) | H | H | $CH_3$ | $OCH_3$ |
| J-9 | Q-32 ($R_7$=H, $R_8$=$CH_3$, $R_3$=$C_2H_5$) | H | 5-$CH_3$ | $OCH_3$ | $OCH_3$ |
| J-9 | Q-33 ($R_7$=H, $R_8$=H, $R_8$=$CH_3$) | H | H | $OCH_3$ | $OCH_3$ |
| J-9 | Q-33 ($R_7$=$C_2H_5$, $R_8$=H, $R_3$=n-$C_4H_9$) | H | H | $CH_3$ | $OCH_3$ |
| J-9 | Q-34 ($R_7$=H, $R_8$=H, $R_3$=H) | H | H | $CH_3$ | $OCH_3$ |
| J-9 | Q-34 ($R_7$=i-$C_3H_7$, $R_8$=H, $R_3$=$CH_3$) | $CH_3$ | H | $CH_3$ | $OCH_3$ |
| J-9 | Q-34 ($R_7$=H, $R_8$=n-$C_4H_9$, $R_3$=$CH_3$) | H | H | $OCH_3$ | $OCH_3$ |

Table 2 (continued)

| J | Q | R | $R_{1c}$ | X | Y |
|---|---|---|---|---|---|
| J-9 | Q-36 ($R_7$=H, $R_8$=H, $R_2$=H) | H | H | $OCH_3$ | $OCH_3$ |
| J-9 | Q-36 ($R_7$=H, $R_8$=H, $R_2$=$C_2H_5$) | H | 5-Cl | $OCH_3$ | $OCH_3$ |
| J-9 | Q-37 ($R_7$=H, $R_8$=H, $R_2$=H) | H | H | $OCH_3$ | $OCH_3$ |
| J-9 | Q-37 ($R_7$=H, $R_8$=H, $R_2$=i-$C_3H_7$) | $CH_3$ | H | $OCH_3$ | $OCH_3$ |
| J-9 | Q-37 ($R_7$=H, $R_8$=H, $R_2$=n-$C_4H_9$) | H | 5-$OCH_3$ | $OCH_3$ | $OCH_3$ |
| J-9 | Q-38 ($R_7$=H, $R_8$=H, $R_2$=$OCF_2H$) | H | H | $OCH_3$ | $OCH_3$ |
| J-9 | Q-38 ($R_7$=H, $R_8$=H, $R_2$=Cl) | H | 5-Cl | $OCH_3$ | $CH_3$ |
| J-9 | Q-39 ($R_7$=H, $R_8$=H, $R_9$=H) | H | H | $CH_3$ | $OCH_3$ |
| J-9 | Q-39 ($R_7$=$C_2H_5$, $R_8$=H, $R_9$=H) | H | H | $CH_3$ | $OCH_3$ |
| J-9 | Q-39 ($R_7$=H, $R_8$=H, $R_9$=$CH_3$) | H | 5-$CH_3$ | $OCH_3$ | $OCH_3$ |
| J-9 | Q-40 ($R_7$=H, $R_8$=H) | H | H | $OCH_3$ | $OCH_3$ |
| J-9 | Q-40 ($R_7$=H, $R_8$=$CH_3$) | H | H | $CH_3$ | $OCH_3$ |
| J-9 | Q-41 ($R_7$=H, $R_8$=H) | H | H | $CH_3$ | $OCH_3$ |
| J-9 | Q-41 ($R_7$=$CH_3$, $R_8$=$CH_3$) | $CH_3$ | H | $CH_3$ | $OCH_3$ |
| J-9 | Q-41 ($R_7$=H, $R_8$=$C_2H_5$) | H | H | $OCH_3$ | $OCH_3$ |
| J-9 | Q-42 ($R_7$=H, $R_8$=H) | H | H | $OCH_3$ | $OCH_3$ |
| J-9 | Q-42 ($R_7$=$CH_3$, $R_8$=$CH_3$) | H | H | $OCH_3$ | $OCH_3$ |
| J-9 | Q-42 ($R_7$=$C_2H_5$, $R_8$=$C_2H_5$) | H | 5-$OCH_3$ | $OCH_3$ | $OCH_3$ |
| J-9 | Q-43 ($R_7$=H, $R_8$=H, $R_3$=H) | H | H | $OCH_3$ | $OCH_3$ |
| J-9 | Q-43 ($R_7$=$CH_3$, $R_8$=H, $R_3$=$CH_3$) | H | 5-Cl | $OCH_3$ | $OCH_3$ |
| J-9 | Q-44 ($R_7$=H, $R_8$=H, $R_3$=$CH_3$) | H | H | $OCH_3$ | $OCH_3$ |
| J-9 | Q-44 ($R_7$=$CH_3$, $R_8$=H, $R_3$=$C_2H_5$) | $CH_3$ | H | $OCH_3$ | $CH_3$ |
| J-9 | Q-44 ($R_7$=H, $R_8$=$CH_3$, $R_3$=$CH_3$) | H | 5-$OCH_3$ | $OCH_3$ | $OCH_3$ |
| J-9 | Q-45 ($R_7$=H, $R_8$=H, $R_3$=H) | H | H | $OCH_3$ | $OCH_3$ |
| J-9 | Q-45 ($R_7$=$CH_3$, $R_8$=$CH_3$, $R_3$=$CH_3$) | H | 5-Cl | $OCH_3$ | $CH_3$ |
| J-9 | Q-47 ($R_7$=H, $R_8$=H, $R_2$=H) | H | H | $OCH_3$ | $OCH_3$ |

Table 2 (continued)

| J | Q | R | $R_{1c}$ | X | Y |
|---|---|---|---|---|---|
| J-9 | Q-47 ($R_7$=H, $R_8$=H, $R_2$=$C_2H_5$) | H | H | $CH_3$ | $OCH_3$ |
| J-9 | Q-48 ($R_7$=H, $R_8$=H, $R_2$=H) | H | H | $CH_3$ | $OCH_3$ |
| J-9 | Q-48 ($R_7$=$CH_3$, $R_8$=$CH_3$, $R_2$=$CH_3$) | $CH_3$ | H | $CH_3$ | $OCH_3$ |
| J-9 | Q-48 ($R_7$=H, $R_8$=$C_2H_5$, $R_2$=$C_2H_5$) | H | H | $OCH_3$ | $OCH_3$ |
| J-9 | Q-49 ($R_7$=H, $R_8$=H, $R_2$=H) | H | H | $OCH_3$ | $OCH_3$ |
| J-9 | Q-49 ($R_7$=$CH_3$, $R_8$=$CH_3$, $R_2$=H) | H | H | $OCH_3$ | $OCH_3$ |
| J-9 | Q-49 ($R_7$=$CH_3$, $R_8$=$CH_3$, $R_2$=$CH_3$) | H | 5-$OCH_3$ | $OCH_3$ | $OCH_3$ |
| J-9 | Q-50 ($R_7$=H, $R_8$=H, $R_2$=H) | H | H | $OCH_3$ | $OCH_3$ |
| J-9 | Q-50 ($R_7$=$CH_3$, $R_8$=$CH_3$, $R_2$=$CH_3$) | H | 5-Cl | $OCH_3$ | $OCH_3$ |
| J-9 | Q-51 ($R_7$=H, $R_8$=H, $R_9$=H) | H | H | $OCH_3$ | $OCH_3$ |
| J-9 | Q-51 ($R_7$=H, $R_8$=H, $R_9$=$CH_3$) | $CH_3$ | H | $OCH_3$ | $OCH_3$ |
| J-9 | Q-51 ($R_7$=H, $R_8$=H, $R_9$=s-$C_4H_9$) | H | 5-$OCH_3$ | $OCH_3$ | $OCH_3$ |
| J-9 | Q-52 ($R_7$=H, $R_8$=H, $R_9$=H) | H | H | $OCH_3$ | $OCH_3$ |
| J-9 | Q-52 ($R_7$=H, $R_8$=H, $R_9$=$CH_3$) | H | 5-Cl | $OCH_3$ | $CH_3$ |
| J-9 | Q-53 ($R_7$=H, $R_8$=H, $R_9$=H) | H | H | $CH_3$ | $OCH_3$ |
| J-9 | Q-53 ($R_7$=$CH_3$, $R_8$=$CH_3$, $R_9$=$CH_3$) | H | H | $CH_3$ | $OCH_3$ |
| J-9 | Q-53 ($R_7$=H, $R_8$=H, $R_9$=$CH_3$) | H | 5-$CH_3$ | $OCH_3$ | $OCH_3$ |
| J-9 | Q-54 ($R_7$=H, $R_8$=H, $R_9$=H) | H | H | $OCH_3$ | $OCH_3$ |
| J-9 | Q-54 ($R_7$=H, $R_8$=H, $R_9$=i-$C_3H_7$) | H | H | $CH_3$ | $OCH_3$ |
| J-9 | Q-55 ($R_7$=H, $R_8$=H, $R_9$=H, $R_3$=H) | H | H | $CH_3$ | $OCH_3$ |
| J-9 | Q-55 ($R_7$=H, $R_8$=H, $R_9$=$CH_3$, $R_3$=$CH_3$) | $CH_3$ | H | $CH_3$ | $OCH_3$ |
| J-9 | Q-55 ($R_7$=H, $R_8$=H, $R_9$=s-$C_4H_9$, $R_3$=H) | H | H | $OCH_3$ | $OCH_3$ |
| J-9 | Q-56 ($R_7$=H, $R_8$=H, $R_9$=H, $R_3$=$C_2H_5$) | H | H | $OCH_3$ | $OCH_3$ |
| J-9 | Q-56 ($R_7$=$CH_3$, $R_8$=H, $R_9$=H, $R_3$=H) | H | H | $OCH_3$ | $OCH_3$ |
| J-9 | Q-56 ($R_7$=H, $R_8$=H, $R_9$=$CH_3$, $R_3$=$CH_3$) | H | 5-$OCH_3$ | $OCH_3$ | $OCH_3$ |
| J-9 | Q-57 ($R_7$=H, $R_8$=H, $R_9$=H, $R_3$=i-$C_3H_7$) | H | H | $OCH_3$ | $OCH_3$ |
| J-9 | Q-57 ($R_7$=H, $R_8$=H, $R_9$=$CH_3$, $R_3$=$CH_3$) | H | 5-Cl | $OCH_3$ | $OCH_3$ |
| J-9 | Q-58 ($R_7$=H, $R_8$=H, $R_9$=H, $R_3$=H) | H | H | $OCH_3$ | $OCH_3$ |
| J-9 | Q-58 ($R_7$=$CH_3$, $R_8$=H, $R_9$=H, $R_3$=$CH_3$) | $CH_3$ | H | $OCH_3$ | $CH_3$ |
| J-9 | Q-58 ($R_7$=H, $R_8$=H, $R_9$=i-$C_3H_7$, $R_3$=$CH_3$) | H | 5-$OCH_3$ | $OCH_3$ | $OCH_3$ |

Table 2 (continued)

| J | Q | R | $R_{1c}$ | X | Y |
|---|---|---|---|---|---|
| J-9 | Q-60 ($R_7$=H, $R_8$=H, $R_9$=H, $R_2$=H) | H | H | $CH_3$ | $OCH_3$ |
| J-9 | Q-60 ($R_7$=$CH_3$, $R_8$=H, $R_9$=H, $R_2$=$O(CH_2)_3CH_2Br$) | H | H | $CH_3$ | $OCH_3$ |
| J-9 | Q-60 ($R_7$=H, $R_8$=H, $R_9$=H, $R_2$=Br) | H | 5-$CH_3$ | $OCH_3$ | $OCH_3$ |
| J-9 | Q-61 ($R_7$=H, $R_8$=H, $R_9$=H, $R_2$=H) | H | H | $OCH_3$ | $OCH_3$ |
| J-9 | Q-61 ($R_7$=H, $R_8$=$CH_3$, $R_9$=H, $R_2$=$O(CH_2)_3CH_3$) | H | H | $CH_3$ | $OCH_3$ |
| J-9 | Q-62 ($R_7$=H, $R_8$=H, $R_9$=H, $R_2$=H) | H | H | $CH_3$ | $OCH_3$ |
| J-9 | Q-62 ($R_7$=$CH_3$, $R_8$=H, $R_9$=H, $R_2$=$CH_3$) | $CH_3$ | H | $CH_3$ | $OCH_3$ |
| J-9 | Q-62 ($R_7$=Cl, $R_8$=H, $R_9$=H, $R_2$=H) | H | H | $OCH_3$ | $OCH_3$ |
| J-9 | Q-63 ($R_7$=H, $R_8$=H, $R_9$=H) | H | H | $OCH_3$ | $OCH_3$ |
| J-9 | Q-63 ($R_7$=$CH_3$, $R_8$=H, $R_9$=H) | H | H | $OCH_3$ | $OCH_3$ |
| J-9 | Q-63 ($R_7$=H, $R_8$=H, $R_9$=$CH_3$) | H | 5-$OCH_3$ | $OCH_3$ | $OCH_3$ |
| J-9 | Q-64 ($R_7$=H, $R_8$=H, $R_9$=H) | H | H | $OCH_3$ | $OCH_3$ |
| J-9 | Q-64 ($R_7$=H, $R_8$=H, $R_9$=$C_2H_5$) | H | 5-Cl | $OCH_3$ | $OCH_3$ |
| J-9 | Q-65 ($R_7$=H, $R_8$=H, $R_9$=H) | H | H | $OCH_3$ | $OCH_3$ |
| J-9 | Q-65 ($R_7$=n-$C_4H_9$, $R_8$=H, $R_9$=H) | $CH_3$ | H | $OCH_3$ | $CH_3$ |
| J-9 | Q-65 ($R_7$=H, $R_8$=$CH_3$, $R_9$=i-$C_3H_7$) | H | 5-$OCH_3$ | $OCH_3$ | $OCH_3$ |
| J-9 | Q-66 ($R_7$=H, $R_8$=H, $R_9$=H) | H | H | $OCH_3$ | $OCH_3$ |
| J-9 | Q-66 ($R_7$=H, $R_8$=$C_2H_5$, $R_9$=$CH_3$) | H | 5-Cl | $OCH_3$ | $CH_3$ |
| J-9 | Q-67 ($R_7$=H, $R_8$=H, $R_9$=H, $R_3$=H) | H | H | $CH_3$ | $OCH_3$ |
| J-9 | Q-67 ($R_7$=$CH_3$, $R_8$=H, $R_9$=H, $R_3$=$CH_3$) | H | H | $CH_3$ | $OCH_3$ |
| J-9 | Q-67 ($R_7$=H, $R_8$=H, $R_9$=$CH_3$, $R_3$=$C_2H_5$) | H | 5-$CH_3$ | $OCH_3$ | $OCH_3$ |
| J-9 | Q-68 ($R_7$=H, $R_8$=H, $R_9$=H, $R_3$=$CH_3$) | H | H | $OCH_3$ | $OCH_3$ |
| J-9 | Q-68 ($R_7$=H, $R_8$=H, $R_9$=$C_2H_5$, $R_3$=n-$C_4H_9$) | H | H | $CH_3$ | $OCH_3$ |
| J-9 | Q-69 ($R_7$=H, $R_8$=H, $R_9$=H, $R_3$=$CH_3$) | H | H | $CH_3$ | $OCH_3$ |
| J-9 | Q-69 ($R_7$=n-$C_4H_9$, $R_8$=H, $R_9$=H, $R_3$=$C_2H_5$) | $CH_3$ | H | $CH_3$ | $OCH_3$ |
| J-9 | Q-69 ($R_7$=H, $R_8$=$CH_3$, $R_9$=i-$C_3H_7$, $R_3$=H) | H | H | $OCH_3$ | $OCH_3$ |
| J-9 | Q-70 ($R_7$=H, $R_8$=H, $R_9$=H, $R_3$=$CH_3$) | H | H | $OCH_3$ | $OCH_3$ |
| J-9 | Q-70 ($R_7$=i-$C_3H_7$, $R_8$=H, $R_9$=H, $R_3$=$CH_3$) | H | H | $OCH_3$ | $OCH_3$ |
| J-9 | Q-70 ($R_7$=H, $R_8$=$C_2H_5$, $R_9$=$CH_3$, $R_3$=H) | H | 5-$OCH_3$ | $OCH_3$ | $OCH_3$ |

Table 2 (continued)

| J | Q | R | $R_{1c}$ | X | Y |
|---|---|---|---|---|---|
| J-9 | Q-72 ($R_7$=H, $R_8$=H, $R_9$=H, $R_2$=H) | H | H | $OCH_3$ | $OCH_3$ |
| J-9 | Q-72 ($R_7$=H, $R_8$=H, $R_9$=H, $R_2$=$CH_3$) | $CH_3$ | H | $OCH_3$ | $CH_3$ |
| J-9 | Q-72 ($R_7$=$CH_3$, $R_8$=H, $R_9$=H, $R_2$=H) | H | 5-$OCH_3$ | $OCH_3$ | $OCH_3$ |
| J-9 | Q-73 ($R_7$=H, $R_8$=H, $R_9$=H, $R_2$=H) | H | H | $OCH_3$ | $OCH_3$ |
| J-9 | Q-73 ($R_7$=$CH_3$, $R_8$=H, $R_9$=$CH_3$, $R_2$=H) | H | 5-Cl | $OCH_3$ | $CH_3$ |
| J-9 | Q-74 ($R_7$=H, $R_8$=H, $R_9$=H, $R_2$=H) | H | H | $CH_3$ | $OCH_3$ |
| J-9 | Q-74 ($R_7$=$CH_3$, $R_8$=H, $R_9$=H, $R_2$=H) | H | H | $CH_3$ | $OCH_3$ |
| J-9 | Q-74 ($R_7$=H, $R_8$=H, $R_9$=H, $R_2$=$CH_3$) | H | 5-$CH_3$ | $OCH_3$ | $OCH_3$ |
| J-9 | Q-75 ($R_7$=H, $R_8$=H, $R_9$=H) | H | H | $OCH_3$ | $OCH_3$ |
| J-9 | Q-75 ($R_7$=$CH_3$, $R_8$=H, $R_9$=$CH_3$) | H | H | $CH_3$ | $OCH_3$ |
| J-9 | Q-76 ($R_7$=H, $R_8$=H, $R_9$=H) | H | H | $CH_3$ | $OCH_3$ |
| J-9 | Q-76 ($R_7$=H, $R_8$=H, $R_9$=$CH_3$) | $CH_3$ | H | $CH_3$ | $OCH_3$ |
| J-9 | Q-76 ($R_7$=$C_2H_5$, $R_8$=H, $R_9$=$C_2H_5$) | H | H | $OCH_3$ | $OCH_3$ |
| J-9 | Q-77 ($R_7$=H, $R_8$=H, $R_9$=H) | H | H | $OCH_3$ | $OCH_3$ |
| J-9 | Q-77 ($R_7$=H, $R_8$=H, $R_9$=n-$C_3H_7$) | H | H | $OCH_3$ | $OCH_3$ |
| J-9 | Q-77 ($R_7$=H, $R_8$=$CH_3$, $R_9$=H) | H | 5-$OCH_3$ | $OCH_3$ | $OCH_3$ |
| J-9 | Q-78 ($R_7$=H, $R_8$=H, $R_9$=H) | H | H | $OCH_3$ | $OCH_3$ |
| J-9 | Q-78 ($R_7$=n-$C_3H_7$, $R_8$=H, $R_9$=H) | H | 5-Cl | $OCH_3$ | $OCH_3$ |
| J-9 | Q-79 ($R_7$=H, $R_8$=H, $R_9$=H, $R_3$=H) | H | H | $OCH_3$ | $OCH_3$ |
| J-9 | Q-79 ($R_7$=H, $R_8$=H, $R_9$=H, $R_3$=$CH_3$) | $CH_3$ | H | $OCH_3$ | $OCH_3$ |
| J-9 | Q-79 ($R_7$=$CH_3$, $R_8$=H, $R_9$=H, $R_3$=$C_2H_5$) | H | 5-$OCH_3$ | $OCH_3$ | $OCH_3$ |
| J-9 | Q-80 ($R_7$=H, $R_8$=H, $R_9$=H, $R_3$=$CH_3$) | H | H | $OCH_3$ | $OCH_3$ |
| J-9 | Q-80 ($R_7$=H, $R_8$=H, $R_9$=$CH_3$, $R_3$=$C_2H_5$) | H | 5-Cl | $OCH_3$ | $CH_3$ |
| J-9 | Q-81 ($R_7$=H, $R_8$=H, $R_9$=H, $R_3$=$CH_3$) | H | H | $CH_3$ | $OCH_3$ |
| J-9 | Q-81 ($R_7$=H, $R_8$=$CH_3$, $R_9$=H, $R_3$=i-$C_3H_7$) | H | H | $CH_3$ | $OCH_3$ |
| J-9 | Q-81 ($R_7$=$CH_3$, $R_8$=H, $R_9$=$CH_3$, $R_3$=$CH_3$) | H | 5-$CH_3$ | $OCH_3$ | $OCH_3$ |
| J-9 | Q-82 ($R_7$=H, $R_8$=H, $R_9$=H, $R_3$=$CH_3$) | H | H | $OCH_3$ | $OCH_3$ |
| J-9 | Q-82 ($R_7$=H, $R_8$=H, $R_9$=$C_2H_5$, $R_3$=$CH_3$) | H | H | $CH_3$ | $OCH_3$ |

Table 2 (continued)

| J | Q | R | $R_{1c}$ | X | Y |
|---|---|---|---|---|---|
| J-9 | Q-83 ($R_7$=H, $R_8$=H, $R_9$=H, $R_{10}$=H) | H | H | $CH_3$ | $OCH_3$ |
| J-9 | Q-83 ($R_7$=$CH_3$, $R_8$=H, $R_9$=H, $R_{10}$=$CH_3$) | $CH_3$ | H | $CH_3$ | $OCH_3$ |
| J-9 | Q-83 ($R_7$=$CH_3$, $R_8$=$CH_3$, $R_9$=H, $R_{10}$=H) | H | H | $OCH_3$ | $OCH_3$ |
| J-9 | Q-84 ($R_7$=H, $R_8$=H, $R_9$=H) | H | H | $OCH_3$ | $OCH_3$ |
| J-9 | Q-84 ($R_7$=H, $R_8$=H, $R_9$=$CH_3$) | H | H | $OCH_3$ | $OCH_3$ |
| J-9 | Q-84 ($R_7$=H, $R_8$=$C_2H_5$, $R_9$=H) | H | 5-$OCH_3$ | $OCH_3$ | $OCH_3$ |
| J-9 | Q-85 ($R_7$=H, $R_8$=H, $R_9$=H) | H | H | $OCH_3$ | $OCH_3$ |
| J-9 | Q-85 ($R_7$=H, $R_8$=H, $R_9$=$C_2H_5$) | H | 5-Cl | $OCH_3$ | $OCH_3$ |
| J-9 | Q-86 ($R_7$=H, $R_8$=H, $R_9$=H) | H | H | $OCH_3$ | $OCH_3$ |
| J-9 | Q-86 ($R_7$=$CH_3$, $R_8$=H, $R_9$=H) | $CH_3$ | H | $OCH_3$ | $OCH_3$ |
| J-9 | Q-86 ($R_7$=H, $R_8$=H, $R_9$=$C_2H_5$) | H | 5-$OCH_3$ | $OCH_3$ | $OCH_3$ |
| J-9 | Q-87 ($R_7$=H, $R_8$=H, $R_9$=H) | H | H | $OCH_3$ | $OCH_3$ |
| J-9 | Q-87 ($R_7$=$CH_3$, $R_8$=H, $R_9$=H) | H | 5-Cl | $OCH_3$ | $CH_3$ |
| J-9 | Q-88 ($R_7$=H, $R_8$=H, $R_9$=H) | H | H | $CH_3$ | $OCH_3$ |
| J-9 | Q-88 ($R_7$=$CH_3$, $R_8$=H, $R_9$=H) | H | H | $CH_3$ | $OCH_3$ |
| J-9 | Q-88 ($R_7$=H, $R_8$=H, $R_9$=$CH_3$) | H | 5-$CH_3$ | $OCH_3$ | $OCH_3$ |
| J-9 | Q-89 ($R_7$=H, $R_8$=H, $R_9$=H) | H | H | $OCH_3$ | $OCH_3$ |
| J-9 | Q-89 ($R_7$=H, $R_8$=$CH_3$, $R_9$=H) | H | H | $CH_3$ | $OCH_3$ |
| J-9 | Q-90 ($R_7$=H, $R_8$=H, $R_9$=H, $R_3$=$CH_3$) | H | H | $CH_3$ | $OCH_3$ |
| J-9 | Q-90 ($R_7$=$CH_3$, $R_8$=H, $R_9$=H, $R_3$=$C_2H_5$) | $CH_3$ | H | $CH_3$ | $OCH_3$ |
| J-9 | Q-90 ($R_7$=H, $R_8$=H, $R_9$=$CH_3$, $R_3$=$CH_3$) | H | H | $OCH_3$ | $OCH_3$ |
| J-9 | Q-91 ($R_7$=H, $R_8$=H, $R_9$=H, $R_3$=$CH_3$) | H | H | $OCH_3$ | $OCH_3$ |
| J-9 | Q-91 ($R_7$=$CH_3$, $R_8$=H, $R_9$=H, $R_3$=H) | H | H | $OCH_3$ | $OCH_3$ |
| J-9 | Q-91 ($R_7$=H, $R_8$=H, $R_9$=$CH_3$, $R_3$=$CH_3$) | H | 5-$OCH_3$ | $OCH_3$ | $OCH_3$ |
| J-9 | Q-92 ($R_7$=H, $R_8$=H, $R_9$=H, $R_{10}$=H) | H | H | $OCH_3$ | $OCH_3$ |
| J-9 | Q-92 ($R_7$=H, $R_8$=$CH_3$, $R_9$=H, $R_{10}$=H) | H | 5-Cl | $OCH_3$ | $OCH_3$ |
| J-9 | Q-93 ($R_7$=H, $R_8$=H, $R_9$=H) | H | H | $OCH_3$ | $OCH_3$ |
| J-9 | Q-93 ($R_7$=H, $R_8$=H, $R_9$=$CH_3$) | $CH_3$ | H | $OCH_3$ | $OCH_3$ |
| J-9 | Q-93 ($R_7$=$CH_3$, $R_8$=H, $R_9$=H) | H | 5-$OCH_3$ | $OCH_3$ | $OCH_3$ |
| J-9 | Q-94 ($R_7$=H, $R_8$=H, $R_9$=H) | H | H | $OCH_3$ | $OCH_3$ |
| J-9 | Q-94 ($R_7$=H, $R_8$=H, $R_9$=n-$C_4H_9$) | H | 5-Cl | $OCH_3$ | $CH_3$ |

Table 2 (continued)

| J | Q | R | $R_{1c}$ | X | Y |
|---|---|---|---|---|---|
| J-9 | Q-95 ($R_7$=H, $R_8$=H, $R_9$=H) | H | H | $CH_3$ | $OCH_3$ |
| J-9 | Q-95 ($R_7$=H, $R_8$=H, $R_9$=$CH_3$) | H | H | $CH_3$ | $OCH_3$ |
| J-9 | Q-95 ($R_7$=H, $R_8$=H, $R_9$=s-$C_4H_9$) | H | 5-$CH_3$ | $OCH_3$ | $OCH_3$ |
| J-9 | Q-96 ($R_7$=H, $R_8$=H, $R_9$=H) | H | H | $OCH_3$ | $OCH_3$ |
| J-9 | Q-96 ($R_7$=H, $R_8$=H, $R_9$=$CH_3$) | H | H | $CH_3$ | $OCH_3$ |
| J-9 | Q-97 ($R_7$=H, $R_8$=H, $R_9$=H) | H | H | $CH_3$ | $OCH_3$ |
| J-9 | Q-97 ($R_7$=$CH_3$, $R_8$=$CH_3$, $R_9$=$CH_3$) | $CH_3$ | H | $CH_3$ | $OCH_3$ |
| J-9 | Q-97 ($R_7$=H, $R_8$=H, $R_9$=$CH_3$) | H | H | $OCH_3$ | $OCH_3$ |
| J-9 | Q-98 ($R_7$=H, $R_8$=H, $R_9$=H) | H | H | $OCH_3$ | $OCH_3$ |
| J-9 | Q-98 ($R_7$=$CH_3$, $R_8$=H, $R_9$=H) | H | H | $OCH_3$ | $OCH_3$ |
| J-9 | Q-98 ($R_7$=H, $R_8$=H, $R_9$=i-$C_3H_7$) | H | 5-$OCH_3$ | $OCH_3$ | $OCH_3$ |
| J-9 | Q-99 ($R_7$=H, $R_8$=H, $R_9$=H, $R_3$=H) | H | H | $OCH_3$ | $OCH_3$ |
| J-9 | Q-99 ($R_7$=H, $R_8$=H, $R_9$=s-$C_4H_9$, $R_3$=H) | H | 5-Cl | $OCH_3$ | $OCH_3$ |
| J-9 | Q-100 ($R_7$=H, $R_8$=H, $R_9$=H, $R_3$=$C_2H_5$) | H | H | $OCH_3$ | $OCH_3$ |
| J-9 | Q-100 ($R_7$=$CH_3$, $R_8$=H, $R_9$=H, $R_3$=H) | $CH_3$ | H | $OCH_3$ | $CH_3$ |
| J-9 | Q-100 ($R_7$=H, $R_8$=H, $R_9$=$CH_3$, $R_3$=$CH_3$) | H | 5-$OCH_3$ | $OCH_3$ | $OCH_3$ |
| J-9 | Q-101 ($R_7$=H, $R_8$=H, $R_9$=H, $R_3$=i-$C_3H_7$) | H | H | $OCH_3$ | $OCH_3$ |
| J-9 | Q-101 ($R_7$=H, $R_8$=H, $R_9$=$CH_3$, $R_3$=$CH_3$) | H | 5-Cl | $OCH_3$ | $CH_3$ |
| J-9 | Q-102 ($R_7$=H, $R_8$=H, $R_9$=H, $R_3$=H) | H | H | $CH_3$ | $OCH_3$ |
| J-9 | Q-102 ($R_7$=$CH_3$, $R_8$=H, $R_9$=H, $R_3$=$CH_3$) | H | H | $CH_3$ | $OCH_3$ |
| J-9 | Q-102 ($R_7$=H, $R_8$=H, $R_9$=i-$C_3H_7$, $R_3$=$CH_3$) | H | 5-$CH_3$ | $OCH_3$ | $OCH_3$ |
| J-9 | Q-104 ($R_7$=H, $R_8$=H, $R_9$=H, $R_2$=H) | H | H | $CH_3$ | $OCH_3$ |
| J-9 | Q-104 ($R_7$=$CH_3$, $R_8$=H, $R_9$=H, $R_2$=$OC_2H_5$) | $CH_3$ | H | $CH_3$ | $OCH_3$ |
| J-9 | Q-104 ($R_7$=H, $R_8$=H, $R_9$=H, $R_2$=Br) | H | H | $OCH_3$ | $OCH_3$ |
| J-9 | Q-105 ($R_7$=H, $R_8$=H, $R_9$=H, $R_2$=H) | H | H | $OCH_3$ | $OCH_3$ |
| J-9 | Q-105 ($R_7$=$CH_3$, $R_8$=H, $R_9$=H, $R_2$=$CH_3$) | H | H | $OCH_3$ | $OCH_3$ |
| J-9 | Q-105 ($R_7$=H, $R_8$=$CH_3$, $R_9$=H, $R_2$=$C_2H_5$) | H | 5-$OCH_3$ | $OCH_3$ | $OCH_3$ |
| J-9 | Q-106 ($R_7$=H, $R_8$=H, $R_9$=H, $R_2$=H) | H | H | $OCH_3$ | $OCH_3$ |

Table 2 (continued)

| J | Q | R | $R_{1c}$ | X | Y |
|---|---|---|---|---|---|
| J-9 | Q-106 ($R_7$=Cl, $R_8$=H, $R_9$=H, $R_2$=H) | H | 5-Cl | $OCH_3$ | $OCH_3$ |
| J-9 | Q-107 ($R_7$=H, $R_8$=H, $R_9$=H, $R_2$=H) | H | H | $OCH_3$ | $OCH_3$ |
| J-9 | Q-107 ($R_7$=$CH_3$, $R_8$=H, $R_9$=H, $R_2$=H) | $CH_3$ | H | $OCH_3$ | $OCH_3$ |
| J-9 | Q-107 ($R_7$=H, $R_8$=H, $R_9$=H, $R_2$=$CH_3$) | H | 5-$OCH_3$ | $OCH_3$ | $OCH_3$ |
| J-9 | Q-108 ($R_7$=H, $R_8$=H, $R_9$=H, $R_2$=H) | H | H | $OCH_3$ | $OCH_3$ |
| J-9 | Q-108 ($R_7$=H, $R_8$=H, $R_9$=H, $R_2$=$CH_3$) | H | 5-Cl | $OCH_3$ | $CH_3$ |
| J-9 | Q-109 ($R_7$=H, $R_8$=H, $R_9$=H) | H | H | $CH_3$ | $OCH_3$ |
| J-9 | Q-109 ($R_7$=$CH_3$, $R_8$=H, $R_9$=H) | H | H | $CH_3$ | $OCH_3$ |
| J-9 | Q-109 ($R_7$=H, $R_8$=H, $R_9$=$CH_3$) | H | 5-$CH_3$ | $OCH_3$ | $OCH_3$ |
| J-9 | Q-110 ($R_7$=H, $R_8$=H, $R_9$=H) | H | H | $OCH_3$ | $OCH_3$ |
| J-9 | Q-110 ($R_7$=H, $R_8$=$CH_3$, $R_9$=H) | H | H | $CH_3$ | $OCH_3$ |
| J-9 | Q-111 ($R_7$=H, $R_8$=H, $R_9$=H) | H | H | $CH_3$ | $OCH_3$ |
| J-9 | Q-111 ($R_7$=$CH_3$, $R_8$=H, $R_9$=H) | $CH_3$ | H | $CH_3$ | $OCH_3$ |
| J-9 | Q-111 ($R_7$=H, $R_8$=H, $R_9$=$CH_3$) | H | H | $OCH_3$ | $OCH_3$ |
| J-9 | Q-112 ($R_7$=H, $R_8$=H, $R_9$=H) | H | H | $OCH_3$ | $OCH_3$ |
| J-9 | Q-112 ($R_7$=$CH_3$, $R_8$=H, $R_9$=H) | H | H | $OCH_3$ | $OCH_3$ |
| J-9 | Q-112 ($R_7$=H, $R_8$=$CH_3$, $R_9$=$CH_3$) | H | 5-$OCH_3$ | $OCH_3$ | $OCH_3$ |
| J-9 | Q-113 ($R_7$=H, $R_8$=H, $R_9$=H, $R_3$=$CH_3$) | H | H | $OCH_3$ | $OCH_3$ |
| J-9 | Q-113 ($R_7$=H, $R_8$=H, $R_9$=$C_2H_5$, $R_3$=n-$C_4H_9$) | H | 5-Cl | $OCH_3$ | $OCH_3$ |
| J-9 | Q-114 ($R_7$=H, $R_8$=H, $R_9$=H, $R_3$=$CH_3$) | H | H | $OCH_3$ | $OCH_3$ |
| J-9 | Q-114 ($R_7$=n-$C_4H_9$, $R_8$=H, $R_9$=H, $R_3$=$C_2H_5$) | $CH_3$ | H | $OCH_3$ | $CH_3$ |
| J-9 | Q-114 ($R_7$=H, $R_8$=$CH_3$, $R_8$=i-$C_3H_7$, $R_3$=H) | H | 5-$OCH_3$ | $OCH_3$ | $OCH_3$ |
| J-9 | Q-115 ($R_7$=H, $R_8$=H, $R_9$=H, $R_3$=$CH_3$) | H | H | $OCH_3$ | $OCH_3$ |
| J-9 | Q-115 ($R_7$=H, $R_8$=$C_2H_5$, $R_9$=$CH_3$, $R_3$=H) | H | 5-Cl | $OCH_3$ | $CH_3$ |
| J-9 | Q-116 ($R_7$=H, $R_8$=H, $R_9$=H, $R_{10}$=H) | H | H | $CH_3$ | $OCH_3$ |
| J-9 | Q-116 ($R_7$=$CH_3$, $R_8$=H, $R_9$=H, $R_{10}$=H) | H | H | $CH_3$ | $OCH_3$ |
| J-9 | Q-116 ($R_7$=H, $R_8$=H, $R_9$=H, $R_{10}$=$CH_3$) | H | 5-$CH_3$ | $OCH_3$ | $OCH_3$ |
| J-9 | Q-118 ($R_7$=H, $R_8$=H, $R_9$=H, $R_{10}$=H) | H | H | $CH_3$ | $OCH_3$ |

Table 2 (continued)

| J | Q | R | $R_{1c}$ | X | Y |
|---|---|---|---|---|---|
| J-9 | Q-118 ($R_7$=$CH_3$, $R_8$=H, $R_9$=H, $R_{10}$=H) | $CH_3$ | H | $CH_3$ | $OCH_3$ |
| J-9 | Q-118 ($R_7$=H, $R_8$=H, $R_9$=H, $R_{10}$=$CH_3$) | H | H | OCH | $OCH_3$ |
| J-9 | Q-119 ($R_7$=H, $R_8$=H, $R_9$=H, $R_2$=H) | H | H | $OCH_3$ | $OCH_3$ |
| J-9 | Q-119 ($R_7$=H, $R_8$=H, $R_9$=H, $R_2$=$CH_3$) | H | H | $OCH_3$ | $OCH_3$ |
| J-9 | Q-119 ($R_7$=H, $R_8$=$CH_3$, $R_9$=H, $R_2$=$C_2H_5$) | H | 5-$OCH_3$ | $OCH_3$ | $OCH_3$ |
| J-9 | Q-120 ($R_7$=H, $R_8$=H, $R_9$=H, $R_2$=H) | H | H | $OCH_3$ | $OCH_3$ |
| J-9 | Q-120 ($R_7$=H, $R_8$=H, $R_9$=$CH_3$, $R_2$=$CH_3$) | H | 5-Cl | $OCH_3$ | $OCH_3$ |
| J-9 | Q-121 ($R_7$=H, $R_8$=H, $R_9$=H, $R_2$=H) | H | H | $OCH_3$ | $OCH_3$ |
| J-9 | Q-121 ($R_7$=H, $R_8$=H, $R_9$=H, $R_2$=$C_2H_5$) | $CH_3$ | H | $OCH_3$ | $CH_3$ |
| J-9 | Q-121 ($R_7$=$CH_3$, $R_8$=$CH_3$, $R_9$=H, $R_2$=H) | H | 5-$OCH_3$ | $OCH_3$ | $OCH_3$ |
| J-9 | Q-122 ($R_7$=H, $R_8$=H, $R_9$=H, $R_2$=H) | H | H | $OCH_3$ | $OCH_3$ |
| J-9 | Q-122 ($R_7$=$CH_3$, $R_8$=H, $R_9$=H, $R_2$=H) | H | 5-Cl | $OCH_3$ | $CH_3$ |
| J-9 | Q-123 ($R_7$=H, $R_8$=H, $R_9$=H) | H | H | $CH_3$ | $OCH_3$ |
| J-9 | Q-123 ($R_7$=H, $R_8$=H, $R_9$=$CH_3$) | H | H | $CH_3$ | $OCH_3$ |
| J-9 | Q-123 ($R_7$=$CH_3$, $R_8$=H, $R_9$=$CH_3$) | H | 5-$CH_3$ | $OCH_3$ | $OCH_3$ |
| J-9 | Q-124 ($R_7$=H, $R_8$=H, $R_9$=H) | H | H | $OCH_3$ | $OCH_3$ |
| J-9 | Q-124 ($R_7$=$C_2H_5$, $R_8$=H, $R_9$=$C_2H_5$) | H | H | $CH_3$ | $OCH_3$ |
| J-9 | Q-125 ($R_7$=H, $R_8$=H, $R_9$=H) | H | H | $CH_3$ | $OCH_3$ |
| J-9 | Q-125 ($R_7$=H, $R_8$=H, $R_9$=<u>n</u>-$C_4H_9$) | $CH_3$ | H | $CH_3$ | $OCH_3$ |
| J-9 | Q-125 ($R_7$=H, $R_8$=$CH_3$, $R_9$=H) | H | H | $OCH_3$ | $OCH_3$ |
| J-9 | Q-126 ($R_7$=H, $R_8$=H, $R_9$=H) | H | H | $OCH_3$ | $OCH_3$ |
| J-9 | Q-126 ($R_7$=H, $R_8$=H, $R_9$=<u>s</u>-$C_3H_7$) | H | H | $OCH_3$ | $OCH_3$ |
| J-9 | Q-126 ($R_7$=<u>n</u>-$C_3H_7$, $R_8$=H, $R_9$=H) | H | 5-$OCH_3$ | $OCH_3$ | $OCH_3$ |
| J-9 | Q-127 ($R_7$=H, $R_8$=H, $R_9$=H, $R_3$=H) | H | H | $OCH_3$ | $OCH_3$ |
| J-9 | Q-127 ($R_7$=$CH_3$, $R_8$=H, $R_9$=H, $R_3$=$C_2H_5$) | H | 5-Cl | $OCH_3$ | $OCH_3$ |
| J-9 | Q-128 ($R_7$=H, $R_8$=H, $R_9$=H, $R_3$=$CH_3$) | H | H | $OCH_3$ | $OCH_3$ |
| J-9 | Q-128 ($R_7$=$CH_3$, $R_8$=H, $R_9$=H, $R_3$=$CH_3$) | $CH_3$ | H | $OCH_3$ | $OCH_3$ |
| J-9 | Q-128 ($R_7$=H, $R_8$=H, $R_9$=$CH_3$, $R_3$=$C_2H_5$) | H | 5-$OCH_3$ | $OCH_3$ | $OCH_3$ |
| J-9 | Q-129 ($R_7$=H, $R_8$=H, $R_9$=H, $R_3$=$CH_3$) | H | H | $OCH_3$ | $OCH_3$ |
| J-9 | Q-129 ($R_7$=$CH_3$, $R_8$=H, $R_9$=$CH_3$, $R_3$=$CH_3$) | H | 5-Cl | $OCH_3$ | $CH_3$ |
| J-9 | Q-130 ($R_7$=H, $R_8$=H, $R_9$=H, $R_3$=$CH_3$) | H | H | $CH_3$ | $OCH_3$ |

Table 2 (continued)

| J | Q | R | $R_{1c}$ | X | Y |
|---|---|---|---|---|---|
| J-9 | Q-130 ($R_7$=$CH_3$, $R_8$=H, $R_9$=$CH_3$, $R_3$=H) | H | H | $CH_3$ | $OCH_3$ |
| J-9 | Q-130 ($R_7$=H, $R_8$=H, $R_9$=$C_2H_5$, $R_3$=$CH_3$) | H | 5-$CH_3$ | $OCH_3$ | $OCH_3$ |
| J-9 | Q-131 ($R_7$=H, $R_8$=H, $R_9$=H, $R_{10}$=H) | H | H | $OCH_3$ | $OCH_3$ |
| J-9 | Q-131 ($R_7$=$CH_3$, $R_8$=$CH_3$, $R_9$=H, $R_{10}$=H) | H | H | $CH_3$ | $OCH_3$ |
| J-9 | Q-132 ($R_7$=H, $R_8$=H, $R_9$=H) | H | H | $CH_3$ | $OCH_3$ |
| J-9 | Q-132 ($R_7$=H, $R_8$=H, $R_9$=$CH_3$) | $CH_3$ | H | $CH_3$ | $OCH_3$ |
| J-9 | Q-132 ($R_7$=H, $R_8$=$C_2H_5$, $R_9$=H) | H | H | $OCH_3$ | $OCH_3$ |
| J-9 | Q-133 ($R_7$=H, $R_8$=H, $R_9$=H) | H | H | $OCH_3$ | $OCH_3$ |
| J-9 | Q-133 ($R_7$=$CH_3$, $R_8$=H, $R_9$=H) | H | H | $OCH_3$ | $OCH_3$ |
| J-9 | Q-133 ($R_7$=H, $R_8$=H, $R_9$=$C_2H_5$) | H | 5-$OCH_3$ | $OCH_3$ | $OCH_3$ |
| J-9 | Q-134 ($R_7$=H, $R_8$=H, $R_9$=H) | H | H | $OCH_3$ | $OCH_3$ |
| J-9 | Q-134 ($R_7$=H, $R_8$=H, $R_9$=$C_2H_5$) | H | 5-Cl | $OCH_3$ | $OCH_3$ |
| J-9 | Q-135 ($R_7$=H, $R_8$=H, R =H) | H | H | $OCH_3$ | $OCH_3$ |
| J-9 | Q-135 ($R_7$=H, $R_8$=H, R =$CH_3$) | $CH_3$ | H | $OCH_3$ | $OCH_3$ |
| J-9 | Q-135 ($R_7$=$CH_3$, $R_8$=H, $R_9$=H) | H | 5-$OCH_3$ | $OCH_3$ | $OCH_3$ |
| J-9 | Q-136 ($R_7$=H, $R_8$=H, $R_9$=H) | H | H | $OCH_3$ | $OCH_3$ |
| J-9 | Q-136 ($R_7$=H, $R_8$=$CH_3$, $R_9$=$C_2H_5$) | H | 5-Cl | $OCH_3$ | $CH_3$ |
| J-9 | Q-137 ($R_7$=H, $R_8$=H, $R_9$=H) | H | H | $CH_3$ | $OCH_3$ |
| J-9 | Q-137 ($R_7$=$CH_3$, $R_8$=H, $R_9$=H) | H | H | $CH_3$ | $OCH_3$ |
| J-9 | Q-137 ($R_7$=H, $R_8$=H, $R_9$=$CH_3$) | H | 5-$CH_3$ | $OCH_3$ | $OCH_3$ |
| J-9 | Q-138 ($R_7$=H, $R_8$=H, $R_9$=H) | H | H | $OCH_3$ | $OCH_3$ |
| J-9 | Q-138 ($R_7$=H, $R_8$=H, $R_9$=$CH_3$) | H | H | $CH_3$ | $OCH_3$ |
| J-9 | Q-139 ($R_7$=H, $R_8$=H, $R_9$=H) | H | H | $CH_3$ | $OCH_3$ |
| J-9 | Q-139 ($R_7$=$CH_3$, $R_8$=H, $R_9$=H) | $CH_3$ | H | $CH_3$ | $OCH_3$ |
| J-9 | Q-139 ($R_7$=$CH_3$, $R_8$=$CH_3$, $R_9$=H) | H | H | $OCH_3$ | $OCH_3$ |
| J-9 | Q-140 ($R_7$=H, $R_8$=H, $R_9$=H, $R_3$=$CH_3$) | H | H | $OCH_3$ | $OCH_3$ |
| J-9 | Q-140 ($R_7$=H, $R_8$=H, $R_9$=$CH_3$, $R_3$=H) | H | H | $OCH_3$ | $OCH_3$ |
| J-9 | Q-140 ($R_7$=H, $R_8$=$CH_3$, $R_9$=H, $R_3$=$C_2H_5$) | H | 5-$OCH_3$ | $OCH_3$ | $OCH_3$ |
| J-9 | Q-141 ($R_7$=H, $R_8$=H, $R_9$=H, $R_3$=$CH_3$) | H | H | $OCH_3$ | $OCH_3$ |
| J-9 | Q-141 ($R_7$=H, $R_8$=H, $R_9$=$CH_3$, $R_3$=$CH_3$) | H | 5-Cl | $OCH_3$ | $OCH_3$ |

Table 2 (continued)

| J | Q | R | $R_{1c}$ | X | Y |
|---|---|---|---|---|---|
| J-9 | Q-142 ($R_7$=H, $R_8$=H, $R_9$=H, $R_{10}$=H) | H | H | $OCH_3$ | $OCH_3$ |
| J-9 | Q-142 ($R_7$=$CH_3$, $R_8$=H, $R_9$=H, $R_{10}$=H) | $CH_3$ | H | $OCH_3$ | $CH_3$ |
| J-9 | Q-142 ($R_7$=H, $R_8$=H, $R_9$=H, $R_{10}$=$CH_3$) | H | 5-$OCH_3$ | $OCH_3$ | $OCH_3$ |
| J-9 | Q-143 ($R_7$=H, $R_8$=H, $R_9$=H, $R_3$=$CH_3$) | H | H | $OCH_3$ | $OCH_3$ |
| J-9 | Q-143 ($R_7$=$CH_3$, $R_8$=H, $R_9$=$CH_3$, $R_3$=$CH_3$) | H | 5-Cl | $OCH_3$ | $CH_3$ |
| J-9 | Q-144 ($R_7$=H, $R_8$=H, $R_9$=H, $R_3$=$CH_3$) | H | H | $CH_3$ | $OCH_3$ |
| J-9 | Q-144 ($R_7$=$CH_3$, $R_8$=H, $R_9$=H, $R_3$=$CH_3$) | H | H | $CH_3$ | $OCH_3$ |
| J-9 | Q-144 ($R_7$=H, $R_8$=$C_2H_5$, $R_9$=H, $R_3$=$CH_3$) | H | 5-$CH_3$ | $OCH_3$ | $OCH_3$ |
| J-9 | Q-145 ($R_7$=H, $R_8$=H, $R_9$=H) | H | H | $OCH_3$ | $OCH_3$ |
| J-9 | Q-145 ($R_7$=$CH_3$, $R_8$=H, $R_9$=H) | H | H | $CH_3$ | $OCH_3$ |
| J-9 | Q-146 ($R_7$=H, $R_8$=H, $R_9$=H) | H | H | $CH_3$ | $OCH_3$ |
| J-9 | Q-146 ($R_7$=$CH_3$, $R_8$=H, $R_9$=H) | $CH_3$ | H | $CH_3$ | $OCH_3$ |
| J-9 | Q-146 ($R_7$=H, $R_8$=H, $R_9$=$\underline{n}$-$C_4H_9$) | H | H | $OCH_3$ | $OCH_3$ |
| J-9 | Q-147 ($R_7$=H, $R_8$=H, $R_9$=H) | H | H | $OCH_3$ | $OCH_3$ |
| J-9 | Q-147 ($R_7$=$CH_3$, $R_8$=H, $R_9$=H) | H | H | $OCH_3$ | $OCH_3$ |
| J-9 | Q-147 ($R_7$=H, $R_8$=H, $R_9$=$\underline{n}$-$C_4H_9$) | H | 5-$OCH_3$ | $OCH_3$ | $OCH_3$ |
| J-9 | Q-148 ($R_7$=H, $R_8$=H, $R_9$=H) | H | H | $OCH_3$ | $OCH_3$ |
| J-9 | Q-148 ($R_7$=$CH_3$, $R_8$=H, $R_9$=H) | H | 5-Cl | $OCH_3$ | $OCH_3$ |
| J-9 | Q-149 ($R_7$=H, $R_8$=H, $R_9$=H) | H | H | $OCH_3$ | $OCH_3$ |
| J-9 | Q-149 ($R_7$=H, $R_8$=H, $R_9$=$CH_3$) | $CH_3$ | H | $OCH_3$ | $OCH_3$ |
| J-9 | Q-149 ($R_7$=$CH_3$, $R_8$=H, $R_9$=H) | H | 5-$OCH_3$ | $OCH_3$ | $OCH_3$ |
| J-9 | Q-150 ($R_7$=H, $R_8$=H, $R_9$=H) | H | H | $OCH_3$ | $OCH_3$ |
| J-9 | Q-150 ($R_7$=H, $R_8$=$CH_3$, $R_9$=$CH_3$) | H | 5-Cl | $OCH_3$ | $CH_3$ |
| J-9 | Q-151 ($R_7$=H, $R_8$=H, $R_9$=H) | H | H | $CH_3$ | $OCH_3$ |
| J-9 | Q-151 ($R_7$=$CH_3$, $R_8$=H, $R_9$=H) | H | H | $CH_3$ | $OCH_3$ |
| J-9 | Q-151 ($R_7$=$CH_3$, $R_8$=$CH_3$, $R_9$=H) | H | 5-$CH_3$ | $OCH_3$ | $OCH_3$ |
| J-9 | Q-152 ($R_7$=H, $R_8$=H, $R_9$=H) | H | H | $OCH_3$ | $OCH_3$ |
| J-9 | Q-152 ($R_7$=$CH_3$, $R_8$=$CH_3$, $R_9$=H) | H | H | $CH_3$ | $OCH_3$ |
| J-9 | Q-153 ($R_7$=H, $R_8$=H, $R_9$=H, $R_{11}$=$CH_3$) | H | H | $CH_3$ | $OCH_3$ |
| J-9 | Q-153 ($R_7$=H, $R_8$=H, $R_9$=H, $R_{11}$=H) | $CH_3$ | H | $CH_3$ | $OCH_3$ |

Table 2 (continued)

| J | Q | R | $R_{1c}$ | X | Y | m.p.(°C) |
|---|---|---|---|---|---|---|
| J-9 | Q-153 ($R_7$=$CH_3$, $R_8$=H, $R_9$=H, $R_{11}$=$CH_3$) | H | H | $OCH_3$ | $OCH_3$ | |
| J-9 | Q-154 ($R_7$=H, $R_8$=H, $R_9$=H, $R_{11}$=$CH_3$) | H | H | $OCH_3$ | $OCH_3$ | |
| J-9 | Q-154 ($R_7$=$CH_3$, $R_8$=H, $R_9$=H) | H | H | $OCH_3$ | $OCH_3$ | |
| J-9 | Q-154 ($R_7$=H, $R_8$=$CH_3$, $R_9$=$CH_3$) | H | 5-$OCH_3$ | $OCH_3$ | $OCH_3$ | |
| J-9 | Q-155 ($R_7$=H, $R_8$=H, $R_9$=H) | H | H | $OCH_3$ | $OCH_3$ | |
| J-9 | Q-155 ($R_7$=$CH_3$, $R_8$=$CH_3$, $R_9$=H) | H | 5-Cl | $OCH_3$ | $OCH_3$ | |
| J-9 | Q-156 ($R_7$=H, $R_8$=H, $R_9$=H) | H | H | $OCH_3$ | $OCH_3$ | |
| J-9 | Q-156 ($R_7$=$C_2H_5$, $R_8$=H, $R_9$=H) | $CH_3$ | H | $OCH_3$ | $OCH_3$ | |
| J-9 | Q-156 ($R_7$=$CH_3$, $R_8$=$CH_3$, $R_9$=H) | H | 5-$OCH_3$ | $OCH_3$ | $OCH_3$ | |
| J-9 | Q-158 ($R_7$=H, $R_8$=H, $R_9$=H) | H | H | $CH_3$ | $OCH_3$ | |
| J-9 | Q-158 ($R_7$=H, $R_8$=H, $R_9$=$CH_3$) | H | H | $CH_3$ | $OCH_3$ | |
| J-9 | Q-158 ($R_7$=$CH_3$, $R_8$=H, $R_9$=H) | H | 5-$CH_3$ | $OCH_3$ | $OCH_3$ | |
| J-9 | Q-159 ($R_7$=H, $R_8$=H, $R_9$=H) | H | H | $OCH_3$ | $OCH_3$ | |
| J-9 | Q-159 ($R_7$=H, $R_8$=H, $R_9$=$CH_3$) | H | H | $CH_3$ | $OCH_3$ | |
| J-9 | Q-160 ($R_7$=H, $R_8$=H, $R_9$=H, $R_{11}$=$CH_3$) | H | H | $CH_3$ | $OCH_3$ | |
| J-9 | Q-160 ($R_7$=H, $R_8$=H, $R_9$=$CH_3$, $R_{11}$=H) | $CH_3$ | H | $CH_3$ | $OCH_3$ | |
| J-9 | Q-160 ($R_7$=$CH_3$,$R_8$=H, $R_9$=H, $R_{11}$=i-$C_3H_7$) | H | H | $OCH_3$ | $OCH_3$ | |
| J-9 | Q-161 ($R_7$=H, $R_8$=H, $R_9$=H, $R_{11}$=$CH_3$) | H | H | $OCH_3$ | $OCH_3$ | |
| J-9 | Q-161 ($R_7$=$CH_3$, $R_8$=H, $R_9$=H, $R_{11}$=H) | H | H | $OCH_3$ | $OCH_3$ | |
| J-9 | Q-161 ($R_7$=H, $R_8$=H, $R_9$=$CH_3$, $R_{11}$=$CH_3$) | H | 5-$OCH_3$ | $OCH_3$ | $OCH_3$ | |
| J-9 | Q-162 ($R_7$=H, $R_8$=H, $R_9$=H, $R_{10}$=H) | H | H | $OCH_3$ | $OCH_3$ | |
| J-9 | Q-162 ($R_7$=H, $R_8$=H, $R_9$=H, $R_{10}$=$CH_3$) | H | 5-Cl | $OCH_3$ | $OCH_3$ | |
| J-9 | Q-163 ($R_2$=H, $R_7$=H, $R_8$=H, $R_9$=H) | H | H | $OCH_3$ | $OCH_3$ | |
| J-9 | Q-164 ($R_7$=H) | H | H | $OCH_3$ | $OCH_3$ | |
| J-9 | Q-165 ($R_3$=H, $R_7$=H) | H | H | $OCH_3$ | $OCH_3$ | |
| J-9 | Q-166 ($R_3$=H, $R_7$=H) | H | H | $OCH_3$ | $OCH_3$ | |
| J-9 | Q-167 ($R_7$=H, $R_8$=H, $R_9$=H, $R_{10}$=H) | H | H | $OCH_3$ | $OCH_3$ | 210-211 |
| J-9 | Q-167 ($R_7$=H, $R_8$=H, $R_9$=H, $R_{10}$=H) | H | H | $OCH_3$ | $CH_3$ | 95-100 |

Table 2 (continued)

| J | Q | R | $R_{1c}$ | X | Y |
|---|---|---|---|---|---|
| J-10 | Q-1 ($R_7$=H, $R_8$=H) | H | H | $CH_3$ | $CH_3$ |
| J-10 | Q-1 ($R_7$=H, $R_8$=H) | H | H | $OCH_3$ | $CH_3$ |
| J-10 | Q-1 ($R_7$=H, $R_8$=H) | H | H | $OCH_3$ | $OCH_3$ |
| J-10 | Q-1 ($R_7$=H, $R_8$=H) | H | H | $OCH_3$ | $CH_2CH_3$ |
| J-10 | Q-1 ($R_7$=H, $R_8$=H) | H | H | $OCH_3$ | $OCH_2(CH_3)_2$ |
| J-10 | Q-1 ($R_7$=H, $R_8$=H) | H | H | $OCH_2CF_3$ | $OCH_3$ |
| J-10 | Q-1 ($R_7$=H, $R_8$=H) | H | H | $CF_3$ | $OCH_3$ |
| J-10 | Q-1 ($R_7$=H, $R_8$=H) | H | H | $SCH_3$ | $OCH_3$ |
| J-10 | Q-1 ($R_7$=H, $R_8$=H) | H | H | $OCH_3$ | $NHCH_3$ |
| J-10 | Q-1 ($R_7$=H, $R_8$=H) | H | H | H | $OCH_3$ |
| J-10 | Q-3 ($R_7$=H, $R_8$=H) | H | H | $OCH_3$ | $OCH_3$ |
| J-10 | Q-4 ($R_7$=H, $R_8$=H, $R_3$=$CH_3$) | H | H | $CH_3$ | $OCH_3$ |
| J-10 | Q-5 ($R_7$-H, $R_8$=H, $R_3$=H) | H | H | $OCH_3$ | $OCH_3$ |
| J-10 | Q-6 ($R_7$=$CH_3$, $R_8$=H, $R_3$=$CH_3$) | H | H | $CH_3$ | $OCH_3$ |
| J-10 | Q-8 ($R_7$=H, $R_8$=H, $R_2$=H) | H | H | $OCH_3$ | $OCH_3$ |
| J-10 | Q-9 ($R_7$=H, $R_8$=H, $R_2$=H) | H | H | $OCH_3$ | $OCH_3$ |
| J-10 | Q-10 ($R_7$=H, $R_8$=H) | H | H | $OCH_3$ | $OCH_3$ |
| J-10 | Q-11 ($R_7$=H, $R_8$=H) | H | H | $CH_3$ | $OCH_3$ |
| J-10 | Q-12 ($R_7$=H, $R_8$=H) | H | H | $OCH_3$ | $OCH_3$ |
| J-10 | Q-13 ($R_7$=H, $R_8$=H, $R_3$=H) | H | H | $CH_3$ | $OCH_3$ |
| J-10 | Q-14 ($R_7$=H, $R_8$=H, $R_3$=$CH_3$) | H | H | $OCH_3$ | $OCH_3$ |
| J-10 | Q-15 ($R_7$=CH , $R_8$=H, $R_3$=H) | H | H | $OCH_3$ | $OCH_3$ |
| J-10 | Q-17 ($R_7$=H, $R_8$=H, $R_2$=H) | H | H | $OCH_3$ | $OCH_3$ |
| J-10 | Q-18 ($R_7$=H, $R_8$=H, $R_2$=H) | H | H | $CH_3$ | $OCH_3$ |
| J-10 | Q-19 ($R_7$=H, $R_8$=H) | H | H | $OCH_3$ | $OCH_3$ |
| J-10 | Q-20 ($R_7$=H, $R_8$=H) | H | H | $CH_3$ | $OCH_3$ |
| J-10 | Q-21 ($R_7$=H, $R_8$=H) | H | H | $OCH_3$ | $OCH_3$ |
| J-10 | Q-22 ($R_7$=H, $R_8$=H, $R_3$=H) | H | H | $OCH_3$ | $OCH_3$ |
| J-10 | Q-23 ($R_7$=H, $R_8$=H, $R_3$=H) | H | H | $OCH_3$ | $OCH_3$ |
| J-10 | Q-24 ($R_7$=H, $R_8$=H, $R_3$=$CH_3$) | H | H | $OCH_3$ | $OCH_3$ |

Table 2 (continued)

| J | Q | R | $R_{1c}$ | X | Y |
|---|---|---|---|---|---|
| J-10 | Q-25 ($R_7$=H, $R_8$=H, $R_9$=H) | H | H | $CH_3$ | $OCH_3$ |
| J-10 | Q-26 ($R_7$=H, $R_8$=H) | H | H | $OCH_3$ | $OCH_3$ |
| J-10 | Q-27 ($R_7$=H, $R_8$=H) | H | H | $CH_3$ | $OCH_3$ |
| J-10 | Q-28 ($R_7$=H, $R_8$=H) | H | H | $OCH_3$ | $OCH_3$ |
| J-10 | Q-29 ($R_7$=H, $R_8$=H) | H | H | $OCH_3$ | $OCH_3$ |
| J-10 | Q-30 ($R_7$=H, $R_8$=H) | H | H | $OCH_3$ | $OCH_3$ |
| J-10 | Q-31 ($R_7$=H, $R_8$=H) | H | H | $OCH_3$ | $OCH_3$ |
| J-10 | Q-32 ($R_7$=H, $R_8$=H, $R_3$=H) | H | H | $CH_3$ | $OCH_3$ |
| J-10 | Q-33 ($R_7$=H, $R_8$=H, $R_8$=$CH_3$) | H | H | $OCH_3$ | $OCH_3$ |
| J-10 | Q-34 ($R_7$=H, $R_8$=H, $R_3$=H) | H | H | $CH_3$ | $OCH_3$ |
| J-10 | Q-36 ($R_7$=H, $R_8$=H, $R_2$=H) | H | H | $OCH_3$ | $OCH_3$ |
| J-10 | Q-37 ($R_7$=H, $R_8$=H, $R_2$=H) | H | H | $OCH_3$ | $OCH_3$ |
| J-10 | Q-38 ($R_7$=H, $R_8$=H, $R_2$=$OCF_2H$) | H | H | $OCH_3$ | $OCH_3$ |
| J-10 | Q-39 ($R_7$=H, $R_8$=H, $R_9$=H) | H | H | $CH_3$ | $OCH_3$ |
| J-10 | Q-40 ($R_7$=H, $R_8$=H) | H | H | $OCH_3$ | $OCH_3$ |
| J-10 | Q-41 ($R_7$=H, $R_8$=H) | H | H | $CH_3$ | $OCH_3$ |
| J-10 | Q-42 ($R_7$=H, $R_8$=H) | H | H | $OCH_3$ | $OCH_3$ |
| J-10 | Q-43 ($R_7$=H, $R_8$=H, $R_3$=H) | H | H | $OCH_3$ | $OCH_3$ |
| J-10 | Q-44 ($R_7$=H, $R_8$=H, $R_3$=$CH_3$) | H | H | $OCH_3$ | $OCH_3$ |
| J-10 | Q-45 ($R_7$=H, $R_8$=H, $R_3$=H) | H | H | $OCH_3$ | $OCH_3$ |
| J-10 | Q-47 ($R_7$=H, $R_8$=H, $R_2$=H) | H | H | $OCH_3$ | $OCH_3$ |
| J-10 | Q-48 ($R_7$=H, $R_8$=H, $R_2$=H) | H | H | $CH_3$ | $OCH_3$ |
| J-10 | Q-49 ($R_7$=H, $R_8$=H, $R_2$=H) | H | H | $OCH_3$ | $OCH_3$ |
| J-10 | Q-50 ($R_7$=H, $R_8$=H, $R_2$=H) | H | H | $OCH_3$ | $OCH_3$ |
| J-10 | Q-51 ($R_7$=H, $R_8$=H, $R_9$=H) | H | H | $OCH_3$ | $OCH_3$ |
| J-10 | Q-52 ($R_7$=H, $R_8$=H, $R_9$=H) | H | H | $OCH_3$ | $OCH_3$ |
| J-10 | Q-53 ($R_7$=H, $R_8$=H, $R_9$=H) | H | H | $CH_3$ | $OCH_3$ |
| J-10 | Q-54 ($R_7$=H, $R_8$=H, $R_9$=H) | H | H | $OCH_3$ | $OCH_3$ |
| J-10 | Q-55 ($R_7$=H, $R_8$=H, $R_9$=H, $R_3$=H) | H | H | $CH_3$ | $OCH_3$ |
| J-10 | Q-56 ($R_7$=H, $R_8$=H, $R_9$=H, $R_3$=$C_2H_5$) | H | H | $CH_3$ | $OCH_3$ |
| J-10 | Q-57 ($R_7$=H, $R_8$=H, $R_9$=H, $R_3$=i-$C_3H_7$) | H | H | $OCH_3$ | $OCH_3$ |

Table 2 (continued)

| J | Q | R | $R_{1c}$ | X | Y |
|---|---|---|---|---|---|
| J-10 | Q-58 ($R_7$=H, $R_8$=H, $R_9$=H, $R_3$=H) | H | H | $OCH_3$ | $OCH_3$ |
| J-10 | Q-60 ($R_7$=H, $R_8$=H, $R_9$=H, $R_2$=H) | H | H | $CH_3$ | $OCH_3$ |
| J-10 | Q-61 ($R_7$=H, $R_8$=H, $R_9$=H, $R_2$=H) | H | H | $OCH_3$ | $OCH_3$ |
| J-10 | Q-62 ($R_7$=H, $R_8$=H, $R_9$=H, $R_2$=H) | H | H | $CH_3$ | $OCH_3$ |
| J-10 | Q-63 ($R_7$=H, $R_8$=H, $R_9$=H) | H | H | $OCH_3$ | $OCH_3$ |
| J-10 | Q-64 ($R_7$=H, $R_8$=H, $R_9$=H) | H | H | $OCH_3$ | $OCH_3$ |
| J-10 | Q-65 ($R_7$=H, $R_8$=H, $R_9$=H) | H | H | $OCH_3$ | $OCH_3$ |
| J-10 | Q-66 ($R_7$=H, $R_8$=H, $R_9$=H) | H | H | $OCH_3$ | $OCH_3$ |
| J-10 | Q-67 ($R_7$=H, $R_8$=H, $R_9$=H, $R_3$=H) | H | H | $CH_3$ | $OCH_3$ |
| J-10 | Q-68 ($R_7$=H, $R_8$=H, $R_9$=H, $R_3$=$CH_3$) | H | H | $OCH_3$ | $OCH_3$ |
| J-10 | Q-69 ($R_7$=H, $R_8$=H, $R_9$=H, $R_3$=$CH_3$) | H | H | $CH_3$ | $OCH_3$ |
| J-10 | Q-70 ($R_7$=H, $R_8$=H, $R_9$=H, $R_3$=$CH_3$) | H | H | $OCH_3$ | $OCH_3$ |
| J-10 | Q-72 ($R_7$=H, $R_8$=H, $R_9$=H, $R_2$=H) | H | H | $OCH_3$ | $OCH_3$ |
| J-10 | Q-73 ($R_7$=H, $R_8$=H, $R_9$=H, $R_2$=H) | H | H | $OCH_3$ | $OCH_3$ |
| J-10 | Q-74 ($R_7$=H, $R_8$=H, $R_9$=H, $R_2$=H) | H | H | $CH_3$ | $OCH_3$ |
| J-10 | Q-75 ($R_7$=H, $R_8$=H, $R_9$=H) | H | H | $OCH_3$ | $OCH_3$ |
| J-10 | Q-76 ($R_7$=H, $R_8$=H, $R_9$=H) | H | H | $CH_3$ | $OCH_3$ |
| J-10 | Q-77 ($R_7$=H, $R_8$=H, $R_9$=H) | H | H | $OCH_3$ | $OCH_3$ |
| J-10 | Q-78 ($R_7$=H, $R_8$=H, $R_9$=H) | H | H | $OCH_3$ | $OCH_3$ |
| J-10 | Q-79 ($R_7$=H, $R_8$=H, $R_9$=H, $R_3$=H) | H | H | $OCH_3$ | $OCH_3$ |
| J-10 | Q-80 ($R_7$=H, $R_8$=H, $R_9$=H, $R_3$=$CH_3$) | H | H | $OCH_3$ | $OCH_3$ |
| J-10 | Q-81 ($R_7$=H, $R_8$=H, $R_9$=H, $R_3$=$CH_3$) | H | H | $CH_3$ | $OCH_3$ |
| J-10 | Q-82 ($R_7$=H, $R_8$=H, $R_9$=H, $R_3$=$CH_3$) | H | H | $OCH_3$ | $OCH_3$ |
| J-10 | Q-83 ($R_7$=H, $R_8$=H, $R_9$=H, $R_{10}$=H) | H | H | $CH_3$ | $OCH_3$ |
| J-10 | Q-84 ($R_7$=H, $R_8$=H, $R_9$=H) | H | H | $OCH_3$ | $OCH_3$ |
| J-10 | Q-85 ($R_7$=H, $R_8$=H, $R_9$=H) | H | H | $OCH_3$ | $OCH_3$ |
| J-10 | Q-86 ($R_7$=H, $R_8$=H, $R_9$=H) | H | H | $OCH_3$ | $OCH_3$ |
| J-10 | Q-87 ($R_7$=H, $R_8$=H, $R_9$=H) | H | H | $OCH_3$ | $OCH_3$ |
| J-10 | Q-88 ($R_7$=H, $R_8$=H, $R_9$=H) | H | H | $CH_3$ | $OCH_3$ |
| J-10 | Q-89 ($R_7$=H, $R_8$=H, $R_9$=H) | H | H | $OCH_3$ | $OCH_3$ |
| J-10 | Q-90 ($R_7$=H, $R_8$=H, $R_9$=H, $R_3$=$CH_3$) | H | H | $CH_3$ | $OCH_3$ |
| J-10 | Q-91 ($R_7$=H, $R_8$=H, $R_9$=H, $R_3$=$CH_3$) | H | H | $OCH_3$ | $OCH_3$ |

Table 2 (continued)

| J | Q | R | $R_{1c}$ | X | Y |
|---|---|---|---|---|---|
| J-10 | Q-92 ($R_7$=H, $R_8$=H, $R_9$=H, $R_{10}$=H) | H | H | $OCH_3$ | $OCH_3$ |
| J-10 | Q-93 ($R_7$=H, $R_8$=H, $R_9$=H) | H | H | $OCH_3$ | $OCH_3$ |
| J-10 | Q-94 ($R_7$=H, $R_8$=H, $R_9$=H) | H | H | $OCH_3$ | $OCH_3$ |
| J-10 | Q-95 ($R_7$=H, $R_8$=H, $R_9$=H) | H | H | $CH_3$ | $OCH_3$ |
| J-10 | Q-96 ($R_7$=H, $R_8$=H, $R_9$=H) | H | H | $OCH_3$ | $OCH_3$ |
| J-10 | Q-97 ($R_7$=H, $R_8$=H, $R_9$=H) | H | H | $CH_3$ | $OCH_3$ |
| J-10 | Q-98 ($R_7$=H, $R_8$=H, $R_9$=H) | H | H | $OCH_3$ | $OCH_3$ |
| J-10 | Q-99 ($R_7$=H, $R_8$=H, $R_9$=H, $R_3$=H) | H | H | $OCH_3$ | $OCH_3$ |
| J-10 | Q-100 ($R_7$=H, $R_8$=H, $R_9$=H, $R_3$=$C_2H_5$) | H | H | $OCH_3$ | $OCH_3$ |
| J-10 | Q-101 ($R_7$=H, $R_8$=H, $R_9$=H, $R_3$=i-$C_2H_5$) | H | H | $OCH_3$ | $OCH_3$ |
| J-10 | Q-102 ($R_7$=H, $R_8$=H, $R_9$=H, $R_3$=H) | H | H | $CH_3$ | $OCH_3$ |
| J-10 | Q-104 ($R_7$=H, $R_8$=H, $R_9$=H, $R_2$=H) | H | H | $CH_3$ | $OCH_3$ |
| J-10 | Q-105 ($R_7$=H, $R_8$=H, $R_9$=H, $R_2$=H) | H | H | $OCH_3$ | $OCH_3$ |
| J-10 | Q-106 ($R_7$=H, $R_8$=H, $R_9$=H, $R_2$=H) | H | H | $OCH_3$ | $OCH_3$ |
| J-10 | Q-107 ($R_7$=H, $R_8$=H, $R_9$=H, $R_2$=H) | H | H | $OCH_3$ | $OCH_3$ |
| J-10 | Q-108 ($R_7$=H, $R_8$=H, $R_9$=H, $R_2$=H) | H | H | $OCH_3$ | $OCH_3$ |
| J-10 | Q-109 ($R_7$=H, $R_8$=H, $R_9$=H,) | H | H | $CH_3$ | $OCH_3$ |
| J-10 | Q-110 ($R_7$=H, $R_8$=H, $R_9$=H,) | H | H | $OCH_3$ | $OCH_3$ |
| J-10 | Q-111 ($R_7$=H, $R_8$=H, $R_9$=H,) | H | H | $CH_3$ | $OCH_3$ |
| J-10 | Q-112 ($R_7$=H, $R_8$=H, $R_9$=H,) | H | H | $OCH_3$ | $OCH_3$ |
| J-10 | Q-113 ($R_7$=H, $R_8$=H, $R_9$=H, $R_3$=$CH_3$) | H | H | $OCH_3$ | $OCH_3$ |
| J-10 | Q-114 ($R_7$=H, $R_8$=H, $R_9$=H, $R_3$=$CH_3$) | H | H | $OCH_3$ | $OCH_3$ |
| J-10 | Q-115 ($R_7$=H, $R_8$=H, $R_9$=H, $R_3$=$CH_3$) | H | H | $OCH_3$ | $OCH_3$ |
| J-10 | Q-116 ($R_7$=H, $R_8$=H, $R_9$=H, $R_{10}$=H) | H | H | $CH_3$ | $OCH_3$ |
| J-10 | Q-118 ($R_7$=H, $R_8$=H, $R_9$=H, $R_{10}$=H) | H | H | $CH_3$ | $OCH_3$ |
| J-10 | Q-119 ($R_7$=H, $R_8$=H, $R_9$=H, $R_2$=H) | H | H | $OCH_3$ | $OCH_3$ |
| J-10 | Q-120 ($R_7$=H, $R_8$=H, $R_9$=H, $R_2$=H) | H | H | $OCH_3$ | $OCH_3$ |
| J-10 | Q-121 ($R_7$=H, $R_8$=H, $R_9$=H, $R_2$=H) | H | H | $OCH_3$ | $OCH_3$ |
| J-10 | Q-122 ($R_7$=H, $R_8$=H, $R_9$=H, $R_2$=H) | H | H | $OCH_3$ | $OCH_3$ |
| J-10 | Q-123 ($R_7$=H, $R_8$=H, $R_9$=H,) | H | H | $CH_3$ | $OCH_3$ |
| J-10 | Q-124 ($R_7$=H, $R_8$=H, $R_9$=H,) | H | H | $OCH_3$ | $OCH_3$ |

Table 2 (continued)

| J | Q | R | $R_{1c}$ | X | Y |
|---|---|---|---|---|---|
| J-10 | Q-125 ($R_7$=H, $R_8$=H, $R_9$=H,) | H | H | $CH_3$ | $OCH_3$ |
| J-10 | Q-126 ($R_7$=H, $R_8$=H, $R_9$=H,) | H | H | $OCH_3$ | $OCH_3$ |
| J-10 | Q-127 ($R_7$=H, $R_8$=H, $R_9$=H, $R_3$=H) | H | H | $OCH_3$ | $OCH_3$ |
| J-10 | Q-128 ($R_7$=H, $R_8$=H, $R_9$=H, $R_3$=$CH_3$) | H | H | $OCH_3$ | $OCH_3$ |
| J-10 | Q-129 ($R_7$=H, $R_8$=H, $R_9$=H, $R_3$=$CH_3$) | H | H | $OCH_3$ | $OCH_3$ |
| J-10 | Q-130 ($R_7$=H, $R_8$=H, $R_9$=H, $R_3$=$CH_3$) | H | H | $CH_3$ | $OCH_3$ |
| J-10 | Q-131 ($R_7$=H, $R_8$=H, $R_9$=H, $R_{10}$=H) | H | H | $OCH_3$ | $OCH_3$ |
| J-10 | Q-132 ($R_7$=H, $R_8$=H, $R_9$=H,) | H | H | $CH_3$ | $OCH_3$ |
| J-10 | Q-133 ($R_7$=H, $R_8$=H, $R_9$=H,) | H | H | $OCH_3$ | $OCH_3$ |
| J-10 | Q-134 ($R_7$=H, $R_8$=H, $R_9$=H,) | H | H | $OCH_3$ | $OCH_3$ |
| J-10 | Q-135 ($R_7$=H, $R_8$=H, $R_9$=H,) | H | H | $OCH_3$ | $OCH_3$ |
| J-10 | Q-136 ($R_7$=H, $R_8$=H, $R_9$=H,) | H | H | $OCH_3$ | $OCH_3$ |
| J-10 | Q-137 ($R_7$=H, $R_8$=H, $R_9$=H,) | H | H | $CH_3$ | $OCH_3$ |
| J-10 | Q-138 ($R_7$=H, $R_8$=H, $R_9$=H,) | H | H | $OCH_3$ | $OCH_3$ |
| J-10 | Q-139 ($R_7$=H, $R_8$=H, $R_9$=H,) | H | H | $CH_3$ | $OCH_3$ |
| J-10 | Q-140 ($R_7$=H, $R_8$=H, $R_9$=H, $R_3$=$CH_3$) | H | H | $OCH_3$ | $OCH_3$ |
| J-10 | Q-141 ($R_7$=H, $R_8$=H, $R_9$=H, $R_3$=$CH_3$) | H | H | $OCH_3$ | $OCH_3$ |
| J-10 | Q-142 ($R_7$=H, $R_8$=H, $R_9$=H, $R_{10}$=H) | H | H | $OCH_3$ | $OCH_3$ |
| J-10 | Q-143 ($R_7$=H, $R_8$=H, $R_9$=H, $R_3$=$CH_3$) | H | H | $OCH_3$ | $OCH_3$ |
| J-10 | Q-144 ($R_7$=H, $R_8$=H, $R_9$=H, $R_3$=$CH_3$) | H | H | $CH_3$ | $OCH_3$ |
| J-10 | Q-145 ($R_7$=H, $R_8$=H, $R_9$=H,) | H | H | $OCH_3$ | $OCH_3$ |
| J-10 | Q-146 ($R_7$=H, $R_8$=H, $R_9$=H,) | H | H | $CH_3$ | $OCH_3$ |
| J-10 | Q-147 ($R_7$=H, $R_8$=H, $R_9$=H,) | H | H | $OCH_3$ | $OCH_3$ |
| J-10 | Q-148 ($R_7$=H, $R_8$=H, $R_9$=H,) | H | H | $OCH_3$ | $OCH_3$ |
| J-10 | Q-149 ($R_7$=H, $R_8$=H, $R_9$=H,) | H | H | $OCH_3$ | $OCH_3$ |
| J-10 | Q-150 ($R_7$=H, $R_8$=H, $R_9$=H,) | H | H | $OCH_3$ | $OCH_3$ |
| J-10 | Q-151 ($R_7$=H, $R_8$=H, $R_9$=H,) | H | H | $CH_3$ | $OCH_3$ |
| J-10 | Q-152 ($R_7$=H, $R_8$=H, $R_9$=H,) | H | H | $OCH_3$ | $OCH_3$ |
| J-10 | Q-153 ($R_7$=H, $R_8$=H, $R_9$=H, $R_{11}$=$CH_3$) | H | H | $CH_3$ | $OCH_3$ |
| J-10 | Q-154 ($R_7$=H, $R_8$=H, $R_9$=H, $R_{11}$=$CH_3$) | H | H | $OCH_3$ | $OCH_3$ |
| J-10 | Q-155 ($R_7$=H, $R_8$=H, $R_9$=H,) | H | H | $OCH_3$ | $OCH_3$ |
| J-10 | Q-156 ($R_7$=H, $R_8$=H, $R_9$=H,) | H | H | $OCH_3$ | $OCH_3$ |

Table 2 (continued)

| J | Q | R | $R_{1c}$ | X | Y |
|---|---|---|---|---|---|
| J-10 | Q-158 ($R_7$=H, $R_8$=H, $R_9$=H,) | H | H | $CH_3$ | $OCH_3$ |
| J-10 | Q-159 ($R_7$=H, $R_8$=H, $R_9$=H,) | H | H | $OCH_3$ | $OCH_3$ |
| J-10 | Q-160 ($R_7$=H, $R_8$=H, $R_9$=H, $R_{11}$=$CH_3$) | H | H | $CH_3$ | $OCH_3$ |
| J-10 | Q-161 ($R_7$=H, $R_8$=H, $R_9$=H, $R_{11}$=$CH_3$) | H | H | $OCH_3$ | $OCH_3$ |
| J-10 | Q-162 ($R_7$=H, $R_8$=H, $R_9$=H, $R_{10}$=H) | H | H | $OCH_3$ | $OCH_3$ |
| J-8* | Q-1 ($R_7$=H, $R_8$=H) | H | H | $OCH_3$ | $OCH_3$ |
| J-8* | Q-1 ($R_7$=H, $R_8$=H) | H | H | $OCH_3$ | $CH_3$ |
| J-9* | Q-1 ($R_7$=H, $R_8$=H) | H | H | $OCH_3$ | $OCH_3$ |
| J-10* | Q-1 ($R_7$=H, $R_8$=H) | H | H | $OCH_3$ | $OCH_3$ |
| J-9* | Q-167 ($R_7$=H, $R_8$=H, $R_9$=H, $R_{10}$=H) | H | H | $OCH_3$ | $OCH_3$ |
| J-9* | Q-167 ($R_7$=H, $R_8$=H, $R_9$=H, $R_{10}$=H) | H | H | OCH | CH |

Table 2 (continued)

| J | Q | R | $R_{1d}$ | X | Y | m.p.(°C) |
|---|---|---|---|---|---|---|
| J-11 | Q-1 ($R_7$=H, $R_8$=H) | H | H | $CH_3$ | $CH_3$ | |
| J-11 | Q-1 ($R_7$=H, $R_8$=H) | H | H | $OCH_3$ | $CH_3$ | 186.5-188.5 |
| J-11 | Q-1 ($R_7$=H, $R_8$=H) | H | H | $OCH_3$ | $OCH_3$ | 178.5-180.5 |
| J-11 | Q-1 ($R_7$=H, $R_8$=H) | $CH_3$ | H | $OCH_3$ | $OCH_3$ | |
| J-11 | Q-1 ($R_7$=H, $R_8$=H) | $CH_3$ | H | $OCH_3$ | $CH_3$ | |
| J-11 | Q-1 ($R_7$=H, $R_8$=H) | H | 5-$CH_3$ | $OCH_3$ | $OCH_3$ | |
| J-11 | Q-1 ($R_7$=H, $R_8$=H) | H | 6-$CH_2CH_3$ | $OCH_3$ | $OCH_3$ | |
| J-11 | Q-1 ($R_7$=H, $R_8$=H) | H | 3-$CH(CH_3)_2$ | $OCH_3$ | $OCH_3$ | |
| J-11 | Q-1 ($R_7$=H, $R_8$=H) | H | 5-$OCH_3$ | $OCH_3$ | $OCH_3$ | |
| J-11 | Q-1 ($R_7$=H, $R_8$=H) | H | 5-$OCH_2CH_3$ | $OCH_3$ | $OCH_3$ | |
| J-11 | Q-1 ($R_7$=H, $R_8$=H) | H | 3-$OCH(CH_3)_2$ | $OCH_3$ | $OCH_3$ | |
| J-11 | Q-1*($R_7$=H, $R_8$=H) | H | 5-$OCF_2H$ | $OCH_3$ | $OCH_3$ | |
| J-11 | Q-1 ($R_7$=H, $R_8$=H) | H | 6-$OCH_2CH_2Br$ | $OCH_3$ | $OCH_3$ | |
| J-11 | Q-1 ($R_7$=H, $R_8$=H) | H | 5-$OCH(CH_3)(CH_2Cl)$ | $OCH_3$ | $OCH_3$ | |
| J-11 | Q-1 ($R_7$=H, $R_8$=H) | H | 5-$CH_2F$ | $OCH_3$ | $OCH_3$ | |
| J-11 | Q-1 ($R_7$=H, $R_8$=H) | H | 5-$CH_2CH_2Br$ | $OCH_3$ | $OCH_3$ | |
| J-11 | Q-1 ($R_7$=H, $R_8$=H) | H | 5-$CH(CH_3)(CH_2Cl)$ | $OCH_3$ | $OCH_3$ | |
| J-11 | Q-1 ($R_7$=H, $R_8$=H) | H | 5-$SCH_3$ | $OCH_3$ | $OCH_3$ | |
| J-11 | Q-1 ($R_7$=H, $R_8$=H) | H | 6-$SCH_2CH_3$ | $OCH_3$ | $OCH_3$ | |
| J-11 | Q-1 ($R_7$=H, $R_8$=H) | H | 5-$SCH(CH_3)_2$ | $OCH_3$ | $OCH_3$ | |
| J-11 | Q-1 ($R_7$=H, $R_8$=H) | H | 5-$SCH_2F$ | $OCH_3$ | $OCH_3$ | |
| J-11 | Q-1 ($R_7$=H, $R_8$=H) | H | 5-$SCH_2CH_2Br$ | $OCH_3$ | $OCH_3$ | |
| J-11 | Q-1 ($R_7$=H, $R_8$=H) | H | 5-$SCH(CH_3)(CH_2Cl)$ | $OCH_3$ | $OCH_3$ | |
| J-11 | Q-1 ($R_7$=H, $R_8$=H) | H | 4-$NH_2$ | $OCH_3$ | $OCH_3$ | |
| J-11 | Q-1 ($R_7$=H, $R_8$=H) | H | 5-$NHCH_3$ | $OCH_3$ | $OCH_3$ | |
| J-11 | Q-1 ($R_7$=H, $R_8$=H) | H | 6-$NHCH_2CH_3$ | $OCH_3$ | $OCH_3$ | |
| J-11 | Q-1 ($R_7$=H, $R_8$=H) | H | 3-$NHCH(CH_3)_2$ | $OCH_3$ | $OCH_3$ | |
| J-11 | Q-1 ($R_7$=H, $R_8$=H) | H | 4-$N(CH_3)_2$ | $OCH_3$ | $OCH_3$ | |
| J-11 | Q-1*($R_7$=H, $R_8$=H) | H | 6-$N(CH_3)(CH_2CH_3)$ | $OCH_3$ | $OCH_3$ | |
| J-11 | Q-1 ($R_7$=H, $R_8$=H) | H | 5-$N(CH_3)(CH(CH_3)_2)$ | $OCH_3$ | $OCH_3$ | |
| J-11 | Q-1 ($R_7$=H, $R_8$=H) | H | 5-Cl | $OCH_3$ | $OCH_3$ | |

Table 2 (continued)

| J | Q | R | $R_{1d}$ | X | Y | m.p.(°C) |
|---|---|---|---|---|---|---|
| J-11 | Q-1 ($R_7$=H, $R_8$=H) | H | 3-Br | $OCH_3$ | $OCH_3$ | |
| J-11 | Q-1 ($R_7$=H, $R_8$=H) | H | 4-F | $OCH_3$ | $OCH_3$ | |
| J-11 | Q-1 ($R_7$=H, $R_8$=H) | H | 5-I | $OCH_3$ | $OCH_3$ | |
| J-11 | Q-1 ($R_7$=H, $R_8$=H) | H | 4-$NO_2$ | $OCH_3$ | $OCH_3$ | |
| J-11 | Q-1 ($R_7$=H, $R_8$=H) | H | 5-$CF_3$ | $OCH_3$ | $OCH_3$ | |
| J-11 | Q-1 ($R_7$=H, $R_8$=H) | H | 5-$OCF_2H$ | $OCH_3$ | $OCH_3$ | |
| J-11 | Q-1 ($R_7$=H, $R_8$=H) | H | H | $OCH_3$ | $CH_2CH_3$ | |
| J-11 | Q-1 ($R_7$=H, $R_8$=H) | H | H | $OCH_3$ | $CH(CH_3)_2$ | |
| J-11 | Q-1 ($R_7$=H, $R_8$=H) | H | H | $OCH_3$ | $(CH_2)_3CH_3$ | |
| J-11 | Q-1 ($R_7$=H, $R_8$=H) | H | H | $OCH_3$ | $OCH_2CH_3$ | |
| J-11 | Q-1 ($R_7$=H, $R_8$=H) | H | H | $OCH_3$ | $OCH(CH_3)_2$ | |
| J-11 | Q-1*($R_7$=H, $R_8$=H) | H | H | $OCH_3$ | $O(CH_2)_3CH_3$ | |
| J-11 | Q-1 ($R_7$=H, $R_8$=H) | H | H | $OCH_2CH_2F$ | $OCH_3$ | |
| J-11 | Q-1 ($R_7$=H, $R_8$=H) | H | H | $OCH_2CHF_2$ | $OCH_3$ | |
| J-11 | Q-1 ($R_7$=H, $R_8$=H) | H | H | $OCH_2CF_3$ | $OCH_3$ | |
| J-11 | Q-1 ($R_7$=H, $R_8$=H) | H | H | $OCH(CH_3)(CH_2Cl)$ | $OCH_3$ | |
| J-11 | Q-1 ($R_7$=H, $R_8$=H) | H | H | $O(CH_2)_3CH_2Br$ | $OCH_3$ | |
| J-11 | Q-1 ($R_7$=H, $R_8$=H) | H | H | $CH_2F$ | $OCH_3$ | |
| J-11 | Q-1 ($R_7$=H, $R_8$=H) | H | H | $CH_2Cl$ | $OCH_3$ | |
| J-11 | Q-1 ($R_7$=H, $R_8$=H) | H | H | $CH_2Br$ | $OCH_3$ | |
| J-11 | Q-1 ($R_7$=H, $R_8$=H) | H | H | $CF_3$ | $OCH_3$ | |
| J-11 | Q-1 ($R_7$=H, $R_8$=H) | H | H | $CH_2CH_2Br$ | $OCH_3$ | |
| J-11 | Q-1 ($R_7$=H, $R_8$=H) | H | H | $CH(CH_3)(CH_2Cl)$ | $OCH_3$ | |
| J-11 | Q-1 ($R_7$=H, $R_8$=H) | H | H | $(CH_2)_3CH_2I$ | $OCH_3$ | |
| J-11 | Q-1 ($R_7$=H, $R_8$=H) | H | H | $SCH_3$ | $OCH_3$ | |
| J-11 | Q-1*($R_7$=H, $R_8$=H) | H | H | $SCH_2CH_3$ | $OCH_3$ | |
| J-11 | Q-1 ($R_7$=H, $R_8$=H) | H | H | $SCH(CH_3)_2$ | $OCH_3$ | |
| J-11 | Q-1 ($R_7$=H, $R_8$=H) | H | H | $S(CH_2)_3CH_3$ | $OCH_3$ | |
| J-11 | Q-1 ($R_7$=H, $R_8$=H) | H | H | $SCHF_2$ | $OCH_3$ | |
| J-11 | Q-1 ($R_7$=H, $R_8$=H) | H | H | $SCH_2CH_2Br$ | $OCH_3$ | |
| J-11 | Q-1 ($R_7$=H, $R_8$=H) | H | H | $SCH(CH_3)(CH_2Cl)$ | $OCH_3$ | |

Table 2 (continued)

| J | Q | R | $R_{1d}$ | X | Y | m.p.(°C) |
|---|---|---|---|---|---|---|
| J-11 | Q-1 ($R_7$=H, $R_8$=H) | H | H | $S(CH_2)_3CH_2F$ | $OCH_3$ | |
| J-11 | Q-1 ($R_7$=H, $R_8$=H) | H | H | $OCH_3$ | $CH_2OCH_3$ | |
| J-11 | Q-1 ($R_7$=H, $R_8$=H) | H | H | $OCH_3$ | $CH_2CH_2OCH(CH_3)_2$ | |
| J-11 | Q-1 ($R_7$=H, $R_8$=H) | H | H | $OCH_3$ | $CH(CH_3)(CH_2OCH_3)$ | |
| J-11 | Q-1*($R_7$=H, $R_8$=H) | H | H | $OCH_3$ | $(CH_2)_4CH_2OCH_2CH_3$ | |
| J-11 | Q-1 ($R_7$=H, $R_8$=H) | H | H | $OCH_2O(CH_2)_3CH_3$ | $OCH_3$ | |
| J-11 | Q-1 ($R_7$=H, $R_8$=H) | H | H | $OCH_2CH_2OCH(CH_3)_2$ | $OCH_3$ | |
| J-11 | Q-1 ($R_7$=H, $R_8$=H) | H | H | $OCH(CH_3)(CH_2OCH_3)$ | $OCH_3$ | |
| J-11 | Q-1 ($R_7$=H, $R_8$=H) | H | H | $O(CH_2)_4CH_2OCH_2CH_3$ | $OCH_3$ | |
| J-11 | Q-1 ($R_7$=H, $R_8$=H) | H | H | $OCH_3$ | $NH_2$ | |
| J-11 | Q-1 ($R_7$=H, $R_8$=H) | H | H | $OCH_3$ | $NHCH_3$ | |
| J-11 | Q-1 ($R_7$=H, $R_8$=H) | H | H | $OCH_3$ | $NHCH_2CH_3$ | |
| J-11 | Q-1 ($R_7$=H, $R_8$=H) | H | H | $OCH_3$ | $NHCH(CH_3)_2$ | |
| J-11 | Q-1 ($R_7$=H, $R_8$=H) | H | H | $OCH_3$ | $N(CH_3)_2$ | |
| J-11 | Q-1 ($R_7$=H, $R_8$=H) | H | H | $OCH_3$ | $N(CH_3)(CH_2CH_3)$ | |
| J-11 | Q-1 ($R_7$=H, $R_8$=H) | H | H | $OCH_3$ | $N(CH_3)(CH(CH_3)_2)$ | |
| J-11 | Q-1 ($R_7$=H, $R_8$=H) | H | H | $OCH_3$ | $(CH_2)_4CH_2OCH_2CH_3$ | |
| J-11 | Q-1 ($R_7$=H, $R_8$=H) | H | H | H | $OCH_3$ | |
| J-11 | Q-1 ($R_7$=H, $R_8$=H) | H | H | $OCH_3$ | $OCH_2CH=CH_2$ | |
| J-11 | Q-1 ($R_7$=H, $R_8$=H) | H | H | $OCH_3$ | $OCH_2C(CH_3)=CH_2$ | |
| J-11 | Q-1 ($R_7$=H, $R_8$=H) | H | H | $OCH_3$ | $OCH_2C\equiv CH$ | |
| J-11 | Q-1*($R_7$=H, $R_8$=H) | H | H | $OCH_3$ | $OCH_2C\equiv CCH_3$ | |
| J-11 | Q-1 ($R_7$=H, $R_8$=H) | H | H | $OCH_3$ | $CH_2S(CH_2)_3CH_3$ | |
| J-11 | Q-1 ($R_7$=H, $R_8$=H) | H | H | $OCH_3$ | $CH_2CH_2SCH(CH_3)_2$ | |
| J-11 | Q-1 ($R_7$=H, $R_8$=H) | H | H | $OCH_3$ | $CH(CH_3)(CH_2SCH_3)$ | |
| J-11 | Q-1 ($R_7$=H, $R_8$=H) | H | H | $OCH_3$ | $(CH_2)_4CH_2SCH_2CH_3$ | |
| J-11 | Q-1 ($R_7$=H, $R_8$=H) | H | H | $OCH_3$ | cyclopropyl | |
| J-11 | Q-1 ($R_7$=H, $R_8$=H) | H | H | $OCH_3$ | 2-methylcyclopropyl | |
| J-11 | Q-1 ($R_7$=H, $R_8$=H) | H | H | $OCH_3$ | cyclopentyl | |
| J-11 | Q-1 ($R_7$=H, $R_8$=H) | H | H | $OCH_3$ | $C\equiv CH$ | |
| J-11 | Q-1 ($R_7$=H, $R_8$=H) | H | H | $OCH_3$ | $C\equiv CCH_3$ | |
| J-11 | Q-1 ($R_7$=H, $R_8$=H) | H | H | $OCH_3$ | -CHO | |

Table 2 (continued)

| J | Q | R | $R_{1d}$ | X | Y | m.p.(°C) |
|---|---|---|---|---|---|---|
| J-11 | Q-1 ($R_7$=H, $R_8$=H) | H | H | $OCH_3$ | $-COCH_3$ | |
| J-11 | Q-1 ($R_7$=H, $R_8$=H) | H | H | $OCH_3$ | $-CH(OCH_3)_2$ | |
| J-11 | Q-1 ($R_7$=H, $R_8$=H) | H | H | $OCH_3$ | $-CH(SCH_3)(OCH_2CH_3)$ | |
| J-11 | Q-1*($R_7$=H, $R_8$=H) | H | H | $OCH_3$ | $-C(CH_3)(SCH_3)_2$ | |
| J-11 | Q-1 ($R_7$=H, $R_8$=H) | H | H | $OCH_3$ | $-CH(SCH_2CH_3)_2$ | |
| J-11 | Q-1 ($R_7$=H, $R_8$=H) | H | H | $OCH_3$ | 1,3-dioxolan-2-yl | |
| J-11 | Q-1 ($R_7$=H, $R_8$=H) | H | H | $OCH_3$ | 2-methyl-1,3-oxathiolan-2-yl | |
| J-11 | Q-1 ($R_7$=H, $R_8$=H) | H | H | $OCH_3$ | 1,3-oxathian-2-yl | |
| J-11 | Q-1 ($R_7$=H, $R_8$=H) | H | H | $OCH_3$ | 2-methyl-1,3-dithian-2-yl | |
| J-11 | Q-1 ($R_7$=H, $R_8$=H) | H | H | $OCH_3$ | 4-methyl-1,3-dioxolan-2-yl | |
| J-11 | Q-1 ($R_7$=H, $R_8$=H) | H | H | $OCH_3$ | 4-methyl-1,3-oxathiolan-2-yl | |
| J-11 | Q-1 ($R_7$=H, $R_8$=H) | H | H | $OCH_3$ | 2,4-dimethyl-1,3-dithiolan-2-yl | |
| J-11 | Q-1 ($R_7$=H, $R_8$=H) | H | H | $OCH_3$ | $N(OCH_3)(CH_3)_2$ | |
| J-11 | Q-1 ($R_7$=H, $R_8$=H) | H | 6-$SO_2N(CH_3)_2$ | $OCH_3$ | $OCH_3$ | |
| J-11 | Q-1*($R_7$=H, $R_8$=H) | H | 6-$SO_2CH_3$ | $OCH_3$ | $OCH_3$ | |
| J-11 | Q-1 ($R_7$=H, $R_8$=H) | H | 6-$SO_2CH(CH_3)_2$ | $OCH_3$ | $OCH_3$ | |
| J-11 | Q-1 ($R_7$=H, $R_8$=H) | H | 6-$CO_2CH_3$ | $OCH_3$ | $OCH_3$ | |
| J-11 | Q-1 ($R_7$=H, $R_8$=$CH_3$) | H | H | $OCH_3$ | $OCH_3$ | 178.5-18 |
| J-11 | Q-1 ($R_7$=H, $R_8$=$CH_3$) | H | H | $CH_3$ | $CH_3$ | |
| J-11 | Q-1 ($R_7$=H, $R_8$=$CH_3$) | H | H | $CH_3$ | $OCH_3$ | 186-188 |
| J-11 | Q-1 ($R_7$=H, $R_8$=$CH_2CH_3$) | H | H | $OCH_3$ | $OCH_3$ | |
| J-11 | Q-1 ($R_7$=H, $R_8$=$CH(CH_3)_2$) | H | H | $OCH_3$ | $OCH_3$ | |
| J-11 | Q-1 ($R_7$=H, $R_8$=$(CH_2)_3CH_3$) | H | H | $OCH_3$ | $OCH_3$ | |
| J-11 | Q-1 ($R_7$=$CH_3$, $R_8$=$CH_3$) | H | H | $OCH_3$ | $OCH_3$ | |
| J-11 | Q-2 ($R_7$=H, $R_8$=H) | H | H | $OCH_3$ | $OCH_3$ | |
| J-11 | Q-2 ($R_7$=H, $R_8$=H) | $CH_3$ | H | $OCH_3$ | $OCH_3$ | |
| J-11 | Q-2 ($R_7$=H, $R_8$=$CH_3$) | H | 5-$OCH_3$ | $OCH_3$ | $OCH_3$ | |
| J-11 | Q-3 ($R_7$=H, $R_8$=H) | H | H | $OCH_3$ | $OCH_3$ | |

Table 2 (continued)

| J | Q | R | $R_{1d}$ | X | Y | m.p. (°C) |
|---|---|---|---|---|---|---|
| J-11 | Q-3 ($R_7$=$CH_3$, $R_8$=H) | H | 5-Cl | $OCH_3$ | $CH_3$ | |
| J-11 | Q-4 ($R_7$=H, $R_8$=H, $R_3$=$CH_3$) | H | H | $CH_3$ | $OCH_3$ | |
| J-11 | Q-4 ($R_7$=H, $R_8$=H, $R_3$=$C_2H_5$) | H | H | $CH_3$ | $OCH_3$ | |
| J-11 | Q-4 ($R_7$=H, $R_8$=H, $R_3$=i-$C_3H_7$) | H | 5-$CH_3$ | $OCH_3$ | $OCH_3$ | |
| J-11 | Q-5 ($R_7$-H, $R_8$=H, $R_3$=H) | H | H | $OCH_3$ | $OCH_3$ | |
| J-11 | Q-5 ($R_7$=H, $R_8$=H, $R_3$=$CH_3$) | H | H | $CH_3$ | $OCH_3$ | |
| J-11 | Q-5 ($R_7$=H, $R_8$=$CH_3$, $R_3$=$CH_3$) | H | H | $CH_3$ | $OCH_3$ | |
| J-11 | Q-6 ($R_7$=$CH_3$, $R_8$=H, $R_3$=$CH_3$) | H | H | $CH_3$ | $OCH_3$ | |
| J-11 | Q-6 ($R_7$-H, $R_8$=H, $R_3$=$C_2H_5$) | $CH_3$ | H | $CH_3$ | $OCH_3$ | |
| J-11 | Q-6 ($R_7$=$C_2H_5$, $R_8$=H, $R_3$=H) | H | H | $OCH_3$ | $OCH_3$ | |
| J-11 | Q-8 ($R_7$=H, $R_8$=H, $R_2$=H) | H | H | $OCH_3$ | $OCH_3$ | |
| J-11 | Q-8 ($R_7$=H, $R_8$=H, $R_2$=$OCH(CH_3)CH_2CH_3$) | H | 5-Cl | $OCH_3$ | $OCH_3$ | |
| J-11 | Q-9 ($R_7$=H, $R_8$=H, $R_2$=H) | H | H | $OCH_3$ | $OCH_3$ | |
| J-11 | Q-9 ($R_7$=$CH_3$, $R_8$=H, $R_2$=$CH_3$) | $CH_3$ | H | $OCH_3$ | $OCH_3$ | |
| J-11 | Q-9 ($R_7$=H, $R_8$=H, $R_2$=Cl) | H | 5-$OCH_3$ | $OCH_3$ | $OCH_3$ | |
| J-11 | Q-10 ($R_7$=H, $R_8$=H) | H | H | $OCH_3$ | $OCH_3$ | |
| J-11 | Q-10 ($R_7$=H, $R_8$=$CH_3$) | H | 5-Cl | $OCH_3$ | $CH_3$ | |
| J-11 | Q-11 ($R_7$=H, $R_8$=H) | H | H | $CH_3$ | $OCH_3$ | |
| J-11 | Q-11 ($R_7$=n-$C_4H_9$, $R_8$=H) | H | H | $CH_3$ | $OCH_3$ | |
| J-11 | Q-11 ($R_7$=$CH_3$, $R_8$=$CH_3$) | H | 5-$CH_3$ | $OCH_3$ | $OCH_3$ | |
| J-11 | Q-12 ($R_7$=H, $R_8$=H) | H | H | $OCH_3$ | $OCH_3$ | |
| J-11 | Q-12 ($R_7$=$CH_3$, $R_8$=H) | H | H | $CH_3$ | $OCH_3$ | |
| J-11 | Q-12 ($R_7$=H, $R_8$=$C_2H_5$) | H | H | $CH_3$ | $OCH_3$ | |
| J-11 | Q-13 ($R_7$=H, $R_8$=H, $R_3$=H) | H | H | $CH_3$ | $OCH_3$ | |
| J-11 | Q-13 ($R_7$=H, $R_8$=H, $R_3$=$CH_3$) | $CH_3$ | H | $CH_3$ | $OCH_3$ | |
| J-11 | Q-13 ($R_7$=$CH_3$, $R_8$=H, $R_3$=$CH_3$) | H | H | $OCH_3$ | $OCH_3$ | |

Table 2 (continued)

| J | Q | R | $R_{1d}$ | X | Y | m.p. (°C) |
|---|---|---|---|---|---|---|
| J-11 | Q-14 ($R_7$=H, $R_8$=H, $R_3$=$CH_3$) | H | H | $OCH_3$ | $OCH_3$ | |
| J-11 | Q-14 ($R_7$=$CH_3$, $R_8$=H, $R_3$=$C_2H_5$) | H | H | $OCH_3$ | $OCH_3$ | |
| J-11 | Q-14 ($R_7$=H, $R_8$=$CH_3$, $R_3$=$CH_3$) | H | 5-$OCH_3$ | $OCH_3$ | $OCH_3$ | |
| J-11 | Q-15 ($R_7$=H, $R_8$=H, $R_3$=H) | H | H | $OCH_3$ | $OCH_3$ | |
| J-11 | Q-15 ($R_7$=$CH_3$, $R_8$=$CH_3$, $R_3$=$CH_3$) | H | 5-Cl | $OCH_3$ | $OCH_3$ | |
| J-11 | Q-17 ($R_7$=H, $R_8$=H, $R_2$=H) | H | H | $OCH_3$ | $OCH_3$ | |
| J-11 | Q-17 ($R_7$=H, $R_8$=H, $R_2$=$C_2H_5$) | H | 5-Cl | $OCH_3$ | $CH_3$ | |
| J-11 | Q-18 ($R_7$=H, $R_8$=H, $R_2$=H) | H | H | $CH_3$ | $OCH_3$ | |
| J-11 | Q-18 ($R_7$=H, $R_8$=$OC_2H_5$, $R_2$=H) | H | H | $CH_3$ | $OCH_3$ | |
| J-11 | Q-18 ($R_7$=$CH_3$, $R_8$=$OCH(CH_3)_2$, $R_2$=$CH_3$) | H | 5-$CH_3$ | $OCH_3$ | $OCH_3$ | |
| J-11 | Q-19 ($R_7$=H, $R_8$=H) | H | H | $OCH_3$ | $OCH_3$ | |
| J-11 | Q-19 ($R_7$=$CH_3$, $R_8$=H) | H | H | $CH_3$ | $OCH_3$ | |
| J-11 | Q-19 ($R_7$=$CH_3$, $R_8$=$CH_3$) | H | H | $CH_3$ | $OCH_3$ | |
| J-11 | Q-20 ($R_7$=H, $R_8$=H) | H | H | $CH_3$ | $OCH_3$ | |
| J-11 | Q-20 ($R_7$=$CH_3$, $R_8$=H) | $CH_3$ | H | $CH_3$ | $OCH_3$ | |
| J-11 | Q-20 ($R_7$=H, $R_8$=$CH_3$) | H | H | $OCH_3$ | $OCH_3$ | |
| J-11 | Q-21 ($R_7$=H, $R_8$=H) | H | H | $OCH_3$ | $OCH_3$ | |
| J-11 | Q-21 ($R_7$=$CH_3$, $R_8$=H) | H | H | $OCH_3$ | $OCH_3$ | |
| J-11 | Q-21 ($R_7$=$CH_3$, $R_8$=$CH_3$) | H | 5-$OCH_3$ | $OCH_3$ | $OCH_3$ | |
| J-11 | Q-22 ($R_7$=H, $R_8$=H, $R_3$=H) | H | H | $OCH_3$ | $OCH_3$ | |
| J-11 | Q-22 ($R_7$=$CH_3$, $R_8$=$CH_3$, $R_3$=$C_2H_5$) | H | 5-Cl | $OCH_3$ | $OCH_3$ | |
| J-11 | Q-23 ($R_7$=H, $R_8$=H, $R_3$=H) | H | H | $OCH_3$ | $OCH_3$ | |
| J-11 | Q-23 ($R_7$=H, $R_8$=H, $R_3$=$CH_3$) | $CH_3$ | H | $OCH_3$ | $OCH_3$ | |
| J-11 | Q-23 ($R_7$=$CH_3$, $R_8$=H, $R_3$=$n$-$C_4H_9$) | H | 5-$OCH_3$ | $OCH_3$ | $OCH_3$ | |
| J-11 | Q-24 ($R_7$=H, $R_8$=H, $R_3$=$CH_3$) | H | H | $OCH_3$ | $OCH_3$ | |

Table 2 (continued)

| J | Q | R | $R_{1d}$ | X | Y | m.p. (°C) |
|---|---|---|---|---|---|---|
| J-11 | Q-24 ($R_7$=$CH_3$, $R_8$=$CH_3$, $R_3$=i-$C_3H_7$) | H | 5-Cl | $OCH_3$ | $CH_3$ | |
| J-11 | Q-25 ($R_7$=H, $R_8$=H, $R_9$=H) | H | H | $CH_3$ | $OCH_3$ | |
| J-11 | Q-25 ($R_7$=$CH_3$, $R_8$=H, $R_9$=H) | H | H | $CH_3$ | $OCH_3$ | |
| J-11 | Q-25 ($R_7$=H, $R_8$=H, $R_9$=$C_2H_5$) | H | 5-$CH_3$ | $OCH_3$ | $OCH_3$ | |
| J-11 | Q-26 ($R_7$=H, $R_8$=H) | H | H | $OCH_3$ | $OCH_3$ | |
| J-11 | Q-26 ($R_7$=$CH_3$, $R_8$=H) | H | H | $CH_3$ | $OCH_3$ | |
| J-11 | Q-26 ($R_7$=$CH_3$, $R_8$=$CH_3$) | H | H | $CH_3$ | $OCH_3$ | |
| J-11 | Q-27 ($R_7$=H, $R_8$=H) | H | H | $CH_3$ | $OCH_3$ | |
| J-11 | Q-27 ($R_7$=$CH_3$, $R_8$=H) | $CH_3$ | H | $CH_3$ | $OCH_3$ | |
| J-11 | Q-27 ($R_7$=H, $R_8$=$CH_3$) | H | H | $OCH_3$ | $OCH_3$ | |
| J-11 | Q-28*($R_7$=H, $R_8$=H) | H | H | $OCH_3$ | $OCH_3$ | |
| J-11 | Q-28 ($R_7$=$CH_3$, $R_8$=H) | H | H | $OCH_3$ | $OCH_3$ | |
| J-11 | Q-28 ($R_7$=$CH_3$, $R_8$=$CH_3$) | H | 5-$OCH_3$ | $OCH_3$ | $OCH_3$ | |
| J-11 | Q-29 ($R_7$=H, $R_8$=H) | H | H | $OCH_3$ | $OCH_3$ | |
| J-11 | Q-29 ($R_7$=H, $R_8$=$CH_3$) | H | 5-Cl | $OCH_3$ | $OCH_3$ | |
| J-11 | Q-30 ($R_7$=H, $R_8$=H) | H | H | $OCH_3$ | $OCH_3$ | |
| J-11 | Q-30 ($R_7$=$CH_3$, $R_8$=$CH_3$) | $CH_3$ | H | $OCH_3$ | $CH_3$ | |
| J-11 | Q-30 ($R_7$=$C_2H_5$, $R_8$=H) | H | 5-$OCH_3$ | $OCH_3$ | $OCH_3$ | |
| J-11 | Q-31 ($R_7$=H, $R_8$=H) | H | H | $OCH_3$ | $OCH_3$ | |
| J-11 | Q-31 ($R_7$=H, $R_8$=n-$C_4H_9$) | H | 5-Cl | $OCH_3$ | $CH_3$ | |
| J-11 | Q-32 ($R_7$=H, $R_8$=H, $R_3$=H) | H | H | $CH_3$ | $OCH_3$ | |
| J-11 | Q-32 ($R_7$=$CH_3$, $R_8$=H, $R_3$=$CH_3$) | H | H | $CH_3$ | $OCH_3$ | |
| J-11 | Q-32 ($R_7$=H, $R_8$=$CH_3$, $R_3$=$C_2H_5$) | H | 5-$CH_3$ | $OCH_3$ | $OCH_3$ | |
| J-11 | Q-33 ($R_7$=H, $R_8$=H, $R_8$=$CH_3$) | H | H | $OCH_3$ | $OCH_3$ | |
| J-11 | Q-33 ($R_7$=$CH_3$, $R_8$=$CH_3$, $R_3$=i-$C_3H_7$) | H | H | $CH_3$ | $OCH_3$ | |
| J-11 | Q-33 ($R_7$=$C_2H_5$, $R_8$=H, $R_3$=n-$C_4H_9$) | H | H | $CH_3$ | $OCH_3$ | |
| J-11 | Q-34 ($R_7$=H, $R_8$=H, $R_3$=H) | H | H | $CH_3$ | $OCH_3$ | |
| J-11 | Q-34 ($R_7$=i-$C_3H_7$, $R_8$=H, $R_3$=$CH_3$) | $CH_3$ | H | $CH_3$ | $OCH_3$ | |
| J-11 | Q-34 ($R_7$=H, $R_8$=n-$C_4H_9$, $R_3$=$CH_3$) | H | H | $OCH_3$ | $OCH_3$ | |

Table 2 (continued)

| J | Q | R | $R_{1d}$ | X | Y | m.p. (°C) |
|---|---|---|---|---|---|---|
| J-11 | Q-36 ($R_7$=H, $R_8$=H, $R_2$=H) | H | H | $OCH_3$ | $OCH_3$ | |
| J-11 | Q-36 ($R_7$=H, $R_8$=H, $R_2$=$C_2H_5$) | H | 5-Cl | $OCH_3$ | $OCH_3$ | |
| J-11 | Q-37 ($R_7$=H, $R_8$=H, $R_2$=H) | H | H | $OCH_3$ | $OCH_3$ | 191 |
| J-11 | Q-37 ($R_7$=H, $R_8$=H, $R_2$=H) | H | H | $OCH_3$ | $CH_3$ | 189 |
| J-11 | Q-37 ($R_7$=H, $R_8$=H, $R_2$=<u>i</u>-$C_3H_7$) | $CH_3$ | H | $OCH_3$ | $OCH_3$ | |
| J-11 | Q-37 ($R_7$=H, $R_8$=H, $R_2$=<u>n</u>-$C_4H_9$) | H | 5-$OCH_3$ | $OCH_3$ | $OCH_3$ | |
| J-11 | Q-38 ($R_7$=H, $R_8$=H, $R_2$=$OCF_2H$) | H | H | $OCH_3$ | $OCH_3$ | |
| J-11 | Q-38 ($R_7$=H, $R_8$=H, $R_2$=Cl) | H | 5-Cl | $OCH_3$ | $CH_3$ | |
| J-11 | Q-39 ($R_7$=H, $R_8$=H, $R_9$=H) | H | H | $CH_3$ | $OCH_3$ | |
| J-11 | Q-39 ($R_7$=$C_2H_5$, $R_8$=H, $R_9$=H) | H | H | $CH_3$ | $OCH_3$ | |
| J-11 | Q-39 ($R_7$=H, $R_8$=H, $R_9$=$CH_3$) | H | 5-$CH_3$ | $OCH_3$ | $OCH_3$ | |
| J-11 | Q-40 ($R_7$=H, $R_8$=H) | H | H | $OCH_3$ | $OCH_3$ | |
| J-11 | Q-40 ($R_7$=$CH_3$, $R_8$=H) | H | H | $CH_3$ | $OCH_3$ | |
| J-11 | Q-40 ($R_7$=H, $R_8$=$CH_3$) | H | H | $CH_3$ | $OCH_3$ | |
| J-11 | Q-41 ($R_7$=H, $R_8$=H) | H | H | $CH_3$ | $OCH_3$ | |
| J-11 | Q-41 ($R_7$=$CH_3$, $R_8$=$CH_3$) | $CH_3$ | H | $CH_3$ | $OCH_3$ | |
| J-11 | Q-41 ($R_7$=H, $R_8$=$C_2H_5$) | H | H | $OCH_3$ | $OCH_3$ | |
| J-11 | Q-42 ($R_7$=H, $R_8$=H) | H | H | $OCH_3$ | $OCH_3$ | |
| J-11 | Q-42 ($R_7$=$CH_3$, $R_8$=$CH_3$) | H | H | $OCH_3$ | $OCH_3$ | |
| J-11 | Q-42 ($R_7$=$C_2H_5$, $R_8$=$C_2H_5$) | H | 5-$OCH_3$ | $OCH_3$ | $OCH_3$ | |
| J-11 | Q-43 ($R_7$=H, $R_8$=H, $R_3$=H) | H | H | $OCH_3$ | $OCH_3$ | |
| J-11 | Q-43 ($R_7$=$CH_3$, $R_8$=H, $R_3$=$CH_3$) | H | 5-Cl | $OCH_3$ | $OCH_3$ | |
| J-11 | Q-44 ($R_7$=H, $R_8$=H, $R_3$=$CH_3$) | H | H | $OCH_3$ | $OCH_3$ | |
| J-11 | Q-44 ($R_7$=$CH_3$, $R_8$=H, $R_3$=$C_2H_5$) | $CH_3$ | H | $OCH_3$ | $CH_3$ | |
| J-11 | Q-44 ($R_7$=H, $R_8$=$CH_3$, $R_3$=$CH_3$) | H | 5-$OCH_3$ | $OCH_3$ | $OCH_3$ | |
| J-11 | Q-45 ($R_7$=H, $R_8$=H, $R_3$=H) | H | H | $OCH_3$ | $OCH_3$ | |
| J-11 | Q-45 ($R_7$=$CH_3$, $R_8$=$CH_3$, $R_3$=$CH_3$) | H | 5-Cl | $OCH_3$ | $CH_3$ | |

Table 2 (continued)

| J | Q | R | $R_{1d}$ | X | Y | m.p. (°C) |
|---|---|---|---|---|---|---|
| J-11 | Q-47 ($R_7$=H, $R_8$=H, $R_2$=H) | H | H | $OCH_3$ | $OCH_3$ | |
| J-11 | Q-47 ($R_7$=H, $R_8$=$CH_3$, $R_2$=$CH_3$) | H | H | $CH_3$ | $OCH_3$ | |
| J-11 | Q-47 ($R_7$=H, $R_8$=H, $R_2$=$C_2H_5$) | H | H | $CH_3$ | $OCH_3$ | |
| J-11 | Q-48 ($R_7$=H, $R_8$=H, $R_2$=H) | H | H | $CH_3$ | $OCH_3$ | |
| J-11 | Q-48 ($R_7$=$CH_3$, $R_8$=$CH_3$, $R_2$=$CH_3$) | $CH_3$ | H | $CH_3$ | $OCH_3$ | |
| J-11 | Q-48 ($R_7$=H, $R_8$=$C_2H_5$, $R_2$=$C_2H_5$) | H | H | $OCH_3$ | $OCH_3$ | |
| J-11 | Q-49 ($R_7$=H, $R_8$=H, $R_2$=H) | H | H | $OCH_3$ | $OCH_3$ | |
| J-11 | Q-49 ($R_7$=$CH_3$, $R_8$=$CH_3$, $R_2$=H) | H | H | $OCH_3$ | $OCH_3$ | |
| J-11 | Q-49 ($R_7$=$CH_3$, $R_8$=$CH_3$, $R_2$=$CH_3$) | H | 5-$OCH_3$ | $OCH_3$ | $OCH_3$ | |
| J-11 | Q-50 ($R_7$=H, $R_8$=H, $R_2$=H) | H | H | $OCH_3$ | $OCH_3$ | |
| J-11 | Q-50 ($R_7$=$CH_3$, $R_8$=$CH_3$, $R_2$=$CH_3$) | H | 5-Cl | $OCH_3$ | $OCH_3$ | |
| J-11 | Q-51 ($R_7$=H, $R_8$=H, $R_9$=H) | H | H | $OCH_3$ | $OCH_3$ | |
| J-11 | Q-51 ($R_7$=H, $R_8$=H, $R_9$=$CH_3$) | $CH_3$ | H | $OCH_3$ | $OCH_3$ | |
| J-11 | Q-51 ($R_7$=H, $R_8$=H, $R_9$=s-$C_4H_9$) | H | 5-$OCH_3$ | $OCH_3$ | $OCH_3$ | |
| J-11 | Q-52 ($R_7$=H, $R_8$=H, $R_9$=H) | H | H | $OCH_3$ | $OCH_3$ | |
| J-11 | Q-52 ($R_7$=H, $R_8$=H, $R_9$=$CH_3$) | H | 5-Cl | $OCH_3$ | $CH_3$ | |
| J-11 | Q-53 ($R_7$=H, $R_8$=H, $R_9$=H) | H | H | $CH_3$ | $OCH_3$ | |
| J-11 | Q-53 ($R_7$=$CH_3$, $R_8$=$CH_3$, $R_9$=$CH_3$) | H | H | $CH_3$ | $OCH_3$ | |
| J-11 | Q-53 ($R_7$=H, $R_8$=H, $R_9$=$CH_3$) | H | 5-$CH_3$ | $OCH_3$ | $OCH_3$ | |
| J-11 | Q-54 ($R_7$=H, $R_8$=H, $R_9$=H) | H | H | $OCH_3$ | $OCH_3$ | |
| J-11 | Q-54 ($R_7$=$CH_3$, $R_8$=H, $R_9$=H) | H | H | $CH_3$ | $OCH_3$ | |
| J-11 | Q-54 ($R_7$=H, $R_8$=H, $R_9$=i-$C_3H_7$) | H | H | $CH_3$ | $OCH_3$ | |
| J-11 | Q-55 ($R_7$=H, $R_8$=H, $R_9$=H, $R_3$=H) | H | H | $CH_3$ | $OCH_3$ | |
| J-11 | Q-55 ($R_7$=H, $R_8$=H, $R_9$=$CH_3$, $R_3$=$CH_3$) | $CH_3$ | H | $CH_3$ | $OCH_3$ | |
| J-11 | Q-55 ($R_7$=H, $R_8$=H, $R_9$=s-$C_4H_9$, $R_3$=H) | H | H | $OCH_3$ | $OCH_3$ | |
| J-11 | Q-56 ($R_7$=H, $R_8$=H, $R_9$=H, $R_3$=$C_2H_5$) | H | H | $OCH_3$ | $OCH_3$ | |
| J-11 | Q-56 ($R_7$=$CH_3$, $R_8$=H, $R_9$=H, $R_3$=H) | H | H | $OCH_3$ | $OCH_3$ | |

Table 2 (continued)

| J | Q | R | $R_{1d}$ | X | Y | m.p. (°C) |
|---|---|---|---|---|---|---|
| J-11 | Q-56 ($R_7$=H, $R_8$=H, $R_9$=$CH_3$, $R_3$=$CH_3$) | H | 5-$OCH_3$ | $OCH_3$ | $OCH_3$ | |
| J-11 | Q-57 ($R_7$=H, $R_8$=H, $R_9$=H, $R_3$=i-$C_3H_7$) | H | H | $OCH_3$ | $OCH_3$ | |
| J-11 | Q-57 ($R_7$=H, $R_8$=H, $R_9$=$CH_3$, $R_3$=$CH_3$) | H | 5-Cl | $OCH_3$ | $OCH_3$ | |
| J-11 | Q-58 ($R_7$=H, $R_8$=H, $R_9$=H, $R_3$=H) | H | H | $OCH_3$ | $OCH_3$ | |
| J-11 | Q-58 ($R_7$=$CH_3$, $R_8$=H, $R_9$=H, $R_3$=$CH_3$) | $CH_3$ | H | $OCH_3$ | $CH_3$ | |
| J-11 | Q-58 ($R_7$=H, $R_8$=H, $R_9$=i-$C_3H_7$, $R_3$=$CH_3$) | H | 5-$OCH_3$ | $OCH_3$ | $OCH_3$ | |
| J-11 | Q-60 ($R_7$=H, $R_8$=H, $R_9$=H, $R_2$=H) | H | H | $CH_3$ | $OCH_3$ | |
| J-11 | Q-60 ($R_7$=$CH_3$, $R_8$=H, $R_9$=H, $R_2$=$O(CH_2)_3CH_2Br$) | H | H | $CH_3$ | $OCH_3$ | |
| J-11 | Q-60 ($R_7$=H, $R_8$=H, $R_9$=H, $R_2$=Br) | H | 5-$CH_3$ | $OCH_3$ | $OCH_3$ | |
| J-11 | Q-61*($R_7$=H, $R_8$=H, $R_9$=H, $R_2$=H) | H | H | $OCH_3$ | $OCH_3$ | |
| J-11 | Q-61 ($R_7$=$CH_3$, $R_8$=H, $R_9$=H, $R_2$=$OCH_2F$) | H | H | $CH_3$ | $OCH_3$ | |
| J-11 | Q-61 ($R_7$=H, $R_8$=$CH_3$, $R_9$=H, $R_2$=$O(CH_2)_3CH_3$) | H | H | $CH_3$ | $OCH_3$ | |
| J-11 | Q-62 ($R_7$=H, $R_8$=H, $R_9$=H, $R_2$=H) | H | H | $CH_3$ | $OCH_3$ | |
| J-11 | Q-62 ($R_7$=$CH_3$, $R_8$=H, $R_9$=H, $R_2$=$CH_3$) | $CH_3$ | H | $CH_3$ | $OCH_3$ | |
| J-11 | Q-62 ($R_7$=Cl, $R_8$=H, $R_9$=H, $R_2$=H) | H | H | $OCH_3$ | $OCH_3$ | |
| J-11 | Q-63 ($R_7$=H, $R_8$=H, $R_9$=H) | H | H | $OCH_3$ | $OCH_3$ | |
| J-11 | Q-63 ($R_7$=$CH_3$, $R_8$=H, $R_9$=H) | H | H | $OCH_3$ | $OCH_3$ | |
| J-11 | Q-63 ($R_7$=H, $R_8$=H, $R_9$=$CH_3$) | H | 5-$OCH_3$ | $OCH_3$ | $OCH_3$ | |
| J-11 | Q-64 ($R_7$=H, $R_8$=H, $R_9$=H) | H | H | $OCH_3$ | $OCH_3$ | |
| J-11 | Q-64 ($R_7$=H, $R_8$=H, $R_9$=$C_2H_5$) | H | 5-Cl | $OCH_3$ | $OCH_3$ | |
| J-11 | Q-65 ($R_7$=H, $R_8$=H, $R_9$=H) | H | H | $OCH_3$ | $OCH_3$ | |
| J-11 | Q-65 ($R_7$=n-$C_4H_9$, $R_8$=H, $R_9$=H) | $CH_3$ | H | $OCH_3$ | $CH_3$ | |
| J-11 | Q-65 ($R_7$=H, $R_8$=$CH_3$, $R_9$=i-$C_3H_7$) | H | 5-$OCH_3$ | $OCH_3$ | $OCH_3$ | |
| J-11 | Q-66 ($R_7$=H, $R_8$=H, $R_9$=H) | H | H | $OCH_3$ | $OCH_3$ | |
| J-11 | Q-66 ($R_7$=H, $R_8$=$C_2H_5$, $R_9$=$CH_3$) | H | 5-Cl | $OCH_3$ | $CH_3$ | |
| J-11 | Q-67 ($R_7$=H, $R_8$=H, $R_9$=H, $R_3$=H) | H | H | $CH_3$ | $OCH_3$ | |

Table 2 (continued)

| J | Q | R | $R_{1d}$ | X | Y | m.p. (°C) |
|---|---|---|---|---|---|---|
| J-11 | Q-67 ($R_7$=$CH_3$, $R_8$=H, $R_9$=H, $R_3$=$CH_3$) | H | H | $CH_3$ | $OCH_3$ | |
| J-11 | Q-67 ($R_7$=H, $R_8$=H, $R_9$=$CH_3$, $R_3$=$C_2H_5$) | H | 5-$CH_3$ | $OCH_3$ | $OCH_3$ | |
| J-11 | Q-68 ($R_7$=H, $R_8$=H, $R_9$=H, $R_3$=$CH_3$) | H | H | $OCH_3$ | $OCH_3$ | |
| J-11 | Q-68 ($R_7$=$C_2H_5$, $R_8$=H, $R_9$=H, $R_3$=i-$C_3H_7$) | H | H | $CH_3$ | $OCH_3$ | |
| J-11 | Q-68 ($R_7$=H, $R_8$=H, $R_9$=$C_2H_5$, $R_3$=n-$C_4H_9$) | H | H | $CH_3$ | $OCH_3$ | |
| J-11 | Q-69 ($R_7$=H, $R_8$=H, $R_9$=H, $R_3$=$CH_3$) | H | H | $CH_3$ | $OCH_3$ | |
| J-11 | Q-69 ($R_7$=n-$C_4H_9$, $R_8$=H, $R_9$=H, $R_3$=$C_2H_5$) | $CH_3$ | H | $CH_3$ | $OCH_3$ | |
| J-11 | Q-69 ($R_7$=H, $R_8$=$CH_3$, $R_9$=i-$C_3H_7$, $R_3$=H) | H | H | $OCH_3$ | $OCH_3$ | |
| J-11 | Q-70 ($R_7$=H, $R_8$=H, $R_9$=H, $R_3$=$CH_3$) | H | H | $OCH_3$ | $OCH_3$ | |
| J-11 | Q-70 ($R_7$=i-$C_3H_7$, $R_8$=H, $R_9$=H, $R_3$=$CH_3$) | H | H | $OCH_3$ | $OCH_3$ | |
| J-11 | Q-70 ($R_7$=H, $R_8$=$C_2H_5$, $R_9$=$CH_3$, $R_3$=H) | H | 5-$OCH_3$ | $OCH_3$ | $OCH_3$ | |
| J-11 | Q-72 ($R_7$=H, $R_8$=H, $R_9$=H, $R_2$=H) | H | H | $OCH_3$ | $OCH_3$ | |
| J-11 | Q-72 ($R_7$=H, $R_8$=H, $R_9$=H, $R_2$=$CH_3$) | $CH_3$ | H | $OCH_3$ | $CH_3$ | |
| J-11 | Q-72 ($R_7$=$CH_3$, $R_8$=H, $R_9$=H, $R_2$=H) | H | 5-$OCH_3$ | $OCH_3$ | $OCH_3$ | |
| J-11 | Q-73 ($R_7$=H, $R_8$=H, $R_9$=H, $R_2$=H) | H | H | $OCH_3$ | $OCH_3$ | |
| J-11 | Q-73 ($R_7$=$CH_3$, $R_8$=H, $R_9$=$CH_3$, $R_2$=H) | H | 5-Cl | $OCH_3$ | $CH_3$ | |
| J-11 | Q-74 ($R_7$=H, $R_8$=H, $R_9$=H, $R_2$=H) | H | H | $CH_3$ | $OCH_3$ | |
| J-11 | Q-74 ($R_7$=$CH_3$, $R_8$=H, $R_9$=H, $R_2$=H) | H | H | $CH_3$ | $OCH_3$ | |
| J-11 | Q-74 ($R_7$=H, $R_8$=H, $R_9$=H, $R_2$=$CH_3$) | H | 5-$CH_3$ | $OCH_3$ | $OCH_3$ | |
| J-11 | Q-75 ($R_7$=H, $R_8$=H, $R_9$=H) | H | H | $OCH_3$ | $OCH_3$ | |
| J-11 | Q-75 ($R_7$=H, $R_8$=H, $R_9$=$CH_3$) | H | H | $CH_3$ | $OCH_3$ | |
| J-11 | Q-75 ($R_7$=$CH_3$, $R_8$=H, $R_9$=$CH_3$) | H | H | $CH_3$ | $OCH_3$ | |
| J-11 | Q-76 ($R_7$=H, $R_8$=H, $R_9$=H) | H | H | $CH_3$ | $OCH_3$ | |
| J-11 | Q-76 ($R_7$=H, $R_8$=H, $R_9$=$CH_3$) | $CH_3$ | H | $CH_3$ | $OCH_3$ | |
| J-11 | Q-76 ($R_7$=$C_2H_5$, $R_8$=H, $R_9$=$C_2H_5$) | H | H | $OCH_3$ | $OCH_3$ | |
| J-11 | Q-77 ($R_7$=H, $R_8$=H, $R_9$=H) | H | H | $OCH_3$ | $OCH_3$ | |
| J-11 | Q-77 ($R_7$=H, $R_8$=H, $R_9$=n-$C_3H_7$) | H | H | $OCH_3$ | $OCH_3$ | |
| J-11 | Q-77 ($R_7$=H, $R_8$=$CH_3$, $R_9$=H) | H | 5-$OCH_3$ | $OCH_3$ | $OCH_3$ | |

Table 2 (continued)

| J | Q | R | $R_{1d}$ | X | Y | m.p. (°C) |
|---|---|---|---|---|---|---|
| J-11 | Q-78 ($R_7$=H, $R_8$=H, $R_9$=H) | H | H | $OCH_3$ | $OCH_3$ | |
| J-11 | Q-78 ($R_7$=n-$C_3H_7$, $R_8$=H, $R_9$=H) | H | 5-Cl | $OCH_3$ | $OCH_3$ | |
| J-11 | Q-79 ($R_7$=H, $R_8$=H, $R_9$=H, $R_3$=H) | H | H | $OCH_3$ | $OCH_3$ | |
| J-11 | Q-79 ($R_7$=H, $R_8$=H, $R_9$=H, $R_3$=$CH_3$) | $CH_3$ | H | $OCH_3$ | $OCH_3$ | |
| J-11 | Q-79 ($R_7$=$CH_3$, $R_8$=H, $R_9$=H, $R_3$=$C_2H_5$) | H | 5-$OCH_3$ | $OCH_3$ | $OCH_3$ | |
| J-11 | Q-80 ($R_7$=H, $R_8$=H, $R_9$=H, $R_3$=$CH_3$) | H | H | $OCH_3$ | $OCH_3$ | |
| J-11 | Q-80 ($R_7$=H, $R_8$=H, $R_9$=$CH_3$, $R_3$=$C_2H_5$) | H | 5-Cl | $OCH_3$ | $CH_3$ | |
| J-11 | Q-81 ($R_7$=H, $R_8$=H, $R_9$=H, $R_3$=$CH_3$) | H | H | $CH_3$ | $OCH_3$ | |
| J-11 | Q-81 ($R_7$=H, $R_8$=$CH_3$, $R_9$=H, $R_3$=i-$C_3H_7$) | H | H | $CH_3$ | $OCH_3$ | |
| J-11 | Q-81 ($R_7$=$CH_3$, $R_8$=H, $R_9$=$CH_3$, $R_3$=$CH_3$) | H | 5-$CH_3$ | $OCH_3$ | $OCH_3$ | |
| J-11 | Q-82 ($R_7$=H, $R_8$=H, $R_9$=H, $R_3$=$CH_3$) | H | H | $OCH_3$ | $OCH_3$ | |
| J-11 | Q-82 ($R_7$=$CH_3$, $R_8$=H, $R_9$=$CH_3$, $R_3$=H) | H | H | $CH_3$ | $OCH_3$ | |
| J-11 | Q-82 ($R_7$=H, $R_8$=H, $R_9$=$C_2H_5$, $R_3$=$CH_3$) | H | H | $CH_3$ | $OCH_3$ | |
| J-11 | Q-83 ($R_7$=H, $R_8$=H, $R_9$=H, $R_{10}$=H) | H | H | $CH_3$ | $OCH_3$ | |
| J-11 | Q-83 ($R_7$=$CH_3$, $R_8$=H, $R_9$=H, $R_{10}$=$CH_3$) | $CH_3$ | H | $CH_3$ | $OCH_3$ | |
| J-11 | Q-83 ($R_7$=$CH_3$, $R_8$=$CH_3$, $R_9$=H, $R_{10}$=H) | H | H | $OCH_3$ | $OCH_3$ | |
| J-11 | Q-84 ($R_7$=H, $R_8$=H, $R_9$=H) | H | H | $OCH_3$ | $OCH_3$ | |
| J-11 | Q-84 ($R_7$=H, $R_8$=H, $R_9$=$CH_3$) | H | H | $OCH_3$ | $OCH_3$ | |
| J-11 | Q-84 ($R_7$=H, $R_8$=$C_2H_5$, $R_9$=H) | H | 5-$OCH_3$ | $OCH_3$ | $OCH_3$ | |
| J-11 | Q-85 ($R_7$=H, $R_8$=H, $R_9$=H) | H | H | $OCH_3$ | $OCH_3$ | |
| J-11 | Q-85 ($R_7$=H, $R_8$=H, $R_9$=$C_2H_5$) | H | 5-Cl | $OCH_3$ | $OCH_3$ | |
| J-11 | Q-86 ($R_7$=H, $R_8$=H, $R_9$=H) | H | H | $OCH_3$ | $OCH_3$ | |
| J-11 | Q-86 ($R_7$=$CH_3$, $R_8$=H, $R_9$=H) | $CH_3$ | H | $OCH_3$ | $OCH_3$ | |
| J-11 | Q-86 ($R_7$=H, $R_8$=H, $R_9$=$C_2H_5$) | H | 5-$OCH_3$ | $OCH_3$ | $OCH_3$ | |
| J-11 | Q-87 ($R_7$=H, $R_8$=H, $R_9$=H) | H | H | $OCH_3$ | $OCH_3$ | |
| J-11 | Q-87 ($R_7$=$CH_3$, $R_8$=H, $R_9$=H) | H | 5-Cl | $OCH_3$ | $CH_3$ | |
| J-11 | Q-88 ($R_7$=H, $R_8$=H, $R_9$=H) | H | H | $CH_3$ | $OCH_3$ | |
| J-11 | Q-88 ($R_7$=$CH_3$, $R_8$=H, $R_9$=H) | H | H | $CH_3$ | $OCH_3$ | |

Table 2 (continued)

| J | Q | R | $R_{1d}$ | X | Y | m.p. (°C) |
|---|---|---|---|---|---|---|
| J-11 | Q-88 ($R_7$=H, $R_8$=H, $R_9$=$CH_3$) | H | 5-$CH_3$ | $OCH_3$ | $OCH_3$ | |
| J-11 | Q-89 ($R_7$=H, $R_8$=H, $R_9$=H) | H | H | $OCH_3$ | $OCH_3$ | |
| J-11 | Q-89 ($R_7$=$CH_3$, $R_8$=H, $R_9$=$C_2H_5$) | H | H | $CH_3$ | $OCH_3$ | |
| J-11 | Q-89 ($R_7$=H, $R_8$=$CH_3$, $R_9$=H) | H | H | $CH_3$ | $OCH_3$ | |
| J-11 | Q-90 ($R_7$=H, $R_8$=H, $R_9$=H, $R_3$=$CH_3$) | H | H | $CH_3$ | $OCH_3$ | |
| J-11 | Q-90 ($R_7$=$CH_3$, $R_8$=H, $R_9$=H, $R_3$=$C_2H_5$) | $CH_3$ | H | $CH_3$ | $OCH_3$ | |
| J-11 | Q-90 ($R_7$=H, $R_8$=H, $R_9$=$CH_3$, $R_3$=$CH_3$) | H | H | $OCH_3$ | $OCH_3$ | |
| J-11 | Q-91 ($R_7$=H, $R_8$=H, $R_9$=H, $R_3$=$CH_3$) | H | H | $OCH_3$ | $OCH_3$ | |
| J-11 | Q-91 ($R_7$=$CH_3$, $R_8$=H, $R_9$=H, $R_3$=H) | H | H | $OCH_3$ | $OCH_3$ | |
| J-11 | Q-91 ($R_7$=H, $R_8$=H, $R_9$=$CH_3$, $R_3$=$CH_3$) | H | 5-$OCH_3$ | $OCH_3$ | $OCH_3$ | |
| J-11 | Q-92 ($R_7$=H, $R_8$=H, $R_9$=H, $R_{10}$=H) | H | H | $OCH_3$ | $OCH_3$ | |
| J-11 | Q-92 ($R_7$=H, $R_8$=$CH_3$, $R_9$=H, $R_{10}$=H) | H | 5-Cl | $OCH_3$ | $OCH_3$ | |
| J-11 | Q-93 ($R_7$=H, $R_8$=H, $R_9$=H) | H | H | $OCH_3$ | $OCH_3$ | |
| J-11 | Q-93 ($R_7$=H, $R_8$=H, $R_9$=$CH_3$) | $CH_3$ | H | $OCH_3$ | $OCH_3$ | |
| J-11 | Q-93 ($R_7$=$CH_3$, $R_8$=H, $R_9$=H) | H | 5-$OCH_3$ | $OCH_3$ | $OCH_3$ | |
| J-11 | Q-94 ($R_7$=H, $R_8$=H, $R_9$=H) | H | H | $OCH_3$ | $OCH_3$ | |
| J-11 | Q-94 ($R_7$=H, $R_8$=H, $R_9$=n-$C_4H_9$) | H | 5-Cl | $OCH_3$ | $CH_3$ | |
| J-11 | Q-95 ($R_7$=H, $R_8$=H, $R_9$=H) | H | H | $CH_3$ | $OCH_3$ | |
| J-11 | Q-95 ($R_7$=H, $R_8$=H, $R_9$=$CH_3$) | H | H | $CH_3$ | $OCH_3$ | |
| J-11 | Q-95 ($R_7$=H, $R_8$=H, $R_9$=s-$C_4H_9$) | H | 5-$CH_3$ | $OCH_3$ | $OCH_3$ | |
| J-11 | Q-96 ($R_7$=H, $R_8$=H, $R_9$=H) | H | H | $OCH_3$ | $OCH_3$ | |
| J-11 | Q-96 ($R_7$=$CH_3$, $R_8$=H, $R_9$=H) | H | H | $CH_3$ | $OCH_3$ | |
| J-11 | Q-96 ($R_7$=H, $R_8$=H, $R_9$=$CH_3$) | H | H | $CH_3$ | $OCH_3$ | |
| J-11 | Q-97 ($R_7$=H, $R_8$=H, $R_9$=H) | H | H | $CH_3$ | $OCH_3$ | |
| J-11 | Q-97 ($R_7$=$CH_3$, $R_8$=$CH_3$, $R_9$=$CH_3$) | $CH_3$ | H | $CH_3$ | $OCH_3$ | |
| J-11 | Q-97 ($R_7$=H, $R_8$=H, $R_9$=$CH_3$) | H | H | $OCH_3$ | $OCH_3$ | |
| J-11 | Q-98 ($R_7$=H, $R_8$=H, $R_9$=H) | H | H | $OCH_3$ | $OCH_3$ | |
| J-11 | Q-98 ($R_7$=$CH_3$, $R_8$=H, $R_9$=H) | H | H | $OCH_3$ | $OCH_3$ | |
| J-11 | Q-98 ($R_7$=H, $R_8$=H, $R_9$=i-$C_3H_7$) | H | 5-$OCH_3$ | $OCH_3$ | $OCH_3$ | |
| J-11 | Q-99 ($R_7$=H, $R_8$=H, $R_9$=H, $R_3$=H) | H | H | $OCH_3$ | $OCH_3$ | |

Table 2 (continued)

| J | Q | R | $R_{1d}$ | X | Y | m.p. (°C) |
|---|---|---|---|---|---|---|
| J-11 | Q-99 ($R_7$=H, $R_8$=H, $R_9$=s-$C_4H_9$, $R_3$=H) | H | 5-Cl | $OCH_3$ | $OCH_3$ | |
| J-11 | Q-100 ($R_7$=H, $R_8$=H, $R_9$=H, $R_3$=$C_2H_5$) | H | H | $OCH_3$ | $OCH_3$ | |
| J-11 | Q-100 ($R_7$=$CH_3$, $R_8$=H, $R_9$=H, $R_3$=H) | $CH_3$ | H | $OCH_3$ | $CH_3$ | |
| J-11 | Q-100 ($R_7$=H, $R_8$=H, $R_9$=$CH_3$, $R_3$=$CH_3$) | H | 5-$OCH_3$ | $OCH_3$ | $OCH_3$ | |
| J-11 | Q-101 ($R_7$=H, $R_8$=H, $R_9$=H, $R_3$=i-$C_3H_7$) | H | H | $OCH_3$ | $OCH_3$ | |
| J-11 | Q-101 ($R_7$=H, $R_8$=H, $R_9$=$CH_3$, $R_3$=$CH_3$) | H | 5-Cl | $OCH_3$ | $CH_3$ | |
| J-11 | Q-102*($R_7$=H, $R_8$=H, $R_9$=H, $R_3$=H) | H | H | $CH_3$ | $OCH_3$ | |
| J-11 | Q-102 ($R_7$=$CH_3$, $R_8$=H, $R_9$=H, $R_3$=$CH_3$) | H | H | $CH_3$ | $OCH_3$ | |
| J-11 | Q-102 ($R_7$=H, $R_8$=H, $R_9$=i-$C_3H_7$, $R_3$=$CH_3$) | H | 5-$CH_3$ | $OCH_3$ | $OCH_3$ | |
| J-11 | Q-104 ($R_7$=H, $R_8$=H, $R_9$=H, $R_2$=H) | H | H | $CH_3$ | $OCH_3$ | |
| J-11 | Q-104 ($R_7$=$CH_3$, $R_8$=H, $R_9$=H, $R_2$=$OC_2H_5$) | $CH_3$ | H | $CH_3$ | $OCH_3$ | |
| J-11 | Q-104 ($R_7$=H, $R_8$=H, $R_9$=H, $R_2$=Br) | H | H | $OCH_3$ | $OCH_3$ | |
| J-11 | Q-105 ($R_7$=H, $R_8$=H, $R_9$=H, $R_2$=H) | H | H | $OCH_3$ | $OCH_3$ | 180 |
| J-11 | Q-105 ($R_7$=H, $R_8$=H, $R_9$=H, $R_2$=H) | H | H | $OCH_3$ | $CH_3$ | 187 |
| J-11 | Q-105 ($R_7$=$CH_3$, $R_8$=H, $R_9$=H, $R_2$=$CH_3$) | H | H | $OCH_3$ | $OCH_3$ | |
| J-11 | Q-105 ($R_7$=H, $R_8$=$CH_3$, $R_9$=H, $R_2$=$C_2H_5$) | H | 5-$OCH_3$ | $OCH_3$ | $OCH_3$ | |
| J-11 | Q-106 ($R_7$=H, $R_8$=H, $R_9$=H, $R_2$=H) | H | H | $OCH_3$ | $OCH_3$ | |
| J-11 | Q-106 ($R_7$=Cl, $R_8$=H, $R_9$=H, $R_2$=H) | H | 5-Cl | $OCH_3$ | $OCH_3$ | |
| J-11 | Q-107 ($R_7$=H, $R_8$=H, $R_9$=H, $R_2$=H) | H | H | $OCH_3$ | $OCH_3$ | |
| J-11 | Q-107 ($R_7$=$CH_3$, $R_8$=H, $R_9$=H, $R_2$=H) | $CH_3$ | H | $OCH_3$ | $OCH_3$ | |
| J-11 | Q-107 ($R_7$=H, $R_8$=H, $R_9$=H, $R_2$=$CH_3$) | H | 5-$OCH_3$ | $OCH_3$ | $OCH_3$ | |
| J-11 | Q-108 ($R_7$=H, $R_8$=H, $R_9$=H, $R_2$=H) | H | H | $OCH_3$ | $OCH_3$ | |
| J-11 | Q-108 ($R_7$=H, $R_8$=H, $R_9$=H, $R_2$=$CH_3$) | H | 5-Cl | $OCH_3$ | $CH_3$ | |
| J-11 | Q-109 ($R_7$=H, $R_8$=H, $R_9$=H) | H | H | $CH_3$ | $OCH_3$ | |
| J-11 | Q-109 ($R_7$=$CH_3$, $R_8$=H, $R_9$=H) | H | H | $CH_3$ | $OCH_3$ | |
| J-11 | Q-109 ($R_7$=H, $R_8$=H, $R_9$=$CH_3$) | H | 5-$CH_3$ | $OCH_3$ | $OCH_3$ | |

Table 2 (continued)

| J | Q | R | $R_{1d}$ | X | Y | m.p. (°C) |
|---|---|---|---|---|---|---|
| J-11 | Q-110 ($R_7$=H, $R_8$=H, $R_9$=H) | H | H | $OCH_3$ | $OCH_3$ | |
| J-11 | Q-110 ($R_7$=$CH_3$, $R_8$=H, $R_9$=H) | H | H | $CH_3$ | $OCH_3$ | |
| J-11 | Q-110 ($R_7$=H, $R_8$=$CH_3$, $R_9$=H) | H | H | $CH_3$ | $OCH_3$ | |
| J-11 | Q-111 ($R_7$=H, $R_8$=H, $R_9$=H) | H | H | $CH_3$ | $OCH_3$ | |
| J-11 | Q-111 ($R_7$=$CH_3$, $R_8$=H, $R_9$=H) | $CH_3$ | H | $CH_3$ | $OCH_3$ | |
| J-11 | Q-111 ($R_7$=H, $R_8$=H, $R_9$=$CH_3$) | H | H | $OCH_3$ | $OCH_3$ | |
| J-11 | Q-112 ($R_7$=H, $R_8$=H, $R_9$=H) | H | H | $OCH_3$ | $OCH_3$ | |
| J-11 | Q-112 ($R_7$=$CH_3$, $R_8$=H, $R_9$=H) | H | H | $OCH_3$ | $OCH_3$ | |
| J-11 | Q-112 ($R_7$=H, $R_8$=$CH_3$, $R_9$=$CH_3$) | H | 5-$OCH_3$ | $OCH_3$ | $OCH_3$ | |
| J-11 | Q-113 ($R_7$=H, $R_8$=H, $R_9$=H, $R_3$=$CH_3$) | H | H | $OCH_3$ | $OCH_3$ | |
| J-11 | Q-113 ($R_7$=H, $R_8$=H, $R_9$=$C_2H_5$, $R_3$=n-$C_4H_9$) | H | 5-Cl | $OCH_3$ | $OCH_3$ | |
| J-11 | Q-114 ($R_7$=H, $R_8$=H, $R_9$=H, $R_3$=$CH_3$) | H | H | $OCH_3$ | $OCH_3$ | |
| J-11 | Q-114 ($R_7$=n-$C_4H_9$, $R_8$=H, $R_9$=H, $R_3$=$C_2H_5$) | $CH_3$ | H | $OCH_3$ | $CH_3$ | |
| J-11 | Q-114 ($R_7$=H, $R_8$=$CH_3$, $R_8$=i-$C_3H_7$, $R_3$=H) | H | 5-$OCH_3$ | $OCH_3$ | $OCH_3$ | |
| J-11 | Q-115 ($R_7$=H, $R_8$=H, $R_9$=H, $R_3$=$CH_3$) | H | H | $OCH_3$ | $OCH_3$ | |
| J-11 | Q-115 ($R_7$=H, $R_8$=$C_2H_5$, $R_9$=$CH_3$, $R_3$=H) | H | 5-Cl | $OCH_3$ | $CH_3$ | |
| J-11 | Q-116 ($R_7$=H, $R_8$=H, $R_9$=H, $R_{10}$=H) | H | H | $CH_3$ | $OCH_3$ | |
| J-11 | Q-116 ($R_7$=$CH_3$, $R_8$=H, $R_9$=H, $R_{10}$=H) | H | H | $CH_3$ | $OCH_3$ | |
| J-11 | Q-116 ($R_7$=H, $R_8$=H, $R_9$=H, $R_{10}$=$CH_3$) | H | 5-$CH_3$ | $OCH_3$ | $OCH_3$ | |
| J-11 | Q-118 ($R_7$=H, $R_8$=H, $R_9$=H, $R_{10}$=H) | H | H | $CH_3$ | $OCH_3$ | |
| J-11 | Q-118 ($R_7$=$CH_3$, $R_8$=H, $R_9$=H, $R_{10}$=H) | $CH_3$ | H | $CH_3$ | $OCH_3$ | |
| J-11 | Q-118 ($R_7$=H, $R_8$=H, $R_9$=H, $R_{10}$=$CH_3$) | H | H | $OCH_3$ | $OCH_3$ | |
| J-11 | Q-119 ($R_7$=H, $R_8$=H, $R_9$=H, $R_2$=H) | H | H | $OCH_3$ | $OCH_3$ | |
| J-11 | Q-119 ($R_7$=H, $R_8$=H, $R_9$=H, $R_2$=$CH_3$) | H | H | $OCH_3$ | $OCH_3$ | |
| J-11 | Q-119 ($R_7$=H, $R_8$=$CH_3$, $R_9$=H, $R_2$=$C_2H_5$) | H | 5-$OCH_3$ | $OCH_3$ | $OCH_3$ | |
| J-11 | Q-120 ($R_7$=H, $R_8$=H, $R_9$=H, $R_2$=H) | H | H | $OCH_3$ | $OCH_3$ | |

Table 2 (continued)

| J | Q | R | $R_{1d}$ | X | Y | m.p. (°C) |
|---|---|---|---|---|---|---|
| J-11 | Q-120 ($R_7$=H, $R_8$=H, $R_9$=$CH_3$, $R_2$=$CH_3$) | H | 5-Cl | $OCH_3$ | $OCH_3$ | |
| J-11 | Q-121 ($R_7$=H, $R_8$=H, $R_9$=H, $R_2$=H) | H | H | $OCH_3$ | $OCH_3$ | |
| J-11 | Q-121 ($R_7$=H, $R_8$=H, $R_9$=H, $R_2$=$C_2H_5$) | $CH_3$ | H | $OCH_3$ | $CH_3$ | |
| J-11 | Q-121 ($R_7$=$CH_3$, $R_8$=$CH_3$, $R_9$=H, $R_2$=H) | H | 5-$OCH_3$ | $OCH_3$ | $OCH_3$ | |
| J-11 | Q-122 ($R_7$=H, $R_8$=H, $R_9$=H, $R_2$=H) | H | H | $OCH_3$ | $OCH_3$ | |
| J-11 | Q-122 ($R_7$=$CH_3$, $R_8$=H, $R_9$=H, $R_2$=H) | H | 5-Cl | $OCH_3$ | $CH_3$ | |
| J-11 | Q-123 ($R_7$=H, $R_8$=H, $R_9$=H) | H | H | $CH_3$ | $OCH_3$ | |
| J-11 | Q-123 ($R_7$=H, $R_8$=H, $R_9$=$CH_3$) | H | H | $CH_3$ | $OCH_3$ | |
| J-11 | Q-123 ($R_7$=$CH_3$, $R_8$=H, $R_9$=$CH_3$) | H | 5-$CH_3$ | $OCH_3$ | $OCH_3$ | |
| J-11 | Q-124 ($R_7$=H, $R_8$=H, $R_9$=H) | H | H | $OCH_3$ | $OCH_3$ | |
| J-11 | Q-124 ($R_7$=H, $R_8$=H, $R_9$=$CH_3$) | H | H | $CH_3$ | $OCH_3$ | |
| J-11 | Q-124 ($R_7$=$C_2H_5$, $R_8$=H, $R_9$=$C_2H_5$) | H | H | $CH_3$ | $OCH_3$ | |
| J-11 | Q-125 ($R_7$=H, $R_8$=H, $R_9$=H) | H | H | $CH_3$ | $OCH_3$ | |
| J-11 | Q-125 ($R_7$=H, $R_8$=H, $R_9$=n-$C_4H_9$) | $CH_3$ | H | $CH_3$ | $OCH_3$ | |
| J-11 | Q-125 ($R_7$=H, $R_8$=$CH_3$, $R_9$=H) | H | H | $OCH_3$ | $OCH_3$ | |
| J-11 | Q-126 ($R_7$=H, $R_8$=H, $R_9$=H) | H | H | $OCH_3$ | $OCH_3$ | |
| J-11 | Q-126 ($R_7$=H, $R_8$=H, $R_9$=s-$C_3H_7$) | H | H | $OCH_3$ | $OCH_3$ | |
| J-11 | Q-126 ($R_7$=n-$C_3H_7$, $R_8$=H, $R_9$=H) | H | 5-$OCH_3$ | $OCH_3$ | $OCH_3$ | |
| J-11 | Q-127 ($R_7$=H, $R_8$=H, $R_9$=H, $R_3$=H) | H | H | $OCH_3$ | $OCH_3$ | |
| J-11 | Q-127 ($R_7$=$CH_3$, $R_8$=H, $R_9$=H, $R_3$=$C_2H_5$) | H | 5-Cl | $OCH_3$ | $OCH_3$ | |
| J-11 | Q-128 ($R_7$=H, $R_8$=H, $R_9$=H, $R_3$=$CH_3$) | H | H | $OCH_3$ | $OCH_3$ | |
| J-11 | Q-128 ($R_7$=$CH_3$, $R_8$=H, $R_9$=H, $R_3$=$CH_3$) | $CH_3$ | H | $OCH_3$ | $OCH_3$ | |
| J-11 | Q-128 ($R_7$=H, $R_8$=H, $R_9$=$CH_3$, $R_3$=$C_2H_5$) | H | 5-$OCH_3$ | $OCH_3$ | $OCH_3$ | |
| J-11 | Q-129 ($R_7$=H, $R_8$=H, $R_9$=H, $R_3$=$CH_3$) | H | H | $OCH_3$ | $OCH_3$ | |
| J-11 | Q-129 ($R_7$=$CH_3$, $R_8$=H, $R_9$=$CH_3$, $R_3$=$CH_3$) | H | 5-Cl | $OCH_3$ | $CH_3$ | |
| J-11 | Q-130 ($R_7$=H, $R_8$=H, $R_9$=H, $R_3$=$CH_3$) | H | H | $CH_3$ | $OCH_3$ | |
| J-11 | Q-130 ($R_7$=$CH_3$, $R_8$=H, $R_9$=$CH_3$, $R_3$=H) | H | H | $CH_3$ | $OCH_3$ | |
| J-11 | Q-130 ($R_7$=H, $R_8$=H, $R_9$=$C_2H_5$, $R_3$=$CH_3$) | H | 5-$CH_3$ | $OCH_3$ | $OCH_3$ | |
| J-11 | Q-131 ($R_7$=H, $R_8$=H, $R_9$=H, $R_{10}$=H) | H | H | $OCH_3$ | $OCH_3$ | |

Table 2 (continued)

| J | Q | R | $R_{1d}$ | X | Y | m.p. (°C) |
|---|---|---|---|---|---|---|
| J-11 | Q-131 ($R_7$=$CH_3$, $R_8$=H, $R_9$=H, $R_{10}$=$CH_3$) | H | H | $CH_3$ | $OCH_3$ | |
| J-11 | Q-131 ($R_7$=$CH_3$, $R_8$=$CH_3$, $R_9$=H, $R_{10}$=H) | H | H | $CH_3$ | $OCH_3$ | |
| J-11 | Q-132 ($R_7$=H, $R_8$=H, $R_9$=H) | H | H | $CH_3$ | $OCH_3$ | |
| J-11 | Q-132 ($R_7$=H, $R_8$=H, $R_9$=$CH_3$) | $CH_3$ | H | $CH_3$ | $OCH_3$ | |
| J-11 | Q-132 ($R_7$=H, $R_8$=$C_2H_5$, $R_9$=H) | H | H | $OCH_3$ | $OCH_3$ | |
| J-11 | Q-133 ($R_7$=H, $R_8$=H, $R_9$=H) | H | H | $OCH_3$ | $OCH_3$ | |
| J-11 | Q-133 ($R_7$=$CH_3$, $R_8$=H, $R_9$=H) | H | H | $OCH_3$ | $OCH_3$ | |
| J-11 | Q-133 ($R_7$=H, $R_8$=H, $R_9$=$C_2H_5$) | H | 5-$OCH_3$ | $OCH_3$ | $OCH_3$ | |
| J-11 | Q-134 ($R_7$=H, $R_8$=H, $R_9$=H) | H | H | $OCH_3$ | $OCH_3$ | |
| J-11 | Q-134 ($R_7$=H, $R_8$=H, $R_9$=$C_2H_5$) | H | 5-Cl | $OCH_3$ | $OCH_3$ | |
| J-11 | Q-135*($R_7$=H, $R_8$=H, R =H) | H | H | $OCH_3$ | $OCH_3$ | |
| J-11 | Q-135 ($R_7$=H, $R_8$=H, R =$CH_3$) | $CH_3$ | H | $OCH_3$ | $OCH_3$ | |
| J-11 | Q-135 ($R_7$=$CH_3$, $R_8$=H, $R_9$=H) | H | 5-$OCH_3$ | $OCH_3$ | $OCH_3$ | |
| J-11 | Q-136 ($R_7$=H, $R_8$=H, $R_9$=H) | H | H | $OCH_3$ | $OCH_3$ | |
| J-11 | Q-136 ($R_7$=H, $R_8$=$CH_3$, $R_9$=$C_2H_5$) | H | 5-Cl | $OCH_3$ | $CH_3$ | |
| J-11 | Q-137 ($R_7$=H, $R_8$=H, $R_9$=H) | H | H | $CH_3$ | $OCH_3$ | |
| J-11 | Q-137 ($R_7$=$CH_3$, $R_8$=H, $R_9$=H) | H | H | $CH_3$ | $OCH_3$ | |
| J-11 | Q-137 ($R_7$=H, $R_8$=H, $R_9$=$CH_3$) | H | 5-$CH_3$ | $OCH_3$ | $OCH_3$ | |
| J-11 | Q-138 ($R_7$=H, $R_8$=H, $R_9$=H) | H | H | $OCH_3$ | $OCH_3$ | 192 |
| J-11 | Q-138 ($R_7$=H, $R_8$=H, $R_9$=H) | H | H | $OCH_3$ | $CH_3$ | 177 |
| J-11 | Q-138 ($R_7$=$CH_3$, $R_8$=H, $R_9$=H) | H | H | $CH_3$ | $OCH_3$ | |
| J-11 | Q-138 ($R_7$=H, $R_8$=H, $R_9$=$CH_3$) | H | H | $CH_3$ | $OCH_3$ | |
| J-11 | Q-139 ($R_7$=H, $R_8$=H, $R_9$=H) | H | H | $CH_3$ | $OCH_3$ | |
| J-11 | Q-139 ($R_7$=$CH_3$, $R_8$=H, $R_9$=H) | $CH_3$ | H | $CH_3$ | $OCH_3$ | |
| J-11 | Q-139 ($R_7$=$CH_3$, $R_8$=$CH_3$, $R_9$=H) | H | H | $OCH_3$ | $OCH_3$ | |
| J-11 | Q-140 ($R_7$=H, $R_8$=H, $R_9$=H, $R_3$=$CH_3$) | H | H | $OCH_3$ | $OCH_3$ | |
| J-11 | Q-140 ($R_7$=H, $R_8$=H, $R_9$=$CH_3$, $R_3$=H) | H | H | $OCH_3$ | $OCH_3$ | |
| J-11 | Q-140 ($R_7$=H, $R_8$=$CH_3$, $R_9$=H, $R_3$=$C_2H_5$) | H | 5-$OCH_3$ | $OCH_3$ | $OCH_3$ | |
| J-11 | Q-141 ($R_7$=H, $R_8$=H, $R_9$=H, $R_3$=$CH_3$) | H | H | $OCH_3$ | $OCH_3$ | |
| J-11 | Q-141 ($R_7$=H, $R_8$=H, $R_9$=$CH_3$, $R_3$=$CH_3$) | H | 5-Cl | $OCH_3$ | $OCH_3$ | |

Table 2 (continued)

| J | Q | R | $R_{1d}$ | X | Y | m.p.(°C) |
|---|---|---|---|---|---|---|
| J-11 | Q-142 ($R_7$=H, $R_8$=H, $R_9$=H, $R_{10}$=H) | H | H | $OCH_3$ | $OCH_3$ | |
| J-11 | Q-142 ($R_7$=$CH_3$, $R_8$=H, $R_9$=H, $R_{10}$=H) | $CH_3$ | H | $OCH_3$ | $CH_3$ | |
| J-11 | Q-142 ($R_7$=H, $R_8$=H, $R_9$=H, $R_{10}$=$CH_3$) | H | 5-$OCH_3$ | $OCH_3$ | $OCH_3$ | |
| J-11 | Q-143 ($R_7$=H, $R_8$=H, $R_9$=H, $R_3$=$CH_3$) | H | H | $OCH_3$ | $OCH_3$ | |
| J-11 | Q-143 ($R_7$=$CH_3$, $R_8$=H, $R_9$=$CH_3$, $R_3$=$CH_3$) | H | 5-Cl | $OCH_3$ | $CH_3$ | |
| J-11 | Q-143 ($R_7$=H, $R_8$=H, $R_9$=H, $R_3$=n-$C_3H_7$) | H | H | $OCH_3$ | $OCH_3$ | 155-160 |
| J-11 | Q-143 ($R_7$=H, $R_8$=H, $R_9$=H, $R_3$=n-$C_3H_7$) | H | H | $CH_3$ | $OCH_3$ | 220-225 |
| J-11 | Q-144 ($R_7$=H, $R_8$=H, $R_9$=H, $R_3$=$CH_3$) | H | H | $CH_3$ | $OCH_3$ | |
| J-11 | Q-144 ($R_7$=$CH_3$, $R_8$=H, $R_9$=H, $R_3$=$CH_3$) | H | H | $CH_3$ | $OCH_3$ | |
| J-11 | Q-144 ($R_7$=H, $R_8$=$C_2H_5$, $R_9$=H, $R_3$=$CH_3$) | H | 5-$CH_3$ | $OCH_3$ | $OCH_3$ | |
| J-11 | Q-145 ($R_7$=H, $R_8$=H, $R_9$=H) | H | H | $OCH_3$ | $OCH_3$ | |
| J-11 | Q-145 ($R_7$=H, $R_8$=H, $R_9$=$CH_3$) | H | H | $CH_3$ | $OCH_3$ | |
| J-11 | Q-145 ($R_7$=$CH_3$, $R_8$=H, $R_9$=H) | H | H | $CH_3$ | $OCH_3$ | |
| J-11 | Q-146 ($R_7$=H, $R_8$=H, $R_9$=H) | H | H | $CH_3$ | $OCH_3$ | |
| J-11 | Q-146 ($R_7$=$CH_3$, $R_8$=H, $R_9$=H) | $CH_3$ | H | $CH_3$ | $OCH_3$ | |
| J-11 | Q-146 ($R_7$=H, $R_8$=H, $R_9$=n-$C_4H_9$) | H | H | $OCH_3$ | $OCH_3$ | |
| J-11 | Q-147 ($R_7$=H, $R_8$=H, $R_9$=H) | H | H | $OCH_3$ | $OCH_3$ | |
| J-11 | Q-147 ($R_7$=$CH_3$, $R_8$=H, $R_9$=H) | H | H | $OCH_3$ | $OCH_3$ | |
| J-11 | Q-147 ($R_7$=H, $R_8$=H, $R_9$=n-$C_4H_9$) | H | 5-$OCH_3$ | $OCH_3$ | $OCH_3$ | |
| J-11 | Q-148 ($R_7$=H, $R_8$=H, $R_9$=H) | H | H | $OCH_3$ | $OCH_3$ | |
| J-11 | Q-148 ($R_7$=$CH_3$, $R_8$=H, $R_9$=H) | H | 5-Cl | $OCH_3$ | $OCH_3$ | |
| J-11 | Q-149 ($R_7$=H, $R_8$=H, $R_9$=H) | H | H | $OCH_3$ | $OCH_3$ | |
| J-11 | Q-149 ($R_7$=H, $R_8$=H, $R_9$=$CH_3$) | $CH_3$ | H | $OCH_3$ | $OCH_3$ | |
| J-11 | Q-149 ($R_7$=$CH_3$, $R_8$=H, $R_9$=H) | H | 5-$OCH_3$ | $OCH_3$ | $OCH_3$ | |
| J-11 | Q-150 ($R_7$=H, $R_8$=H, $R_9$=H) | H | H | $OCH_3$ | $OCH_3$ | |
| J-11 | Q-150 ($R_7$=H, $R_8$=$CH_3$, $R_9$=$CH_3$) | H | 5-Cl | $OCH_3$ | $CH_3$ | |
| J-11 | Q-151 ($R_7$=H, $R_8$=H, $R_9$=H) | H | H | $CH_3$ | $OCH_3$ | |
| J-11 | Q-151 ($R_7$=$CH_3$, $R_8$=H, $R_9$=H) | H | H | $CH_3$ | $OCH_3$ | |
| J-11 | Q-151 ($R_7$=$CH_3$, $R_8$=$CH_3$, $R_9$=H) | H | 5-$CH_3$ | $OCH_3$ | $OCH_3$ | |
| J-11 | Q-152 ($R_7$=H, $R_8$=H, $R_9$=H) | H | H | $OCH_3$ | $OCH_3$ | |
| J-11 | Q-152 ($R_7$=$C_2H_5$, $R_8$=H, $R_9$=H) | H | H | $CH_3$ | $OCH_3$ | |

Table 2 (continued)

| J | Q | R | $R_{1d}$ | X | Y | m.p. (°C) |
|---|---|---|---|---|---|---|
| J-11 | Q-152 ($R_7$=$CH_3$, $R_8$=$CH_3$, $R_9$=H) | H | H | $CH_3$ | $OCH_3$ | |
| J-11 | Q-153 ($R_7$=H, $R_8$=H, $R_9$=H, $R_{11}$=$CH_3$) | H | H | $CH_3$ | $OCH_3$ | |
| J-11 | Q-153 ($R_7$=H, $R_8$=H, $R_9$=H, $R_{11}$=H) | $CH_3$ | H | $CH_3$ | $OCH_3$ | |
| J-11 | Q-153 ($R_7$=$CH_3$, $R_8$=H, $R_9$=H, $R_{11}$=$CH_3$) | H | H | $OCH_3$ | $OCH_3$ | |
| J-11 | Q-154 ($R_7$=H, $R_8$=H, $R_9$=H, $R_{11}$=$CH_3$) | H | H | $OCH_3$ | $OCH_3$ | |
| J-11 | Q-154 ($R_7$=$CH_3$, $R_8$=H, $R_9$=H) | H | H | $OCH_3$ | $OCH_3$ | |
| J-11 | Q-154 ($R_7$=H, $R_8$=$CH_3$, $R_9$=$CH_3$) | H | 5-$OCH_3$ | $OCH_3$ | $OCH_3$ | |
| J-11 | Q-155 ($R_7$=H, $R_8$=H, $R_9$=H) | H | H | $OCH_3$ | $OCH_3$ | |
| J-11 | Q-155 ($R_7$=$CH_3$, $R_8$=$CH_3$, $R_9$=H) | H | 5-Cl | $OCH_3$ | $OCH_3$ | |
| J-11 | Q-156 ($R_7$=H, $R_8$=H, $R_9$=H) | H | H | $OCH_3$ | $OCH_3$ | |
| J-11 | Q-156 ($R_7$=$C_2H_5$, $R_8$=H, $R_9$=H) | $CH_3$ | H | $OCH_3$ | $OCH_3$ | |
| J-11 | Q-156 ($R_7$=$CH_3$, $R_8$=$CH_3$, $R_9$=H) | H | 5-$OCH_3$ | $OCH_3$ | $OCH_3$ | |
| J-11 | Q-158 ($R_7$=H, $R_8$=H, $R_9$=H) | H | H | $CH_3$ | $OCH_3$ | |
| J-11 | Q-158 ($R_7$=H, $R_8$=H, $R_9$=$CH_3$) | H | H | $CH_3$ | $OCH_3$ | |
| J-11 | Q-158 ($R_7$=$CH_3$, $R_8$=H, $R_9$=H) | H | 5-$CH_3$ | $OCH_3$ | $OCH_3$ | |
| J-11 | Q-159 ($R_7$=H, $R_8$=H, $R_9$=H) | H | H | $OCH_3$ | $OCH_3$ | |
| J-11 | Q-159 ($R_7$=$CH_3$, $R_8$=H, $R_9$=H) | H | H | $CH_3$ | $OCH_3$ | |
| J-11 | Q-159 ($R_7$=H, $R_8$=H, $R_9$=$CH_3$) | H | H | $CH_3$ | $OCH_3$ | |
| J-11 | Q-160 ($R_7$=H, $R_8$=H, $R_9$=H, $R_{11}$=$CH_3$) | H | H | $CH_3$ | $OCH_3$ | |
| J-11 | Q-160 ($R_7$=H, $R_8$=H, $R_9$=$CH_3$, $R_{11}$=H) | $CH_3$ | H | $CH_3$ | $OCH_3$ | |
| J-11 | Q-160 ($R_7$=$CH_3$, $R_8$=H, $R_9$=H, $R_{11}$=i-$C_3H_7$ | H | H | $OCH_3$ | $OCH_3$ | |
| J-11 | Q-161 ($R_7$=H, $R_8$=H, $R_9$=H, $R_{11}$=$CH_3$) | H | H | $OCH_3$ | $OCH_3$ | |
| J-11 | Q-161 ($R_7$=$CH_3$, $R_8$=H, $R_9$=H, $R_{11}$=H) | H | H | $OCH_3$ | $OCH_3$ | |
| J-11 | Q-161 ($R_7$=H, $R_8$=H, $R_9$=$CH_3$, $R_{11}$=$CH_3$) | H | 5-$OCH_3$ | $OCH_3$ | $OCH_3$ | |
| J-11 | Q-162 ($R_7$=H, $R_8$=H, $R_9$=H, $R_{10}$=H) | H | H | $OCH_3$ | $OCH_3$ | |
| J-11 | Q-162 ($R_7$=H, $R_8$=H, $R_9$=H, $R_{10}$=$CH_3$) | H | 5-Cl | $OCH_3$ | $OCH_3$ | |

TABLE 3
General Formula 3

| J | $W_3$ | Q | R | $R_{1b}$ | $X_1$ | $Y_1$ |
|---|---|---|---|---|---|---|
| J-5 | S | Q-1 ($R_7$=H, $R_8$=H) | H | H | $CH_3$ | $CH_2$ |
| J-6 | S | Q-1 ($R_7$=H, $R_8$=H) | H | H | $CH_3$ | $CH_2$ |
| J-5 | S | Q-1 ($R_7$=H, $R_8$=H) | $CH_3$ | H | $OCH_3$ | $CH_2$ |
| J-6 | S | Q-1 ($R_7$=H, $R_8$=H) | $CH_3$ | H | $OC_2H_5$ | $CH_2$ |
| J-5 | S | Q-1 ($R_7$=H, $R_8$=H) | $CH_3$ | H | $OCF_2H$ | $CH_2$ |
| J-6 | S | Q-1 ($R_7$=H, $R_8$=H) | $CH_3$ | H | $CH_3$ | O |
| J-5 | S | Q-1 ($R_7$=H, $R_8$=H) | $CH_3$ | H | $OCH_3$ | O |
| J-6 | S | Q-1 ($R_7$=H, $R_8$=H) | $CH_3$ | H | $OC_2H_5$ | O |
| J-5 | S | Q-1 ($R_7$=H, $R_8$=H) | $CH_3$ | H | $OCF_2H$ | O |
| J-6 | O | Q-1 ($R_7$=H, $R_8$=H) | H | H | $CH_3$ | $CH_2$ |
| J-7 | S | Q-1 ($R_7$=H, $R_8$=H) | H | 5-$CH_3$ | $CH_3$ | $CH_2$ |
| J-5 | S | Q-1 ($R_7$=H, $R_8$=H) | H | 5-$CH_2F$ | $CH_3$ | $CH_2$ |
| J-6 | S | Q-1 ($R_7$=H, $R_8$=H) | H | 5-$CH_2CH_2Br$ | $CH_3$ | $CH_2$ |
| J-5 | S | Q-1 ($R_7$=H, $R_8$=H) | H | 5-$CH(CH_3)(CH_2Cl)$ | $CH_3$ | $CH_2$ |
| J-6 | S | Q-1 ($R_7$=H, $R_8$=H) | H | 4-$NH_2$ | $CH_3$ | $CH_2$ |
| J-5 | S | Q-1 ($R_7$=H, $R_8$=H) | H | 4-$N(CH_3)_2$ | $CH_3$ | $CH_2$ |
| J-5 | S | Q-1 ($R_7$=H, $R_8$=H) | H | 5-Cl | $CH_3$ | $CH_2$ |
| J-5 | S | Q-1 ($R_7$=H, $R_8$=H) | H | 4-F | $CH_3$ | $CH_2$ |
| J-6 | S | Q-1 ($R_7$=H, $R_8$=H) | H | 5-I | $CH_3$ | $CH_2$ |
| J-5 | S | Q-1 ($R_7$=H, $R_8$=H) | H | 4-$NO_2$ | $CH_3$ | $CH_2$ |
| J-6 | S | Q-1 ($R_7$=H, $R_8$=H) | H | 5-$CF_3$ | $CH_3$ | $CH_2$ |
| J-5 | S | Q-1 ($R_7$=H, $R_8$=H) | H | 5-$CH_3$ | $CH_3$ | O |
| J-5 | O | Q-1 ($R_7$=H, $R_8$=H) | H | 5-$CH_2F$ | $CH_3$ | O |
| J-6 | S | Q-1 ($R_7$=H, $R_8$=H) | H | 5-$CH_2CH_2Br$ | $CH_3$ | O |
| J-7 | S | Q-1 ($R_7$=H, $R_8$=H) | H | 5-$CH(CH_3)(CH_2Cl)$ | $CH_3$ | O |
| J-5 | S | Q-1 ($R_7$=H, $R_8$=H) | H | 4-$NH_2$ | $CH_3$ | O |
| J-5 | S | Q-1 ($R_7$=H, $R_8$=H) | H | 4-$N(CH_3)_2$ | $CH_3$ | O |
| J-5 | S | Q-1 ($R_7$=H, $R_8$=H) | H | 5-Cl | $CH_3$ | O |
| J-6 | S | Q-1 ($R_7$=H, $R_8$=H) | H | 4-F | $CH_3$ | O |
| J-5 | S | Q-1 ($R_7$=H, $R_8$=H) | H | 5-I | $CH_3$ | O |
| J-6 | S | Q-1 ($R_7$=H, $R_8$=H) | H | 4-$NO_2$ | $CH_3$ | O |
| J-5 | S | Q-1 ($R_7$=H, $R_8$=H) | H | 5-$CF_3$ | $CH_3$ | O |
| J-6 | S | Q-1 ($R_7$=H, $R_8$=H) | H | H | $CH_3$ | O |

Table 3 (continued)

| J | $W_3$ | Q | R | $R_{1b}$ | $X_1$ | $Y_1$ |
|---|---|---|---|---|---|---|
| J-5 | S | Q-1 ($R_7$=H, $R_8$=H) | H | H | $OCH_3$ | O |
| J-6 | O | Q-1 ($R_7$=H, $R_8$=H) | H | H | $OC_2H_5$ | O |
| J-5 | S | Q-1 ($R_7$=H, $R_8$=H) | H | H | $OCF_2H$ | O |
| J-6 | S | Q-1 ($R_7$=H, $R_8$=H) | H | H | $OCH_3$ | $CH_2$ |
| J-7 | S | Q-1 ($R_7$=H, $R_8$=H) | H | H | $OC_2H_5$ | $CH_2$ |
| J-5 | S | Q-1 ($R_7$=H, $R_8$=H) | H | H | $OCF_2H$ | $CH_2$ |
| J-6 | S | Q-1 ($R_7$=H, $R_8$=$CH_3$) | H | H | $CH_3$ | O |
| J-5 | S | Q-1 ($R_7$=H, $R_8$=$CH_3$) | H | H | $CH_3$ | $CH_2$ |
| J-6 | S | Q-1 ($R_7$=H, $R_8$=$CH_2CH_3$) | H | H | $CH_3$ | $CH_2$ |
| J-5 | NH | Q-1 ($R_7$=H, $R_8$=$CH(CH_3)_2$) | H | H | $OCH_3$ | $CH_2$ |
| J-6 | S | Q-1 ($R_7$=H, $R_8$=n-$C_4H_9$) | H | H | $OCH_3$ | O |
| J-5 | O | Q-1 ($R_7$=H, $R_8$=$CH_3$) | H | H | $OCH_3$ | O |
| J-5 | S | Q-37 ($R_7$=H, $R_8$=H, $R_2$=H) | H | H | $CH_3$ | $CH_2$ |
| J-6 | S | Q-37 ($R_7$=H, $R_8$=H, $R_2$=i-$C_3H_7$) | H | H | $CH_3$ | O |
| J-5 | S | Q-37 ($R_7$=H, $R_8$=H, $R_2$=n-$C_4H_9$) | H | 5-$CH_3$ | $OCH_3$ | $CH_2$ |
| J-6 | S | Q-98 ($R_7$=H, $R_8$=H, $R_9$=H) | H | H | $CH_3$ | $CH_2$ |
| J-7 | O | Q-98 ($R_7$=$CH_3$, $R_8$=H, $R_9$=H) | H | H | $CH_3$ | O |
| J-5 | S | Q-98 ($R_7$=H, $R_8$=H, $R_9$=i-$C_3H_7$) | H | 5-$CH_3$ | $OCH_3$ | $CH_2$ |
| J-5 | O | Q-105 ($R_7$=H, $R_8$=H, $R_9$=H, $R_2$=H) | H | H | $CH_3$ | $CH_2$ |
| J-6 | S | Q-105 ($R_7$=$CH_3$, $R_8$=H, $R_9$=H, $R_2$=$CH_3$) | H | H | $CH_3$ | O |
| J-7 | NH | Q-105 ($R_7$=H, $R_8$=$CH_3$, $R_9$=H, $R_2$=$C_2H_5$) | H | 5-$CH_3$ | $OCH_3$ | $CH_2$ |
| J-5 | S | Q-138 ($R_7$=H, $R_8$=H, $R_9$=H) | H | H | $CH_3$ | $CH_2$ |
| J-6 | O | Q-138 ($R_7$=$CH_3$, $R_8$=H, $R_9$=H) | H | H | $CH_3$ | O |
| J-7 | S | Q-138 ($R_7$=H, $R_8$=H, $R_9$=$CH_3$) | H | H | $OCH_3$ | $CH_2$ |
| J-6 | S | Q-143 ($R_7$=H, $R_8$=H, $R_9$=H, $R_3$=$CH_3$) | H | H | $CH_3$ | $CH_2$ |
| J-5 | S | Q-143 ($R_7$=H, $R_8$=H, $R_9$=H, $R_3$=H) | H | H | $CH_3$ | O |
| J-6 | S | Q-143 ($R_7$=$CH_3$, $R_8$=H, $R_9$=$CH_3$, $R_3$=$CH_3$) | H | 5-Cl | $OCH_3$ | $CH_2$ |

Table 3 (continued)

| J | Q | R | $R_{1c}$ | $X_1$ | $Y_1$ |
|---|---|---|---|---|---|
| J-8 | Q-1 ($R_7$=H, $R_8$=H) | H | H | $CH_3$ | $CH_2$ |
| J-8 | Q-1 ($R_7$=H, $R_8$=H) | H | 5-$CH_3$ | $CH_3$ | $CH_2$ |
| J-8 | Q-1 ($R_7$=H, $R_8$=H) | H | 6-$OCH_2CH_2Br$ | $CH_3$ | $CH_2$ |
| J-8 | Q-1 ($R_7$=H, $R_8$=H) | H | 5-$CH_2F$ | $CH_3$ | $CH_2$ |
| J-8 | Q-1 ($R_7$=H, $R_8$=H) | H | 5-$SCH_3$ | $CH_3$ | $CH_2$ |
| J-8 | Q-1 ($R_7$=H, $R_8$=H) | H | 5-$SCH_2F$ | $CH_3$ | $CH_2$ |
| J-8 | Q-1 ($R_7$=H, $R_8$=H) | H | 5-Cl | $CH_3$ | $CH_2$ |
| J-8 | Q-1 ($R_7$=H, $R_8$=H) | H | 4-$NO_2$ | $CH_3$ | $CH_2$ |
| J-8 | Q-1 ($R_7$=H, $R_8$=H) | H | 5-$CF_3$ | $CH_3$ | $CH_2$ |
| J-8 | Q-1 ($R_7$=H, $R_8$=H) | H | 5-$OCH_3$ | $CH_3$ | O |
| J-8 | Q-1 ($R_7$=H, $R_8$=H) | H | 5-$OCH_2F$ | $CH_3$ | O |
| J-8 | Q-1 ($R_7$=H, $R_8$=H) | H | 5-$CH_2F$ | $CH_3$ | O |
| J-8 | Q-1 ($R_7$=H, $R_8$=H) | H | 5-$SCH_3$ | $CH_3$ | O |
| J-8 | Q-1 ($R_7$=H, $R_8$=H) | H | 5-$SCH_2F$ | $CH_3$ | O |
| J-8 | Q-1 ($R_7$=H, $R_8$=H) | H | 5-$NHCH_3$ | $CH_3$ | O |
| J-8 | Q-1 ($R_7$=H, $R_8$=H) | H | 5-Cl | $CH_3$ | O |
| J-8 | Q-1 ($R_7$=H, $R_8$=H) | H | 4-$NO_2$ | $CH_3$ | O |
| J-8 | Q-1 ($R_7$=H, $R_8$=H) | H | 5-$CF_3$ | $CH_3$ | O |
| J-8 | Q-1 ($R_7$=H, $R_8$=H) | H | H | $CH_3$ | O |
| J-8 | Q-1 ($R_7$=H, $R_8$=H) | H | H | $OCH_3$ | O |
| J-8 | Q-1 ($R_7$=H, $R_8$=H) | H | H | $OCH_3$ | $CH_2$ |
| J-8 | Q-37 ($R_7$=H, $R_8$=H, $R_2$=H) | H | H | $CH_3$ | $CH_2$ |
| J-8 | Q-98 ($R_7$=H, $R_8$=H, $R_9$=H) | H | H | $CH_3$ | $CH_2$ |
| J-8 | Q-105 ($R_7$=H, $R_8$=H, $R_9$=H, $R_2$=H) | H | H | $CH_3$ | $CH_2$ |
| J-8 | Q-138 ($R_7$=H, $R_8$=H, $R_9$=H) | H | H | $CH_3$ | $CH_2$ |
| J-8 | Q-143 ($R_7$=H, $R_8$=H, $R_9$=H, $R_3$=$CH_3$) | H | H | $CH_3$ | $CH_2$ |
| J-9 | Q-1 ($R_7$=H, $R_8$=H) | H | 5-$SCH_3$ | $CH_3$ | $CH_2$ |
| J-9 | Q-1 ($R_7$=H, $R_8$=H) | H | 5-$SOCH_3$ | $CH_3$ | $CH_2$ |
| J-9 | Q-1 ($R_7$=H, $R_8$=H) | H | 5-$CO_2CH_3$ | $CH_3$ | $CH_2$ |
| J-9 | Q-1 ($R_7$=H, $R_8$=H) | H | 5-$SO_2N(CH_3)_2$ | $CH_3$ | $CH_2$ |
| J-9 | Q-1 ($R_7$=H, $R_8$=H) | H | 5-$NO_2$ | $CH_3$ | $CH_2$ |

Table 3 (continued)

| J | Q | R | $R_{1c}$ | $X_1$ | $Y_1$ |
|---|---|---|---|---|---|
| J-9 | Q-1 ($R_7$=H, $R_8$=H) | H | 5-Cl | $CH_3$ | $CH_2$ |
| J-9 | Q-1 ($R_7$=H, $R_8$=H) | H | 4-$NO_2$ | $CH_3$ | $CH_2$ |
| J-9 | Q-1 ($R_7$=H, $R_8$=H) | H | 6-Cl | $CH_3$ | $CH_2$ |
| J-9 | Q-1 ($R_7$=H, $R_8$=H) | H | 6-$COOCH_3$ | $CH_3$ | $CH_2$ |
| J-9 | Q-1 ($R_7$=H, $R_8$=H) | H | 6-$NO_2$ | $CH_3$ | $CH_2$ |
| J-9 | Q-1 ($R_7$=H, $R_8$=H) | H | 6-$SO_2CH_3$ | $CH_3$ | $CH_2$ |
| J-9 | Q-1 ($R_7$=H, $R_8$=H) | H | 6-$OCH_3$ | $CH_3$ | $CH_2$ |
| J-9 | Q-1 ($R_7$=H, $R_8$=H) | H | 5-$OCH_3$ | $CH_3$ | $CH_2$ |
| J-9 | Q-1 ($R_7$=H, $R_8$=H) | H | H | $CH_3$ | $CH_2$ |
| J-9 | Q-1 ($R_7$=H, $R_8$=H) | H | H | $CH_3$ | $CH_2$ |
| J-9 | Q-1 ($R_7$=H, $R_8$=H) | $CH_3$ | H | $OCH_3$ | $CH_2$ |
| J-9 | Q-1 ($R_7$=H, $R_8$=H) | $CH_3$ | H | $OC_2H_5$ | $CH_2$ |
| J-9 | Q-1 ($R_7$=H, $R_8$=H) | $CH_3$ | H | $OCF_2H$ | $CH_2$ |
| J-9 | Q-1 ($R_7$=H, $R_8$=H) | $CH_3$ | H | $CH_3$ | O |
| J-9 | Q-1 ($R_7$=H, $R_8$=H) | $CH_3$ | H | $OCH_3$ | O |
| J-9 | Q-1 ($R_7$=H, $R_8$=H) | $CH_3$ | H | $OC_2H_5$ | O |
| J-9 | Q-1 ($R_7$=H, $R_8$=H) | $CH_3$ | H | $OCF_2H$ | O |
| J-9 | Q-1 ($R_7$=H, $R_8$=H) | H | H | $CH_3$ | $CH_2$ |
| J-9 | Q-1 ($R_7$=H, $R_8$=H) | H | 5-$CH_3$ | $CH_3$ | $CH_2$ |
| J-9 | Q-1 ($R_7$=H, $R_8$=H) | H | 6-$CH_2CH_3$ | $CH_3$ | $CH_2$ |
| J-9 | Q-1 ($R_7$=H, $R_8$=H) | H | 5-$CH(CH_3)$ | $CH_3$ | $CH_2$ |
| J-9 | Q-1 ($R_7$=H, $R_8$=H) | H | 5-$OCH_2CH_3$ | $CH_3$ | $CH_2$ |
| J-9 | Q-1 ($R_7$=H, $R_8$=H) | H | 5-$OCH_2(CH_3)_2$ | $CH_3$ | $CH_2$ |
| J-9 | Q-1 ($R_7$=H, $R_8$=H) | H | 5-$OCH_2F$ | $CH_3$ | $CH_2$ |
| J-9 | Q-1 ($R_7$=H, $R_8$=H) | H | 6-$OCH_2CH_2Br$ | $CH_3$ | $CH_2$ |
| J-9 | Q-1 ($R_7$=H, $R_8$=H) | H | 5-$OCH(CH_3)(CH_2Cl)$ | $CH_3$ | $CH_2$ |
| J-9 | Q-1 ($R_7$=H, $R_8$=H) | H | 5-$CH_2F$ | $CH_3$ | $CH_2$ |
| J-9 | Q-1 ($R_7$=H, $R_8$=H) | H | 5-$CH_2CH_2Br$ | $CH_3$ | $CH_2$ |
| J-9 | Q-1 ($R_7$=H, $R_8$=H) | H | 5-$CH(CH_3)(CH_2Cl)$ | $CH_3$ | $CH_2$ |
| J-9 | Q-1 ($R_7$=H, $R_8$=H) | H | 6-$SCH_2CH_3$ | $CH_3$ | $CH_2$ |
| J-9 | Q-1 ($R_7$=H, $R_8$=H) | H | 5-$SCH(CH_3)_2$ | $CH_3$ | $CH_2$ |
| J-9 | Q-1 ($R_7$=H, $R_8$=H) | H | 5-$SCH_2F$ | $CH_3$ | $CH_2$ |

Table 3 (continued)

| J | Q | R | $R_{1c}$ | $X_1$ | $Y_1$ |
|---|---|---|---|---|---|
| J-9 | Q-1 ($R_7$=H, $R_8$=H) | H | 5-$SCH_2CH_2Br$ | $CH_3$ | $CH_2$ |
| J-9 | Q-1 ($R_7$=H, $R_8$=H) | H | 5-$SCH(CH_3)(CH_2Cl)$ | $CH_3$ | $CH_2$ |
| J-9 | Q-1 ($R_7$=H, $R_8$=H) | H | 4-$NH_2$ | $CH_3$ | $CH_2$ |
| J-9 | Q-1 ($R_7$=H, $R_8$=H) | H | 5-$NHCH_3$ | $CH_3$ | $CH_2$ |
| J-9 | Q-1 ($R_7$=H, $R_8$=H) | H | 6-$NHCH_2CH_3$ | $CH_3$ | $CH_2$ |
| J-9 | Q-1 ($R_7$=H, $R_8$=H) | H | 5-$NHCH(CH_3)_2$ | $CH_3$ | $CH_2$ |
| J-9 | Q-1 ($R_7$=H, $R_8$=H) | H | 4-$N(CH_3)_2$ | $CH_3$ | $CH_2$ |
| J-9 | Q-1 ($R_7$=H, $R_8$=H) | H | 6-$N(CH_3)(CH_2CH_3)$ | $CH_3$ | $CH_2$ |
| J-9 | Q-1 ($R_7$=H, $R_8$=H) | H | 5-$N(CH_3)(CH(CH_3)_2)$ | $CH_3$ | $CH_2$ |
| J-9 | Q-1 ($R_7$=H, $R_8$=H) | H | 5-Br | $CH_3$ | $CH_2$ |
| J-9 | Q-1 ($R_7$=H, $R_8$=H) | H | 4-F | $CH_3$ | $CH_2$ |
| J-9 | Q-1 ($R_7$=H, $R_8$=H) | H | 5-I | $CH_3$ | $CH_2$ |
| J-9 | Q-1 ($R_7$=H, $R_8$=H) | H | 5-$CF_3$ | $CH_3$ | $CH_2$ |
| J-9 | Q-1 ($R_7$=H, $R_8$=H) | H | 5-$OCF_2H$ | $CH_3$ | $CH_2$ |
| J-9 | Q-1 ($R_7$=H, $R_8$=H) | H | 5-$CH_3$ | $CH_3$ | O |
| J-9 | Q-1 ($R_7$=H, $R_8$=H) | H | 6-$CH_2CH_3$ | $CH_3$ | O |
| J-9 | Q-1 ($R_7$=H, $R_8$=H) | H | 5-$CH(CH_3)_2$ | $CH_3$ | O |
| J-9 | Q-1 ($R_7$=H, $R_8$=H) | H | 5-$OCH_3$ | $CH_3$ | O |
| J-9 | Q-1 ($R_7$=H, $R_8$=H) | H | 5-$OC_2H_5$ | $CH_3$ | O |
| J-9 | Q-1 ($R_7$=H, $R_8$=H) | H | 5-$OCH(CH_3)_2$ | $CH_3$ | O |
| J-9 | Q-1 ($R_7$=H, $R_8$=H) | H | 5-$OCH_2F$ | $CH_3$ | O |
| J-9 | Q-1 ($R_7$=H, $R_8$=H) | H | 6-$OCH_2CH_2Br$ | $CH_3$ | O |
| J-9 | Q-1 ($R_7$=H, $R_8$=H) | H | 5-$OCH(CH_3)(CH_2Cl)$ | $CH_3$ | O |
| J-9 | Q-1 ($R_7$=H, $R_8$=H) | H | 5-$CH_2F$ | $CH_3$ | O |
| J-9 | Q-1 ($R_7$=H, $R_8$=H) | H | 5-$CH_2CH_2Br$ | $CH_3$ | O |
| J-9 | Q-1 ($R_7$=H, $R_8$=H) | H | 5-$CH(CH_3)(CH_2Cl)$ | $CH_3$ | O |
| J-9 | Q-1 ($R_7$=H, $R_8$=H) | H | 5-$SCH_3$ | $CH_3$ | O |
| J-9 | Q-1 ($R_7$=H, $R_8$=H) | H | 6-$SCH_2CH_3$ | $CH_3$ | O |
| J-9 | Q-1 ($R_7$=H, $R_8$=H) | H | 5-$SCH(CH_3)_2$ | $CH_3$ | O |
| J-9 | Q-1 ($R_7$=H, $R_8$=H) | H | 5-$SCH_2F$ | $CH_3$ | O |
| J-9 | Q-1 ($R_7$=H, $R_8$=H) | H | 5-$SCH_2CH_2Br$ | $CH_3$ | O |
| J-9 | Q-1 ($R_7$=H, $R_8$=H) | H | 5-$SCH(CH_3)(CH_2Cl)$ | $CH_3$ | O |

Table 3 (continued)

| J | Q | R | $R_{1c}$ | $X_1$ | $Y_1$ |
|---|---|---|---|---|---|
| J-9 | Q-1 ($R_7$=H, $R_8$=H) | H | 4-$NH_2$ | $CH_3$ | O |
| J-9 | Q-1 ($R_7$=H, $R_8$=H) | H | 5-$NHCH_3$ | $CH_3$ | O |
| J-9 | Q-1 ($R_7$=H, $R_8$=H) | H | 6-OCH CH BrH | $CH_3$ | O |
| J-9 | Q-1 ($R_7$=H, $R_8$=H) | H | 5-$NHCH(CH_3)_2$ | $CH_3$ | O |
| J-9 | Q-1 ($R_7$=H, $R_8$=H) | H | 4-$N(CH_3)_2$ | $CH_3$ | O |
| J-9 | Q-1 ($R_7$=H, $R_8$=H) | H | 6-$N(CH_3)(CH_2CH_3)$ | $CH_3$ | O |
| J-9 | Q-1 ($R_7$=H, $R_8$=H) | H | 5-$N(CH_3)(CH(CH_3)_2)$ | $CH_3$ | O |
| J-9 | Q-1 ($R_7$=H, $R_8$=H) | H | 5-Cl | $CH_3$ | O |
| J-9 | Q-1 ($R_7$=H, $R_8$=H) | H | 5-Br | $CH_3$ | O |
| J-9 | Q-1 ($R_7$=H, $R_8$=H) | H | 4-F | $CH_3$ | O |
| J-9 | Q-1 ($R_7$=H, $R_8$=H) | H | 5-I | $CH_3$ | O |
| J-9 | Q-1 ($R_7$=H, $R_8$=H) | H | 4-$NO_2$ | $CH_3$ | O |
| J-9 | Q-1 ($R_7$=H, $R_8$=H) | H | 5-$CF_3$ | $CH_3$ | O |
| J-9 | Q-1 ($R_7$=H, $R_8$=H) | H | 5-$OCF_2H$ | $OCH_3$ | O |
| J-9 | Q-1 ($R_7$=H, $R_8$=H) | H | H | $CH_3$ | O |
| J-9 | Q-1 ($R_7$=H, $R_8$=H) | H | H | $OCH_3$ | O |
| J-9 | Q-1 ($R_7$=H, $R_8$=H) | H | H | $OC_2H_5$ | O |
| J-9 | Q-1 ($R_7$=H, $R_8$=H) | H | H | $OCF_2H$ | O |
| J-9 | Q-1 ($R_7$=H, $R_8$=H) | H | H | $OCH_3$ | $CH_2$ |
| J-9 | Q-1 ($R_7$=H, $R_8$=H) | H | H | $OC_2H_5$ | $CH_2$ |
| J-9 | Q-1 ($R_7$=H, $R_8$=H) | H | H | $OCF_2H$ | $CH_2$ |
| J-9 | Q-1 ($R_7$=H, $R_8$=$CH_3$) | H | H | $CH_3$ | O |
| J-9 | Q-1 ($R_7$=H, $R_8$=$CH_3$) | H | H | $CH_3$ | $CH_2$ |
| J-9 | Q-1 ($R_7$=H, $R_8$=$CH_2CH_3$) | H | H | $CH_3$ | $CH_2$ |
| J-9 | Q-1 ($R_7$=H, $R_8$=$CH(CH_3)_2$) | H | H | $OCH_3$ | $CH_2$ |
| J-9 | Q-1 ($R_7$=H, $R_8$=n-$C_4H_9$) | H | H | $OCH_3$ | O |
| J-9 | Q-1 ($R_7$=H, $R_8$=$CH_3$) | H | H | $OCH_3$ | O |
| J-9 | Q-37 ($R_7$=H, $R_8$=H, $R_2$=H) | H | H | $CH_3$ | $CH_2$ |
| J-9 | Q-98 ($R_7$=H, $R_8$=H, $R_9$=H) | H | H | $CH_3$ | $CH_2$ |
| J-9 | Q-105 ($R_7$=H, $R_8$=H, $R_9$=H, $R_2$=H) | H | H | $CH_3$ | $CH_2$ |
| J-9 | Q-138 ($R_7$=H, $R_8$=H, $R_9$=H) | H | H | $CH_3$ | $CH_2$ |
| J-9 | Q-143 ($R_7$=H, $R_8$=H, $R_9$=H, $R_3$=H) | H | H | $CH_3$ | O |

Table 3 (continued)

| J | Q | R | $R_{1d}$ | $X_1$ | $Y_1$ | m.p.(°C) |
|---|---|---|---|---|---|---|
| J-11 | Q-1 ($R_7$=H, $R_8$=H) | H | H | $CH_3$ | $CH_2$ | |
| J-11 | Q-1 ($R_7$=H, $R_8$=H) | H | H | $CH_3$ | $CH_2$ | |
| J-11 | Q-1 ($R_7$=H, $R_8$=H) | $CH_3$ | H | $OCH_3$ | $CH_2$ | |
| J-11 | Q-1 ($R_7$=H, $R_8$=H) | $CH_3$ | H | $OC_2H_5$ | $CH_2$ | |
| J-11 | Q-1 ($R_7$=H, $R_8$=H) | $CH_3$ | H | $OCF_2H$ | $CH_2$ | |
| J-11 | Q-1 ($R_7$=H, $R_8$=H) | $CH_3$ | H | $CH_3$ | O | |
| J-11 | Q-1 ($R_7$=H, $R_8$=H) | $CH_3$ | H | $OCH_3$ | O | |
| J-11 | Q-1 ($R_7$=H, $R_8$=H) | $CH_3$ | H | $OC_2H_5$ | O | |
| J-11 | Q-1 ($R_7$=H, $R_8$=H) | $CH_3$ | H | $OCF_2H$ | O | |
| J-11 | Q-1 ($R_7$=H, $R_8$=H) | H | H | $CH_3$ | $CH_2$ | |
| J-11 | Q-1 ($R_7$=H, $R_8$=H) | H | 5-$CH_3$ | $CH_3$ | $CH_2$ | |
| J-11 | Q-1 ($R_7$=H, $R_8$=H) | H | 6-$CH_2CH_3$ | $CH_3$ | $CH_2$ | |
| J-11 | Q-1 ($R_7$=H, $R_8$=H) | H | 3-$CH(CH_3)_2$ | $CH_3$ | $CH_2$ | |
| J-11 | Q-1 ($R_7$=H, $R_8$=H) | H | 5-$OCH_3$ | $CH_3$ | $CH_2$ | |
| J-11 | Q-1 ($R_7$=H, $R_8$=H) | H | 5-$OCH_2CH_3$ | $CH_3$ | $CH_2$ | |
| J-11 | Q-1 ($R_7$=H, $R_8$=H) | H | 3-$OCH_2(CH_3)_2$ | $CH_3$ | $CH_2$ | |
| J-11 | Q-1 ($R_7$=H, $R_8$=H) | H | 5-$OCH_2F$ | $CH_3$ | $CH_2$ | |
| J-11 | Q-1 ($R_7$=H, $R_8$=H) | H | 6-$OCH_2CH_2Br$ | $CH_3$ | $CH_2$ | |
| J-11 | Q-1 ($R_7$=H, $R_8$=H) | H | 5-$OCH(CH_3)(CH_2Cl)$ | $CH_3$ | $CH_2$ | |
| J-11 | Q-1 ($R_7$=H, $R_8$=H) | H | 5-$CH_2F$ | $CH_3$ | $CH_2$ | |
| J-11 | Q-1 ($R_7$=H, $R_8$=H) | H | 5-$CH_2CH_2Br$ | $CH_3$ | $CH_2$ | |
| J-11 | Q-1 ($R_7$=H, $R_8$=H) | H | 5-$CH(CH_3)(CH_2Cl)$ | $CH_3$ | $CH_2$ | |
| J-11 | Q-1 ($R_7$=H, $R_8$=H) | H | 5-$SCH_3$ | $CH_3$ | $CH_2$ | |
| J-11 | Q-1 ($R_7$=H, $R_8$=H) | H | 6-$SCH_2CH_3$ | $CH_3$ | $CH_2$ | |
| J-11 | Q-1 ($R_7$=H, $R_8$=H) | H | 5-$SCH(CH_3)_2$ | $CH_3$ | $CH_2$ | |
| J-11 | Q-1 ($R_7$=H, $R_8$=H) | H | 5-$SCH_2F$ | $CH_3$ | $CH_2$ | |
| J-11 | Q-1 ($R_7$=H, $R_8$=H) | H | 5-$SCH_2CH_2Br$ | $CH_3$ | $CH_2$ | |
| J-11 | Q-1 ($R_7$=H, $R_8$=H) | H | 5-$SCH(CH_3)(CH_2Cl)$ | $CH_3$ | $CH_2$ | |
| J-11 | Q-1 ($R_7$=H, $R_8$=H) | H | 4-$NH_2$ | $CH_3$ | $CH_2$ | |
| J-11 | Q-1 ($R_7$=H, $R_8$=H) | H | 5-$NHCH_3$ | $CH_3$ | $CH_2$ | |

Table 3 (continued)

| J | Q | R | $R_{1d}$ | $X_1$ | $Y_1$ | m.p. (°C) |
|---|---|---|---|---|---|---|
| J-11 | Q-1 ($R_7$=H, $R_8$=H) | H | 6-$NHCH_2CH_3$ | $CH_3$ | $CH_2$ | |
| J-11 | Q-1 ($R_7$=H, $R_8$=H) | H | 3-$NHCH(CH_3)_2$ | $CH_3$ | $CH_2$ | |
| J-11 | Q-1 ($R_7$=H, $R_8$=H) | H | 4-$N(CH_3)_2$ | $CH_3$ | $CH_2$ | |
| J-11 | Q-1 ($R_7$=H, $R_8$=H) | H | 6-$N(CH_3)(CH_2CH_3)$ | $CH_3$ | $CH_2$ | |
| J-11 | Q-1 ($R_7$=H, $R_8$=H) | H | 5-$N(CH_3)(CH(CH_3)_2)$ | $CH_3$ | $CH_2$ | |
| J-11 | Q-1 ($R_7$=H, $R_8$=H) | H | 5-Cl | $CH_3$ | $CH_2$ | |
| J-11 | Q-1 ($R_7$=H, $R_8$=H) | H | 3-Br | $CH_3$ | $CH_2$ | |
| J-11 | Q-1 ($R_7$=H, $R_8$=H) | H | 4-F | $CH_3$ | $CH_2$ | |
| J-11 | Q-1 ($R_7$=H, $R_8$=H) | H | 5-I | $CH_3$ | $CH_2$ | |
| J-11 | Q-1 ($R_7$=H, $R_8$=H) | H | 4-$NO_2$ | $CH_3$ | $CH_2$ | |
| J-11 | Q-1 ($R_7$=H, $R_8$=H) | H | 5-$CF_3$ | $CH_3$ | $CH_2$ | |
| J-11 | Q-1 ($R_7$=H, $R_8$=H) | H | 5-$OCF_2H$ | $CH_3$ | $CH_2$ | |
| J-11 | Q-1 ($R_7$=H, $R_8$=H) | H | 5-$CH_3$ | $CH_3$ | O | |
| J-11 | Q-1 ($R_7$=H, $R_8$=H) | H | 6-$CH_2CH_3$ | $CH_3$ | O | |
| J-11 | Q-1 ($R_7$=H, $R_8$=H) | H | 3-$CH(CH_3)_2$ | $CH_3$ | O | |
| J-11 | Q-1 ($R_7$=H, $R_8$=H) | H | 5-$OCH_3$ | $CH_3$ | O | |
| J-11 | Q-1 ($R_7$=H, $R_8$=H) | H | 5-$OC_2H_5$ | $CH_3$ | O | |
| J-11 | Q-1 ($R_7$=H, $R_8$=H) | H | 3-$OCH(CH_3)_2$ | $CH_3$ | O | |
| J-11 | Q-1 ($R_7$=H, $R_8$=H) | H | 5-$OCH_2F$ | $CH_3$ | O | |
| J-11 | Q-1 ($R_7$=H, $R_8$=H) | H | 6-$OCH_2CH_2Br$ | $CH_3$ | O | |
| J-11 | Q-1 ($R_7$=H, $R_8$=H) | H | 5-$OCH(CH_3)(CH_2Cl)$ | $CH_3$ | O | |
| J-11 | Q-1 ($R_7$=H, $R_8$=H) | H | 5-$CH_2F$ | $CH_3$ | O | |
| J-11 | Q-1 ($R_7$=H, $R_8$=H) | H | 5-$CH_2CH_2Br$ | $CH_3$ | O | |
| J-11 | Q-1 ($R_7$=H, $R_8$=H) | H | 5-$CH(CH_3)(CH_2Cl)$ | $CH_3$ | O | |
| J-11 | Q-1 ($R_7$=H, $R_8$=H) | H | 5-$SCH_3$ | $CH_3$ | O | |
| J-11 | Q-1 ($R_7$=H, $R_8$=H) | H | 6-$SCH_2CH_3$ | $CH_3$ | O | |
| J-11 | Q-1 ($R_7$=H, $R_8$=H) | H | 5-$SCH(CH_3)_2$ | $CH_3$ | O | |
| J-11 | Q-1 ($R_7$=H, $R_8$=H) | H | 5-$SCH_2F$ | $CH_3$ | O | |
| J-11 | Q-1 ($R_7$=H, $R_8$=H) | H | 5-$SCH_2CH_2Br$ | $CH_3$ | O | |
| J-11 | Q-1 ($R_7$=H, $R_8$=H) | H | 5-$SCH(CH_3)(CH_2Cl)$ | $CH_3$ | O | |
| J-11 | Q-1 ($R_7$=H, $R_8$=H) | H | 4-$NH_2$ | $CH_3$ | O | |
| J-11 | Q-1 ($R_7$=H, $R_8$=H) | H | 5-$NHCH_3$ | $CH_3$ | O | |

Table 3 (continued)

| J | Q | R | $R_{1d}$ | $X_1$ | $Y_1$ | m.p. (°C) |
|---|---|---|---|---|---|---|
| J-11 | Q-1 ($R_7$=H, $R_8$=H) | H | 6-$OCH_2CH_2Br$ | $CH_3$ | O | |
| J-11 | Q-1 ($R_7$=H, $R_8$=H) | H | 3-$NHCH(CH_3)_2$ | $CH_3$ | O | |
| J-11 | Q-1 ($R_7$=H, $R_8$=H) | H | 4-$N(CH_3)_2$ | $CH_3$ | O | |
| J-11 | Q-1 ($R_7$=H, $R_8$=H) | H | 6-$N(CH_3)(CH_2CH_3)$ | $CH_3$ | O | |
| J-11 | Q-1 ($R_7$=H, $R_8$=H) | H | 5-$N(CH_3)(CH(CH_3)_2)$ | $CH_3$ | O | |
| J-11 | Q-1 ($R_7$=H, $R_8$=H) | H | 5-Cl | $CH_3$ | O | |
| J-11 | Q-1 ($R_7$=H, $R_8$=H) | H | 3-Br | $CH_3$ | O | |
| J-11 | Q-1 ($R_7$=H, $R_8$=H) | H | 4-F | $CH_3$ | O | |
| J-11 | Q-1 ($R_7$=H, $R_8$=H) | H | 5-I | $CH_3$ | O | |
| J-11 | Q-1 ($R_7$=H, $R_8$=H) | H | 4-$NO_2$ | $CH_3$ | O | |
| J-11 | Q-1 ($R_7$=H, $R_8$=H) | H | 5-$CF_3$ | $CH_3$ | O | |
| J-11 | Q-1 ($R_7$=H, $R_8$=H) | H | 5-$OCF_2H$ | $CH_3$ | O | |
| J-11 | Q-1 ($R_7$=H, $R_8$=H) | H | 6-$SO_2N(CH_3)_2$ | $OCH_3$ | O | |
| J-11 | Q-1 ($R_7$=H, $R_8$=H) | H | 6-$SOCH_3$ | $OCH_3$ | O | |
| J-11 | Q-1 ($R_7$=H, $R_8$=H) | H | 6-$SO_2CH(CH_3)_2$ | $OCH_3$ | O | |
| J-11 | Q-1 ($R_7$=H, $R_8$=H) | H | 6-$CO_2CH_3$ | $OCH_3$ | O | |
| J-11 | Q-1 ($R_7$=H, $R_8$=H) | H | H | $CH_3$ | O | |
| J-11 | Q-1 ($R_7$=H, $R_8$=H) | H | H | $OCH_3$ | O | |
| J-11 | Q-1 ($R_7$=H, $R_8$=H) | H | H | $OC_2H_5$ | O | |
| J-11 | Q-1 ($R_7$=H, $R_8$=H) | H | H | $OCF_2H$ | O | |
| J-11 | Q-1 ($R_7$=H, $R_8$=H) | H | H | $OCH_3$ | $CH_2$ | |
| J-11 | Q-1 ($R_7$=H, $R_8$=H) | H | H | $OC_2H_5$ | $CH_2$ | |
| J-11 | Q-1 ($R_7$=H, $R_8$=H) | H | H | $OCF_2H$ | $CH_2$ | |
| J-11 | Q-1 ($R_7$=H, $R_8$=$CH_3$) | H | H | $CH_3$ | O | |
| J-11 | Q-1 ($R_7$=H, $R_8$=$CH_3$) | H | H | $CH_3$ | $CH_2$ | |
| J-11 | Q-1 ($R_7$=H, $R_8$=$CH_2CH_3$) | H | H | $CH_3$ | $CH_2$ | |
| J-11 | Q-1 ($R_7$=H, $R_8$=$CH(CH_3)_2$) | H | H | $OCH_3$ | $CH_2$ | |
| J-11 | Q-1 ($R_7$=H, $R_8$=n-$C_4H_9$) | H | H | $OCH_3$ | O | |
| J-11 | Q-1 ($R_7$=H, $R_8$=$CH_3$) | H | H | $OCH_3$ | O | |
| J-11 | Q-37 ($R_7$=H, $R_8$=H, $R_2$=H) | H | H | $CH_3$ | $CH_2$ | |
| J-11 | Q-37 ($R_7$=H, $R_8$=H, $R_2$=i-$C_3H_7$) | H | H | $CH_3$ | O | |
| J-11 | Q-37 ($R_7$=H, $R_8$=H, $R_2$=n-$C_4H_9$) | H | 5-$OCH_3$ | $OCH_3$ | $CH_2$ | |

Table 3 (continued)

| J | Q | R | $R_{1d}$ | $X_1$ | $Y_1$ | m.p. (°C) |
|---|---|---|---|---|---|---|
| J-11 | Q-98 ($R_7$=H, $R_8$=H, $R_9$=H) | H | H | $CH_3$ | $CH_2$ | |
| J-11 | Q-98 ($R_7$=$CH_3$, $R_8$=H, $R_9$=H) | H | H | $CH_3$ | O | |
| J-11 | Q-98 ($R_7$=H, $R_8$=H, $R_9$=i-$C_3H_7$) | H | 5-$OCH_3$ | $OCH_3$ | $CH_2$ | |
| J-11 | Q-105 ($R_7$=H, $R_8$=H, $R_9$=H, $R_2$=H) | H | H | $CH_3$ | $CH_2$ | |
| J-11 | Q-105 ($R_7$=$CH_3$, $R_8$=H, $R_9$=H, $R_2$=$CH_3$) | H | H | $CH_3$ | O | |
| J-11 | Q-105 ($R_7$=H, $R_8$=$CH_3$, $R_9$=H, $R_2$=$C_2H_5$) | H | 5-$OCH_3$ | $OCH_3$ | $CH_2$ | |
| J-11 | Q-138 ($R_7$=H, $R_8$=H, $R_9$=H) | H | H | $CH_3$ | $CH_2$ | |
| J-11 | Q-138 ($R_7$=$CH_3$, $R_8$=H, $R_9$=H) | H | H | $CH_3$ | O | |
| J-11 | Q-138 ($R_7$=H, $R_8$=H, $R_9$=$CH_3$) | H | H | $OCH_3$ | $CH_2$ | |
| J-11 | Q-143 ($R_7$=H, $R_8$=H, $R_9$=H, $R_3$=$CH_3$) | H | H | $CH_3$ | $CH_2$ | |
| J-11 | Q-143 ($R_7$=H, $R_8$=H, $R_9$=H, $R_3$=H) | H | H | $CH_3$ | O | |
| J-11 | Q-143 ($R_7$=$CH_3$, $R_8$=H, $R_9$=$CH_3$, $R_3$=$CH_3$) | H | 5-Cl | $OCH_3$ | $CH_2$ | |

TABLE 4
General Formula 4

| J | $W_3$ | Q | R | $R_{1b}$ | $X_1$ |
|---|---|---|---|---|---|
| J-5 | S | Q-1 ($R_7$=H, $R_8$=H) | H | H | $CH_3$ |
| J-6 | S | Q-1 ($R_7$=H, $R_8$=H) | H | H | $CH_3$ |
| J-5 | S | Q-1 ($R_7$=H, $R_8$=H) | $CH_3$ | H | $OCH_3$ |
| J-6 | S | Q-1 ($R_7$=H, $R_8$=H) | $CH_3$ | H | $OC_2H_5$ |
| J-5 | S | Q-1 ($R_7$=H, $R_8$=H) | $CH_3$ | H | $OCF_2H$ |
| J-6 | S | Q-1 ($R_7$=H, $R_8$=H) | H | 5-$CH_3$ | $CH_3$ |
| J-5 | S | Q-1 ($R_7$=H, $R_8$=H) | H | 5-$CH_2F$ | $CH_3$ |
| J-6 | S | Q-1 ($R_7$=H, $R_8$=H) | H | 5-$CH_2CH_2Br$ | $CH_3$ |
| J-7 | S | Q-1 ($R_7$=H, $R_8$=H) | H | 5-$CH(CH_3)(CH_2Cl)$ | $CH_3$ |
| J-5 | O | Q-1 ($R_7$=H, $R_8$=H) | H | 4-$NH_2$ | $CH_3$ |
| J-6 | S | Q-1 ($R_7$=H, $R_8$=H) | H | 4-$N(CH_3)_2$ | $CH_3$ |
| J-5 | NH | Q-1 ($R_7$=H, $R_8$=H) | H | 5-Cl | $CH_3$ |
| J-6 | S | Q-1 ($R_7$=H, $R_8$=H) | H | 4-F | $CH_3$ |
| J-5 | S | Q-1 ($R_7$=H, $R_8$=H) | H | 5-I | $CH_3$ |
| J-6 | S | Q-1 ($R_7$=H, $R_8$=H) | H | 4-$NO_2$ | $CH_3$ |
| J-5 | S | Q-1 ($R_7$=H, $R_8$=H) | H | 5-$CF_3$ | $CH_3$ |
| J-6 | S | Q-1 ($R_7$=H, $R_8$=H) | H | H | $OCH_3$ |
| J-5 | S | Q-1 ($R_7$=H, $R_8$=H) | H | H | $OC_2H_5$ |
| J-6 | S | Q-1 ($R_7$=H, $R_8$=H) | H | H | $OCF_2H$ |
| J-7 | O | Q-1 ($R_7$=H, $R_8$=$CH_3$) | H | H | $CH_3$ |
| J-5 | S | Q-1 ($R_7$=H, $R_8$=$CH_2CH_3$) | H | H | $CH_3$ |
| J-6 | S | Q-1 ($R_7$=H, $R_8$=$CH(CH_3)_2$) | H | H | $OCH_3$ |
| J-5 | S | Q-1 ($R_7$=H, $R_8$=$\underline{n}$-$C_4H_9$) | H | H | $OCH_3$ |
| J-6 | S | Q-1 ($R_7$=H, $R_8$=$CH_3$) | H | H | $OCH_3$ |
| J-7 | O | Q-37 ($R_7$=H, $R_8$=H, $R_2$=H) | H | H | $CH_3$ |
| J-5 | S | Q-37 ($R_7$=H, $R_8$=H, $R_2$=$\underline{i}$-$C_3H_7$) | H | H | $CH_3$ |
| J-6 | S | Q-37 ($R_7$=H, $R_8$=H, $R_2$=$\underline{n}$-$C_4H_9$) | H | 5-$CH_3$ | $OCH_3$ |
| J-5 | NH | Q-98 ($R_7$=H, $R_8$=H, $R_9$=H) | H | H | $CH_3$ |
| J-6 | S | Q-98 ($R_7$=$CH_3$, $R_8$=H, $R_9$=H) | H | H | $CH_3$ |
| J-5 | S | Q-98 ($R_7$=H, $R_8$=H, $R_9$=$\underline{i}$-$C_3H_7$) | H | 5-$CH_3$ | $OCH_3$ |
| J-6 | S | Q-105 ($R_7$=H, $R_8$=H, $R_9$=H, $R_2$=H) | H | H | $CH_3$ |
| J-5 | S | Q-105 ($R_7$=$CH_3$, $R_8$=H, $R_9$=H, $R_2$=$CH_3$) | H | H | $CH_3$ |

Table 4 (continued)

| J | $W_3$ | Q | R | $R_{1b}$ | $X_1$ |
|---|---|---|---|---|---|
| J-6 | S | Q-105 ($R_7$=H, $R_8$=$CH_3$, $R_9$=H, $R_2$=$C_2H_5$) | H | 5-$CH_3$ | $OCH_3$ |
| J-6 | S | Q-138 ($R_7$=H, $R_8$=H, $R_9$=H) | H | H | $CH_3$ |
| J-5 | S | Q-138 ($R_7$=$CH_3$, $R_8$=H, $R_9$=H) | H | H | $CH_3$ |
| J-6 | S | Q-138 ($R_7$=H, $R_8$=H, $R_9$=$CH_3$) | H | H | $OCH_3$ |
| J-6 | S | Q-143 ($R_7$=H, $R_8$=H, $R_9$=H, $R_3$=$CH_3$) | H | H | $CH_3$ |
| J-7 | S | Q-143 ($R_7$=H, $R_8$=H, $R_9$=H, $R_3$=H) | H | H | $CH_3$ |
| J-5 | O | Q-143 ($R_7$=$CH_3$, $R_8$=H, $R_9$=$CH_3$, $R_3$=$CH_3$) | H | 5-Cl | $OCH_3$ |

Table 4 (continued)

| J | Q | R | $R_{1c}$ | $X_1$ |
|---|---|---|---|---|
| J-8 | Q-1 ($R_7$=H, $R_8$=H) | H | H | $CH_3$ |
| J-8 | Q-1 ($R_7$=H, $R_8$=H) | H | H | $CH_3$ |
| J-8 | Q-1 ($R_7$=H, $R_8$=H) | H | 5-$CH_3$ | $CH_3$ |
| J-8 | Q-1 ($R_7$=H, $R_8$=H) | H | 5-$OCH_3$ | $CH_3$ |
| J-8 | Q-1 ($R_7$=H, $R_8$=H) | H | 6-$OCH_2CH_2Br$ | $CH_3$ |
| J-8 | Q-1 ($R_7$=H, $R_8$=H) | H | 5-$CH_2F$ | $CH_3$ |
| J-8 | Q-1 ($R_7$=H, $R_8$=H) | H | 5-$SCH_3$ | $CH_3$ |
| J-8 | Q-1 ($R_7$=H, $R_8$=H) | H | 5-$SCH_2F$ | $CH_3$ |
| J-8 | Q-1 ($R_7$=H, $R_8$=H) | H | 5-$NHCH_3$ | $CH_3$ |
| J-8 | Q-1 ($R_7$=H, $R_8$=H) | H | 5-Cl | $CH_3$ |
| J-8 | Q-1 ($R_7$=H, $R_8$=H) | H | 4-$NO_2$ | $CH_3$ |
| J-8 | Q-1 ($R_7$=H, $R_8$=H) | H | 5-$CF_3$ | $CH_3$ |
| J-8 | Q-1 ($R_7$=H, $R_8$=H) | H | H | $CH_3$ |
| J-8 | Q-37 ($R_7$=H, $R_8$=H, $R_2$=H) | H | H | $CH_3$ |
| J-8 | Q-98 ($R_7$=H, $R_8$=H, $R_9$=H) | H | H | $CH_3$ |
| J-8 | Q-105 ($R_7$=H, $R_8$=H, $R_9$=H, $R_2$=H) | H | H | $CH_3$ |
| J-8 | Q-138 ($R_7$=H, $R_8$=H, $R_9$=H) | H | H | $CH_3$ |
| J-8 | Q-143 ($R_7$=H, $R_8$=H, $R_9$=H, $R_3$=$CH_3$) | H | H | $CH_3$ |
| J-9 | Q-1 ($R_7$=H, $R_8$=H) | H | 5-$SO_2N(CH_3)_2$ | $CH_3$ |
| J-9 | Q-1 ($R_7$=H, $R_8$=H) | H | 5-$NO_2$ | $CH_3$ |
| J-9 | Q-1 ($R_7$=H, $R_8$=H) | H | 5-Cl | $CH_3$ |
| J-9 | Q-1 ($R_7$=H, $R_8$=H) | H | 4-$NO_2$ | $CH_3$ |
| J-9 | Q-1 ($R_7$=H, $R_8$=H) | H | 6-Cl | $CH_3$ |
| J-9 | Q-1 ($R_7$=H, $R_8$=H) | H | 6-$COOCH_3$ | $CH_3$ |
| J-9 | Q-1 ($R_7$=H, $R_8$=H) | H | 6-$NO_2$ | $CH_3$ |
| J-9 | Q-1 ($R_7$=H, $R_8$=H) | H | 6-$SO_2CH_3$ | $CH_3$ |
| J-9 | Q-1 ($R_7$=H, $R_8$=H) | H | 6-$OCH_3$ | $CH_3$ |
| J-9 | Q-1 ($R_7$=H, $R_8$=H) | H | 5-$OCH_3$ | $CH_3$ |
| J-9 | Q-1 ($R_7$=H, $R_8$=H) | H | H | $CH_3$ |
| J-9 | Q-1 ($R_7$=H, $R_8$=H) | H | H | $CH_3$ |

Table 4 (continued)

| J | Q | R | $R_{1c}$ | $X_1$ |
|---|---|---|---|---|
| J-9 | Q-1 ($R_7$=H, $R_8$=H) | $CH_3$ | H | $OCH_3$ |
| J-9 | Q-1 ($R_7$=H, $R_8$=H) | $CH_3$ | H | $OC_2H_5$ |
| J-9 | Q-1 ($R_7$=H, $R_8$=H) | $CH_3$ | H | $OCF_2H$ |
| J-9 | Q-1 ($R_7$=H, $R_8$=H) | H | 5-$CH_3$ | $CH_3$ |
| J-9 | Q-1 ($R_7$=H, $R_8$=H) | H | 6-$CH_2CH_3$ | $CH_3$ |
| J-9 | Q-1 ($R_7$=H, $R_8$=H) | H | 4-$CH(CH_3)_2$ | $CH_3$ |
| J-9 | Q-1 ($R_7$=H, $R_8$=H) | H | 5-$OCH_2CH_3$ | $CH_3$ |
| J-9 | Q-1 ($R_7$=H, $R_8$=H) | H | 4-$OCH_2(CH_3)_2$ | $CH_3$ |
| J-9 | Q-1 ($R_7$=H, $R_8$=H) | H | 5-$OCH_2F$ | $CH_3$ |
| J-9 | Q-1 ($R_7$=H, $R_8$=H) | H | 6-$OCH_2CH_2Br$ | $CH_3$ |
| J-9 | Q-1 ($R_7$=H, $R_8$=H) | H | 5-$OCH(CH_3)(CH_2Cl)$ | $CH_3$ |
| J-9 | Q-1 ($R_7$=H, $R_8$=H) | H | 5-$CH_2F$ | $CH_3$ |
| J-9 | Q-1 ($R_7$=H, $R_8$=H) | H | 5-$CH_2CH_2Br$ | $CH_3$ |
| J-9 | Q-1 ($R_7$=H, $R_8$=H) | H | 5-$CH(CH_3)(CH_2Cl)$ | $CH_3$ |
| J-9 | Q-1 ($R_7$=H, $R_8$=H) | H | 6-$SCH_2CH_3$ | $CH_3$ |
| J-9 | Q-1 ($R_7$=H, $R_8$=H) | H | 5-$SCH(CH_3)_2$ | $CH_3$ |
| J-9 | Q-1 ($R_7$=H, $R_8$=H) | H | 5-$SCH_2F$ | $CH_3$ |
| J-9 | Q-1 ($R_7$=H, $R_8$=H) | H | 5-$SCH_2CH_2Br$ | $CH_3$ |
| J-9 | Q-1 ($R_7$=H, $R_8$=H) | H | 5-$SCH(CH_3)(CH_2Cl)$ | $CH_3$ |
| J-9 | Q-1 ($R_7$=H, $R_8$=H) | H | 4-$NH_2$ | $CH_3$ |
| J-9 | Q-1 ($R_7$=H, $R_8$=H) | H | 5-$NHCH_3$ | $CH_3$ |
| J-9 | Q-1 ($R_7$=H, $R_8$=H) | H | 6-$NHCH_2CH_3$ | $CH_3$ |
| J-9 | Q-1 ($R_7$=H, $R_8$=H) | H | 5-$NHCH(CH_3)_2$ | $CH_3$ |
| J-9 | Q-1 ($R_7$=H, $R_8$=H) | H | 4-$N(CH_3)_2$ | $CH_3$ |
| J-9 | Q-1 ($R_7$=H, $R_8$=H) | H | 6-$N(CH_3)(CH_2CH_3)$ | $CH_3$ |
| J-9 | Q-1 ($R_7$=H, $R_8$=H) | H | 5-$N(CH_3)(CH(CH_3)_2)$ | $CH_3$ |
| J-9 | Q-1 ($R_7$=H, $R_8$=H) | H | 5-Br | $CH_3$ |
| J-9 | Q-1 ($R_7$=H, $R_8$=H) | H | 4-F | $CH_3$ |
| J-9 | Q-1 ($R_7$=H, $R_8$=H) | H | 5-I | $CH_3$ |
| J-9 | Q-1 ($R_7$=H, $R_8$=H) | H | 5-$CF_3$ | $CH_3$ |
| J-9 | Q-1 ($R_7$=H, $R_8$=H) | H | 5-$OCF_2H$ | $CH_3$ |
| J-9 | Q-1 ($R_7$=H, $R_8$=H) | H | H | $OCH_3$ |
| J-9 | Q-1 ($R_7$=H, $R_8$=H) | H | H | $OC_2H_5$ |

Table 4 (continued)

| J | Q | R | $R_{1c}$ | $X_1$ |
|---|---|---|---|---|
| J-9 | Q-1 ($R_7$=H, $R_8$=H) | H | H | $OCF_2H$ |
| J-9 | Q-1 ($R_7$=H, $R_8$=$CH_3$) | H | H | $CH_3$ |
| J-9 | Q-1 ($R_7$=H, $R_8$=$CH_2CH_3$) | H | H | $CH_3$ |
| J-9 | Q-1 ($R_7$=H, $R_8$=$CH(CH_3)_2$) | H | H | $OCH_3$ |
| J-9 | Q-1 ($R_7$=H, $R_8$=n-$C_4H_9$) | H | H | $OCH_3$ |
| J-9 | Q-1 ($R_7$=H, $R_8$=$CH_3$) | H | H | $OCH_3$ |
| J-9 | Q-37 ($R_7$=H, $R_8$=H, $R_2$=H) | H | H | $CH_3$ |
| J-9 | Q-98 ($R_7$=H, $R_8$=H, $R_9$=H) | H | H | $CH_3$ |
| J-9 | Q-105 ($R_7$=H, $R_8$=H, $R_9$=H, $R_2$=H) | H | H | $CH_3$ |
| J-9 | Q-138 ($R_7$=H, $R_8$=H, $R_9$=H) | H | H | $CH_3$ |
| J-9 | Q-143 ($R_7$=H, $R_8$=H, $R_9$=H, $R_3$=$CH_3$) | H | H | $CH_3$ |

Table 4 (continued)

| J | Q | R | $R_{1d}$ | $X_1$ | m.p.(°C) |
|---|---|---|---|---|---|
| J-11 | Q-1 ($R_7$=H, $R_8$=H) | H | H | $CH_3$ | |
| J-11 | Q-1 ($R_7$=H, $R_8$=H) | $CH_3$ | H | $OCH_3$ | |
| J-11 | Q-1 ($R_7$=H, $R_8$=H) | $CH_3$ | H | $OC_2H_5$ | |
| J-11 | Q-1 ($R_7$=H, $R_8$=H) | $CH_3$ | H | $OCF_2H$ | |
| J-11 | Q-1 ($R_7$=H, $R_8$=H) | H | 5-$CH_3$ | $CH_3$ | |
| J-11 | Q-1 ($R_7$=H, $R_8$=H) | H | 6-$CH_2CH_3$ | $CH_3$ | |
| J-11 | Q-1 ($R_7$=H, $R_8$=H) | H | 3-$CH(CH_3)_2$ | $CH_3$ | |
| J-11 | Q-1 ($R_7$=H, $R_8$=H) | H | 5-$OCH_3$ | $CH_3$ | |
| J-11 | Q-1 ($R_7$=H, $R_8$=H) | H | 5-$OCH_2CH_3$ | $CH_3$ | |
| J-11 | Q-1 ($R_7$=H, $R_8$=H) | H | 3-$OCH_2(CH_3)_2$ | $CH_3$ | |
| J-11 | Q-1 ($R_7$=H, $R_8$=H) | H | 5-$OCH_2F$ | $CH_3$ | |
| J-11 | Q-1 ($R_7$=H, $R_8$=H) | H | 6-$OCH_2CH_2Br$ | $CH_3$ | |
| J-11 | Q-1 ($R_7$=H, $R_8$=H) | H | 5-$OCH(CH_3)(CH_2Cl)$ | $CH_3$ | |
| J-11 | Q-1 ($R_7$=H, $R_8$=H) | H | 5-$CH_2F$ | $CH_3$ | |
| J-11 | Q-1 ($R_7$=H, $R_8$=H) | H | 5-$CH_2CH_2Br$ | $CH_3$ | |
| J-11 | Q-1 ($R_7$=H, $R_8$=H) | H | 5-$CH(CH_3)(CH_2Cl)$ | $CH_3$ | |
| J-11 | Q-1 ($R_7$=H, $R_8$=H) | H | 5-$SCH_3$ | $CH_3$ | |
| J-11 | Q-1 ($R_7$=H, $R_8$=H) | H | 6-$SCH_2CH_3$ | $CH_3$ | |
| J-11 | Q-1 ($R_7$=H, $R_8$=H) | H | 5-$SCH(CH_3)_2$ | $CH_3$ | |
| J-11 | Q-1 ($R_7$=H, $R_8$=H) | H | 5-$SCH_2F$ | $CH_3$ | |
| J-11 | Q-1 ($R_7$=H, $R_8$=H) | H | 5-$SCH_2CH_2Br$ | $CH_3$ | |
| J-11 | Q-1 ($R_7$=H, $R_8$=H) | H | 5-$SCH(CH_3)(CH_2Cl)$ | $CH_3$ | |
| J-11 | Q-1 ($R_7$=H, $R_8$=H) | H | 4-$NH_2$ | $CH_3$ | |
| J-11 | Q-1 ($R_7$=H, $R_8$=H) | H | 5-$NHCH_3$ | $CH_3$ | |
| J-11 | Q-1 ($R_7$=H, $R_8$=H) | H | 6-$NHCH_2CH_3$ | $CH_3$ | |
| J-11 | Q-1 ($R_7$=H, $R_8$=H) | H | 3-$NHCH(CH_3)_2$ | $CH_3$ | |
| J-11 | Q-1 ($R_7$=H, $R_8$=H) | H | 4-$N(CH_3)_2$ | $CH_3$ | |
| J-11 | Q-1 ($R_7$=H, $R_8$=H) | H | 6-$N(CH_3)(CH_2CH_3)$ | $CH_3$ | |
| J-11 | Q-1 ($R_7$=H, $R_8$=H) | H | 5-$N(CH_3)(CH(CH_3)_2)$ | $CH_3$ | |
| J-11 | Q-1 ($R_7$=H, $R_8$=H) | H | 5-Cl | $CH_3$ | |
| J-11 | Q-1 ($R_7$=H, $R_8$=H) | H | 3-Br | $CH_3$ | |
| J-11 | Q-1 ($R_7$=H, $R_8$=H) | H | 4-F | $CH_3$ | |

Table 4 (continued)

| J | Q | R | $R_{1d}$ | $X_1$ | m.p. (°C) |
|---|---|---|---|---|---|
| J-11 | Q-1 ($R_7$=H, $R_8$=H) | H | 5-I | $CH_3$ | |
| J-11 | Q-1 ($R_7$=H, $R_8$=H) | H | 4-$NO_2$ | $CH_3$ | |
| J-11 | Q-1 ($R_7$=H, $R_8$=H) | H | 5-$CF_3$ | $CH_3$ | |
| J-11 | Q-1 ($R_7$=H, $R_8$=H) | H | 5-$OCF_2H$ | $CH_3$ | |
| J-11 | Q-1 ($R_7$=H, $R_8$=H) | H | 6-$SO_2N(CH_3)_2$ | $CH_3$ | |
| J-11 | Q-1 ($R_7$=H, $R_8$=H) | H | 6-$SOCH_3$ | $CH_3$ | |
| J-11 | Q-1 ($R_7$=H, $R_8$=H) | H | 6-$SO_2CH(CH_3)_2$ | $CH_3$ | |
| J-11 | Q-1 ($R_7$=H, $R_8$=H) | H | 6-$CO_2CH_3$ | $CH_3$ | |
| J-11 | Q-1 ($R_7$=H, $R_8$=H) | H | H | $OCH_3$ | |
| J-11 | Q-1 ($R_7$=H, $R_8$=H) | H | H | $OC_2H_5$ | |
| J-11 | Q-1 ($R_7$=H, $R_8$=H) | H | H | $OCF_2H$ | |
| J-11 | Q-1 ($R_7$=H, $R_8$=$CH_3$) | H | H | $CH_3$ | |
| J-11 | Q-1 ($R_7$=H, $R_8$=$CH_2CH_3$) | H | H | $CH_3$ | |
| J-11 | Q-1 ($R_7$=H, $R_8$=$CH(CH_3)_2$) | H | H | $OCH_3$ | |
| J-11 | Q-1 ($R_7$=H, $R_8$=n-$C_4H_9$) | H | H | $OCH_3$ | |
| J-11 | Q-1 ($R_7$=H, $R_8$=$CH_3$) | H | H | $OCH_3$ | |
| J-11 | Q-37 ($R_7$=H, $R_8$=H, $R_2$=H) | H | H | $CH_3$ | |
| J-11 | Q-37 ($R_7$=H, $R_8$=H, $R_2$=i-$C_3H_7$) | H | H | $CH_3$ | |
| J-11 | Q-37 ($R_7$=H, $R_8$=H, $R_2$=n-$C_4H_9$) | H | 5-$OCH_3$ | $OCH_3$ | |
| J-11 | Q-98 ($R_7$=H, $R_8$=H, $R_9$=H) | H | H | $CH_3$ | |
| J-11 | Q-98 ($R_7$=$CH_3$, $R_8$=H, $R_9$=H) | H | H | $CH_3$ | |
| J-11 | Q-98 ($R_7$=H, $R_8$=H, $R_9$=i-$C_3H_7$) | H | 5-$OCH_3$ | $OCH_3$ | |
| J-11 | Q-105 ($R_7$=H, $R_8$=H, $R_9$=H, $R_2$=H) | H | H | $CH_3$ | |
| J-11 | Q-105 ($R_7$=$CH_3$, $R_8$=H, $R_9$=H, $R_2$=$CH_3$) | H | H | $CH_3$ | |
| J-11 | Q-105 ($R_7$=H, $R_8$=$CH_3$, $R_9$=H, $R_2$=$C_2H_5$) | H | 5-$OCH_3$ | $OCH_3$ | |
| J-11 | Q-138 ($R_7$=H, $R_8$=H, $R_9$=H) | H | H | $CH_3$ | |
| J-11 | Q-138 ($R_7$=$CH_3$, $R_8$=H, $R_9$=H) | H | H | $CH_3$ | |
| J-11 | Q-138 ($R_7$=H, $R_8$=H, $R_9$=$CH_3$) | H | H | $OCH_3$ | |
| J-11 | Q-143 ($R_7$=H, $R_8$=H, $R_9$=H, $R_3$=$CH_3$) | H | H | $CH_3$ | |
| J-11 | Q-143 ($R_7$=H, $R_8$=H, $R_9$=H, $R_3$=H) | H | H | $CH_3$ | |
| J-11 | Q-143 ($R_7$=$CH_3$, $R_8$=H, $R_9$=$CH_3$, $R_3$=$CH_3$) | H | 5-Cl | $OCH_3$ | |

TABLE 5
General Formula 5

| J | $W_3$ | Q | R | $R_{1b}$ | $X_1$ | $Y_2$ |
|---|---|---|---|---|---|---|
| J-5 | S | Q-1 ($R_7$=H, $R_8$=H) | H | H | $CH_3$ | $CH_3$ |
| J-6 | S | Q-1 ($R_7$=H, $R_8$=H) | H | H | $CH_3$ | H |
| J-5 | S | Q-1 ($R_7$=H, $R_8$=H) | H | H | $OCH_3$ | $CH_3$ |
| J-6 | S | Q-1 ($R_7$=H, $R_8$=H) | H | H | $OC_2H_5$ | $CH_3$ |
| J-5 | S | Q-1 ($R_7$=H, $R_8$=H) | H | H | $OCF_2H$ | $CH_3$ |
| J-6 | S | Q-1 ($R_7$=H, $R_8$=H) | H | H | $OCH_3$ | H |
| J-5 | NH | Q-1 ($R_7$=H, $R_8$=H) | H | H | $OC_2H_5$ | H |
| J-6 | S | Q-1 ($R_7$=H, $R_8$=H) | H | H | $OCF_2H$ | H |
| J-5 | S | Q-1 ($R_7$=H, $R_8$=H) | $CH_3$ | H | $CH_3$ | $CH_3$ |
| J-6 | O | Q-1 ($R_7$=H, $R_8$=H) | $CH_3$ | H | $OCH_3$ | $CH_3$ |
| J-7 | S | Q-1 ($R_7$=H, $R_8$=H) | $CH_3$ | H | $CH_3$ | H |
| J-5 | S | Q-1 ($R_7$=H, $R_8$=H) | H | 5-$CH_3$ | $OCH_3$ | H |
| J-6 | S | Q-1 ($R_7$=H, $R_8$=H) | H | 5-$CH_2F$ | $OCH_3$ | H |
| J-5 | S | Q-1 ($R_7$=H, $R_8$=H) | H | 5-$CH_2CH_2Br$ | $OCH_3$ | H |
| J-6 | S | Q-1 ($R_7$=H, $R_8$=H) | H | 5-$CH(CH_3)(CH_2Cl)$ | $OCH_3$ | H |
| J-5 | S | Q-1 ($R_7$=H, $R_8$=H) | H | 4-$NH_2$ | $OCH_3$ | H |
| J-5 | S | Q-1 ($R_7$=H, $R_8$=H) | H | 4-$N(CH_3)_2$ | $OCH_3$ | H |
| J-5 | S | Q-1 ($R_7$=H, $R_8$=H) | H | 5-Cl | $OCH_3$ | H |
| J-6 | S | Q-1 ($R_7$=H, $R_8$=H) | H | 4-F | $OCH_3$ | H |
| J-5 | S | Q-1 ($R_7$=H, $R_8$=H) | H | 5-I | $OCH_3$ | H |
| J-6 | S | Q-1 ($R_7$=H, $R_8$=H) | H | 4-$NO_2$ | $OCH_3$ | H |
| J-7 | O | Q-1 ($R_7$=H, $R_8$=H) | H | 5-$CF_3$ | $OCH_3$ | H |
| J-5 | S | Q-1 ($R_7$=H, $R_8$=$CH_3$) | H | H | $OCH_3$ | H |
| J-6 | S | Q-1 ($R_7$=H, $R_8$=$CH_3$) | H | H | $OCH_3$ | $CH_3$ |
| J-5 | S | Q-1 ($R_7$=H, $R_8$=$CH_2CH_3$) | H | H | $CH_3$ | H |
| J-6 | S | Q-1 ($R_7$=H, $R_8$=$CH(CH_3)_2$) | H | H | $CH_3$ | $CH_3$ |
| J-5 | S | Q-1 ($R_7$=H, $R_8$=n-$C_4H_9$) | H | H | $OCF_2H$ | H |
| J-6 | S | Q-1 ($R_7$=$CH_3$, $R_8$=$CH_3$) | H | H | $OCH_3$ | H |
| J-7 | S | Q-37 ($R_7$=H, $R_8$=H, $R_2$=H) | H | H | $OCH_3$ | H |
| J-5 | S | Q-37 ($R_7$=H, $R_8$=H, $R_2$=i-$C_3H_7$) | H | H | $OCH_3$ | $CH_3$ |
| J-6 | S | Q-37 ($R_7$=H, $R_8$=H, $R_2$=n-$C_4H_9$) | H | 5-$CH_3$ | $OCF_2H$ | H |

Table 5 (continued)

| J | $W_3$ | Q | R | $R_{1b}$ | $X_1$ | $Y_2$ |
|---|---|---|---|---|---|---|
| J-5 | S | Q-98 ($R_7$=H, $R_8$=H, $R_9$=H) | H | H | $OCH_3$ | H |
| J-6 | S | Q-98 ($R_7$=$CH_3$, $R_8$=H, $R_9$=H) | H | H | $OCH_3$ | H |
| J-5 | S | Q-98 ($R_7$=H, $R_8$=H, $R_9$=i-$C_3H_7$) | H | 5-$CH_3$ | $OCH_3$ | H |
| J-6 | S | Q-105 ($R_7$=H, $R_8$=H, $R_9$=H, $R_2$=H) | H | H | $OCH_3$ | H |
| J-5 | S | Q-105 ($R_7$=$CH_3$, $R_8$=H, $R_9$=H, $R_2$=$CH_3$) | H | H | $OCH_3$ | H |
| J-6 | S | Q-105 ($R_7$=H, $R_8$=$CH_3$, $R_9$=H, $R_2$=$C_2H_5$) | H | 5-$CH_3$ | $OCH_3$ | H |
| J-6 | S | Q-138 ($R_7$=H, $R_8$=H, $R_9$=H) | H | H | $OCH_3$ | H |
| J-5 | S | Q-138 ($R_7$=$CH_3$, $R_8$=H, $R_9$=H) | H | H | $OCH_3$ | H |
| J-6 | S | Q-138 ($R_7$=H, $R_8$=H, $R_9$=$CH_3$) | H | H | $OCH_3$ | H |
| J-6 | S | Q-143 ($R_7$=H, $R_8$=H, $R_9$=H, $R_3$=$CH_3$) | H | H | $OCH_3$ | H |
| J-5 | O | Q-143 ($R_7$=H, $R_8$=H, $R_9$=H, $R_3$=H) | H | H | $OCH_3$ | $CH_3$ |
| J-6 | S | Q-143 ($R_7$=$CH_3$, $R_8$=H, $R_9$=$CH_3$, $R_3$=$CH_3$) | H | 5-Cl | $CH_3$ | H |

Table 5 (continued)

| J | Q | R | $R_{1c}$ | $X_1$ | $Y_2$ |
|---|---|---|---|---|---|
| J-8 | Q-1 ($R_7$=H, $R_8$=H) | H | H | $CH_3$ | $CH_3$ |
| J-8 | Q-1 ($R_7$=H, $R_8$=H) | H | H | $CH_3$ | H |
| J-8 | Q-1 ($R_7$=H, $R_8$=H) | H | H | $OCH_3$ | $CH_3$ |
| J-8 | Q-1 ($R_7$=H, $R_8$=H) | H | H | $OCF_2H$ | $CH_3$ |
| J-8 | Q-1 ($R_7$=H, $R_8$=H) | H | H | $OCH_3$ | H |
| J-8 | Q-1 ($R_7$=H, $R_8$=H) | H | H | $OCF_2H$ | H |
| J-8 | Q-1 ($R_7$=H, $R_8$=H) | H | 5-$CH_3$ | $OCH_3$ | H |
| J-8 | Q-1 ($R_7$=H, $R_8$=H) | H | 5-$OCH_3$ | $OCH_3$ | H |
| J-8 | Q-1 ($R_7$=H, $R_8$=H) | H | 6-$OCH_2CH_2Br$ | $OCH_3$ | H |
| J-8 | Q-1 ($R_7$=H, $R_8$=H) | H | 5-$CH_2F$ | $OCH_3$ | H |
| J-8 | Q-1 ($R_7$=H, $R_8$=H) | H | 5-$SCH_3$ | $OCH_3$ | H |
| J-8 | Q-1 ($R_7$=H, $R_8$=H) | H | 5-$SCH_2F$ | $OCH_3$ | H |
| J-8 | Q-1 ($R_7$=H, $R_8$=H) | H | 5-$NHCH_3$ | $OCH_3$ | H |
| J-8 | Q-1 ($R_7$=H, $R_8$=H) | H | 5-Cl | $OCH_3$ | H |
| J-8 | Q-1 ($R_7$=H, $R_8$=H) | H | 4-$NO_2$ | $OCH_3$ | H |
| J-8 | Q-1 ($R_7$=H, $R_8$=H) | H | 5-$CF_3$ | $OCH_3$ | H |
| J-8 | Q-37 ($R_7$=H, $R_8$=H, $R_2$=H) | H | H | $OCH_3$ | H |
| J-8 | Q-105 ($R_7$=H, $R_8$=H, $R_9$=H, $R_2$=H) | H | H | $OCH_3$ | H |
| J-8 | Q-138 ($R_7$=H, $R_8$=H, $R_9$=H) | H | H | $OCH_3$ | H |
| J-8 | Q-143 ($R_7$=H, $R_8$=H, $R_9$=H, $R_3$=$CH_3$) | H | H | $OCH_3$ | H |
| J-9 | Q-1 ($R_7$=H, $R_8$=H) | H | 5-$SCH_3$ | $CH_3$ | $CH_3$ |
| J-9 | Q-1 ($R_7$=H, $R_8$=H) | H | 5-$SOCH_3$ | $CH_3$ | $CH_3$ |
| J-9 | Q-1 ($R_7$=H, $R_8$=H) | H | 5-$CO_2CH_3$ | $CH_3$ | $CH_3$ |
| J-9 | Q-1 ($R_7$=H, $R_8$=H) | H | 5-$SO_2N(CH_3)_2$ | $CH_3$ | $CH_3$ |
| J-9 | Q-1 ($R_7$=H, $R_8$=H) | H | 5-$NO_2$ | $CH_3$ | $CH_3$ |
| J-9 | Q-1 ($R_7$=H, $R_8$=H) | H | 5-Cl | $CH_3$ | $CH_3$ |
| J-9 | Q-1 ($R_7$=H, $R_8$=H) | H | 4-$NO_2$ | $CH_3$ | $CH_3$ |
| J-9 | Q-1 ($R_7$=H, $R_8$=H) | H | 6-Cl | $CH_3$ | $CH_3$ |
| J-9 | Q-1 ($R_7$=H, $R_8$=H) | H | 6-$COOCH_3$ | $CH_3$ | $CH_3$ |
| J-9 | Q-1 ($R_7$=H, $R_8$=H) | H | 6-$NO_2$ | $CH_3$ | $CH_3$ |
| J-9 | Q-1 ($R_7$=H, $R_8$=H) | H | 6-$SO_2CH_3$ | $CH_3$ | $CH_3$ |
| J-9 | Q-1 ($R_7$=H, $R_8$=H) | H | 6-$OCH_3$ | $CH_3$ | $CH_3$ |

Table 5 (continued)

| J | Q | R | $R_{1c}$ | $X_1$ | $Y_2$ |
|---|---|---|---|---|---|
| J-9 | Q-1 ($R_7$=H, $R_8$=H) | H | 5-$OCH_3$ | $CH_3$ | $CH_3$ |
| J-9 | Q-1 ($R_7$=H, $R_8$=H) | H | H | $CH_3$ | $CH_3$ |
| J-9 | Q-1 ($R_7$=H, $R_8$=H) | H | H | $CH_3$ | H |
| J-9 | Q-1 ($R_7$=H, $R_8$=H) | H | H | $OCH_3$ | $CH_3$ |
| J-9 | Q-1 ($R_7$=H, $R_8$=H) | H | H | $OC_2H_5$ | $CH_3$ |
| J-9 | Q-1 ($R_7$=H, $R_8$=H) | H | H | $OCF_2H$ | $CH_3$ |
| J-9 | Q-1 ($R_7$=H, $R_8$=H) | H | H | $OCH_3$ | H |
| J-9 | Q-1 ($R_7$=H, $R_8$=H) | H | H | $OC_2H_5$ | H |
| J-9 | Q-1 ($R_7$=H, $R_8$=H) | H | H | $OCF_2H$ | H |
| J-9 | Q-1 ($R_7$=H, $R_8$=H) | $CH_3$ | H | $CH_3$ | $CH_3$ |
| J-9 | Q-1 ($R_7$=H, $R_8$=H) | $CH_3$ | H | $OCH_3$ | $CH_3$ |
| J-9 | Q-1 ($R_7$=H, $R_8$=H) | $CH_3$ | H | $CH_3$ | H |
| J-9 | Q-1 ($R_7$=H, $R_8$=H) | H | 5-$CH_3$ | $OCH_3$ | H |
| J-9 | Q-1 ($R_7$=H, $R_8$=H) | H | 6-$CH_2CH_3$ | $OCH_3$ | H |
| J-9 | Q-1 ($R_7$=H, $R_8$=H) | H | 5-$CH(CH_3)_2$ | $OCH_3$ | H |
| J-9 | Q-1 ($R_7$=H, $R_8$=H) | H | 5-$OCH_2CH_3$ | $OCH_3$ | H |
| J-9 | Q-1 ($R_7$=H, $R_8$=H) | H | 5-$OCH(CH_3)_2$ | $OCH_3$ | H |
| J-9 | Q-1 ($R_7$=H, $R_8$=H) | H | 5-$OCF_2H$ | $OCH_3$ | H |
| J-9 | Q-1 ($R_7$=H, $R_8$=H) | H | 6-$OCH_2CH_2Br$ | $OCH_3$ | H |
| J-9 | Q-1 ($R_7$=H, $R_8$=H) | H | 6-$OCH(CH_3)(CH_2Cl)$ | $OCH_3$ | H |
| J-9 | Q-1 ($R_7$=H, $R_8$=H) | H | 5-$CH_2F$ | $OCH_3$ | H |
| J-9 | Q-1 ($R_7$=H, $R_8$=H) | H | 5-$CH_2CH_2Br$ | $OCH_3$ | H |
| J-9 | Q-1 ($R_7$=H, $R_8$=H) | H | 5-$CH(CH_3)(CH_2Cl)$ | $OCH_3$ | H |
| J-9 | Q-1 ($R_7$=H, $R_8$=H) | H | 5-$SCH_3$ | $OCH_3$ | H |
| J-9 | Q-1 ($R_7$=H, $R_8$=H) | H | 6-$SCH_2CH_3$ | $OCH_3$ | H |
| J-9 | Q-1 ($R_7$=H, $R_8$=H) | H | 5-$SCH(CH_3)_2$ | $OCH_3$ | H |
| J-9 | Q-1 ($R_7$=H, $R_8$=H) | H | 5-$SCH_2F$ | $OCH_3$ | H |
| J-9 | Q-1 ($R_7$=H, $R_8$=H) | H | 5-$SCH_2CH_2Br$ | $OCH_3$ | H |
| J-9 | Q-1 ($R_7$=H, $R_8$=H) | H | 5-$SCH(CH_3)(CH_2Cl)$ | $OCH_3$ | H |
| J-9 | Q-1 ($R_7$=H, $R_8$=H) | H | 4-$NH_2$ | $OCH_3$ | H |
| J-9 | Q-1 ($R_7$=H, $R_8$=H) | H | 5-$NHCH_3$ | $OCH_3$ | H |
| J-9 | Q-1 ($R_7$=H, $R_8$=H) | H | 6-$NHCH_2CH_3$ | $OCH_3$ | H |
| J-9 | Q-1 ($R_7$=H, $R_8$=H) | H | 5-$NHCH(CH_3)_2$ | $OCH_3$ | H |

Table 5 (continued)

| J | Q | R | $R_{1c}$ | $X_1$ | $Y_2$ |
|---|---|---|---|---|---|
| J-9 | Q-1 ($R_7$=H, $R_8$=H) | H | 4-$N(CH_3)_2$ | $OCH_3$ | H |
| J-9 | Q-1 ($R_7$=H, $R_8$=H) | H | 6-$N(CH_3)(CH_2CH_3)$ | $OCH_3$ | H |
| J-9 | Q-1 ($R_7$=H, $R_8$=H) | H | 5-$N(CH_3)(CH(CH_3)_2)$ | $OCH_3$ | H |
| J-9 | Q-1 ($R_7$=H, $R_8$=H) | H | 5-Br | $OCH_3$ | H |
| J-9 | Q-1 ($R_7$=H, $R_8$=H) | H | 4-F | $OCH_3$ | H |
| J-9 | Q-1 ($R_7$=H, $R_8$=H) | H | 5-I | $OCH_3$ | H |
| J-9 | Q-1 ($R_7$=H, $R_8$=H) | H | 5-$CF_3$ | $OCH_3$ | H |
| J-9 | Q-1 ($R_7$=H, $R_8$=H) | H | 5-$OCF_2H$ | $OCH_3$ | H |
| J-9 | Q-1 ($R_7$=H, $R_8$=$CH_3$) | H | H | $OCH_3$ | H |
| J-9 | Q-1 ($R_7$=H, $R_8$=$CH_3$) | H | H | $OCH_3$ | $CH_3$ |
| J-9 | Q-1 ($R_7$=H, $R_8$=$CH_2CH_3$) | H | H | $CH_3$ | H |
| J-9 | Q-1 ($R_7$=H, $R_8$=$CH(CH_3)_2$) | H | H | $CH_3$ | $CH_3$ |
| J-9 | Q-1 ($R_7$=H, $R_8$=$\underline{n}$-$C_4H_9$) | H | H | $OCF_2H$ | H |
| J-9 | Q-1 ($R_7$=$CH_3$, $R_8$=$CH_3$) | H | H | $OCH_3$ | H |
| J-9 | Q-37 ($R_7$=H, $R_8$=H, $R_2$=H) | H | H | $OCH_3$ | H |
| J-9 | Q-98 ($R_7$=$CH_3$, $R_8$=H, $R_9$=H) | H | H | $OCH_3$ | H |
| J-9 | Q-105 ($R_7$=H, $R_8$=H, $R_9$=H, $R_2$=H) | H | H | $OCH_3$ | H |
| J-9 | Q-138 ($R_7$=H, $R_8$=H, $R_9$=H) | H | H | $OCH_3$ | H |
| J-9 | Q-143 ($R_7$=H, $R_8$=H, $R_9$=H, $R_3$=H) | H | H | $OCH_3$ | $CH_3$ |

Table 5 (continued)

| J | Q | R | $R_{1d}$ | $X_1$ | $Y_2$ | m.p.(°C) |
|---|---|---|---|---|---|---|
| J-11 | Q-1 ($R_7$=H, $R_8$=H) | H | H | $CH_3$ | $CH_3$ | |
| J-11 | Q-1 ($R_7$=H, $R_8$=H) | H | H | $CH_3$ | H | |
| J-11 | Q-1 ($R_7$=H, $R_8$=H) | H | H | $OCH_3$ | $CH_3$ | |
| J-11 | Q-1 ($R_7$=H, $R_8$=H) | H | H | $OC_2H_5$ | $CH_3$ | |
| J-11 | Q-1 ($R_7$=H, $R_8$=H) | H | H | $OCF_2H$ | $CH_3$ | |
| J-11 | Q-1 ($R_7$=H, $R_8$=H) | H | H | $OCH_3$ | H | |
| J-11 | Q-1 ($R_7$=H, $R_8$=H) | H | H | $OC_2H_5$ | H | |
| J-11 | Q-1 ($R_7$=H, $R_8$=H) | H | H | $OCF_2H$ | H | |
| J-11 | Q-1 ($R_7$=H, $R_8$=H) | $CH_3$ | H | $CH_3$ | $CH_3$ | |
| J-11 | Q-1 ($R_7$=H, $R_8$=H) | $CH_3$ | H | $OCH_3$ | $CH_3$ | |
| J-11 | Q-1 ($R_7$=H, $R_8$=H) | $CH_3$ | H | $CH_3$ | H | |
| J-11 | Q-1 ($R_7$=H, $R_8$=H) | H | 5-$CH_3$ | $OCH_3$ | H | |
| J-11 | Q-1 ($R_7$=H, $R_8$=H) | H | 6-$CH_2CH_3$ | $OCH_3$ | H | |
| J-11 | Q-1 ($R_7$=H, $R_8$=H) | H | 3-$CH(CH_3)_2$ | $OCH_3$ | H | |
| J-11 | Q-1 ($R_7$=H, $R_8$=H) | H | 5-$OCH_3$ | $OCH_3$ | H | |
| J-11 | Q-1 ($R_7$=H, $R_8$=H) | H | 5-$OCH_2CH_3$ | $OCH_3$ | H | |
| J-11 | Q-1 ($R_7$=H, $R_8$=H) | H | 3-$OCH(CH_3)_2$ | $OCH_3$ | H | |
| J-11 | Q-1 ($R_7$=H, $R_8$=H) | H | 5-$OCF_2H$ | $OCH_3$ | H | |
| J-11 | Q-1 ($R_7$=H, $R_8$=H) | H | 6-$OCH_2CH_2Br$ | $OCH_3$ | H | |
| J-11 | Q-1 ($R_7$=H, $R_8$=H) | H | 6-$OCH(CH_3)(CH_2Cl)$ | $OCH_3$ | H | |
| J-11 | Q-1 ($R_7$=H, $R_8$=H) | H | 5-$CH_2F$ | $OCH_3$ | H | |
| J-11 | Q-1 ($R_7$=H, $R_8$=H) | H | 5-$CH_2CH_2Br$ | $OCH_3$ | H | |
| J-11 | Q-1 ($R_7$=H, $R_8$=H) | H | 5-$CH(CH_3)(CH_2Cl)$ | $OCH_3$ | H | |
| J-11 | Q-1 ($R_7$=H, $R_8$=H) | H | 5-$SCH_3$ | $OCH_3$ | H | |
| J-11 | Q-1 ($R_7$=H, $R_8$=H) | H | 6-$SCH_2CH_3$ | $OCH_3$ | H | |
| J-11 | Q-1 ($R_7$=H, $R_8$=H) | H | 5-$SCH(CH_3)_2$ | $OCH_3$ | H | |
| J-11 | Q-1 ($R_7$=H, $R_8$=H) | H | 5-$SCH_2F$ | $OCH_3$ | H | |
| J-11 | Q-1 ($R_7$=H, $R_8$=H) | H | 5-$SCH_2CH_2Br$ | $OCH_3$ | H | |
| J-11 | Q-1 ($R_7$=H, $R_8$=H) | H | 5-$SCH(CH_3)(CH_2Cl)$ | $OCH_3$ | H | |
| J-11 | Q-1 ($R_7$=H, $R_8$=H) | H | 4-$NH_2$ | $OCH_3$ | H | |
| J-11 | Q-1 ($R_7$=H, $R_8$=H) | H | 5-$NHCH_3$ | $OCH_3$ | H | |

Table 5 (continued)

| J | Q | R | $R_{1d}$ | $X_1$ | $Y_2$ | m.p. (°C) |
|---|---|---|---|---|---|---|
| J-11 | Q-1 ($R_7$=H, $R_8$=H) | H | 6-$NHCH_2CH_3$ | $OCH_3$ | H | |
| J-11 | Q-1 ($R_7$=H, $R_8$=H) | H | 3-$NHCH(CH_3)_2$ | $OCH_3$ | H | |
| J-11 | Q-1 ($R_7$=H, $R_8$=H) | H | 4-$N(CH_3)_2$ | $OCH_3$ | H | |
| J-11 | Q-1 ($R_7$=H, $R_8$=H) | H | 6-$N(CH_3)(CH_2CH_3)$ | $OCH_3$ | H | |
| J-11 | Q-1 ($R_7$=H, $R_8$=H) | H | 5-$N(CH_3)(CH(CH_3)_2)$ | $OCH_3$ | H | |
| J-11 | Q-1 ($R_7$=H, $R_8$=H) | H | 5-Cl | $OCH_3$ | H | |
| J-11 | Q-1 ($R_7$=H, $R_8$=H) | H | 3-Br | $OCH_3$ | H | |
| J-11 | Q-1 ($R_7$=H, $R_8$=H) | H | 4-F | $OCH_3$ | H | |
| J-11 | Q-1 ($R_7$=H, $R_8$=H) | H | 5-I | $OCH_3$ | H | |
| J-11 | Q-1 ($R_7$=H, $R_8$=H) | H | 4-$NO_2$ | $OCH_3$ | H | |
| J-11 | Q-1 ($R_7$=H, $R_8$=H) | H | 5-$CF_3$ | $OCH_3$ | H | |
| J-11 | Q-1 ($R_7$=H, $R_8$=H) | H | 5-$OCF_2H$ | $OCH_3$ | H | |
| J-11 | Q-1 ($R_7$=H, $R_8$=H) | H | 6-$SO_2N(CH_3)_2$ | $OCH_3$ | H | |
| J-11 | Q-1 ($R_7$=H, $R_8$=H) | H | 6-$SOCH_3$ | $OCH_3$ | H | |
| J-11 | Q-1 ($R_7$=H, $R_8$=H) | H | 6-$SO_2CH(CH_3)_2$ | $OCH_3$ | H | |
| J-11 | Q-1 ($R_7$=H, $R_8$=H) | H | 6-$CO_2CH_3$ | $OCH_3$ | H | |
| J-11 | Q-1 ($R_7$=H, $R_8$=$CH_3$) | H | H | $OCH_3$ | H | |
| J-11 | Q-1 ($R_7$=H, $R_8$=$CH_3$) | H | H | $OCH_3$ | $CH_3$ | |
| J-11 | Q-1 ($R_7$=H, $R_8$=$CH_2CH_3$) | H | H | $CH_3$ | H | |
| J-11 | Q-1 ($R_7$=H, $R_8$=$CH(CH_3)_2$) | H | H | $CH_3$ | $CH_3$ | |
| J-11 | Q-1 ($R_7$=H, $R_8$=n-$C_4H_9$) | H | H | $OCF_2H$ | H | |
| J-11 | Q-1 ($R_7$=$CH_3$, $R_8$=$CH_3$) | H | H | $OCH_3$ | H | |
| J-11 | Q-37 ($R_7$=H, $R_8$=H, $R_2$=H) | H | H | $OCH_3$ | H | |
| J-11 | Q-37 ($R_7$=H, $R_8$=H, $R_2$=i-$C_3H_7$) | H | H | $OCH_3$ | $CH_3$ | |
| J-11 | Q-37 ($R_7$=H, $R_8$=H, $R_2$=n-$C_4H_9$) | H | 5-$OCH_3$ | $OCF_2H$ | H | |
| J-11 | Q-98 ($R_7$=H, $R_8$=H, $R_9$=H) | H | H | $OCH_3$ | H | |
| J-11 | Q-98 ($R_7$=$CH_3$, $R_8$=H, $R_9$=H) | H | H | $OCH_3$ | H | |
| J-11 | Q-98 ($R_7$=H, $R_8$=H, $R_9$=i-$C_3H_7$) | H | 5-$OCH_3$ | $OCH_3$ | H | |

Table 5 (continued)

| J | Q | R | $R_{1d}$ | $X_1$ | $Y_2$ | m.p. (°C) |
|---|---|---|---|---|---|---|
| J-11 | Q-105 ($R_7$=H, $R_8$=H, $R_9$=H, $R_2$=H) | H | H | $OCH_3$ | H | |
| J-11 | Q-105 ($R_7$=$CH_3$, $R_8$=H, $R_9$=H, $R_2$=$CH_3$) | H | H | $OCH_3$ | H | |
| J-11 | Q-105 ($R_7$=H, $R_8$=$CH_3$, $R_9$=H, $R_2$=$C_2H_5$) | H | 5-$OCH_3$ | $OCH_3$ | H | |
| J-11 | Q-138 ($R_7$=H, $R_8$=H, $R_9$=H) | H | H | $OCH_3$ | H | |
| J-11 | Q-138 ($R_7$=$CH_3$, $R_8$=H, $R_9$=H) | H | H | $OCH_3$ | H | |
| J-11 | Q-138 ($R_7$=H, $R_8$=H, $R_9$=$CH_3$) | H | H | $OCH_3$ | H | |
| J-11 | Q-143 ($R_7$=H, $R_8$=H, $R_9$=H, $R_3$=$CH_3$) | H | H | $OCH_3$ | H | |
| J-11 | Q-143 ($R_7$=H, $R_8$=H, $R_9$=H, $R_3$=H) | H | H | $OCH_3$ | $CH_3$ | |
| J-11 | Q-143 ($R_7$=$CH_3$, $R_8$=H, $R_9$=$CH_3$, $R_3$=$CH_3$) | H | 5-Cl | $CH_3$ | H | |

TABLE 6
General Formula 6

| J | $W_3$ | Q | R | $R_{1b}$ | $X_2$ | $Y_3$ |
|---|---|---|---|---|---|---|
| J-5 | S | Q-1 ($R_7$=H, $R_8$=H) | H | H | $CH_3$ | $CH_3$ |
| J-6 | S | Q-1 ($R_7$=H, $R_8$=H) | H | H | $OCH_3$ | $CH_3$ |
| J-5 | S | Q-1 ($R_7$=H, $R_8$=H) | H | H | $SCH_3$ | $CH_3$ |
| J-6 | S | Q-1 ($R_7$=H, $R_8$=H) | H | H | $CH_3$ | $CH_2CH_3$ |
| J-5 | S | Q-1 ($R_7$=H, $R_8$=H) | H | H | $OCH_3$ | $CH_2CH_3$ |
| J-6 | S | Q-1 ($R_7$=H, $R_8$=H) | H | H | $SCH_3$ | $CH_2CH_3$ |
| J-5 | S | Q-1 ($R_7$=H, $R_8$=H) | H | H | $CH_3$ | $CH_2CF_3$ |
| J-6 | S | Q-1 ($R_7$=H, $R_8$=H) | H | H | $OCH_3$ | $CH_2CF_3$ |
| J-5 | S | Q-1 ($R_7$=H, $R_8$=H) | H | H | $SCH_3$ | $CH_2CF_3$ |
| J-6 | O | Q-1 ($R_7$=H, $R_8$=H) | $CH_3$ | 5-$CH_3$ | $CH_3$ | $CH_3$ |
| J-7 | S | Q-1 ($R_7$=H, $R_8$=H) | H | 5-$CH_2F$ | $OCH_3$ | $CH_3$ |
| J-5 | S | Q-1 ($R_7$=H, $R_8$=H) | H | 5-$CH_2CH_2Br$ | $OCH_3$ | $CH_3$ |
| J-6 | S | Q-1 ($R_7$=H, $R_8$=H) | H | 5-$CH(CH_3)(CH_2Cl)$ | $OCH_3$ | $CH_3$ |
| J-5 | S | Q-1 ($R_7$=H, $R_8$=H) | H | 4-$NH_2$ | $OCH_3$ | $CH_3$ |
| J-6 | S | Q-1 ($R_7$=H, $R_8$=H) | H | 4-$N(CH_3)_2$ | $OCH_3$ | $CH_3$ |
| J-5 | S | Q-1($R_7$=H, $R_8$=H) | H | 5-Cl | $OCH_3$ | $CH_3$ |
| J-6 | S | Q-1 ($R_7$=H, $R_8$=H) | H | 4-F | $OCH_3$ | $CH_3$ |
| J-5 | S | Q-1 ($R_7$=H, $R_8$=H) | H | 5-I | $OCH_3$ | $CH_3$ |
| J-6 | S | Q-1 ($R_7$=H, $R_8$=H) | H | 4-$NO_2$ | $OCH_3$ | $CH_3$ |
| J-5 | O | Q-1 ($R_7$=H, $R_8$=H) | H | 5-$CF_3$ | $OCH_3$ | $CH_3$ |
| J-6 | NH | Q-1 ($R_7$=H, $R_8$=$CH_3$) | H | H | $OCH_3$ | $CH_3$ |
| J-7 | S | Q-1 ($R_7$=H, $R_8$=$CH_3$) | H | H | $CH_3$ | $CH_2CF_3$ |
| J-5 | S | Q-1 ($R_7$=H, $R_8$=$CH_2CH_3$) | H | H | $CH_3$ | $CH_3$ |
| J-6 | S | Q-1 ($R_7$=H, $R_8$=$CH(CH_3)_2$) | H | H | $OCH_3$ | $CH_3$ |
| J-5 | S | Q-1 ($R_7$=H, $R_8$=$\underline{n}$-$C_4H_9$) | H | H | $SCH_3$ | $CH_3$ |
| J-6 | S | Q-1 ($R_7$=$CH_3$, $R_8$=$CH_3$) | H | H | $OCH_3$ | $CH_3$ |
| J-7 | S | Q-37 ($R_7$=H, $R_8$=H, $R_2$=H) | H | H | $OCH_3$ | $CH_3$ |
| J-5 | S | Q-37 ($R_7$=H, $R_8$=H, $R_2$=$\underline{i}$-$C_3H_7$) | H | H | $OCH_3$ | $CH_3$ |
| J-6 | S | Q-37 ($R_7$=H, $R_8$=H, $R_2$=$\underline{n}$-$C_4H_9$) | H | 5-$CH_3$ | $CH_3$ | $CH_2CF_3$ |
| J-5 | S | Q-98 ($R_7$=H, $R_8$=H, $R_9$=H) | H | H | $OCH_3$ | $CH_3$ |
| J-6 | $NCH_3$ | Q-98 ($R_7$=$CH_3$, $R_8$=H, $R_9$=H) | H | H | $OCH_3$ | $CH_3$ |
| J-5 | S | Q-98 ($R_7$=H, $R_8$=H, $R_9$=$\underline{i}$-$C_3H_7$) | H | 5-$CH_3$ | $CH_3$ | $CH_2CF_3$ |

Table 6 (continued)

| J | $W_3$ | Q | R | $R_{1b}$ | $X_2$ | $Y_3$ |
|---|---|---|---|---|---|---|
| J-6 | S | Q-105 ($R_7$=H, $R_8$=H, $R_9$=H, $R_2$=H) | H | H | $OCH_3$ | $CH_3$ |
| J-5 | S | Q-105 ($R_7$=$CH_3$, $R_8$=H, $R_9$=H, $R_2$=$CH_3$) | H | H | $OCH_3$ | $CH_3$ |
| J-6 | S | Q-105 ($R_7$=H, $R_8$=$CH_3$, $R_9$=H, $R_2$=$C_2H_5$) | H | 5-$CH_3$ | $CH_3$ | $CH_2CF_3$ |
| J-6 | S | Q-138 ($R_7$=H, $R_8$=H, $R_9$=H) | H | H | $OCH_3$ | $CH_3$ |
| J-5 | S | Q-138 ($R_7$=$CH_3$, $R_8$=H, $R_9$=H) | H | H | $OCH_3$ | $CH_3$ |
| J-6 | S | Q-138 ($R_7$=H, $R_8$=H, $R_9$=$CH_3$) | H | H | $OCH_3$ | $CH_3$ |
| J-6 | O | Q-143 ($R_7$=H, $R_8$=H, $R_9$=H, $R_3$=$CH_3$) | H | H | $OCH_3$ | $CH_3$ |
| J-7 | S | Q-143 ($R_7$=H, $R_8$=H, $R_9$=H, $R_3$=H) | H | H | $OCH_3$ | $CH_3$ |
| J-5 | S | Q-143 ($R_7$=$CH_3$, $R_8$=H, $R_9$=$CH_3$, $R_3$=$CH_3$) | H | 5-Cl | $CH_3$ | $CH_3$ |

Table 6 (continued)

| J | Q | R | $R_{1c}$ | $X_2$ | $Y_3$ |
|---|---|---|---|---|---|
| J-8 | Q-1 ($R_7$=H, $R_8$=H) | H | H | $CH_3$ | $CH_3$ |
| J-8 | Q-1 ($R_7$=H, $R_8$=H) | H | H | $OCH_3$ | $CH_3$ |
| J-8 | Q-1 ($R_7$=H, $R_8$=H) | H | H | $SCH_3$ | $CH_3$ |
| J-8 | Q-1 ($R_7$=H, $R_8$=H) | H | H | $CH_3$ | $CH_2CF_3$ |
| J-8 | Q-1 ($R_7$=H, $R_8$=H) | H | H | $OCH_3$ | $CH_2CF_3$ |
| J-8 | Q-1 ($R_7$=H, $R_8$=H) | H | H | $SCH_3$ | $CH_2CF_3$ |
| J-8 | Q-1 ($R_7$=H, $R_8$=H) | H | 5-$OCH_3$ | $OCH_3$ | $CH_3$ |
| J-8 | Q-1 ($R_7$=H, $R_8$=H) | H | 6-$OCH_2CH_2Br$ | $OCH_3$ | $CH_3$ |
| J-8 | Q-1 ($R_7$=H, $R_8$=H) | H | 5-$CH_2F$ | $OCH_3$ | $CH_3$ |
| J-8 | Q-1 ($R_7$=H, $R_8$=H) | H | 5-$SCH_3$ | $OCH_3$ | $CH_3$ |
| J-8 | Q-1 ($R_7$=H, $R_8$=H) | H | 5-$SCH_2F$ | $OCH_3$ | $CH_3$ |
| J-8 | Q-1 ($R_7$=H, $R_8$=H) | H | 5-$NHCH_3$ | $OCH_3$ | $CH_3$ |
| J-8 | Q-1 ($R_7$=H, $R_8$=H) | H | 5-Cl | $OCH_3$ | $CH_3$ |
| J-8 | Q-1 ($R_7$=H, $R_8$=H) | H | 4-$NO_2$ | $OCH_3$ | $CH_3$ |
| J-8 | Q-1 ($R_7$=H, $R_8$=H) | H | 5-$CF_3$ | $OCH_3$ | $CH_3$ |
| J-8 | Q-37 ($R_7$=H, $R_8$=H, $R_2$=H) | H | H | $OCH_3$ | $CH_3$ |
| J-8 | Q-98 ($R_7$=H, $R_8$=H, $R_9$=H) | H | H | $OCH_3$ | $CH_3$ |
| J-8 | Q-105 ($R_7$=H, $R_8$=H, $R_9$=H, $R_2$=H) | H | H | $OCH_3$ | $CH_3$ |
| J-8 | Q-138 ($R_7$=H, $R_8$=H, $R_9$=H) | H | H | $OCH_3$ | $CH_3$ |
| J-8 | Q-143 ($R_7$=H, $R_8$=H, $R_9$=H, $R_3$=$CH_3$) | H | H | $OCH_3$ | $CH_3$ |
| J-9 | Q-1 ($R_7$=H, $R_8$=H) | H | 5-$SCH_3$ | $OCH_3$ | $CH_3$ |
| J-9 | Q-1 ($R_7$=H, $R_8$=H) | H | 5-$SOCH_3$ | $OCH_3$ | $CH_3$ |
| J-9 | Q-1 ($R_7$=H, $R_8$=H) | H | 5-$CO_2CH_3$ | $OCH_3$ | $CH_3$ |
| J-9 | Q-1 ($R_7$=H, $R_8$=H) | H | 5-$SO_2N(CH_3)_2$ | $OCH_3$ | $CH_3$ |
| J-9 | Q-1 ($R_7$=H, $R_8$=H) | H | 5-$NO_2$ | $OCH_3$ | $CH_3$ |
| J-9 | Q-1 ($R_7$=H, $R_8$=H) | H | 5-Cl | $OCH_3$ | $CH_3$ |
| J-9 | Q-1 ($R_7$=H, $R_8$=H) | H | 4-$NO_2$ | $OCH_3$ | $CH_3$ |
| J-9 | Q-1 ($R_7$=H, $R_8$=H) | H | 6-Cl | $OCH_3$ | $CH_3$ |
| J-9 | Q-1 ($R_7$=H, $R_8$=H) | H | 6-$COOCH_3$ | $OCH_3$ | $CH_3$ |
| J-9 | Q-1 ($R_7$=H, $R_8$=H) | H | 6-$NO_2$ | $OCH_3$ | $CH_3$ |

Table 6 (continued)

| J | Q | R | $R_{1c}$ | $X_2$ | $Y_3$ |
|---|---|---|---|---|---|
| J-9 | Q-1 ($R_7$=H, $R_8$=H) | H | 6-$SO_2CH_3$ | $OCH_3$ | $CH_3$ |
| J-9 | Q-1 ($R_7$=H, $R_8$=H) | H | 6-$OCH_3$ | $OCH_3$ | $CH_3$ |
| J-9 | Q-1 ($R_7$=H, $R_8$=H) | H | 5-$OCH_3$ | $OCH_3$ | $CH_3$ |
| J-9 | Q-1 ($R_7$=H, $R_8$=H) | H | H | $CH_3$ | $CH_3$ |
| J-9 | Q-1 ($R_7$=H, $R_8$=H) | H | H | $OCH_3$ | $CH_3$ |
| J-9 | Q-1 ($R_7$=H, $R_8$=H) | H | H | $SCH_3$ | $CH_3$ |
| J-9 | Q-1 ($R_7$=H, $R_8$=H) | H | H | $CH_3$ | $CH_2CH_3$ |
| J-9 | Q-1 ($R_7$=H, $R_8$=H) | H | H | $OCH_3$ | $CH_2CH_3$ |
| J-9 | Q-1 ($R_7$=H, $R_8$=H) | H | H | $SCH_3$ | $CH_2CH_3$ |
| J-9 | Q-1 ($R_7$=H, $R_8$=H) | H | H | $CH_3$ | $CH_2CF_3$ |
| J-9 | Q-1 ($R_7$=H, $R_8$=H) | H | H | $OCH_3$ | $CH_2CF_3$ |
| J-9 | Q-1 ($R_7$=H, $R_8$=H) | H | H | $SCH_3$ | $CH_2CF_3$ |
| J-9 | Q-1 ($R_7$=H, $R_8$=H) | $CH_3$ | 5-$CH_3$ | $CH_3$ | $CH_3$ |
| J-9 | Q-1 ($R_7$=H, $R_8$=H) | $CH_3$ | 6-$CH_2CH_3$ | $OCH_3$ | $CH_3$ |
| J-9 | Q-1 ($R_7$=H, $R_8$=H) | $CH_3$ | 5-$CH(CH_3)_2$ | $SCH_3$ | $CH_3$ |
| J-9 | Q-1 ($R_7$=H, $R_8$=H) | H | 5-$OCH_3$ | $OCH_3$ | $CH_3$ |
| J-9 | Q-1 ($R_7$=H, $R_8$=H) | H | 5-$OCH_2CH_3$ | $OCH_3$ | $CH_3$ |
| J-9 | Q-1 ($R_7$=H, $R_8$=H) | H | 5-$OCH(CH_3)_2$ | $OCH_3$ | $CH_3$ |
| J-9 | Q-1 ($R_7$=H, $R_8$=H) | H | 5-$OCF_2H$ | $OCH_3$ | $CH_3$ |
| J-9 | Q-1 ($R_7$=H, $R_8$=H) | H | 6-$OCH_2CH_2Br$ | $OCH_3$ | $CH_3$ |
| J-9 | Q-1 ($R_7$=H, $R_8$=H) | H | 6-$OCH(CH_3)(CH_2Cl)$ | $OCH_3$ | $CH_3$ |
| J-9 | Q-1 ($R_7$=H, $R_8$=H) | H | 5-$CH_2F$ | $OCH_3$ | $CH_3$ |
| J-9 | Q-1 ($R_7$=H, $R_8$=H) | H | 5-$CH_2CH_2Br$ | $OCH_3$ | $CH_3$ |
| J-9 | Q-1 ($R_7$=H, $R_8$=H) | H | 5-$CH(CH_3)(CH_2Cl)$ | $OCH_3$ | $CH_3$ |
| J-9 | Q-1 ($R_7$=H, $R_8$=H) | H | 6-$SCH_2CH_3$ | $OCH_3$ | $CH_3$ |
| J-9 | Q-1 ($R_7$=H, $R_8$=H) | H | 5-$SCH(CH_3)_2$ | $OCH_3$ | $CH_3$ |
| J-9 | Q-1 ($R_7$=H, $R_8$=H) | H | 5-$SCH_2F$ | $OCH_3$ | $CH_3$ |
| J-9 | Q-1 ($R_7$=H, $R_8$=H) | H | 5-$SCH_2CH_2Br$ | $OCH_3$ | $CH_3$ |
| J-9 | Q-1 ($R_7$=H, $R_8$=H) | H | 5-$SCH(CH_3)(CH_2Cl)$ | $OCH_3$ | $CH_3$ |
| J-9 | Q-1 ($R_7$=H, $R_8$=H) | H | 4-$NH_2$ | $OCH_3$ | $CH_3$ |

Table 6 (continued)

| J | Q | R | $R_{1c}$ | $X_2$ | $Y_3$ |
|---|---|---|---|---|---|
| J-9 | Q-1 ($R_7$=H, $R_8$=H) | H | 5-$NHCH_3$ | $OCH_3$ | $CH_3$ |
| J-9 | Q-1 ($R_7$=H, $R_8$=H) | H | 6-$NHCH_2CH_3$ | $OCH_3$ | $CH_3$ |
| J-9 | Q-1 ($R_7$=H, $R_8$=H) | H | 5-$NHCH(CH_3)_2$ | $OCH_3$ | $CH_3$ |
| J-9 | Q-1 ($R_7$=H, $R_8$=H) | H | 4-$N(CH_3)_2$ | $OCH_3$ | $CH_3$ |
| J-9 | Q-1 ($R_7$=H, $R_8$=H) | H | 6-$N(CH_3)(CH_2CH_3)$ | $OCH_3$ | $CH_3$ |
| J-9 | Q-1 ($R_7$=H, $R_8$=H) | H | 5-$N(CH_3)(CH(CH_3)_2)$ | $OCH_3$ | $CH_3$ |
| J-9 | Q-1 ($R_7$=H, $R_8$=H) | H | 5-Br | $OCH_3$ | $CH_3$ |
| J-9 | Q-1 ($R_7$=H, $R_8$=H) | H | 4-F | $OCH_3$ | $CH_3$ |
| J-9 | Q-1 ($R_7$=H, $R_8$=H) | H | 5-I | $OCH_3$ | $CH_3$ |
| J-9 | Q-1 ($R_7$=H, $R_8$=H) | H | 4-$NO_2$ | $OCH_3$ | $CH_3$ |
| J-9 | Q-1 ($R_7$=H, $R_8$=H) | H | 5-$CF_3$ | $OCH_3$ | $CH_3$ |
| J-9 | Q-1 ($R_7$=H, $R_8$=H) | H | 5-$OCF_2H$ | $OCH_3$ | $CH_3$ |
| J-9 | Q-1 ($R_7$=H, $R_8$=$CH_3$) | H | H | $OCH_3$ | $CH_3$ |
| J-9 | Q-1 ($R_7$=H, $R_8$=$CH_3$) | H | H | $CH_3$ | $CH_2CF_3$ |
| J-9 | Q-1 ($R_7$=H, $R_8$=$CH_2CH_3$) | H | H | $CH_3$ | $CH_3$ |
| J-9 | Q-1 ($R_7$=H, $R_8$=$CH(CH_3)_2$) | H | H | $OCH_3$ | $CH_3$ |
| J-9 | Q-1 ($R_7$=H, $R_8$=$\underline{n}$-$C_4H_9$) | H | H | $SCH_3$ | $CH_3$ |
| J-9 | Q-1 ($R_7$=$CH_3$, $R_8$=$CH_3$) | H | H | $OCH_3$ | $CH_3$ |
| J-9 | Q-37 ($R_7$=H, $R_8$=H, $R_2$=H) | H | H | $OCH_3$ | $CH_3$ |
| J-9 | Q-98 ($R_7$=H, $R_8$=H, $R_9$=H) | H | H | $OCH_3$ | $CH_3$ |
| J-9 | Q-105 ($R_7$=H, $R_8$=H, $R_9$=H, $R_2$=H) | H | H | $OCH_3$ | $CH_3$ |
| J-9 | Q-138 ($R_7$=H, $R_8$=H, $R_9$=H) | H | H | $OCH_3$ | $CH_3$ |
| J-9 | Q-143 ($R_7$=H, $R_8$=H, $R_9$=H, $R_3$=H) | H | H | $OCH_3$ | $CH_3$ |

Table 6 (continued)

| J | Q | R | $R_{1d}$ | $X_2$ | $Y_3$ | m.p.(°C) |
|---|---|---|---|---|---|---|
| J-11 | Q-1 ($R_7$=H, $R_8$=H) | H | H | $CH_3$ | $CH_3$ | |
| J-11 | Q-1 ($R_7$=H, $R_8$=H) | H | H | $OCH_3$ | $CH_3$ | |
| J-11 | Q-1 ($R_7$=H, $R_8$=H) | H | H | $SCH_3$ | $CH_3$ | |
| J-11 | Q-1 ($R_7$=H, $R_8$=H) | H | H | $CH_3$ | $CH_2CH_3$ | |
| J-11 | Q-1 ($R_7$=H, $R_8$=H) | H | H | $OCH_3$ | $CH_2CH_3$ | |
| J-11 | Q-1 ($R_7$=H, $R_8$=H) | H | H | $SCH_3$ | $CH_2CH_3$ | |
| J-11 | Q-1 ($R_7$=H, $R_8$=H) | H | H | $CH_3$ | $CH_2CF_3$ | |
| J-11 | Q-1 ($R_7$=H, $R_8$=H) | H | H | $OCH_3$ | $CH_2CF_3$ | |
| J-11 | Q-1 ($R_7$=H, $R_8$=H) | H | H | $SCH_3$ | $CH_2CF_3$ | |
| J-11 | Q-1 ($R_7$=H, $R_8$=H) | $CH_3$ | 5-$CH_3$ | $CH_3$ | $CH_3$ | |
| J-11 | Q-1 ($R_7$=H, $R_8$=H) | $CH_3$ | 6-$CH_2CH_3$ | $OCH_3$ | $CH_3$ | |
| J-11 | Q-1 ($R_7$=H, $R_8$=H) | $CH_3$ | 3-$CH(CH_3)_2$ | $SCH_3$ | $CH_3$ | |
| J-11 | Q-1 ($R_7$=H, $R_8$=H) | H | 5-$OCH_3$ | $OCH_3$ | $CH_3$ | |
| J-11 | Q-1 ($R_7$=H, $R_8$=H) | H | 5-$OCH_2CH_3$ | $OCH_3$ | $CH_3$ | |
| J-11 | Q-1 ($R_7$=H, $R_8$=H) | H | 3-$OCH(CH_3)_2$ | $OCH_3$ | $CH_3$ | |
| J-11 | Q-1 ($R_7$=H, $R_8$=H) | H | 5-$OCF_2H$ | $OCH_3$ | $CH_3$ | |
| J-11 | Q-1 ($R_7$=H, $R_8$=H) | H | 6-$OCH_2CH_2Br$ | $OCH_3$ | $CH_3$ | |
| J-11 | Q-1 ($R_7$=H, $R_8$=H) | H | 6-$OCH(CH_3)(CH_2Cl)$ | $OCH_3$ | $CH_3$ | |
| J-11 | Q-1 ($R_7$=H, $R_8$=H) | H | 5-$CH_2F$ | $OCH_3$ | $CH_3$ | |
| J-11 | Q-1 ($R_7$=H, $R_8$=H) | H | 5-$CH_2CH_2Br$ | $OCH_3$ | $CH_3$ | |
| J-11 | Q-1 ($R_7$=H, $R_8$=H) | H | 5-$CH(CH_3)(CH_2Cl)$ | $OCH_3$ | $CH_3$ | |
| J-11 | Q-1 ($R_7$=H, $R_8$=H) | H | 5-$SCH_3$ | $OCH_3$ | $CH_3$ | |
| J-11 | Q-1 ($R_7$=H, $R_8$=H) | H | 6-$SCH_2CH_3$ | $OCH_3$ | $CH_3$ | |
| J-11 | Q-1 ($R_7$=H, $R_8$=H) | H | 5-$SCH(CH_3)_2$ | $OCH_3$ | $CH_3$ | |
| J-11 | Q-1 ($R_7$=H, $R_8$=H) | H | 5-$SCH_2F$ | $OCH_3$ | $CH_3$ | |
| J-11 | Q-1 ($R_7$=H, $R_8$=H) | H | 5-$SCH_2CH_2Br$ | $OCH_3$ | $CH_3$ | |
| J-11 | Q-1 ($R_7$=H, $R_8$=H) | H | 5-$SCH(CH_3)(CH_2Cl)$ | $OCH_3$ | $CH_3$ | |
| J-11 | Q-1 ($R_7$=H, $R_8$=H) | H | 4-$NH_2$ | $OCH_3$ | $CH_3$ | |
| J-11 | Q-1 ($R_7$=H, $R_8$=H) | H | 5-$NHCH_3$ | $OCH_3$ | $CH_3$ | |
| J-11 | Q-1 ($R_7$=H, $R_8$=H) | H | 6-$NHCH_2CH_3$ | $OCH_3$ | $CH_3$ | |
| J-11 | Q-1 ($R_7$=H, $R_8$=H) | H | 3-$NHCH(CH_3)_2$ | $OCH_3$ | $CH_3$ | |

Table 6 (continued)

| J | Q | R | $R_{1d}$ | $X_2$ | $Y_3$ | m.p. (°C) |
|---|---|---|---|---|---|---|
| J-11 | Q-1 ($R_7$=H, $R_8$=H) | H | 4-$N(CH_3)_2$ | $OCH_3$ | $CH_3$ | |
| J-11 | Q-1 ($R_7$=H, $R_8$=H) | H | 6-$N(CH_3)(CH_2CH_3)$ | $OCH_3$ | $CH_3$ | |
| J-11 | Q-1 ($R_7$=H, $R_8$=H) | H | 5-$N(CH_3)(CH(CH_3)_2)$ | $OCH_3$ | $CH_3$ | |
| J-11 | Q-1 ($R_7$=H, $R_8$=H) | H | 5-Cl | $OCH_3$ | $CH_3$ | |
| J-11 | Q-1 ($R_7$=H, $R_8$=H) | H | 3-Br | $OCH_3$ | $CH_3$ | |
| J-11 | Q-1 ($R_7$=H, $R_8$=H) | H | 4-F | $OCH_3$ | $CH_3$ | |
| J-11 | Q-1 ($R_7$=H, $R_8$=H) | H | 5-I | $OCH_3$ | $CH_3$ | |
| J-11 | Q-1 ($R_7$=H, $R_8$=H) | H | 4-$NO_2$ | $OCH_3$ | $CH_3$ | |
| J-11 | Q-1 ($R_7$=H, $R_8$=H) | H | 5-$CF_3$ | $OCH_3$ | $CH_3$ | |
| J-11 | Q-1 ($R_7$=H, $R_8$=H) | H | 5-$OCF_2H$ | $OCH_3$ | $CH_3$ | |
| J-11 | Q-1 ($R_7$=H, $R_8$=H) | H | 6-$SO_2N(CH_3)_2$ | $OCH_3$ | $CH_3$ | |
| J-11 | Q-1 ($R_7$=H, $R_8$=H) | H | 6-$SOCH_3$ | $OCH_3$ | $CH_3$ | |
| J-11 | Q-1 ($R_7$=H, $R_8$=H) | H | 6-$SO_2CH(CH_3)_2$ | $OCH_3$ | $CH_3$ | |
| J-11 | Q-1 ($R_7$=H, $R_8$=H) | H | 6-$CO_2CH_3$ | $OCH_3$ | $CH_3$ | |
| J-11 | Q-1 ($R_7$=H, $R_8$=$CH_3$) | H | H | $OCH_3$ | $CH_3$ | |
| J-11 | Q-1 ($R_7$=H, $R_8$=$CH_3$) | H | H | $CH_3$ | $CH_2CF_3$ | |
| J-11 | Q-1 ($R_7$=H, $R_8$=$CH_2CH_3$) | H | H | $CH_3$ | $CH_3$ | |
| J-11 | Q-1 ($R_7$=H, $R_8$=$CH(CH_3)_2$) | H | H | $OCH_3$ | $CH_3$ | |
| J-11 | Q-1 ($R_7$=H, $R_8$=n-$C_4H_9$) | H | H | $SCH_3$ | $CH_3$ | |
| J-11 | Q-1 ($R_7$=$CH_3$, $R_8$=$CH_3$) | H | H | $OCH_3$ | $CH_3$ | |
| J-11 | Q-37 ($R_7$=H, $R_8$=H, $R_2$=H) | H | H | $OCH_3$ | $CH_3$ | |
| J-11 | Q-37 ($R_7$=H, $R_8$=H, $R_2$=i-$C_3H_7$) | H | H | $OCH_3$ | $CH_3$ | |
| J-11 | Q-37 ($R_7$=H, $R_8$=H, $R_2$=n-$C_4H_9$) | H | 5-$OCH_3$ | $CH_3$ | $CH_2CF_3$ | |
| J-11 | Q-98 ($R_7$=H, $R_8$=H, $R_9$=H) | H | H | $OCH_3$ | $CH_3$ | |
| J-11 | Q-98 ($R_7$=$CH_3$, $R_8$=H, $R_9$=H) | H | H | $OCH_3$ | $CH_3$ | |
| J-11 | Q-98 ($R_7$=H, $R_8$=H, $R_9$=i-$C_3H_7$) | H | 5-$OCH_3$ | $CH_3$ | $CH_2CF_3$ | |
| J-11 | Q-105 ($R_7$=H, $R_8$=H, $R_9$=H, $R_2$=H) | H | H | $OCH_3$ | $CH_3$ | |
| J-11 | Q-105 ($R_7$=$CH_3$,$R_8$=H, $R_9$=H, $R_2$=$CH_3$) | H | H | $OCH_3$ | $CH_3$ | |
| J-11 | Q-105 ($R_7$=H,$R_8$=$CH_3$,$R_9$=H, $R_2$=$C_2H_5$) | H | 5-$OCH_3$ | $CH_3$ | $CH_2CF_3$ | |

Table 6 (continued)

| J | Q | R | $R_{1d}$ | $X_2$ | $Y_3$ | m.p. (°C) |
|---|---|---|---|---|---|---|
| J-11 | Q-138 ($R_7$=H, $R_8$=H, $R_9$=H) | H | H | $OCH_3$ | $CH_3$ | |
| J-11 | Q-138 ($R_7$=$CH_3$, $R_8$=H, $R_9$=H) | H | H | $OCH_3$ | $CH_3$ | |
| J-11 | Q-138 ($R_7$=H, $R_8$=H, $R_9$=$CH_3$) | H | H | $OCH_3$ | $CH_3$ | |
| J-11 | Q-143 ($R_7$=H, $R_8$=H, $R_9$=H, $R_3$=$CH_3$) | H | H | $OCH_3$ | $CH_3$ | |
| J-11 | Q-143 ($R_7$=H, $R_8$=H, $R_9$=H, $R_3$=H) | H | H | $OCH_3$ | $CH_3$ | |
| J-11 | Q-143 ($R_7$=$CH_3$,$R_8$=H,$R_9$=$CH_3$,$R_3$=$CH_3$) | H | 5-Cl | $CH_3$ | $CH_3$ | |

TABLE 7
General Formula 7

| J | $W_3$ | Q | R | $R_{1b}$ | $X_3$ |
|---|---|---|---|---|---|
| J-5 | S | Q-1 ($R_7$=H, $R_8$=H) | H | H | $CH_3$ |
| J-6 | S | Q-1 ($R_7$=H, $R_8$=H) | H | H | $OCH_3$ |
| J-5 | S | Q-1 ($R_7$=H, $R_8$=H) | $CH_3$ | H | $OCH_3$ |
| J-6 | S | Q-1 ($R_7$=H, $R_8$=$CH_3$) | H | H | $CH_3$ |
| J-5 | S | Q-1 ($R_7$=H, $R_8$=H) | H | 5-$CH_3$ | $OCH_3$ |
| J-6 | S | Q-2 ($R_7$=H, $R_8$=H) | H | H | $OCH_3$ |
| J-5 | S | Q-3 ($R_7$=$C_2H_5$, $R_8$=H) | H | H | $OCH_3$ |
| J-6 | S | Q-4 ($R_3$=$CH_3$, $R_7$=H, $R_8$=H ) | H | H | $OCH_3$ |
| J-5 | S | Q-5 ($R_3$=H, $R_7$=H, $R_8$=H ) | H | H | $OCH_3$ |
| J-7 | S | Q-8 ($R_2$=H, $R_7$=H, $R_8$=H) | H | H | $OCH_3$ |
| J-5 | S | Q-9 ($R_2$=H, $R_7$=H, $R_8$=H) | H | 5-$CH_2F$ | $OCH_3$ |
| J-6 | S | Q-9 ($R_2$=H, $R_7$=H, $R_8$=H) | H | H | $OCH_3$ |
| J-5 | S | Q-11 ($R_7$=H, $R_8$=H) | H | 5-$CH_2CH_2Br$ | $OCH_3$ |
| J-6 | S | Q-11 ($R_7$=H, $R_8$=H) | H | H | $OCH_3$ |
| J-5 | S | Q-13 ($R_3$=$CH_3$, $R_7$=H, $R_8$=H) | H | 5-$CH(CH_3)(CH_2Cl)$ | $OCH_3$ |
| J-6 | S | Q-13 ($R_3$=$CH_3$, $R_7$=H, $R_8$=H) | H | H | $OCH_3$ |
| J-5 | NH | Q-14 ($R_3$=$CH_3$, $R_7$=H, $R_8$=H) | H | H | $OCH_3$ |
| J-5 | O | Q-17 ($R_2$=H, $R_7$=H, $R_8$=H) | H | H | $OCH_3$ |
| J-6 | S | Q-18 ($R_2$=H, $R_7$=H, $R_8$=H) | H | H | $OCH_3$ |
| J-7 | S | Q-29 ($R_7$=H, $R_8$=H) | H | H | $OCH_3$ |
| J-5 | S | Q-30 ($R_7$=H, $R_8$=H) | H | H | $OCH_3$ |
| J-6 | S | Q-32 ($R_3$=$CH_3$, $R_7$=H, $R_8$=H) | H | 4-$NH_2$ | $OCH_3$ |
| J-5 | S | Q-32 ($R_3$=$CH_3$, $R_7$=H, $R_8$=H) | H | H | $OCH_3$ |
| J-6 | S | Q-36 ($R_2$=H, $R_7$=H, $R_8$=H) | H | H | $OCH_3$ |
| J-5 | S | Q-51 ($R_7$=H, $R_8$=H, $R_9$=H) | H | H | $OCH_3$ |
| J-6 | S | Q-52 ($R_7$=H, $R_8$=H, $R_9$=H) | H | H | $OCH_3$ |
| J-5 | S | Q-55 ($R_3$=$CH_3$, $R_7$=H, $R_8$=H, $R_9$=H) | H | 4-$N(CH_3)_2$ | $OCH_3$ |
| J-6 | O | Q-55 ($R_3$=$CH_3$, $R_7$=H, $R_8$=H, $R_9$=H) | H | H | $OCH_3$ |
| J-6 | S | Q-60 ($R_2$=H, $R_7$=H, $R_8$=H, $R_9$=H) | H | H | $OCH_3$ |

Table 7 (continued)

| J | $W_3$ | Q | R | $R_{1b}$ | $X_3$ |
|---|---|---|---|---|---|
| J-7 | S | Q-69 ($R_3$=$CH_3$, $R_7$=H, $R_8$=H, $R_9$=H) | H | 5-Cl | $OCH_3$ |
| J-5 | S | Q-69 ($R_3$=$CH_3$, $R_7$=H, $R_8$=H, $R_9$=H) | H | H | $OCH_3$ |
| J-6 | S | Q-70 ($R_3$=$CH_3$, $R_7$=H, $R_8$=H, $R_9$=H) | H | H | $OCH_3$ |
| J-5 | S | Q-72 ($R_2$=H, $R_7$=H, $R_8$=H, $R_9$=H) | H | 5-I | $OCH_3$ |
| J-6 | S | Q-72 ($R_2$=H, $R_7$=H, $R_8$=H, $R_9$=H) | H | H | $OCH_3$ |
| J-5 | O | Q-74 ($R_2$=H, $R_7$=H, $R_8$=H, $R_9$=H) | H | 5-$NO_2$ | $OCH_3$ |
| J-6 | S | Q-74 ($R_2$=H, $R_7$=H, $R_8$=H, $R_9$=H) | H | H | $OCH_3$ |
| J-5 | S | Q-95 ($R_7$=H, $R_8$=H, $R_9$=H) | H | 5-$CF_3$ | $OCH_3$ |
| J-6 | S | Q-95 ($R_7$=H, $R_8$=H, $R_9$=H) | H | H | $OCH_3$ |
| J-7 | S | Q-104 ($R_2$=H, $R_7$=H, $R_8$=H, $R_9$=H) | H | H | $OCH_3$ |

Table 7 (continued)

| J | Q | R | $R_{1c}$ | $X_3$ |
|---|---|---|---|---|
| J-9 | Q-1 ($R_7$=H, $R_8$=H) | H | H | $CH_3$ |
| J-9 | Q-2 ($R_7$=H, $R_8$=H) | H | H | $OCH_3$ |
| J-9 | Q-3 ($R_7$=H, $R_8$=H) | H | H | $CH_3$ |
| J-9 | Q-4 ($R_7$=H, $R_8$=H, $R_3$=$CH_3$) | H | H | $OCH_3$ |
| J-9 | Q-5 ($R_7$-H, $R_8$=H, $R_3$=H) | H | H | $CH_3$ |
| J-9 | Q-6 ($R_7$=$CH_3$, $R_8$=H, $R_3$=$CH_3$) | H | H | $OCH_3$ |
| J-9 | Q-8 ($R_7$=H, $R_8$=H, $R_2$=H) | H | H | $OCH_3$ |
| J-9 | Q-9 ($R_7$=H, $R_8$=H, $R_2$=H) | H | H | $CH_3$ |
| J-9 | Q-13 ($R_7$=H, $R_8$=H, $R_3$=H) | H | H | $OCH_3$ |
| J-9 | Q-14 ($R_7$=H, $R_8$=H, $R_3$=$CH_3$) | H | H | $CH_3$ |
| J-9 | Q-15 ($R_7$=H, $R_8$=H, $R_3$=H) | H | H | $OCH_3$ |
| J-9 | Q-29 ($R_7$=H, $R_8$=H) | H | H | $OCH_3$ |
| J-9 | Q-30 ($R_7$=H, $R_8$=H) | H | H | $CH_3$ |
| J-9 | Q-31 ($R_7$=H, $R_8$=H) | H | H | $OCH_3$ |
| J-9 | Q-32 ($R_7$=H, $R_8$=H, $R_3$=H) | H | H | $CH_3$ |
| J-9 | Q-33 ($R_7$=H, $R_8$=H, $R_8$=$CH_3$) | H | H | $OCH_3$ |
| J-9 | Q-34 ($R_7$=H, $R_8$=H, $R_3$=H) | H | H | $CH_3$ |
| J-9 | Q-36 ($R_7$=H, $R_8$=H, $R_2$=H) | H | H | $CH_3$ |
| J-9 | Q-37 ($R_7$=H, $R_8$=H, $R_2$=H) | H | H | $OCH_3$ |
| J-9 | Q-38 ($R_7$=H, $R_8$=H, $R_2$=$OCF_2H$) | H | H | $CH_3$ |
| J-9 | Q-39 ($R_7$=H, $R_8$=H, $R_9$=H) | H | H | $OCH_3$ |
| J-9 | Q-43 ($R_7$=H, $R_8$=H, $R_3$=H) | H | H | $CH_3$ |
| J-9 | Q-44 ($R_7$=H, $R_8$=H, $R_3$=$CH_3$) | H | H | $OCH_3$ |
| J-9 | Q-45 ($R_7$=H, $R_8$=H, $R_3$=H) | H | H | $CH_3$ |

Table 7 (continued)

| J | Q | R | $R_{1c}$ | $X_3$ |
|---|---|---|---|---|
| J-9 | Q-47 ($R_7$=H, $R_8$=H, $R_2$=H) | H | H | $CH_3$ |
| J-9 | Q-51 ($R_7$=H, $R_8$=H, $R_9$=H) | H | H | $OCH_3$ |
| J-9 | Q-52 ($R_7$=H, $R_8$=H, $R_9$=H) | H | H | $CH_3$ |
| J-9 | Q-53 ($R_7$=H, $R_8$=H, $R_9$=H) | H | H | $OCH_3$ |
| J-9 | Q-54 (R =H, R =H, R =H) | H | H | $CH_3$ |
| J-9 | Q-55 ($R_7$=H, $R_8$=H, $R_9$=H, $R_3$=H) | H | H | $OCH_3$ |
| J-9 | Q-56 ($R_7$=H, $R_8$=H, $R_9$=H, $R_3$=$C_2H_5$) | H | H | $CH_3$ |
| J-9 | Q-57 ($R_7$=H, $R_8$=H, $R_9$=H, $R_3$=i-$C_3H_7$) | H | H | $OCH_3$ |
| J-9 | Q-58 ($R_7$=H, $R_8$=H, $R_9$=H, $R_3$=H) | H | H | $CH_3$ |
| J-9 | Q-95 ($R_7$=H, $R_8$=H, $R_9$=H) | H | H | $CH_3$ |
| J-9 | Q-96 ($R_7$=H, $R_8$=H, $R_9$=H) | H | H | $OCH_3$ |
| J-9 | Q-97 ($R_7$=H, $R_8$=H, $R_9$=H) | H | H | $CH_3$ |
| J-9 | Q-98 ($R_7$=H, $R_8$=H, $R_9$=H) | H | H | $OCH_3$ |
| J-9 | Q-99 ($R_7$=H, $R_8$=H, $R_9$=H, $R_3$=H) | H | H | $CH_3$ |
| J-9 | Q-100 ($R_7$=H, $R_8$=H, $R_9$=H, $R_3$=$C_2H_5$) | H | H | $OCH_3$ |
| J-9 | Q-101 ($R_7$=H, $R_8$=H, $R_9$=H, $R_3$=i-$C_3H_7$) | H | H | $CH_3$ |
| J-9 | Q-102 ($R_7$=H, $R_8$=H, $R_9$=H, $R_3$=H) | H | H | $OCH_3$ |

Table 7 (continued)

| J | Q | R | $R_{1d}$ | $X_3$ | m.p.(°C) |
|---|---|---|---|---|---|
| J-11 | Q-1 ($R_7$=H, $R_8$=H) | H | H | $CH_3$ | |
| J-11 | Q-1 ($R_7$=H, $R_8$=H) | H | H | $OCH_3$ | |
| J-11 | Q-1 ($R_7$=H, $R_8$=H) | $CH_3$ | H | $OCH_3$ | |
| J-11 | Q-1 ($R_7$=H, $R_8$=$CH_3$) | H | H | $CH_3$ | |
| J-11 | Q-1 ($R_7$=H, $R_8$=H) | H | 5-$CH_3$ | $OCH_3$ | |
| J-11 | Q-1 ($R_7$=H, $R_8$=H) | H | 6-$SO_2N(CH_3)_2$ | $OCH_3$ | |
| J-11 | Q-1 ($R_7$=H, $R_8$=H) | H | 6-$SOCH_3$ | $OCH_3$ | |
| J-11 | Q-1 ($R_7$=H, $R_8$=H) | H | 6-$SO_2CH(CH_3)_2$ | $OCH_3$ | |
| J-11 | Q-1 ($R_7$=H, $R_8$=H) | H | 6-$CO_2CH_3$ | $OCH_3$ | |
| J-11 | Q-2 ($R_7$=H, $R_8$=H) | H | 6$CH_2CH_3$ | $OCH_3$ | |
| J-11 | Q-2 ($R_7$=H, $R_8$=H) | H | 3-$CH(CH_3)_2$ | $OCH_3$ | |
| J-11 | Q-2 ($R_7$=H, $R_8$=H) | H | H | $OCH_3$ | |
| J-11 | Q-3 ($R_7$=H, $R_8$=H) | H | 5-$OCH_3$ | $OCH_3$ | |
| J-11 | Q-3 ($R_7$=$C_2H_5$, $R_8$=H) | H | 5-$OCH_2CH_3$ | $OCH_3$ | |
| J-11 | Q-3 ($R_7$=$C_2H_5$, $R_8$=H) | H | H | $OCH_3$ | |
| J-11 | Q-4 ($R_3$=$CH_3$, $R_7$=H, $R_8$=H ) | H | 3-$OCH(CH_3)_2$ | $OCH_3$ | |
| J-11 | Q-4 ($R_3$=$CH_3$, $R_7$=H, $R_8$=H ) | H | H | $OCH_3$ | |
| J-11 | Q-5 ($R_3$=H, $R_7$=H, $R_8$=H ) | H | 5-$OCF_2H$ | $OCH_3$ | |
| J-11 | Q-5 ($R_3$=H, $R_7$=H, $R_8$=H ) | H | H | $OCH_3$ | |
| J-11 | Q-8 ($R_2$=H, $R_7$=H, $R_8$=H) | H | 5-$OCH(CH_3)(CH_2Cl)$ | $OCH_3$ | |
| J-11 | Q-8 ($R_2$=H, $R_7$=H, $R_8$=H) | H | H | $OCH_3$ | |
| J-11 | Q-9 ($R_2$=H, $R_7$=H, $R_8$=H) | H | 5-$CH_2F$ | $OCH_3$ | |
| J-11 | Q-9 ($R_2$=H, $R_7$=H, $R_8$=H) | H | H | $OCH_3$ | |
| J-11 | Q-11 ($R_7$=H, $R_8$=H) | H | 5-$CH_2CH_2Br$ | $OCH_3$ | |
| J-11 | Q-11 ($R_7$=H, $R_8$=H) | H | H | $OCH_3$ | |
| J-11 | Q-13 ($R_3$=$CH_3$, $R_7$=H, $R_8$=H) | H | 5-$CH(CH_3)(CH_2Cl)$ | $OCH_3$ | |
| J-11 | Q-13 ($R_3$=$CH_3$, $R_7$=H, $R_8$=H) | H | H | $OCH_3$ | |
| J-11 | Q-14 ($R_3$=$CH_3$, $R_7$=H, $R_8$=H) | H | 5-$SCH_3$ | $OCH_3$ | |
| J-11 | Q-14 ($R_3$=$CH_3$, $R_7$=H, $R_8$=H) | H | H | $OCH_3$ | |
| J-11 | Q-17 ($R_2$=H, $R_7$=H, $R_8$=H) | H | 5-$SCH(CH_3)_2$ | $OCH_3$ | |
| J-11 | Q-17 ($R_2$=H, $R_7$=H, $R_8$=H) | H | H | $OCH_3$ | |
| J-11 | Q-18 ($R_2$=H, $R_7$=H, $R_8$=H) | H | 5-$SCH_2F$ | $OCH_3$ | |
| J-11 | Q-18 ($R_2$=H, $R_7$=H, $R_8$=H) | H | H | $OCH_3$ | |

Table 7 (Continued)

| J | Q | R | $R_{1d}$ | $X_3$ | m.p.(°C) |
|---|---|---|---|---|---|
| J-11 | Q-29 ($R_7$=H, $R_8$=H) | H | 5-$SCH_2CH_2Br$ | $OCH_3$ | |
| J-11 | Q-29 ($R_7$=H, $R_8$=H) | H | H | $OCH_3$ | |
| J-11 | Q-30 ($R_7$=H, $R_8$=H) | H | 5-$SCH(CH_3)(CH_2Cl)$ | $OCH_3$ | |
| J-11 | Q-30 ($R_7$=H, $R_8$=H) | H | H | $OCH_3$ | |
| J-11 | Q-32 ($R_3$=$CH_3$, $R_7$=H, $R_8$=H) | H | 4-$NH_2$ | $OCH_3$ | |
| J-11 | Q-32 ($R_3$=$CH_3$, $R_7$=H, $R_8$=H) | H | H | $OCH_3$ | |
| J-11 | Q-36 ($R_2$=H, $R_7$=H, $R_8$=H) | H | 5-$NHCH_3$ | $OCH_3$ | |
| J-11 | Q-36 ($R_2$=H, $R_7$=H, $R_8$=H) | H | H | $OCH_3$ | |
| J-11 | Q-51 ($R_7$=H, $R_8$=H, $R_9$=H) | H | 6-$NHCH_2CH_3$ | $OCH_3$ | |
| J-11 | Q-51 ($R_7$=H, $R_8$=H, $R_9$=H) | H | H | $OCH_3$ | |
| J-11 | Q-52 ($R_7$=H, $R_8$=H, $R_9$=H) | H | 3-$NHCH(CH_3)_2$ | $OCH_3$ | |
| J-11 | Q-52 ($R_7$=H, $R_8$=H, $R_9$=H) | H | H | $OCH_3$ | |
| J-11 | Q-55 ($R_3$=$CH_3$, $R_7$=H, $R_8$=H, $R_9$=H) | H | 4$N(CH_3)_2$ | $OCH_3$ | |
| J-11 | Q-55 ($R_3$=$CH_3$, $R_7$=H, $R_8$=H, $R_9$=H) | H | H | $OCH_3$ | |
| J-11 | Q-60 ($R_2$=H, $R_7$=H, $R_8$=H, $R_9$=H) | H | 5-$N(CH_3)(CH(CH_3)_2)$ | $OCH_3$ | |
| J-11 | Q-60 ($R_2$=H, $R_7$=H, $R_8$=H, $R_9$=H) | H | H | $OCH_3$ | |
| J-11 | Q-69 ($R_3$=$CH_3$, $R_7$=H, $R_8$=H, $R_9$=H) | H | 5-Cl | $OCH_3$ | |
| J-11 | Q-69 ($R_3$=$CH_3$, $R_7$=H, $R_8$=H, $R_9$=H) | H | H | $OCH_3$ | |
| J-11 | Q-70 ($R_3$=$CH_3$, $R_7$=H, $R_8$=H, $R_9$=H) | H | 3-Br | $OCH_3$ | |
| J-11 | Q-70 ($R_3$=$CH_3$, $R_7$=H, $R_8$=H, $R_9$=H) | H | H | $OCH_3$ | |
| J-11 | Q-72 ($R_2$=H, $R_7$=H, $R_8$=H, $R_9$=H) | H | 5-I | $OCH_3$ | |
| J-11 | Q-72 ($R_2$=H, $R_7$=H, $R_8$=H, $R_9$=H) | H | H | $OCH_3$ | |
| J-11 | Q-74 ($R_2$=H, $R_7$=H, $R_8$=H, $R_9$=H) | H | 5-$NO_2$ | $OCH_3$ | |
| J-11 | Q-74 ($R_2$=H, $R_7$=H, $R_8$=H, $R_9$=H) | H | H | $OCH_3$ | |
| J-11 | Q-95 ($R_7$=H, $R_8$=H, $R_9$=H) | H | 5-$CF_3$ | $OCH_3$ | |
| J-11 | Q-95 ($R_7$=H, $R_8$=H, $R_9$=H) | H | H | $OCH_3$ | |
| J-11 | Q-104 ($R_2$=H, $R_7$=H, $R_8$=H, $R_9$=H) | H | 5-$OCF_2H$ | $OCH_3$ | |
| J-11 | Q-104 ($R_2$=H, $R_7$=H, $R_8$=H, $R_9$=H) | H | H | $OCH_3$ | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

| | Active Ingredient | Weight Percent* Diluent(s) | Surfactant(s) |
|---|---|---|---|
| Wettable Powders | 20—90 | 0—74 | 1—10 |
| Oil Suspensions, Emulsions, Solutions, (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

* Active ingredient plus at least one of a Surfactant or a Diluent equals 100 weight per cent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley and Sons, Inc., New York, 1961, pp. 81—96; and

J. D. Fryer and S. A. Evans, "Weed Control Handbook", 5th Ed., Blackwell, Scientific Publications, Oxford, 1968, pp. 101—103.

In the following examples, all parts are by weight unless otherwise indicated.

### Example 13

Wettable Powder

| | |
|---|---|
| N-[(4,6-dimethoxypyrimidin-2-yl)aminocarbonyl]-2-(tetrahydro-2-oxo-3-furanyl)benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 14

Wettable Powder

| | |
|---|---|
| N-[(4-methoxy-6-methyl-1,3,5-triazin-2-yl)-aminocarbonyl]-2-(tetrahydro-2-oxo-3-furanyl)-benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 15

Granule

| | |
|---|---|
| Wettable Powder of Example 14 | 5% |
| attapulgite granules (U.S.S. 20—40 mesh; 0.84—0.42 mm) | 95% |

A slurry of wettable powder containing 25% solids is sprayed on the surface of attapulgite granules while tumbling in a double-cone blender. The granules are dried and packaged.

Example 16

Extruded Pellet

| | |
|---|---|
| N-[(4-methoxy-6-methyl-1,3,5-triazin-2-yl)amino-carbonyl]-2-(tetrahydro-2-oxo-3-furanyl)-benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 17

Oil Suspension

| | |
|---|---|
| N-[(4,6-dimethoxypyrimidin-2-yl)aminocarbonyl]-2-(tetrahydro-2-oxo-3-furanyl)benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 18

Wettable Powder

| | |
|---|---|
| N-[(4,6-dimethoxypyrimidin-2-yl)aminocarbonyl]-2-(tetrahydro-2-oxo-3-furanyl)benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 19

Low Strength Granule

| | |
|---|---|
| N-[(4,6-dimethoxypyrimidin-2-yl)aminocarbonyl]-2-(tetrahydro-2-oxo-3-furanyl)benzenesulfonamide | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 20

Aqueous Suspension

| | |
|---|---|
| N-[(4,6-dimethoxypyrimidin-2-yl)aminocarbonyl]-2-(tetrahydro-2-oxo-3-furanyl)benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 21

Solution

| | |
|---|---|
| N-[(4,6-dimethoxypyrimidin-2-yl)aminocarbonyl]-2-(tetrahydro-2-oxo-3-furanyl)benzenesulfonamide, sodium salt | 5% |
| water | 95% |

The salt is added directly to the water with stirring to produce the solution, which may then be packaged for use.

Example 22

Low Strength Granule

| | |
|---|---|
| N-[(4-methoxy-6-methyl-1,3,5-triazin-2-yl)amino-carbonyl]-2-(tetrahydro-2-oxo-3-furanyl)-benzenesulfonamide | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 23

Granule

| | |
|---|---|
| N-[(4,6-dimethoxypyrimidin-2-yl)aminocarbonyl]-2-(tetrahydro-2-oxo-3-furanyl)-benzenesulfonamide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is prayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 24

High Strength Concentrate

| | |
|---|---|
| N-[(4-methoxy-6-methyl-1,3,5-triazin-2-yl)amino-carbonyl]-2-(tetrahydro-2-oxo-3-furanyl)-benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 25

Wettable Powder

| | |
|---|---|
| N-[(4,6-dimethoxypyrimidin-2-yl)aminocarbonyl]-2-(tetrahydro-2-oxo-3-furanyl)benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 26

Wettable Powder

| | |
|---|---|
| N-[(4-methoxy-6-methyl-1,3,5-triazin-2-yl)amino-carbonyl]-2-(tetrahydro-2-oxo-3-furanyl)-benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 27

Oil Suspension

| | |
|---|---|
| N-[(4,6-dimethoxypyrimidin-2-yl)aminocarbonyl]-2-(tetrahydro-2-oxo-3-furanyl)benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 28

Dust

| | |
|---|---|
| N-[(4,6-dimethoxypyrimidin-2-yl)aminocarbonyl]-2-(tetrahydro-2-oxo-3-furanyl)benzenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 29

Emulsifiable Concentrate

| | |
|---|---|
| N-[(4-methoxy-6-methyl-1,3,5-triazin-2-yl)-aminocarbonyl]-2-(tetrahydro-2-oxo-3-furanyl)-benzenesulfonamide | 20% |
| chlorobenzene | 74% |
| sorbitan monostearate and polyoxyethylene condensates thereof | 6% |

The ingredients are combined and stirred to produce a solution which can be emulsified in water for application.

Utility

Test results indicate that the compounds of the present invention are highly active preemergent or postemergent herbicides or plant growth regulants. Many of them have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Some of the compounds may be used as selective herbicides in such crops as soybeans. Alternatively, the subject compounds are useful to modify plant growth.

The rates of application for the compounds of the invention are determined by a number of factors, including their use as plant growth modifiers or as herbicides, the crop species involved, the types of weeds to be controlled, weather and climate, formulations selected, mode of application, amount of foliage present, etc. In general terms, the subject compounds should be applied at levels of around 0.01 to 10 kg/ha, the lower rates being suggested for use on lighter soils and/or those having a low organic matter content, for plant growth modification or for situations where only short-term persistence is required.

The compounds of the invention may be used in combination with any other commercial herbicide, examples of which are those of the triazine, triazole, uracil, urea, amide, diphenylether, carbamate and bipyridylium types.

The herbicidal properties of the subject compounds were discovered in a number of greenhouse tests. The test procedures and results follow.

Test A

Seeds of crabgrass (*Digitaria* spp.), barnyardgrass (*Echinochloa crusgalli*), wild oats (*Avena fatua*), cheatgrass (*Bromus secalinus*), velvetleaf (*Abutilon theophrasti*), morningglory (*Ipomoea* spp.), cocklebur (*Xanthium pensylvanicum*), sorghum, corn, soybena, sugar beet, cotton, rice, wheat, and purple nutsedge (*Cyperus rotundus*) tubers were planted and treated preemergence with the test chemicals dissolved in a non-phytotoxic solvent. At the same time, these crop and weed species were treated with a soil/foilage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to & = complete kill. The accompanying descriptive symbols have the following meanings:

C = chlorosis/necrosis;
B = burn;
D = defoliation;
E = emergence inhibition;
G = growth retardation;
H = formative effect;
U = unusual pigmentation;
X = axillary stimulation;
S = albinism; and
6Y = abscised buds or flowers.

Compounds

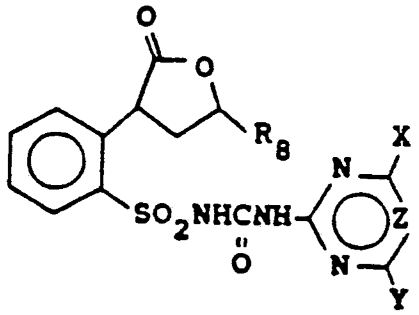

| Compound No. | $R_8$ | X | Y | Z |
|---|---|---|---|---|
| 1 | H | $OCH_3$ | $OCH_3$ | CH |
| 2 | H | $OCH_3$ | $CH_3$ | CH |
| 3 | H | $CH_3$ | $CH_3$ | CH |
| 4 | H | $OCH_3$ | $OCH_3$ | N |
| 5 | H | $OCH_3$ | $CH_3$ | N |
| 6 | $CH_3$ | $OCH_3$ | $OCH_3$ | CH |
| 7 | $CH_3$ | $CH_3$ | $CH_3$ | CH |
| 8 | $CH_3$ | $OCH_3$ | $OCH_3$ | N |
| 9 | $CH_3$ | $OCH_3$ | $CH_3$ | N |
| 10 | $CH_3$ | $OCH_3$ | $CH_3$ | CH |
| 11 | H | Cl | $OCH_3$ | CH |
| 12 | H | $OCF_2H$ | $OCH_3$ | CH |

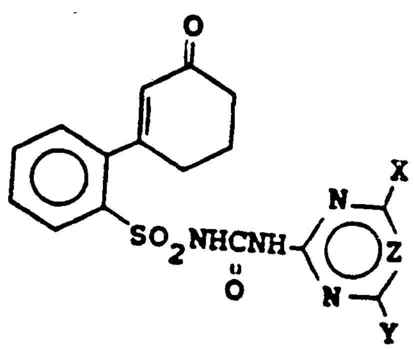

| Compound No. | X | Y | Z |
|---|---|---|---|
| 13 | $OCH_3$ | $OCH_3$ | CH |
| 14 | $OCH_3$ | $OCH_3$ | N |
| 15 | $OCH_3$ | $CH_3$ | CH |
| 16 | Cl | $OCH_3$ | CH |
| 17 | $CH_3$ | $CH_3$ | CH |
| 18 | $OCH_3$ | $CH_3$ | N |

Compounds (continued)

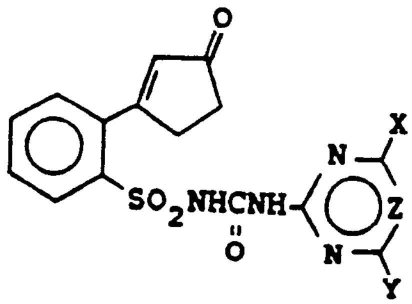

| Compound No. | X | Y | Z |
|---|---|---|---|
| 19 | $OCH_3$ | $OCH_3$ | CH |
| 20 | $OCH_3$ | $OCH_3$ | N |
| 21 | Cl | $OCH_3$ | CH |
| 22 | $CH_3$ | $CH_3$ | CH |
| 23 | $OCH_3$ | $CH_3$ | N |
| 24 | $OCH_3$ | $CH_3$ | CH |

Compound 25

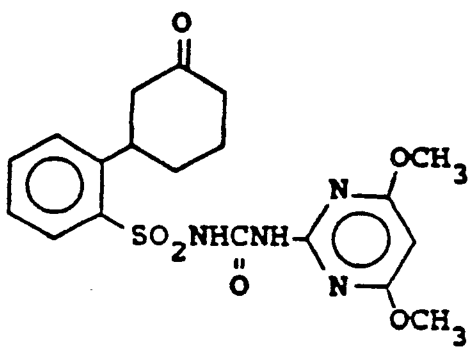

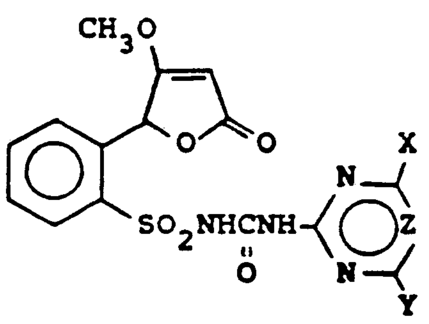

| Compound No. | X | Y | Z |
|---|---|---|---|
| 26 | $OCH_3$ | $OCH_3$ | CH |
| 27 | $OCH_3$ | $CH_3$ | CH |
| 28 | Cl | $OCH_3$ | CH |
| 29 | $CH_3$ | $CH_3$ | CH |

Compounds (continued)

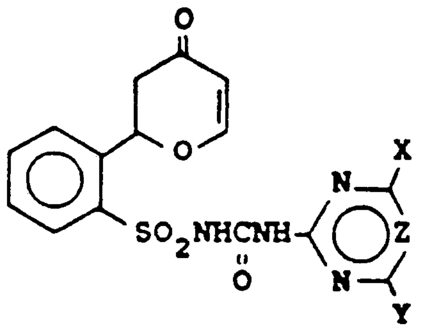

| Compound No. | X | Y | Z |
|---|---|---|---|
| 30 | $OCH_3$ | $OCH_3$ | CH |
| 31 | $OCH_3$ | $CH_3$ | CH |
| 32 | Cl | $OCH_3$ | CH |

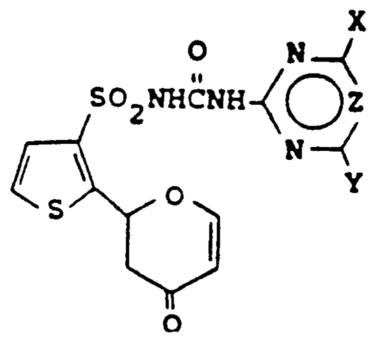

| Compound No. | X | Y | Z |
|---|---|---|---|
| 33 | $OCH_3$ | $CH_3$ | CH |
| 34 | $OCH_3$ | $OCH_3$ | CH |
| 35 | $OCH_3$ | $OCH_3$ | N |

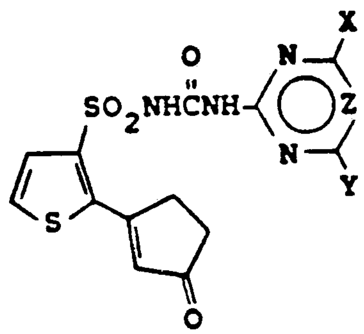

| Compound No. | X | Y | Z |
|---|---|---|---|
| 36 | $CH_3$ | $CH_3$ | CH |
| 37 | $OCH_3$ | $CH_3$ | CH |
| 38 | $OCH_3$ | $OCH_3$ | CH |

Compounds (continued)

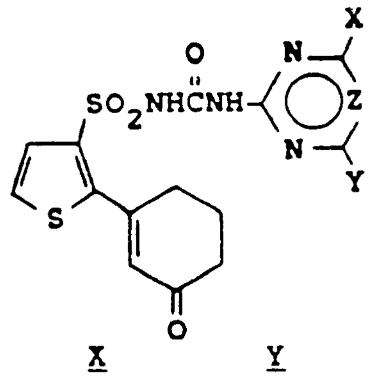

| Compound No. | X | Y | Z |
|---|---|---|---|
| 39 | $CH_3$ | $CH_3$ | CH |
| 40 | $OCH_3$ | $CH_3$ | CH |
| 41 | $OCH_3$ | $OCH_3$ | CH |
| 42 | $OCH_3$ | $OCH_3$ | N |
| 43 | Cl | $OCH_3$ | CH |

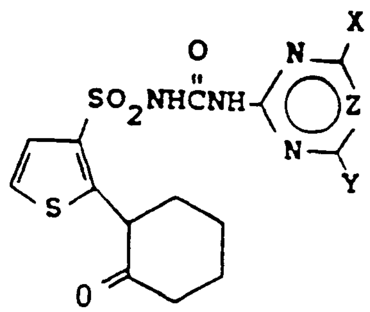

| Compound No. | X | Y | Z |
|---|---|---|---|
| 44 | $CH_3$ | $CH_3$ | CH |
| 45 | $OCH_3$ | $CH_3$ | CH |
| 46 | $OCH_3$ | $OCH_3$ | CH |
| 47 | $OCH_3$ | $CH_3$ | N |

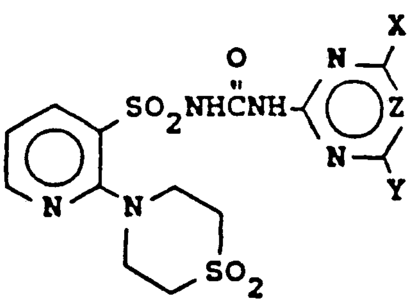

| Compound No. | X | Y | Z |
|---|---|---|---|
| 48 | $OCH_3$ | $OCH_3$ | CH |
| 49 | $OCH_3$ | $CH_3$ | CH |

TABLE A

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 1 | POST | 0.05 | &C | &C | &C | 6C | 6C | 9C | 6C | 4C | | 1C | 9C | 4C | 6C | 9C | 9C | 9C |
| | POST | 0.05 | | | | 9G | 9G | | 9G | 9G | | 6G | | 9G | 9G | | | |
| | PRE | 0.05 | 4C | 9H | 4C | &E | 2C | 3C | 3C | 3C | | 2C | 2C | 2C | 3C | 3C | 5C | 2C |
| | PRE | 0.05 | 9G | | 9G | | 6G | 9H | 8G | 8G | | 8G | 9G | 5G | 9H | 9H | 9G | 8G |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 2 | POST | 0.05 | &C | 9C | &C | 3C | 5C | 9C | 9C | &C | | 6C | 5C | 5C | 6C | 9C | &C | 9C |
| | POST | 0.05 | | | | 9G | 9G | | | | | 9G | 9G | 9G | 9G | | | |
| | PRE | 0.05 | &C | 9H | 6C | 3C | 4C | 5C | &E | 5C | | 4C | 4C | 3C | &E | 5C | 9C | 3C |
| | PRE | 0.05 | | | 9G | 7G | 9G | 9H | | 9H | | 9H | 9H | 6H | | 9H | | 9G |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 3 | POST | 0.05 | &C | &C | 9C | 2C | 3C | 9C | 9C | 9C | | 9C | 4C | 3C | 9C | 3C | &C | 4C |
| | POST | 0.05 | | | | 3G | 7H | | | | | | 9H | 8G | | 9H | | 9H |
| | PRE | 0.05 | 7G | 3C | 3C | 0 | 1C | 3C | 2C | 4C | | 4C | 3C | 0 | 2C | 3C | 9C | 3C |
| | PRE | 0.05 | | 5H | 7H | | | 7G | 7G | 8G | | 8G | 8H | | 9G | 8H | | 6G |

TABLE A (continued)

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 4 | POST | 0.05 | 4C | 1C | 2C | 0 | 3C | 3C | 4C | 4C | | 5C | 9C | 2G | 9C | 5C | 3C | 2C |
| | POST | 0.05 | 9H | 3G | 3G | | 5H | 9H | 8G | 9G | | 9H | | | | 9H | 7H | 5G |
| | PRE | 0.05 | 3H | 4G | 0 | 0 | 1C | 1C | 2C | 2C | | 2C | 3C | 0 | 3C | 3C | 8H | 5G |
| | PRE | 0.05 | | | | | | | 5G | 5G | | 5G | 8H | | 8H | 8H | | |
| Compound 5 | POST | 0.05 | 3C | 3C | 2C | 0 | 2C | 9C | 4C | 9C | | 9C | &C | 0 | 9C | 9C | 4C | 4C |
| | POST | 0.05 | 6H | 9H | 2G | | 7G | | 8G | | | | | | | | 8H | 8H |
| | PRE | 0.05 | 0 | 0 | 0 | 0 | 0 | 1C | 4G | 4C | | 3C | 4C | 0 | 3C | 4C | 3C | 1C |
| | PRE | 0.05 | | | | | | | | 8G | | 7G | 9H | | 8H | 9H | 7G | 2G |
| Compound 6 | POST | 0.05 | &C | &C | 9C | 6C | 0 | 4C | 3C | 1C | | 2G | 3C | 3C | 5C | 9C | 3C | 4C |
| | POST | 0.05 | | | | 9G | | 9H | 5G | | | | 9H | 9G | 9G | | 9H | 9G |
| | PRE | 0.05 | 8H | 8G | 8G | 0 | 0 | 3C | 3C | 3C | | 7G | 3C | 3C | 4C | &H | 5C | 2C |
| | PRE | 0.05 | | | | | | 5G | 8G | 8G | | | 8H | 3H | 8H | | 9G | 8G |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 7 | POST | 0.05 | 4C | 3C | 3C | 5G | 0 | 3C | 0 | 0 | | 0 | 2C | 3C | 2C | 3C | 2C | 3C |
| | POST | 0.05 | 8G | 7H | 7G | | | 7H | | | | | 8H | 8H | 8G | 9H | 3H | 9G |
| | PRE | 0.05 | 3C | 5G | 2C | 0 | 0 | 0 | 0 | 3C | | 0 | 2C | 1C | 2C | 3C | 3C | 2C |
| | PRE | 0.05 | 6H | | | | | | | 6G | | | 5G | | 6H | 8H | 5H | |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 8 | POST | 0.05 | 3C | 2C | 2C | 0 | 0 | 2C | 0 | 0 | | 0 | 3C | 3C | 5G | 3C | 3C | 1C |
| | POST | 0.05 | 5H | 5G | | | | 3H | | | | | 8H | 3H | | 8H | 5H | |
| | PRE | 0.05 | 0 | 0 | 0 | 0 | 0 | 0 | 0 | 0 | | 0 | 0 | 0 | 0 | 0 | 0 | 0 |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 9 | POST | 0.05 | 3C | 6G | 2C | 0 | 2G | 3C | 0 | 2C | | 0 | 3C | 3C | 6G | 3C | 3C | 2C |
| | POST | 0.05 | 5H | | 3G | | | 9H | | 3G | | | 9H | 3H | | 9H | 4H | 5H |
| | PRE | 0.05 | 1C | 0 | 0 | 0 | 0 | 0 | 0 | 0 | | 0 | 0 | 0 | 0 | 2C | 2H | 0 |
| | PRE | 0.05 | | | | | | | | | | | | | | 8H | | |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 10 | POST | 0.05 | 6C | 9C | 6C | 2C | 3C | 4C | 3C | 3C | | 2C | 4C | 4C | 5C | 3C | 5C | 4C |
| | POST | 0.05 | 9G | | 9G | 8G | 4H | 9H | 6G | 6G | | 6G | 9G | 9G | 9G | 9H | 9G | 9G |
| | PRE | 0.05 | 8G | 8H | 3C | 5G | 0 | 3C | 2C | 3C | | 7G | 3C | 3C | 5C | 4C | 5C | 3C |
| | PRE | 0.05 | | | 6G | | | 7H | 5G | 8G | | | 9G | 6H | 9G | 9H | 9G | 7G |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 11 | POST | 0.05 | &C | 8G | 8G | 0 | 0 | 9G | 0 | 0 | | 0 | 8G | 0 | 7G | &C | &C | 9G |
| | POST | 0.05 | | | 6C | | | 9C | | | | | 5H | | 3C | | | 9C |
| | PRE | 0.05 | 8G | 6G | 8G | 5G | 4G | 9G | 6G | 5G | | 4G | 7G | 0 | 9G | 8G | &G | 8G |
| | PRE | 0.05 | | | | | | 5G | | | | | 5H | | 9C | 8C | | |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 12 | POST | 0.05 | &C | &C | 9C | 3C | 3C | 5C | 2C | 4C | | 0 | 2C | 4C | 5C | 5C | 9C | 5C |
| | POST | 0.05 | | | | 8G | 6G | 9H | 8G | 9G | | | 6H | 9G | 9G | 9G | | 9G |
| | PRE | 0.05 | 9C | 9H | 4C | 5G | 2C | 4C | 3C | 2C | | 2G | 2C | 2C | 2C | 3C | &C | 2C |
| | PRE | 0.05 | | | 8G | | 5G | 8H | 9H | 8G | | | 6G | 2H | 8H | 9H | | 8G |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 13 | POST | 0.05 | 2G | 9C | 6C | 2C | 0 | 2C | 4G | 0 | | 0 | 0 | 2C | 8G | 3C | 9C | 4C |
| | POST | 0.05 | | | 9G | 5G | | 8H | | | | | | 8G | | 7G | | 8G |
| | PRE | 0.05 | 4G | 5H | 5G | 0 | 0 | 1H | 3G | 0 | | 2G | 5G | 0 | 2C | 3C | 3C | 2G |
| | PRE | 0.05 | | | | | | | | | | | | | 8G | 9H | 8G | |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 14 | POST | 0.05 | 4C | 2C | 3C | 0 | 2G | 2C | 2G | 0 | | 2G | 0 | 2G | 0 | 0 | 3C | 4C |
| | POST | 0.05 | 8H | 8G | 8G | | | 5G | | | | | | | | | 5G | 7G |
| | PRE | 0.05 | 3G | 4G | 2C | 0 | 0 | 0 | 0 | 0 | | 0 | 0 | 0 | 0 | 0 | 3C | 3G |
| | PRE | 0.05 | | | | | | | | | | | | | | | 8G | |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 15 | POST | 0.05 | 5C | 9C | 6C | 7G | 7G | 5C | 6G | 2C | | 4G | 3H | 5H | 7G | 3C | 4C | 4C |
| | POST | 0.05 | 9H | | 9G | | | 8H | | 6G | | | | | | 8H | 7H | 8H |
| | PRE | 0.05 | 6G | 9H | 5C | 7G | 8G | 3C | 8G | 2C | | 8G | 7G | 2C | 5C | 3C | 2C | 7G |
| | PRE | 0.05 | | | 8G | | | 7H | | 8G | | | | 2G | 9H | 9H | 8G | |

TABLE A (continued)

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 16 | POST | 0.05 | 4G | 7G | 2C | 0 | 2G | 2C | 0 | 3G | | 3G | 2G | 2G | 6G | 6G | 1H | 5G |
| | POST | 0.05 | | | 7H | | | 6H | | | | | | | | | | |
| | PRE | 0.05 | 2G | | 0 | 0 | 4G | 0 | 2G | 0 | | 0 | 2G | 0 | 6G | 2C | 7G | 4G |
| | PRE | 0.05 | | | | | | | | | | | | | | 7G | | |
| Compound 17 | POST | 0.05 | 3G | 4C | 4C | 0 | 5G | 2C | 2C | 7G | | 4G | 0 | 2H | 8G | 2C | 2C | 2C |
| | POST | 0.05 | | 9G | 8H | | | 7H | 4G | | | | | | | 7G | 5G | 6G |
| | PRE | 0.05 | 2C | 2C | 2C | 0 | 7G | 2C | 2G | 2C | | 2G | 2G | 0 | 5G | 2C | 5G | 2G |
| | PRE | 0.05 | 5G | 4H | 4H | | | | | 6G | | | | | | 7G | | |
| Compound 18 | POST | 0.05 | 5C | 4C | 4C | 3G | 0 | 2G | 0 | 0 | | 0 | 2G | 2C | 0 | 0 | 2C | 4C |
| | POST | 0.05 | 9G | 9G | 9H | | | | | | | | | 4H | | | 5G | 8G |
| | PRE | 0.05 | 3C | 2C | 2C | 0 | 2G | 0 | 0 | 0 | | 0 | 2G | 0 | 0 | 0 | 5G | 0 |
| | PRE | 0.05 | 5H | 5H | 2H | | | | | | | | | | | | | |

TABLE A (continued)

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 19 | POST | 0.05 | 3C | &C | &C | 6C | 3G | 3H | 2C | 0 | | 0 | 0 | 4C | 5G | 2C | 9C | 4C |
| | POST | 0.05 | 8H | | | 8G | | | 7G | | | | | 9G | | 5H | | 9G |
| | PRE | 0.05 | 2C | 3C | 4C | 7G | 0 | 0 | 2G | 2C | | 0 | 2G | 3C | 2C | 3C | 2C | 8G |
| | PRE | 0.05 | 6G | 6H | 8H | | | | | 3H | | | | 4H | 6G | 7H | 8G | |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 20 | POST | 0.05 | 4C | 4C | 3C | 0 | 0 | 3H | 0 | 0 | | 0 | 0 | 2C | 0 | 0 | 4C | 3C |
| | POST | 0.05 | 9G | 9G | 7G | | | | | | | | | 3H | | | 8G | 8H |
| | PRE | 0.05 | 2C | 2G | 1C | 0 | 0 | 0 | 0 | 0 | | 0 | 2C | 0 | 0 | 2G | 2C | 2C |
| | PRE | 0.05 | 4G | | | | | | | | | | 5G | | | | 7G | 5G |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 21 | POST | 0.05 | 3C | 2C | 2C | 2G | 2G | 2H | 0 | 0 | | 0 | 0 | 1H | 2G | 2G | 2C | 3C |
| | POST | 0.05 | 7G | 8H | 7G | | | | | | | | | | | | 3G | 7G |
| | PRE | 0.05 | 2C | 2H | 4G | 2G | 0 | 2H | 3G | 0 | | 2G | 3G | 0 | 2G | 2C | 2C | 6G |
| | PRE | 0.05 | 5G | | | | | | | | | | | | | 7G | 7G | |

TABLE A (continued)

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 22 | POST | 0.05 | 2C | 3C | 4C | 6G | 5G | 7H | 0 | 9G | | 4G | 1H | 3H | 8G | 3C | 3C | 3C |
| | POST | 0.05 | 6H | 8H | 9G | | | | | | | | | | | 8G | 7G | 6G |
| | PRE | 0.05 | 2C | 2C | 2C | 0 | 4G | 1C | 0 | 3C | | 3C | 3C | 1H | 8H | 9H | 7G | 7G |
| | PRE | 0.05 | 6G | 5G | 6H | | | | | 8H | | 8H | 7G | | | | | |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 23 | POST | 0.05 | 3C | 3C | 3C | 4G | 0 | 2H | 0 | 0 | | 0 | 6H | 2C | 0 | 3H | 4C | 4C |
| | POST | 0.05 | 9G | 9H | 7H | | | | | | | | | 5G | | | 9G | 7G |
| | PRE | 0.05 | 3C | 1C | 3C | 0 | 0 | 0 | 0 | 0 | | 0 | 3C | 3G | 2G | 4G | 7G | 7G |
| | PRE | 0.05 | 5H | | | | | | | | | | 6G | | | | | |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 24 | POST | 0.05 | 7G | 2C | 2C | 3G | 0 | 7H | 2G | 0 | | 0 | 6G | 2C | 4G | 2C | 3C | 4C |
| | POST | 0.05 | | 8H | 8H | | | | | | | | | 5G | | 8H | 6H | 9H |
| | PRE | 0.05 | 9G | 8H | 5C | 7G | 5G | 3C | 6G | 2C | | 2C | 2C | 3C | 2C | 2C | 5C | 2C |
| | PRE | 0.05 | | | 9G | | | 7H | | 8G | | 8H | 9G | 4H | 7G | 8G | 9G | 8G |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 25 | POST | 0.05 | 5C | 9C | 5C | 5C | 0 | 3C | 5G | 2C | | 3G | 2G | 2C | 2C | 3C | 9C | 4C |
| | POST | 0.05 | 9G | | 9G | 9G | | 6H | | 3G | | | | 8H | 8G | 8H | | 9G |
| | PRE | 0.05 | 8G | 8H | 5C | 7G | 3H | 3C | 6G | 2C | | 3G | 6G | 3C | 3C | 3C | 5C | 8G |
| | PRE | 0.05 | | | 9G | | | 4G | | 3G | | | | 4G | 8H | 9H | 9G | |
| Compound 26 | POST | 0.05 | 2H | 5C | 3C | 7G | 4C | 5C | 4C | 9C | | 5C | 6C | 9C | 5C | &C | 5C | 4C |
| | POST | 0.05 | | 9G | 7H | | 8H | 9H | 9G | | | 9G | | | 9G | | 9G | 9H |
| | PRE | 0.05 | 2C | 3C | 5C | 0 | 4G | 4C | 5C | 5C | | 6C | 5C | 3C | &E | 5C | 5C | 7G |
| | PRE | 0.05 | 2H | 6H | 8H | | | 8H | 9G | 9G | | 9H | 9H | 8H | | 9H | 9G | |
| Compound 27 | POST | 0.05 | 2C | 5C | 3C | 2G | 2C | 5C | 4C | 5C | | 3C | 6C | 9C | 9C | 5C | 5C | 4C |
| | POST | 0.05 | 8H | 9G | 7H | | 3H | 9H | 9G | 9G | | 9G | 9G | | | 9G | 8H | 8H |
| | PRE | 0.05 | | 7H | 2C | 4G | 4C | 3C | 4C | 2C | | 2C | 3C | 3C | 4C | 4C | 5C | 7H |
| | PRE | 0.05 | | | 6H | | 9G | 5G | 8H | 7G | | 8G | 9H | 7G | 9H | 9H | 9G | |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 28 | POST | 0.05 | 2C | 2C | 3G | 0 | 3G | 3C | 2C | 4G | | 4G | 4C | 2C | 2C | 3C | 3C | 2C |
| | POST | 0.05 | 5G | 6G | | | | 9H | 7G | | | | 9H | 3H | 8G | 8H | 6H | 3G |
| | PRE | 0.05 | 3C | 2G | 3C | 0 | 0 | 2G | 5G | 4G | | 4G | 3C | 2C | 4C | 3C | 3C | 2C |
| | PRE | 0.05 | 7H | | 3H | | | | | | | | 8H | 5G | 9H | 9H | 7G | 2H |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 29 | POST | 0.05 | 2C | 2C | 1C | 0 | 0 | 2C | 3C | 2C | | 2C | 3C | 3C | 6G | 3C | 2C | 2C |
| | POST | 0.05 | 4G | 4G | | | | 6H | 8G | 5G | | 5G | 8H | 7H | | 6H | 5H | 2H |
| | PRE | 0.05 | 2C | 2C | 2C | 0 | 0 | 0 | 0 | 0 | | 0 | 2C | 2C | 6G | 3C | 7G | 0 |
| | PRE | 0.05 | 6H | | 4G | | | | | | | | 4G | | | 5G | | |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 30 | POST | 0.05 | 2C | 2C | 2C | 3C | 2C | 5C | 2C | 3C | | 4G | 3C | 9C | 3C | 2U | 9C | 4C |
| | POST | 0.05 | 5H | 6G | 5H | 8G | 5G | 9H | 8G | 8G | | | 9G | | 9G | 9H | | 6H |
| | PRE | 0.05 | 7G | 2H | 1C | 0 | 0 | 1H | 0 | 0 | | 0 | 2C | 1C | 3G | 2C | 5G | 2G |
| | PRE | 0.05 | | | | | | | | | | | 3G | | | 5G | | |

TABLE A (continued)

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 31 | POST | 0.05 | 2C | 2C | 1C | 2C | 0 | 9H | 2C | 0 | | 0 | 3C | 3C | 2C | 3C | 3C | 2G |
| | POST | 0.05 | 6H | 6G | 3H | | | | 5G | | | | 9H | 8G | 6G | 9H | 7H | |
| | PRE | 0.05 | 0 | 0 | 0 | 0 | 0 | 0 | 0 | 0 | | 2G | 3C | 2G | 3G | 2C | 2G | 0 |
| | PRE | 0.05 | | | | | | | | | | | 6G | | | 4G | | |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 32 | POST | 0.05 | 3G | 0 | 0 | 0 | 0 | 2C | 0 | 0 | | 0 | 2H | 2H | 4G | 3C | 3H | 0 |
| | POST | 0.05 | | | | | | 9H | | | | | | | | 9H | | |
| | PRE | 0.05 | 0 | 4G | 0 | 0 | 0 | 0 | 0 | 0 | | 0 | 2G | 0 | 0 | 2C | 0 | 0 |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 33 | POST | 0.05 | 2C | 2C | 3C | 2C | 2G | 3C | 0 | 0 | | 0 | 3C | 3C | 2C | 3C | 3C | 3C |
| | POST | 0.05 | 5G | 3G | 8G | 5G | | 9H | | | | | 9H | 8G | 8G | 9H | 8G | 8H |
| | PRE | 0.05 | 0 | | 2G | 0 | 0 | 0 | 0 | 0 | | 0 | 2C | 0 | 2C | 2C | 3C | 3G |
| | PRE | 0.05 | | | | | | | | | | | 5G | | 5G | 3G | 6G | |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 34 | POST | 0.05 | 0 | 0 | 0 | 0 | 0 | 3C | 0 | 2C | | 0 | 2C | 2C | 6G | 3C | 4C | 2C |
| | POST | 0.05 | | | | | | 8H | | 4G | | | 5G | 5H | | 8H | 8G | |
| | PRE | 0.05 | 0 | | 4G | 0 | 0 | 0 | 0 | 0 | | 0 | 0 | 0 | 2G | 2G | 4G | 0 |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 35 | POST | 0.05 | 0 | 0 | 5G | 0 | 0 | 2C | 0 | 0 | | 0 | 3G | 0 | 0 | 0 | 2C | 0 |
| | POST | 0.05 | | | | | | 4H | | | | | | | | | 5G | |
| | PRE | 0.05 | 0 | | 2G | 0 | 0 | 0 | 0 | 0 | | 0 | 0 | 0 | 0 | 0 | 4H | 0 |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 36 | POST | 0.05 | 2G | 2G | 0 | 5G | 0 | 7H | 0 | 2C | | 0 | 7H | 1H | 7G | 4C | 3C | 3G |
| | POST | 0.05 | | | | | | | | 8G | | | | 7G | | 8H | 5G | |
| | PRE | 0.05 | 5G | 0 | 5G | 0 | 0 | 0 | 0 | 3G | | 0 | 0 | 1H | 4G | 4G | 5H | 2G |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 37 | POST | 0.05 | 2G | 7G | 7G | 9G | 5G | 9H | 4G | 4G | | 0 | 3C | 5C | 3C | 3C | 9C | 4C |
| | POST | 0.05 | | | | | | | | | | | 8H | 9G | 6G | 9H | | 8G |
| | PRE | 0.05 | 3G | 2H | 8G | &E | 3G | 8H | 7G | 5G | | 2G | 3C | 2H | 2H | 3C | 5C | 2G |
| | PRE | 0.05 | | | | | | | | | | | 7H | | | 8G | 9G | |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 38 | POST | 0.05 | 5G | 3C | 5G | 8G | 0 | 4H | 0 | 0 | | 0 | 2C | 5C | 0 | 2C | 4C | 4G |
| | POST | 0.05 | | 9H | | | | | | | | | 7H | 9G | | 7G | 8G | |
| | PRE | 0.05 | 5G | 2H | 7G | 0 | 0 | 0 | 0 | 0 | | 0 | 2G | 0 | 0 | 3G | 7G | 2G |

TABLE A (continued)

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 39 | POST | 2 | 3C | 9C | &C | 9G | 2C | 9C | 4C | 4C | | 9G | 3C | 8G | 9C | 2C | 5C | 5C |
| | POST | 2 | 9G | | | | 7G | | 9G | 9G | | | 9H | | | 9G | 9G | 9G |
| | PRE | 2 | 9G | 9H | 9C | &E | 5G | 3C | 4C | 3C | | 3C | 2C | 6G | &E | &E | 5C | 9G |
| | PRE | 2 | | | | | | 9H | 9H | 9G | | 9H | 9G | | | | 9G | |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 40 | POST | 0.05 | 7G | 6C | 2C | 2G | 9H | 8G | 0 | | 0 | 5H | 2C | 3C | 3C | 5C | 5C | |
| | POST | 0.05 | | | | 8G | | | | | | | | 8G | 9G | 8H | 9G | 9G |
| | PRE | 0.05 | 9G | 6H | 5C | 3C | 2C | 3C | 7G | 2C | | 4G | 7G | 2C | 6G | 2C | 9C | 2C |
| | PRE | 0.05 | | | 9G | 9G | 4G | 8H | | 6G | | | | 3G | | 8G | | 8H |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 41 | POST | 0.05 | 2C | 3C | &C | 2C | 5H | 9H | 4G | 0 | | 0 | 0 | 3C | 2C | 3C | 4C | 9C |
| | POST | 0.05 | 7G | 9G | | 9G | | | | | | | | 9G | 7G | 5H | 9G | |
| | PRE | 0.05 | 8G | 8H | 9G | 9G | 2G | 3C | 6G | 0 | | 0 | 2G | 1H | 4G | 3C | 4C | 5G |
| | PRE | 0.05 | | | | | | 5G | | | | | | | | 7G | 9G | |

TABLE A (continued)

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 42 | POST | 0.05 | 3C | 3C | 2G | 0 | 0 | 0 | 0 | 0 | | 0 | 0 | 2C | 4G | 0 | 3C | 2C |
| | POST | 0.05 | 7G | 9H | | | | | | | | | | 7G | | | 5H | 7G |
| | PRE | 0.05 | 5H | 5G | 2G | 0 | 0 | 0 | 0 | 0 | | 0 | 0 | 1C | 2G | 0 | 5G | 2C |
| | PRE | 0.05 | | | | | | | | | | | | | | | | 3G |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 43 | POST | 0.05 | 0 | 4G | 2G | 0 | 0 | 0 | 0 | 0 | | 0 | 0 | 0 | 2G | 2G | 2G | 4C |
| | POST | 0.05 | | | | | | | | | | | | | | | | 9G |
| | PRE | 0.05 | 8G | 0 | 5G | 0 | 0 | 0 | 0 | 0 | | 0 | 0 | 0 | 3G | 3G | 4G | 3G |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 44 | POST | 0.05 | 0 | 2C | 5G | 0 | 0 | 0 | 0 | 0 | | 0 | 0 | 0 | 8G | 3G | 3C | 1C |
| | POST | 0.05 | | 6G | | | | | | | | | | | | | 8H | |
| | PRE | 0.05 | 1H | 1H | 0 | 0 | 0 | 0 | 0 | 0 | | 0 | 0 | 0 | 1C | 0 | 6G | 0 |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 45 | POST | 0.05 | 1H | 2C | 9C | 4C | 2G | 2C | 0 | 2G | | 2G | 2C | 4C | 2C | 3C | &C | 4C |
| | POST | 0.05 | | 8G | | 9G | | 7H | | | | | 9H | 9G | 9G | 9H | | 9H |
| | PRE | 0.05 | 8H | 8H | 8G | 0 | 0 | 0 | 0 | 0 | | 0 | 2C | 3C | 6G | 2C | 4C | 2C |
| | PRE | 0.05 | | | | | | | | | | | 7G | 3G | | | 9G | 5G |

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 46 | POST | 0.05 | 7G | 8H | 5C | 6C | 0 | 0 | 0 | 0 | | 0 | 2H | 5C | 3G | 0 | 9C | 4C |
| | POST | 0.05 | | | 9G | | | | | | | | | 9G | | | | 9H |
| | PRE | 0.05 | 5G | 8H | 5G | 3G | 0 | 0 | 0 | 0 | | 0 | 0 | 2C | 2G | 0 | 6G | 3G |

TABLE A (continued)

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 47 | POST | 0.05 | 2H | 2C | 3C | 0 | 0 | 0 | 0 | 0 | | 0 | 0 | 2C | 0 | 0 | 3C | 3C |
| | POST | 0.05 | | 8G | 7G | | | | | | | | | 8G | | | 8G | 8H |
| | PRE | 0.05 | 0 | 0 | 0 | 0 | 0 | 1C | 0 | 0 | | 0 | 0 | 0 | 2C | 0 | 2H | 0 |

| | Type Test | Rate K/Ha | Morn-ing Glory | Cockle Bur | Velvet Leaf | Nut Sedge | Crab Grass | Barn-yard Grass | Cheat Grass | Wild Oats | Sickle Pod | Wheat | Corn | Soy-bean | Rice | Sor-ghum | Sugar beet | Cotton |
|---|---|---|---|---|---|---|---|---|---|---|---|---|---|---|---|---|---|---|
| Compound 48 | POST | 0.4 | 9C | 4C | 4C | 5C | 4G | 3C | 3C | 0 | | 0 | 0 | 3C | 2C | 2C | 9C | 3C |
| | POST | 0.4 | | 9H | 9H | 9G | | 7H | 9G | | | | | 9G | 5G | 6G | | 9H |
| | POST | 0.05 | 4C | 4C | 3C | 2C | 2G | 5G | 7G | 0 | | 0 | 0 | 2C | 5G | 4G | 4C | 4C |
| | POST | 0.05 | 9G | 8H | 7H | 8G | | | | | | | | 8G | | | 8H | 8H |
| | PRE | 0.4 | 3C | 2C | 3C | 5G | 0 | 2C | 6G | 0 | | 0 | 3G | 2C | 2C | 2C | 4C | 5G |
| | PRE | 0.4 | 8H | | 4H | | | 4G | | | | | | 2H | 6G | 5G | 8G | |
| | PRE | 0.05 | 2C | 1C | 2C | 0 | 0 | 0 | 0 | 0 | | 0 | 2G | 2G | 2G | 3C | 5G | 5G |
| | PRE | 0.05 | 8H | | 5H | | | | | | | | | | 3H | | | |

TABLE A (continued)

| | Type Test | Rate K/Ha | Morning Glory | Cockle Bur | Velvet Leaf | Nut Sedge | Crab Grass | Barnyard Grass | Cheat Grass | Wild Oats | Sickle Pod | Wheat | Corn | Soybean | Rice | Sorghum | Sugar beet | Cotton |
|---|---|---|---|---|---|---|---|---|---|---|---|---|---|---|---|---|---|---|
| Compound 49 | POST | 0.4 | 5C | 5C | 5C | 2C | 3C | 3C | 9C | 0 | | 0 | 3H | 4C | 3C | 3C | 9C | 5C |
| | POST | 0.4 | 9G | 9G | 9H | 9G | 6G | 8H | | | | | | 8G | 7G | 7H | | 9H |
| | POST | 0.05 | 4C | 4C | 4C | 0 | 2G | 7H | 3C | 0 | | 0 | 1H | 3C | 3G | 1H | 4C | 4C |
| | POST | 0.05 | 9H | 9G | 7H | | | | 9G | | | | | 7H | | | 8G | 8G |
| | PRE | 0.4 | 3C | 3C | 3C | 9G | 0 | 3C | 4C | 2C | | 4G | 3C | 3C | 3C | 3C | &E | 3C |
| | PRE | 0.4 | 8G | 4H | 7H | | | 7H | 9H | 5G | | | 7H | 5H | 7H | 7H | | 6G |
| | PRE | 0.05 | 3C | 2C | 2C | 0 | 0 | 0 | 0 | 0 | | 0 | 0 | 1H | 0 | 0 | 4C | 4G |
| | PRE | 0.05 | 5H | | 5G | | | | | | | | | | | | 6G | |

## Claims

1. A compound of the formula:

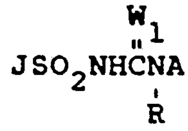

I

wherein

J is

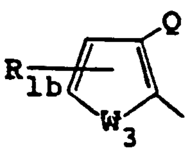 ,

J-5

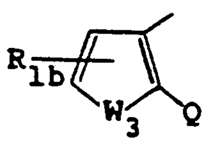 ,

J-6

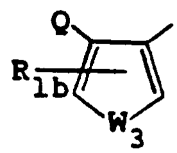 ,

J-7

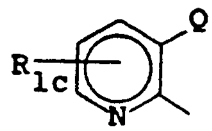 ,

J-8

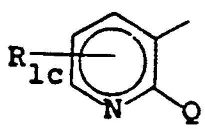 ,

J-9

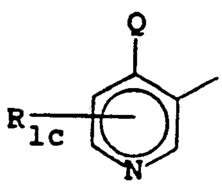 or

J-10

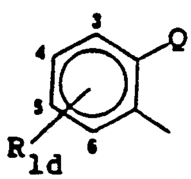 ;

J-11

$W_1$ is O or S;
R is H or $CH_3$;
$W_3$ is O, S or $NR''$;
$R_{1b}$, $R_{1c}$ and $R_{1d}$ are independently H, $C_1$—$C_3$ alkyl, $C_1$—$C_3$ haloalkyl, $C_1$—$C_3$ haloalkoxy, halogen, nitro, $C_1$—$C_3$ alkoxy, $SO_2NR'R''$, $C_1$—$C_3$ alkylthio, $C_1$—$C_3$ alkylsulfinyl, $C_1$—$C_3$ haloalkylthio, $C_1$—$C_3$ alkylsulfonyl, $CO_2R'''$, amino, $C_1$—$_3$ alkylamino, di($C_1$—$C_3$ alkyl)amino, $CH_2CN$, $CH_2OCH_3$ or $CH_2SCH_3$;
$R'$ is H, $C_1$—$C_4$ alkyl, $C_2$—$C_3$ cyanoalkyl, methoxy or ethoxy;
$R''$ is H, $C_1$—$C_4$ alkyl or $C_3$—$C_4$ alkenyl; or
$R'$ and $R''$ may be taken together as —$(CH_2)_3$—, —$(CH_2)_4$—, —$(CH_2)_5$— or —$CH_2CH_2OCH_2CH_2$—;
$R'''$ is $C_1$—$C_4$ alkyl, $C_3$—$C_4$ alkenyl, $C_3$—$C_4$ alkynyl, $C_2$—$C_4$ haloalkyl, $C_2$—$C_3$ cyanoalkyl, $C_5$—$C_6$ cycloalkyl, $C_4$—$C_7$ cycloalkylalkyl or $C_2$—$C_4$ alkoxy-alkyl;
Q is

Q is 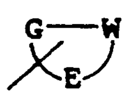 ,

$Q_1$

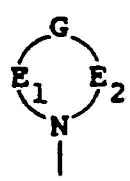 or

$Q_2$

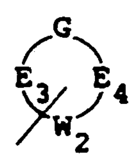 ;

$Q_3$

G is C=O or $SO_2$;
W is O, S, $CHR_2$ or $NR_3$;
$W_2$ is O, S, $SO_2$, $CHR_2$ or $NR_3$;
$R_2$ is H, $C_1$—$C_2$ alkyl, Cl, F or Br;
$R_3$ is H, $C_1$—$C_4$ alkyl, $C_1$—$C_4$ haloalkyl, $C_2$—$C_4$ alkoxyalkyl, $C_2$—$C_4$ cyanoalkyl, $C_3$—$C_4$ alkenyl or $C_3$—$C_4$ alkynyl;
E is $C_3$—$C_4$ alkylene, $C_3$—$C_4$ alkenylene or $C_4$ alkenyldienyl;
$E_1$ and $E_3$ are independently $C_1$—$C_2$ alkylene or $C_2$ alkenylene)
$E_2$ and $E_4$ are independently $C_2$—$C_3$ alkylene or $C_2$—$C_3$ alkenylene; and
E, $E_1$, $E_2$, $E_3$ and $E_4$ may optionally be substituted by 1—4 groups selected from $C_1$—$C_4$ alkyl, $C_1$—$C_4$ alkoxy, $C_1$—$C_4$ alkenyl, OH, halogen and $C_1$—$C_4$ haloalkoxy; further, when W is O, $CHR_2$ or $NR_3$, one of the carbon atoms of E may be in the form of a carbonyl group, and when $W_2$ is O, $CHR_2$ or $NR_3$, one of the

carbon atoms of $E_3$ or $E_4$ may be in the form of a carbonyl group, provided that said carbonyl groups are not bonded directly to G;

A is 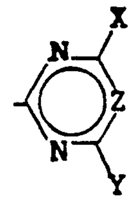 , 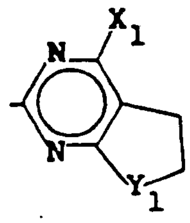 , 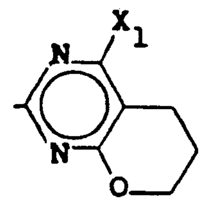 ,

A-1 A-2 A-3

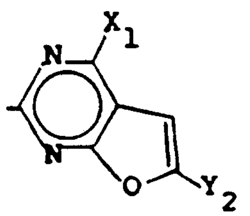 , 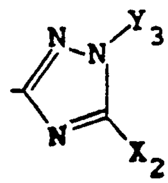 or 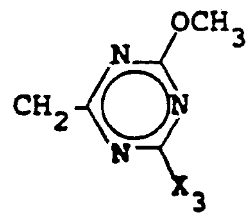 ;

A-4 A-5 A-6

X is H, $C_1$—$C_4$ alkyl, $C_1$—$C_4$ alkoxy, $C_1$—$C_4$ haloalkoxy, $C_1$—$C_4$ haloalkyl, $C_1$—$C_4$ haloalkylthio, $C_1$—$C_4$ alkylthio, halogen, $C_2$—$C_5$ alkoxyalkyl, $C_2$—$C_5$ alkoxyalkoxy, amino, $C_1$—$C_3$ alkylamino or di($C_1$—$C_3$ alkyl)amino;

Y is H, $C_1$—$C_4$ alkyl, $C_1$—$C_4$ alkoxy, $C_1$—$C_4$ haloalkoxy, $C_1$—$C_4$ haloalkylthio, $C_1$—$C_4$ alkylthio, $C_2$—$C_5$ alkoxyalkyl, $C_2$—$C_5$ alkoxyalkoxy, amino, $C_1$—$C_3$ alkylamino, di($C_1$—$C_3$ alkyl)amino, $C_3$—$C_4$ alkenyloxy, $C_3$—$C_4$ alkynyloxy, $C_2$—$C_5$ alkylthioalkyl, $C_1$—$C_4$ haloalkyl, $C_3$—$C_5$ cycloalkyl, $C_2$—$C_4$ alkynyl,

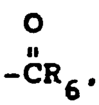 , 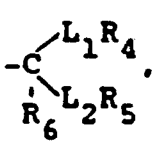 , 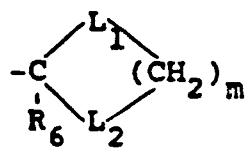 ,

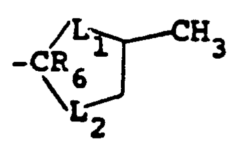 ,

$N(OCH_3)CH_3$, $C_2$—$C_5$ alkylsulfinylalkyl or $C_2$—$C_5$ alkylsulfonylalkyl;

m is 2 or 3;

$L_1$ and $L_2$ are independently O or S;

$R_4$ and $R_5$ are independently $C_1$—$C_2$ alkyl;

$R_6$ is H or $CH_3$;

Z is CH or N;

$Y_1$ is O or $CH_2$;

$X_1$ is $CH_3$, $OCH_3$, $OC_2H_5$ or $OCF_2H$;

$Y_2$ is H or $CH_3$;

$X_2$ is $CH_3$, $OCH_3$ or $SCH_3$;

$Y_3$ is $CH_3$, $CH_2CH_3$ or $CH_2CF_3$; and

$X_3$ is $CH_3$ or $OCH_3$;

and their agriculturally suitable salts;

provided that

a) when G is $SO_2$, then W is O, $CHR_2$ or $NR_3$;

b) when $E_1$ or $E_3$ is $C_2$ alkylene or $C_2$ alkenylene, then $E_2$ or $E_4$ is $C_2$ alkylene or $C_2$ alkenylene;

c) when X is Cl, F, Br or I, then Z is CH and Y is $OCH_3$, $OC_2H_5$, $N(OCH_3)CH_3$, $NHCH_3$, $N(CH_3)_2$ or $OCF_2H$;

d) when X or Y is $OCF_2H$, then Z is CH;

e) when the total number of carbon atoms of X and Y is greater than four, then the number of carbons of $R_{1b}$, $R_{1c}$ or $R_{1d}$ is less than or equal to two and the number of carbon atoms of Q is less than or equal to eight; and

f) when $W_1$ is S, then R is H, A is A—1, and Y is

$CH_3$, $OCH_3$, $OC_2H_5$, $CH_2OCH_3$, $C_2H_5$, $CF_3$, $SCH_3$, $OCH_2CH{=}CH_2$,

$OCH_2C{\equiv}CH$, $OCH_2CH_2OCH_3$, $CH(OCH_3)_2$ or 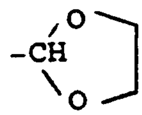 .

2. Compounds of Claim 1 wherein $W_1$ is O; and R is H.

3. Compounds of Claim 2 where
$R_{1b}$ is H, Cl, Br, $NO_2$, $CH_3$, $OCH_3$ or $CF_3$;
$R_{1c}$ is H, Cl, $CH_3$, $OCH_3$ or $N(CH_3)_2$;
$R_{1d}$ is H, $CH_3$, $OCH_3$, Cl, Br, F, $NO_2$, $CF_3$ or $OCF_2H$, and is not in the 4-position;
$W_3$ is S;
X is $CH_3$, $OCH_3$, $OCH_2CH_3$, Cl, F, Br, I, $OCF_2H$, $CH_2F$, $OCH_2CH_2F$, $OCH_2CHF_2$, $OCH_2CF_3$, $CF_3$, $CH_2Cl$ or $CH_2Br$;
Y is

H, $CH_3$, $OCH_3$, $OC_2H_5$, $CH_2OCH_3$, $NHCH_3$, $N(OCH_3)CH_3$, $N(CH_3)_2$,

$CH_2CH_3$, $CF_3$, $SCH_3$, $OCH_2CH{=}CH_2$, $OCH_2C{\equiv}CH$, $CH_2OCH_2CH_3$,

$OCH_2CH_2OCH_3$, $CH_2SCH_3$, 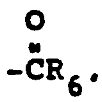 , 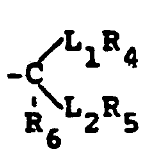 , 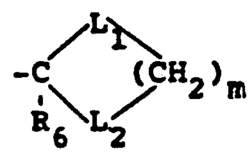 ,

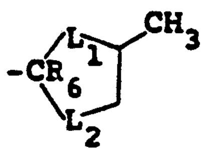 , $OCF_2H$, $SCF_2H$, cyclopropyl, $C{\equiv}CH$ or $C{\equiv}CCH_3$.

4. Compounds of Claim 3 where Q is $Q_1$.

5. Compounds of Claim 3 where Q is $Q_2$.

6. Compounds of Claim 3 where Q is $Q_3$.

7. Compounds of Claim 3 where Q is

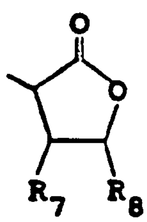 , 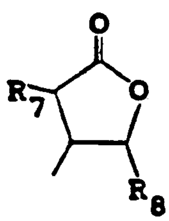 , 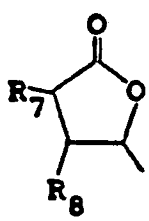 , 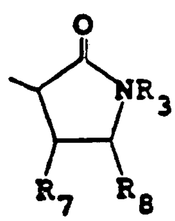 , 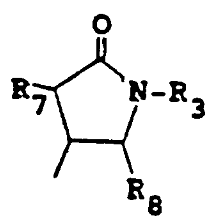 ,

Q-1 Q-2 Q-3 Q-4 Q-5

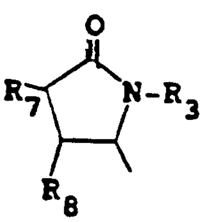 , 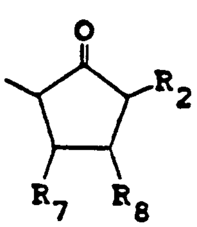 , 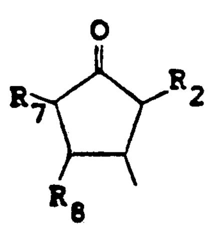 , 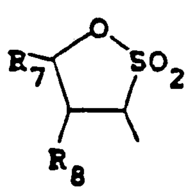 , 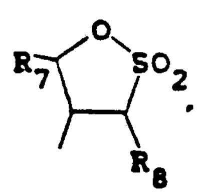 ,

Q-6 Q-8 Q-9 Q-10 Q-11

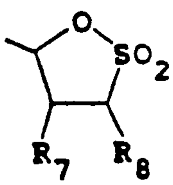 ,
Q-12

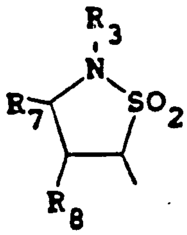 ,
Q-13

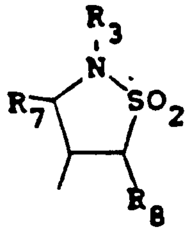 ,
Q-14

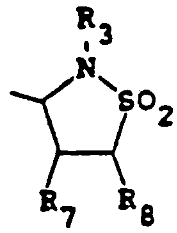 ,
Q-15

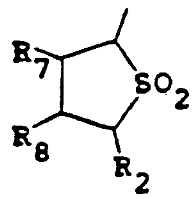 ,
Q-17

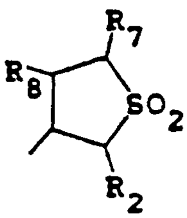 ,
Q-18

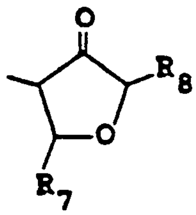 ,
Q-19

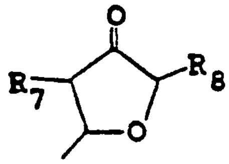 ,
Q-20

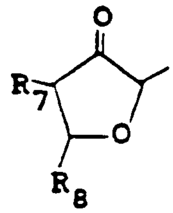 ,
Q-21

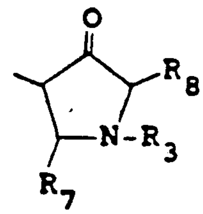 ,
Q-22

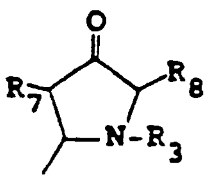 ,
Q-23

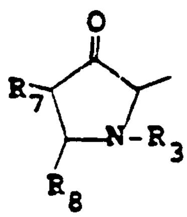 ,
Q-24

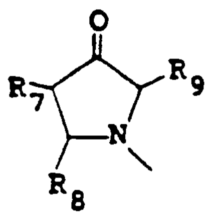 ,
Q-25

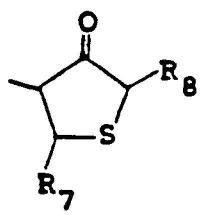 ,
Q-26

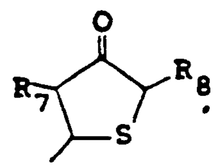 ,
Q-27

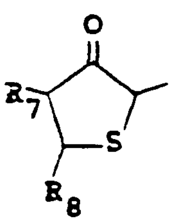 ,
Q-28

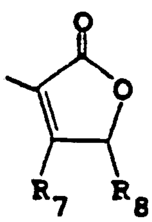 ,
Q-29

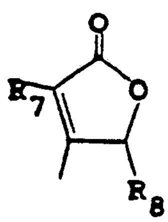 ,
Q-30

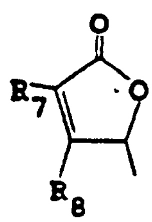 ,
Q-31

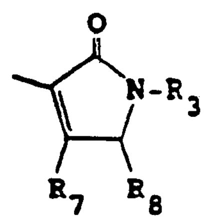 ,
Q-32

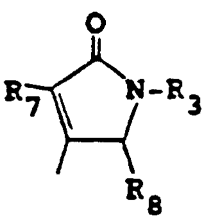 ,
Q-33

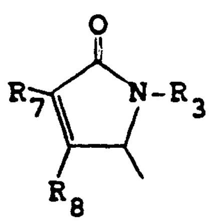 ,
Q-34

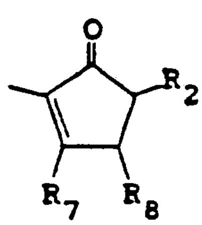 ,
Q-36

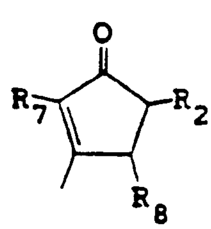 ,
Q-37

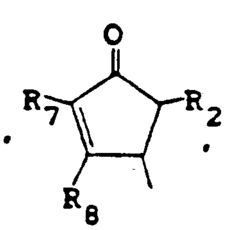 ,
Q-38

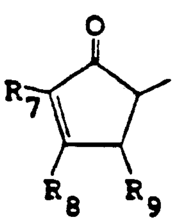 ,
Q-39

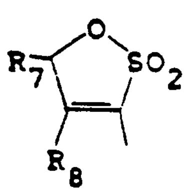 ,
Q-40

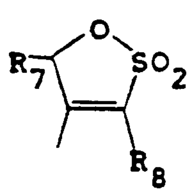 ,
Q-41

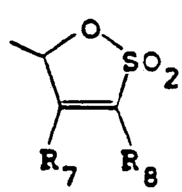 ,
Q-42

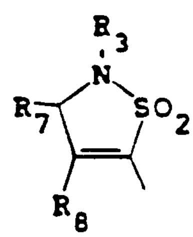 ,
Q-43

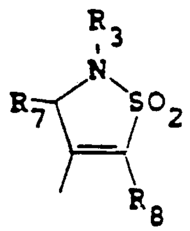
Q-44 ,

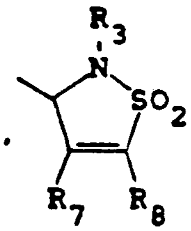
Q-45 ,

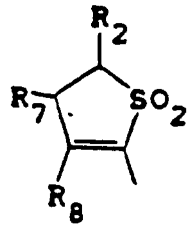
Q-47 ,

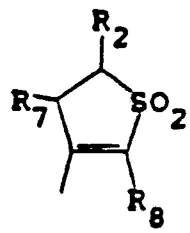
Q-48 ,

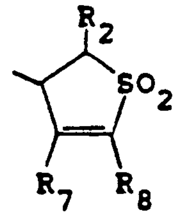
Q-49 ,

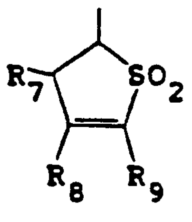
Q-50 ,

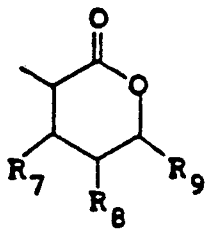
Q-51 ,

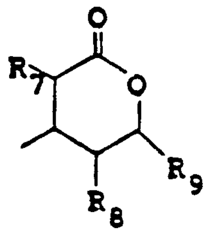
Q-52 ,

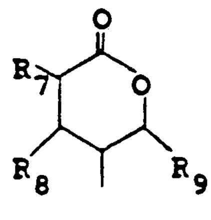
Q-53 ,

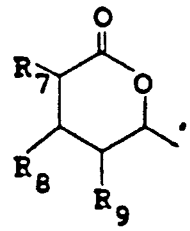
Q-54 ,

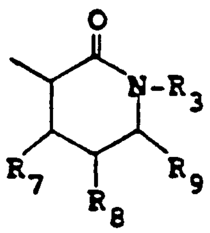
Q-55 ,

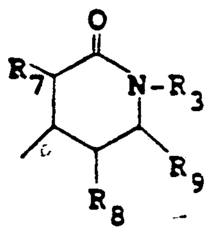
Q-56 ,

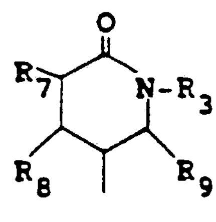
Q-57 ,

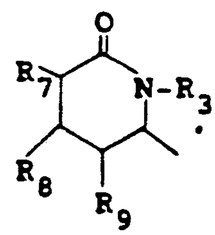
Q-58 ,

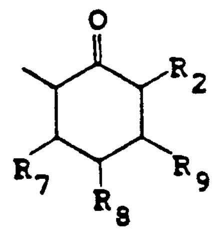
Q-60 ,

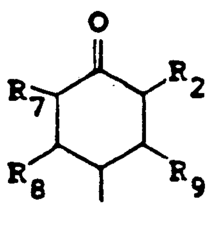
Q-61 ,

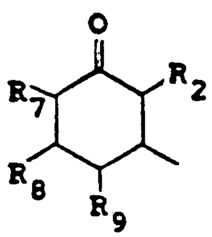
Q-62 ,

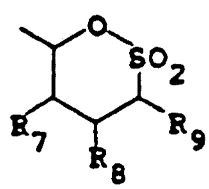
Q-63 ,

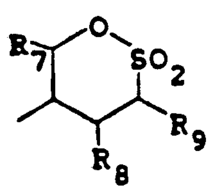
Q-64 ,

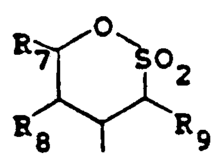
Q-65 ,

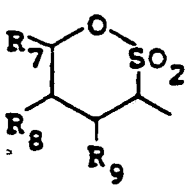
Q-66 ,

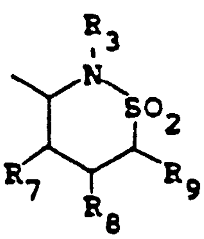
Q-67 ,

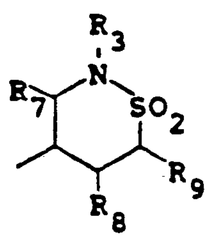
Q-68 ,

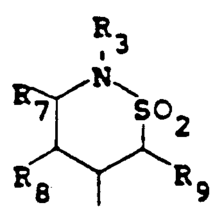
Q-69 ,

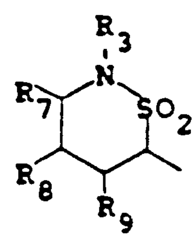
Q-70 ,

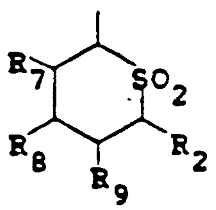
Q-72 ,

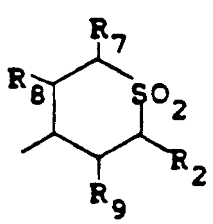
Q-73 ,

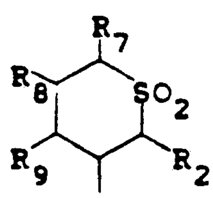
Q-74 ,

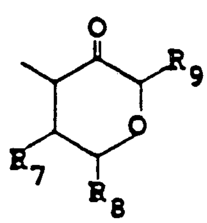
Q-75 ,

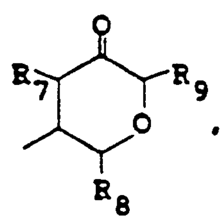
Q-76 ,

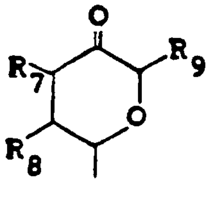
Q-77 ,

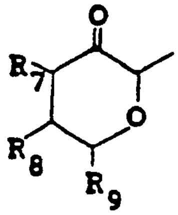
Q-78 ,

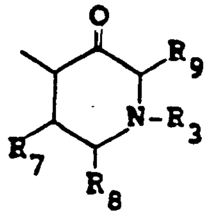
Q-79 ,

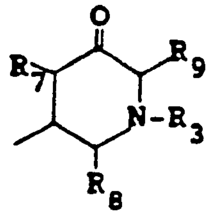
Q-80 ,

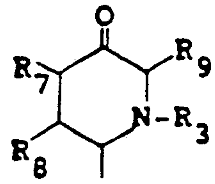
Q-81 ,

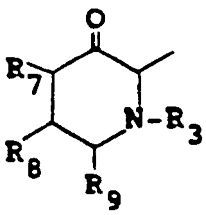
Q-82 ,

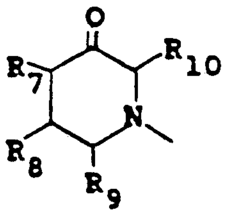
Q-83 ,

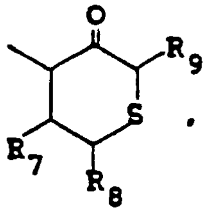
Q-84 ,

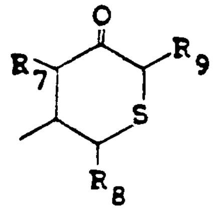
Q-85 ,

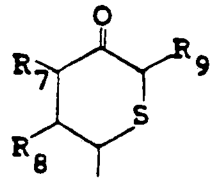
Q-86 ,

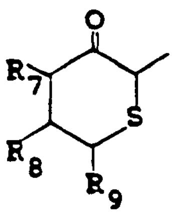
Q-87 ,

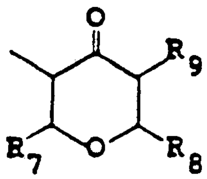
Q-88 ,

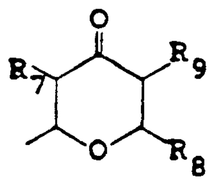
Q-89 ,

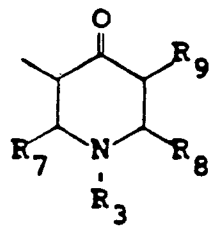
Q-90 ,

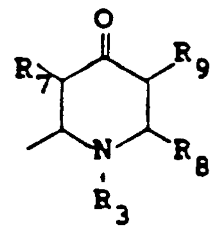
Q-91 ,

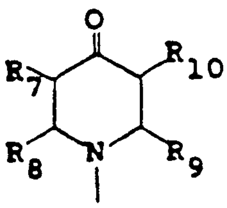
Q-92 ,

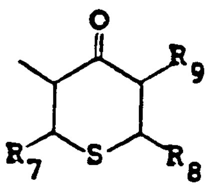
Q-93 ,

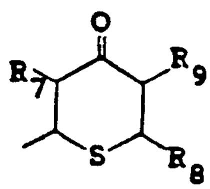
Q-94 ,

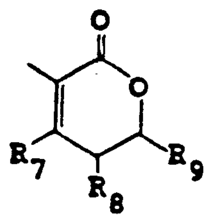
Q-95 ,

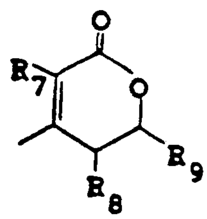
Q-96 ,

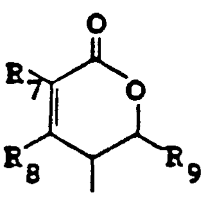
Q-97 ,

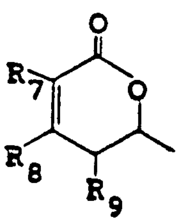
Q-98 ,

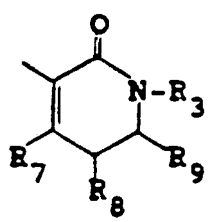
Q-99 ,

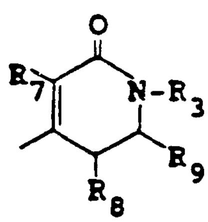
Q-100 ,

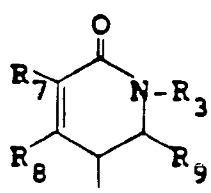
Q-101 ,

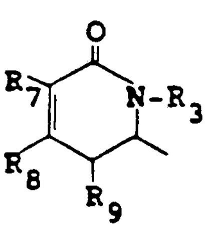
Q-102 ,

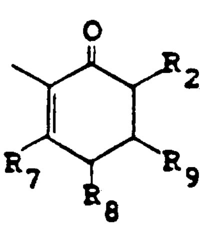
Q-104 ,

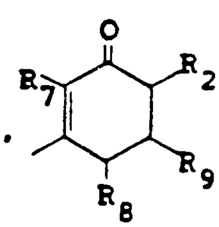
Q-105 ,

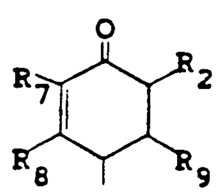
Q-106 ,

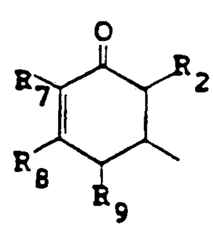
Q-107 ,

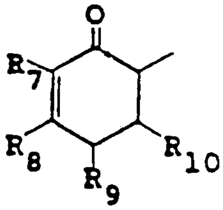
Q-108 ,

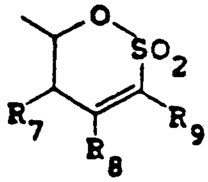
Q-109 ,

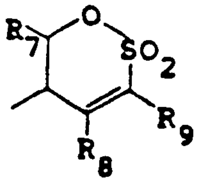
Q-110 ,

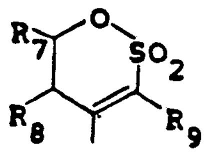
Q-111 ,

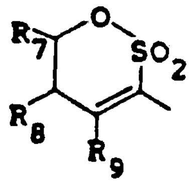
Q-112 ,

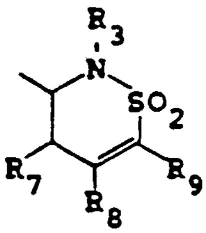
Q-113 ,

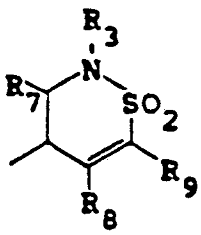
Q-114 ,

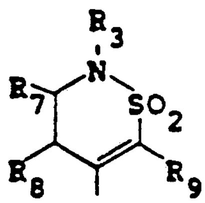
Q-115 ,

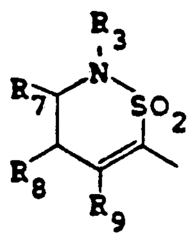
Q-116 ,

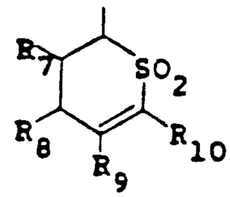
Q-118 ,

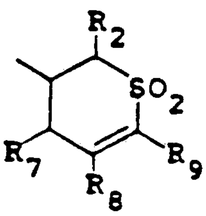
Q-119 ,

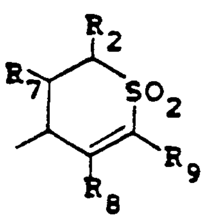
Q-120 ,

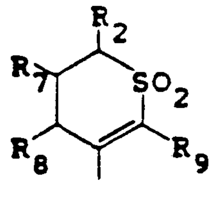
Q-121 ,

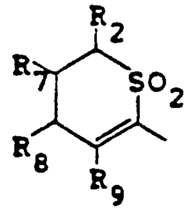
Q-122 ,

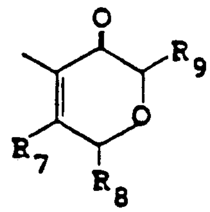
Q-123 ,

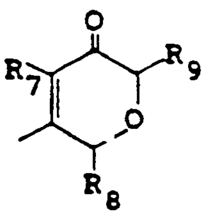
Q-124 ,

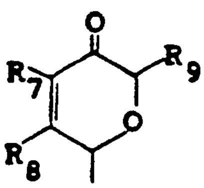
Q-125 ,

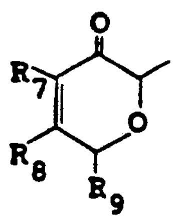
Q-126 ,

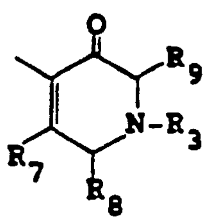
Q-127 ,

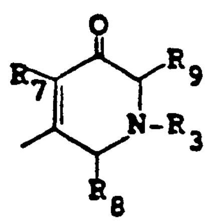
Q-128 ,

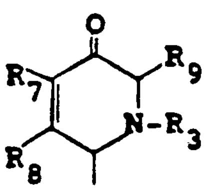
Q-129 ,

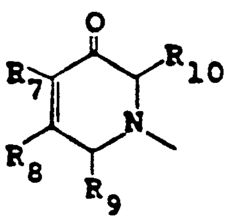
Q-130 ,

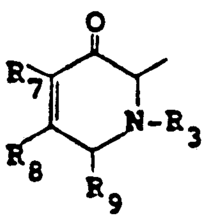
Q-131 ,

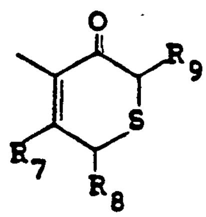
Q-132 ,

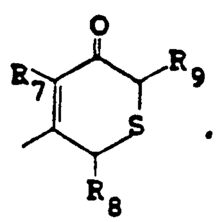
Q-133 ,

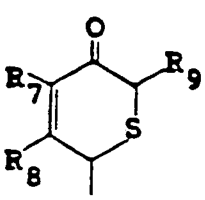
Q-134 ,

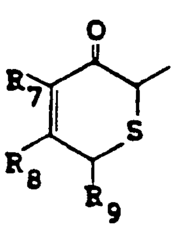
Q-135 ,

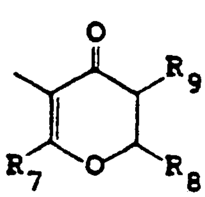
Q-136 ,

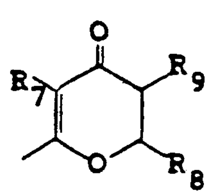
Q-137 ,

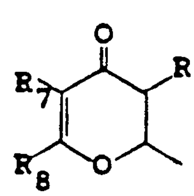
Q-138 ,

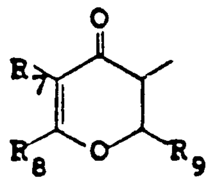 , 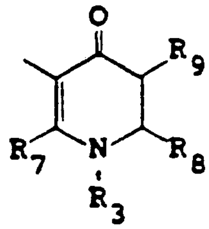 , 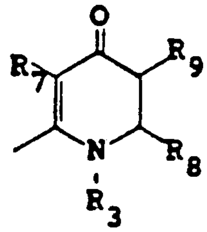 , 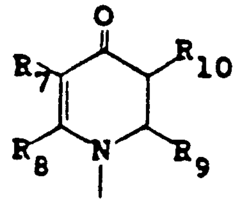 , 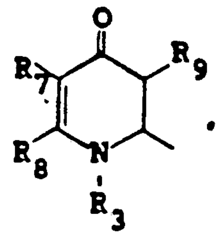 ,

Q-139 Q-140 Q-141 Q-142 Q-143

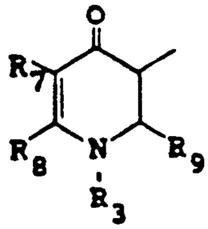 , 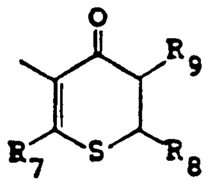 , 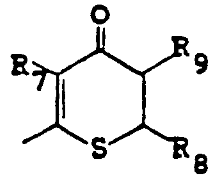 , 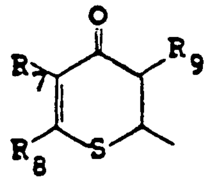 , 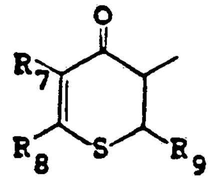 ,

Q-144 Q-145 Q-146 Q-147 Q-148

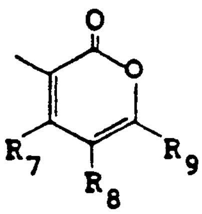 , 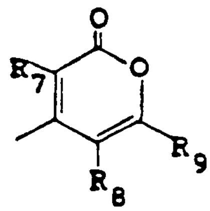 , 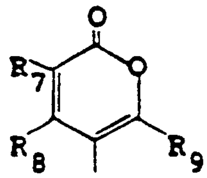 , 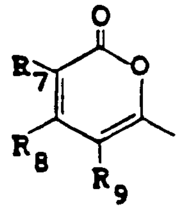 , 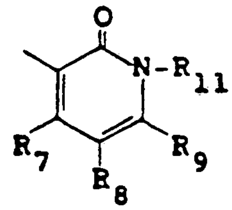 ,

Q-149 Q-150 Q-151 Q-152 Q-153

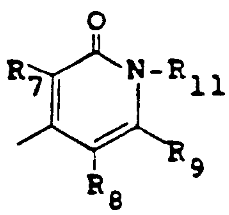 , 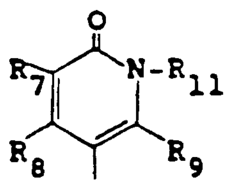 , 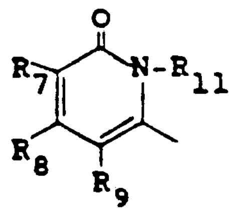 , 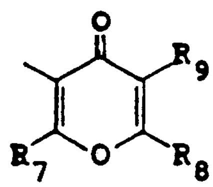 , 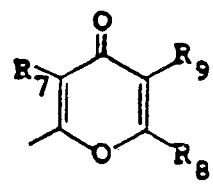 ,

Q-154 Q-155 Q-156 Q-158 Q-159

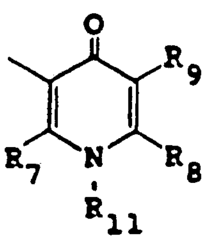 , 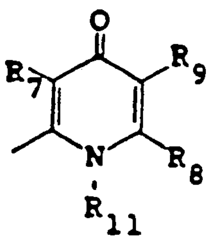 , 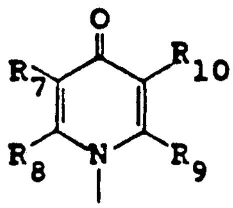 , 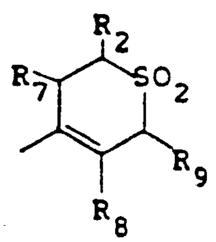 , 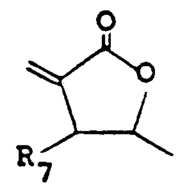 ,

Q-160 Q-161 Q-162 Q-163 Q-164

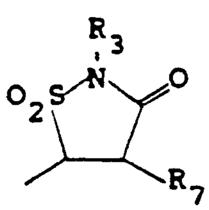 , 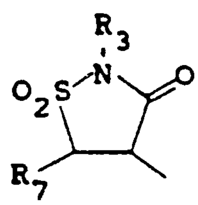 or 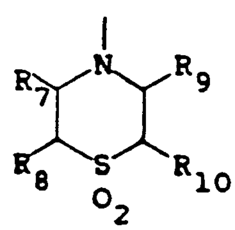 ;

Q-165 Q-166 Q-167

$R_7$, $R_8$, $R_9$ and $R_{10}$ are independently H or $CH_3$; and $R_{11}$ is H, $CH_3$ or $CH_2CH_3$.

8. Compounds of Claim 7 where

J is J—5, J—6, J—8, J—9 or J—11;

$R_{1b}$ is H, $CH_3$ or Cl;
$R_{1c}$ is H;
$R_{1d}$ is H, $CH_3$, $OCH_3$ or Cl; and
Y is $CH_3$, $OCH_3$, $C_2H_5$, $OC_2H_5$, $CH_2OCH_3$, $NHCH_3$, $CH(OCH_3)_2$ or cyclopropyl.

9. Compounds of Claim 8 where
$R_2$ is H or $CH_3$; and
$R_3$ is H, $CH_3$ or $C_2H_5$.

10. Compounds of Claim 9 where
A is A—1; and
X is $CH_3$, $OCH_3$, Cl or $OCF_2H$.

11. The compound of Claim 1 which is N-[(4,6-dimethoxypyrimidin-2-yl)aminocarbonyl]-2-tetrahydro-2-oxo-3-furanylbenzenesulfonamide.

12. The compound of Claim 1 which is N-[(4-methoxy-6-methyltriazin-2-yl)aminocarbonyl]-2-tetrahydro-2-oxo-3-furanylbenzenesulfonamide.

13. The compound of Claim 1 which is N-[(4,6-dimethoxypyrimidin-2-yl)aminocarbonyl]-2-(tetrahydro-2-oxofuran-3-yl)-3-thiophenesulfonamide.

14. The compound of Claim 1 which is N-[(4-methoxy-6-methyltriazin-2-yl)aminocarbonyl]-2-(tetrahydro-2-oxofuran-3-yl)-3-thiophenesulfonamide.

15. The compound of Claim 1 which is N-[(4,6-dimethylpyrimidin-2-yl)aminocarbonyl]-2-(3-oxo-1-cyclohexen-1-yl)-3-thiophenesulfonamide.

16. The compound of Claim 1 which is N-[(4,6-dimethoxypyrimidin-2-yl)aminocarbonyl]-2-(tetrahydro-2-oxofuran-3-yl)-3-pyridinesulfonamide.

17. The compound of Claim 1 which is N-[(4-methoxy-6-methyltriazin-2-yl)aminocarbonyl]-2-(tetrahydro-2-oxofuran-3-yl)-3-pyridinesulfonamide.

18. The compound of Claim 1 which is N-[(4,6-dimethoxypyrimidin-2-yl)aminocarbonyl]-2-(4-thiomorpholinyl)-3-pyridinesulfonamide, S,S-dioxide.

19. Compounds of claim 1 wherein:
J is J—11;
$R_{1d}$ is $H_1$, $C_1$—$C_3$ alkyl, $C_1$—$C_3$ haloalkyl, halogen, nitro, $C_1$—$C_3$ alkoxy, $C_1$—$C_3$ alkylthio, $C_1$—$C_3$ alkylsulfinyl, $C_1$—$C_3$ alkylsulfonyl, $SO_2NR^IR^{II}$, $CO_2R^{III}$, amino, $C_1$—$C_3$ alkylamino or di ($C_1$—$C_3$ alkyl)amino;
$W_2$ is not $SO_2$;
E, $E_1$, $E_2$, $E_3$ and $E_4$ may optionally be substituted by 1—4 groups selected from $C_1$—$C_4$ alkyl, $C_1$—$C_4$ alkoxy, halogen or $C_1$—$C_4$ haloalkoxy;
Y is not $C_2$—$C_5$ alkylsulfinylalkyl or $C_2$—$C_5$ alkylsulfonylalkyl;
and the remaining substituents are as defined in claim 1.

20. Compounds of claim 1 wherein
J is J—5, J—6 or J—7;
$R_{1b}$ is H, $C_1$—$C_3$ alkyl, $C_1$—$C_3$ haloalkyl, halogen, nitro, $C_1$—$C_3$ alkoxy, $SO_2NR^IR^{II}$, $C_1$—$C_3$ alkylthio, $C_1$—$C_3$ alkylsulfinyl, $C_1$—$C_3$ alkylsulfonyl or $CO_2R^{III}$;
$W_2$ is not $SO_2$;
and E, $E_1$, $E_2$, $E_3$, $E_4$ and Y are as defined in claim 19;
the remaining substituents being as defined in claim 1.

21. A composition suitable for controlling the growth of undesired vegetation which comprises an effective amount of a compound of any of claims 1 to 20 and at least one of the following: surfactant, solid or liquid diluent.

22. A method for controlling the growth of undesired vegetation which comprises applying to the locus to be protected an effective amount of a compouond of any of claims 1 to 20.

23. A method for regulating the growth of plants which comprises applying to the locus of such plants an effective but substantially non-phytotoxic amount of a plant growth regulant selected from compounds of any of claims 1 to 20.

24. A process for the preparation of a compound of claim 1 which comprises:
(a) reacting a sulfonyl isocyanate or isothiocyanate of formula

$$J—SO_2NCW_1 \quad (II)$$

with a heterocyclic amine of formula

$$A—NHR \quad (III); \text{ or}$$

(b) reacting a sulfonamide of formula

$$J—SO_2NH_2 \quad (IV)$$

with the methyl or phenyl ester of a pyrimidine or triazine carbamic acid of formula

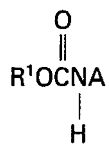 (V, VI)

wherein J, A, R and $W_1$ are as defined in claim 1 and $R^1$ is Me or Ph.

25. Compounds of formulae:

$J—SO_2NH_2$ and $J—SO_2NCW_1$

wherein J and $W_1$ are as defined in claim 1.

**Patentansprüche**

1. Verbindung der Formel

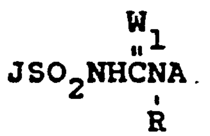

I

worin

J

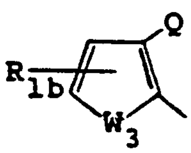, 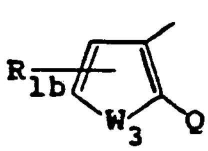, 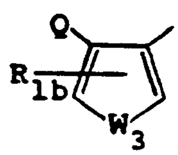, 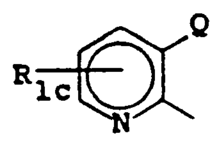, 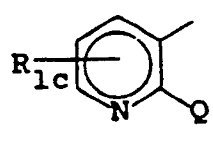,

J-5 J-6 J-7 J-8 J-9

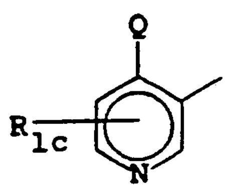 oder 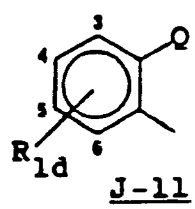 ist;

J-10 J-11

$W_1$ O oder S ist;

R H oder $CH_3$ ist;

$W_3$ O, S oder NR'' ist;

$R_{1b}$, $R_{1c}$ und $R_{1d}$ unabhängig H, $C_1$—$C_3$-Alkyl, $C_1$—$C_3$-Halogenalkyl, $C_1$—$C_3$-Halogenalkoxy, Halogen, Nitro, $C_1$—$C_3$-Alkoxy, $SO_2NR'R''$, $C_1$—$C_3$-Alkylthio, $C_1$—$C_3$-Alkylsulfinyl, $C_1$—$C_3$-Halogenalkylthio, $C_1$—$C_3$-Alkylsulfonyl, $CO_2R'''$, Amino, $C_1$—$C_3$-Alkylamino, Di($C_1$—$C_3$-alkyl)amino, $CH_2CN$, $CH_2OCH_3$ oder $CH_2SCH_3$ sind;

R' H, $C_1$—$C_4$-Alkyl, $C_2$—$C_3$-Cyanalkyl, Methoxy oder Ethoxy ist;

R'' H, $C_1$—$C_4$-Alkyl oder $C_3$—$C_4$-Alkenyl ist; oder

R' und R''' als $—(CH_2)_3—$, $—(CH_2)_4—$, $—(CH_2)_5—$ oder $—CH_2CH_2OCH_2CH_2—$ zusammengenommen werden können; R''' $C_1$—$C_4$-Akyl, $C_3$—$C_4$-Alkenyl, $C_3$—$C_4$-Alkinyl, $C_2$—$C_4$-Halogenalkyl, $C_2$—$C_3$-Cyanalkyl, $C_5$—$C_6$-Cycloalkyl, $C_4$—$C_7$-Cycloalkylalkyl oder $C_2$—$C_4$-Alkoxyalkyl ist;

Q 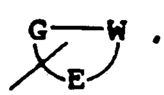, 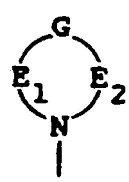 oder 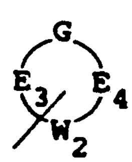 ist;

$Q_1$ $Q_2$ $Q_3$

G C=O oder $SO_2$ ist;

W, O, S, $CHR_2$ oder $NR_3$ ist;

$W_2$ O, S, $SO_2$, $CHR_2$ oder $NR_3$ ist;

$R_2$ H, $C_1$—$C_2$-Alkyl, Cl, F oder Br ist;

$R_3$ H, $C_1$—$C_4$-Alkyl, $C_1$—$C_4$-Halogenalkyl, $C_2$—$C_4$-Alkoxyalkyl, $C_2$—$C_4$-Cyanalkyl, $C_3$—$C_4$-Alkenyl oder $C_3$—$C_4$-Alkinyl ist;

E $C_3$—$C_4$-Alkylen, $C_3$—$C_4$-Alkenylen oder $C_4$-Alkenyl-dienyl ist;

$E_1$ und $E_3$ unabhängig $C_1$—$C_2$-Alkylen oder $C_2$-Alkenylen sind;

$E_3$ und $E_4$ unabhängig $C_2$—$C_3$-Alkylen oder $C_2$—$C_3$-Alkenylen sind; und

E, $E_1$, $E_2$, $E_3$ und $E_4$ gegebenenfalls durch 1 bis 4 aus $C_1$—$C_4$-Alkyl, $C_1$—$C_4$-Alkoxy, $C_1$—$C_4$-Alkenyl, OH, Halogen und $C_1$—$C_4$-Halogenalkoxy ausgewählte Gruppen subsituiert sein können; ferner, wenn W O, $CHR_2$ oder $NR_3$ ist, eines der Kohlenstoffatome von E in Form einer Carbonylgruppe vorliegen kann, und wenn $W_2$ O, $CHR_2$ oder $NR_3$ ist, eines der Kohlenstoffatome von $E_3$ oder $E_4$ in Form einer Carbonylgruppe vorliegen kann, mit der Massgabe, dass diese Carbonylgruppen nicht direkt an G gebunden sind;

A 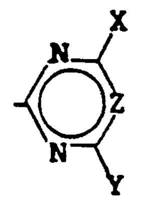 , 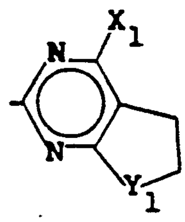 , 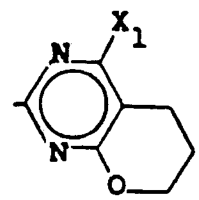 ,

A-1 A-2 A-3

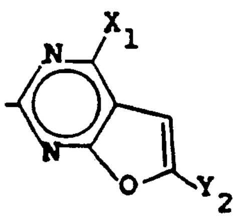 , 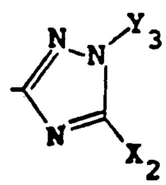 oder 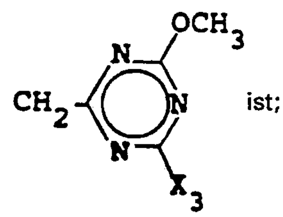 ist;

A-4 A-5 A-6

X H, $C_1$—$C_4$-Aklyl, $C_1$—$C_4$-Alkoxy, $C_1$—$C_4$-Halogenalkoxy, $C_1$—$C_4$-Halogenalkyl, $C_1$—$C_4$-Halogenalkylthio, $C_1$—$C_4$-Alkylthio, Halogen, $C_2$—$C_5$-Alkoxyalkyl, $C_2$—$C_5$-Akoxyalkoxy, Amino, $C_1$—$C_3$-Alkylamino oder Di($C_1$—$C_3$-alkyl)amino ist;

Y H, $C_1$—$C_4$-Alkyl, $C_1$—$C_4$-Alkoxy, $C_1$—$C_4$-Halogenalkoxy, $C_1$—$C_4$-Halogenalkylthio, $C_1$—$C_4$-Alkylthio, $C_2$—$C_5$-Alkoxyalkyl, $C_2$—$C_5$-Alkoxyalkoxy, Amino, $C_1$—$C_3$-Alkylamino, Di($C_1$—$C_3$-alkyl)amino, $C_3$—$C_4$-Alkenyloxy, $C_3$—$C_4$-Alkinyloxy, $C_2$—$C_5$-Alkylthioalkyl, $C_1$—$C_4$-Halogenalkyl, $C_3$—$C_5$-Cycloalkyl, $C_2$—$C_4$-Alkinyl,

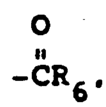 , 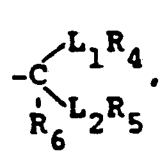 , 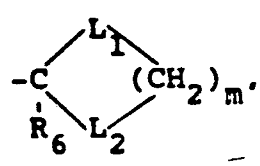 ,

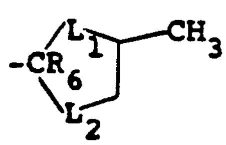 , $N(OCH_3)CH_3$,

$C_2$—$C_5$-Alkylsulfinylalkyl oder $C_2$—$C_5$-Alkylsulfonylalkyl ist;

m 2 oder 3 ist;

$L_1$ und $L_2$ unabhängig O oder S sind;

$R_4$ und $R_5$ unabhängig $C_1$—$C_2$-Alkyl sind;

$R_6$ H oder $CH_3$ ist;

Z CH oder N ist;

$Y_1$ O oder $CH_2$ ist;

$X_1$ $CH_3$, $OCH_3$, $OC_2H_5$ oder $OCF_2H$ ist;
$Y_2$ H oder $CH_3$ ist;
$X_2$ $CH_2$, $OCH_3$ oder $SCH_3$ ist;
$Y_3$ $CH_3$, $CH_2CH_3$ oder $CH_2CF_3$ ist; und
$X_3$ $CH_3$ oder $OCH_3$ ist;
und ihre landwirtschaftlich geeigneten Salze;
mit der Massgabe, dass
a) wenn G $SO_2$ ist, dann W O, $CHR_2$ oder $NR_3$ ist;
b) wenn $E_1$ oder $E_3$ $C_2$-Alkylen oder $C_2$-Alkenylen ist, dann $E_2$ oder $E_4$ $C_2$-Alkylen oder $C_2$-Alkenylen ist;
c) wenn X Cl, F, Br oder I ist, dann Z CH und Y $OCH_3$, $OC_2H_5$, $N(OCH_3)CH_3$, $NHCH_3$, $N(CH_3)_2$ oder $OCF_2H$ ist;
d) wenn X oder Y $OCF_2H$ ist, dann Z CH ist;
e) wenn die Gesamtzahl der Kohlenstoffatome von X und Y grösser als 4 ist, dann die Zahl der Kohlenstoffe von $R_{1b}$, $R_{1c}$ oder $R_{1d}$ weniger als oder gleich 2 ist und die Zahl der Kohlenstoffe von Q weniger als gleich 8 ist; und
f) wenn $W_1$ S ist, dann R H ist, A A—1 ist und Y

$DH_3$, $OCH_3$, $OC_2H_5$, $CH_2OCH_3$, $C_2H_5$, $CF_3$, $SCH_3$, $OCH_2CH{=}CH_2$, $OCH_2C{\equiv}CH$, $OCH_2CH_2OCH_3$, $CH(OCH_3)_2$ oder 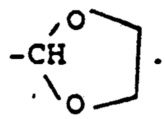 ist.

2. Verbindungen nach Anspruch 1, worin $W_1$ O ist und R H ist.
3. Verbindungen nach Anspruch 2, worin
$R_{1b}$ H, Cl, Br, $NO_2$, $CH_3$, $OCH_3$, oder $CF_3$ ist;
$R_{1c}$ H, Cl, $CH_3$, $OCH_3$ oder $N(CH_3)_2$ ist;
$R_{1d}$ H, $CH_3$, $OCH_3$, Cl, Br, F, $NO_2$, $CF_3$ oder $OCF_2H$ ist und nicht in der 4-Stellung ist;
$W_3$ S ist;
X $CH_3$, $OCH_3$, $OCH_2CH_3$, Cl, F, Br, I, $OCF_2H$, $CH_2F$, $OCH_2CH_2F$, $OCH_2CHF_2$, $OCH_2CF_3$, $CF_3$, $CH_2Cl$ oder $CH_2Br$ ist;
Y

N, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH{=}CH_2$, $OCH_2C{\equiv}CH$, $CH_2OCH_2CH_3$, $OCH_2CH_2OCH_3$, $CH_2SCH_3$, 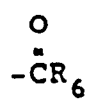, 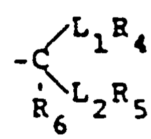, 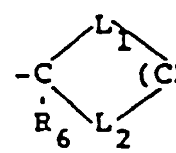, 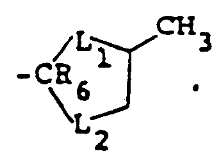, $OCF_2H$, $SCF_2H$, Cyclopropyl, $C{\equiv}CH$ oder $C{\equiv}CCH_3$ ist.

4. Verbindungen nach Anspruch 3, worin Q $Q_1$ ist.
5. Verbindungen nach Anspruch 3, worin Q $Q_2$ ist.
6. Verbindungen nach Anspruch 3, worin Q $Q_3$ ist.
7. Verbindungen nach Anspruch 3, worin Q

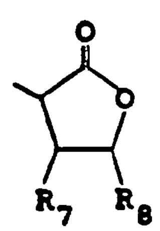
Q-1

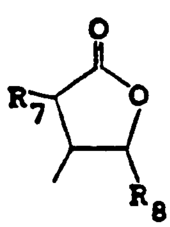
Q-2

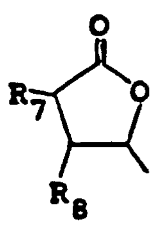
Q-3

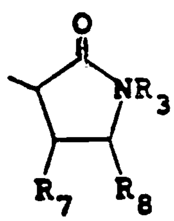
Q-4

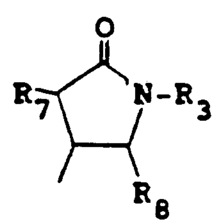
Q-5

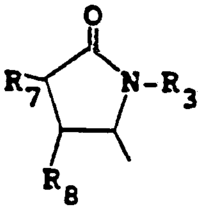,

Q-6

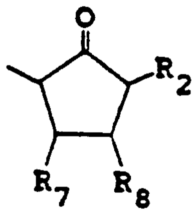,

Q-8

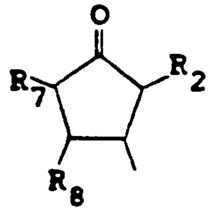,

Q-9

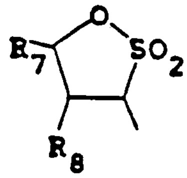,

Q-10

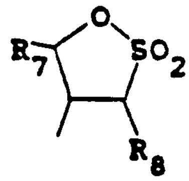,

Q-11

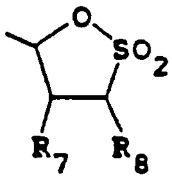,

Q-12

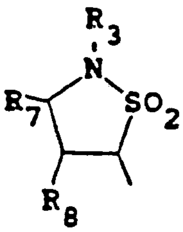,

Q-13

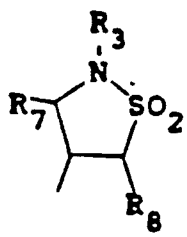,

Q-14

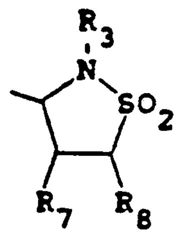,

Q-15

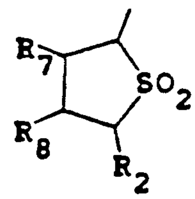,

Q-17

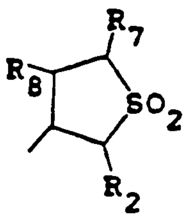,

Q-18

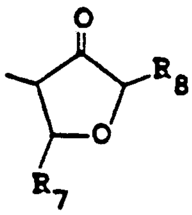,

Q-19

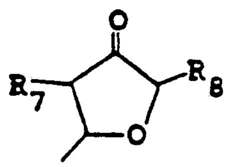,

Q-20

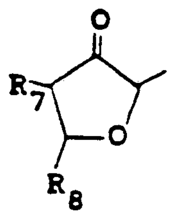,

Q-21

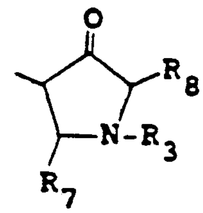,

Q-22

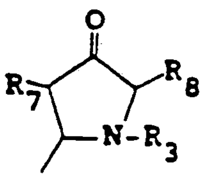,

Q-23

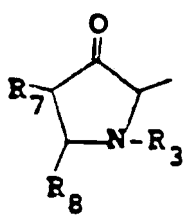,

Q-24

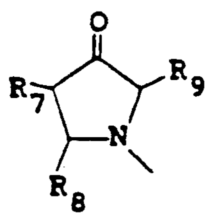,

Q-25

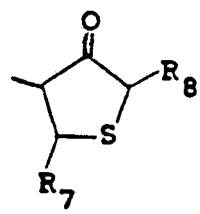,

Q-26

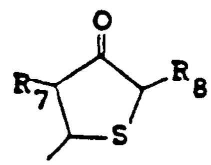,

Q-27

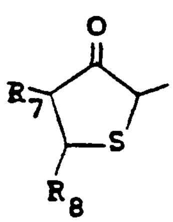,

Q-28

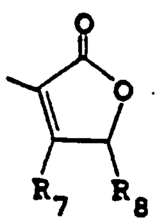,

Q-29

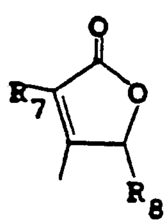,

Q-30

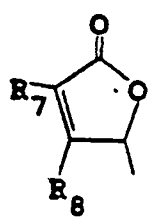,

Q-31

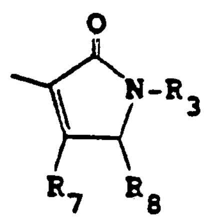,

Q-32

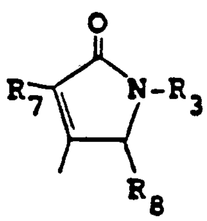,

Q-33

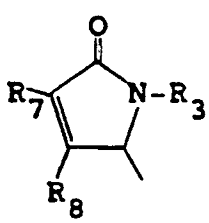,

Q-34

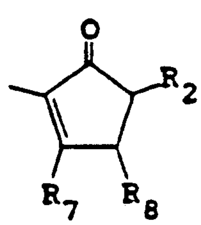,

Q-36

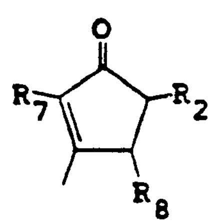,

Q-37

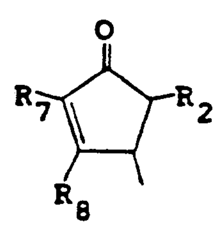,

Q-38

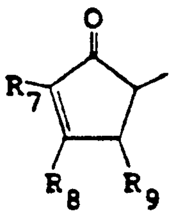 ,
Q-39

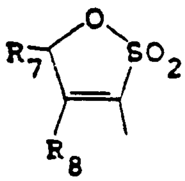 ,
Q-40

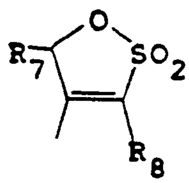 ,
Q-41

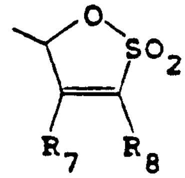 ,
Q-42

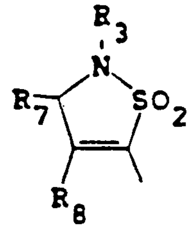 ,
Q-43

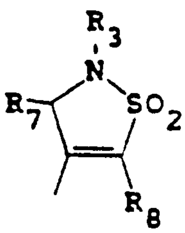 ,
Q-44

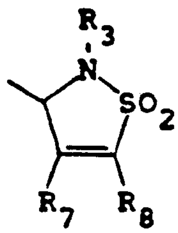 ,
Q-45

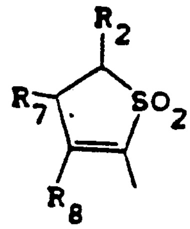 ,
Q-47

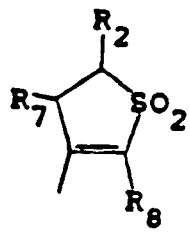 ,
Q-48

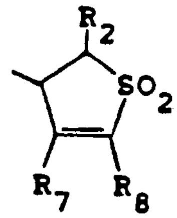 ,
Q-49

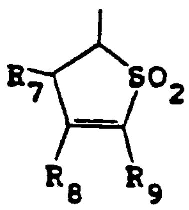 ,
Q-50

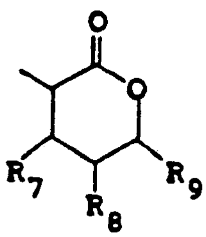 ,
Q-51

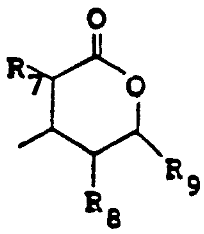 ,
Q-52

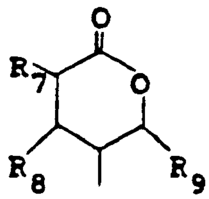 ,
Q-53

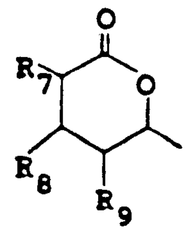 ,
Q-54

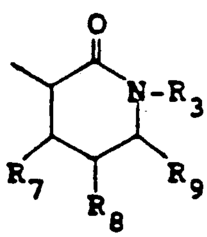 ,
Q-55

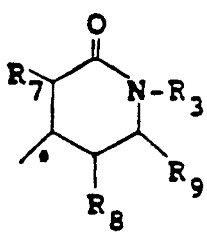 ,
Q-56

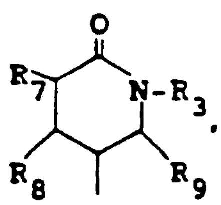 ,
Q-57

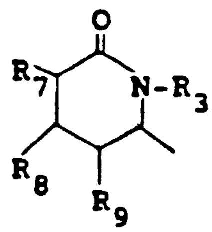 ,
Q-58

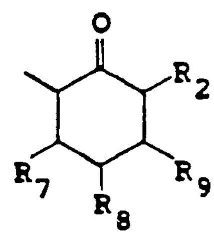 ,
Q-60

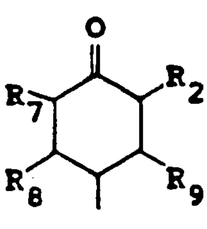 ,
Q-61

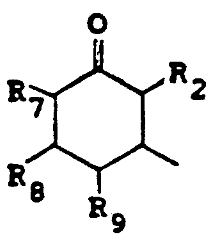 ,
Q-62

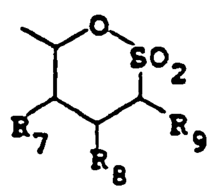 ,
Q-63

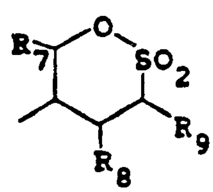 ,
Q-64

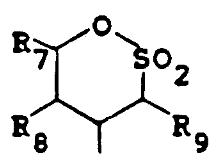 ,
Q-65

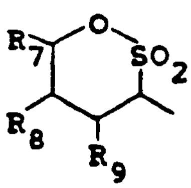 ,
Q-66

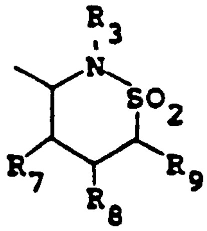 ,
Q-67

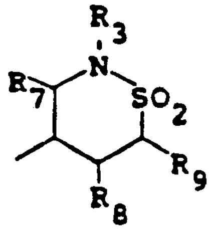 ,
Q-68

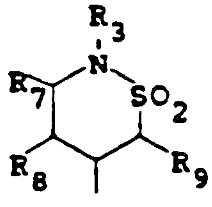 ,
Q-69

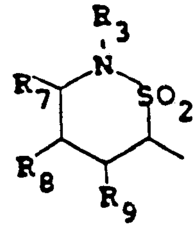 ,
Q-70

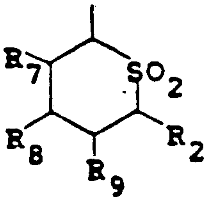 , 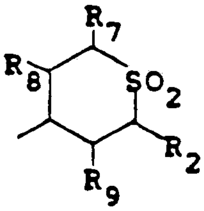 , 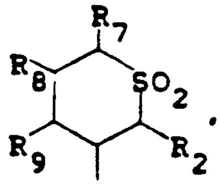 , 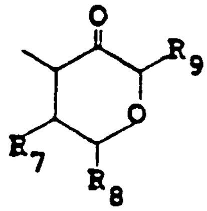 , 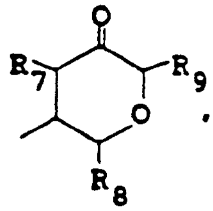 ,

Q-72 Q-73 Q-74 Q-75 Q-76

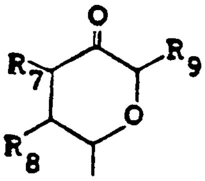 , 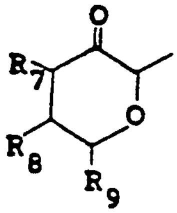 , 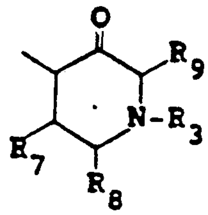 , 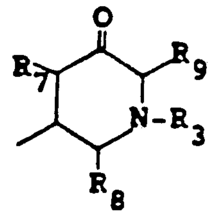 , 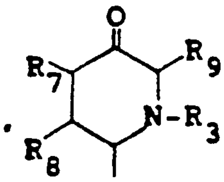 ,

Q-77 Q-78 Q-79 Q-80 Q-81

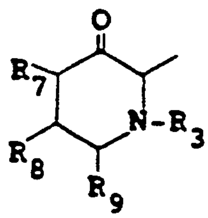 , 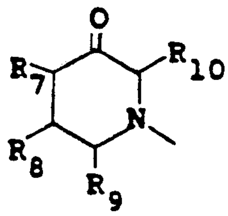 , 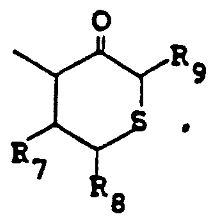 , 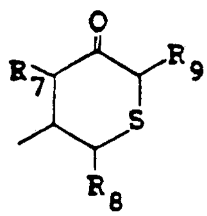 , 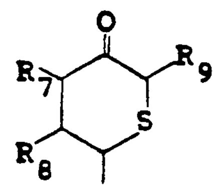 ,

Q-82 Q-83 Q-84 Q-85 Q-86

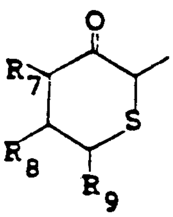 , 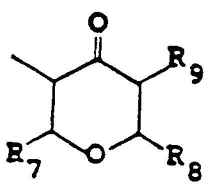 , 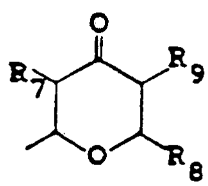 , 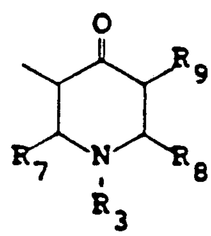 , 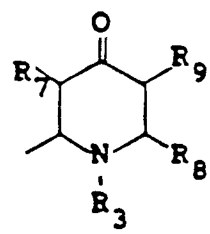 ,

Q-87 Q-88 Q-89 Q-90 Q-91

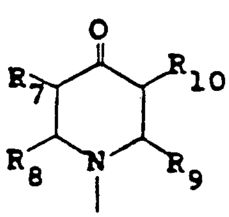 , 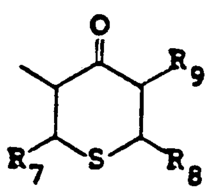 , 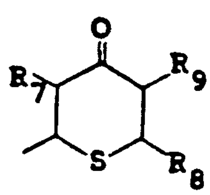 , 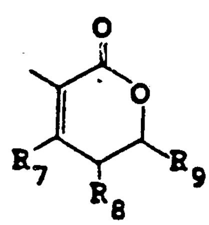 , 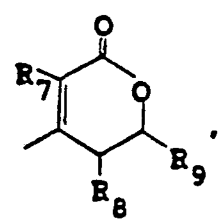 ,

Q-92 Q-93 Q-94 Q-95 Q-96

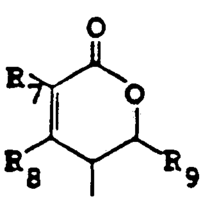 , 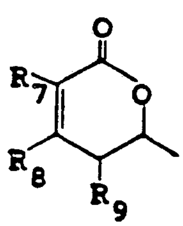 , 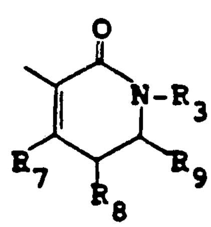 , 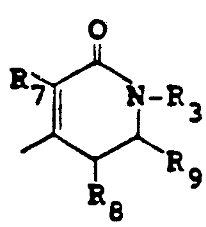 , 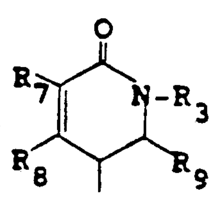 ,

Q-97 Q-98 Q-99 Q-100 Q-101

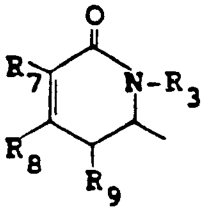 , 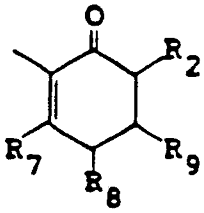 , 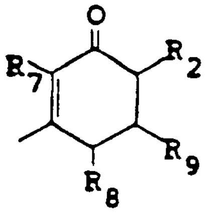 , 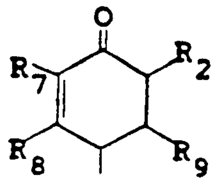 , 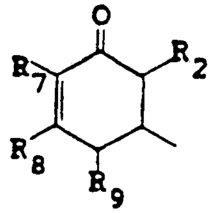 ,

Q-102 Q-104 Q-105 Q-106 Q-107

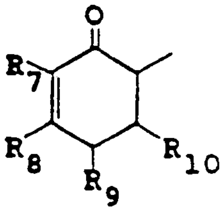 , 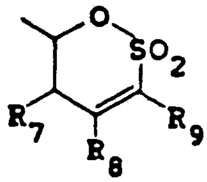 , 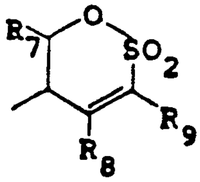 , 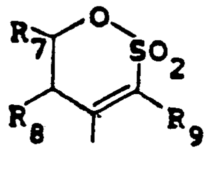 , 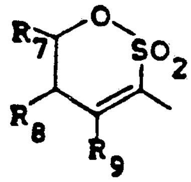 ,

Q-108 Q-109 Q-110 Q-111 Q-112

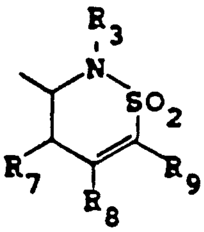 , 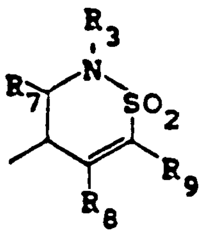 , 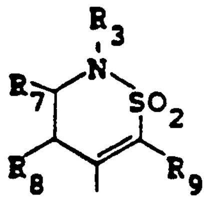 , 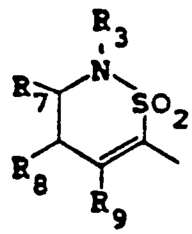 , 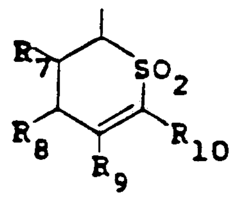 ,

Q-113 Q-114 Q-115 Q-116 Q-118

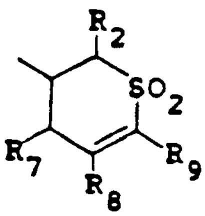 , 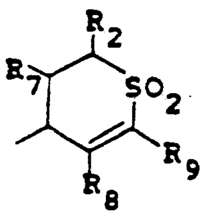 , 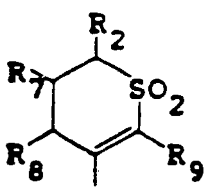 , 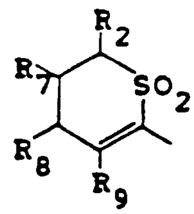 , 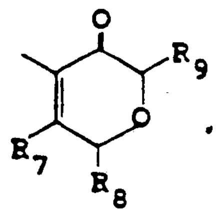 ,

Q-119 Q-120 Q-121 Q-122 Q-123

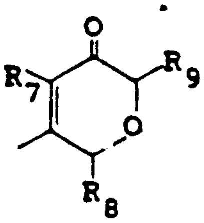 , 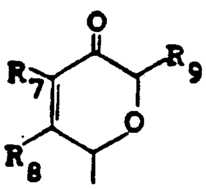 , 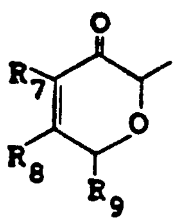 , 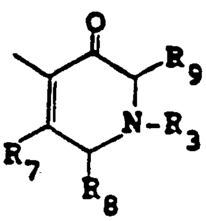 , 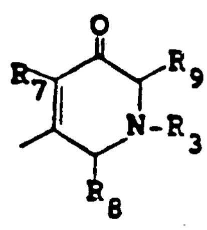 ,

Q-124 Q-125 Q-126 Q-127 Q-128

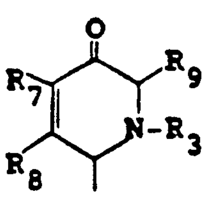 , 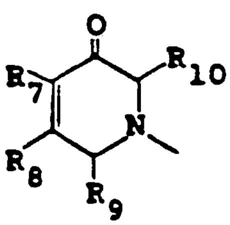 , 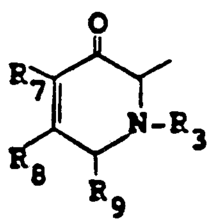 , 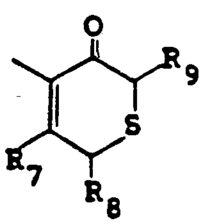 , 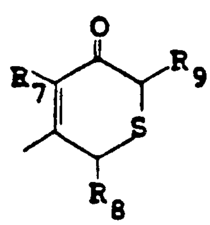 ,

Q-129 Q-130 Q-131 Q-132 Q-133

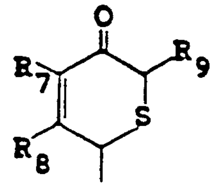 , 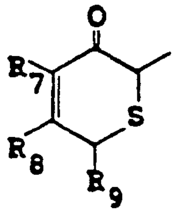 , 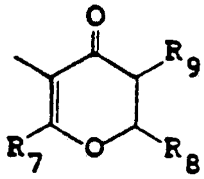 , 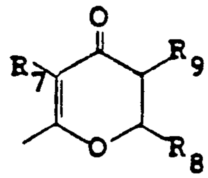 , 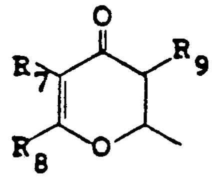 ,

Q-134 Q-135 Q-136 Q-137 Q-138

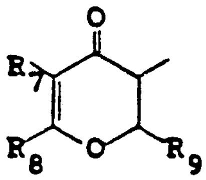 , 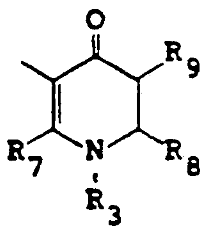 , 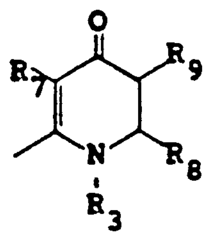 , 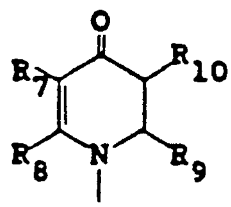 , 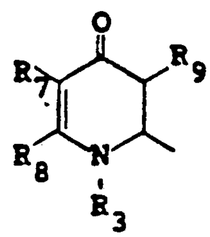 ,

Q-139 Q-140 Q-141 Q-142 Q-143

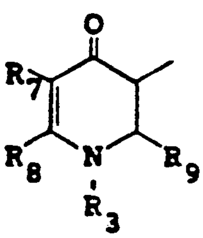 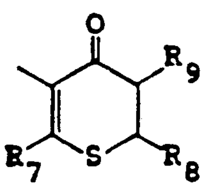 , 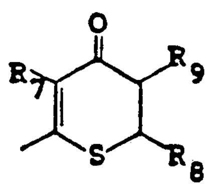 , 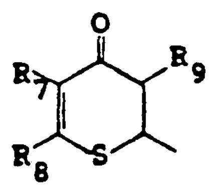 , 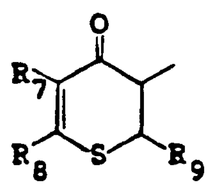 ,

Q-144 Q-145 Q-146 Q-147 Q-148

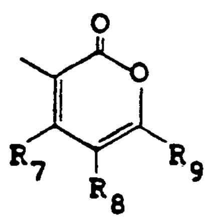 , 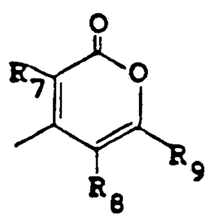 , 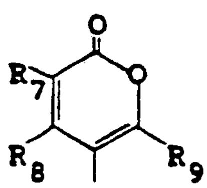 , 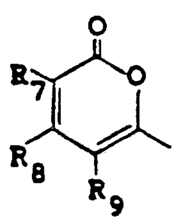 , 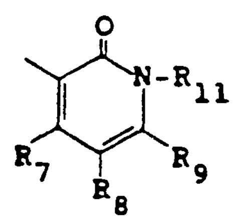 ,

Q-149 Q-150 Q-151 Q-152 Q-153

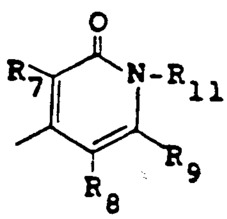 , 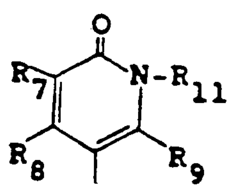 , 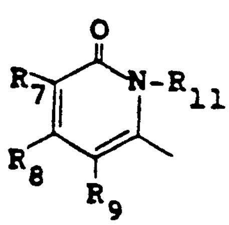 , 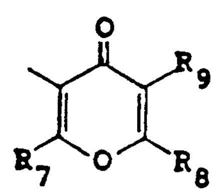 , 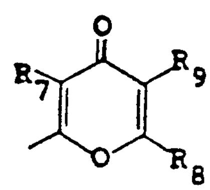 ,

Q-154 Q-155 Q-156 Q-158 Q-159

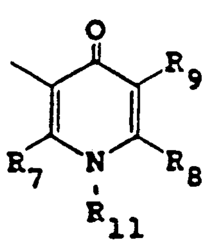 , 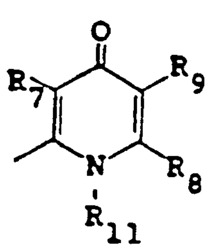 , 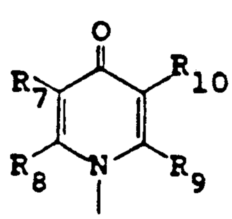 , 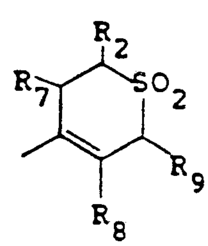 , 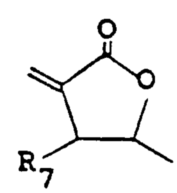 ,

Q-160 Q-161 Q-162 Q-163 Q-164

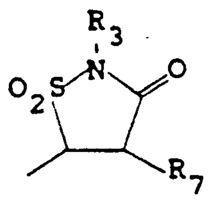, 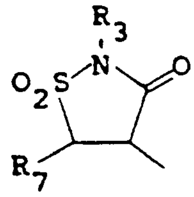 oder 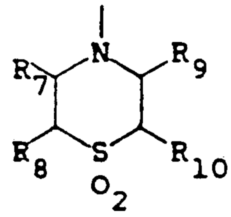 ist;

Q-165 Q-166 Q-167

$R_7$, $R_8$, $R_9$ und $R_{10}$ unabhängig H oder $CH_3$ sind; und $R_{11}$ H, $CH_3$ oder $CH_2CH_3$ ist.

8. Verbindungen nach Anspruch 7, worin J J—5, J—6, J—8, J—9 oder J—11 ist;
$R_{1b}$ H, $CH_3$ oder Cl ist;
$R_{1c}$ H ist;
$R_{1d}$ H, $CH_3$, $OCH_3$ oder Cl ist; und
Y $CH_3$, $OCH_3$, $C_2H_5$, $OC_2H_5$, $CH_2OCH_3$, $NHCH_3$, $CH(OCH_3)_2$ oder Cyclopropyl ist.

9. Verbindung nach Anspruch 8, worin
$R_2$ H oder $CH_3$ ist; und
$R_3$ H, $CH_3$ oder $C_2H_5$ ist.

10. Verbindungen nach Anspruch 9, worin
A A—1 ist; und
X $CH_3$, $OCH_3$, Cl oder $OCF_2H$ ist.

11. Verbindung nach Anspruch 1, welche N-[(4,6-Di-methoxypyrimidin-2-yl)aminocarbonyl]-2-tetrahydro-2-oxo-3-furanylbenzolsulfonamid ist.

12. Verbindung nacn Anspruch 1, welche N-[(4-Methoxy-6-methyltriazin-2-yl)aminocarbonyl]-2-tetrahydro-2-oxo-3-furanylbenzolsulfonamid ist.

13. Verbindung nach Anspruch 1, welche N-[(4,6-Dimethoxypyrimidin-2-yl)aminocarbonyl]-2-(tetrahydro-2-oxofuran-3-yl)-3-thiophensulfonamid ist.

14. Verbindung nach Anspruch 1, welche N-[(4-Methoxy-6-methyltriazin-2-yl)aminocarbonyl]-2-(tetrahydro-2-oxofuran-3-yl)-3-thiophensulfonamid ist.

15. Verbindung nach Anspruch 1, welche N-[(4,6-Dimethyl-pyrimidin-2-yl)aminocarbonyl]-2-(3-oxo-1-cyclohexen-1-yl)-3-thiophensulfonamid ist.

16. Verbindung nach Anspruch 1, welche N-[(4,6-Dimethoxypyrimidin-2-yl)aminocarbonyl]-2-(tetrahydro-2-oxofuran-3-yl)-3-pyridinsulfonamid ist.

17. Verbindung nach Anspruch 1, welche N-[(4-Methoxy-6-methyltriazin-2-yl)aminocarbonyl]-2-(tetrahydro-2-oxofuran-3-yl)-3-pyridinsulfonamid ist.

18. Verbindung nach Anspruch 1, welche N-[(4,6-Dimethoxypyrimidin-2-yl)aminocarbonyl]-2-(4-thiomorpholinyl)-3-pyridinsulfonamid-S,S-dioxid ist.

19. Verbindungen nach Anspruch 1, worin
J J—11 ist;
$R_{1d}$ $H_1$, $C_1$—$C_3$-Alkyl, $C_1$—$C_3$-Halogenalkyl, Halogen, Nitro, $C_1$—$C_3$-Alkoxy, $C_1$—$C_3$-Alkylthio, $C_1$—$C_3$-Alkylsulfinyl, $C_1$—$C_3$-Alkylsulfonyl, $SO_2NR^IR^{II}$, $CO_2R^{III}$, Amino, $C_1$—$C_3$-Alkylamino oder Di-($C_1$—$C_3$-alkyl)amino ist;
$W_2$ nicht $SO_2$ ist;
E, $E_1$, $E_2$, $E_3$ und $E_4$ gegebenenfalls durch ein bis vier aus $C_1$—$C_4$-Alkyl, $C_1$—$C_4$-Alkoxy, Halogen oder $C_1$—$C_4$-Halogenalkoxy ausgewählte Gruppen substituiert sein können;
Y nicht $C_2$—$C_5$-Alkylsulfinylalkyl oder $C_2$—$C_5$-Alkylsulfonylalkyl ist;
und die verbleibenden Substituenten wie in Anspruch 1 definiert sind.

20. Verbindungen nach Anspruch 1, worin
J J—5, J—6 oder J—7 ist;
$R_{1b}$ H, $C_1$—$C_3$-Alkyl, $C_1$—$C_3$-Halogenalkyl, Halogen, Nitro, $C_1$—$C_3$-Alkoxy, $SO_2NR^IR^{II}$, $C_1$—$C_3$-Alkylthio, $C_1$—$C_3$-Alkylsulfinyl, C1—$C_3$-Alkylsulfonyl oder $CO_2R^{III}$ ist;
$W_2$ nicht $SO_2$ ist;
und E, $E_1$, $E_2$, $E_3$, $E_4$ und Y wie in Anspruch 19 definiert sind;
die verbleibenden Substituenten wie in Anspruch 1 definiert sind.

21. Zur Bekämpfung des Wachstums unerwünschter Vegetation geeignete Zusammensetzung, welche eine wirksame Menge einer Verbindung nach einem der Ansprüche 1 bis 20 und wenigstens eines der folgenden: oberflächenaktives Mittel, festes oder flüssiges Verdünnungsmittel, umfasst.

22. Verfahren zur Bekämpfung des Wachstums unerwünschter Vegetation, welches die Anwendung einer wirksamen Menge einer Verbindung nach einem der Ansprüche 1 bis 20 auf den zu schützenden Ort umfasst.

23. Verfahren zur Steuerung des Wachstums von Pflanzen, welches die Anwendung einer wirksamen, jedoch im wesentlichen nicht-phytotoxischen Menge eines Pflanzenwachstumsregulanz, ausgewählt aus Verbindungen nach einem der Ansprüche 1 bis 20, auf den Ort solcher Pflanzen umfasst.

24. Verfahren zur Herstellung einer Verbindung nach Anspruch 1 durch
(a) Umsetzen eines Sulfonylisocyanates oder Isothiocyanats der Formel

$$J{-}SO_2NCW_1 \qquad (II)$$

mit einem heterocyclischen Amin der Formel

$$A{-}NHR \qquad (III);\ \text{oder}$$

(b) Umsetzen eines Sulfonamids der Formel

$$J{-}SO_2HN_2 \qquad (IV)$$

mit dem Methyl- oder Phenylester einer Pyrimidin-oder Triazincarbaminsäure der Formel

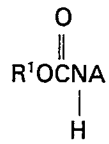

(V, VI)

worin J, A, R und $W_1$ wie in Anspruch 1 definiert sind und $R^1$ Me oder Ph sind.

25. Verbindungen der Formeln

$$J{-}SO_2NH_2 \text{ und } J{-}SO_2NCW_1$$

worin J und $W_1$ wie in Anspruch 1 definiert sind.

**Revendications**

1. Un composé de la formule:

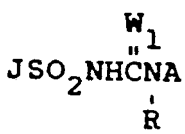

où

I

J est

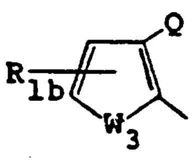, 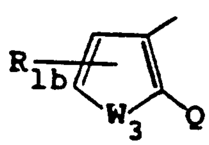, 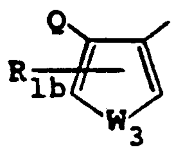, 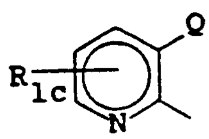, 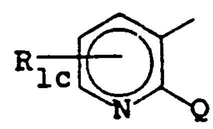,

J-5 J-6 J-7 J-8 J-9

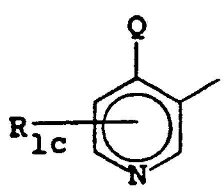 ou 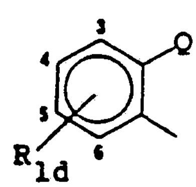 ;

J-10 J-11

$W_1$ est O ou S;
R est H ou $CH_3$;
$W_3$ est O. S ou $NR^{II}$;
$R_{1b}$, $R_{1c}$, et $R_{1d}$ sont chacun indépendamment H ou un groupe alkyle en $C_1$—$C_3$, halogénoalkyle en $C_1$—$C_3$ halogénoalcoxy en $C_1$—$C_3$, halogéno, nitro, alcoxy en $C_1$—$C_3$, $SO_2NR^IR^{II}$, alkylthio en $C_1$—$C_3$, alkylsulfinyle en $C_1$—$C_3$, halogénoalkylthio en $C_1$—$C_3$. alkylsulfonyle en $C_1$—$C_3$, $CO_2R^{III}$, amino, alkylamino en $C_1$—$C_3$, di(alkyl en $C_1$—$C_3$)amino, $CH_2CN$, $CH_2OCH_3$ ou $CH_2SCH_3$;

$R^I$ est H ou un groupe alkyle en $C_1$—$C_4$, cyanoalkyle en $C_2$—$C_3$, méthoxy ou éthoxy;

$R^{II}$ est H ou un groupe alkyle en $C_1$—$C_4$ ou alcényle en $C_3$—$C_4$; ou bien

$R^I$ et $R^{II}$ peuvent être pris ensemble pour former —$(CH_2)_3$—, —$(CH_2)_4$—, —$(CH_2)_5$— ou —$CH_2CH_2OCH_2CH_2$—;

$R^{III}$ est un groupe alkyle en $C_1$—$C_4$, alcényle en $C_3$—$C_4$, alcynyle en $C_3$—$C_4$, halogénoalkyle en $C_2$—$C_4$, cyanoalkyle en $C_2$—$C_3$, cycloalkyle en $C_5$—$C_6$, cycloalkylalkyle en $C_4$—$C_7$ ou alcoxyalkyle en $C_2$—$C_4$;

Q est 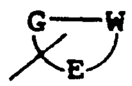 , 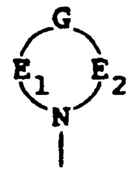 ou 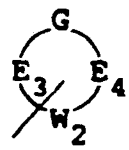 ;

$Q_1$ $Q_2$ $Q_3$

G est C=O ou $SO_2$;

W est O. S. $CHR_2$ ou $NR_3$;

$W_2$ est O. S. $SO_2$ $CHR_2$ ou $NR_7$;

$R_2$ est H, un groupe alkyle en $C_1$—$C_2$, Cl, F ou Br;

$R_3$ est H, un groupe alkyle en $C_1$—$C_4$, halogénoalkyle en $C_1$—$C_4$, alcoxyalkyle en $C_2$—$C_4$, cyanoalkyle en $C_2$—$C_4$, alcényle en $C_3$—$C_4$ ou alcynyle en $C_3$—$C_4$;

E est un groupe alkylène en $C_3$—$C_4$, alcénylène en $C_3$—$C_4$ ou alcényldiényle en $C_4$;

$E_1$ et $E_3$ sont chacun indépendamment un groupe alkylène en $C_1$—$C_2$ ou alcénylène en $C_2$;

$E_2$ et $E_4$ sont chacun indépendamment un groupe alkylène en $C_2$—$C_3$ ou alcénylène en $C_2$—$C_3$; et

E, $E_1$, $E_2$, $E_3$ et $E_4$ peuvent être facultativement substitués par 1 à 4 groupes choisis parmi les groupes alkyles en $C_1$—$C_4$, alcoxy en $C_1$—$C_4$, alcényles en $C_1$—$C_4$, OH, halogéno et halogénalcoxy en $C_1$—$C_4$; de plus, lorsque W est O, $CHR_2$ ou $NR_3$, l'un des atomes de carbone de E peut être sous la forme d'un groupe carbonyle, et lorsque $W_2$ est O, $CHR_2$ ou $NR_3$, l'un des atomes de carbone de $E_3$ ou $E_4$ peut être sous la forme d'un groupe carbonyle, avec la condition que lesdits groupes carbonyle ne soient pas directement liés à G;

A est 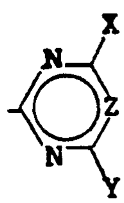 , 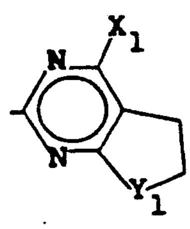 , 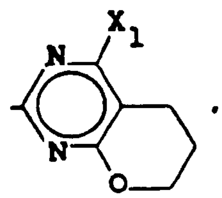 ,

A-1 A-2 A-3

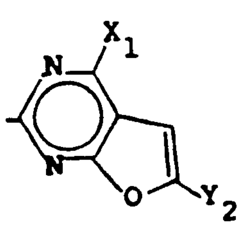 , 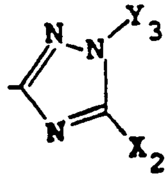 ou 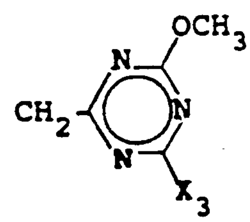 ;

A-4 A-5 A-6

X est H ou un groupe alkyle en $C_1$—$C_4$, alcoxy en $C_1$—$C_4$, halogénoalcoxy en $C_1$—$C_4$, halogénoalkyle en $C_1$—$C_4$, halogénoalkylthio en $C_1$—$C_4$, alkylthio en $C_1$—$C_4$, halogéno, alcoxyalkyle en $C_2$—$C_5$, alcoxyalcoxy en $C_2$—$C_5$, amino, alkylamino en $C_1$—$C_3$ ou di(alkyl en $C_1$—$C_3$)amino;

Y est H ou un groupe alkyle en $C_1$—$C_4$, alcoxy en $C_1$—$C_4$halogénoalcoxy en $C_1$—$C_4$, halogénoalkylthio en $C_1$—$C_4$, alkylthio en $C_1$—$C_4$, alcoxyalkyle en $C_2$—$C_5$, alcoxyalcoxy en $C_2$—$C_5$, amino, alkylamino en $C_1$—$C_3$, di(alkyl en $C_1$—$C_3$)amino, alcényloxy en $C_3$—$C_4$, alcynyloxy en $C_3$—$C_4$, alkylthiolakyle en $C_2$—$C_5$, halogénoalkyle en $C_1$—$C_4$, cycloalkyle en $C_3$—$C_5$, alcynyle en $C_2$—$C_4$,

$C_2$—$C_4$ alkynyl, 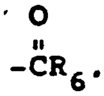 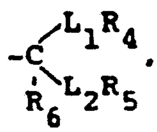 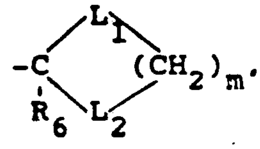

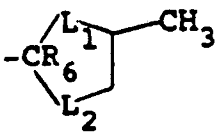 , N(OCH$_3$)CH$_3$.

alkylsulfinylalkyle en $C_2$—$C_5$ ou alkylsulfonylalkyle en $C_2$—$C_5$;

m est 2 ou 3;

$L_1$ et $L_2$ sont chacun indépendamment O ou S;

$R_4$ et $R_5$ sont chacun indépendamment un groupe alkyl $C_1$—C2;

$R_6$ est H ou $CH_3$;

Z est CH ou N;

$Y_1$ est O ou $CH_2$;

$X_1$ est $CH_3$, $OCH_3$, $OC_2H_5$ ou $OCF_2H$;

$Y_2$ est H ou $CH_3$;

$X_2$ est $CH_3$, $OCH_3$ ou $SCH_3$;

$Y_3$ est $CH_3$, $CH_2CH_3$ ou $CH_2CF_3$; et

$X_3$ est $CH_3$ ou $OCH_3$;

et ses sels utilisables en agriculture;

avec les conditions suivantes:

a) si G est $SO_2$, alors W est O, $CHR_2$ ou $NR_3$;

b) si $E_1$ ou $E_2$ est un groupe alkylène en $C_2$ ou alcénylène en $C_2$, alors $E_2$ ou $E_4$ est un groupe alkylène en $C_2$ ou alcénylène en $C_2$;

c) si X est Cl, F, Br ou I, alors Z est CH et Y est $OCH_3$, $OC_2H_5$, $N(OCH_3)CH_3$, $NHCH_3$, $N(CH_3)_2$ ou $OCF_2H$;

d) si X ou Y est $OCF_2H$, alors Z est CH;

e) si le nombre total d'atomes de carbone de X et Y est supérieur à quatre, alors le nombre d'atomes de carbone de $R_{1b}$, $R_{1c}$ ou $R_{1d}$ est inférieur ou égal à deux et le nombre d'atomes de carbone de Q est inférieur ou égal à huit; et

f) si W est S, alors R est H, A est A—1 et Y est

$CH_3$, $OCH_3$, $OC_2H_5$, $CH_2OCH_3$, $C_2H_5$, $CF_3$, $SCH_3$, $OCH_2CH = CH_2$, $OCH_2$ -CH, $OCH_2CH_2OCH_3$, $CH(OCH_3)_2CH_3$. $OCH_3$. $OC_2H_5$. $CH_2OCH_3$. $C_2H_5$. $CF_3$. $SCH_3$. $OCH_2CH=CH_2$. $OCH_2C{\equiv}CH$. $OCH_2CH_2OCH_3$. $CH(OCH_3)_2$

ou 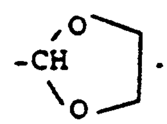 .

2. Composés de la revendication 1, où $W_1$ est O; et R est H.

3. Composés de la revendication 2, où

$R_{1b}$ est H, Cl, Br, $NO_2$, $CH_3$, $OCH_3$ ou $CF_3$;

$R_{1c}$ est H, Cl, $CH_3$, $OCH_3$ ou $N(CH_3)_2$;

$R_{1d}$ est H, $CH_3$, $OCH_3$, Cl, Br, F, $NO_2$, $CF_3$ ou $OCF_2H$ et n'est pas à la position 4;

$W_3$ est S;

X est $CH_3$, $OCH_3$, $OCH_2CH_3$, Cl, F, Br, I, $OCF_2H$, $CH_2F$, $OCH_2CH_2F$, $OCH_2CHF_2$, $OCH_2CF_3$, $CF_3$, $CH_2CL$ ou $CH_2Br$;

Y est

H, $CH_3$. $OCH_3$. $OC_2H_5$. $CH_2OCH_3$. $NHCH_3$. $N(OCH_3)CH_3$. $N(CH_3)_2$. $CH_2CH_3$. $CF_3$. $SCH_3$. $OCH_2CH=CH_2$. $OCH_2C{\equiv}CH$. $CH_2OCH_2CH_3$. $OCH_2CH_2OCH_3$.

$CH_2SCH_3$. 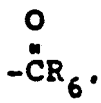 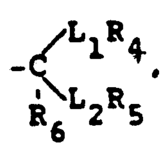 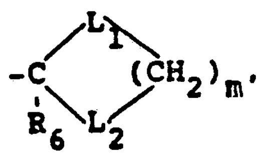 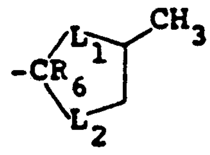 .

$OCF_2H$, $SCF_2H$, cyclopropyl, C≡CH or C≡$CCH_3$.

4. Composés de la revendication 3, où Q est $Q_1$.
5. Composés de la revendication 3, où Q est $Q_2$.
6. Composés de la revendication 3, où Q est $Q_3$.
7. Composés de la revendication 3, où Q est

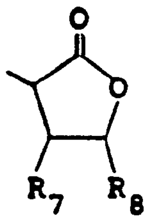
Q-1
,
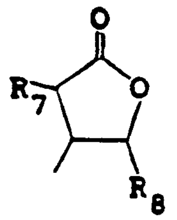
Q-2
,
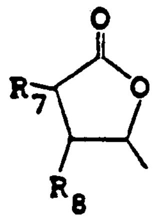
Q-3
,
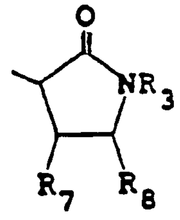
Q-4
,
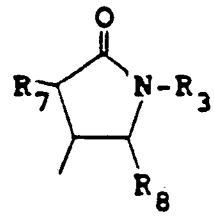
Q-5
,
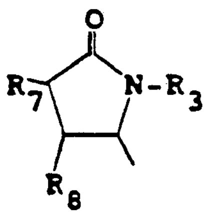
Q-6
,
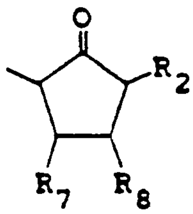
Q-8
,
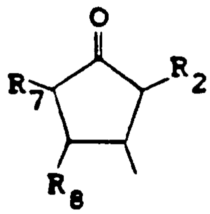
Q-9
,
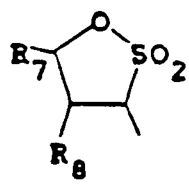
Q-10
,
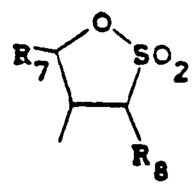
Q-11
,
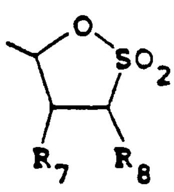
Q-12
,
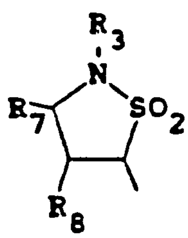
Q-13
,
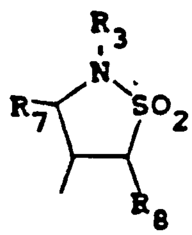
Q-14
,
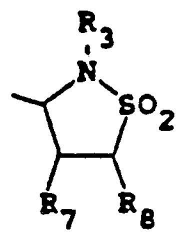
Q-15
,
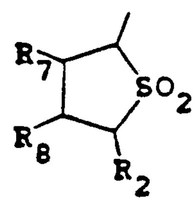
Q-17
,
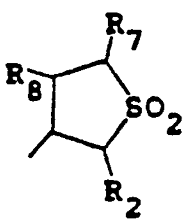
Q-18
,
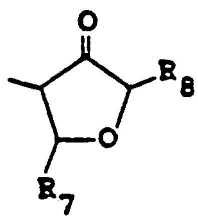
Q-19
,
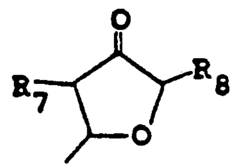
Q-20
,
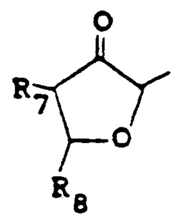
Q-21
,
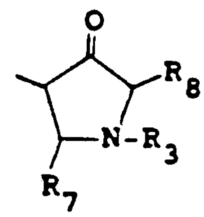
Q-22
,
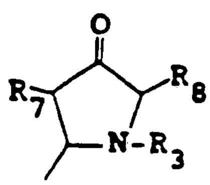
Q-23
,
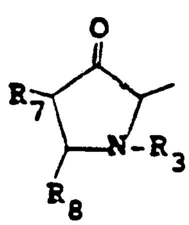
Q-24
,
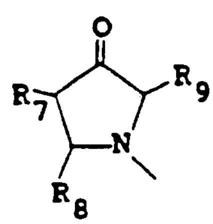
Q-25
,
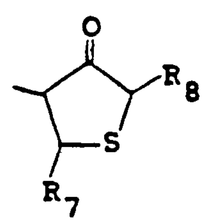
Q-26
,
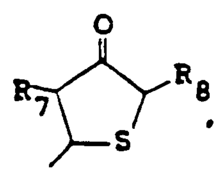
Q-27
,

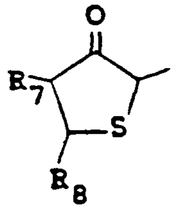

Q-28 ,

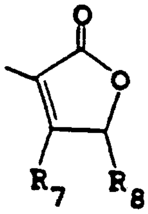

Q-29 ,

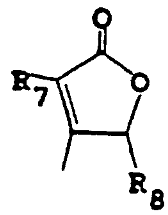

Q-30 ,

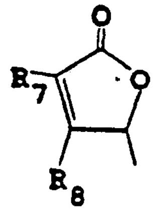

Q-31 ,

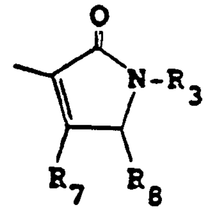

Q-32 ,

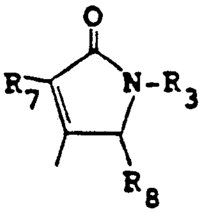

Q-33 ,

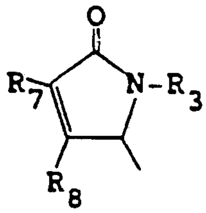

Q-34 ,

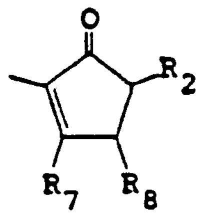

Q-36 ,

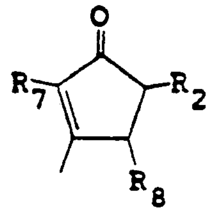

Q-37 ,

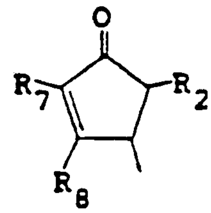

Q-38 ,

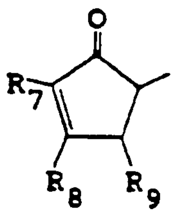

Q-39 ,

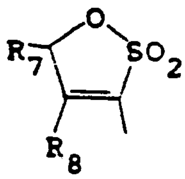

Q-40 ,

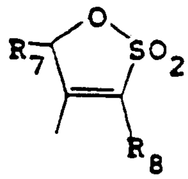

Q-41 ,

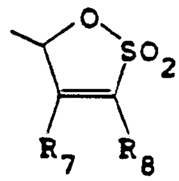

Q-42 ,

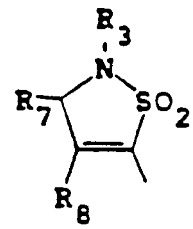

Q-43 ,

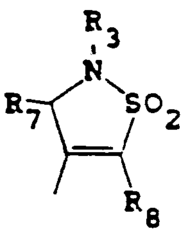

Q-44 ,

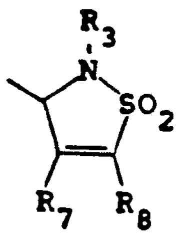

Q-45 ,

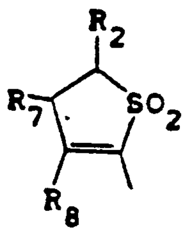

Q-47 ,

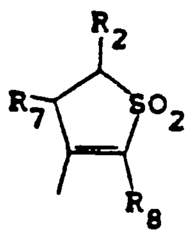

Q-48 ,

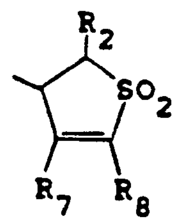

Q-49 ,

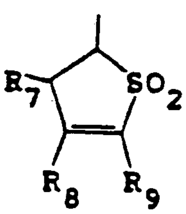

Q-50 ,

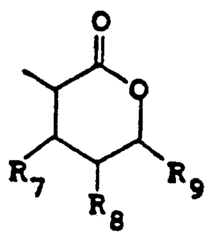

Q-51 ,

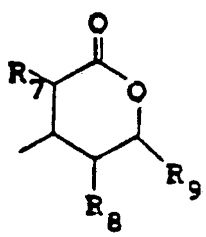

Q-52 ,

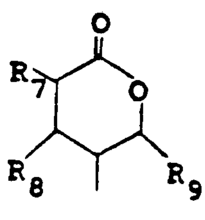

Q-53 ,

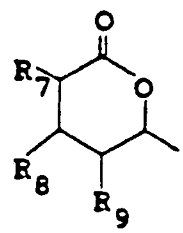

Q-54 ,

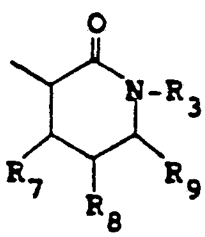

Q-55 ,

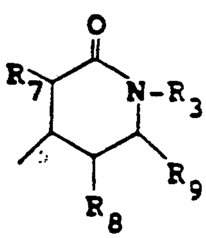

Q-56

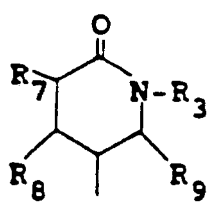

Q-57 ,

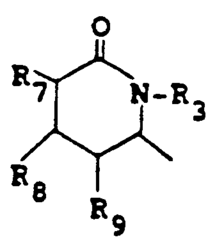

Q-58 ,

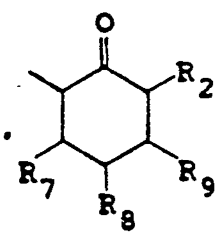

Q-60 ,

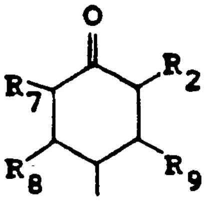
Q-61 ,

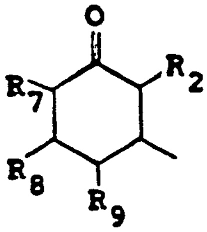
Q-62 ,

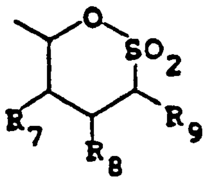
Q-63 ,

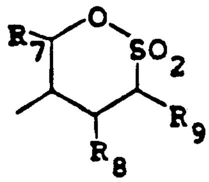
Q-64 ,

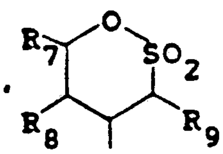
Q-65 ,

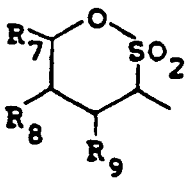
Q-66 ,

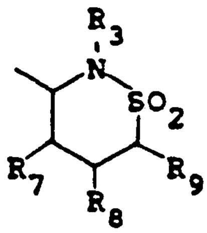
Q-67 ,

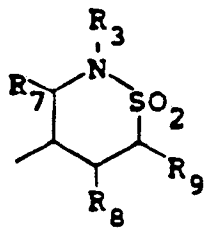
Q-68

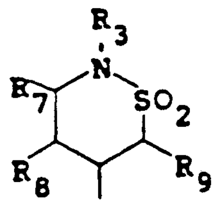
Q-69 ,

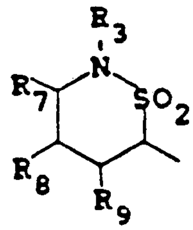
Q-70 ,

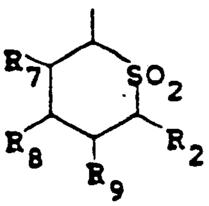
Q-72 ,

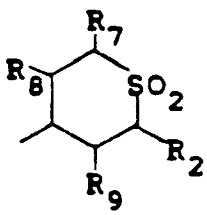
Q-73 ,

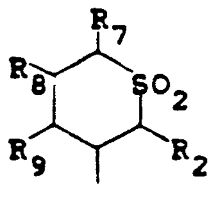
Q-74 ,

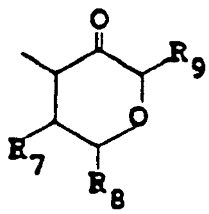
Q-75 ,

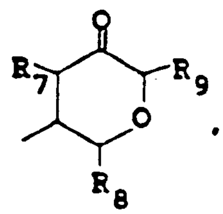
Q-76 ,

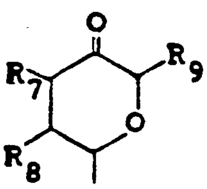
Q-77 ,

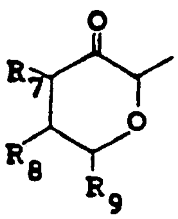
Q-78 ,

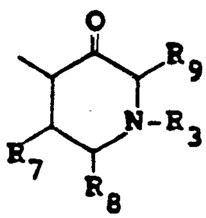
Q-79 ,

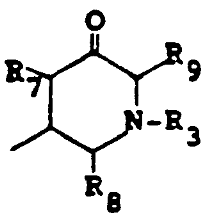
Q-80 ,

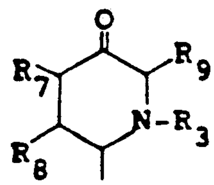
Q-81 ,

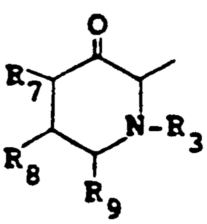
Q-82 ,

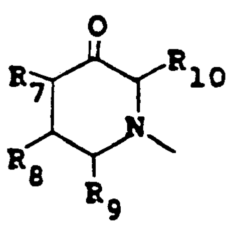
Q-83 ,

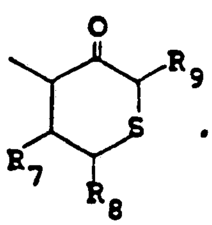
Q-84 ,

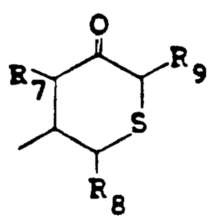
Q-85 ,

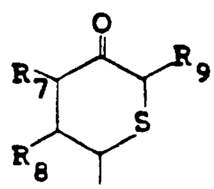
Q-86 ,

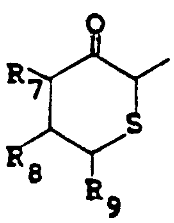
Q-87 ,

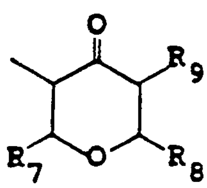
Q-88 ,

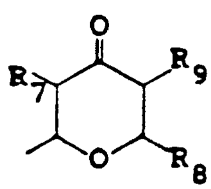
Q-89 ,

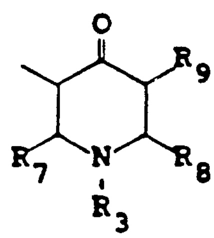
Q-90 ,

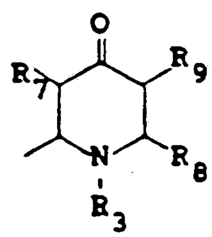
Q-91 ,

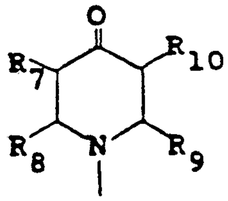

Q-92

,

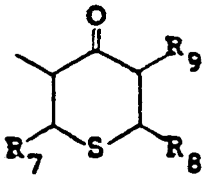

Q-93

,

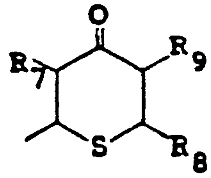

Q-94

,

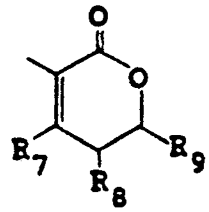

Q-95

,

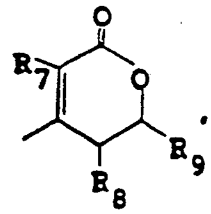

Q-96

,

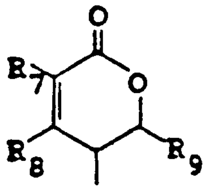

Q-97

,

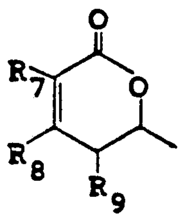

Q-98

,

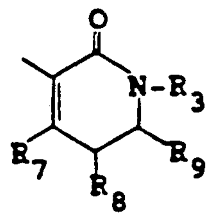

Q-99

,

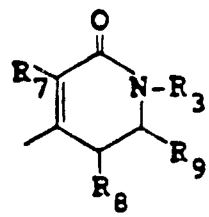

Q-100

,

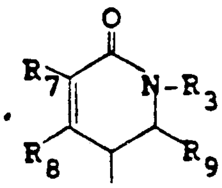

Q-101

,

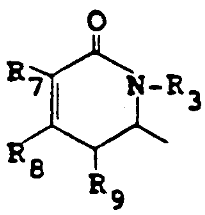

Q-102

,

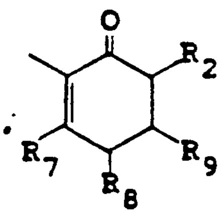

Q-104

,

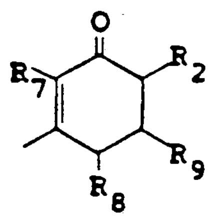

Q-105

,

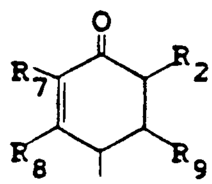

Q-106

,

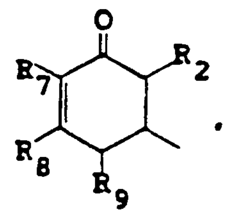

Q-107

,

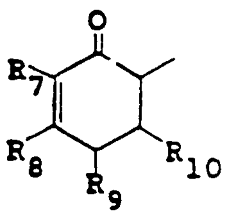

Q-108

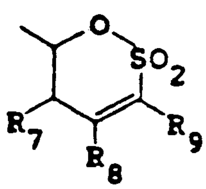

Q-109

,

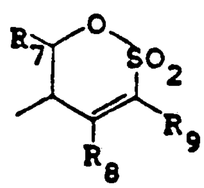

Q-110

,

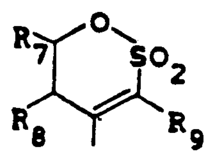

Q-111

,

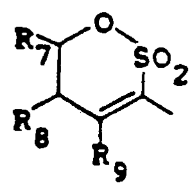

Q-112

,

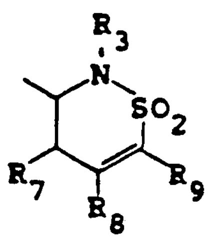

Q-113

,

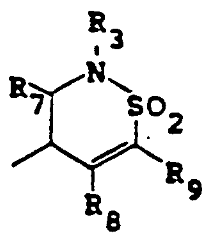

Q-114

,

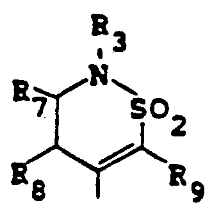

Q-115

,

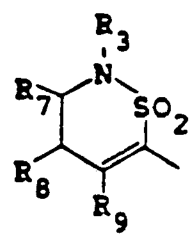

Q-116

,

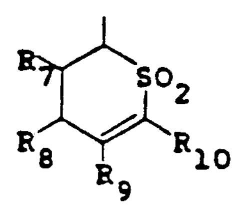

Q-118

,

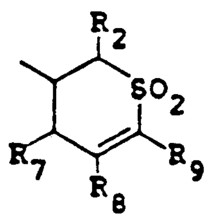

Q-119

,

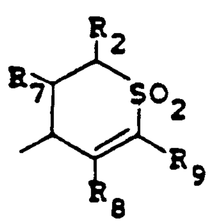

Q-120

,

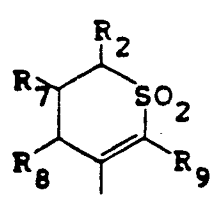

Q-121

,

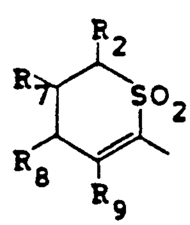

Q-122

,

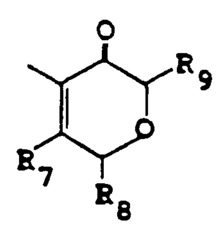

Q-123

,

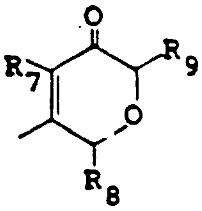

Q-124 ,

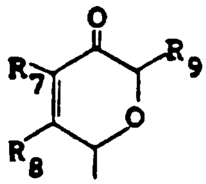

Q-125 ,

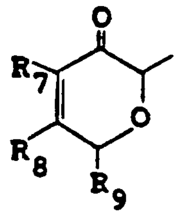

Q-126 ,

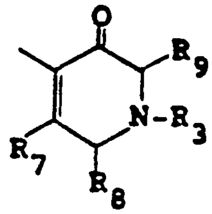

Q-127 ,

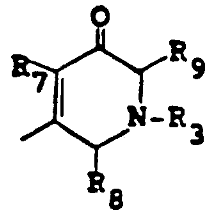

Q-128 ,

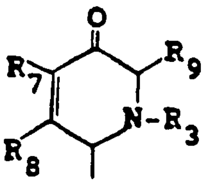

Q-129 ,

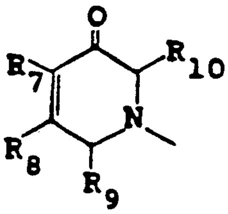

Q-130 ,

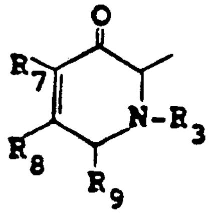

Q-131 ,

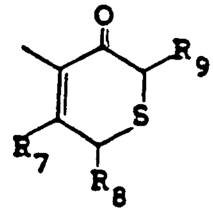

Q-132 ,

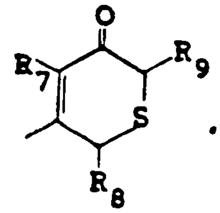

Q-133 ,

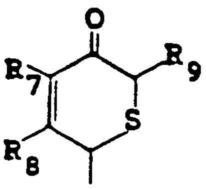

Q-134 ,

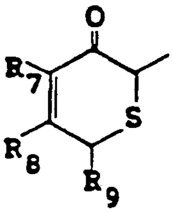

Q-135 ,

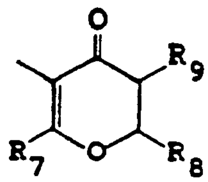

Q-136 ,

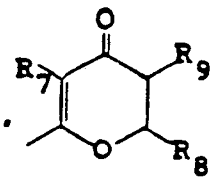

Q-137 ,

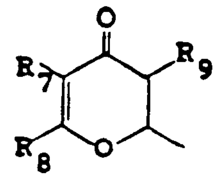

Q-138 ,

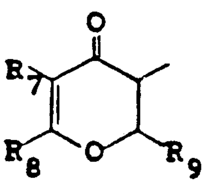

Q-139 ,

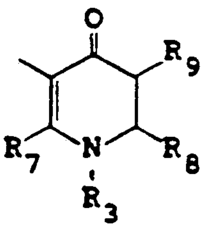

Q-140 ,

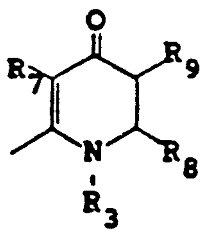

Q-141 ,

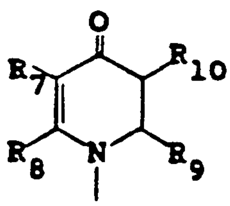

Q-142 ,

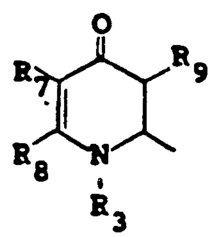

Q-143 ,

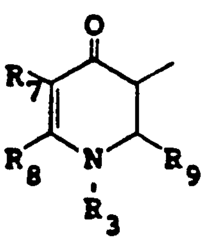

Q-144 ,

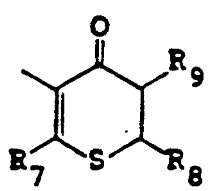

Q-145 ,

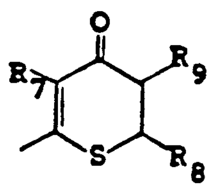

Q-146 ,

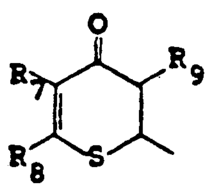

Q-147 ,

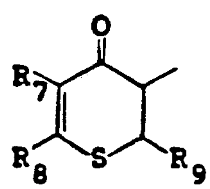

Q-148 ,

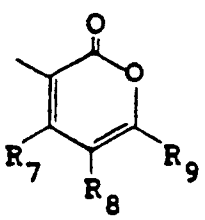

Q-149 ,

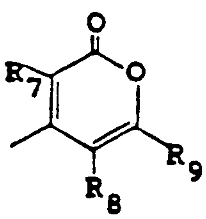

Q-150 ,

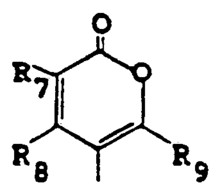

Q-151 ,

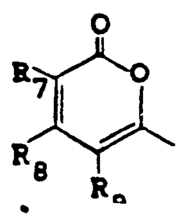

Q-152 ,

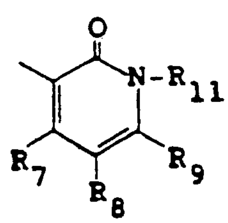

Q-153 ,

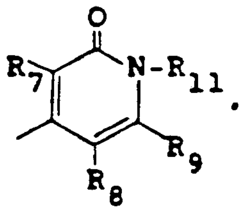
Q-154
,
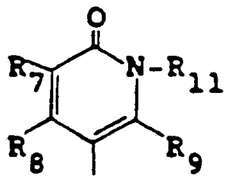
Q-155
,
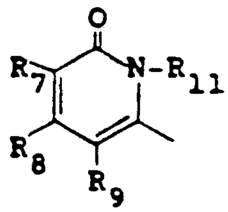
Q-156
,
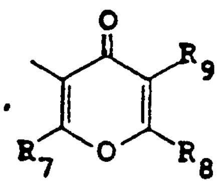
Q-158
,
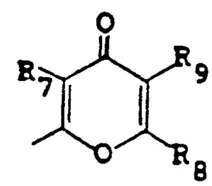
Q-159
,
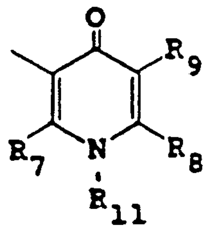
Q-160
,
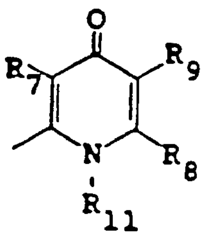
Q-161
,
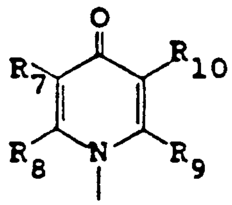
Q-162
,
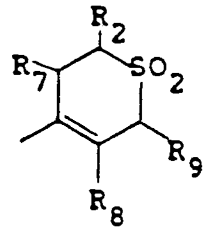
Q-163
,
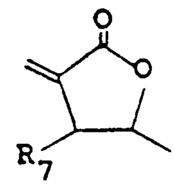
Q-164
,
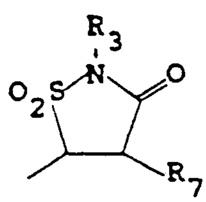
Q-165
,
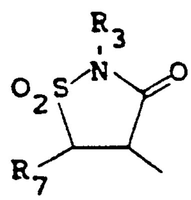
Q-166
or
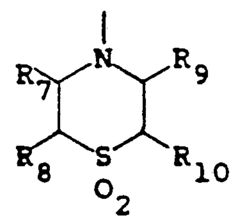
Q-167
;

$R_7$, $R_8$, $R_9$ et $R_{10}$ sont chacun indépendamment H ou $CH_3$; et
$R_{11}$ est H, $CH_3$ ou $CH_2CH_3$.

8. Composés de la revendication 7, où
J est J—5, J—6, J—8, J—9 ou J—11;
$R_{1b}$ est H, $CH_3$ ou Cl;
$R_{1c}$ est H
$R_{1d}$ est H, $CH_3$, $OCH_3$ ou Cl; et
Y est $CH_3$, $OCH_3$, $C_2H_5$, $OC_2H_5$, $CH_2OCH_3$, $NHCH_3$, $CH(OCH_3)_2$ ou cyclopropyle.

9. Composés de la revendication 8, où
$R_2$ est H ou $CH_3$; et
$R_3$ est H, $CH_3$ ou $C_2H_5$.

10. Composés de la revendication 9, où
A est A—1; et
X est $CH_3$, $OCH_3$, Cl ou $OCF_2H$.

11. Le composé de la revendication 1, qui est le N-[(4,6-diméthoxypyrimidine-2-yl)aminocarbonyl]-2-tétrahydro-2-oxo-3-furannylbenzènesulfonamide.

12. Le composé de la revendication 1, qui est le N-[(4-méthoxy-6-méthyltriazine-2-yl)aminocarbonyl]-2-tétrahydro-2-oxo-3-furannylbenzènesulfonamide.

13. Le composé de la revendication 1, qui est le N-[(4,6-diméthoxypyrimidine-2-yl)aminocarbonyl]-2-(tétrahydro-2-oxofuranne-3-yl)-3-thiophènesulfonamide.

14. Le composé de la revendication 1, qui est le N-5(4-méthoxy-6-méthyltriazine-2-yl)aminocarbonyl]-2-(tétrahydro-2-oxofuranne-3-yl)-3-thiophènesulfonamide.

15. Le composé de la revendication 1, qui est le N-[(4,6-diméthylpyrimidine-2-yl)aminocarbonyl]-2-(3-oxo-1-cyclohexène-1-yl)-3-thiophènesulfonamide.

16. Le composé de la revendication 1, qui est le N-[(4,6-diméthoxypyrimidine-2-yl)aminocarbonyl]-2-(tétrahydro-2-oxofuranne-3-yl)-3-pyridinesulfonamide

17. Le composé de la revendication 1, qui est le N-[(4-méthoxy-6-méthyltriazine-2-yl)aminocarbonyl]-2-(tétrahydro-2-oxofuranne-3-yl)-3-pyridinesulfonamide.

18. Le composé de la revendication 1, qui est le S,S-dioxyde de N-[(4,6-diméthoxypyrimidine-2-yl)aminocarbonyl]-2-(4-thiomorpholinyl)-3-pyridinesulfonamide.

19. Composés de la revendication 1, où
J est J—11;

$R_{1d}$ est H ou un groupe alkyle en $C_1$—$C_3$, halogénoalkyle en $C_1$—$C_3$, halogéno, nitro, alcoxy en $C_1$—$C_3$, alkylthio en $C_1$—$C_3$, alkylsulfinyle en $C_1$—$C_3$, alkylsulfonyle en $C_1$—$C_3$, $SO_2NR^IR^{II}$, $CO_2R^{III}$, amino, alkylamino en $C_1$—$C_3$ ou di(alkyl en $C_1$—$C_3$) amino;

$W_2$ n'est pas $SO_2$;

$E_1$, $E_2$, $E_3$ et $E_4$ peuvent être facultativement substitués par 1 à 4 groupes choisis parmi les groupes alkyles en $C_1$—$C_4$, alcoxy en $C_1$—$C_4$, halogéno et halogénoalkoxy en $C_1$—$C_4$;

Y n'est pas un groupe alkylsulfinylalkyle en $C_2$—$C_5$ ou alkylsulfonylalkyle en $C_2$—$C_5$;

et les substituants restants sont tels que définis dans la revendication 1.

20. Composés de la revendication 1, où

J est J—5, J—6 ou J—7;

$R_{1b}$ est H ou un groupe alkyle en $C_1$—$C_3$ halogénoalkyle en $C_1$—$C_3$, halogéno, nitro, alcoxy en $C_1$—$C_3$, $SO_2NR^IR^{II}$, alkylthio en $C_1$—$C_3$, alkylsulfinyle en $C_1$—$C_3$ alkylsulfonyle en $C_1$—$C_3$ ou $CO_2R^{III}$;

$W_2$ n'est pas $SO_2$; et

$E_1$, $E_2$, $E_3$, $E_4$ et Y sont tels que définis dans la revendication 19,

les substituants restants étant tels que définis dans la revendication 1.

21. Une composition convenant pour lutter contre la croissance de végétation indésirable, qui comprend une quantité efficace d'un composé de l'une quelconque des revendications 1 à 20 et au moins l'un des ingrédients suivants: agent tensio-actif, diluant solide ou liquide.

22. Un procédé pour lutter contre la croissance de végétation indésirable, qui consiste à appliquer à l'emplacement à protéger une quantité efficace d'un composé de l'une quelconque des revendications 1 à 20.

23. Un procédé pour réguler la croissance de plantes, qui consiste à appliquer à l'emplacement où se trouvent ces plantes une quantité efficace mais sensiblement non phytotoxique d'un régulateur de croissance des plantes choisi parmi les composés de l'une quelconque des revendications 1 à 20.

24. Un procédé pour la préparation d'un composé de la revendication 1, qui consiste à:

(a) faire réagir un sulfonyl isocyanate ou isothiocyanate de formule

$$J—SO_2—NCW_1 \quad \text{(II)}$$

avec une amine hétérocyclique de formule

$$A—NHR \quad \text{(III); ou}$$

(b) faire réagir un sulfonamide de formule

$$J—SO_2NH_2 \quad \text{(IV)}$$

avec l'ester de méthyle ou de phényle d'un acide pyrimidine- ou triazine-carbamique de formule

$$R^1O\overset{\overset{O}{\|}}{C}\underset{\underset{H}{|}}{N}A \quad \text{(V, VI)}$$

où J, A, R et $W_1$ sont tels que définis dans la revendication 1 et $R^1$ est Me ou Ph.

25. Composés de formules:

$$J—SO_2NH_2 \text{ et } J—SO_2NCW_1$$

où J et $W_1$ sont tels que définis dans la revendication 1.